(19) Europäisches Patentamt
European Patent Office
Office européen des brevets

(11) EP 1 260 586 A2

(12) # EUROPEAN PATENT APPLICATION

(43) Date of publication:
**27.11.2002 Bulletin 2002/48**

(21) Application number: **02013004.3**

(22) Date of filing: **23.02.1995**

(51) Int Cl.[7]: **C12N 15/52**, C12N 9/00, A61K 31/70, C07H 19/04, C07H 19/10, C07H 19/20, C12N 15/10, A61K 48/00, C12N 15/86, C12N 15/87

(84) Designated Contracting States:
**AT BE CH DE DK ES FR GB GR IE IT LI LU MC NL PT SE**

(30) Priority:
**23.02.1994 US 201109**
**29.03.1994 US 218934**
**04.04.1994 US 222795**
**07.04.1994 US 224483**
**15.04.1994 US 228041**
**15.04.1994 US 227958**
**18.05.1994 US 245736**
**06.07.1994 US 271280**
**15.08.1994 US 291932**
**16.08.1994 US 291433**
**17.08.1994 US 292620**
**19.08.1994 US 293520**
**02.09.1994 US 300000**
**08.09.1994 US 303039**
**23.09.1994 US 311486**
**23.09.1994 US 311749**
**28.09.1994 US 314397**
**03.10.1994 US 316771**
**07.10.1994 US 319492**
**11.10.1994 US 321993**
**04.11.1994 US 334847**
**10.11.1994 US 337608**
**28.11.1994 US 345516**
**16.12.1994 US 357577**
**23.12.1994 US 363233**
**30.01.1995 US 380734**

(62) Document number(s) of the earlier application(s) in accordance with Art. 76 EPC:
**95909920.1 / 0 746 614**

(71) Applicant: **RIBOZYME PHARMACEUTICALS, INC.
Boulder, CO 80301 (US)**

(72) Inventors:
- **Stinchcomb, Dan T.
Boulder, CO 80301 (US)**
- **Dudycz, Lech W.
Worcester, MA 01603 (US)**
- **Chowrira, Bharat
Boulder, CO 80301 (US)**
- **Grimm, Susan
Boulder, CO 80303 (US)**
- **Direnzo, Anthony
Boulder, CO 80303 (US)**
- **Karpeisky, Alexander
Boulder, CO 80301 (US)**
- **Draper, Kenneth G.
Boulder, CO 80301 (US)**
- **Kisich, Kevin
Lafayette, CO 80026 (US)**
- **Matulic-Adamic, Jasenka
Boulder, CO 80303 (US)**
- **McSwiggen, James A.
Boulder, CO 80301 (US)**
- **Modak, Anil
Boulder, CO 80301 (US)**
- **Pavco, Pamela
Lafayette, CO 80026 (US)**
- **Beigelman, Leonid
Longmont, CO 80503 (US)**
- **Sullivan, Sean M.
Alameda, CA 94501 (US)**
- **Sweedler, David
Louisville, CO 80027 (US)**
- **Thompson, James D.
Boulder, CO 80301 (US)**
- **Tracz, Danuta
Boulder, CO 80301 (US)**
- **Usman, Nassim
Boulder, CO 80304 (US)**
- **Wincott, Francine E.
Longmont, CO 80501 (US)**
- **Woolf, Tod
Watertown, MA 02172 (US)**

(74) Representative: **Viering, Jentschura & Partner
Steinsdorfstrasse 6
80538 München (DE)**

Remarks:
This application was filed on 12 - 06 - 2002 as a divisional application to the application mentioned under INID code 62.

(54) **Method and reagent for inhibiting the expression of disease related genes**

(57) Enzymatic RNA molecules which cleave ICAM-1 mRNA, IL-5 mRNA, *rel A* mRNA, TNF-α mRNA, RSV mRNA



EP 1 260 586 A2

or RSV genomic RNA, or CML associated mRNA, and use of these molecules for the treatment of pathological conditions related to those mRNA-levels; ribonucleosides or nucleotides modified in 2', 3' or 5', methods for their synthesis, purification and deprotection; vectors containing multiple enzymatic nucleic acids, optionally in chimeric form with tRNAs; method for introducing enzymatic nucleic acids into cells by forming a complex with a second nucleic acid, where the complex is capable of taking an R-loop base-paired structure; method for altering a mutant nucleic acid in vivo by hybridization with an oligonucleotide capable of activating ds RNA deaminase, comprising an enzymatic activity or a chemical mutagen. Further are disclosed trans-cleaving or -ligating hairpin ribozymes lacking a substrate RNA moiety, as well as hammerhead ribozymes having an interconnecting loop between base pairs in stem II.

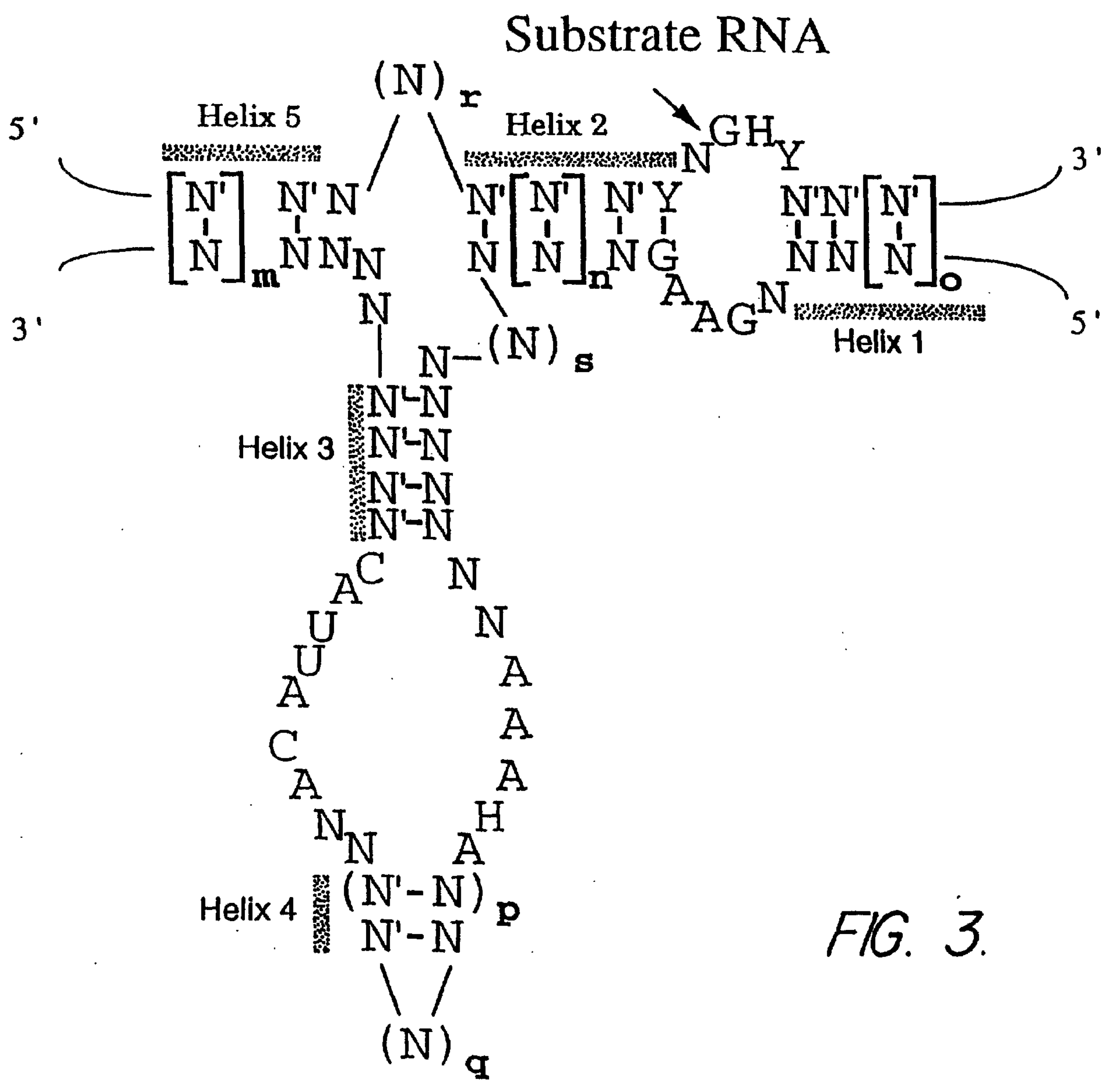


FIG. 3.

## Description

Background of the Invention

This invention relates to reagents useful as inhibitors of gene expression relating to diseases such as inflammatory or autoimmune disorders, chronic myelogenous leukemia, or respiratory tract illness.

Summary of the Invention

The invention features novel enzymatic RNA molecules, or ribozymes, and methods for their use for inhibiting the expression of disease related genes, e.g., ICAM-1, IL-5, relA, TNF-α, p210 $^{bcr-abl}$, and respiratory syncytial virus genes. Such ribozymes can be used in a method for treatment of diseases caused by the expression of these genes in man and other animals, including other primates.

Ribozymes are RNA molecules having an enzymatic activity which is able to repeatedly cleave other separate RNA molecules in a nucleotide base sequence specific manner. Such enzymatic RNA molecules can be targeted to virtually any RNA transcript, and efficient cleavage has been achieved *in vitro.* Kim et al., 84 Proc. Natl. Acad. Sci. USA 8788, 1987; Haseloff and Gerlach, 334 Nature 585, 1988; Cech, 260 JAMA 3030, 1988; and Jefferies et al., 17 Nucleic Acids Research 1371, 1989.

Six basic varieties of naturally-occurring enzymatic RNAs are known presently. Each can catalyze the hydrolysis of RNA phosphodiester bonds in trans (and thus can cleave other RNA molecules) under physiological conditions. Table 1 summarizes some of the characteristics of these ribozymes.

Ribozymes act by first binding to a target RNA. Such binding occurs through the target RNA binding portion of a ribozyme which is held in close proximity to an enzymatic portion of the RNA which acts to cleave the target RNA. Thus, the ribozyme first recognizes and then binds a target RNA through complementary base-pairing, and once bound to the correct site, acts enzymatically to cut the target RNA. Strategic cleavage of such a target RNA will destroy its ability to direct synthesis of an encoded protein. After a ribozyme has bound and cleaved its RNA target it is released from that RNA to search for another target and can repeatedly bind and cleave new targets.

The enzymatic nature of a ribozyme is advantageous over other technologies, such as antisense technology (where a nucleic acid molecule simply binds to a nucleic acid target to block its translation) since the effective concentration of ribozyme necessary to effect a therapeutic treatment is lower than that of an antisense oligonucleotide. The advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding, but also on the mechanism by which the molecule inhibits the expression of the RNA to which it binds. That is, the inhibition is caused by cleavage of the RNA target and so specificity is defined as the ration of the rate of cleavage of the targeted RNA over the rate of cleavage of non-targeted RNA. This cleavage mechanism is dependent upon factors additional to those involved in base pairing. Thus, it is thought that the specificity of action of a ribozyme is greater than that of antisense oligonucleotide binding the same RNA site. With their catalytic activity and increased site specificity, ribozymes represent more potent and safe therapeutic molecules than antisense oligonucleotides.

Thus, in a first aspect, this invention relates to ribozymes, or enzymatic RNA molecules, directed to cleave RNA species encoding ICAM-1, IL-5, relA, TNF-α, p210$^{bcr-abl}$, or RSV proteins. In particular, applicant describes the selection and function of ribozymes capable of cleaving these RNAs and their use to reduce levels of ICAM-1, IL-5, relA, TNF-α, p210 bor-abl or RSV proteins in various tissues to treat the diseases discussed herein. Such ribozymes are also useful for diagnostic uses.

Applicant indicates that these ribozymes are able to inhibit expression of ICAM-1, IL-5, rel A, TNF-α, p210$^{bcr-abl}$, or RSV genes and that the catalytic activity of the ribozymes is required for their inhibitory effect. Those of ordinary skill in the art, will find that it is clear from the examples described that other ribozymes that cleave target ICAM-1, IL-5, rel A, TNF-α, p210$^{bcr-abl}$, or RSV encoding mRNAs may be readily designed and are within the invention.

These chemically or enzymatically synthesized RNA molecules contain substrate binding domains that bind to accessible regions of their target mRNAs. The RNA molecules also contain domains that catalyze the cleavage of RNA. Upon binding, the ribozymes cleave the target encoding mRNAs, preventing translation and protein accumulation. In the absence of the expression of the target gene, a therapeutic effect may be observed.

By "gene" is meant to refer to either the protein coding regions of the cognate mRNA, or any regulatory regions in the RNA which regulate synthesis of the protein or stability of the mRNA; the term also refers to those regions of an mRNA which encode the ORF of a cognate polypeptide product, and the proviral genome.

By "enzymatic RNA molecule" it is meant an RNA molecule which has complementarity in a substrate binding region to a specified gene target, and also has an enzymatic activity which is active to specifically cleave RNA in that target. That is, the enzymatic RNA molecule is able to intermolecularly cleave RNA and thereby inactivate a target

RNA molecule. This complementarity functions to allow sufficient hybridization of the enzymatic RNA molecule to the target RNA to allow the cleavage to occur. One hundred percent complementarity is preferred, but complementarity as low as 50-75% may also be useful in this invention. By "equivalent" RNA to a virus is meant to include those naturally occurring viral encoded RNA molecules associated with viral caused diseases in various animals, including humans, cats, simians, and other primates. These viral or viral-encoded RNAs have similar structures and equivalent genes to each other.

By "complementarity" it is meant a nucleaic acid that can form hydrogen bond(s) with other RNA sequence by either traditional Watson-Crick or other non-traditional types (for examplke, Hoogsteen type) of base-paired interactions.

In preferred embodiments of this invention, the enzymatic nucleic acid molecule is formed in a hammerhead or hairpin motif, but may also be formed in the motif of a hepatitis delta virus, group I intron or RNaseP RNA (in associateion with an RNA guide sequence) or *Neurospora* VS RNA. Examples of such hammerhead motifs are described by Rossi *et al.,* 1992, *Aids Research and Human Retroviruses ,* 8,183, of hairpin motifs by Hampel and Tritz, 1989 *Biochemistry*, 28, 4929, EP 0360257 and Hampel et al., 1990, *Nucleic Acids Res.* 18,299 and an example of the hepatitis delta virus motif is described by Perotta and Been, 1992 *Biochemistry*, 31 16 of the RNaseP motif by Guerrier-Takada et al., 1983 *Cell*, 35 849, *Neurospora* VS RNA ribozyme motif is described by Collins (Seville and Collins, 1990 *Cell* 61, 685-696; Saville and Collins, 1991 Proc. Natl. Acad. Sci., USA 88, 8826-8830; Collins and Olive, 1993 *Biochemistry* 32, 2795-2799 Guo and Collins, 1995 EMBO. J., 14, 368) and of the Group I intron by Cech et al., U.S. Patent 4,987,071. These specific motifs are not limiting in the invention and those skilled in the art will recognize that all that is important in an enzymatic nucleic acid molecule of this invention is that it has a specific substrate binding site which is complementary to one or more of the target gene RNA regions, and that it has nucleotide sequences within or surrounding that substrate binding site which impart an RNA cleaving activity to the molecule.

The invention provides a method for producing a class of enzymatic cleaving agents which exhibit a high degree of specificity for the RNA of a desired target. The enzymatic nucleic acid molecule is preferably targeted to a highly conserved sequence region of a target (i.e., I CAM-1, IL-5, reLA, TNF-α, p210 bcr-abl or RSV proteins) encoding mRNA such that specific treatment of a disease or condition can be provided with either one or several enzymatic nucleic acids. Such enzymatic nucleic acid molecules can be delivered exogenously to specific cells as required., Alternatively, the ribozymes can be expressed from vectors that are delivered to specific cells. By "vectors" is meant any nucleic acid and/or viral-based technique used to deliver a desired nucleic acid.

Synthesis of nucleic acids greater than 100 nucleotides in length is difficult using automated methods, and the therapeutic cost of such molecules is prohibitive. In this invention small enzymatic nucleic acid motifs (e.g., of the hammerhead or the hairpin structure) are used for exogenous delivery. The simple structure of these molecules increases the ability of the enzymatic nucleic acid to invade targeted regions of the mRNA structrure. However, these catalytic RNA molecules can also be expressed within cells from eukaryotic promoters (e.g. Scanion, K.J. et al., 1991, *Proc. Natl. Acad. Sci., USA*, 88, 10591-5; Kashani-Sabet, M., et al., 1992, *Antisense Res. Dev.*, 2, 3-15; Dropoulic, B., et al., 1992, *J. Virol*, 66, 1432-41; Weerasinghe, M., et al., 191, *J. Virol.* 65, 5531-4; Ojwang, J.O., et al., 1992, *Proc. Natl. Acad. Sci., USA*, 89 10802-6; Chen C.J., et al., 1992, Nucleic Acids Res., 20, 4581-9; Sarver, H., et al., 1990 *Science*, 247, 1222-1225). Those skilled in the art would realize that any ribozyme can be expressed in eukaryotic cells from the appropriate DNA or RNA vector. The activity of such ribozymes can be augmented by their release from the primary transcript by a second ribozyme (Draper et al., PCT WO93/23569, and Sullivan et al., PCT WO94/02595, both hereby incorporated in their totality by reference herein; Ohkawa, J., et al., 1992, *Nucleic Acids Symp. Ser.* 27, 15-6; Taira, K. et al., *Nucleic Acids Res.*, 19, 5125-30; Ventura, M., et al., 1993, *Nucleic Acids Res.*, 21, 3249-55, Chowrira et al., 1994 *J. Biol. Chem.*, 269, 25856 ).

By "inhibit" is meant that the activity or level of ICAM-1,Rel A, IL-5, TNF-α, $p210^{bcr\text{-}abl}$ or RSV encoding mRNA is reduced below that observed in the absense of the ribozyme, and preferably is below that level observed in the presence of an inactive RNA molecule able to bind to the same site on the mRNA, but unable to cleave that RNA.

Such ribozymes are useful for the prevention of the diseases and conditions discussed above, and any other diseases or conditions that are related to the level of ICAM-1, IL-5, Rel A, TNF-α, $p210^{bcr\text{-}abl}$ or RSV protein or activity in a cell or tissue. By "related" is meant that the inhibition of ICAM-1, IL-5, Rel A, TNF-α, $p210^{bcr\text{-}abl}$ or RSV mRNA translation, and thus reduction in the level of, ICAM-1, IL-5, Rel A, TNF-α, $p210^{bcr\text{-}abl}$ or RSV proteins will relieve to some extent the symptoms of the disease or condition.

Ribozymes are added directly, or can be complexed with cationic lipids, packaged within liposomes, or otherwise delivered to target cells. The RNA or RNA complexes can be locally administered to relevant tissues through the use of a catheter, infusion pump or stent, with or without their incorporation in biopolymers. In preferred embodiments, the ribozymes have binding arms which are complementary to the sequences in Tables 2,3,6-9, 11, 13, 15-23, 27, 28, 31, 33, 34, 36 and 37.

Examples of such ribozymes are shown in Tables 4-8, 10, 12, 14-16, 19-22, 24, 26-28, 30, 32, 34 and 36-38. Examples of such ribozymes consist essentially of sequences defined in these Tables. By "consists essentially of" is

meant that the active ribozyme contains an enzymatic center equivalent to those in the examples, and binding arms able to bind mRNA such that cleavage at the target site occurs. Other sequences may be present which do not interfere with such cleavage.

Those in the art will recognize that these sequences are representative only of many more such sequences where the enzymatic portion of the ribozyme (all but the binding arms) is altered to affect activity. For example, stem-loop II sequence of hammerhead ribozymes listed in the above identified Tables can be altered (substitution, deletion, and/or insertion) to contain any sequences provided a minimum of two base-paired stem structure can form. Similarly, stem-loop IV sequence of hairpin ribozymes listed in the above identified Tables can be altered (substitution, deletion, and/or insertion) to contain any sequence, provided a minimum of two base-paired stem structure can form. The sequence listed in the above identified Tables may be formed of ribonucleotides or other nucleotides or non-nucleotides. Such ribozymes are equivalent to the ribozymes described specifically in the Tables.

In another aspect of the invention, ribozymes that cleave target molecules and inhibit ICAM-1, IL-5, Rel A, TNF-$\alpha$, p210$^{bcr\text{-}abl}$ or RSV gene expression are expressed from transcription units inserted into DNA, RNA, or viral vectors. Another means of accumulating high concentrations of a ribozyme(s) within cells is to incorporate the ribozyme-encoding sequences into a DNA or RNA expression vector. Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990 *Proc. Natl. Acad. Sci. USA*, 87, 6743-7; Gao and Huang 1993 *Nucleic Acids Res., 21* 2867-72; Lieber et al., 1993 *Methods Enzymol.,* 217, 47-66; Zhou et al., 1990 *Mol. Cell. Biol*., 10, 4529-37). Several investigators have demonstrated that ribozymes expressed from such promoters can function in mammalian cells (e.g. Kashani-Sabet et al., 1992 *Antisense Res. Dev.,* 2, 3-15; Ojwang et al., 1992 *Proc. Natl. Acad. Sci. USA*, 90, 6340-4; L'Huiller et al., 1992 *EMBO J.* 11, 4411-8; Lisziewicz et al., 1993 *Proc. Natl. Acad. Sci. U.S.A*., 90 8000-4). The above ribozyme transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors).

Other features and advantages of the invention will be apparent from the following description of the preferred embodiments thereof, and from the claims.

## Description Of The Preferred Embodiments

The drawings will first briefly be described.

## Drawings:

Figure 1 is a diagrammatic representation of the hammerhead ribozyme domain known in the art. Stem II can be ≥ 2 base-pair long. Figure 2(a) is a diagrammatic representation of the hammerhead ribozyme domain known in the art; Figure 2(b) is a diagrammatic representation of the hammerhead ribozyme as divided by Uhlenbeck (1987, *Nature*, 327, 596-600) into a substrate and enzyme portion; Figure 2(c) is a similar diagram showing the hammerhead divided by Haseloff and Gerlach (1988, *Nature*, 334, 585-591) into two portions; and Figure 2(d) is a similar diagram showing the hammerhead divided by Jeffries and Symons (1989, *Nucl. Acids. Res.,* 17, 1371-1371) into two portions.

Figure 3 is a diagrammatic representation of the general structure of a hairpin ribozyme. Helix 2 (H2) is provided with a least 4 base pairs (*i.e., n* is 1,2,3 or 4) and helix 5 can be optionally provided of length 2 or more bases (preferably 3-20 bases, *i.e.*, m is from 1-20 or more). Helix 2 and helix 5 may be covalently linked by one or more bases (*i.e.*, r is ≥ 1 base). Helix 1, 4 or 5 may also be extended by 2 or more base pairs (*e.g.*, 4-20 base pairs) to stabilize the ribozyme structure, and preferably is a protein binding site. In each instance, each N and N' independently is any normal or modified base and each dash represents a potential base-pairing interaction. These nucleotides may be modified at the sugar, base or phosphate. Complete base-pairing is not required in the helices, but is preferred. Helix 1 and 4 can be of any size (*i.e.*, o and p is each independently from 0 to any number, *e.g.* 20) as long as some base-pairing is maintained. Essential bases are shown as specific bases in the structure, but those in the art will recognize that one or more may be modified chemically (abasic, base, sugar and/or phosphate modifications) or replaced with another base without significant effect. Helix 4 can be formed from two separate molecules, *i.e.,* without a connecting loop. The connecting loop when present may be a ribonucleotide with or without modifications to its base, sugar or phosphate. "q" is ≥ 2 bases. The connecting loop can also be replaced with a non-nucleotide linker molecule. H refers to bases A, U, or C. Y refers to pyrimidine bases. "—" refers to a covalent bond.

Figure 4 is a representation of the general structure of the hepatitis delta virus ribozyme domain known in the art.

Figure 5 is a representation of the general structure of the self-cleaving VS RNA ribozyme domain.

Figure 6 is a diagrammatic representation of the genetic map of RSV strain A2.

Figure 7 is a diagrammatic representation of the solid-phase synthesis of RNA.

Figure 8 is a diagrammatic representation of exocyclic amino protecting groups for nucleic acid synthesis.

Figure 9 is a diagrammatic representation of the deprotection of RNA.

Figure 10 is a graphical representation of the cleavage of an RNA substrate by ribozymes synthesized, deprotected and purified using the improved methods described herein.

Figure 11 is a schematic representation of a two pot deprotection protocol. Base deprotection is carried out with aqueous methyl amine at 65 °C for 10 min. The sample is dried in a speed-vac for 2-24 hours depending on the scale of RNA synthesis. Silyl protecting group at the 2'-hydroxyl position is removed by treating the sample with 1.4 M anhydrous HF at 65°C for 1.5 hours.

Figure 12 is a schematic representation of a one pot deprotection of RNA synthesized using RNA phosphoramidite chemistry. Anhydrous methyl amine is used to deprotect bases at 65°C for 15 min. The sample is allowed to cool for 10 min before adding TEA•3HF reagent, to the same pot, to remove protecting groups at the 2'-hydroxyl position. The deprotection is carried out for 1.5 hours.

Figs. 13a - b is a HPLC profile of a 36 nt long ribozyme, targeted to site B. The RNA is deprotected using either the two pot or the one pot deprotection protocol. The peaks corresponding to full-length RNA is indicated. The sequence for site B is CCUGGGCCAGGGAUUA AUGGAGAUGCCCACU.

Figure 14 is a graph comparing RNA cleavage activity of ribozymes deprotected by two pot vs one pot deprotection protocols.

Figure 15 is a schematic representation of an improved method of synthesizing RNA containing phosphorothioate linkages.

Figure 16 shows RNA cleavage reaction catalyzed by ribozymes containing phosphorothioate linkages. Hammerhead ribozyme targeted to site C is synthesized such that 4 nts at the 5' end contain phosphorothioate linkages. P=O refers to ribozyme without phosphorothioate linkages. P=S refers to ribozyme with phosphorothioate linkages. The sequence for site C is UCAUUUUGGCCAUCUC UUCCUUCAGGCGUGG.

Figure 17 is a schematic representation of synthesis of 2'-N-phtalimido-nucleoside phosphoramidite.

Figure 18 is a diagrammatic representation of a prior art method for the solid-phase synthesis of RNA using silyl ethers, and the method of this invention using SEM as a 2'-protecting group.

Figure 19 is a diagrammatic representation of the synthesis of 2'-SEM-protected nucleosides and phosphoramidites useful for the synthesis of RNA. B is any nucleotide base as exemplified in the Figure, P is purine and I is inosine. Standard abbreviations are used throughout this application, well known to those in the art.

Figure 20 is a diagrammatic representation of a prior art method for deprotection of RNA using TBDMS protection of the 2'-hydroxyl group.

Figure 21 is a diagrammatic representation of the deprotection of RNA having SEM protection of the 2'-hydroxyl group.

Figure 22 is a representation of an HPLC chromatogram of a fully deprotected 10-mer of uridylic acid.

Figs. 23 - 25 are diagrammatic representations of hammerhead, hairpin or hepatitis delta virus ribozyme containing self-processing RNA transcript. Solid arrows indicate self-processing sites. Boxes indicate the sites of nucleotide substitution. Solid lines are drawn to show the binding sites of primers used in a primer-extension assay. Lower case letters indicate vector sequence present in the RNA when transcribed from a *Hin*dIII-linearized plasmid. (**23**) HH Cassette, transcript containing the hammerhead trans-acting ribozyme linked to a 3' cis-acting hammerhead ribozyme. The structure of the hammerhead ribozyme is based on phylogenetic and mutational analysis (reviewed by Symons, 1992 supra). The trans ribozyme domain extends from nucleotide 1 through 49. After 3'-end processing, the trans-ribozyme contains 2 non-ribozyme nucleotides (UC at positions 50 and 51) at its 3' end. The 3' processing ribozyme is comprised of nucleotides 44 through 96. Roman numerals I, II and III, indicate the three helices that contribute to the structure of the 3' cis-acting hammerhead ribozyme (Hertel et al., 1992 Nucleic Acids Res. 20, 3252). Substitution of $G_{70}$ and $A_{71}$ to U and G respectively, inactivates the hammerhead ribozyme (Ruffner et al., 1990 Biochemistry 29, 10695) and generates the HH(mutant) construct. **(24)** HP Cassette, transcript containing the hammerhead trans-acting ribozyme linked to a 3' cis-acting hairpin ribozyme. The structure of the hairpin ribozyme is based on phylogenetic and mutational analysis (Berzal-Herranz et al., 1993 EMBO. J 12, 2567). The trans-ribozyme domain extends from nucleotide 1 through 49. After 3'-end processing, the trans-ribozyme contains 5 non-ribozyme nucleotides (UGGCA at positions 50 to 54) at its 3' end. The 3' cis-acting ribozyme is comprised of nucleotides 50 through 115. The transcript named HP(GU) was constructed with a potential wobble base pair between $G_{52}$ and $U_{77}$; HP(GC) has a Watson-Crick base pair between $G_{52}$ and $C_{77}$. A shortened helix 1 (5 base pairs) and a stable tetraloop (GAAA) at the end of helix 1 was used to connect the substrate with the catalytic domain of the hairpin ribozyme (Feldstein & Bruening, 1993 Nucleic Acids Res. 21, 1991; Altschuler et al., 1992 supra). (**25**) HDV Cassette, transcript containing the trans-acting hammerhead ribozyme linked to a 3' cis-acting hepatitis delta virus (HDV) ribozyme. The secondary structure of the

HDV ribozyme is as proposed by Been and coworkers (Been et al., 1992 Biochemistry 31, 11843). The trans-ribozyme domain extends from nucleotides 1 through 48. After 3'-end processing, the trans-ribozyme contains 2 non-ribozyme nucleotides (AA at positions 49 to 50) at its 3' end. The 3' cis-acting HDV ribozyme is comprised of nucleotides 50 through 114. Roman numerals I, II, III & IV, indicate the location of four helices within the 3' cis-acting HDV ribozyme (Perrota & Been, 1991 Nature 350, 434). The ΔHDV transcript contains a 31 nucleotide deletion in the HDV portion of the transcript (nucleotides 84 through 115 deleted).

Fig. 26 is a schematic representation of a plasmid containing the insert encoding self-processing cassette. The figure is not drawn to scale.

Fig. 27 demonstrates the effect of 3' flanking sequences on RNA self-processing *in vitro.* **H**, Plasmid templates linearized with *Hind*III restriction enzyme. Transcripts from **H** templates contain four non-ribozyme nucleotides at the 3' end. **N**, Plasmid templates linearized with *NdeI* restriction enzyme. Transcripts from **N** templates contain 220 non-ribozyme nucleotides at the 3' end. **R**, Plasmid templates linearized with *RcaI* restriction enzyme. Transcripts from **R** templates contain 450 non-ribozyme nucleotides at the 3' end.

Fig. 28 shows the effect of 3' flanking sequences on the trans-cleavage reaction catalyzed by a hammerhead ribozyme. A 622 nt internally-labeled RNA (<10 nM) was incubated with ribozyme (1000 nM) under single turn-over conditions (Herschlag and Cech, 1990 Biochemistry 29, 10159). **HH+2, HH+37,** and **HH+52** are trans-acting ribozymes produced by transcription from the HH, ΔHDV, and HH(mutant) constructs, respectively, and that contain 2, 37 and 52 extra nucleotides on the 3' end. The plot of the fraction of uncleaved substrate versus time was fit to a double exponential curve using the KaleidaGraph graphing program (Synergy Software, Reading, PA). A double exponential curve fit was used because the data points did not fall on a single exponential curve, presumably due to varying conformers of ribozyme and/or substrate RNA.

Fig. 29 shows RNA self-processing in OST7-1 cells. *In vitro* lanes contain full-length, unprocessed transcripts that were added to cellular lysates prior to RNA extraction. These RNAs were either pre-incubated with $MgCl_2$ (+) or with DEPC-treated water (-) prior to being hybridized with 5' end-labeled primers. Cellular lanes contain total cellular RNA from cells transfected with one of the four self-processing constructs. Cellular RNA are probed for ribozyme expression using a sequence specific primer-extension assay. Solid arrows indicate the location of primer extension bands corresponding to Full-Length RNA and 3' Cleavage Products.

Figs. 30,31 are diagrammatic representations of self-processing cassettes that will release trans-acting ribozymes with defined, stable stem-loop structures at the 5' and the 3' end following self-processing. 30, shows various permutations of a hammerhead self-processing cassette. 31, shows various permutations of a hairpin self-processing cassette.

Figs. 32a-b Schematic representation of RNA polymerse III promoter structure. Arrow indicates the transcription start site and the direction of coding region. A, B and C, refer to consensus A, B and C box promoter sequences. I, refers to intermediate cis-acting promoter sequence. PSE, refers to proximal sequence element. DSE, refers to distal sequence element. ATF, refers to activating transcription factor binding element. ?, refers to cis-acting sequence element that has not been fully characterized. EBER, Epstein-Barr-virus-encoded-RNA. TATA is a box well known in the art.

Figs. 33a-e Sequence of the primary $tRNA_i^{met}$ and Δ3-5 transcripts. The A and B box are internal promoter regions necessary for pol III transcription. Arrows indicate the sites of endogenous tRNA processing. The Δ3-5 transcript is a truncated version of tRNA wherein the sequence 3' of B box has been deleted (Adeniyi-Jones et al., 1984 *supra).* This modification renders the Δ 3-5 RNA resistant to endogenous tRNA processing.

Figure 34. Schematic representation of RNA structural motifs inserted into the Δ3-5 RNA. Δ3-5/HHI- a hammerhead (HHI) ribozyme was cloned at the 3' region of Δ3-5 RNA; S3- a stable stem-loop structure was incorporated at the 3' end of the Δ3-5/HHI chimera; S5- stable stem-loop structures were incorporated at the 5' and the 3' ends of Δ3-5/HHI ribozyme chimera; S35- sequence at the 3' end of the Δ3-5/HHI ribozyme chimera was altered to enable duplex formation between the 5' end and a complementary 3' region of the same RNA; S35Plus- in addition to structural alterations of S35, sequences were altered to facilitate additional duplex formation within the non-ribozyme sequence of the Δ3-5/HHI chimera.

Figures 35 and 36. Northern analysis to quantitate ribozyme expression in T cell lines transduced with Δ3-5 vectors. 35) Δ3-5/HHI and its variants were cloned individually into the DC retroviral vector (Sullenger et al., 1990 *supra*). Northern analysis of ribozyme chimeras expressed in MT-2 cells was performed. Total RNA was isolated from cells (Chomczynski & Sacchi, 1987 *Analytical Biochemistry* 162, 156-159), and transduced with various constructs described in Fig. 34. Northern analysis was carried out using standard protocols (*Curr. Protocols Mol. Biol.* 1992, ed. Ausubel et al., Wiley & Sons, NY). Nomenclature is same as in Figure 34. This assay measures the level of expression from the type 2 pol III promoter. 36) Expression of S35 constructs in MT2 cells. S35 (+ribozyme), S35 construct containing HHI ribozyme. S35 (-ribozyme), S35 construct containing no ribozyme.

Figure 37. Ribozyme activity in total RNA extracted from transduced MT-2 cells. Total RNA was isolated from cells transduced with Δ3-5 constructs described in Figs. 35 and 36 In a standard ribozyme cleavage reaction, 5 μg total RNA and trace amounts of 5' terminus-labeled ribozyme target RNA were denatured separately by heating to

90°C for 2 min in the presence of 50 mM Tris-HCl, pH 7.5 and 10 mM $MgCl_2$. RNAs were renatured by cooling the reaction mixture to 37°C for 10-15 min. Cleavage reaction was initiated by mixing the labeled substrate RNA and total cellular RNA at 37°C. The reaction was allowed to proceed for - 18h, following which the samples were resolved on a 20 % urea-polyacrylamide gel. Bands were visualized by autoradiography.

Figures 38 and 39. Ribozyme expression and activity levels in S35-transduced clonal CEM cell lines. 38) Northern analysis of S35-transduced clonal CEM cell lines. Standard curve was generated by spiking known concentrations of in vitro transcribed S5 RNA into total cellular RNA isolated from non-transduced CEM cells. Pool, contains RNA from pooled cells transduced with S35 construct. Pool (-G418 for 3 Mo), contains RNA from pooled cells that were initially selected for resistance to G418 and then grown in the absence of G418 for 3 months. Lanes A through N contain RNA from individual clones that were generated from the pooled cells transduced with S35 construct. $tRNA_i^{met}$, refers to the endogenous tRNA. S35, refers to the position of the ribozyme band. M, marker lane. 39) Activity levels in S35-transduced clonal CEM cell lines. RNA isolation and cleavage reactions were as described in Fig.37. Nomenclature is same as in Figs. 35 and 36 except, S, 5' terminus-labeled substrate RNA. P, 8 nt 5' terminus-labeled ribozyme-mediated RNA cleavage product.

Figures 40 and 41 are proposed secondary structures of S35 and S35 containing a desired RNA (HHI), respectively. The position of HHI ribozyme is indicated in figure 41. Intramolecular stem refers to the stem structure formed due to an intramolecular base-paired interaction between the 3' sequence and the complementary 5' terminus. The length of the stem ranges from 15-16 base-pairs. Location of the A and the B boxes are shown.

Figures 42 and 43 are proposed secondary structures of S35 plus and S35 plus containing HHI ribozyme.

Figures 44, 45, 46 and 47 are the nucleotide base sequences of S35, HHIS35, S35 Plus, and HHIS35 Plus respectively.

Figs. 48a-b is a general formula for pol III RNA of this invention.

Figure 49 is a digrammatic representation of 5T construct. In this construct the desired RNA is located 3' of the intramolecular stem.

Figures 50 and 51 contain proposed secondary structures of 5T construct alone and 5T contruct containing a desired RNA (HHI ribozyme) respectively.

Figure 52 is a diagrammatic representation of TRZ-tRNA chimeras. The site of desired RNA insertion is indicated.

Figure 53 shows the general structure of HHITRZ-A ribozyme chimera. A hammerhead ribozyme targeted to site I is inserted into the stem II region of TRZ-tRNA chimera.

Figure 54 shows the general structure of HPITRZ-A ribozyme chimera. A hairpin ribozyme targeted to site I is cloned into the indicated region of TRZ-tRNA chimera.

Figure 55 shows a comparison of RNA cleavage activity of HHITRZ-A, HHITRZ-B and a chemically synthesized HHI hammerhead ribozymes.

Figure 56 shows expression of ribozymes in T cell lines that are stably transduced with viral vectors. M, markers; lane 1, non-transduced CEM cells; lanes 2 and 3, MT2 and CEM cells transduced with retroviral vectors; lanes 4 and 5, MT2 and CEM cells transduced with AAV vectors.

Figs. 57a-b Schematic diagram of adeno-associated virus and adenovirues vectors for ribozyme delivery. Both vectors utilize one or more ribozyme encoding transcription units (RZ) based on RNA polymerase II or RNA polymerase III promoters. A. Diagram of an AAV-based vector containing minimal AAV sequences comprising the inverted terminal repeats (ITR) at each end of the vector genome, an optional selectable marker (Neo) driven by an exogenous promoter (Pro), a ribozyme transcription unit, and sufficient additional sequences (stuffer) to maintain a vector length suitable for efficient packaging. B. Diagram of ribozyme expressing adenovirus vectors containing deletions of one or more wild type adenoviorus coding regions (cross-hatched boxes marked as E1, pIX, E3, and E4), and insertion of the ribozyme transcription unit at any or several of those regions of deletions.

Fig. 58 is a graph showing the effect of arm length variation on the activity of ligated hammerhead (HH) ribozymes. Nomenclature 5/5, 6/6, 7/7, 8/8 and so on refers to the number of base-pairs being formed between the ribozyme and the target. For example, 5/8 means that the HH ribozyme forms 5 bp on the 5' side and 8 bp on the 3' side of the cleavage site for a total of 13 bp. $-\Delta G$ refers to the free energy of binding calculated for base-paired interactions between the ribozyme and the substrate RNA (Turner and Sugimoto, 1988 Ann. Rev. Biophys. Chem. 17, 167). RPI A is a HH ribozyme with 6/6 binding arms.

Figs. 59 and 60 and 61 show cleavage of long substrate (622 nt) by ligated HH ribozymes.

Fig. 62 is a diagrammatic representation of a hammerhead ribozyme (HH-H) targeted against a site termed H. Variants of HH-H are also shown that contain either a 2 base-paired stem II (HH-H1 and HH-H2) or a 3 base-paired stem II (HH-H3 and HH-H4).

Figs. 63 and 64 show RNA cleavage activity of HH-I and its variants (see Fig.62). 63) cleavage of matched substrate RNA (15 nt). 64) cleavage of long substrate RNA (613 nt).

Figs. 65a-b is a schematic representation of a method of this invention to synthesize a full length hairpin

ribozyme. No splint strand is required for ligation but rather the two fragments hybridize together at helix 4 prior to ligation. The only prerequisite is that the 3' fragment is phosphorylated at its 5' end and that the 3' end of the 5' fragment have a hydroxyl group. The hairpin ribozyme is targeted against site J. H1 and H2 are intermolecular helices formed between the ribozyme and the substrate. H3 and H4 are intramolecular helices formed within the hairpin ribozyme motif. Arrow indicates the cleavage site.

Fig. 66 shows RNA cleavage activity of ligated hairpin ribozymes targeted against site J.

Figs. 67a-b is a diagrammatic representation of a Site K Hairpin Ribozyme (HP-K) showing the proposed secondary structure of the hairpin ribozyme •substrate complex as described in the art (Berzal-Herranz *et al*., 1993 *EMBO. J*.12, 2567). The ribozyme has been assembled from two fragments (bimolecular ribozyme; Chowrira and Burke, 1992 *Nucleic Acids Res.* 20, 2835); #H1 and H2 represent intermolecular helix formation between the ribozyme and the substrate. H3 and H4 represent intramolecular helix formation within the ribozyme (intermolecular helix in the case of bimolecular ribozyme). Left panel (HP-K1) indicates 4 base-paired helix 2 and the right panel (HP-K2) indicates 6 base-paired helix 2. Arrow indicates the site of RNA cleavage. All the ribozymes discussed herein were chemically synthesized by solid phase synthesis using RNA phosphoramadite chemistry, unless otherwise indicated. Those skilled in the art will recognize that these ribozymes could also be made transcriptionally *in vitro* and *in vivo.*

Figure 68 is a graph showing RNA cleavage by hairpin ribozymes targeted to site K. A plot of fraction of the target RNA uncleaved (fraction uncleaved) as a function of time is shown. HP-K2 (6 bp helix 2) cleaves a 422 target RNA to a greater extent than the HP-K1 (4 bp helix 2).

To make internally-labeled substrate RNA for trans-ribozyme cleavage reactions, a 422 nt region (containing hairpin site A) was synthesized by PCR using primers that place the T7 RNA promoter upstream of the amplified sequence. Target RNA was transcribed in a standard transcription buffer in the presence of [$\alpha$-$^{32}$P]CTP (Chowrira & Burke, 1991 *supra*). The reaction mixture was treated with 15 units of ribonuclease-free DNaseI, extracted with phenol followed chloroform:isoamyl alcohol (25:1), precipitated with isopropanol and washed with 70% ethanol. The dried pellet was resuspended in 20 µl DEPC-treated water and stored at -20°C.

Unlabeled ribozyme (1µM) and internally labeled 422 nt substrate RNA (<10 nM) were denatured and renatured separately in a standard cleavage buffer (containing 50 mM Tris·HCl pH 7.5 and 10 mM $MgCl_2$) by heating to 90°C for 2 min. and slow cooling to 37°C for 10 min. The reaction was initiated by mixing the ribozyme and substrate mixtures and incubating at 37°C. Aliquots of 5 µl were taken at regular time intervals, quenched by adding an equal volume of 2X formamide gel loading buffer and frozen on dry ice. The samples were resolved on 5% polyacrylamide sequencing gel and results were quantitatively analyzed by radioanalytic imaging of gels with a PhosphorImager (Molecular Dynamics, Sunnyvale, CA).

Figs. 69a-b is the Site L Hairpin Ribozyme (HP-L) showing proposed secondary structure of the hairpin ribozyme·substrate complex. The ribozyme was assembled from two fragments as described above. The nomenclature is the same as above.

Figure 70 shows RNA cleavage by hairpin ribozymes targeted to site L. A. plot of fraction of the target RNA uncleaved (fraction uncleaved) as a function of time is shown. HP-L2 (6 bp helix 2) cleaves a 2 KB target RNA to a greater extent than the HP-L1 (4 bp helix 2). To make internally-labeled substrate RNA for *trans*-ribozyme cleavage reactions, a 2 kB region (containing hairpin site L) was synthesized by PCR using primers that place the T7 RNA promoter upstream of the amplified sequence. The cleavage reactions were carried out as described above.

Figs. 71a-b shows a Site M Hairpin Ribozyme (HP-M) with the proposed secondary structure of the hairpin ribozyme•substrate complex. The ribozyme was assembled from two fragments as described above.

Figure 72 is a graph showing RNA cleavage by hairpin ribozymes targeted to site M. The ribozymes were tested at both 20°C and at 26°C. To make internally-labeled substrate RNA for trans-ribozyme cleavage reactions, a 1.9 KB region (containing hairpin site M) was synthesized by PCR using primers that place the T7 RNA promoter upstream of the amplified sequence. Cleavage reactions were carried out as described above except that 20°C and at 26°C temperatures were used.

Figs. 73a-d shows various structural modifications of the present invention. A) Hairpin ribozyme lacking helix 5. Nomenclature is same as described under figure 3. B) Hairpin ribozyme lacking helix 4 and helix 5. Helix 4 is replaced by a nucleotide loop wherein q is ≥ 2 bases. Nomenclature is same as described under figure 3. C) Hairpin ribozyme lacking helix 5. Helix 4 loop is replaced by a linker 103"L", wherein L is a non-nucleotide linker molecule (Benseler *et al.,* 1993 *J. Am. Chem. Soc.* 115, 8483; Jennings *et al*., WO 94/13688). Nomenclature is same as described under figure 3. D) Hairpin ribozyme lacking helix 4 and helix 5. Helix 4 is replaced by non-nucleotide linker molecule "L" (Benseler *et al.,* 1993 *supra;* Jennings *et al., supra*). Nomenclature is same as described under figure 3.

Figs. 74a-b shows Hairpin ribozymes containing nucleotide spacer region "**s**" at the indicated location, wherein **s** is ≥ 1 base. Hairpin ribozymes containing spacer region, can be synthesized as one fragment or can be assembled from multiple fragments. Nomenclature is same as described under figure 3.

Figs. 75a-e shows the structures of the 5'-C-alkyl-modified nucleotides. $R_1$ is as defined above. R is OH, H, O-protecting group, NH, or any group described by the publications discussed above, and those described below. B

is as defined in the Figure or any other equivalent nucleotide base. CE is cyanoethyl, DMT is a standard blocking group. Other abbreviations are standard in the art.

Figure 76 is a diagrammatic representation of the synthesis of 5'-*C*-alkyl-D-**allose** nucleosides and their phosphoramidites.

Figure 77 is a diagrammatic representation of the synthesis of 5'-*C*-alkyl-L-**talose** nucleosides and their phosphoramidites.

Figure 78 is a diagrammatic representation of hammerhead ribozymes targeted to site O containing 5'-C-methyl-L-talo modifications at various positions.

Figure 79 shows RNA cleavage activity of HH-O ribozymes. Fraction of target RNA uncleaved as a function of time is shown.

Figure 80 is a diagrammatic representation of a position numbered hammerhead ribozyme (according to Hertel *et al. Nucleic Acids Res.* **1992,** 20, 3252) showing specific substitutions.

Figs. 81a-j shows the structures of various 2'-alkyl modified nucleotides which exemplify those of this invention. R groups are alkyl groups, Z is a protecting group.

Figure 82 is a diagrammatic representation of the synthesis of 2'-*C*-allyl uridine and cytidine.

Figure 83 is a diagrammatic representation of the synthesis of 2'-*C*-methylene and 2'-*C*-difluoromethylene uridine.

Figure 84 is a diagrammatic representation of the synthesis of 2'-*C*-methylene and 2'-*C*-difluoromethylene cytidine.

Figure 85 is a diagrammatic representation of the synthesis of 2'-*C*-methylene and 2'-*C*-difluoromethylene adenosine.

Figure 86 is a diagrammatic representation of the synthesis of 2'-*C*-carboxymethylidine uridine, 2'-*C*-methoxycarboxymethylidine uridine and derivatized amidites thereof. X is $CH_3$ or alkyl as discussed above, or another substituent.

Figure 87 is a diagrammatic representation of a synthesis of nucleoside 5'-deoxy-5'-difluoromethylphosphonates.

Figure 88 is a diagrammatic representation of the synthesis of nucleoside 5'-deoxy-5'-difluoromethylphosphonate 3'-phosphoramidites, dimers and solid supported dimers.

Figure 89 is a diagrammatic representation of the synthesis of nucleoside 5'-deoxy-5'-difluoromethylene triphosphates.

Figures 90 and 91 are diagrammatic representations of the synthesis of 3'-deoxy-3'-difluoromethylphosphonates and dimers.

Figure 92 is a schematic representation of synthesizing RNA phosphoramidite of a nucleotide containing a 2'-hydroxyl group modification of the present invention.

Figs. 93a-b describes a method for deprotection of oligonucleotides containing a 2'-hydroxyl group modification of the present invention.

Figure 94 is a diagrammatic representation of a hammerhead ribozyme targeted to site N. Positions of 2'-hydroxyl group substitution is indicated.

Figure 95 shows RNA cleavage activity of ribozymes containing a 2'-hydroxyl group modification of the present invention. All RNA, represents hammerhead ribozyme (HHN) with no 2'-hydroxyl group modifications. U7-ala, represents HHN ribozyme containing 2'-NH-alanine modification at the U7 position. U4/U7-ala, represents HHA containing 2'-NH-alanine modifications at U4 and U7 positions. U4 lys, represents HHA containing 2'-NH-lysine modification at U4 position. U7 lys, represents HHA containing 2'-NH-lysine modification at U7 position. U4/U7-lys, represents HHN containing 2'-NH-lysine modification at U4 and U7 positions.

Figures 96 and 97 are schematic representations of synthesizing (solid-phase synthesis) 3' ends of RNA with modification of the present invention. B, refers to either a base, modified base or an H.

Figure 98 and 99 are schematic representations of synthesizing (solid-phase synthesis) 5' ends of RNA with modification of the present invention. B, refers to either a base, modified base or an H.

Figures 100 and 101 are general schematic representations of the invention.

Fig. 102a-d is a schematic representation of a method of the invention.

Fig. 103 is a graph of the results of the experiment diagrammed in figure 104.

Figure 104 is a diagrammatic representation of a fusion mRNA used in the experiment diagrammed in Fig. 102.

Figure 105 is a diagrammatic representation of a method for selection of useful ribozymes of this invention.

Figure 106 generally shows R-loop formation, and an R-loop complex. In addition, it indicates the location at which ligands can be provided to target the R-loop complex to cells using at least three different procedures, such as ligand receptor interaction, lipid or calcium phosphate mediated delivery, or electroporation.

Figure 107 shows a method for use of self-processing ribozymes to generate therapeutic ribozymes of unit length. This method is essentially described by Draper et al., PCT WO 93/23509.

Figure 108 shows a method of linking ligands like folate, carbohydrate or peptides to R-loop forming RNA.

Ribozymes of this invention block to some extent ICAM-1, IL-5, rel A, TNF-α, $p210^{bcr\text{-}abl}$, or RSV genes expression and can be used to treat diseases or diagnose such diseases. Ribozymes will be delivered to cells in culture and to tissues in animal models. Ribozyme cleavage of ICAM-1, Il-5, rel A, TNF-α ,$p210^{bcr\text{-}abl}$, or RSV mRNA in these systems may prevent or alleviate disease symptoms or conditions.

## I. Target sites

Targets for useful ribozymes can be determined as disclosed in Draper et al PCT WO93/23509, Sullivan *et al.,* PCT WO94/02595 as well as by Draper et al., PCT/US94/13129 and hereby incorporated by reference herein in totality. Rather than repeat the guidance provided in those documents here, below are provided specific examples of such methods, not limiting to those in the art. Ribozymes to such targets are designed as described in those applications and synthesized to be tested *in vitro* and *in vivo,* as also described. Such ribozymes can also be optimized and delivered as described therein. While specific examples to animal and human RNA are provided, those in the art will recognize that the equivalent human RNA targets described can be used as described below. Thus, the same target may be used, but binding arms suitable for targeting human RNA sequences are present in the ribozyme. Such targets may also be selected as described below.

It must be established that the sites predicted by the computer-based RNA folding algorithm correspond to potential cleavage sites. Hammerhead or hairpin ribozymes are designed that could bind and are individually analyzed by computer folding (Jaeger et al., 1989 Proc. Natl. Acad. Sci., USA, 86 7706-7710) to assess whether the ribozyme sequences fold into the appropriate secondary structure. Those ribozymes with unfavorable intramolecular interactions between the binding arms and the catalytic core are eliminated from consideration. Varying binding arm lengths can be chosen to optimize activity. Generally, at least 5 bases on each arm are able to bind to, or otherwise interact with, the target RNA.

mRNA is screened for accessible cleavage sites by the method described generally in Draper et al., PCT WO93/23569 hereby incorporated by reference herein. Briefly, DNA oligonucleotides representing potential hammerhead or hairpin ribozyme cleavage sites are synthesized. A polymerase chain reaction is used to generate a substrate for T7 RNA polymerase transcription from cDNA clones. Labeled RNA transcripts are synthesized *in vitro* from DNA templates. The oligonucleotides and the labeled trascripts are annealed, RNaseH is added and the mixtures are incubated for the designated times at 37°C. Reactions are stopped and RNA separated on sequencing polyacrylamide gels. The percentage of the substrate cleaved is determined by autoradiographic quantitation using a phosphor imaging system. From these data, hammerhead or hairpin ribozynme sites are chosen as the most accessible.

Ribozymes of the hammerhead or hairpin motif are designed to anneal to various sites in the mRNA message. The binding arms are complementary to the target site sequences desribed above. The ribozymes are chemically synthesized. The method of synthesis used follows the procedure for normal RNA synthesis as described in Usman et al., 1987 *J. Am. Chem. Soc.,* 109, 7845 and in Scaringe et al., 1990 *Nucleic Acids Res.,* 18, 5433 and made use of common nucleic acid protecting and coupling groups, such as dimethoxytrityl at the 5'-end, phosphoramidites at the 3'-end. The average stepwise coupling yeilds are >98%. Inactive ribozymes are synthesized by substituting a U for $G_5$ and a U for A14 (numbering from Hertel et al., 1992 Nucleic Acids Res., 20, 3252). Hairpin ribozymes are synthesized in two parts and annealed to reconstruct the active ribozyme (Chowrira and Burke, 1992 *Nucleic Acids Res.,* 20, 2835-2840). Ribozymes are also synthesized from DNA templates using bacteriophage T7 RNA polymerase (Milligan and Uhlenbach, 1989, *Methods Enzymol,* 180, 51). All ribozymes are modified extensively to enhance stability by modification with nuclease resistant groups, for example, 2'-amino, 2'-*C*-allyl, 2'-flouro, 2'-*O*-methyl, 2'H (for a review see Usman and Cedergren, 1992 *TIBS* 17,34). Ribozymes are purified by gel electrophoresis using heneral methods or are purified by high pressure liquid chromatography and are resuspended in water.

### Example 1: ICAM-1

Ribozymes that cleave ICAM-1 mRNA represent a novel therapeutic approach to inflammatory or autoimmune disorders. ICAM-1 function can be blocked therapeutically using monoclonal antibodies. Ribozymes have the advantage of being generally immunologically inert, whereas significant neutralizing anti-IgG responses can be observed with some monoclonal antibody treatments.

The following is a brief description of the physiological role of ICAM-1. The discussion is not meant to be complete and is provided only for understanding of the invention that follows. This summary is not an admission that any of the work described below is prior art to the claimed invention.

Intercellular adhesion molecule-1 (ICAM-1) is a cell surface protein whose expression is induced by inflammatory mediators. ICAM-1 is required for adhesion of leukocytes to endothelial cells and for several immunological functions including antigen presentation, immunoglobulin production and cytotoxic cell activity. Blocking ICAM-1 function prevents immune cell recognition and activity during transplant rejection and in animal models of rheumatoid arthritis, asthma and reperfusion injury.

Cell-cell adhesion plays a pivotal role in inflammatory and immune responses (Springer et al., 1987 *Ann. Rev. Immunol*. 5, 223-252). Cell adhesion is required for leukocytes to bind to and migrate through vascular endothelial cells. In addition, cell-cell adhesion is required for antigen presentation to T cells, for B cell induction by T cells, as well as for the cytotoxicity activity of T cells, NK cells, monocytes or granulocytes. Intercellular adhesion molecule-1 (ICAM-1) is a 110 kilodalton member of the immunoglobulin superfamily that is involved in all of these cell-cell interactions (Simmons et al., 1988 *Nature (London)* 331, 624-627).

ICAM-1 is expressed on only a limited number of cells and at low levels in the absence of stimulation (Dustin et al., 1986 *J. Immunol.* 137, 245-254). Upon treatment with a number of inflammatory mediators (lipopolysaccharide, γ-interferon, tumor necrosis factor-α, or interleukin-1), a variety of cell types (endothelial, epithelial, fibroblastic and hematopoietic cells) in a variety of tissues express high levels of ICAM-1 on their surface (Sringer *et. al. supra;* Dustin *et al., supra;* and Rothlein et al., 1988 *J. Immunol.* 141, 1665-1669). Induction occurs via increased transcription of ICAM-1 mRNA (Simmons *et al., supra*). Elevated expression is detectable after 4 hours and peaks after 16 - 24 hours of induction.

ICAM-1 induction is critical for a number of inflammatory and immune responses. *In vitro,* antibodies to ICAM-1 block adhesion of leukocytes to cytokine-activated endothelial cells (Boyd, 1988 *Proc. Natl. Acad. Sci. USA* 85, 3095-3099; Dustin and Springer, 1988 J. *Cell Biol.* 107, 321-331). Thus, ICAM-1 expression may be required for the extravasation of immune cells to sites of inflammation. Antibodies to ICAM-1 also block T cell killing, mixed lymphocyte reactions, and T cell-mediated B cell differentiation, suggesting that ICAM-1 is required for these cognate cell interactions (Boyd *et al*., *supra).* The importance of ICAM-1 in antigen presentation is underscored by the inability of ICAM-1 defective murine B cell mutants to stimulate antigen-dependent T cell proliferation (Dang et al., 1990 *J. Immunol*. 144, 4082-4091). Conversely, murine L cells require transfection with human ICAM-1 in addition to HLA-DR in order to present antigen to human T cells (Altmann et al., 1989 *Nature (London)* 338, 512-514), In summary, evidence *in vitro* indicates that ICAM-1 is required for cell-cell interactions critical to inflammatory responses, cellular immune responses, and humoral antibody responses.

By engineering ribozyme motifs we have designed several ribozymes directed against ICAM-1 mRNA sequences. These have been synthesized with modifications that improve their nuclease resistance. These ribozymes cleave ICAM-1 target sequences *in vitro*.

The sequence of human, rat and mouse ICAM-1 mRNA can be screened for accessible sites using a compter folding algorithm. Regions of the mRNA that did not form secondary folding structures and that contain potential hammerhead or hairpin ribozyme cleavage sites can be identified. These sites are shown in Tables 2, 3, and 6-9. (All sequences are 5' to 3' in the tables) While rat, mouse and human sequences can be screened and ribozymes thereafter designed, the human targeted sequences are of most utility.

The sequences of the chemically synthesized ribozymes useful in this study are shown in Tables 4 - 8 and 10. Those in the art will recognize that these sequences are representative only of many more such sequences where the enzymatic portion of the ribozyme (all but the binding arms) is altered to affect activity and may be formed of ribonucleotides or other nucleotides or non-nucleotides. Such ribozymes are equivalent to the ribozymes described specifically in the Tables.

The ribozymes will be tested for function *in vivo* by exogenous delivery to human umbilical vein endothelial cells (HUVEC). Ribozymes will be delivered by incorporation into liposomes, by complexing with cationic lipids, by microinjection, or by expression from DNA or RNA vectors described above. Cytokine-induced ICAM-1 expression will be monitored by ELISA, by indirect immunofluoresence, and/or by FACS analysis. ICAM-1 mRNA levels will be assessed by Northern, by RNAse protection, by primer extension or by quantitative RT-PCR analysis. Ribozymes that block the induction of ICAM-1 protein and mRNA by more than 90% will be identified.

As disclosed by Sullivan et al., PCT WO94/02595, incorporated by reference herein, ribozymes and/or genes encoding them will be locally delivered to transplant tissue *ex vivo* in animal models. Expression of the ribozyme will be monitored by its ability to block *ex vivo* induction of ICAM-1 mRNA and protein. The effect of the anti-ICAM-1 ribozymes on graft rejection will then be assessed. Similarly, ribozymes will be introduced into joints of mice with collagen-induced arthritis or rabbits with *Streptococcal* cell wall-induced arthritis. Liposome delivery, cationic lipid delivery, or adeno-associated virus vector delivery can be used. One dose (or a few infrequent doses) of a stable anti-ICAM-1 ribozyme or a gene construct that constitutively expresses the ribozyme may abrogate inflammatory and immune responses in these diseases.

Uses

ICAM-1 plays a central role in immune cell recognition and function. Ribozyme inhibition of ICAM-1 expression can reduce transplant rejection and alleviate symptoms in patients with rheumatoid arthritis, asthma or other acute and

chronic inflammatory disorders. We have engineered several ribozymes that cleave ICAM-1 mRNA. Ribozymes that efficiently inhibit ICAM-1 expression in cells can be readily found and their activity measured with regard to their ability to block transplant rejection and arthritis symptoms in animal models. These anti-ICAM-1 ribozymes represent a novel therapeutic for the treatment of immunological or inflammatory disorders.

The therapeutic utility of reduction of activity of ICAM-1 function is evident in the following disease targets. The noted references indicate the role of ICAM-1 and the therapeutic potential of ribozymes described herein. Thus, these targets can be therapeutically treated with agents that reduce ICAM-1 expression or function. These diseases and the studies that support a critical role for ICAM-1 in their pathology are listed below. This list is not meant to be complete and those in the art will recognize further conditions and diseases that can be effectively treated using ribozymes of the present invention.

- Transplant rejection

  ICAM-1 is expressed on venules and capillaries of human cardiac biopsies with histological evidence of graft rejection (Briscoe et al., 1991 *Transplantation* 51, 537-539).

  Antibody to ICAM-1 blocks renal (Cosimi et al., 1990J. *Immunol.* 144, 4604-4612) and cardiac (Flavin et al., 1991 *Transplant. Proc*. 23, 533-534) graft rejection in primates.

  A Phase I clinical trial of a monoclonal anti-ICAM-1 antibody showed significant reduction in rejection and a significant increase in graft function in human kidney transplant patients (Haug, et al., 1993 *Transplantation* 55, 766-72).

- Rheumatoid arthritis

  (CAM-1 overexpression is seen on synovial fibroblasts, endothelial cells, macrophages, and some lymphocytes (Chin et al., 1990 *Arthritis Rheum* 33, 1776-86; Koch et al., 1991 *Lab Invest* 64, 313-20).

  Soluble ICAM-1 levels correlate with disease severity (Mason et al., 1993 *Arthritis Rheum* 36, 519-27).

  Anti-ICAM antibody inhibits collagen-induced arthritis in mice (Kakimoto et al., 1992 *Cell Immunol* 142, 326-37).

  Anti-ICAM antibody inhibits adjuvant-induced arthritis in rats (Iigo et al., 1991 *J Immunol* 147, 4167-71).

- Myocardial ischemia, stroke, and reperfusion injury

  Anti-ICAM-1 antibody blocks adherence of neutrophils to anoxic endothelial cells (Yoshida et al., 1992 *Am J Physiol* 262, H1891-8).

  Anti-ICAM-1 antibody reduces neurological damage in a rabbit model of cerebral stroke (Bowes et al., 1993 *Exp Neurol* 119, 215-9).

  Anti-ICAM-1 antibody protects against reperfusion injury in a cat model of myocardial ischemia (Ma et al., 1992*Circulation* 86, 937-46).

- Asthma

  Antibody to ICAM-1 partially blocks eosinophil adhesion to endothelial cells and is overexpressed on inflamed airway endothelium and epithelium *in vivo* (Wegner et al., 1990 *Science* 247, 456-9).

  In a primate model of asthma, anti-ICAM-1 antibody blocks airway eosinophilia (Wegneret al., *supra*) and prevents the resurgence of airway inflammation and hyper-responsiveness after dexamethosone treatment (Gundel et al., 1992 *Clin Exp Allergy* 22, 569-75).

- Psoriasis

  Surface ICAM-1 and a clipped, soluble version of ICAM-1 is expressed in psoriatic lesions and expression correlates with inflammation (Kellner et al., 1991 *Br J Dermatol* 125, 211-6; Griffiths 1989 J *Am Acad Dermatol* 20, 617-29; Schopf et al., 1993 *Br J Dermatol* 128, 34-7). Anti-ICAM antibody blocks keratinocyte antigen presentation to T cells (Nickoloff et al., 1993 *J Immunol* 150, 2148-59).

- Kawasaki disease

  Surface ICAM-1 expression correlates with the disease and is reduced by effective immunoglobulin treatment (Leung, et al., 1989 *Lancet* 2, 1298-302).

  Soluble ICAM levels are elevated in Kawasaki disease patients; particularly high levels are observed in patients with coronary artery lesions (Furukawa et al., 1992*Arthritis Rheum* 35, 672-7; Tsuji, 1992 *Arerugi* 41, 1507-14).

  Circulating LFA-$1^+$ T cells are depleted (presumably due to ICAM-1 mediated extravasation) in Kawasaki disease patients (Furukawa et al., 1993*Scand J Immunol* **37**, 377-80).

Example 2: IL-5

Ribozymes that cleave IL-5 mRNA represent a novel therapeutic approach to inflammatory disorders like asthma. The invention features use of ribozymes to treat chronic asthma, e.g., by inhibiting the synthesis of IL-5 in lymphocytes and preventing the recruitment and activation of eosinophils.

A number of cytokines besides IL-5 may also be involved in the activation of inflammation in asthmatic patients, including platelet activating factor, IL-1, IL-3, IL-4, GM-CSF, TNF-$\alpha$, gamma interferon, VCAM, ILAM-1, ELAM-1 and NF-$\kappa$B. In addition to these molecules, it is appreciated that any cellular receptors which mediate the activities of the cytokines are also good targets for intervention in inflammatory diseases. These targets include, but are not limited to, the IL-1R and TNF-$\alpha$R on keratinocytes, epithelial and endothelial cells in airways. Recent data suggest that certain neuropeptides may play a role in asthmatic symptoms. These peptides include substance P, neurokinin A and calcitonin-gene-related peptides. These target genes may have more general roles in inflammatory diseases, but are currently assumed to have a role only in asthma.

Ribozymes of this invention block to some extent IL-5 expression and can be used to treat disease or diagnose such disease. Ribozymes will be delivered to cells in culture and to cells or tissues in animal models of asthma (Clutterbuck et al., 1989 supra; Garssen et al., 1991 Am. Rev. Respir. Dis. 144, 931-938; Larsen et al., 1992 J. Clin. Invest. 89, 747-752; Mauser et al., 1993 supra). Ribozyme cleavage of IL-5 mRNA in these systems may prevent inflammatory cell function and alleviate disease symptoms.

The sequence of human and mouse IL-5 mRNA were screened for accessible sites using a computer folding algorithm. Potential hammerhead or hairpin ribozyme cleavage sites were identified. These sites are shown in Tables 11, 13, and 14, 15. (All sequences are 5' to 3' in the tables.) While mouse and human sequences can be screened and ribozymes thereafter designed, the human targeted sequences are of most utility. However, mouse targeted ribozymes are useful to test efficacy of action of the ribozyme prior to testing in humans. The nucleotide base position is noted in the Tables as that site to be cleaved by the designated type of ribozyme. (In Table 12, lower case letters indicate positions that are not conserved between the Human and the Mouse IL-5 sequences.)

The sequences of the chemically synthesized ribozymes useful in this study are shown in Tables 12, 14 - 16. Those in the art will recognize that these sequences are representative only of many more such sequences where the enzymatic portion of the ribozyme (all but the binding arms) is altered to affect activity. For example, stem loop II sequence of hammerhead ribozymes listed in Tables 12 and 14 (5'-GGCCGAAAGGCC-3') can be altered (substitution, deletion and/or insertion) to contain any sequence provided, a minimum of two base-paired stem structure can form. Similarly, stem-loop IV sequence of hairpin ribozymes listed in Tables 15 and 16 (5'-CACGUUGUG-3') can be altered (substitution, deletion and/or insertion) to contain any sequence provided, a minimum of two base-paired stem structure can form. The sequences listed in Tables 12, 14 - 16 may be formed of ribonucleotides or other nucleotides or non-nucleotides. Such ribozymes are equivalent to the ribozymes described specifically in the Tables.

By engineering ribozyme motifs we have designed several ribozymes directed against IL-5 mRNA sequences. These ribozymes are synthesized with modifications that improve their nuclease resistance. The ability of ribozymes to cleave IL-5 target sequences *in vitro* is evaluated.

The ribozymes will be tested for function *in vivo* by analyzing IL-5 expression levels. Ribozymes will be delivered to cells by incorporation into liposomes, by complexing with cationic lipids, by microinjection, or by expression from DNA or RNA vectors. IL-5 expression will be monitored by biological assays, ELISA, by indirect immunofluorescence, and/or by FACS analysis. IL-5 mRNA levels will be assessed by Northern analysis, RNAse protection or primer extension analysis or quantitative RT-PCR. Ribozymes that block the induction of IL-5 activity and/or IL-5 mRNA by more than 90% will be identified.

Uses

Interleukin 5 (IL-5), a cytokine produced by CD4+ T helper cells and mast cells, was originally termed B cell growth factor II (reviewed by Takatsu et al., 1988 Immunol. Rev. 102, 107). It stimulates proliferation of activated B cells and induces production of IgM and IgA. IL-5 plays a major role in eosinophil function by promoting differentiation (Clutterbuck et al., 1989 Blood 73, 1504-12), vascular adhesion (Walsh et al., 1990 Immunology 71, 258-65) and *in vitro* survival of eosinophils (Lopez et al., 1988 J. Exp. Med. 167, 219-24). This cytokine also enhances histamine release from basophils (Hirai et al., 1990 J. Exp. Med. 172, 1525-8). The following summaries of clinical results support the selection of IL-5 as a primary target for the treatment of asthma:

Several studies have shown a direct correlation between the number of activated T cells and the number of eosinophils from asthmatic patients vs. normal patients (Oehling et al., 1992 J. Investig. Allergol. Clin. Immunol. 2, 295-9). Patients with either allergic asthma or intrinsic asthma were treated with corticosteroids. The bronchoalveolar lavage was monitored for eosinophils, activated T helper cells and recovery of pulmonary function over a 28 to 30 day period. The number of eosinophils and activated T helper cells decreased progressively with subsequent improvement

in pulmonary function compared to intrinsic asthma patients with no corticosteroid treatment.

Bronchoalveolar lavage cells were screened for production of cytokines using *in situ* hybridization for mRNA. *In situ* hybridization signals were detected for IL-2, IL-3, IL-4, IL-5 and GM-CSF. Upregulation of mRNA was observed for IL-4, IL-5 and GM-CSF (Robinson et al., 1993 J. Allergy Clin. Immunol. 92, 313-24). Another study showed that upregulation of IL-5 transcripts from allergen challenged vs. saline challenged asthmatic patients (Krishnaswamy et al., 1993 Am. J. Respir. Cell. Mol. Biol. 9, 279-86).

An 18 patient study was performed to determine a mechanism of action for corticosteroid improvement of asthma symptoms. Improvement was monitored by methacholine responsiveness. A correlation was observed between the methacholine responsiveness, a reduction in the number of eosinophils, a reduction in the number of cells expressing IL-4 and IL-5 mRNA and an increase in number of cells expressing interferon-gamma.

Bronchial biopsies from 15 patients were analyzed 24 hours after allergen challenge (Bentley et al., 1993 Am. J. Respir. Cell. Mol. Biol. 8, 35-42). Increased numbers of eosinophils and IL-2 receptor positive cells were found in the biopsies. No differences in the numbers of total leukocytes, T lymphocytes, elastase-positive neutrophils, macrophages or mast cell subtypes were observed. The number of cells expressing IL-5 and GM-CSF mRNA significantly increased.

In another patient study, the eosinophil phenotype was the same for asthmatic patients and normal individuals. However, eosinophils from asthmatic patients had greater leukotriene C4 producing capacity and migration capacity. There were elevated levels of IL-3, IL-5 and GM-CSF in the circulation of asthmatics but not in normal individuals (Bruijnzeel et al., 1992 Schweiz. Med. Wochenschr. 122, 298-301).

Efficacy of antibody to IL-5 was assessed in a guinea pig asthma model. The animals were challenged with ovalbumin and assayed for eosinophilia and the responsiveness to the bronchioconstriction substance P. A 30 mg/kg dose of antibody administered i.p. blocked ovalbumin-induced increased sensitivity to substance P and blocked increases in bronchoalveolar and lung tissue accumulation of eosinophils (Mauser et al., 1993 Am. Rev. Respir. Dis. 148, 1623-7). In a separate study guinea pigs challenged for eight days with ovalbumin were treated with monoclonal antibody to IL-5. Treatment produced a reduction in the number of eosinophils in bronchoalveolar lavage. No reduction was observed for unchallenged guinea pigs and guinea pigs treated with a control antibody. Antibody treatment completely inhibited the development of hyperreactivity to histamine and arecoline after ovalbumin challenge (van Oosterhout et al., 1993 Am. Rev. Respir. Dis. 147, 548-52)

Results obtained from human clinical analysis and animal studies indicate the role of activated T helper cells, cytokines and eosinophils in asthma. The role of IL-5 in eosinophil development and function makes IL-5 a good candidate for target selection. The antibody studies neutralized IL-5 in the circulation thus preventing eosinophilia. Inhibition of the production of IL-5 will achieve the same goal.

**Asthma** - a prominent feature of asthma is the infiltration of eosinophils and deposition of toxic eosinophil proteins (e.g. major basic protein, eosinophil-derived neurotoxin) in the lung. A number of T-cell-derived factors like IL-5 are responsible for the activation and maintainance of eosinophils (Kay, 1991 J. Allergy Clin. Immun. 87, 893). Inhibition of IL-5 expression in the lungs can decrease the activation of eosinophils and will help alleviate the symptoms of asthma.

**Atopy -** is characterized by the developement of type I hypersensitive reactions associated with exposure to certain environmental antigens. One of the common clinical manifestations of atopy is eosinophilia (accumulation of abnormally high levels of eosinophils in the blood). Antibodies against IL-5 have been shown to lower the levels of eosinophils in mice (Cook et al., 1993 in Immunopharmacol. Eosinophils ed. Smith and Cook, pp. 193-216, Academic, London, UK)

**Parasitic infection**-related eosinophilia- infections with parasites like helminths, can lead to severe eosinophilia (Cook et al., 1993 supra). Animal models for eosinophilia suggest that infection of mice, for example, can lead to blood, peritoneal and/or tissue eosinophilia, all of which seem to be lowered to varying degrees by antibodies directed against IL-5.

**Pulmonary infiltration eosinophilia**- is characterised by accumulation of high levels of eosinophils in pulmonary parenchyma (Gleich, 1990 J. Allergy Clin. Immunol. 85, 422).

**L-Tryptophan-associated eosinophilia-myalgia syndrome (EMS)-** The EMS disease is closely linked to the consumption of L-tryptophan, an essential aminoacid used to treat conditions like insomnia (for review see Varga et al., 1993 J Invest. Dermatol. 100, 97s). Pathologic and histologic studies have demonstrated high levels of eosinophils and mononuclear inflammatory cells in patients with EMS. It appears that IL-5 and transforming growth factor play a significant role in the development of EMS (Varga et al., 1993 supra) by activating eosinophils and other inflammatory cells.

Thus, ribozymes of the present invention that cleave IL-5 mRNA and thereby IL-5 activity have many potential therapeutic uses, and there are reasonable modes of delivering the ribozymes in a number of the possible indications. Development of an effective ribozyme that inhibits IL-5 function is described above; available cellular and activity assays are numerous, reproducible, and accurate. Animal models for IL-5 function and for each of the suggested disease

targets exist (Cook et al., 1993 supra) and can be used to optimize activity.

Example 3: NF-κB

Ribozymes that cleave *rel A* mRNA represent a novel therapeutic approach to inflammatory or autoimmune disorders. Inflammatory mediators such as lipopolysaccharide (LPS), interleukin-1 (IL-1) or tumor necrosis factor-a (TNF-α) act on cells by inducing transcription of a number of secondary mediators, including other cytokines and adhesion molecules. In many cases, this gene activation is known to be mediated by the transcriptional regulator, NF-κB. One subunit of NF-κB, the *rel*A gene product (termed RelA or p65) is implicated specifically in the induction of inflammatory responses. Ribozyme therapy, due to its exquisite specificity, is particularly well-suited to target intracellular factors that contribute to disease pathology. Thus, ribozymes that cleave mRNA encoded by rel A or TNF-α may represent novel therapeutics for the treatment of inflammatory and autoimmune disorders.

The nuclear DNA-binding activity, NF-κB, was first identified as a factor that binds and activates the immunoglobulin κ light chain enhancer in B cells. NF-κB now is known to activate transcription of a variety of other cellular genes (*e.g.*, cytokines, adhesion proteins, oncogenes and viral proteins) in response to a variety of stimuli (*e.g.*, phorbol esters, mitogens, cytokines and oxidative stress). In addition, molecular and biochemical characterization of NF-κB has shown that the activity is due to a homodimer or heterodimer of a family of DNA binding subunits. Each subunit bears a stretch of 300 amino acids that is homologous to the oncogene, v-*rel*. The activity first described as NF-κB is a heterodimer of p49 or p50 with p65. The p49 and p50 subunits of NF-κB (encoded by the nf-κB2 or nf-κB1 genes, respectively) are generated from the precursors NF-κB1 (p105) or NF-κB2 (p100). The p65 subunit of NF-κB (now termed Rel A) is encoded by the *rel* A locus.

The roles of each specific transcription-activating complex now are being elucidated in cells (N.D. Perkins, et al., 1992 Proc. Natl Acad. Sci USA 89, 1529-1533). For instance, the heterodimer of NF-κB1 and Rel A (p50/p65) activates transcription of the promoter for the adhesion molecule, VCAM-1, while NF-κB2/RelA heterodimers (p49/p65) actually inhibit transcription (H.B. Shu, et al., Mol. Cell. Biol. 13, 6283-6289 (1993)). Conversely, heterodimers of NF-κB2/RelA (p49/p65) act with Tat-I to activate transcription of the HIV genome, while NP-κB1/RelA (p50/p65) heterodimers have little effect (J. Liu, N.D. Perkins, R.M. Schmid, G.J. Nabel, J. Virol. 1992 66, 3883-3887). Similarly, blocking *rel* A gene expression with antisense oligonucleotides specifically blocks embryonic stem cell adhesion; blocking NF-κB1 gene expression with antisense oligonucleotides had no effect on cellular adhesion (Narayanan et al., 1993 Mol. Cell. Biol. 13, 3802-3810). Thus, the promiscuous role initially assigned to NF-κB in transcriptional activation (M.J. Lenardo, D. Baltimore, 1989 Cell 58, 227-229) represents the sum of the activities of the rel family of DNA-binding proteins. This conclusion is supported by recent transgenic "knock-out" mice of individual members of the *rel* family. Such "knockouts" show few developmental defects, suggesting that essential transcriptional activation functions can be performed by more than one member of the rel family.

A number of specific inhibitors of NF-κB function in cells exist, including treatment with phosphorothioate antisense oliogonucleotide, treatment with double-stranded NF-κB binding sites, and over expression of the natural inhibitor MAD-3 (an IκB family member). These agents have been used to show that NF-κB is required for induction of a number of molecules involved in inflammation, as described below.

- NF-κB is required for phorbol ester-mediated induction of IL-6 (I. Kitajima, et al., Science 258, 1792-5 (1992)) and IL-8 (Kunsch and Rosen, 1993 Mol. Cell. Biol. 13, 6137-46).

- NF-κB is required for induction of the adhesion molecules ICAM-1 (Eck, et al., 1993 Mol. Cell. Biol. 13, 6530-6536), VCAM-1 (Shu et al., *supra),* and E-selectin (Read, et al., 1994 J. Exp. Med. 179, 503-512) on endothelial cells.

- NF-κB is involved in the induction of the integrin subunit, CD18, and other adhesive properties of leukocytes (Eck et al., 1993 *supra).*

  The above studies suggest that NF-κB is integrally involved in the induction of cytokines and adhesion molecules by inflammatory mediators. Two recent papers point to another connection between NF-κB and inflammation: glucocorticoids may exert their anti-inflammatory effects by inhibiting NF-κB. The glucocorticoid receptor and p65 both act at NF-κB binding sites in the ICAM-1 promoter (van de Stolpe, et al., 1994 J. Biol. Chem. 269, 6185-6192). Glucocorticoid receptor inhibits NF-κB-mediated induction of IL-6 (Ray and Prefontaine, 1994 Proc. Natl Acad. Sci USA 91, 752-756). Conversely, overexpression of p65 inhibits glucocorticoid induction of the mouse mammary tumor virus promoter. Finally, protein cross-linking and co-immunoprecipitation experiments demonstrated direct physical interaction between p65 and the glucocorticoid receptor *(Id.).*

  Ribozymes of this invention block to some extent NF-κB expression and can be used to treat disease or diagnose such disease. Ribozymes will be delivered to cells in culture and to cells or tissues in animal models of restenosis, transplant rejection and rheumatoid arthritis. Ribozyme cleavage of *relA* mRNA in these systems may

prevent inflammatory cell function and alleviate disease symptoms.

The sequence of human and mouse *relA* mRNA can be screened for accessible sites using a computer folding algorithm. Potential hammerhead or hairpin ribozyme cleavage sites were identified. These sites are shown in Tables 17, 18 and 21-22. (All sequences are 5' to 3' in the tables.) While mouse and human sequences can be screened and ribozymes thereafter designed, the human targetted sequences are of most utility.

The sequences of the chemically synthesized ribozymes useful in this study are shown in Tables 19 - 22. Those in the art will recognize that these sequences are representative only of many more such sequences

where the enzymatic portion of the ribozyme (all but the binding arms) is altered to affect activity and may be formed of ribonucleotides or other nucleotides or non-nucleotides. Such ribozymes are equivalent to the ribozymes described specifically in the Tables.

By engineering ribozyme motifs we have designed several ribozymes directed against *rel* A mRNA sequences. These ribozymes are synthesized with modifications that improve their nuclease resistance. The ability of ribozymes to cleave *rel*A target sequences *in vitro* is evaluated.

The ribozymes will be tested for function *in vivo* by analyzing cytokine-induced VCAM-1, ICAM-1, IL-6 and IL-8 expression levels. Ribozymes will be delivered to cells by incorporation into liposomes, by complexing with cationic lipids, by microinjection, or by expression from DNA and RNA vectors. Cytokine-induced VCAM-1, ICAM-1, IL-6 and IL-8 expression will be monitored by ELISA, by indirect immunofluoresence, and/or by FACS analysis. *Rel* A mRNA levels will be assessed by Northern analysis, RNAse protection or primer extension analysis or quantitative RT-PCR. Activity of NF-κB will be monitored by gel-retardation assays. Ribozymes that block the induction of NF-κB activity and/or *rel A* mRNA by more than 50% will be identified.

RNA ribozymes and/or genes encoding them will be locally delivered to transplant tissue *ex vivo* in animal models. Expression of the ribozyme will be monitored by its ability to block *ex vivo* induction of VCAM-1, ICAM-1, IL-6 and IL-8 mRNA and protein. The effect of the anti-*rel A* ribozymes on graft rejection will then be assessed. Similarly, ribozymes will be introduced into joints of mice with collagen-induced arthritis or rabbits with *Streptococcal* cell wall-induced arthritis. Liposome delivery, cationic lipid delivery, or adeno-associated virus vector delivery can be used. One dose (or a few infrequent doses) of a stable anti-*relA* ribozyme or a gene construct that constitutively expresses the ribozyme may abrogate inflammatory and immune responses in these diseases.

## Uses

A therapeutic agent that inhibits cytokine gene expression, inhibits adhesion molecule expression, and mimics the anti-inflammatory effects of glucocorticoids (without inducing steroid-responsive genes) is ideal for the treatment of inflammatory and autoimmune disorders. Disease targets for such a drug are numerous. Target indications and the delivery options each entails are summarized below. In all cases, because of the potential immunosuppressive properties of a ribozyme that cleaves *rel A* mRNA, uses are limited to local delivery, acute indications, or *ex vivo* treatment.

- Rheumatoid arthritis (RA).

  Due to the chronic nature of RA, a gene therapy approach is logical. Delivery of a ribozyme to inflamed joints is mediated by adenovirus, retrovirus, or adeno-associated virus vectors. For instance, the appropriate adenovirus vector can be administered by direct injection into the synovium: high efficiency of gene transfer and expression for several months would be expected (B.J. Roessler, E.D. Allen, J.M. Wilson, J.W. Hartman, B. L. Davidson, J. Clin. Invest. 92, 1085-1092 (1993)). It is unlikely that the course of the disease could be reversed by the transient, local administration of an anti-inflammatory agent. Multiple administrations may be necessary. Retrovirus and adeno-associated virus vectors would lead to permanent gene transfer and expression in the joint. However, permanent expression of a potent anti-inflammatory agent may lead to local immune deficiency.

- Restenosis.

  Expression of NF-κB in the vessel wall of pigs causes a narrowing of the luminal space due to excessive deposition of extracellular matrix components. This phenotype is similar to matrix deposition that occurs subsequent to coronary angioplasty. In addition, NF-κB is required for the expression of the oncogene c-myb (F.A. La Rosa, J.W. Pierce, G.E. Soneneshein, Mol. Cell. Biol. 14, 1039-44 (1994)). Thus NF-κB induces smooth muscle proliferation and the expression of excess matrix components: both processes are thought to contribute to reocclusion of vessels after coronary angioplasty.

- Transplantation.

  NF-κB is required for the induction of adhesion molecules (Eck et al., *supra,* K. O'Brien, et al., J. Clin. Invest. 92, 945-951 (1993)) that function in immune recognition and inflammatory responses. At least two potential modes

of treatment are possible. In the first, transplanted organs are treated *ex vivo* with ribozymes or ribozyme expression vectors. Transient inhibition of NF-κB in the transplanted endothelium may be sufficient to prevent transplant-associated vasculitis and may significantly modulate graft rejection. In the second, donor B cells are treated *ex vivo* with ribozymes or ribozyme expression vectors. Recipients would receive the treatment prior to transplant. Treatment of a recipient with B cells that do not express T cell co-stimulatory molecules (such as ICAM-1, VCAM-1, and/or B7 an B7-2) can induce antigen-specific anergy. Tolerance to the donor's histocompatibility antigens could result; potentially, any donor could be used for any transplantation procedure.

- Asthma.
  Granulocyte macrophage colony stimulating factor (GM-CSF) is thought to play a major role in recruitment of eosinophils and other inflammatory cells during the late phase reaction to asthmatic trauma. Again, blocking the local induction of GM-CSF and other inflammatory mediators is likely to reduce the persistent inflammation observed in chronic asthmatics. Aerosol delivery of ribozymes or adenovirus ribozyme expression vectors is a feasible treatment.

- Gene Therapy.
  Immune responses limit the efficacy of many gene transfer techniques. Cells transfected with retrovirus vectors have short lifetimes in immune competent individuals. The length of expression of adenovirus vectors in terminally differentiated cells is longer in neonatal or immune-compromised animals. Insertion of a small ribozyme expression cassette that modulates inflammatory and immune responses into existing adenovirus or retrovirus constructs will greatly enhance their potential.
  Thus, ribozymes of the present invention that cleave *rel A* mRNA and thereby NF-κB activity have many potential therapeutic uses, and there are reasonable modes of delivering the ribozymes in a number of the possible indications. Development of an effective ribozyme that inhibits NF-κB function is described above; available cellular and activity assays are number, reproducible, and accurate. Animal models for NF-κB function (Kitajima, et al., *supra*) and for each of the suggested disease targets exist and can be used to optimize activity.

Example 4: TNF-α

Ribozymes that cleave the specific cites in TNF-α mRNA represent a novel therapeutic approach to inflammatory or autoimmune disorders.

Tumor necrosis factor-α (TNF-α) is a protein, secreted by activated leukocytes, that is a potent mediator of inflammatory reactions. Injection of TNF-α into experimental animals can simulate the symptoms of systemic and local inflammatory diseases such as septic shock or rheumatoid arthritis.

TNF-α was initially described as a factor secreted by activated macrophages which mediates the destruction of solid tumors in mice (Old, 1985 Science 230, 4225-4231). TNP-α subsequently was found to be identical to cachectin, an agent responsible for the weight loss and wasting syndrome associated with tumors and chronic infections (Beutler, et al., 1985 Nature 316, 552-554). The cDNA and the genomic locus for TNF-α have been cloned and found to be related to TNF-β (Shakhov et al., 1990 J. Exp. Med. 171, 35-47). Both TNF-α and TNF-β bind to the same receptors and have nearly identical biological activities. The two TNF receptors have been found on most cell types examined (Smith, et al., 1990 Science 248, 1019-1023). TNF-α secretion has been detected from monocytes/macrophages, CD4+ and CD8+ T-cells, B-cells, lymphokine activated killer cells, neutrophils, astrocytes, endothelial cells, smooth muscle cells, as well as various non-hematopoietic tumor cell lines ( for a review see Turestskaya et al., 1991 in Tumor Necrosis Factor: Structure, Function, and Mechanism of Action B. B. Aggarwal, J. Vilcek, Eds. Marcel Dekker, Inc., pp. 35-60). TNF-α is regulated transcriptionally and translationally, and requires proteolytic processing at the plasma membrane in order to be secreted (Kriegler et al., 1988 Cell 53, 45-53). Once secreted, the serum half life of TNF-α is approximately 30 minutes. The tight regulation of TNF-α is important due to the extreme toxicity of this cytokine. Increasing evidence indicates that overproduction of TNF-α during infections can lead to severe systemic toxicity and death (Tracey & Cerami, 1992 Am. J. Trop. Med. Hyg. 47, 2-7).

Antisense RNA and Hammerhead ribozymes have been used in an attempt to lower the expression level of TNF-α by targeting specified cleavage sites [Sioud et al., 1992 J. Mol. Biol. 223; 831; Sioud WO 94/10301; Kisich and co-workers, 1990 abstract (FASEB J. 4, A1860; 1991 slide presentation (J. Leukocyte Biol. sup. 2, 70); December, 1992 poster presentation at Anti-HIV Therapeutics Conference in SanDiego, CA; and "Development of anti-TNF-α ribozymes for the control of TNF-α gene expression"- Kisich, Doctoral Dissertation, 1993 University of California, Davis] listing various TNFα targeted ribozymes.

Ribozymes of this invention block to some extent TNF-α expression and can be used to treat disease or diagnose such disease. Ribozymes will be delivered to cells in culture and to cells or tissues in animal models of septic shock and rheumatoid arthritis. Ribozyme cleavage of TNF-α mRNA in these systems may prevent inflammatory cell

function and alleviate disease symptoms.

The sequence of human and mouse TNF-α mRNA can be screened for accessible sites using a computer folding algorithm. Hammerhead or hairpin ribozyme cleavage sites were identified. These sites are shown in Tables 23, 25, and 27 - 28. (All sequences are 5' to 3' in the tables.) While mouse and human sequences can be screened and ribozymes thereafter designed, the human targeted sequences are of most utility. However, mouse targeted ribozymes are useful to test efficacy of action of the ribozyme prior to testing in humans. The nucleotide base position is noted in the Tables as that site to be cleaved by the designated type of ribozyme. (In Table 24, lower case letters indicate positions that are not conserved between the human and the mouse TNF-α sequences.)

The sequences of the chemically synthesized ribozymes useful in this study are shown in Tables 24, 26 - 28. Those in the art will recognize that these sequences are representative only of many more such sequences where the enzymatic portion of the ribozyme (all but the binding arms) is altered to affect activity. For example, stem-loop II sequence of hammerhead ribozymes listed in Tables 24 and 26 (5'-GGCCGAAAGGCC3') can be altered (substitution, deletion, and/or insertion) to contain any sequences provided a minimum of two base-paired stem structure can form. Similarly, stem-loop IV sequence of hairpin ribozymes listed in Tables 27 and 28 (5'-CACGUUQUG-3') can be altered (substitution, deletion, and/or insertion) to contain any sequence, provided a minimum of two base-paired stem structure can form. The sequences listed in Tables 24, 26 - 28 may be formed of ribonucleotides or other nucleotides or non-nucleotides. Such ribozymes are equivalent to the ribozymes described specifically in the Tables or AAV.

In a preferred embodiment of the invention, a transcription unit expressing a ribozyme that cleaves TNF-α RNA is inserted into a plasmid DNA vector or an adenovirus DNA viral vector or AAV or alpha virus or retroviris vectors. Viral vectors have been used to transfer genes to the intact vasculature or to joints of live animals (Willard et al., 1992 <u>Circulation,</u> 86, I-473.; Nabel et al., 1990 <u>Science</u>, 249, 1285-1288) and both vectors lead to transient gene expression. The adenovirus vector is delivered as recombinant adenoviral particles. DNA may be delivered alone or complexed with vehicles (as described for RNA above). The DNA, DNA/vehicle complexes, or the recombinant adenovirus particles are locally administered to the site of treatment, e.g., through the use of an injection catheter, stent or infusion pump or are directly added to cells or tissues *ex vivo*.

In another preferred embodiment of the invention, a transcription unit expressing a ribozyme that cleaves TNF-∝ RNA is inserted into a retrovirus vector for sustained expression of ribozyme(s).

By engineering ribozyme motifs we have designed several ribozymes directed against TNF-α mRNA sequences. These ribozymes are synthesized with modifications that improve their nuclease resistance. The ability of ribozymes to cleave TNF-α target sequences *in vitro* is evaluated.

The ribozymes will be tested for function in cells by analyzing bacterial lipopolysaccharide (LPS)-induced TNF-α expression levels. Ribozymes will be delivered to cells by incorporation into liposomes, by complexing with cationic lipids, by microinjection, or by expression from DNA vectors. TNF-α expression will be monitored by ELISA, by indirect immunofluoresence, and/or by FACS analysis. TNF-α mRNA levels will be assessed by Northern analysis, RNAse protection, primer extension analysis or quantitative RT-PCR. Ribozymes that block the induction of TNF-α activity and/or TNF-α mRNA by more than 90% will be identified.

RNA ribozymes and/or genes encoding them will be locally delivered to macrophages by intraperitoneal injection. After a period of ribozyme uptake, the peritoneal macrophages are harvested and induced *ex vivo* with LPS. The ribozymes that significantly reduce TNP-α secretion are selected. The TNF-α can also be induced after ribozyme treatment with fixed *Streptococcus* in the peritoneal cavity instead of *ex vivo*. In this fashion the ability of TNF-α ribozymes to block TNF-α secretion in a localized inflammatory response are evaluated. In addition, we will determine if the ribozymes can block an ongoing inflammatory response by delivering the TNF-α ribozymes after induction by the injection of fixed *Streptococcus.*

To examine the effect of anti-TNF-α ribozymes on systemic inflammation, the ribozymes are delivered by intravenous injection. The ability of the ribozymes to inhibit TNF-α secretion and lethal shock caused by systemic LPS administration are assessed. Similarly, TNF-α ribozymes can be introduced into the joints of mice with collagen-induced arthritis. Either free delivery, liposome delivery, cationic lipid delivery, adeno-associated virus vector delivery, adenovirus vector delivery, retrovirus vector delivery or plasmid vector delivery in these animal model experiments can be used to supply ribozymes. One dose (or a few infrequent doses) of a stable anti-TNF-α ribozyme or a gene construct that constitutively expresses the ribozyme may abrogate tissue damage in these inflammatory diseases.

*Macrophage isolation.*

To produce responsive macrophages 1 ml of sterile fluid thioglycollate broth (Difco, Detroit, MI.) was injected i.p. into 6 week old female C57bl/6NCR mice 3 days before peritoneal lavage. Mice were maintained as specific pathogen free in autoclaved cages in a laminar flow hood and given sterilized water to minimize "spontaneous" activation of macrophages. The resulting peritoneal exudate cells (PEC) were obtained by lavage using Hanks balanced salt solution (HBSS) and were plated at $2.5X10^5$/well in 96 well plates (Costar, Cambridge, MA.) with Eagles minimal

essential medium (EMEM) containing 10% heat inactivated fetal bovine serum. After adhering for 2 hours the wells were washed to remove non-adherent cells. The resulting cultures were 97% macrophages as determined by morphology and staining for non-specific esterase.

*Transfection of ribozymes into macrophages:*

The ribozymes were diluted to 2X final concentration, mixed with an equal volume of 11nM lipofectamine (Life Technologies, Gaithersburg, MD.), and vortexed. 100 ml of lipid:ribozyme complex was then added directly to the cells, followed immediately by 10 ml fetal bovine serum. Three hours after ribozyme addition 100 ml of 1 mg/ml bacterial lipopolysaccaride (LPS) was added to each well to stimulate TNF production.

*Quantitation of TNF-α in mouse macrophages*:

Supernatants were sampled at 0, 2, 4, 8, and 24 hours post LPS stimulation and stored at -70°C. Quantitation of TNF-α was done by a specific ELISA. ELISA plates were coated with rabbit anti-mouse TNF-α serum at 1:1000 dilution (Genzyme) followed by blocking with milk proteins and incubation with TNF-α containing supernatants. TNF-α was then detected using a murine TNF-α specific hamster monoclonal antibody (Genzyme). The ELISA was developed with goat anti-hamster IgG coupled to alkaline phosphatase.

*Assessment of reagent toxicity:*

Following ribozyme/lipid treatment of macrophages and harvesting of supernatants viability of the cells was assessed by incubation of the cells with 5 mg/ml of 3-(4,5-dimethylthiazol-2-yl)-2,5-diphenyl tetrazolium bromide (MTT). This compound is reduced by the mitochondrial dihydrogenases, the activity of which correlates well with cell viability. After 12 hours the absorbance of reduced MTT is measured at 585 nm.

Uses

The association between TNF-α and bacterial sepsis, rheumatoid arthritis, and autoimmune disease make TNF-α an attractive target for therapeutic intervention [Tracy & Cerami 1992 supra; Williams et al., 1992 Proc. Natl. Acad. Sci. USA 89, 9784-9788; Jacob, 1992 J. Autoimmun. 5 (Supp. A), 133-143].

Septic Shock

Septic shock is a complication of major surgery, bacterial infection, and polytrauma characterized by high fever, increased cardiac output, reduced blood pressure and a neutrophilic infiltrate into the lungs and other major organs. Current treatment options are limited to antibiotics to reduce the bacterial load and non-steroidal anti-inflammatories to reduce fever. Despite these treatments in the best intensive care settings, mortality from septic shock averages 50%, due primarily to multiple organ failure and disseminated vascular coagulation. Septic shock, with an incidence of 200,000 cases per year in the United States, is the major cause of death in intensive care units. In septic shock syndrome, tissue injury or bacterial products initiate massive immune activation, resulting in the secretion of pro-inflammatory cytokines which are not normally detected in the serum, such as TNF-α, interleukin-1β (IL-1β), γ-interferon (IFN-γ), interleukin-6 (IL-6), and interleukin-8 (IL-8). Other non-cytokine mediators such as leukotriene b4, prostaglandin E2, C3a and C3d also reach high levels (de Boer et al., 1992 Immunopharmacology 24, 135-148).

TNF-α is detected early in the course of septic shock in a large fraction of patients (de Boer et al., 1992 supra). In animal models, injection of TNF-α has been shown to induce shock-like symptoms similar to those induced by LPS injection (Beutler et al., 1985 Science 229, 869-871); in contrast, injection of IL-1β, IL-6, or IL-8 does not induce shock. Injection of TNF-α also causes an elevation of IL-1β, IL-6, IL-8, $PgE_2$, acute phase proteins, and $TxA_2$ in the serum of experimental animals (de Boer et al., 1992 supra). In animal models the lethal effects of LPS can be blocked by pre-administration of anti-TNF-α antibodies. The cumulative evidence indicates that TNF-α is a key player in the pathogenesis of septic shock, and therefore a good candidate for therapeutic intervention.

Rheumatoid Arthritis

Rheumatoid arthritis (RA) is an autoimmune disease characterized by chronic inflammation of the joints leading to bone destruction and loss of joint function. At the cellular level, autoreactive T- lymphocytes and monocytes are typically present, and the synoviocytes often have altered morphology and immunostaining patterns. RA joints have been shown to contain elevated levels of TNF-α, IL-1α and IL-1β, IL-6, GM-CSF, and TGF-β (Abney et al., 1991 Imm.

Rev. 119, 105-123), some or all of which may contribute to the pathological course of the disease.

Cells cultured from RA joints spontaneously secrete all of the pro-inflammatory cytokines detected *in vivo*. Addition of antisera against TNF-α to these cultures has been shown to reduce IL-1α/β production by these cells to undetectable levels (Abney et al., 1991 Supra). Thus, TNF-α may directly induce the production of other cytokines in the RA joint. Addition of the anti-inflammatory cytokine, TGF-β, has no effect on cytokine secretion by RA cultures. Immunocytochemical studies of human RA surgical specimens clearly demonstrate the production of TNF-α, IL-1α/β, and IL-6 from macrophages near the cartilage/pannus junction when the pannus in invading and overgrowing the cartilage (Chu et al., 1992 Br. J. Rheumatology 31, 653-661). GM-CSF was shown to be produced mainly by vascular endothelium in these samples. Both TNF-α and TGF-β have been shown to be fibroblast growth factors, and may contribute to the accumulation of scar tissue in the RA joint. TNF-α has also been shown to increase osteoclast activity and bone resorbtion, and may have a role in the bone erosion commonly found in the RA joint (Cooper et al., 1992 Clin. Exp. Immunol. 89, 244-250).

Elimination of TNF-α from the rheumatic joint would be predicted to reduce overall inflammation by reducing induction of MHC class II, IL-1α/β, Il-6, and GM-CSF, and reducing T-cell activation. Osteoclast activity might also fall, reducing the rate of bone erosion at the joint. Finally, elimination of TNF-α would be expected to reduce accumulation of scar tissue within the joint by removal of a fibroblast growth factor.

Treatment with an anti-TNF-α antibody reduces joint swelling and the histological severity of collagen-induced arthritis in mice (Williams et al., 1992 Proc. Natl. Acad. Sci. USA 89, 9784-9788). In addition, a study of RA patients who have received i.v. infusions of anti-TNF-α monoclonal antibody reports a reduction in the number and severity of inflamed joints after treatment. The benefit of monoclonal antibody treatment in the long term may be limited by the expense and immunogenicity of the antibody.

Psoriasis

Psoriasis is an inflammatory disorder of the skin characterized by keratinocyte hyperproliferation and immune cell infiltrate (Kupper, 1990 J. Clin. Invest. 86, 1783-1789). It is a fairly common condition, affecting 1.5-2.0% of the population. The disorder ranges in severity from mild, with small flaky patches of skin, to severe, involving inflammation of the entire epidermis. The cellular infiltrate of psoriasis includes T-lymphocytes, neutrophils, macrophages, and dermal dendrocytes. The majority of T-lymphocytes are activated $CD4^+$ cells of the $T_H$-1 phenotype, although some $CD8^+$ and $CD4^-/CD8^-$ are also present. B lymphocytes are typically not found in abundance in psoriatic plaques.

Numerous hypotheses have been offered as to the proximal cause of psoriasis including auto-antibodies and auto-reactive T-cells, overproduction of growth factors, and genetic predisposition. Although there is evidence to support the involvement of each of these factors in psoriasis, they are neither mutually exclusive nor are any of them necessary and sufficient for the pathogenesis of psoriasis (Reeves, 1991 Semin. Dermatol. 10, 217).

The role of cytokines in the pathogenesis of psoriasis has been investigated. Among those cytokines found to be abnormally expressed were TGF-α, IL-1α, IL-1β, IL-ira, IL-6, IL-8, IFN-γ, and TNF-α. In addition to abnormal cytokine production, elevated expression of ICAM-1, ELAM-1, and VCAM has been observed (Reeves, 1991 supra). This cytokine profile is similar to that of normal wound healing, with the notable exception that cytokine levels subside upon healing. Keratinocytes themselves have recently been shown to be capable of secreting EGF, TGF-α, IL-6, and TNF-α, which could increase proliferation in an autocrine fashion (Oxholm et al., 1991 APMIS 99, 58-64).

Nickoloff et al., 1993 (J Dermatol Sci. 6, 127-33) have proposed the following model for the initiation and maintenance of the psoriatic plaque:

Tissue damage induces the wound healing response in the skin. Keratinocytes secrete IL-1α, IL-1β, IL-6, IL-8, TNF-α. These factors activate the endothelium of dermal capillaries, recruiting PMNs, macrophages, and T-cells into the wound site.

Dermal dendrocytes near the dermal/epidermal junction remain activated when they should return to a quiescent state, and subsequently secrete cytokines including TNF-α, IL-6, and IL-8. Cytokine expression, in turn, maintains the activated state of the endothelium, allowing extravasation of additional immunocytes, and the activated state of the keratinocytes which secrete TGF-α and IL-8. Keratinocyte IL-8 recruits immunocytes from the dermis into the epidermis. During passage through the dermis, T-cells encounter the activated dermal dendrocytes which efficiently activate the $T_H$-1 phenotype. The activated T-cells continue to migrate into the epidermis, where they are stimulated by keratinocyte-expressed ICAM-1 and MHC class II. IFN-γ secreted by the T-cells synergizes with the TNF-α from dermal dendrocytes to increase keratinocyte proliferation and the levels of TGF-α, IL-8, and IL-6 production. IFN-γ also feeds back to the dermal dendrocyte, maintaining the activated phenotype and the inflammatory cycle.

Elevated serum titres of IL-6 increases synthesis of acute phase proteins including complement factors by the liver, and antibody production by plasma cells. Increased complement and antibody levels increases the probability of autoimmune reactions.

Maintenance of the psoriatic plaque requires continued expression of all of these processes, but attractive

points of therapeutic intervention are TNF-α expression by the dermal dendrocyte to maintain activated endothelium and keratinocytes, and IFN-γ expression by T-cells to maintain activated dermal dendrocytes.

There are 3 million patients in the United States afflicted with psoriasis. The available treatments for psoriasis are corticosteroids. The most widely prescribed are TEMOVATE (clobetasol propionate), LIDEX (fluocinonide), DIPROLENE (betamethasone propionate), PSORCON (diflorasone diacetate) and TRIAMCINOLONE formulated for topical application. The mechanism of action of corticosteroids is multifactorial. This is a palliative therapy because the underlying cause of the disease remains, and upon discontinuation of the treatment the disease returns. Discontinuation of treatment is often prompted by the appearance of adverse effects such as atrophy, telangiectasias and purpura. Corticosteroids are not recommended for prolonged treatments or when treatment of large and/or inflamed areas is required. Alternative treatments include retinoids, such as etretinate, which has been approved for treatment of severe, refractory psoriasis. Alternative retinoid-based treatments are in advanced clinical trials. Retinoids act by converting keratinocytes to a differentiated state and restoration of normal skin development. Immunosuppressive drugs such as cyclosporine are also in the advanced stages of clinical trials. Due to the nonspecific mechanism of action of corticosteroids, retinoids and immunosuppressives, these treatments exhibit severe side effects and should not be used for extended periods of time unless the condition is life-threatening or disabling. There is a need for a less toxic, effective therapeutic agent in psoriatic patients.

HIV and AIDS

The human immunodeficiency virus (HIV) causes several fundamental changes in the human immune system from the time of infection until the development of full-blown acquired immunodeficiency syndrome (AIDS). These changes include a shift in the ratio of CD4+ to CD8+ T-cells, sustained elevation of IL-4 levels, episodic elevation of TNF-α and TNF-β levels, hypergammaglobulinemia, and lymphoma/leukemia (Rosenberg & Fauci, 1990 Immun. Today 11, 176; Weiss 1993 Science 260, 1273). Many patients experience a unique tumor, Kaposi's sarcoma and/or unusual opportunistic infections (e.g. *Pneumocystis carinii*, cytomegalovirus, herpesviruses, hepatitis viruses, papilloma viruses, and tuberculosis). The immunological dysfunction of individuals with AIDS suggests that some of the pathology may be due to cytokine dysregulation.

Levels of serum TNF-α and IL-6 are often found to be elevated in AIDS patients (Weiss, 1993 supra). In tissue culture, HIV infection of monocytes isolated from healthy individuals stimulates secretion of both TNF-α and IL-6. This response has been reproduced using purified gp120, the viral coat protein responsible for binding to CD-4 (Buonaguro et al., 1992 J. Virol. 66, 7159). It has also been demonstrated that the viral gene regulator, Tat, can directly induce TNF transcription. The ability of HIV to directly stimulate secretion of TNF-α and IL-6 may be an adaptive mechanism of the virus. TNF-α has been shown to upregulate transcription of the LTR of HIV, increasing the number of HIV-specific transcripts in infected cells. IL-6 enhances HIV production, but at a post-transcriptional level, apparently increasing the efficiency with which HIV transcripts are translated into protein. Thus, stimulation of TNF-α secretion by the HIV virus may promote infection of neighboring CD4+ cells both by enhancing virus production from latently infected cells and by driving replication of the virus in newly infected cells.

The role of TNF-α in HIV replication has been well established in tissue culture models of infection (Sher et al., 1992 Immun. Rev. 127, 183), suggesting that the mutual induction of HIV replication and TNF-α replication may create positive feedback *in vivo*. However, evidence for the presence of such positive feedback in infected patients is not abundant. TNF-α levels are found to be elevated in some, but not all patients tested. Children with AIDS who were given zidovudine had reduced levels of TNF-α compared to those not given zidovudine (Cremoni et al., 1993 AIDS 7, 128). This correlation lends support to the hypothesis that reduced viral replication is physiologically linked to TNF-α levels. Furthermore, recently it has been shown that the polyclonal B cell activation associated with HIV infection is due to membrane-bound TNF-α. Thus, levels of secreted TNF-α may not accurately reflect the contribution of this cytokine to AIDS pathogenesis.

Chronic elevation of TNF-α has been shown to shown to result in cachexia (Tracey et al., 1992 Am. J. Trop. Med. Hyg. 47, 2-7), increased autoimmune disease (Jacob, 1992 supra), lethargy, and immune suppression in animal models (Aderka et al., 1992 Isr. J. Med. Sci. 28, 126-130). The cachexia associated with AIDS may be associated with chronically elevated TNF-α frequently observed in AIDS patients. Similarly, TNF-α can stimulate the proliferation of spindle cells isolated from Kaposi's sarcoma lesions of AIDS patients (Barillari et al., 1992 J Immunol 149, 3727).

A therapeutic agent that inhibits cytokine gene expression, inhibits adhesion molecule expression, and mimics the anti-inflammatory effects of glucocorticoids (without inducing steroid-responsive genes) is ideal for the treatment of inflammatory and autoimmune disorders. Disease targets for such a drug are numerous. Target indications and the delivery options each entails are summarized below. In all cases, because of the potential immunosuppressive properties of a ribozyme that cleaves the specified sites in TNF-α mRNA, uses are limited to local delivery, acute indications, or *ex vivo* treatment.

- Septic shock.

  Exogenous delivery of ribozymes to macrophages can be achieved by intraperitoneal or intravenous injections. Ribozymes will be delivered by incorporation into liposomes or by complexing with cationic lipids.

- Rheumatoid arthritis (RA).

  Due to the chronic nature of RA, a gene therapy approach is logical. Delivery of a ribozyme to inflamed joints is mediated by adenovirus, retrovirus, or adeno-associated virus vectors. For instance, the appropriate adenovirus vector can be administered by direct injection into the synovium: high efficiency of gene transfer and expression for several months would be expected (B.J. Roessler, E.D. Allen, J.M. Wilson, J.W. Hartman, B. L. Davidson, J. Clin. Invest. 92, 1085-1092 (1993)). It is unlikely that the course of the disease could be reversed by the transient, local administration of an anti-inflammatory agent. Multiple administrations may be necessary. Retrovirus and adeno-associated virus vectors would lead to permanent gene transfer and expression in the joint. However, permanent expression of a potent anti-inflammatory agent may lead to local immune deficiency.

- Psoriasis

  The psoriatic plaque is a particularly good candidate for ribozyme or vector delivery. The stratum corneum of the plaque is thinned, providing access to the proliferating keratinocytes. T-cells and dermal dendrocytes can be efficiently targeted by trans-epidermal diffusion .

  Organ culture systems for biopsy specimens of psoriatic and normal skin are described in current literature (Nickoloff et al., 1993 Supra). Primary human keratinocytes are easily obtained and will be grown into epidermal sheets in tissue culture. In addition to these tissue culture models, the flaky skin mouse develops psoriatic skin in response to UV light. This model would allow demonstration of animal efficacy for ribozyme treatments of psoriasis.

- Gene Therapy.

Immune responses limit the efficacy of many gene transfer techniques. Cells transfected with retrovirus vectors have short lifetimes in immune competent individuals. The length of expression of adenovirus vectors in terminally differentiated cells is longer in neonatal or immune-compromised animals. Insertion of a small ribozyme expression cassette that modulates inflammatory and immune responses into existing adenovirus or retrovirus constructs will greatly enhance their potential.

Thus, ribozymes of the present invention that cleave TNF-α mRNA and thereby TNF-α activity have many potential therapeutic uses, and there are reasonable modes of delivering the ribozymes in a number of the possible indications. Development of an effective ribozyme that inhibits TNF-α function is described above; available cellular and activity assays are number, reproducible, and accurate. Animal models for TNF-α function and for each of the suggested disease targets exist and can be used to optimize activity.

Example 5: $p210^{bcr\text{-}abl}$

Chronic myelogenous leukemia exhibits a characteristic disease course, presenting initially as a chronic granulocytic hyperplasia, and invariably evolving into an acute leukemia which is caused by the clonal expansion of a cell with a less differentiated phenotype (i.e., the blast crisis stage of the disease). CML is an unstable disease which ultimately progresses to a terminal stage which resembles acute leukemia. This lethal disease affects approximately 16,000 patients a year. Chemotherapeutic agents such as hydroxyurea or busulfan can reduce the leukemic burden but do not impact the life expectancy of the patient (e.g. approximately 4 years). Consequently, CML patients are candidates for bone marrow transplantation (BMT) therapy. However, for those patients which survive BMT, disease recurrence remains a major obstacle (Apperley et al., 1988 Br. J. Haematol. 69, 239).

The Philadelphia (Ph) chromosome which results from the translocation of the *abl* oncogene from chromosome 9 to the *bcr* gene on chromosome 22 is found in greater than 95% of CML patients and in 10-25% of all cases of acute lymphoblastic leukemia [(ALL); Fourth International Workshop on Chromosomes in Leukemia 1982, Cancer Genet. Cytogenet. 11, 316]. In virtually all Ph-positive CMLs and approximately 50% of the Ph-positive ALLs, the leukemic cells express *bcr-abl* fusion mRNAs in which exon 2 (b2-a2 junction) or exon 3 (b3-a2 junction) from the major breakpoint cluster region of the *bcr* gene is spliced to exon 2 of the *abl* gene. Heisterkamp et al., 1985 Nature 315, 758; Shtivelman et al., 1987, Blood 69, 971). In the remaining cases of Ph-positive ALL, the first exon of the *bcr* gene is spliced to exon 2 of the *abl* gene (Hooberman et al., 1989 Proc. Nat. Acad. Sci. USA 86, 4259; Heisterkamp et al., 1988 Nucleic Acids Res. 16, 10069).

The b3-a2 and b2-a2 fusion mRNAs encode 210 kd bcr-abl fusion proteins which exhibit oncogenic activity (Daley et al., 1990 Science 247, 824; Heisterkamp et al., 1990 Nature 344, 251). The importance of the bcr-abl fusion protein ($p210^{bcr\text{-}abl}$) in the evolution and maintenance of the leukemic phenotype in human disease has been demonstrated using antisense oligonucleotide inhibition of p210$^{bcr-abl}$ expression. These inhibitory molecules have been shown to inhibit the in vitro proliferation of leukemic cells in bone marrow from CML patients. Szczylik et al., 1991 Science 253, 562).

Reddy, U.S. Patent 5,246,921 (hereby incorporated by reference herein) describes use of ribozymes as therapeutic agents for leukemias, such as chronic myelogenous leukemia (CML) by targeting the specific junction region of *bcr-abl* fusion transcripts. It indicates causing cleavage by a ribozyme at or near the breakpoint of such a hybrid chromosome, specifically it includes cleavage at the sequence GUX, where X is A, U or G. The one example presented is to cleave the sequence 5' AGC AG AGUU (cleavage site) CAA AAGCCCU-3'.

Scanlon WO 91/18625, WO 91/18624, and WO 91/18913 and Snyder et al., WO93/03141 and WO94/13793 describe a ribozyme effective to cleave oncogenic variants of *H-ras* RNA. This ribozyme is said to inhibit H-*ras* expression in response to external stimuli.

The invention features use of ribozymes to inhibit the development or expression of a transformed phenotype in man and other animals by modulating expression of a gene that contributes to the expression of CML. Cleavage of targeted mRNAs expressed in pre-neoplastic and transformed cells elicits inhibition of the transformed state.

The invention can be used to treat cancer or pre-neoplastic conditions. Two preferred administration protocols can be used, either in vivo administration to reduce the tumor burden, or ex vivo treatment to eradicate transformed cells from tissues such as bone marrow prior to reimplantation.

This invention features an enzymatic RNA molecule (or ribozyme) which cleaves mRNA associated with development or maintenance of CML. The mRNA targets are present in the 425 nucleotides surrounding the fusion sites of the *bcr* and *abl* sequences in the b2-a2 and b3-a2 recombinant mRNAs. Other sequences in the 5' portion of the *bcr* mRNA or the 3' portion of the *abl* mRNA may also be targeted for ribozyme cleavage. Cleavage at any of these sites in the fusion mRNA molecules will result in inhibition of translation of the fusion protein in treated cells.

The invention provides a class of chemical cleaving agents which exhibit a high degree of specificity for the mRNA causative of CML. Such enzymatic RNA molecules can be delivered exogenously or endogenously to afflicted cells. In the preferred hammerhead motif the small size (less than 40 nucleotides, preferably between 32 and 36 nucleotides in length) of the molecule allows the cost of treatment to be reduced.

The smallest ribozyme delivered for any type of treatment reported to date (by Rossi et al., 1992 supra) is an in vitro transcript having a length of 142 nucleotides. Synthesis of ribozymes greater than 100 nucleotides in length is very difficult using automated methods, and the therapeutic cost of such molecules is prohibitive. Delivery of ribozymes by expression vectors is primarily feasible using only ex vivo treatments. This limits the utility of this approach. In this invention, an alternative approach uses smaller ribozyme motifs and exogenous delivery. The simple structure of these molecules also increases the ability of the ribozyme to invade targeted regions of the mRNA structure. Thus, unlike the situation when the hammerhead structure is included within longer transcripts, there are no non-ribozyme flanking sequences to interfere with correct folding of the ribozyme structure, as well as complementary binding of the ribozyme to the mRNA target.

The enzymatic RNA molecules of this invention can be used to treat human CML or precancerous conditions. Affected animals can be treated at the time of cancer detection or in a prophylactic manner. This timing of treatment will reduce the number of affected cells and disable cellular replication. This is possible because the ribozymes are designed to disable those structures required for successful cellular proliferation.

Ribozymes of this invention block to some extent p210$^{bcr-abl}$ expression and can be used to treat disease or diagnose such disease. Ribozymes will be delivered to cells in culture and to tissues in animal models of CML. Ribozyme cleavage of *bcr*/*abl* mRNA in these systems may prevent or alleviate disease symptoms or conditions.

The sequence of human *bcr*/*abl* mRNA can be screened for accessible sites using a computer folding algorithm. Regions of the mRNA that did not form secondary folding structures and that contain potential hammerhead or hairpin ribozyme cleavage sites can be identified. These sites are shown in Table 29 (All sequences are 5' to 3' in the tables). The nucleotide base position is noted in the Tables as that site to be cleaved by the designated type of ribozyme.

The sequences of the chemically synthesized ribozymes most useful in this study are shown in Table 30. Those in the art will recognize that these sequences are representative only of many more such sequences where the enzymatic portion of the ribozyme (all but the binding arms) is altered to affect activity. For example, stem-loop II sequence of hammerhead ribozymes listed in Table 30 (5'-GGCCGAAAGGCC-3') can be altered (substitution, deletion, and/or insertion) to contain any sequence provided, a minimum of two base-paired stem structure can form. The sequences listed in Tables 30 may be formed of ribonucleotides or other nucleotides or non-nucleotides. Such ribozymes are equivalent to the ribozymes described specifically in the Tables.

By engineering ribozyme motifs we have designed several ribozymes directed against *bcr-abl* mRNA sequences. These have been synthesized with modifications that improve their nuclease resistance as described above. These ribozymes cleave *bcr-abl* target sequences *in vitro.*

The ribozymes are tested for function *in vivo* by exogenous delivery to cells expressing *bcr-abl*. Ribozymes are delivered by incorporation into liposomes, by complexing with cationic lipids, by microinjection, or by expression

from DNA vectors. Expression of *bcr-abl* is monitored by ELISA, by indirect immunofluoresence, and/or by FACS analysis. Levels of *bcr-abl* mRNA are assessed by Northern analysis, RNase protection, by primer extension analysis or by quantitative RT-PCR techniques. Ribozymes that block the induction of p210$^{bcr\text{-}abl}$) protein and mRNA by more than 20% are identified.

Example 6: RSV

This invention relates to the use of ribozymes as inhibitors of respiratory syncytial virus (RSV) production, and in particular, the inhibition of RSV replication.

RSV is a member of the virus family paramyxoviridae and is classified under the genus *Pneumovirus* (for a review see Mclntosh and Chanock, 1990 in Virology ed. B.N. Fields, pp. 1045, Raven Press Ltd. NY). The infectious virus particle is composed of a nucleocapsid enclosed within an envelope. The nucleocapsid is composed of a linear negative single-stranded non-segmented RNA associated with repeating subunits of capsid proteins to form a compact structure and thereby protect the RNA from nuclease degradation. The entire nucleocapsid is enclosed by the envelope. The size of the virus particle ranges from 150- 300 nm in diameter. The complete life cycle of RSV takes place in the cytoplasm of infected cells and the nucleocapsid never reaches the nuclear compartment (Hall, 1990 in Principles and Practice of Infectious Diseases ed. Mandell et al., Churchill Livingstone, NY).

The RSV genome encodes ten viral proteins essential for viral production. RSV protein products include two structural glycoproteins (G and F) found in the envelope spikes, two matrix proteins [M and M2 (22K)] found in the inner membrane, three proteins localized in the nucleocapsid (N, P and L), one protein that is present on the surface of the infected cell (SH), and two nonstructural proteins [NS1 (1C) and NS2 (1B)] found only in the infected cell. The mRNAs for the 10 RSV proteins have similar 5' and 3' ends. UV-inactivation studies suggest that a single promoter is used with multiple transcription initiation sites (Barik *et al*., 1992 J. Virol. 66, 6813). The order of transcription corresponding to the protein assignment on the genomic RNA is 1C, 1B, N, P, M, SH, G, F, 22K and L genes (Huang *et al.,* 1985 Virus Res. 2, 157) and transcript abundance corresponds to the order of gene assignment (for example the 1C and 1B mRNAs are much more abundant than the L mRNA. Synthesis of viral message begins immediately after RSV infection of cells and reaches a maximum at 14 hours post-infection (Mclntosh and Chanock, *supra).*

There are two antigenic subgroups of RSV, A and B, which can circulate simultaneously in the community in varying proportions in different years (Mclntosh and Chanock, *supra).* Subgroup A usually predominates. Within the two subgroups there are numerous strains. By the limited sequence analysis available it seems that homology at the nucleotide level is more complete within than between subgroups, although sequence divergence has been noted within subgroups as well. Antigenic determinates result primarily from both surface glycoproteins, F and G. For F, at least half of the neutralization epitopes have been stably maintained over a period of 30 years. For G however, A and B subgroups may be related antigenically by as little as a few percent. On the nucleotide level, however, the majority of the divergence in the coding region of G is found in the sequence for the extracellular domain (Johnson et al., 1987, *Proc. Natl. Acad. Sci.* USA 84, 5625).

Respiratory Syncytial Virus (RSV) is the major cause of lower respiratory tract illness during infancy and childhood (Hall, *supra)* and as such is associated with an estimated 90,000 hospitalizations and 4500 deaths in the United States alone (Update: respiratory syncytial virus activity - United States, 1993, Mmwr Morb Mortal Wkly Rep, 42, 971). Infection with RSV generally outranks all other microbial agents leading to both pneumonia and bronchitis. While primarily affecting children under two years of age, immunity is not complete and reinfection of older children and adults, especially hospital care givers (McIntosh and Chanock, *supra),* is not uncommon. Immunocompromised patients are severely affected and RSV infection is a major complication for patients undergoing bone marrow transplantation .

Uneventful RSV respiratory disease resembles a common cold and recovery is in 7 to 12 days. Initial symptoms (rhinorrhea, nasal congestion, slight fever, etc.) are followed in 1 to 3 days by lower respiratory tract signs of infection that include a cough and wheezing. In severe cases, these mild symptoms quickly progress to tachypnea, cyanosis, and listlessness and hospitalization is required. In infants with underlying cardiac or respiratory disease, the progression of symptoms is especially rapid and can lead to respiratory failure by the second or third day of illness. With modem intensive care however, overall mortality is usually less than 5% of hospitalized patients (McIntosh and Chanock, *supra*).

At present, neither an efficient vaccine nor a specific antiviral agent is available. An immune response to the viral surface glycoproteins can provide resistance to RSV in a number of experimental animals, and a subunit vaccine has been shown to be effective for up to 6 months in children previously hospitalized with an RSV infection (Tristam *et al*., 1993, J. Infect. Dis. 167, 191). An attenuated bovine RSV vaccine has also been shown to be effective in calves for a similar length of time (Kubota *et al*., 1992 J. Vet. Med. Sci. 54, 957). Previously however, a formalin-inactivated RSV vaccine was implicated in greater frequency of severe disease in subsequent natural infections with RSV (Connors *et al*., 1992 J. Virol. 66, 7444).

The current treatment for RSV infection requiring hospitalization is the use of aerosolized ribavirin, a guanosine analog [Antiviral Agents and Viral Diseases of Man, 3rd edition. 1990, (eds. G.J. Galasso, R.J. Whitley, and T.C. Merigan) Raven Press Ltd., NY.]. Ribavirin therapy is associated with a decrease in the severity of the symptoms, improved arterial oxygen and a decrease in the amount of viral shedding at the end of the treatment period. It is not certain, however, whether ribavirin therapy actually shortens the patients' hospital stay or diminishes the need for supportive therapies (McIntosh and Chanock, *supra).* The benefits of ribavirin therapy are especially clear for high risk infants, those with the most serious symptoms or for patients with underlying bronchopulmonary or cardiac disease. Inhibition of the viral polymerase complex is supported as the main mechanism for inhibition of RSV by ribavirin, since viral but not cellular polypeptide synthesis is inhibited by ribavirin in RSV-infected cells (Antiviral Agents and Viral Diseases of Man, 3rd edition. 1990. (eds. G.J. Galasso, R.J. Whitley, and T.C. Merigan) Raven Press Ltd., NY]. Since ribavirin is at least partially effective against RSV infection when delivered by aerosolization, it can be assumed that the target cells are at or near the epithelial surface. In this regard, RSV antigen had not spread any deeper than the superficial layers of the respiratory epithelium in autopsy studies of fatal pneumonia (McIntosh and Chanock, *supra).*

Jennings *et al*., WO 94/13688 indicates that targets for specific types of ribozymes include respiratory syncytical virus.

The invention features novel enzymatic RNA molecules, or ribozymes, and methods for their use for inhibiting production of respiratory syncytial virus (RSV). Such ribozymes can be used in a method for treatment of diseases caused by these related viruses in man and other animals. The invention also features cleavage of the genomic RNA and mRNA of these viruses by use of ribozymes. In particular, the ribozyme molecules described are targeted to the *NS1 (1C), NS2 (1B)* and *N* viral genes. These genes are known in the art (for a review see McIntosh and Chanock, 1990 *supra ).*

Ribozymes that cleave the specified sites in RSV mRNAs represent a novel therapeutic approach to respiratory disorders. Applicant indicates that ribozymes are able to inhibit the activity of RSV and that the catalytic activity of the ribozymes is required for their inhibitory effect. Those of ordinary skill in the art, will find that it is clear from the examples described that other ribozymes that cleave these sites in RSV mRNAs encoding 1C, 1B and N proteins may be readily designed and are within the invention. Also, those of ordinary skill in the art, will find that it is clear from the examples described that ribozymes cleaving other mRNAs encoded by RSV (*P, M, SH, G, F,* 22*K* and *L*) and the genomic RNA may be readily designed and are within the invention.

In preferred embodiments, the ribozymes have binding arms which are complementary to the sequences in Tables 31, 33, 35, 37 and 38. Examples of such ribozymes are shown in Tables 32, 34, 36-38. Examples of such ribozymes consist essentially of sequences defined in these Tables. By "consists essentially of" is meant that the active ribozyme contains an enzymatic center equivalent to those in the examples, and binding arms able to bind mRNA such that cleavage at the target site occurs. Other sequences may be present which do not interfere with such cleavage.

Ribozymes of this invention block to some extent RSV production and can be used to treat disease or diagnose such disease. Ribozymes will be delivered to cells in culture and to cells or tissues in animal models of respiratory disorders. Ribozyme cleavage of RSV encoded mRNAs or the genomic RNA in these systems may alleviate disease symptoms.

While all ten RSV encoded proteins (1C, 1B, N, P, M, SH, 22K, F, G, and L) are essential for viral life cycle and are all potential targets for ribozyme cleavage, certain proteins (mRNAs) are more favorable for ribozyme targeting than the others. For example RSV encoded proteins 1C, 1B, SH and 22K are not found in other members of the family paramyxoviridae and appear to be unique to RSV. In contrast the ectodomain of the G protein and the signal sequence of the F protein show significant sequence divergence at the nucleotide level among various RSV sub-groups (Johnson *et al.,* 1987 *supra).,* RSV proteins 1C, 1B and N are highly conserved among various subtypes at both the nucleotide and amino acid levels. Also, 1C, 1B and N are the most abundant of all RSV proteins.

The sequence of human RSV mRNAs encoding 1C, 1B and N proteins are screened for accessible sites using a computer folding algorithm. Hammerhead or hairpin ribozyme cleavage sites were identified. These sites are shown in Tables 31, 33, 34, 37 and 38 (All sequences are 5' to 3' in the tables.) The nucleotide base position is noted in the Tables as that site to be cleaved by the designated type of ribozyme.

Ribozymes of the hammerhead or hairpin motif are designed to anneal to various sites in the mRNA message. The binding arms are complementary to the target site sequences described above. The ribozymes are chemically synthesized. The method of synthesis used follows the procedure for normal RNA synthesis as described in Usman *et al*., 1987 J. Am. Chem. Soc., 109, 7845-7854 and in Scaringe et al., 1990 Nucleic Acids Res., 18, 5433-5441 and makes use of common nucleic acid protecting and coupling groups, such as dimethoxytrityl at the 5'-end, and phosphoramidites at the 3'-end. The average stepwise coupling yields were >98%. Inactive ribozymes were synthesized by substituting a U for $G_5$ and a U for $A_{14}$ (numbering from Hertel et al., 1992 Nucleic Acids Res., 20, 3252). Hairpin ribozymes are synthesized in two parts and annealed to reconstruct the active ribozyme (Chowrira and Burke, 1992 *Nucleic Acids Res.,* 20, 2835-2840). Hairpin ribozymes are also synthesized from DNA templates using bacteriophage T7 RNA polymerase (Milligan and Uhlenbeck, 1989, *Methods Enzymol.* 180, 51). All ribozymes are modified extensively

to enhance stability by modification with nuclease resistant groups, for example, 2'-amino, 2'-C-allyl, 2'-flouro, 2'-o-methyl, 2'-H (for a review see Usman and Cedergren, 1992 *TIBS* 17, 34). Ribozymes are purified by gel electrophoresis using general methods or are purified by high pressure liquid chromatography and are resuspended in water.

The sequences of the chemically synthesized ribozymes useful in this study are shown in Tables 32, 34, 36, 37 and 38. Those in the art will recognize that these sequences are representative only of many more such sequences where the enzymatic portion of the ribozyme (all but the binding arms) is altered to affect activity. For example, stem-loop II sequence of hammerhead ribozymes listed in Tables 32 and 34(5'-GGCCGAAAGGCC-3') can be altered (substitution, deletion, and/or insertion) to contain any sequences provided a minimum of two base-paired stem structure can form. Similarly, stem-loop IV sequence of hairpin ribozymes listed in Tables 37 and 38 (5'-CACGUUGUG-3') can be altered (substitution, deletion, and/or insertion) to contain any sequence, provided a minimum of two base-paired stem structure can form. The sequences listed in Tables 32, 34, 36, 37 and 38 may be formed of ribonucleotides or other nucleotides or non-nucleotides. Such ribozymes are equivalent to the ribozymes described specifically in the Tables.

By engineering ribozyme motifs we have designed several ribozymes directed against RSV encoded mRNA sequences. These ribozymes are synthesized with modifications that improve their nuclease resistance. The ability of ribozymes to cleave target sequences *in vitro* is evaluated.

Numerous common cell lines can be infected with RSV for experimental purposes. These include *HeLa, Vero* and several primary epithelial cell lines. A cotton rat animal model of experimental human RSV infection is also available, and the bovine RSV is quite homologous to the human viruses. Rapid clinical diagnosis is through the use of kits designed for the immunofluorescence staining of RSV-infected cells or an ELISA assay, both of which are adaptable for experimental study. RSV encoded mRNA levels will be assessed by Northern analysis, RNAse protection, primer extension analysis or quantitative RT-PCR. Ribozymes that block the induction of RSV activity and/or 1C, 1B and N protein encoding mRNAs by more than 90% will be identified.

Optimizing Ribozyme Activity

Ribozyme activity can be optimized as described by Draper et al., PCT WO93/23569. The details will not be repeated here, but include altering the length of the ribozyme binding arms or chemically synthesizing ribozymes with modifications that prevent their degradation by serum ribonucleases (see *e.g.,* Eckstein *et al*., International Publication No. WO 92/07065; Perrault *et al.,* 1990 Nature 344, 565; Pieken et al., 1991 Science 253, 314; Usman and Cedergren, 1992 Trends in Biochem. Sci. 17, 334; Usman et al., International Publication No. WO 93/15187; and Rossi et al., International Publication No. WO 91/03162, as well as Jennings *et al*., WO 94/13688, which describe various chemical modifications that can be made to the sugar moieties of enzymatic RNA molecules. All these publications are hereby incorporated by reference herein.), modifications which enhance their efficacy in cells, and removal of stem II bases to shorten RNA synthesis times and reduce chemical requirements.

Sullivan, et al., PCT WO94/02595, incorporated by reference herein, describes the general methods for delivery of enzymatic RNA molecules . Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. The RNA/vehicle combination is locally delivered by direct injection or by use of a catheter, infusion pump or stent. Alternative routes of delivery include, but are not limited to, intravenous injection, intramuscular injection, subcutaneous injection, aerosol inhalation, oral (tablet or pill form), topical, systemic, ocular, intraperitoneal and/or intrathecal delivery. More detailed descriptions of ribozyme delivery and administration are provided in Sullivan, et al., supra and Draper, et al., supra which have been incorporated by reference herein.

Another means of accumulating high concentrations of a ribozyme(s) within cells is to incorporate the ribozyme-encoding sequences into a DNA expression vector. Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990 Proc. Natl. Acad. Sci. U S A, 87, 6743-7; Gao and Huang 1993 Nucleic Acids Res., 21, 2867-72; Lieber et al., 1993 Methods Enzymol., 217, 47-66; Zhou et al., 1990 Mol. Cell. Biol., 10, 4529-37). Several investigators have demonstrated that ribozymes expressed from such promoters can function in mammalian cells (e.g. Kashani-Sabet et al., 1992 Antisense Res. Dev., 2, 3-15; Ojwang et al., 1992 Proc. Natl. Acad. Sci. U S A, 89, 10802-6; Chen et al., 1992 Nucleic Acids Res., 20, 4581-9; Yu et al., 1993 Proc. Natl. Acad. Sci. U S A, 90, 6340-4; L'Huillier et al., 1992 EMBO J. 11, 4411-8; Lisziewicz et al., 1993 Proc. Natl. Acad. Sci. U. S. A., 90, 8000-4). The above ribozyme transcription units can be incorporated into a variety of vectors for introduction into mammalian cells, including but not restricted to, plasmid DNA vectors, viral DNA vectors (such as adenovirus or

adeno-associated virus vectors), or viral RNA vectors (such as retroviral, or alpha virus vectors).

In a preferred embodiment of the invention, a transcription unit expressing a ribozyme that cleaves target RNA is inserted into a plasmid DNA vector, a retrovirus DNA viral vector, an adenovirus DNA viral vector or an adeno-associated virus vector or alpha virus vector. These and other vectors have been used to transfer genes to live animals (for a review see Friedman, 1989 Science 244, 1275-1281; Roemer and Friedman, 1992 Eur. J. Biochem. 208, 211-225) and leads to transient or stable gene expression. The vectors are delivered as recombinant viral particles. DNA may be delivered alone or complexed with vehicles (as described for RNA above). The DNA, DNA/vehicle complexes, or the recombinant virus particles are locally administered to the site of treatment, *e.g.*, through the use of a catheter, stent or infusion pump.

Diagnostic uses

Ribozymes of this invention may be used as diagnostic tools to examine genetic drift and mutations within diseased cells. The close relationship between ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structure and function *in vitro*, as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple ribozymes targeted to different genes, ribozymes coupled with known small molecule inhibitors, or intermittent treatment with combinations of ribozymes and/or other chemical or biological molecules). Other in vitro uses of ribozymes of this invention are well known in the art, and include detection of the presence of mRNA associated with ICAM-1, relA, TNF-$\alpha$, p210, $^{bcr\text{-}abl}$ or RSV related condition. Such RNA is detected by determining the presence of a cleavage product after treatment with a ribozyme using standard methodology.

In a specific example, ribozymes which can cleave only wild-type or mutant forms of the target RNA are used for the assay. The first ribozyme is used to identify wild-type RNA present in the sample and the second ribozyme will be used to identify mutant RNA in the sample. As reaction controls, synthetic substrates of both wild-type and mutant RNA will be cleaved by both ribozymes to demonstrate the relative ribozyme efficiencies in the reactions and the absence of cleavage of the "non-targeted" RNA species. The cleavage products from the synthetic substrates will also serve to generate size markers for the analysis of wild-type and mutant RNAs in the sample population. Thus each analysis will require two ribozymes, two substrates and one unknown sample which will be combined into six reactions. The presence of cleavage products will be determined using an RNAse protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype (i.e., ICAM-1, rel A, TNF$\propto$, p210$^{bcr\text{-}abl}$ or RSV) is adequate to establish risk. If probes of comparable specific activity are used for both transcripts, then a qualitative comparison of RNA levels will be adequate and will decrease the cost of the initial diagnosis. Higher mutant form to wild-type ratios will be correlated with higher risk whether RNA levels are compared qualitatively or quantitatively.

## II. Chemical Synthesis Of Ribozymes

There follows the chemical synthesis, deprotection, and purification of RNA, enzymatic RNA or modified RNA molecules in greater than milligram quantities with high biological activity. Applicant has determined that the synthesis of enzymatically active RNA in high yield and quantity is dependent upon certain critical steps used during its preparation. Specifically, it is important that the RNA phosphoramidites are coupled efficiently in terms of both yield and time, that correct exocyclic amino protecting groups be used, that the appropriate conditions for the removal of the exocyclic amino protecting groups and the alkylsilyl protecting groups on the 2'-hydroxyl are used, and that the correct work-up and purification procedure of the resulting ribozyme be used.

To obtain a correct synthesis in terms of yield and biological activity of a large RNA molecule (*i.e.*, about 30 to 40 nucleotide bases), the protection of the amino functions of the bases requires either amide or substituted amide protecting groups, which must be, on the one hand, stable enough to survive the conditions of synthesis, and on the other hand, removable at the end of the synthesis. These requirements are met by the amide protecting groups shown in Figure 8, in particular, benzoyl for adenosine, isobutyryl or benzoyl for cytidine, and isobutyryl for guanosine, which may be removed at the end of the synthesis by incubating the RNA in $NH_3$/EtOH (ethanolic ammonia) for 20 h at 65 °C. In the case of the phenoxyacetyl type protecting groups shown in Figure 8 on guanosine and adenosine and acetyl protecting groups on cytidine, an incubation in ethanolic ammonia for 4 h at 65 °C is used to obtain complete removal

of these protecting groups. Removal of the alkylsilyl 2'-hydroxyl protecting groups can be accomplished using a tetrahydrofuran solution of TBAF at room temperature for 8-24 h.

The most quantitative procedure for recovering the fully deprotected RNA molecule is by either ethanol precipitation, or an anion exchange cartridge desalting, as described in Scaringe *et al. Nucleic Acids Res*. **1990**, *18*, 5433-5341. The purification of the long RNA sequences may be accomplished by a two-step chromatographic procedure in which the molecule is first purified on a reverse phase column with either the trityl group at the 5' position on or off. This purification is accomplished using an acetonitrile gradient with triethylammonium or bicarbonate salts as the aqueous phase. In the case of the trityl on purification, the trityl group may be removed by the addition of an acid and drying of the partially purified RNA molecule. The final purification is carried out on an anion exchange column, using alkali metal perchlorate salt gradients to elute the fully purified RNA molecule as the appropriate metal salts, *e. g.* $Na^+$, $Li^+$ *etc.* A final de-salting step on a small reverse-phase cartridge completes the purification procedure. Applicant has found that such a procedure not only fails to adversely affect activity of a ribozyme, but may improve its activity to cleave target RNA molecules.

Applicant has also determined that significant (see Tables 39-41) improvements in the yield of desired full length product (FLP) can be obtained by:

1. Using 5-*S*-alkyltetrazole at a delivered or effective concentration of 0.25-0.5 M or 0.15-0.35 M for the activation of the RNA (or analogue) amidite during the coupling step. (By delivered is meant that the actual amount of chemical in the reaction mix is known. This is possible for large scale synthesis since the reaction vessel is of size sufficient to allow such manipulations. The term effective means that available amount of chemical actually provided to the reaction mixture that is able to react with the other reagents present in the mixture. Those skilled in the art will recognize the meaning of these terms from the examples provided herein.) The time for this step is shortened from 10-15 m, *vide supra,* to 5-10 m. Alkyl, as used herein, refers to a saturated aliphatic hydrocarbon, including straight-chain, branched-chain, and cyclic alkyl groups. Preferably, the alkyl group has 1 to 12 carbons. More preferably it is a lower alkyl of from 1 to 7 carbons, more preferably 1 to 4 carbons. The alkyl group may be substituted or unsubstituted. When substituted the substituted group(s) is preferably, hydroxyl, cyano, alkoxy, =O, =S, $NO_2$ or $N(CH_3)_2$, amino, or SH. The term also includes alkenyl groups which are unsaturated hydrocarbon groups containing at least one carbon-carbon double bond, including straight-chain, branched-chain, and cyclic groups. Preferably, the alkenyl group has 1 to 12 carbons. More preferably it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, =O, =S, $NO_2$, halogen, $N(CH_3)_2$, amino, or SH. The term "alkyl" also includes alkynyl groups which have an unsaturated hydrocarbon group containing at least one carbon-carbon triple bond, including straight-chain, branched-chain, and cyclic groups. Preferably, the alkynyl group has 1 to 12 carbons. More preferably it is a lower alkynyl of from 1 to 7 carbons, more preferably 1 to 4 carbons. The alkynyl group may be substituted or unsubstituted. When substituted the substituted group(s) is preferably, hydroxyl, cyano, alkoxy, =O, =S, $NO_2$ or $N(CH_3)_2$, amino or SH.
   Such alkyl groups may also include aryl, alkylaryl, carbocyclic aryl, heterocyclic aryl, amide and ester groups. An "aryl" group refers to an aromatic group which has at least one ring having a conjugated n electron system and includes carbocyclic aryl, heterocyclic aryl and biaryl groups, all of which may be optionally substituted. The preferred substituent(s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above. Carbocyclic aryl groups are groups wherein the ring atoms on the aromatic ring are all carbon atoms. The carbon atoms are optionally substituted. Heterocyclic aryl groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the like, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

2. Using 5-S-alkyltetrazole at an effective, or final, concentration of 0.1-0.35 M for the activation of the RNA (or analogue) amidite during the coupling step. The time for this step is shortened from 10-15 m, *vide supra*, to 5-10 m.

3. Using alkylamine (MA, where alkyl is preferably methyl, ethyl, propyl or butyl) or $NH_4OH$/alkylamine (AMA, with the same preferred alkyl groups as noted for MA) @ 65 °C for 10-15 m to remove the exocyclic amino protecting groups *(vs* 4-20 h @ 55-65 °C using $NH_4OH$/EtOH or $NH_3$/EtOH, *vide supra).* Other alkylamines, *e.g.* ethylamine, propylamine, butylamine *etc.* may also be used.

4. Using anhydrous triethylamine•hydrogen fluoride (aHF•TEA) @ 65 °C for 0.5-1.5 h to remove the 2'-hydroxyl

alkylsilyl protecting group *(vs* 8 - 24 h using TBAF, *vide supra* or TEA•3HF for 24 h (Gasparutto *et al. Nucleic Acids Res.* **1992,** 20, 5159-5166). Other alkylamine•HF complexes may also be used, *e.g.* trimethylamine or diisopropylethylamine.

5. The use of anion-exchange resins to purify and/or analyze the fully deprotected RNA. These resins include, but are not limited to, quartenary or tertiary amino derivatized stationary phases such as silica or polystyrene. Specific examples include Dionex-NA100®, Mono-Q®, Poros-Q®.

Thus, the invention features an improved method for the coupling of RNA phosphoramidites; for the removal of amide or substituted amide protecting groups; and for the removal of 2'-hydroxyl alkylsilyl protecting groups. Such methods enhance the production of RNA or analogs of the type described above (*e.g.*, with substituted 2'-groups), and allow efficient synthesis of large amounts of such RNA. Such RNA may also have enzymatic activity and be purified without loss of that activity. While specific examples are given herein, those in the art will recognize that equivalent chemical reactions can be performed with the alternative chemicals noted above, which can be optimized and selected by routine experimentation.

In another aspect, the invention features an improved method for the purification or analysis of RNA or enzymatic RNA molecules (*e.g*. 28-70 nucleotides in length) by passing said RNA or enzymatic RNA molecule over an HPLC, *e.g*., reverse phase and/or an anion exchange chromatography column. The method of purification improves the catalytic activity of enzymatic RNAs over the gel purification method (see Figure 10).

Draper et al., PCT WO93/23569, incorporated by reference herein, disclosed reverse phase HPLC purification. The purification of long RNA molecules may be accomplished using anion exchange chromatography, particularly in conjunction with alkali perchlorate salts. This system may be used to purify very long RNA molecules. In particular, it is advantageous to use a Dionex NucleoPak 100© or a Pharmacia Mono Q® anion exchange column for the purification of RNA by the anion exchange method. This anion exchange purification may be used following a reverse-phase purification or prior to reverse phase purification. This method results in the formation of a sodium salt of the ribozyme during the chromatography. Replacement of the sodium alkali earth salt by other metal salts, *e.g.*, lithium, magnesium or calcium perchlorate, yields the corresponding salt of the RNA molecule during the purification.

In the case of the 2-step purification procedure, in which the first step is a reverse phase purification followed by an anion exchange step, the reverse phase purification is best accomplished using polymeric, *e.g.* polystyrene based, reverse-phase media, using either a 5'-trityl-on or 5'-trityl-off method. Either molecule may be recovered using this reverse-phase method, and then, once detritylated, the two fractions may be pooled and then submitted to an anion exchange purification step as described above.

The method includes passing the enzymatically active RNA molecule over a reverse phase HPLC column; the enzymatically active RNA molecule is produced in a synthetic chemical method and not by an enzymatic process; and the enzymatic RNA molecule is partially blocked, and the partially blocked enzymatically active RNA molecule is passed over a reverse phase HPLC column to separate it from other RNA molecules.

In more preferred embodiments, the enzymatically active RNA molecule, after passage over the reverse phase HPLC column, is deprotected and passed over a second reverse phase HPLC column (which may be the same as the reverse phase HPLC column), to remove the enzymatic RNA molecule from other components. In addition, the column is a silica or organic polymer-based C4, C8 or C18 column having a porosity of at least 125 Å, preferably 300 Å, and a particle size of at least 2 μm, preferably 5 μm.

Activation

The synthesis of RNA molecules may be accomplished chemically or enzymatically. In the case of chemical synthesis the use of tetrazole as an activator of RNA phosphoramidites is known (Usman *et al. J. Am. Chem. Soc*. **1987**, 109, 7845-7854). In this, and subsequent reports, a 0.5 M solution of tetrazole is allowed to react with the RNA phosphoramidite and couple with the polymer bound 5'-hydroxyl group for 10 m. Applicant has determined that using 0.25-0.5 M solutions of 5-*S*-alkyltetrazoles for only 5 min gives equivalent or better results. The following exemplifies the procedure.

Example 7: Synthesis of RNA and Ribozymes Using 5-*S*-Alkyltetrazoles as Activating Agent

The method of synthesis used follows the general procedure for RNA synthesis as described in Usman et al., 1987 *supra* and in Scaringe et al., *Nucleic Acids Res.* 1990, 18, 5433-5441 and makes use of common nucleic acid protecting and coupling groups, such as dimethoxytrityl at the 5'-end, and phosphoramidites at the 3'-end. The major difference used was the activating agent, 5-*S*-ethyl or -methyltetrazole @ 0.25 M concentration for 5 min.

All small scale syntheses were conducted on a 394 (ABI) synthesizer using a modified 2.5 μmol scale protocol

with a reduced 5 min coupling step for alkylsilyl protected RNA and 2.5 m coupling step for 2'-*O*-methylated RNA. A 6.5-fold excess (162.5 µL of 0.1 M = 32.5 µmol) of phosphoramidite and a 40-fold excess of S-ethyl tetrazole (400 µL of 0.25 M = 100 µmol) relative to polymer-bound 5'-hydroxyl was used in each coupling cycle. Average coupling yields on the 394, determined by colorimetric quantitation of the trityl fractions, was 97.5-99%. Other oligonucleotide synthesis reagents for the 394: Detritylation solution was 2% TCA in methylene chloride; capping was performed with 16% *N*-Methyl imidazole in THF and 10% acetic anhydride/10% 2,6-lutidine in THF; oxidation solution was 16.9 mM $I_2$, 49 mM pyridine, 9% water in THF. Fisher Synthesis Grade acetonitrile was used directly from the reagent bottle. *S*-Ethyl tetrazole solution (0.25 M in acetonitrile) was made up from the solid obtained from Applied Biosystems.

All large scale syntheses were conducted on a modified (eight amidite port capacity) 390Z (ABI) synthesizer using a 25 µmol scale protocol with a 5-15 min coupling step for alkylsilyl protected RNA and 7.5 m coupling step for 2'-*O*-methylated RNA. A six-fold excess (1.5 mL of 0.1 M = 150 µmol) of phosphoramidite and a forty-five-fold excess of *S*-ethyl tetrazole (4.5 mL of 0.25 M = 1125 µmol) relative to polymer-bound 5'-hydroxyl was used in each coupling cycle. Average coupling yields on the 390Z, determined by colorimetric quantitation of the trityl fractions, was 95.0-96.7%. Oligonucleotide synthesis reagents for the 390Z: Detritylation solution was 2% DCA in methylene chloride; capping was performed with 16% *N*-Methyl imidazole in THF and 10% acetic anhydride/10% 2,6-lutidine in THF; oxidation solution was 16.9 mM $I_2$, 49 mM pyridine, 9% water in THF. Fisher Synthesis Grade acetonitrile was used directly from the reagent bottle. S-Ethyl tetrazole solution (0.25-0.5 M in acetonitrile) was made up from the solid obtained from Applied Biosystems.

Deprotection

The first step of the deprotection of RNA molecules may be accomplished by removal of the exocyclic amino protecting groups with. either $NH_4OH$/EtOH:3/1 (Usman *et al. J. Am. Chem. Soc.* 1987, *109*, 7845-7854) or $NH_3$/EtOH (Scaringe *et al. Nucleic Acids Res.* 1990, *18*, 5433-5341) for ~20 h @ 55-65 °C. Applicant has determined that the use of methylamine or $NH_4OH$/methylamine for 10-15 min @ 55-65 °C gives equivalent or better results. The following exemplifies the procedure.

Example 8: RNA and Ribozyme Deprotection of Exocyclic Amino

Protecting Groups Using Methylamine (MA) or $NH_4OH$/Methylamine (AMA)

The polymer-bound oligonucleotide, either trityl-on or off, was suspended in a solution of methylamine (MA) or $NH_4OH$/methylamine (AMA) @ 55-65 °C for 5-15 min to remove the exocyclic amino protecting groups. The polymer-bound oligoribonucleotide was transferred from the synthesis column to a 4 mL glass screw top vial. $NH_4OH$ and aqueous methylamine were pre-mixed in equal volumes. 4 mL of the resulting reagent was added to the vial, equilibrated for 5 m at RT and then heated at 55 or 65 °C for 5-15 min. After cooling to -20 °C, the supernatant was removed from the polymer support. The support was washed with 1.0 mL of EtOH:MeCN:$H_20$/3:1:1, vortexed and the supernatant was then added to the first supernatant. The combined supernatants, containing the oligoribonucleotide, were dried to a white powder. The same procedure was followed for the aqueous methylamine reagent.

Table 40 is a summary of the results obtained using the improvements outlined in this application for base deprotection.

The second step of the deprotection of RNA molecules may be accomplished by removal of the 2'-hydroxyl alkylsilyl protecting group using TBAF for 8-24 h (Usman *et al. J. Am. Chem. Soc.* 1987, *109*, 7845-7854). Applicant has determined that the use of anhydrous TEA•HF in *N*-methylpyrrolidine (NMP) for 0.5-1.5 h @ 55-65 °C gives equivalent or better results. The following exemplifies this procedure.

Example 9: RNA and Ribozyme Deprotection of 2'-Hydroxyl Alkylsilyl Protecting Groups Using Anhydrous TEA•HF

To remove the alkylsilyl protecting groups, the ammonia-deprotected oligoribonucleotide was resuspended in 250 µL of 1.4 M anhydrous HF solution (1.5 mL *N*-methylpyrrolidine, 750 µL TEA and 1.0 mL TEA•3HF) and heated to 65 °C for 1.5 h. 9 mL of 50 mM TEAB was added to quench the reaction. The resulting solution was loaded onto a Qiagen 500® anion exchange cartridge (Qiagen Inc.) prewashed with 10 mL of 50 mM TEAB. After washing the cartridge with 10 mL of 50 mM TEAB, the RNA was eluted with 10 mL of 2 M TEAB and dried down to a white powder.

Table 41 is a summary of the results obtained using the improvements outlined in this application for alkylsilyl deprotection.

Example 10: HPLC Purification, Anion Exchange column

For a small scale synthesis, the crude material was diluted to 5 mL with diethylpyrocarbonate treated water. The sample was injected onto either a Pharmacia Mono Q® 16/10 or Dionex NucleoPac® column with 100% buffer A (10 mM $NaClO_4$). A gradient from 180-210 mM $NaClO_4$ at a rate of 0.85 mM/void volume for a Pharmacia Mono Q® anion-exchange column or 100-150 mM $NaClO_4$ at a rate of 1.7 mM/void volume for a Dionex NucleoPac® anion-exchange column was used to elute the RNA. Fractions were analyzed by a HP-1090 HPLC with a Dionex NucleoPac® column. Fractions containing full length product at ≥80% by peak area were pooled.

For a trityl-off large scale synthesis, the crude material was desalted by applying the solution that resulted from quenching of the desilylation reaction to a 53 mL Pharmacia HiLoad 26/10 Q-Sepharose® Fast Flow column. The column was thoroughly washed with 10 mM sodium perchlorate buffer. The oligonucleotide was eluted from the column with 300 mM sodium perchlorate. The eluent was quantitated and an analytical HPLC was run to determine the percent full length material in the synthesis. The eluent was diluted four fold in sterile $H_2O$ to lower the salt concentration and applied to a Pharmacia Mono Q® 16/10 column. A gradient from 10-185 mM sodium perchlorate was run over 4 column volumes to elute shorter sequences, the full length product was then eluted in a gradient from 185-214 mM sodium perchlorate in 30 column volumes. The fractions of interest were analyzed on a HP-1090 HPLC with a Dionex NucleoPac® column. Fractions containing over 85% full length material were pooled. The pool was applied to a Pharmacia RPC® column for desalting.

For a trityl-on large scale synthesis, the crude material was desalted by applying the solution that resulted from quenching of the desilylation reaction to a 53 mL Pharmacia HiLoad 26/10 Q-Sepharose® Fast Flow column. The column was thoroughly washed with 20 mM $NH_4CO_3H$/10% $CH_3CN$ buffer. The oligonucleotide was eluted from the column with 1.5 M $NH_4CO_3H$/10% acetonitrile. The eluent was quantitated and an analytical HPLC was run to determine the percent full length material present in the synthesis. The oligonucleotide was then applied to a Pharmacia Resource RPC column. A gradient from 20-55% B (20 mM $NH_4CO_3H$/25% $CH_3CN$, buffer A = 20 mM $NH_4CO_3H$/10% $CH_3CN$) was run over 35 column volumes. The fractions of interest were analyzed on a HP-1090 HPLC with a Dionex NucleoPac® column. Fractions containing over 60% full length material were pooled. The pooled fractions were then submitted to manual detritylation with 80% acetic acid, dried down immediately, resuspended in sterile $H_2O$, dried down and resuspended in $H_2O$ again. This material was analyzed on a HP 1090-HPLC with a Dionex NucleoPac® column. The material was purified by anion exchange chromatography as in the trityl-off scheme (*vide supra).*

Example 11 Ribozyme Activity Assay

Purified 5'-end labeled RNA substrates (15-25-mers) and purified 5'-end labeled ribozymes (~36-mers) were both heated to 95 °C, quenched on ice and equilibrated at 37 °C, separately. Ribozyme stock solutions were 1 μM, 200 nM, 40 nM or 8 nM and the final substrate RNA concentrations were - 1 nM. Total reaction volumes were 50 μL. The assay buffer was 50 mM Tris-Cl, pH 7,5 and 10 mM $MgCl_2$. Reactions were initiated by mixing substrate and ribozyme solutions at t = 0. Aliquots of 5 μL were removed at time points of 1, 5, 15, 30, 60 and 120 m. Each aliquot was quenched in formamide loading buffer and loaded onto a 15% denaturing polyacrylamide gel for analysis. Quantitative analyses were performed using a phosphorimager (Molecular Dynamics).

Example 12: One pot deprotection of RNA

Applicant has shown that aqueous methyl amine is an efficient reagent to deprotect bases in an RNA molecule. However, in a time consuming step (2-24 hrs), the RNA sample needs to be dried. completely prior to the deprotection of the sugar 2'-hydroxyl groups. Additionally, deprotection of RNA synthesized on a large scale (e.g., 100 μmol) becomes challenging since the volume of solid support used is quite large. In an attempt to minimize the time required for deprotection and to simplify the process of deprotection of RNA synthesized on a large scale, applicant' describes a one pot deprotection protocol (Fig. 12). According to this protocol, anhydrous methylamine is used in place of aqueous methyl amine. Base deprotection is carried out at 65 °C for 15 min and the reaction is allowed to cool for 10 min. Deprotection of 2'-hydroxyl groups is then carried out in the same container for 90 min in a TEA•3HF reagent. The reaction is quenched with 16 mM TEAB solution.

Referring to Fig. 13, hammerhead ribozyme targeted to site B is synthesized using RNA phosphoramadite chemistry and deprotected using either a two pot or a one pot protocol. Profiles of these ribozymes on an HPLC column are compared. The figure shows that RNAs deprotected by either the one pot or the two pot protocols yield similar full-length product profiles. Applicant has shown that using a one pot deprotection protocol, time required for RNA deprotection can be reduced considerably without compromising the quality or the yield of full length RNA.

Referring to Fig. 14, hammerhead ribozymes targeted to site B (from Fig. 13) are tested for their ability to cleave RNA. As shown in the figure 14, ribozymes that are deprotected using one pot protocol have catalytic activity

comparable to ribozymes that are deprotected using a two pot protocol.

Example 12a: Improved protocol for the synthesis of phosphorothioate containing RNA and ribozymes using 5-S-Alkyltetrazoles as Activating Agent

The two sulfurizing reagents that have been used to synthesize ribophosphorothioates are tetraethylthiuram disulfide (TETD; Vu and Hirschbein, 1991 *Tetrahedron Letter* 31, 3005), and 3H-1,2-benzodithiol-3-one 1,1-dioxide (Beaucage reagent; Vu and Hirschbein, 1991 *supra*). TETD requires long sulfurization times (600 seconds for DNA and 3600 seconds for RNA). It has recently been shown that for sulfurization of DNA oligonucleotides, Beaucage reagent is more efficient than TETD (Wyrzykiewicz and Ravikumar, 1994 *Bioorganic Med. Chem.* 4, 1519). Beaucage reagent has also been used to synthesize phosphorothioate oligonucleotides containing 2'-deoxy-2'-fluoro modifications wherein the wait time is 10 min (Kawasaki et al., 1992 *J. Med. Chem).*

The method of synthesis used follows the procedure for RNA synthesis as described herein and makes use of common nucleic acid protecting and coupling groups, such as dimethoxytrityl at the '5'-end, and phosphoramidites at the 3'-end. The sulfurization step for RNA described in the literature is a 8 second delivery and 10 min wait steps (Beaucage and lyer, 1991 *Tetrahedron* 49, 6123). These conditions produced about 95% sulfurization as measured by HPLC analysis (Morvan et al., 1990 *Tetrahedron Letter* 31, 7149). This 5% contaminating oxidation could arise from the presence of oxygen dissolved in solvents and/or slow release of traces of iodine adsorbed on the inner surface of delivery lines during previous synthesis.

A major improvement is the use of an activating agent, 5-*S*-ethyltetrazole or 5-*S*-methyltetrazole at a concentration of 0.25 M for 5 min. Additionally, for those linkages which are phosporothioate, the iodine solution is replaced with a 0.05 M solution of 3H-1,2-benzodithiole-3-one 1,1-dioxide (Beaucage reagent) in acetonitrile. The delivery time for the sulfurization step is reduced to 5 seconds and the wait time is reduced to 300 seconds.

RNA synthesis is conducted on a 394 (ABI) synthesizer using a modified 2.5 μmol scale protocol with a reduced 5 min coupling step for alkylsilyl protected RNA and 2.5 min coupling step for 2'-*O*-methylated RNA. A 6.5-fold excess (162.5 μL of 0.1 M = 32.5 μmol) of phosphoramidite and a 40-fold excess of *S*--ethyl tetrazole (400 μL of 0.25 M = 100 μmol) relative to polymer-bound 5'-hydroxyl was used in each coupling cycle. Average coupling yields on the 394 synthesizer, determined by colorimetric quantitation of the trityl fractions, was 97.5-99%. Other oligonucleotide synthesis reagents for the 394 synthesizer: detritylation solution was 2% TCA in methylene chloride; capping was performed with 16% *N*-Methyl imidazole in THF and 10% acetic anhydride/10% 2,6-lutidine in THF; oxidation solution was 16.9 mM $I_2$, 49 mM pyridine, 9% water in THF. Fisher Synthesis Grade acetonitrile was used directly from the reagent bottle. *S*-Ethyl tetrazole solution (0.25 M in acetonitrile) was made up from the solid obtained from Applied Biosystems. Sulfurizing reagent was obtained from Glen Research.

Average sulfurization efficiency (ASE) is determined using the formula:

$$ASE = (PS/Total)^{1/n-1}$$

where, PS = integrated $^{31}P$ NMR values of the P=S diester
Total = integration value of all peaks
n = length of oligo

Referring to tables 42 and 43, effects of varying the delivery and the wait time for sulfurization with Beaucage's reagent is described. These data suggest that 5 second wait time and 300 second delivery time is the condition under which ASE is maximum.

Using the above conditions a 36 mer hammerhead ribozyme is synthesized which is targeted to site C. The ribozyme is synthesized to contain phosphorothioate linkages at four positions towards the 5' end. RNA cleavage activity of this ribozyme is shown in Fig. 16. Activity of the phosphorothioate ribozyme is comparable to the activity of a ribozyme lacking any phosphorothioate linkages.

Example 13: Protocol for the synthesis of 2'-N-phtalimido-nucleoside phosphoramidite

The 2'-amino group of a 2'-deoxy-2'-amino nucleoside is normally protected with N-(9-flourenylmethoxycarbonyl) (Fmoc; Imazawa and Eckstein, 1979 *supra*; Pieken et al., 1991 *Science* 253, 314). This protecting group is not stable in $CH_3CN$ solution or even in dry form during prolonged storage at -20 °C. These problems need to be overcome in order to achieve large scale synthesis of RNA.

Applicant describes the use of alternative protecting groups for the 2'-amino group of 2'-deoxy-2'-amino nucleoside. Referring to Figure 17, phosphoramidite **17** was synthesized starting from 2'-deoxy-2'-aminonucleoside (**12**) using transient protection with Markevich reagent (Markiewicz *J. Chem. Res.* 1979, S, 24). An intermediate **13** was

obtained in 50% yield, however subsequent introduction of N-phtaloyl (Pht) group by Nefken's method (Nefkens, 1960 *Nature* 185, 306), desilylation (**15**), dimethoxytrytilation (**16**) and phosphitylation led to phosphoramidite **17.** Since overall yield of this multi-step procedure was low (20%) applicant investigated some alternative approaches, concentrating on selective introduction of N-phtaloyl group without acylation of 5' and 3' hydroxyls.

When 2'-deoxy-2'-amino-nucleoside was reacted with 1.05 equivalents of Nefkens reagent in DMF overnight with subsequent treatment with $Et_3N$ (1 hour) only 10-15% of N and 5'(3')-bis-phtaloyl derivatives were formed with the major component being N-Pht-derivative **15**. The N,O-bis by-products could be selectively and quantitively converted to N-Pht derivative **15** by treatment of crude reaction mixture with cat. KCN/MeOH.

A convenient "one-pot" procedure for the synthesis of key intermediate **16** involves selective N-phthaloylation with subsequent dimethoxytritylation by DMTCl/$Et_3N$ and resulting in the preparation of DMT derivative **16** in 85% overall yield as follows. Standard phosphytilation of **16** produced phosphoramidite **17** in 87% yield. One gram of 2'-amino nucleoside, for example 2'-amino uridine (US Biochemicals® part # 77140) was co-evaporated twice from dry dimethyl formamide (Dmf) and dried in vacuo overnight. 50 mls of Aldrich sure-seal Dmf was added to the dry 2'-amino uridine via syringe and the mixture was stirred for 10 minutes to produce a clear solution. 1.0 grams (1.05 eq.) of N-carbethoxyphthalimide (Nefken's reagent, 98% Jannsen Chimica) was added and the solution was stirred overnight. Thin layer chromatography (TLC) showed 90% conversion to a faster moving products (10% ETOH in $CHCl_3$) and 57 µl of TEA (0.1 eq.) was added to effect closure of the phthalimide ring. After 1 hour an additional 855 µl (1.5 eq.) of TEA was added followed by the addition of 1.53 grams (1.1 eq.) of DMT-Cl (Lancaster Synthesis®, 98%). The reaction mixture was left to stir overnight and quenched with ETOH after TLC showed greater than 90% desired product. Dmf was removed under vacuum and the mixture was washed with sodium bicarbonate solution (5% aq., 500 mls) and extracted with ethyl acetate (2x 200 mls). A 25mm x 300mm flash column (75 grams Merck flash silica) was used for purification. Compound eluted at 80 to 85% ethyl acetate in hexanes (yield: 80% purity: >95% by $^1$HNMR). Phosphoramidites were then prepared using standard protocols described above.

With phosphoramidite 17 in hand applicant synthesized several ribozymes with 2'-deoxy-2'-amino modifications. Analysis of the synthesis demonstrated coupling efficiency in 97-98% range. RNA cleavage activity of ribozymes containing 2'-deoxy-2'-amino-U modifications at U4 and/or U7 positions (see Figure 1), wherein the 2'-amino positions were either protected with Fmoc or Pht, was identical. Additionally, complete deprotection of 2'-deoxy-2'-amino-Uridine was confirmed by base-composition analysis. The coupling efficiency of phosphoramidite 17 was not effected over prolonged storage (1-2 months) at low temperatures.

Protecting 2' Position with a SEM Group

There follows a method using the 2'-(trimethylsilyl)ethoxymethyl protecting group (SEM) in the synthesis of oligoribonucleotides, and in particular those enzymatic molecules described above. For the synthesis of RNA it is important that the 2'-hydroxyl protecting group be stable throughout the various steps of the synthesis and base deprotection. At the same time, this group should also be readily removed when desired. To that end the *t*-butyldimethylsilyl group has been efficacious (Usman,N.; Ogilvie,K.K.; Jiang,M.-Y.; Cedergren,R.J. *J. Am. Chem. Soc.* **1987,** *109*, 7845-7854 and Scaringe,S.A.; Franklyn,C.; Usman,N. *Nucl. Acids Res.* **1990**, *18,* 5433-5441). However, long exposure times to tetra-*n*-butylammonium fluoride (TBAF) are generally required to fully remove this protecting group from the 2'-hydroxyl. In addition, the bulky alkyl substituents can prove to be a hindrance to coupling thereby necessitating longer coupling times. Finally, it has been shown that the TBDMS group is base labile and is partially deprotected during treatment with ethanolic ammonia (Scaringe,S.A.; Franklyn,C.; Usman,N. *Nucl. Acids Res.* **1990**, *18,* 5433-5441 and Stawinski,J.; Stromberg,R.; Thelin,M.; Westman,E. *Nucleic Acids Res.* **1988**, *16*, 9285-9298).

The (trimethylsilyl)ethoxymethyl ether (SEM) seems a suitable substitute. This protecting group is stable to base and all but the harshest acidic conditions. Therefore it is stable under the conditions required for oligonucleotide synthesis. It can be readily introduced and the oxygen carbon bond makes it unable to migrate. Finally, the SEM group can be removed with $BF_3 \cdot OEt_2$ very quickly.

There follows a method for synthesis of RNA by protecting the 2'-position of a nucleotide during RNA synthesis with a (trimethylsilyl)ethoxymethyl (SEM) group. The method can involve use of standard RNA synthesis conditions as discussed below, or any other equivalent steps. Those in the art are familiar with such steps. The nucleotide used can be any normal nucleotide or may be substituted in various positions by methods well known in the art, e.g., as described by Eckstein *et al., International Publication* No. WO 92/07065, Perrault *et al.*, Nature **1990**, 344, 565-568, Pieken *et al.,* Science **1991**, *253*, 314-317, Usman,N.; Cedergren,R.J. *Trends in Biochem. Sci.* **1992**, *17*, 334-339, Usman et al., PCT WO93/15187, and Sproat,B. *European Patent Application 92110298.4*.

This invention also features a method for covalently linking a SEM group to the 2'-position of a nucleotide. The method involves contacting a nucleoside with an SEM-containing molecule under SEM bonding conditions. In a preferred embodiment, the conditions are dibutyltin oxide, tetrabutylammonium fluoride and SEM-CI. Those in the art, however, will recognize that other equivalent conditions can also be used.

In another aspect, the invention features a method for removal of an SEM group from a nucleoside molecule or an oligonucleotide. The method involves contacting the molecule or oligonucleotide with boron trifluoride etherate ($BF_3$•$OEt_2$) under SEM removing conditions, *e.g.*, in acetonitrile.

Referring to Figure 18, there is shown the method for solid phase synthesis of RNA. A 2',5'-protected nucleotide is contacted with a solid phase bound nucleotide under RNA synthesis conditions to form a dinucleotide. The protecting group (R) at the 2'-position in prior art methods can be a silyl ether, as shown in the Figure. In the method of the present invention, an SEM group is used in place of the silyl ether. Otherwise RNA synthesis can be performed by standard methodology.

Referring to Figure 19, there is shown the synthesis of 2'-*O*-SEM protected nucleosides and phosphoramadites. Briefly, a 5'-protected nucleoside (**1**) is protected at the 2'- or 3'-position by contacting with a derivative of SEM under appropriate conditions. Specifically, those conditions include contacting the nucleoside with dibutyltin oxide and SEM chloride. The 2 regioisomers are separated by chromatography and the 2'-protected moiety is converted into a phosphoramidite by standard procedure. The 3'-protected nucleoside is converted into a succinate derivative suitable for derivatization of a solid support.

Referring to Figure 20, a prior art method for deprotection of RNA using silyl ethers is shown. This contrasts with the method shown in Figure 21 in which deprotection of RNA containing an SEM group is performed. In step 1, the base protecting groups and cyanoethyl groups are removed by standard procedure. The SEM group is then removed as shown in the Figure. The details of the synthesis of phosphoramidites and SEM protected nucleosides and their use in synthesis of oligonucleotides and subsequent deprotection of

Example 14: Synthesis of 2'-*O*-((trimethylsilyl)ethoxymethyl)-5'-*O*-Di-methoxytrityl Uridine (**2**)

Referring to Figure 19, 5'-*O*-dimethoxytrityl uridine **1** (1.0 g, 1.83 mmol) in $CH_3CN$ (18 mL) was added dibutyltin oxide (1.0 g, 4.03 mmol) and TBAF (1 M, 2.38 mL, 2.38 mmol). The mixture was stirred for 2 h at RT (about 20-25°C) at which time (trimethylsilyl)ethoxymethyl chloride (SEM-Cl) (487 μL, 2.75 mmol) was added. The reaction mixture was stirred overnight and then filtered and evaporated. Flash chromatography (30% hexanes in ethyl acetate) yielded 347 mg (28.0%) of 2'-hydroxyl protected nucleoside **2** and 314 mg (25.3%) of 3'-hydroxyl protected nucleoside **3**.

Example 15: Synthesis of 2'-*O*-((trimethylsilyl)ethoxymethyl) Uridine (**4**)

Nucleoside **2** was detritylated following standard methods, as shown in Figure 19.

Example 16: Synthesis of 2'-*O*-((trimethylsilyl)ethoxymethyl)-5',3'-*O*-Acetyl Uridine (**5**)

Nucleoside **4** was acetylated following standard methods, as shown in Figure 19.

Example 17: Synthesis of 5',3'-*O*-Acetyl Uridine (**6**)

Referring to Figure 19, the fully protected uridine **5** (32 mg, 0.07 mmol) was dissolved in $CH_3CN$ (700 μL) and $BF_3$•$OEt_2$ (17.5 μL, 0.14 mmol) was added. The reaction was stirred 15 m and MeOH was added to quench the reaction. Flash chromatography (5% MeOH in $CH_2Cl_2$) gave 20 mg (88%) of SEM deprotected nucleoside **6**.

Example 18: Synthesis of 2'-*O*-((trimethylsilyl)ethoxymethyl)-3'-*O*-Succinyl-5'-*O*-Dimethoxytrityl Uridine (**2**)

Nucleoside **3** was succinylated and coupled to the support following standard procedures, as shown in Figure 19.

Example 19: Synthesis of 2'-*O*-((trimethylsilyl)ethoxymethyl)-5'-*O*-Di-methoxytrityl Uridine 3'-(2-Cyanoethyl *N,N*-diisopropylphosphoramidite) (8)

Nucleoside **3** was phosphitylated following standard methods, as shown in Figure 19.

Example 20: Synthesis of RNA Using 2'-*O*-SEM Protection

Referring to Figure 18, the method of synthesis used follows the general procedure for RNA synthesis as described in Usman,N.; Ogilvie,K.K.; Jiang,M.-Y.; Cedergren,R.J. *J. Am. Chem. Soc.* **1987**, *109*, 7845-7854 and in Scaringe,S.A.; Franklyn,C.; Usman,N. *Nucl. Acids Res.* **1990,** *18*, 5433-5441. The phosphoramidite **8** was coupled following standard RNA methods to provide a 10-mer of uridylic acid. Syntheses were conducted on a 394 (ABI) synthesizer using a modified 2.5 μmol scale protocol with a 10 m coupling step. A thirteen-fold excess (325 μL of 0.1 M = 32.5 μmol) of phosphoramidite and a 80-fold excess of tetrazole (400 μL of 0.5 M = 200 μmol) relative to polymer-bound 5'-hydroxyl was used in each coupling cycle. Average coupling yields on the 394, determined by colorimetric quantitation of the trityl fractions, were 98-99%. Other oligonucleotide synthesis reagents for the 394: Detritylation solution was 2% TCA in methylene chloride; capping was performed with 16% N-Methyl imidazole in THF and 10% acetic anhydride/10% 2,6-lutidine in THF; oxidation solution was 16.9 mM $I_2$, 49 mM pyridine, 9% water in THF. Fisher Synthesis Grade acetonitrile was used directly from the reagent bottle.

Referring to Figure 21, the homopolymer was base deprotected with $NH_3$/EtOH at 65 °C. The solution was decanted and the support was washed twice with a solution of 1:1:1 $H_2O$:$CH_3CN$:MeOH. The combined solutions were dried down and then diluted with $CH_3CN$ (1 mL). $BF_3$•$OEt_2$ (2.5 μL, 30 μmol) was added to the solution and aliquots were removed at ten time points. The results indicate that after 30 min deprotection is complete, as shown in Figure 22.

### III. Vectors Expressing Ribozymes

There follows a method for expression of a ribozyme in a bacterial or eucaryotic cell, and for production of large amounts of such a ribozyme. In general, the invention features a method for preparing multi-copy cassettes encoding a defined ribozyme structure for production of a ribozyme at a decreased cost. A vector is produced which encodes a plurality of ribozymes which are cleaved at their 3' and 5' ends from an RNA transcript producted from the vector by only one other ribozyme. The system is useful for scaling up production of a ribozyme, which may be either modified or unmodified, *in situ* or *in vitro.* Such vector systems can be used to express a desired ribozyme in a specific cell, or can be used in an *in vitro* system to allow productiuon of large amounts of a desired riboqyne, The vectors of this invention allow a higher yield synthesis of a ribozyme in the form of an RNA transcript which is cleaved *in situ* or *in vitro* before or after transcript isolation.

Thus, this invention is distinct from the prior art in that a single ribozyme is used to process the 3' and 5' ends of each therapeutic, trans-acting or desired ribozyme instead of processing only one end, or only one ribozyme. This allows smaller vectors to be derived with multiple trans-acting ribozymes released by only one other ribozyme from the mRNA transcript. Applicant has also provided methods by which the activity of such ribozymes is increased compared to those in the art, by designing ribozyme-encoding vectors and the corresponding transcript such that folding of the mRNA does not interfere with processing by the releasing ribozyme.

The stability of the ribozyme produced in this method can be enhanced by provision of sequences at the termini of the ribozymes as described by Draper et al., PCT WO 93/23509, hereby incorporated by reference herein.

The method of this invention is advantageous since it provides high yield synthesis of ribozymes by use of low cost transcription-based protocols, compared to existing chemical ribozyme synthesis, and can use isolation techniques currently used to purify chemically synthesized oligonucleotides. Thus, the method allows synthesis of ribozymes in high yield at low cost for analytical, diagnostic, or therapeutic applications.

The method is also useful for synthesis of ribozymes *in vitro* for ribozyme structural studies, enzymatic studies, target RNA accessibility studies, transcription inhibition studies and nuclease protection studies, much is described by Draper et al., PCT WO 93/23509 hereby incorporated by reference herein.

The method can also be used to produce ribozymes *in situ* either to increase the intracellular concentration of a desired therapeutic ribozyme, or to produce a concatameric transcript for subsequent *in vitro* isolation of unit length ribozyme. The desired ribozyme can be used to inhibit gene expression in molecular genetic analyses or in infectious cell systems, and to test the efficacy of a therapeutic molecule or treat afflicted cells.

Thus, in general, the invention features a vector which includes a bacterial, viral or eucaryotic promoter within a plasmid, cosmid, phagmid, virus, viroid, virusoid or phage vector. Other vectors are equally suitable and include double-stranded, or partially double-stranded DNA, formed by an amplification method such as the polymerase chain reaction, or double-stranded, partially double-stranded or single-stranded RNA, formed by site-directed homologous recombination into viral or viroid RNA genomes. Such vectors need not be circular. Transcriptionally linked to the promoter region is a first ribozyme-encoding region, and nucleotide sequences encoding a ribozyme cleavage sequence which is placed on either side of a region encoding a therapeutic or otherwise desired second ribozyme. Suitable restriction endonuclease sites can be provided to ease construction of this vector in DNA vectors or in requisite DNA vectors of an RNA expression system. The desired second ribozyme may be any desired type of ribozyme, such as a hammerhead, hairpin , hepatitis delta virus (HDV) or other catalytic center, and can include group I and group II introns, as discussed above. The first ribozyme is chosen to cleave the encoded cleavage sequence, and may also be any desired ribozyme, for example, a *Tetrahymena* derived ribozyme, which may, for example, include an imbedded restriction endonuclease site in the center of a self-recognition sequence to aid in vector construction. This endonuclease site is useful for construction of the vector, and subsequent analysis of the vector.

When the promoter of such a vector is activated an RNA transcript is produced which includes the first and second ribozyme sequences. The first ribozyme sequence is able to act, under appropriate conditions, to cause cleavage at the cleavage sites to release the second ribozyme sequences. These second ribozyme sequences can then act at their target RNA sites, or can be isolated for later use or analysis.

Thus, in one aspect the invention features a vector which includes a first nucleic acid sequence (encoding a first ribozyme having intramolecular cleaving activity), and a second nucleic acid sequence (encoding a second ribozyme having intermolecular cleaving enzymatic activity) flanked by nucleic acid sequences encoding RNA which is cleaved by the first ribozyme to release the second ribozyme from the RNA transcript encoded by the vector. The second ribozyme may be flanked by the first ribozyme either on the 5' side or 3' side. If desired, the first ribozyme may be encoded on a separate vector and may have intermolecular cleaving activity.

As discussed above, the first ribozyme can be chosen to be any self-cleaving ribozyme, and the second ribozyme may be chosen to be any desired ribozyme. The flanking sequences are chosen to include sequences recognized by the first ribozyme. When the vector is caused to express RNA from these nucleic acid sequences, that RNA has the ability under appropriate conditions to cleave each of the flanking regions and thereby release one or more copies of the second ribozyme. If desired, several different second ribozymes can be produced by the same vector, or several different vectors can be placed in the same vessel or cell to produce different ribozymes.

In preferred embodiments, the vector includes a plurality of the nucleic acid sequences encoding the second ribozyme, each flanked by nucleic acid sequences recognized by the first ribozyme. Most preferably, such a plurality includes at least six to nine or even between 60 - 100 nucleic acid sequences. In other preferred embodiments, the vector includes a promoter which regulates expression of the nucleic acid encoding the ribozymes from the vector; and the vector'is chosen from a plasmid, cosmid, phagmid, virus, viroid or phage. In a most preferred embodiment, the plurality of nucleic acid sequences are identical and are arranged in sequential order such that each has an identical end nearest to the promoter. If desired, a poly(A) sequence adjacent to the sequence encoding the first or second ribozyme may be provided to increase stability of the RNA produced by the vector; and a restriction endonuclease site adjacent to the nucleic acid encoding the first ribozyme is provided to allow insertion of nucleic acid encoding the second ribozyme during construction of the vector.

In a second aspect, the invention features a method for formation of a ribozyme expression vector by providing a vector including nucleic acid encoding a first ribozyme, as discussed above, and providing a single-stranded DNA encoding a second ribozyme, as discussed above. The single-stranded DNA is then allowed to anneal to form a partial duplex DNA which can be filled in by a treatment with an appropriate enzyme, such as a DNA polymerase in the presence of dNTPs, to form a duplex DNA which can then be ligated to the vector. Large vectors resulting from this method can then be selected to insure that a high copy number of the single-stranded DNA encoding the second ribozyme is incorporated into the vector.

In a further aspect, the invention features a method for production of ribozymes by providing a vector as described above, expressing RNA from that vector, and allowing cleavage by the first ribozyme to release the second ribozyme.

In preferred embodiments, three different ribozyme motifs are used as cis-cleaving ribozymes. The hammerhead, hairpin, and hepatitis delta virus (HDV) ribozyme motifs consist of small, well-defined sequences that rapidly self-cleave *in vitro* (Symons, 1992 Annu. Rev. Biochem. 61, 641). While structural and functional differences exist among the three ribozyme motifs, they self-process efficiently *in vivo.* All three ribozyme motifs self-process to 87-95% completion in the absence of 3' flanking sequences. *In vitro,* the self-processing constructs described in this invention are significantly more active than those reported by Taira et al., 1990 supra; and Altschuler et al., 1992 Gene 122, 85. The present invention enables the use of cis-cleaving ribozymes to efficiently truncate RNA molecules at specific sites *in vivo* by ensuring lack of secondary structure which prevents processing.

## Isolation of Therapeutic Ribozyme

The preferred method of isolating therapeutic ribozyme is by a chromatographic technique. The HPLC purification methods and reverse HPLC purification methods described by Draper et al., PCT WO 93/23509, hereby incorporated by reference herein, can be used. Alternatively, the attachment of complementary oligonucleotides to cellulose or other chromatography columns allows isolation of the therapeutic second ribozyme, for example, by hybridization to the region between the flanking arms and the enzymatic RNA. This hybridization will select against the short flanking sequences without the desired enzymatic RNA, and against the releasing first ribozyme; The hybridization can be accomplished in the presence of a chaotropic agent to prevent nuclease degradation. The oligonucleotides on the matrix can be modified to minimize nuclease activity, for example, by provision of 2'-O-methyl RNA oligonucleotides. Such modifications of the oligonucleotide attached to the column matrix will allow the multiple use of the column with minimal oligo degradation. Many such modifications are known in the art, but a chemically stable non-reducible modification is preferred. For example, phosphorothioate modifications can also be used.

The expressed ribozyme RNA can be isolated from bacterial or eucaryotic cells by routine procedures such as lysis followed by guanidine isothiocyanate isolation.

The current known self-cleaving site of *Tetrahymena* can be used in an alternative vector of this invention. If desired, the full-length *Tetrahymena* sequence may be used, or a shorter sequence may be used. It is preferred that, in order to decrease the superfluous sequences in the self-cleaving site at the 5' cleavage end, the hairpin normally present in the *Tetrahymena* ribozyme should contain the therapeutic second ribozyme 3' sequence and its complement. That is, the first releasing ribozyme-encoding DNA is provided in two portions, separated by DNA encoding the desired second ribozyme. For example, if the therapeutic second ribozyme recognition sequence is CGGACGA/CGAGGA, then CGAGGA is provided in the self-cleaving site loop such that it is in a stem structure recognized by the *Tetrahymena* ribozyme. The loop of the stem may include a restriction endonuclease site into which the desired second ribozyme-ericoding DNA is placed.

If desired, the vector may be used in a therapeutic protocol by use of the systems described by Lechner, PCT WO 92/13070, hereby incorporated by reference herein, to allow a timed expression of the therapeutic second ribozyme, as well as an appropriate shut off of cell or gene function. Thus, the vector will include a promoter which appropriately expresses enzymatically active RNA only in the presence of an RNA or another molecule which indicates the presence of an undesired organism or state. Such enzymatically active RNA will then kill or harm the cell in which it exists, as described by Lechner, id., or act to cause reduced expression of a desired protein product.

A number of suitable RNA vectors may also be used in this invention. The vectors include plant viroids, plant viruses which contain single or double-stranded RNA genomes and animal viruses which contain RNA genomes, such as the picornaviruses, myxoviruses, paramyxoviruses, hepatitis A virus, reovirus and retroviruses. In many instances cited, use of these viral vectors also results in tissue specific delivery of the ribozymes.

Example 21: Design of self-processing cassettes

In a preferred embodiment, applicant compared the *in vitro* and *in vivo* cis-cleaving activity of three different ribozyme motifs-the hammerhead, the hairpin and the hepatitis delta virus ribozyme-in order to assess their potential to process the ends of transcripts *in vivo*. To make a direct comparison among the three, however, it is important to design the ribozyme-containing transcripts to be as similar as possible. To this end, all the ribozyme cassettes contained the same trans-acting hammerhead ribozyme followed immediately by one of the three cis-acting ribozymes (Figure 23-25). For simplicity, applicant refers to each cassette by an abbreviation that indicates the downstream cis-cleaving ribozyme only. Thus HH refers to the cis-cleaving cassette containing a hammerhead ribozyme, while HP and HDV refer to the cassettes containing hairpin and hepatitis delta virus cis-cleaving ribozymes, respectively. The general design of the ribozyme cassettes, as well as specific differences among the cassettes, are outlined below.

A sequence predicted to form a stable stem-loop structure is included at the 5' end of all the transcripts. The hairpin stem contains the T7 RNA polymerase initiation sequence (Milligan & Uhlenbeck, 1989 Methods Enzymol. 180, 51) and its complement, separated be a stable tetra-loop (Antao et al., 1991 Nucleic Acids Res. 19, 5901). By incorporating the T7 initiation sequence into a stem-loop structure, applicant hoped to avoid nonproductive base pairing interactions with either the trans-acting ribozyme or with the cis-acting ribozyme. The presence of a hairpin at the end of a transcript may also contribute to the stability of the transcript *in vivo.* These are non-limiting examples. Those in the art will recognize that other embodiments can be readily generated using a variety of promoters, initiator sequences and stem-loop structure combinations generally known in the art.

The trans-acting ribozyme used in this study is targeted to a site B (5'...CUGGAGUC↓GACCUUC...3'). The 5' binding arm of the ribozyme, 5'-GAAGGUC-3', and' the core of the ribozyme, 5'-CUGAUGAGGCCGAAAGGCCGAA-3', remain constant in all cases. In addition, all transcripts also contain a single nucleotide between the 5' stem-loop and the first nucleotide of the ribozyme. The linker nucleotide was required to obtain the same activity *in vitro* that was measured with an identical ribozyme lacking the 5' hairpin. Because the three cis-cleaving ribozymes have different requirements at the site of cleavage, slight differences were unavoidable at the 3' end of the processed transcript. The junction between the trans- and cis-acting ribozyme is, however, designed so that there is minimal extraneous sequence left at the 3' end of the trans-cleaving ribozyme once cis-cleavage occurs. The only differences between the constructs lie in the 3' binding arm of the ribozyme, where either 6 or 7 nucleotides, 5'-ACUCCA(+/-G)-3', complementary to the target sequence are present and where, after processing, two to five extra nucleotides remain.

The cis-cleaving hammerhead ribozyme used in the HH cassette is based on the design of Grosshans and Cech, 1991 supra. As shown in Figure 23, the 3' binding arm of the trans-acting ribozyme is included in the required base-pairing interactions of the cis-cleaving ribozyme to form stem I. Two extra nucleotides, UC, were included at the end of the 3' binding arm to form the self-processing hammerhead ribozyme site (Ruffner et al., 1990 supra) which remain on the 3' end of the trans-acting ribozyme following self-processing.

The hairpin ribozyme portion of the HP self-processing construct is based on the minimal wild-type sequence (Hampel & Tritz, 1989 supra). A tetra-loop at the end of helix 1 (3' side of the cleavage site) serves to link the two portions and thus allows a minimal five nucleotides to remain at the end of the released trans-acting ribozyme following self-processing. Two variants of HP were designed: HP(GU) and HP(GC). The HP(GU) was constructed with a G·U

wobble base pair in helix 2 ($A_{52}G$ substitution; Figure 24). This slight destabilization of helix 2 was intended to improve self-processing activity by promoting product release and preventing the reverse reaction (Berzal-Herranz et al., 1992 Genes & Dev. 6, 129; Chowrira et al., 1993 Biochemistry 32, 1088). The HP(GC) cassette was constructed as a control for strong base-pairing interactions in helix 2 (U77C and $A_{52}G$ substitution; Figure 24). Another modification to discourage the reverse ligation reaction of the hairpin ribozyme was to shorten helix 1 (Figure 24) by one base pair relative to the wild-type sequence (Chowrira & Burke, 1991 Biochemistry 30, 8518).

The HDV ribozyme self-processes efficiently when the nucleotide 5' to the cleavage site is a pyrimidine, and somewhat less so when adenosine is in that position. No other sequence requirements have been identified upstream of the cleavage site, however, we have observed some decrease in activity when a stem-loop structure was present within 2 nt of the cleavage site. The HDV self-processing construct (Fig 25) was designed to generate the trans-acting hammerhead ribozyme with only two additional nucleotides at its 3' end after self-processing. The HDV sequence used here is based on the anti-genomic sequence (Perrota & Been, 1992 supra) but includes the modifications of Been et al., 1992 (Biochemistry 31, 11843) in which cis-cleavage activity of the ribozyme was improved by the substitution of a shortened helix 4 for a wild-type stem-loop (Figure 25).

To prepare DNA inserts that encode self-processing ribozyme cassettes, partially overlapping top- and bottom-strand oligonucleotides (60-90 nucleotides) were designed to include sequences for the T7 promoter, the trans-acting ribozyme, the cis-cleaving ribozyme and appropriate restriction sites for use in cloning (see Fig. 26). The single-strand portions of annealed oligonucleotides were converted to double-strands using Sequenase® (U.S. Biochemicals). Insert DNA was ligated into *EcoR1*/*HindIII*-digested puc18 and transformed into *E. coli* strain DH5α using standard protocols (Maniatis et al., 1982 in Molecular Cloning Cold Spring Harbor Press). The identity of positive clones was confirmed by sequencing small-scale plasmid preparations.

Larger scale preparations of plasmid DNA for use as *in vitro* transcription templates and in transactions were prepared using the protocol and columns from QIAGEN Inc. (Studio City, CA) except that an additional ethanol precipitation was included as the final step.

Example 22: RNA Processing *in vitro*

Transcription reactions containing linear plasmid templates were carried out essentially as described (Milligan & Uhlenbeck, 1989 Supra; Chowrira & Burke, 1991 Supra). In order to prepare 5' end-labeled transcripts, standard transcription reactions were carried out in the presence of 10-20 μCi [$\gamma$-$^{32}$P]GTP, 200 μM each NTP and 0.5 to 1 μg of linearized plasmid template. The concentration of $MgCl_2$ was maintained at 10 mM above the total nucleotide concentration.

To compare the ability of the different ribozyme cassettes to self-process *in vitro*, each construct was transcribed and allowed to undergo self-processing under identical conditions at 37°C. For these comparisons, equal amounts of linearized DNA templates bearing the various ribozyme cassettes were transcribed in the presence of [$\gamma$-$^{32}$P]GTP to generate 5' end-labeled transcripts. In this manner only the full-length, unprocessed transcripts and the released trans-ribozymes are visualized by autoradiography. In all reactions, $Mg^{2+}$ was included at 10 mM above the nucleotide concentration so that cleavage by all the ribozyme cassettes would be supported. Transcription templates were linearized at several positions by digestion with different restriction enzymes so that self-processing in the presence of increasing lengths of downstream sequence could be compared (see Fig. 26). The resulting transcripts have either 4-5 non-ribozyme nucleotides at the 3' end (*Hind*III-digested template), 220 nucleotides *(NdeI* digested templates) or 454 nucleotides of downstream sequence *(RcaI* digested template).

As shown in Figure 27, all four ribozyme cassettes are capable of self-processing and yield RNA products of expected sizes. Two nucleotides essential for hammerhead ribozyme activity (Ruffner et al., 1990 supra) have been changed in the HH(mutant) core sequence (see Figure 23) and so this transcript is unable to undergo self-processing (Fig. 27). This is evidenced by the lack of a released 5' RNA in the HH(mutant), although the full-length RNAs are present . Comparison of the amounts of released trans-ribozyme (Fig. 27) indicate that there are differences in the ability of these ribozymes to self-process *in vitro*, especially with respect to the presence of downstream sequence. For the two HP constructs, it is clear that HP(GC) is more efficient than the HP(GU) ribozyme, both in the presence and in the absence of extra downstream sequence. In addition, the activity of HP(GU) falls off more dramatically when downstream sequence is present. The stronger G:C base pair likely contributes to the HP(GC) construct's ability to fold correctly (and/or more quickly) into the productive structure, even when as much as 216 extra nucleotides are present downstream. The HH ribozyme construct is also quite efficient at self-processing, and slightly better than the HP(GU) construct even when downstream sequence is present.

Of the three ribozyme motifs, the presence of extra downstream sequence seems to most affect the efficiency of HDV. When no extra sequence is present downstream, HDV is quite efficient and self-processes to approximately the same level as the HH and HP(GC) cassettes. However, when extra downstream sequence is present, the self-processing activity seems to decrease almost as dramatically as is seen with the (sub-optimal) HP(GU) cassette.

Example 23: Kinetics of self-processing reaction *Hind*III-digested template (250 ng) was used in a standard transcription reaction mixture containing: 50 mM Tris·HCl pH 8.3; 1 mM ATP, GTP and UTP; 50 µM CTP; 40 µCi [$\alpha$-$^{32}$P]CTP; 12 mM $MgCl_2$; 10 mM DTT. The transcription/self-processing reaction was initiated by the addition of T7 RNA polymerase (15 U/µl). Aliquots of 5 µl were taken at regular time intervals and the reaction was stopped by adding an equal volume of 2x formamide loading buffer (95% formamide, 15 mM EDTA, & dyes) and freezing on dry ice. The samples were resolved on a 10% polyacrylamide sequencing gel and results were quantitated by PhosphorImager (Molecular Dynamics, Sunnyvale, CA). Ribozyme self-cleavage rates were determined from non-linear, least-squares fits (KaleidaGraph, Synergy Software,Reeding, PA) of the data to the equation:

$$(\text{Fraction Uncleaved Transcript}) = \frac{1}{kt}\,(1\text{-}e^{-kt})$$

where t represents time and k represents the unimolecular rate constant for cleavage (Long & Uhlenbeck, 1994 Proc. Natl. Acad. Sci. USA 91, 6977).

Linear templates were prepared by digesting the plasmids with *Hind*III so that transcripts will contain only four to five vector-derived nucleotides at the 3' end (see Figure 23-25). By comparison of the unimolecular rate constant (k) determined for each construct, it is clear that HH is the most efficient at self-processing (Table 44). The HH transcript self-processes 2-fold faster than HDV and 3-fold faster than HP(GC) transcripts. Although the HP(GU) RNA undergoes self-processing, it is at least 6-fold slower than the HP(GC) construct. This is consistent with previous observations that the stability of helix 2 is essential for self-processing and trans-cleavage activity of the hairpin ribozyme (Hampel et al., 1990 supra; Chowrira & Burke, 1991 supra). The rate of HH self-cleavage during transcription measured here (1.2 $min^{-1}$) is similar to the rate measured by Long and Uhlenbeck 1994 supra using a HH that has a different stem I and stem III. Self-processing rates during transcription for HP and HDV have not been previously reported. However, self-processing of the HDV ribozyme-as measured here during transcription-is significantly slower than when tested after isolation from a denaturing gel (Been et al., 1992 supra). This decrease likely reflects the difference in protocol as well as the presence of 5' flanking sequence in the HDV construct used here.

Example 24: Effect of downstream sequences on trans-cleavage *in vitro*

Transcripts containing the trans ribozyme with or without 3' flanking sequences were assayed for their ability to cleave their target in trans. To this end, transcripts from three templates were resolved on a preparative gel and bands corresponding both to processed trans-acting ribozymes from the HH transcription reaction, and to full-length HH(mutant) and ΔHDV transcripts were isolated. In all three transcripts the trans-acting ribozyme portion is identical-with the exception of sequences at their 3' ends. The HH trans-acting ribozyme contains only an additional UC at its 3' end, while HH(mutant) and ΔHDV have 52 and 37 nucleotides, respectively, at their 3' ends. A 622 nucleotide, internally-labeled target RNA was incubated, under ribozyme excess conditions, along with the three ribozyme transcripts in a standard reaction buffer.

To make internally-labeled substrate RNA for trans-ribozyme cleavage reactions, a 622 nt region (containing hammerhead site P) was synthesized by PCR using primers that place the T7 RNA promoter upstream of the amplified sequence. Target RNA was transcribed in a standard transcription buffer in the presence of [$\alpha$-$^{32}$P]CTP (Chowrira & Burke, 1991 supra). The reaction mixture was treated with 15 units of ribonuclease-free DNaseI, extracted with phenol followed chloroform:isoamyl alcohol (25:1), precipitated with isopropanol and washed with 70% ethanol. The dried pellet was resuspended in 20 µl DEPC-treated water and stored at -20°C.

Unlabeled ribozyme (1µM) and internally labeled 622 nt substrate RNA (<10 nM) were denatured and renatured separately in a standard cleavage buffer (containing 50 mM Tris·HCl pH 7.5 and 10 mM $MgCl_2$) by heating to 90°C for 2 min. and slow cooling to 37°C for 10 min. The reaction was initiated by mixing the ribozyme and substrate mixtures and incubating at 37°C. Aliquots of 5 µl were taken at regular time intervals, quenched by adding an equal volume of 2X formamide gel loading buffer and frozen on dry ice. The samples were resolved on 5% polyacrylamide sequencing gel and results were quantitatively analyzed by radioanalytic imaging of gels with a PhosphorImager® (Molecular Dynamics, Sunnyvale, CA).

The HH trans-acting ribozyme cleaves the target RNA approximately 10-fold faster than the ΔHDV transcript and greater than 20-fold faster than the HH(mutant) transcript (Figure 28). The additional nucleotides at the end of HH (mutant) form 7 base-pairs with the 3' target-binding arm of the trans-acting ribozyme (Figure 23). This interaction must be disrupted (at a cost of 6 kcal/mole) to make the trans-acting ribozyme available for binding the target sequence. In contrast, the additional nucleotides at the end of ΔHDV were not designed to form any strong, alternative base-pairing with the trans-ribozyme. Nevertheless, the ΔHDV sequences are predicted to form multiple structures involving the 3' target-binding arm of the trans ribozyme that have stabilities ranging from 1-2 kcal/mole. Thus, the observed reductions in activity for the ΔHDV and HH(mutant) constructs are consistent with the predicted folded structures, and it reinforces

the view that the flanking sequences can decrease the catalytic efficiency of a ribozyme through nonproductive interactions with either the ribozyme or the substrate or both.

Example 25: RNA self-processing *in vivo*

Since three of the constructs (HH, HDV and HP(GC)) self-process efficiently in solution, the affect of the mammalian cellular milieu on ribozyme self-processing was next explored by applicant. A transient expression system was employed to investigate ribozyme activity *in vivo. A* mouse cell line (OST7-1) that constitutively expresses T7 RNA polymerase in the cytoplasm was chosen for this study (Elroy-Stein and Moss, 1990 Proc. Natl. Acad. Sci. USA 87, 6743). In these cells plasmids containing a ribozyme cassette downstream of the T7 promoter will be transcribed efficiently in the cytoplasm (Elroy-Stein & Moss, 1990 supra).

Monolayers of a mouse L9 fibroblast cell line (OST7-1; Elroy-Stein and Moss, 1990 supra) were grown in 6-well plates with ~ $5x10^5$ cells/well. Cells were transfected with circular plasmids (5 µg/well) using the calcium phosphate-DNA precipitation method (Maniatis et al., 1982 supra). Cells were lysed (4 hours post-transfection) by the addition of standard lysis buffer (200 µl/well) containing 4M guanadinium isothiocyanate, 25 mM sodium citrate (pH 7.0), 0.5% sarkosyl (Chomczynski and Sacchi, 1987 Anal. Biochem. 162, 156), and 50 mM EDTA pH 8.0. The lysate was extracted once with water-saturated phenol followed by one extraction with chloroform:isoamyl alcohol (25:1). Total cellular RNA was precipitated with an equal volume of isopropanol. The RNA pellet was resuspended in 0.2 M ammonium acetate and reprecipitated with ethanol. The pellet was then washed with 70% ethanol and resuspended in DEPC-treated water.

Purified cellular RNA (3 µg/reaction) was first denatured in the presence of a 5' end-labeled DNA primer (100 pmol) by heating to 90°C for 2 min. in the absence of $Mg^{2+}$, and then snap-cooling on ice for at least 15 min. This protocol allows for efficient annealing of the primer to its complementary RNA sequence. The primer was extended using Superscript II reverse transcriptase (8 U/µl; BRL) in a buffer containing 50 mM Tris·HCl pH 8.3; 10 mM DTT; 75 mM KCl; 1 mM $MgCl_2$; 1 mM each dNTP. The extension reaction was carried out at 42°C for 10 min. The reaction was terminated by adding an equal volume of 2x formamide gel loading buffer and freezing on crushed dry ice. The samples were resolved on a 10% polyacrylamide sequencing gel. The primer sequences are as follows: HH primer, 5'-CTCCAGTTTCGAGCTTT-3'; HDV primer, 5'-AAGTAGCCCAGGTCGGACC-3'; HP primer, 5'-ACCAGGTAATATACCACAAC-3'.

As shown in Figure 29, specific bands corresponding to full-length precursor RNA and 3' cleavage products were detected from cells transfected with the self-processing cassettes. All three constructs, in addition to being transcriptionally active, appear to self-process efficiently in the cytoplasm of OST7-1 cells. In particular, the HH and HP (GC) constructs self-process to greater than 95%. The overall extent of self-processing in OST7-1 cells appears to be strikingly similar to the extent of self-processing *in vitro* (Figure 29 "In Vitro +$MgCl_2$" vs. "Cellular").

Consistent with the *in vitro* self-processing results, the HP(GU) cassette self-processed to approximately 50% in OST7-1 cells. As expected, transfection with plasmids containing the HH(mutant) cassette yielded a primer-extension product corresponding to the full-length RNA with no detectable cleavage products (Figure 29). The latter result strongly suggests that the primer extension band corresponding to the 3' cleavage product is not an artifact of reverse transcription.

Applicant was concerned with the possibility that RNA self-processing might occur during cell lysis, RNA isolation and /or the primer extension assay. Two precautions were taken to exclude this possibility. First, 50 mM EDTA was included in the lysis buffer. EDTA is a strong chelator of divalent metal ions such as $Mg^{2+}$ and $Ca^{2+}$ that are necessary for ribozyme activity. Divalent metal ions are therefore unavailable to self-processing RNAs following cell lysis. A second precaution involved using primers in the primer-extension assay that were designed to hybridize to essential regions of the processing ribozyme. Binding of these primers should prevent the 3' cis-acting ribozymes from folding into the conformation essential for catalytic activity.

Two experiments were carried out to further eliminate the possibility that self-processing is occurring either during RNA preparations or during the primer extension analysis. The first experiment involves primer extension analysis on full-length precursor RNAs that were added to non-transfected OST7-1 lysates after cell lysis. Thus, only if self-processing is occurring at some point after lysis would cleavage products be detected. Full-length precursor RNAs were prepared by transcribing under conditions of low $Mg^{2+}$ (5 mM) and high NTP concentration (total 12 mM) in an attempt to eliminate the free $Mg^{2+}$ required for the self-processing reaction (Michel et al. 1992 Genes & Dev. 6, 1373). The full-length precursor RNAs were gel-purified, and a known amount was added to lysates of non-transfected OST7-1 cells. RNA was purified from these lysates and incubated for 1 hr in DEPC-treated water at 37° C prior to the standard primer extension analysis (Figure 29, *in vitro* "-$MgCl_2$" control). The predominant RNA detected in all cases corresponds to the primer extension product of full-length precursor RNAs. If, instead, the purified RNA containing the full-length precursor is incubated in 10 mM $MgCl_2$ prior to the primer extension analysis, most or all of the RNA detected by primer extension analysis undergoes cleavage (Figure 29, *in vitro* "+$MgCl_2$" control). These results indicate that the standard

RNA isolation and primer extension protocols used here do not provide a favorable environment for RNA self-processing, even though the RNA in question is inherently able to undergo self-cleavage.

In a second experiment to demonstrate lack of self-processing during work up, internally-labeled precursor RNAs were prepared and added to non-transfected OST7-1 lysates as in the previous control. The internally-labeled precursor RNAs were carried through the RNA purification and primer extension reactions (in the presence of unlabeled primers) and analyzed to determine the extent of self-processing. By this analysis, the vast majority of the added full-length RNA remained intact during the entire process of RNA isolation and primer extension.

These two control experiments validate the protocols used and support applicant's conclusion that the self-processing reactions catalyzed by HH, HDV and HP(GC) cassettes are occurring in the cytoplasm of OST7-1 cells.

Sequences in figures 23 through 25 are meant to be non-limiting examples. Those in the art will recognize that other embodiments can be readily generated using techniques generally known in the art..

In addition, those in the art will recognize that Applicant provides guidance through the above examples as to how to best design vectors of this invention so that secondary structure of the mRNA allows efficient cleavage by releasing ribozymes. Thus, the specific constructs are not limiting in this invention. Such constructs can be readily tested as described above for such secondary structure, either by computer folding algorithms or empirically. Such constructs will then allow at least 80% completion of release of ribozymes, which can be readily determined as described above or by methods known in the art. That is, any such secondary structure in the RNA does not reduce release of the ribozymes by more than 20%.

## IV. Ribozymes Expressed by RNA Polymerase III

Applicant has determined that the level of production of a foreign RNA, using a RNA polymerase III (pol III) based system, can be significantly enhanced by ensuring that the RNA is produced with the 5' terminus and a 3' region of the RNA molecule base-paired together to form a stable intramolecular stem structure. This stem structure is formed by hydrogen bond interactions (either Watson-Crick or non-Watson-Crick) between nucleotides in the 3' region (at least 8 bases) and complementary nucleotides in the 5' terminus of the same RNA molecule.

Although the example provided below involves a type 2 pol III gene unit, a number of other pof III promoter systems can also be used, for example, tRNA (Hall et al., 1982 *Cell 29,* 3-5), 5S RNA (Nielsen et al., 1993, *Nucleic Acids Res.* 21, 3631-3636), adenovirus VA RNA (Fowlkes and Shenk, 1980 *Cell* 22, 405-413), U6 snRNA (Gupta and Reddy, 1990 *Nucleic Acids Res.* 19, 2073-2075), vault RNA (Kickoefer et al., 1993 *J. Biol. Chem.* 268, 7868-7873), telomerase RNA (Romero and Blackburn, 1991 *Cell* 67, 343-353), and others.

The construct described in this invention is able to accumulate RNA to a significantly higher level than other constructs, even those in which 5' and 3' ends are involved in hairpin loops. Using such a construct the level of expression of a foreign RNA can be increased to between 20,000 and 50,000 copies per cell. This makes such constructs, and the vectors encoding such constructs, excellent for use in decoy, therapeutic editing and antisense protocols as well as for ribozyme formation. In addition, the molecules can be used as agonist or antagonist RNAs (affinity RNAs). Generally, applicant believes that the intramolecular base-paired interaction between the 5' terminus and the 3' region of the RNA should be in a double-stranded structure in order to achieve enhanced RNA accumulation.

Thus, in one preferred embodiment the invention features a pol III promoter system (e.g., a type 2 system) used to synthesize a chimeric RNA molecule which includes tRNA sequences and a desired RNA (e.g., a tRNA-based molecule).

The following exemplifies this invention with a type 2 pol III promoter and a tRNA gene. Specifically to illustrate the broad invention, the RNA molecule in the following example has an A box and a B box of the type 2 pol III promoter system and has a 5' terminus or region able to base-pair with at least 8 bases of a complementary 3' end or region of the same RNA molecule. This is meant to be a specific example. Those in the art will recognize that this is but one example, and other embodiments can be readily generated using other pol III promoter systems and techniques generally known in the art.

By "terminus" is meant the terminal bases of an RNA molecule, ending in a 3' hydroxyl or 5' phosphate or 5' cap moiety. By "region" is meant a stretch of bases 5' or 3' from the terminus that are involved in base-paired interactions. It need not be adjacent to the end of the RNA. Applicant has determined that base pairing of at least one end of the RNA molecule with a region not more than about 50 bases, and preferably only 20 bases, from the other end of the molecule provides a useful molecule able to be expressed at high levels.

By "3' region" is meant a stretch of bases 3' from the terminus that are involved in intramolecular bas-paired interaction with complementary nucleotides in the 5' terminus of the same molecule. The 3' region can be designed to include the 3' terminus. The 3' region therefore is $\geq 0$ nucleotides from the 3' terminus. For example, in the S35 construct described in the present invention (Fig. 40) the 3' region is one nucleotide from the 3' terminus. In another example, the 3' region is ~ 43 nt from 3' terminus. These examples are not meant to be limiting. Those in the art will recognize that other embodiments can be readily generated using techniques generally known in the art. Generally, it is preferred

to have the 3' region within 100 bases of the 3' terminus.

By "tRNA molecule" is meant a type 2 pol III driven RNA molecule that is generally derived from any recognized tRNA gene. Those in the art will recognize that DNA encoding such molecules is readily available and can be modified as desired to alter one or more bases within the DNA encoding the RNA molecule and/or the promoter system. Generally, but not always, such molecules include an A box and a B box that consist of sequences which are well known in the art (and examples of which can be found throughout the literature). These A and B boxes have a certain consensus sequence which is essential for a optimal pol III transcription.

By "chimeric tRNA molecule" is meant a RNA molecule that includes a pol III promoter (type 2) region. A chimeric tRNA molecule, for example, might contain an intramolecular base-paired structure between the 3' region and complementary 5' terminus of the molecule, and includes a foreign RNA sequence at any location within the molecule which does not affect the activity of the type 2 pol III promoter boxes. Thus, such a foreign RNA may be provided at the 3' end of the B box, or may be provided in between the A and the B box, with the B box moved to an appropriate location either within the foreign RNA or another location such that it is effective to provide pol III transcription. In one example, the RNA molecule may include a hammerhead ribozyme with the B box of a type 2 pol III promoter provided in stem II of the ribozyme. In a second example, the B box may be provided in stem IV region of a hairpin ribozyme. A specific example of such RNA molecules is provided below. Those in the art will recognize that this is but one example, and other embodiments can be readily generated using techniques generally known in the art.

By "desired RNA" molecule is meant any foreign RNA molecule which is useful from a therapeutic, diagnostic, or other viewpoint. Such molecules include antisense RNA molecules, decoy RNA molecules, enzymatic RNA, therapeutic editing RNA and agonist and antagonist RNA.

By "antisense RNA" is meant a non-enzymatic RNA molecule that binds to another RNA (target RNA) by means of RNA-RNA interactions and alters the activity of the target RNA (Eguchi et al., 1991 Annu. Rev. Biochem. 60, 631-652). By "enzymatic RNA" is meant an RNA molecule with enzymatic activity (Cech, 1988 J.*American. Med. Assoc.* 260, 3030-3035). Enzymatic nucleic acids (ribozymes) act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through base-pairing, and once bound to the correct site, acts enzymatically to cut the target RNA.

By "decoy RNA" is meant an RNA molecule that mimics the natural binding domain for a ligand. The decoy RNA therefore competes with natural binding target for the binding of a specific ligand. For example, it has been shown that over-expression of HIV trans-activation response (TAR) RNA can act as a "decoy" and efficiently binds HIV *tat* protein, thereby preventing it from binding to TAR sequences encoded in the HIV RNA (Sullenger et al., 1990 Cell 63, 601-608). This is meant to be a specific example. Those in the art will recognize that this is but one example, and other embodiments can be readily generated using techniques generally known in the art.

By "therapeutic editing RNA" is meant an antisense RNA that can bind to its cellular target (RNA or DNA) and mediate the modification of a specific base.

By "agonist RNA" is meant an RNA molecule that can bind to protein receptors with high affinity and cause the stimulation of specific cellular pathways.

By "antagonist RNA" is meant an RNA molecule that can bind to cellular proteins and prevent it from performing its normal biological function (for example, see Tsai et al., 1992 *Proc. Natl. Acad. Sci.* USA 89, 8864-8868).

In other aspects, the invention includes vectors encoding RNA molecules as described above, cells including such vectors, methods for producing the desired RNA, and use of the vectors and cells to produce this RNA.

Thus, the invention features a transcribed non-naturally occuring RNA molecule which includes a desired therapeutic RNA portion and an intramolecular stem formed by base-pairing interactions between a 3' region and complementary nucleotides at the 5' terminus in the RNA. The stem preferably includes at least 8 base pairs, but may have more, for example, 15 or 16 base pairs.

In preferred embodiments, the 5' terminus of the chimeric tRNA includes a portion of the precursor molecule of the primary tRNA molecule, of which ≥ 8 nucleotides are involved in base-pairing interaction with the 3' region; the chimeric tRNA contains A and B boxes; natural sequences 3' of the B box are deleted, which prevents endogenous RNA processing; the desired RNA molecule is at the 3' end of the B box; the desired RNA molecule is between the A and the B box; the desired RNA molecule includes the B box; the desired RNA molecule is selected from the group consisting of antisense RNA, decoy RNA, therapeutic editing RNA, enzymatic RNA, agonist RNA and antagonist RNA; the molecule has an intramolecular stem resulting from a base-paired interaction between the 5' terminus of the RNA and a complementary 3' region within the same RNA, and includes at least 8 bases; and the 5' terminus is able to base pair with at least 15 bases of the 3' region.

!n most preferred embodiments, the molecule is transcribed by a RNA polymerase III based promoter system, *e.g.*, a type 2 pol III promoter system; the molecule is a chimeric tRNA, and may have the A and B boxes of a type 2 pol III promoter separated by between 0 and 300 bases; DNA vector encoding the RNA molecule of claim 51.

In other related aspects, the invention features an RNA or DNA vector encoding the above RNA molecule,

with the portions of the vector encoding the RNA functioning as a RNA pol III promoter; or a cell containing the vector ; or a method to provide a desired RNA molecule in a cell, by introducing the molecule into a cell with an RNA molecule as described above. The cells can be derived from animals, plants or human beings.

In order for RNA-based gene therapy approaches to be effective, sufficient amounts of the therapeutic RNA must accumulate in the appropriate intracellular compartment of the treated cells. Accumulation is a function of both promoter strength of the antiviral gene, and the intracellular stability of the antiviral RNA. Both RNA polymerase II (pol II) and RNA polymerase III (pol III) based expression systems have been used to produce therapeutic RNAs in cells (Sarver & Rossi, 1993 *AIDS Res. & Human Retroviruses* 9, 483-487; Yu et al., 1993 *P.N.A.S.*(USA) 90, 6340-6344). However, pol III based expression cassettes are theoretically more attractive for use in expressing antiviral RNAs for the following reasons. Pol II produces messenger RNAs located exclusively in the cytoplasm, whereas pol III produces functional RNAs found in both the nucleus and the cytoplasm. Pol II promoters tend to be more tissue restricted, whereas pol III genes encode tRNAs and other functional RNAs necessary for basic "housekeeping" functions in all cell types. Therefore, pol III promoters are likely to be expressed in all tissue types. Finally, pol III transcripts from a given gene accumulate to much greater levels in cells relative to pol II genes.

Intracellular accumulation of therapeutic RNAs is also dependent on the method of gene transfer used. For example, the retroviral vectors presently used to accomplish stable gene transfer, integrate randomly into the genome of target cells. This random integration leads to varied expression of the transferred gene in individual cells comprising the bulk treated cell population. Therefore, for maximum effectiveness, the transferred gene must have the capacity to express therapeutic amounts of the antiviral RNA in the entire treated cell population, regardless of the integration site.

Pol III System

The following is just one non-limiting example of the invention. A pol III based genetic element derived from a human $tRNA_i^{met}$ gene and termed Δ3-5 (Fig. 33; Adeniyi-Jones et al., 1984 *supra*), has been adapted to express antiviral RNAs (Sullenger et al., 1990 *Mol. Cell. Biol*. 10, 6512-6523). This element was inserted into the DC retroviral vector (Sullenger et al., 1990 *Mol. Cell. Biol*. 10, 6512-6523) to accomplish stable gene transfer, and used to express antisense RNAs against moloney murine leukemia virus and anti-HIV decoy RNAs (Sullenger et al., 1990 *Mol. Cell. Biol.* 10, 6512-6523; Sullenger et al., 1990 *Cell 63,* 601-608; Sullenger et al., 1991 *J. Virol.* 65, 6811-6816; Lee et al., 1992 *The New Biologist* 4, 66-74). Clonal lines are expanded from individual cells present in the bulk population, and therefore express similar amounts of the therapeutic RNA in all cells. Development of a vector system that generates therapeutic levels of therapeutic RNA in all treated cells would represent a significant advancement in RNA based gene therapy modalities.

Applicant examined hammerhead (HHI) ribozyme (RNA with enzymatic activity) expression in human T cell lines using the Δ3-5 vector system (These constructs are termed "Δ3-5/HHI"; Fig. 34). On average, ribozymes were found to accumulate to less than 100 copies per cell in the bulk T cell populations. In an attempt to improve expression levels of the Δ3-5 chimera, the applicant made a series of modified Δ3-5 gene units containing enhanced promoter elements to increase transcription rates, and inserted structural elements to improve the intracellular stability of the ribozyme transcripts (Fig. 34). One of these modified gene units, termed S35, gave rise to more than a 100-fold increase in ribozyme accumulation in bulk T cell populations relative to the original Δ3-5/HHI vector system. Ribozyme accumulation in individual clonal lines from the pooled T cell populations ranged from 10 to greater than 100 fold more than those achieved with the original Δ3-5/HHI version of this vector. The S35 gene unit may be used to express other therapeutic RNAs including, but not limited to, ribozymes, antisense, decoy, therapeutic editing, agonist and antagonist RNAs. Application of the S35 gene unit would not be limited to antiviral therapies, but also to other diseases, such as cancer, in which therapeutic RNAs may be effective. The S35 gene unit may be used in the context of other vector systems besides retroviral vectors, including but not limited to, other stable gene transfer systems such as adeno-associated virus (AAV; Carter, 1992 *Curr. Opin. Genet. Dev.* 3, 74), as well as transient vector systems such as plasmid delivery and adenoviral vectors (Berkner, 1988 *BioTechniques 6,* 616-629).

As described below, the S35 vector encodes a truncated version of a tRNA wherein the 3' region of the RNA is base-paired to complementary nucleotides at the 5' terminus, which includes the 5' precursor portion that is normally processed off during tRNA maturation. Without being bound by any theory, Applicant believes this feature is important in the level of expression observed. Thus, those in the art can now design equivalent RNA molecules with such high expression levels. Below are provided examples of the methodology by which such vectors and tRNA molecules can be made.

Δ3-5 Vectors

The use of a truncated human $tRNA_i$met gene, termed Δ3-5 (Fig. 33; Adeniyi-Jones et al., 1984 *supra),* to

drive expression of antisense RNAs, and subsequently decoy RNAs (Sullenger et al., 1990 *supra)* has recently been reported. Because tRNA genes utilize internal pol III promoters, the antisense and decoy RNA sequences were expressed as chimeras containing $tRNA_i^{met}$ sequences. The truncated tRNA genes were placed into the U3 region of the 3' moloney murine leukemia virus vector LTR (Sullenger et al., 1990 *supra).*

Base-Paired Structures

Since the Δ3-5 vector combination has been successfully used to express inhibitory levels of both antisense and decoy RNAs, applicant cloned ribozyme-encoding sequences (termed as "Δ3-5/HHI") into this vector to explore its utility for expressing therapeutic ribozymes. However, low ribozyme accumulation in human T cell lines stably transduced with this vector was observed (Fig. 35). To try and improve accumulation of the ribozyme, applicant incorporated various RNA structural elements (Fig. 34) into one of the ribozyme chimeras (Δ3-5/HHI).

Two strategies were used to try and protect the termini of the chimeric transcripts from exonucleolytic degredation. One strategy involved the incorporation of stem-loop structures into the termini of the transcript. Two such constructs were cloned, S3 which contains a stem-loop structure at the 3' end, and S5 which contains stem-loop structures at both ends of the transcript (Figure 34). The second strategy involved modification of the 3' terminal sequences such that the 5' terminus and the 3' end sequences can form a stable base-paired stem. Two such constructs were made: S35 in which the 3' end was altered to hybridize to the 5' leader and acceptor stem of the $tRNA_i^{met}$ domain, and S35Plus which was identical to S35 but included more extensive structure formation within the non-ribozyme portion of the Δ3-5 chimeras (Figure 34). These stem-loop structures are also intended to sequester non-ribozyme sequences in structures that will prevent them from interfering with the catalytic activity of the ribozyme. These constructs were cloned, producer cell lines were generated, and stably-transduced human MT2 (Harada et al., 1985 *supra*) and CEM (Nara & Fischinger, 1988 *supra)* cell lines were established *(Curr. Protocols Mol. Biol*. 1992, ed. Ausubel et al., Wiley & Sons, NY). The RNA sequences and structure of S35 and S35 Plus are provided in Figures 40-47.

Referring to Figure 48, there is provided a general structure for a chimeric RNA molecule of this invention. Each N independently represents none or a number of bases which may or may not be base paired. The A and B boxes are optional and can be any known A or B box, or a consensus sequence as exemplified in the figure. The desired nucleic acid to be expressed can be any location in the molecule, but preferably is on those places shown adjacent to or between the A and B boxes (designated by arrows). Figure 49 shows one example of such a structure in which a desired RNA is provided 3' of the intramolecular stem. A specific example of such a construct is provided in Figures 50 and 51.

Example 26: Cloning of Δ3-5-Ribozyme Chimera

Oligonucleotides encoding the S35 insert that overlap by at least 15 nucleotides were designed (5' GATCCACTCTGCTGTTCTGTTTTTGA 3' and 5' CGCGTCAAAAACAGAACAGCAGAGTG 3'). The oligonucleotides (10 μM each) were denatured by boiling for 5 min in a buffer containing 40 mM Tris.HCl, pH8.0. The oligonucleotides were allowed to anneal by snap cooling on ice for 10-15 min.

The annealed oligonucleotide mixture was converted into a double-stranded molecule using Sequenase® enzyme (US Biochemicals) in a buffer containing 40 mM Tris.HCl, pH7.5, 20 mM $MgCl_2$, 50 mM NaCl, 0.5 mM each of the four deoxyribonucleotide triphosphates, 10 mM DTT. The reaction was allowed to proceed at 37°C for 30 min. The reaction was stopped by heating to 70°C for 15 min.

The double stranded DNA was digested with appropriate restriction endonucleases (*Bam*HI and *Mlu*I) to generate ends that were suitable for cloning into the Δ3-5 vector.

The double-stranded insert DNA was ligated to the Δ3-5 vector DNA by incubating at room temperature (about 20°C) for 60 min in a buffer containing 66 mM Tris.HCl, pH 7.6, 6.6 mM $MgCl_2$, 10 mM DTT, 0.066 μM ATP and 0.1U/μl T4 DNA Ligase (US Biochemicals).

Competent *E. coli* bacterial strain was transformed with the recombinant vector DNA by mixing the cells and DNA on ice for 60 min. The mixture was heat-shocked by heating to 37°C for 1 min. The reaction mixture was diluted with LB media and the cells were allowed to recover for 60 min at 37°C. The cells were plated on LB agar plates and incubated at 37°C for ~ 18 h.

Plasmid DNA was isolated from an overnight culture of recombinant clones using standard protocols (Ausubel et al., *Curr. Protocols Mol. Biology* 1990, Wiley & Sons, NY).

The identity of the clones were determined by sequencing the plasmid DNA using the Sequenase® DNA sequencing kit (US Biochemicals).

The resulting recombinant Δ3-5 vector contains the S35 sequence. The HHI encoding DNA was cloned into this Δ3-5-S35 containing vector using *Sac*II and *Bam*HI restriction sites.

Example 27: Northern analysis

RNA from the transduced MT2 cells were extracted and the presence of Δ3-5/ribozyme chimeric transcripts were assayed by Northern analysis (*Curr. Protocols Mol. Biol.* 1992, ed. Ausubel et al., Wiley & Sons, NY). Northern analysis of RNA extracted from MT2 transductants showed that Δ3-5/ribozyme chimeras of appropriate sizes were expressed (Fig. 35,36). In addition, these results demonstrated the relative differences in accumulation among the different constructs (Figure 35,36). The pattern of expression seen from the Δ3-5/HHI ribozyme chimera was similar to 12 other ribozymes cloned into the Δ3-5 vector (not shown). In MT-2 cell line, Δ3-5/HHI ribozyme chimeras accumulated, on average, to less than 100 copies per cell.

Addition of a stem-loop onto the 3' end of Δ3-5/HHI did not lead to increased Δ3-5 levels (S3 in Fig. 35,36). The S5 construct containing both 5' and 3' stem-loop structures also did not lead to increased ribozyme levels (Fig. 35,36).

Interestingly, the S35 construct expression in MT2 cells was about 100-fold more abundant relative to the original Δ3-5/HHI vector transcripts (Fig. 35.36). This may be due to increased stability of the S35 transcript.

Example 28: Cleavage activity

To assay whether ribozymes transcribed in the transduced cells contained cleavage activity, total RNA extracted from the transduced MT2 T cells were incubated with a labeled substrate containing the HHI cleavage site (Figure 37). Ribozyme activity in all but the S35 constructs, was too low to detect. However, ribozyme activity was detectable in S35-transduced T cell RNA. Comparison of the activity observed in the S35-transduced MT2 RNA with that seen with MT2 RNA in which varying amounts of in vitro transcribed S5 ribozyme chimeras, indicated that between 1-3 nM of S35 ribozyme was present in S35-transduced MT2 RNA. This level of activity corresponds to an intracellular concentration of 5,000-15,000 ribozyme molecules per cell.

Example 29: Clonal variation

Variation in the ribozyme expression levels among cells making up the bulk population was determined by generating several clonal cell lines from the bulk S35 transduced CEM line (*Curr. Protocols Mol. Biol.* 1992, ed. Ausubel et al., Wiley & Sons, NY) and the ribozyme expression and activity levels in the individual clones were measured (Figure 38 and 39). All the individual clones were found to express active ribozyme. The ribozyme activity detected from each clone correlated well with the relative amounts of ribozyme observed by Northern analysis. Steady state ribozyme levels among the clones ranged from approximately 1,000 molecules per cell in clone G to 11,000 molecules per cell in clone H (Fig. 38). The mean accumulation among the clones, calculated by averaging the ribozyme levels of the clones, exactly equaled the level measured in the parent bulk population. This suggests that the individual clones are representative of the variation present in the bulk population.

The fact that all 14 clones were found to express ribozyme indicate that the percentage of cells in the bulk population expressing ribozyme is also very high. In addition, the lowest level of expression in the clones was still more than 10-fold that seen in bulk cells transduced with the original Δ3-5 vector. Therefore, the S35 gene unit should be much more effective in a gene therapy setting in which bulk cells are removed, transduced and then reintroduced back into a patient.

Example 30: Stability

Finally, the bulk S35-transduced line, resistant to G418, was propogated for a period of 3 months (in the absence of G418) to determine if ribozyme expression was stable over extended periods of time. This situation mimicks that found in the clinic in which bulk cells are transduced and then reintroduced into the patient and allowed to propogate. There was a modest 30% reduction of ribozyme expression after 3 months. This difference probably arose from cells with varying amount of ribozyme expression and exhibiting different growth rates in the culture becoming slightly more prevalent in the culture. However, ribozyme expression is apparently stable for at least this period of time.

Example 31: Design and construction of TRZ-tRNA Chimera

A transcription unit, termed **TRZ**, is designed that contains the **S35** motif (Figure 52). A desired RNA (e.g. ribozyme) can be inserted into the indicated region of **TRZ** tRNA chimera. This construct might provide additional stability to the desired RNA. TRZ-A and TRZ-B are non-limiting examples of the TRZ-tRNA chimera.

Referring to Fig. 53-54, a hammerhead ribozyme targeted to site I (HHITRZ-A; Fig. 53) and a hairpin ribozyme (HPITRZ-A; Fig. 54), also targeted to site I, is cloned individually into the indicated region of TRZ tRNA chimera. The

resulting ribozyme trancripts retain full RNA cleavage activity (see for example Fig. 55). Applicant has shown that efficient expression of these TRZ tRNA chimera can be achieved in mammalian cells.

Besides ribozymes, desired RNAs like antisense, therapeutic editing RNAs, decoys, can be readily inserted into the indicated region of TRZ-tRNA chimera to achieve therapeutic levels of RNA expression in mammalian cells.

Sequences listed in Figures 40-47 and 50 - 54 are meant to be non-limiting examples. Those skilled in the art will recognize that variants (mutations, insertions and deletions) of the above examples can be readily generated using techniques known in the art, are within the scope of the present invention.

Example 32: Ribozyme expression in T cell lines

Ribozyme expression in T cell lines stably-transduced with either a retroviral-based or an Adeno-associated virus (AAV)-based ribozyme expression vector (Figure 56). The human T cell lines MT2 and CEM were transduced with either retroviral or AAV vectors encoding a neomycin slelctable marker and a ribozyme (S35/HHI) expressed from pol III $met_i$ tRNA-driven promoter. Cells stably-transduced with the vectors were selectivelyt expanded medium containing the neomycin antibiotic derivative, G418 (0.7 mg/ml). Ribozyme expression in the stable cell lines was then alalyzed by Northern analysis. The probe used to detect ribozyme transcripts also cross-hybridized with human $met_i$ tRNA sequences. Refering to Figure 56, S35/HHI RNA accumulates to significant levels in MT2 and CEM cells when transduced with either the retrovirus or the AAV vector.

These are meant to be non-limiting examples, those skilled in the art will recognize that other vectors such as adenovirus vector (Figure 57), plasmid DNA vector, alpha virus vectors and the other derivatives there of, can be readily generated to deliver the desired RNA, using techniques known in the art and are within the scope of this invention. Additionally, the transcription units can be expressed individually or in multiples using pol II and/or pol III promoters.

References cited herein, as well as Draper WO 93/23569, 94/02495, 94/06331, Sullenger WO 93/12657, Thompson WO 93/04573, and Sullivan WO 94/04609, and 93/11253 describe methods for use of vectors decribed herein, and are incorporated by reference herein. In particular these vectors are useful for administration of antisense and decoy RNA molecules.

Example 33: Ligated Ribozymes are catalytically active

The ability of ribozymes generated by ligation methods, described in Draper et al., PCT WO 93/23569, to cleave target RNA was tested on either matched substrate RNA (Fig. 58) or long (622 nt) RNA (Fig. 59, 60 and 61).

Matched substrate RNAs were chemically synthesized using solid-phase RNA synthesis chemistry (Scaringe et al., 1990 Nucleic Acids Res. 18, 5433-5441). Substrate RNA was 5' end-labeled using [$\gamma$-$^{32}$P] ATP and polynucleotide kinase (Curr. Protocols Mol. Biol. 1992, ed. Ausubel et al., Wiley & Sons, NY). Ribozyme reactions were carried out under ribozyme excess conditions ($k_{cat}/K_M$; Herschlag and Cech, 1990 Biochemistry 29, 10159-10171). Briefly, ribozyme and substrate RNA were denatured and renatured separately by heating to 90°C and snap cooling on ice for 10 min in a buffer containing 50 mM Tris. HCl pH 7.5 and 10 mM $MgCl_2$. Cleavage reaction was initiated by mixing the ribozyme with the substrate at 37°C. Aliquots of 5 µl were taken at regular intervals of time and the reaction was stopped by mixing with equal volume of formamide gel loading buffer (Curr. Protocols Mol. Biol. 1992, ed. Ausubel et al., Wiley & Sons, NY). The samples were resolved on 20 % polyacrylamide-urea gel. Refering to Fig. 58, -$\Delta$G refers to the free energy of binding calculated for base-paired interactions between the ribozyme and the substrate RNA (Turner and Sugimoto, 1988 Supra). RPI A is a HH ribozyme with 6/6 binding arms. This ribozyme was synthesized chemically either as a one piece ribozyme or was synthesized in two fragments followed by ligation to generate a one piece ribozyme. The $k_{cat}/K_M$ values for the two ribozymes were comparable.

A template containing T7 RNA polymerase promoter upstream of 622 nt long target sequence, was PCR amplified from a DNA clone. The target RNA (containing HH ribozyme cleavage sites B, C and D) was transcribed from this PCR amplified template using T7 RNA polymerase. The transcript was internally labeled during transcription by including [$\alpha$-$^{32}$P] CTP as one of the four ribonucleotide triphosphates. The transcription mixture was treated with DNase-1, following transcription at 37°C for 2 hours, to digest away the DNA template used in the transcription. RNA was precipitated with Isopropanol and the pellet was washed two times with 70% ethanol to get rid of salt and nucleotides used in the transcription reaction. RNA is resuspended in DEPC-treated water and stored at 4°C. Ribozyme cleavage reactions were carried out under ribozyme excess ($k_{cat}/K_M$) conditions [Herschlag and Cech 1990 supra]. Briefly, 1000 nM ribozyme and 10 nM internally labeled target RNA were denatured separately by heating to 90°C for 2 min in the presence of 50 mM Tris.HCl, pH 7.5 and 10 mM $MgCl_2$. The RNAs were renatured by cooling to 37°C for 10-20 min. Cleavage reaction was initiated by mixing the ribozyme and target RNA at 37°C. Aliquots of 5 µl were taken at regular intervals of time and the reaction was quenched by adding equal volume of stop buffer. The samples were resolved on a sequencing gel.

Example 34: Hammerhead ribozymes with ≥ 2 base-paired stem II are catalytically active

To decrease the cost of chemical synthesis of RNA, applicant was interested in determining whether the length of stem II region of a typical hammerhead ribozyme (≥ 4 bp stem II) can be shortened without decreasing the catalytic efficiency of the HH ribozyme. The length of stem II was systematically shortened by one base-pair at a time. HH ribozymes with three and two base-paired stem II were chemically synthesized using solid-phase RNA phosphoramidite chemistry (Scaringe et al., 1990 supra).

Matched and long substrate RNAs were synthesized and ribozyme assays were carried out as described in example 33. Referring to figures 62, 63 and 64, data shows that shortening stem II of a hammerhead ribozyme does not significantly alter the catalytic efficiency. It is applicant's opinion that hammerhead ribozymes with ≥ 2 base-paired stem II region are catalytically active.

Example 35: Synthesis of catalytically active hairpin ribozymes

RNA molecules were chemically synthesized having the nucleotide base sequence shown in Fig. 65 for both the 5' and 3' fragments. The 3' fragments are phosphorylated and ligated to the 5' fragment essentially as described in example 37. As is evident from the Figure 65, the 3' and 5' fragments can hybridize together at helix 4 and are covalently linked via GAAA sequence. When this structure hybridizes to a substrate, a ribozyme•substrate complex structure is formed. While helix 4 is shown as 3 base pairs it may be formed with only 1 or 2 base pairs.

40 nM mixtures of ligated ribozymes were incubated with 1-5 nM 5' end-labeled matched substrates (chemically synthesized by solid-phase synthesis using RNA phosphoramidite chemistry) for different times in 50 mM Tris/HCl pH 7.5, 10 mM $MgCl_2$ and shown to cleave the substrate efficiently (Fig.66).

The target and the ribozyme sequences shown in Fig. 62 and 65 are meant to be non-limiting examples. Those in the art will recognize that other embodiments can be readily generated using other sequences and techniques generally known in the art.

**V. Constructs of Hairpin Ribozymes**

There follows an improved trans-cleaving hairpin ribozyme in which a new helix (*i.e.*, a sequence able to form a double-stranded region with another single-stranded nucleic acid) is provided in the ribozyme to base-pair with a 5' region of a separate substrate nucleic acid. This helix is provided at the 3' end of the ribozyme after helix 3 as shown in Figure 3. In addition, at least two extra bases may be provided in helix 2 and a portion of the substrate corresponding to helix 2 may be either directly linked to the 5' portion able to hydrogen bond to the 3' end of the hairpin or may have a linker of atleast one base. By trans-cleaving is meant that the ribozyme is able to act in *trans* to cleave another RNA molecule which is not covalently linked to the ribozyme itself. Thus, the ribozyme is not able to act on itself in an intramolecular cleavage reaction.

By "base-pair" is meant a nucleic acid that can form hydrogen bond(s) with other RNA sequence by either traditional Watson-Crick or other non-traditional types (for example Hoogsteen type) of interactions.

The increase in length of helix 2 of a hairpin ribozyme (with or without helix 5) has several advantages. These include improved stability of the ribozyme-target complex *in vivo* . In addition, an increase in the recognition sequence of the hairpin ribozyme improves the specificity of the ribozyme. This also makes possible the targeting of potential hairpin ribozyme sites that would otherwise be inaccessible due to neighboring secondary structure.

The increase in length of helix 2 of a hairpin ribozyme (with or without helix 5) enhances *trans*-ligation reaction catalyzed by the ribozyme. *Trans*ligation reactions catalyzed by the regular hairpin ribozyme (4 bp helix 2) is very inefficient (Komatsu *et al.,* 1993 *Nucleic Acids Res.* 21, 185). This is attributed to weak base-pairing interactions between substrate RNAs and the ribozyme. By increasing the length of helix 2 (with or without helix 5) the rate of ligation *(in vitro* and *in vivo)* can be enhanced several fold.

Results of experiments suggest that the length of H2 can be 6 bp without significantly reducing the activity of the hairpin ribozyme. The H2 arm length variation does not appear to be sequence dependent. HP ribozymes with 6 bp H2 have been designed against five different target RNAs and all five ribozymes efficiently cleaved their cognate target RNA. Additionally, two of these ribozymes were able to successfully inhibit gene expression (e.g., TNF-$\alpha$) in mammalian cells. Results of these experiments are shown below.

HP ribozymes with 7 and 8 bp H2 are also capable of cleaving target RNA in a sequence-specific manner, however, the rate of the cleavage reaction is lower than those catalyzed by HP ribozymes with 6 bp H2.

Example 36: 4 and 6 base pair H2

Referring to Figures 67-72, HP ribozymes were synthesized as described above and tested for activity. Surprisingly, those with 6 base pairs in H2 were still as active as those with 4 base pairs.

## VI. Chemical Modification

### Oligonucleotides with 5'-C-alkyl Group

The introduction of an alkyl group at the 5'-position of a nucleoside or nucleotide sugar introduces an additional center of chirality into the sugar moiety. Referring to Fig. 75, the general structures of 5'-*C*-alkylnucleotides belonging to the D-allose, **2**, and L-talose, **3**, sugar families are shown. The family names are derived from the known sugars D-allose and L-talose ($R_1 = CH_3$ in **2** and **3** in Figure 75). Useful specific D-allose and L-talose nucleotide derivatives are shown in Figure 76, **29-32** and Figure 77, **58-61** respectively.

This invention relates to the use of 5'-*C*-alkylnucleotides in oligonucleotides, which are particularly useful for enzymatic cleavage of RNA or single-stranded DNA, and also as antisense oligonucleotides. As the term is used in this application, 5'-*C*-alkylnucleotide-containing enzymatic nucleic acids are catalytic nucleic molecules that contain 5'-*C*-alkylnucleotide components replacing, but not limited to, double stranded stems, single stranded "catalytic core" sequences, single-stranded loops or single-stranded recognition sequences. These molecules are able to cleave (preferably, repeatedly cleave) separate RNA or DNA molecules in a nucleotide base sequence specific manner. Such catalytic nucleic acids can also act to cleave intramolecularly if that is desired. Such enzymatic molecules can be targeted to virtually any RNA transcript.

Also within the invention are 5'-*C*-alkylnucleotides which may be present in enzymatic nucleic acid or even in antisense oligonucleotides. Such nucleotides are useful since they enhance the stability of the antisense or enzymatic molecule, and can be used in locations which do not affect the desired activity of the molecule. That is, while the presence of the 5'-*C*-alkyl group may reduce binding affinity of the oligonucleotide containing this modification, if that moiety is not in an essential base pair forming region then the enhanced stability that it provides to the molecule is advantageous. In addition, while the reduced binding may reduce enzymatic activity, the enhanced stability may make the loss of activity of less consequence. Thus, for example, if a 5'-*C*-alkyl-containing molecule has 10% the activity of the unmodified molecule, but has 10-fold higher stability *in vivo* then it has utility in the present invention. The same analysis is true for antisense oligonucleotides containing such modifications. The invention also relates to novel intermediates useful in the synthesis of such nucleotides and oligonucleotides (examples of which are shown in the Figures), and to methods for their synthesis,

Thus, in one aspect, the invention features 5'-*C*-alkylnucleosides, that is a nucleotide base having at the 5'-position on the sugar molecule an alkyl moiety. In a related aspect, the invention also features 5'-*C*-alkylnucleotides, and in preferred embodiments features those where the nucleotide is not uridine or thymidine. That is, the invention preferably includes all those nucleotides useful for making enzymatic nucleic acids or antisense molecules that are not described by the art discussed above. In preferred embodiments, the sugar of the nucleoside or nucleotide is in an optically pure form, as the talose or allose sugar.

Examples of various alkyl groups useful in this invention are shown in Figure 75, where each $R_1$ group is any alkyl. These examples are not limiting in the invention. Specifically, an "alkyl" group refers to a saturated aliphatic hydrocarbon, including straight-chain, branched-chain, and cyclic alkyl groups. Preferably, the alkyl group has 1 to 12 carbons. More preferably it is a lower alkyl of from 1 to 7 carbons, more preferably 1 to 4 carbons. The alkyl group may be substituted or unsubstituted. When substituted the substituted group(s) is preferably, hydroxyl, cyano, alkoxy, =O, =S, $NO_2$ or $N(CH_3)_2$, amino, or SH. The term also includes alkenyl groups which are unsaturated hydrocarbon groups containing at least one carbon-carbon double bond, including straight-chain, branched-chain, and cyclic groups. Preferably, the alkenyl group has 1 to 12 carbons. More preferably it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, =O, =S, $NO_2$, halogen, $N(CH_3)_2$, amino, or SH. The term "alkyl" also includes alkynyl groups which have an unsaturated hydrocarbon group containing at least one carbon-carbon triple bond, including straight-chain, branched-chain, and cyclic groups. Preferably, the alkynyl group has 1 to 12 carbons. More preferably it is a lower alkynyl of from 1 to 7 carbons, more preferably 1 to 4 carbons. The alkynyl group may be substituted or unsubstituted. When substituted the substituted group(s) is preferably, hydroxyl, cyano, alkoxy, =O, =S, $NO_2$ or $N(CH_3)_2$, amino or SH.

Such alkyl groups may also include aryl, alkylaryl, carbocyclic aryl, heterocyclic aryl, amide and ester groups. An "aryl" group refers to an aromatic group which has at least one ring having a conjugated $\pi$ electron system and includes carbocyclic aryl, heterocyclic aryl and biaryl groups, all of which may be optionally substituted. The preferred substituent(s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above. Carbocyclic aryl groups are groups wherein the ring atoms on the aromatic ring are all carbon atoms. The carbon atoms are optionally substituted. Heterocyclic aryl groups are groups having from 1 to 3 heteroatoms as

ring atoms in the aromatic ring and the remainder of the ring atoms are-carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the like, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

In other aspects, also related to those discussed above, the invention features oligonucleotides having one or more 5'-*C*-alkylnucleotides; e.g. enzymatic nucleic acids having a 5'-*C*-alkylnucleotide; and a method for producing an enzymatic nucleic acid molecule having enhanced activity to cleave an RNA or single-stranded DNA molecule, by forming the enzymatic molecule with at least one nucleotide having at its 5'-position an alkyl group. In other related aspects, the invention features 5'-*C*-alkylnucleotide triphosphates. These triphosphates can be used in standard protocols to form useful oligonucleotides of this invention.

The 5'-*C*-alkyl derivatives of this invention provide enhanced stability to the oligonulceotides containing them. While they may also reduce absolute activity in an *in vitro* assay they will provide enhanced overall activity *in vivo.* Below are provided assays to determine which such molecules are useful. Those in the art will recognize that equivalent assays can be readily devised.

In another aspect, the invention features a method for conversion of a protected allo sugar to a protected talo sugar. In the method, the protected allo sugar is contacted with triphenyl phosphine, diethylazodicarboxylate, and *p*-nitrobenzoic acid under inversion causing conditions to provide the protected talo sugar. While one example of such conditions is provided below, those in the art will recognize other such conditions. Applicant has found that such conversion allows for ready synthesis of all types of nucleotide bases as exemplified in the figures.

While this invention is applicable to all oligonucleotides, applicant has found that the modified molecules of this invention are particulary useful for enzymatic RNA molecules. Thus, below is provided examples of such molecules. Those in the art will recognize that equivalent procedures can be used to make other molecules without such enzymatic activity. Specifically, Figure 1 shows base numbering of a hammerhead motif in which the numbering of various nucleotides in a hammerhead ribozyme is provided. This is not to be taken as an indication that the Figure is prior art to the pending claims, or that the art discussed is prior art to those claims. Referring to Figure 1, the preferred sequence of a hammerhead ribozyme in a 5'- to 3'-direction of the catalytic core is CUGANGAG[base paired with]CGAAA. In this invention, the use of 5'-*C*-alkyl substituted nucleotides that maintain or enhance the catalytic activity and or nuclease resistance of the hammerhead ribozyme is described. Substitutions of any nucleotide with any of the modified nucleotides shown in Figure 75 are possible.

The following are non-limiting examples showing the synthesis of nucleic acids using 5'-*C*-alkyl-substituted phosphoramidites and the syntheses of the amidites.

Example 37: Synthesis of Hammerhead Ribozymes Containing 5'-*C*-Alkyl-nucleotides & Other Modified Nucleotides

The method of synthesis would follow the procedure for normal RNA synthesis as described in Usman,N.; Ogilvie,K.K.; Jiang,M.-Y.; Cedergren, R.J. *J. Am. Chem. Soc.* **1987,** *109*, 7845-7854 and in Scaringe,S.A.; Franklyn, C.; Usman,N. *Nucleic Acids Res.* **1990**, *18*, 5433-5441 and makes use of common nucleic acid protecting and coupling groups, such as dimethoxytrityl at the 5'-end, and phosphoramidites at the 3'-end (compounds **26-29** and **56-59**). These 5'-*C*-alkyl substituted phosphoramidites may be incorporated not only into hammerhead ribozymes, but also into hairpin, hepatitis delta virus, Group 1 or Group 2 ntron catalytic nucleic acids, or into antisense oligonucleotides. They are, herefore, of general use in any nucleic acid structure.

Example 38: Methyl-2,3-*O*-Isopropylidine-6-Deoxy-β-D-allofuranoside (**4**)

A suspension of L-rhamnose (100 g, 0.55 mol), $CuSO_4$ (120 g) and onc. $H_2SO_4$ (4.0 mL) in 1.0 L of dry acetone was mixed for 24 h at RT, then filtered. Conc. $NH_4OH$ (5 mL) was added to the filtrate and the newly armed precipitate was filtered. The residue was concentrated *in vacuo,* oevaporated with pyridine (2 x 300 mL), dissolved in pyridine (500 mL) nd cooled to 0 °C. A solution of *p*-toluenesufonylchloride (107 g , 0.56 mmol) in dry DCE (500 mL) was added dropwise over 0.5 h. The reaction mixture was left for 16 h at RT. The reaction was quenched by adding ice-water (0.5 L) and, after mixing for 0.5 h, was extracted with chloroform (0.75 L). The organic layer was washed with $H_2O$ (2 x 500 mL), 10% $H_2SO_4$ (2 x 300 mL), water (2 x 300 mL), sat. $NaHCO_3$ (2 x 300 mL), brine (2 x 300 mL), dried over $MgSO_4$ and evaporated to dryness. The residue (115 g) was dissolved in dry MeOH (1 L) and treated with NaOMe (23.2 g, 0.42 mmol) in MeOH. The reaction mixture was left for 16 h at 20 °C, neutralized with dry $CO_2$ and evaporated to dryness. The residue was suspended in chloroform (750 mL), filtered , concentrated to 100 mL and purified by flash chromatography in $CHCl_3$ to yield 45 g (37%) of compound **4**.

Example 39: Methyl-2,3-*O*-Isopropylidine-5-*O*-*t*-Butyldiphenylsilyl-6-Deoxy-β-D-Allofuranoside (**5**).

To solution of methylfuranoside **4** (12.5 g 62.2 mmol) and $AgNO_3$ (21.25 g, 125.0 mmol) in dry DMF (300 mL) *t*-butyldiphenylsilyl chloride (22.2 g , 81 mmol) was added dropwise under Ar over 0.5 h. The reaction mixture was stirred for 4 h at RT, diluted with $CHCl_3$ (200 mL), filtered and evaporated to dryness (below 40 °C using a high vacuum oil pump). The residue was dissolved in $CH_2Cl_2$ (300 mL) washed with sat. $NaHCO_3$ (2 x 50 mL), brine (2 x 50 mL), dried over $MgSO_4$ and evaporated to dryness. The residue was purified by flash chromatography in $CH_2Cl_2$ to yield 20.0 g (75%) of compound 5.

Example 40: Methyl-5-*O*-*t*-Butyldiphenylsilyl-6-Deoxy-β-D-Allofuranoside (6).

Methylfuranoside **5** (13.5 g, 30.6 mmol) was dissolved in $CF_3COOH$:dioxane:$H_2O$ / 2:1:1 (v/v/v, 200 mL) and stirred at 24 °C for 45 m. The reaction mixture was cooled to -10 °C, neutralized with conc. $NH_4OH$ (140 mL) and extracted with $CH_2Cl_2$ (500 mL). The organic layer was separated, washed with sat. $NaHCO_3$ (2 x 75 mL), brine (2 x 75 mL), dried over $MgSO_4$ and evaporated to dryness. The product **6** was purified by flash chromatography using a 0-10% MeOH gradient in $CH_2Cl_2$. Yield 9.0 g (76%).

Example 41: Methyl-2,3-di-*O*-Benzoyl-5-*O*-*t*-Butyldiphenylsilyl-6-Deoxy-β-D-Allofuranoside (**7**).

Methylfuranoside **6** (7.0 g, 17.5 mmol) was coevaporated with pyridine (2 x 100 mL) and dissolved in pyridine (100 mL). Benzoyl chloride (5.4 g, 38.5 mmol) was added and the reaction mixture was left at RT for 16 h. Dry EtOH (50 mL) was added and the reaction mixture was evaporated to dryness after 0.5 h. The residue was dissolved in $CH_2Cl_2$ (300 mL), washed with sat. $NaHCO_3$ (2 x 75 mL), brine (2 x 75 mL) dried over $MgSO_4$ and evaporated to dryness. The product was purified by flash chromatography in $CH_2Cl_2$ to yield 9.5 g (89%) of compound **7**.

Example 42: 1-*O*-Acetyl-2,3-di-*O*-benzoyl-5-*O*-*t*-Butyldiphenylsilyl-6-Deoxy-β-D-Allofuranose (**8**).

Dibenzoate **7** (4.7 g, 7.7 mmol) was dissolved in a mixture of AcOH (10.0 mL), $Ac_2O$ (20.0 mL) and EtOAc (30 mL) and the reaction mixture was cooled 0 °C. 98% $H_2SO_4$ (0.15 mL) was then added. The reaction mixture was kept at 0 °C for 16 h, and then poured into a cold 1:1 mixture of sat. $NaHCO_3$ and EtOAc (150 mL). After 0.5 h of vigorous stirring the organic phase was separated, washed with brine (2 x 75 mL), dried over $MgSO_4$, evaporated to dryness and coevaporated with toluene (2 x 50 mL). The product was purified by flash chromatography using a gradient of 0-5% MeOH in $CH_2Cl_2$. Yield: 4.0 g (82% as a mixture of α and β isomers).

Example 43: 1-(2',3'-di-*O*-Benzoyl-5'-*O*-*t*-Butyldiphenylsilyl-6'-Deoxy-β-D-Allofuranosyl)uracil (**9**).

Uracil (1.44 g, 11.5 mmol) was suspended in mixture of hexamethyldisilazane (100 mL) and pyridine (50 mL) and boiled under reflux until complete dissolution (3 h) occurred, and then for an additional hour. The reaction mixture was cooled to RT, evaporated to dryness and coevaporated with dry toluene (2 x 50 mL). To the residue was added a solution of acetates 8 (6.36 g, 10.0 mmol) in dry $CH_3CN$ (100 mL), followed by $CF_3SO_3SiMe_3$ (2.8 g, 12.6 mmol). The reaction mixture was kept at 24 °C for 16 h, concentrated to 1/3 of its original volume, diluted with 100 mL of $CH_2Cl_2$ and extracted with sat. $NaHCO_3$ (2 x 50 mL), brine (2 x 50 mL) dried over $MgSO_4$, and evaporated to dryness. The product **9** was purified by flash chromatography using a gradient of 0-5% MeOH in $CH_2Cl_2$. Yield: 5.7 g (80%).

Example 44: $N^4$-Benzoyl-1-(2',3'-Di-*O*-Benzoyl-5'-*O*-*t*-Butyldiphenylsilyl-6'-Deoxy-β-D-Allofuranosyl)Cytosine (**10**).

$N^4$-benzoylcytosine (1.84 g, 8.56 mmol) was suspended in mixture of hexamethyldisilazane (100 mL) and pyridine (50 mL) and boiled under reflux until complete dissolution (3 h) occurred, and then for an additional hour. The reaction mixture was cooled to RT evaporated to dryness and coevaporated with dry toluene (2 x 50 mL). To the residue was added a solution of of acetates **8** (3.6 g, 5.6 mmol) in dry $CH_3CN$ (100 mL), followed by $CF_3SO_3SiMe_3$ (4.76 g, 21.4 mmol). The reaction mixture was boiled under reflux for 5 h, cooled to RT, concentrated to 1/3 of its original volume, diluted with $CH_2Cl_2$ (100 mL) and extracted with sat. $NaHCO_3$ (2 x 50 mL), brine (2 x 50 mL) dried over $MgSO_4$ and evaporated to dryness. Purification by flash chromatography using a gradient of 0-5% MeOH in $CH_2Cl_2$ yielded 1.8 g (55%) of compound **10**.

Example 45: $N^6$-Benzoyl-9-(2',3'-di-*O*-Benzoyl-5'-*O*-*t*-Butyldiphenylsilyl-6'-Deoxy-β-D-Allofuranosyl)adenine (**11**).

$N^6$-benzoyladenine (2.86 g, 11.86 mmol) was suspended in mixture of hexamethyldisilazane (100 mL) and

pyridine (50 mL) and boiled under reflux until complete dissolution (7 h) occurred, and then for an additional hour. The reaction mixture was cooled to RT evaporated to dryness and coevaporated with dry toluene (2 x 50 mL). To the residue was added a solution of of acetates **8** (3.6 g, 5.6 mmol) in dry $CH_3CN$ (100 mL) followed by $CF_3SO_3SiMe_3$ (6.59 g, 29.7 mmol). The reaction mixture was boiled under reflux for 8 h, cooled to RT, concentrated to 1/3 of its original volume, diluted with $CH_2Cl_2$ (100 mL) and extracted with sat. $NaHCO_3$ (2 x 50 mL), brine (2 x 50 mL) dried over $MgSO_4$ and evaporated to dryness. The product **11** was purified by flash chromatography using a gradient of 0-5% MeOH in $CH_2Cl_2$. Yield: 2.7 g (60%).

Example 46: *N*$^2$-Isobutyryl-9-(2',3'-di-*O*-Benzoyl-5'-*O*-*t*-Butyldiphenylsilyl-6'-Deoxy-β-D-Allofuranosyl)guanine (**12**).

*N*$^2$-Isobutyrylguanine (1.47 g , 11.2 mmol) was suspended in mixture of hexamethyldisilazane (100 mL) and pyridine (50 mL) and boiled under reflux until complete dissolution (6 h) occurred, and then for an additional hour. The reaction mixture was cooled to RT evaporated to dryness and coevaporated with dry toluene (2 x 50 mL). To the residue was added a solution of of acetates **8** (3.4 g, 5.3 mmol) in dry $CH_3CN$ (100 mL) followed by $CF_3SO_3SiMe_3$ (6.22 g, 28.0 mmol). The reaction mixture was boiled under reflux for 8 h, cooled to RT, concentrated to 1/3 of its original volume, diluted with $CH_2Cl_2$ (100 mL) and extracted with sat. $NaHCO_3$ (2 x 50 mL), brine (2 x 50 mL) dried over $MgSO_4$ and evaporated to dryness. The product **12** was purified by flash chromatography using a gradient of 0-2% MeOH in $CH_2Cl_2$. Yield: 2.1g (54%).

Example 47: *N*$^6$-Benzoyl-9-(2',3'-di-*O*-benzoyl-6'-Deoxy-β-D-Allofurano -syl)adenine (**15**).

Nucleoside **11** (1.65 g, 2.0 mmol) was dissolved in THF (50 mL) and a 1 M solution of TBAF in THF (4 mL) was added. The reaction mixture was kept at RT for 4 h, evaporated to dryness and the product purified by flash chromatography using a gradient of 0-5% MeOH in $CH_2Cl_2$ to yield 1.0 g (85%) of compound 15.

Example 48: *N*$^6$-Benzoyl-9-(2',3'-di-*O*-Benzoyl-5'-*O*-Dimethoxytrityl-6'-Deoxy-β-D-Allofuranosyl)-adenine (**19**).

Nucleoside **15** (0.55 g, 0.92 mmol) was dissolved in dry $CH_2Cl_2$ (50 mL). $AgNO_3$ (0.34 g, 2.0 mmol), dimethoxytrityl chloride (0.68 g, 2.0 mmol) and sym-collidine (0.48 g) were added under Ar. The reaction mixture was stirred for 2h, diluted with $CH_2Cl_2$ (100 mL), filtered, evaporated to dryness and coevaporated with toluene (2 x 50 mL). Purification by flash chromatography using a gradient of 0-5% MeOH in $CH_2Cl_2$ yielded 0.8 g (97%) of compound **19**.

Example 49: *N*$^6$-Benzoyl-9-(-5'-*O*-Dimethoxytrityl-6'-Deoxy-β-D-Allo-furanosyl)adenine (**23**).

Nucleoside **19** (1.8 g, 2 mmol) was dissolved in dioxane (50 mL), cooled to 0 °C and 2 M NaOH (50 mL) was added. The reaction mixture was kept at 0 °C for 45 m, neutralized with Dowex 50 ($Pyr^+$ form), filtered and the resin was washed with MeOH (2 x 50 mL). The filtrate was then evaporated to dryness. Purification by flash chromatography using a gradient of 0-10% MeOH in $CH_2Cl_2$ yielded 1.1 g (80%) of **23**.

Example 50: *N*$^6$-Benzoyl-9-(-5'-*O*-Dimethoxytrityl-2'-*O*-*t*-butyldimethylsilyl-6'-Deoxy-β-D-Allofuranosyl)adenine (**27**).

Nucleoside **23** (1.2 g, 1.8 mmol) was dissolved in dry THF (50 mL). Pyridine (0.50 g, 8 mmol) and $AgNO_3$ (0.4 g, 2.3 mmol) were added. After the $AgNO_3$ dissolved (1.5 h), *t*-butyldimethylsilyl chloride (0.35 g , 2.3 mmol) was added and the reaction mixture was stirred at RT for 16 h. The reaction mixture was diluted with $CH_2Cl_2$ (100 mL), filtered into sat. $NaHCO_3$ (50 mL), extracted, the organic layer washed with brine (2 x 50 mL), dried over $MgSO_4$ and evaporated to dryness. The product **27** was purified by flash chromatography using a hexanes:EtOAc / 7:3 gradient. Yield: 0.7 g (50%).

Example 51: *N*$^6$-Benzoyl-9-(-5'-*O*-Dimethoxytrityl-2'-*O*-*t*-butyldimethylsilyl-6'-Deoxy-β-D-Allofuranosyl)adenine-3'-(2-Cyanoethyl *N,N*-diisopropyl-phosphoramidite) (**31**).

Standard phosphitylation of **27** according to Scaringe,S.A.; Franklyn,C.; Usman,N. *Nucleic Acids Res.* **1990**, *18*, 5433-5441 yielded phosphoramidite **31** in 73% yield.

Example 52: Methyl-5-*O*-*p*-Nitrobenzoyl-2,3-*O*-Isopropylidine-6-deoxy-β-L-Tallofuranoside (**5**)

Methylfuranoside **4** (3.1 g 14.2 mmol) was dissolved in dry dioxane (200 mL), *p*-nitrobenzoic acid (10.0 g, 60 mmol) and triphenylphosphine (15.74 g, 60.0 mmol) were added followed by DEAD (10.45 g, 60.0 mmol). The reaction

mixture was left at RT for 16 h, EtOH (5 mL) was added, and after 0.5 h the reaction mixture was evaporated to dryness. The residue was dissolved in $CH_2Cl_2$ (300 mL) washed with sat. $NaHCO_3$ (2 x 75 mL), brine (2 x 75 mL) dried over $MgSO_4$ and evaporated to dryness. Purification by flash chromatography using a hexanes:EtOAc / 9:1 gradient yielded 4.1 g (78%) of compound **33**. Subsequent debenzoylation (NaOMe/MeOH) and silylation (see preparation of 5) led to L-talofuranoside **34** which was converted to phosphoramidites **58**-**61** using the same methodology as described above for the preparation of the phosphoramidites of the D-allo-isomers **29-32.**

The alkyl substituted nucleotides of this invention can be used to form stable oligonucleotides as discussed above for use in enzymatic cleavage or antisense situations. Such oligonucleotides can be formed enzymatically using triphosphate forms by standard procedure. Administration of such oligonucleotides is by standard procedure. See Sullivan et al., PCT WO 94/02595.

The ribozymes and the target RNA containing site O were synthesized, deprotected and purified as described above. RNA cleavage assay was carried our at 37°C in the presence of 10 mM $MgCl_2$ as described above.

Applicant has substituted 5'-C-Me-L-talo nucleotides at positions A6, A9, A9 + G10, C11.1 and C11.1 + G10, as shown in Figure 78 (**HH-O1 to HH-05**). ***HH-O 1,2,4*** and ***5*** showed almost wild type activity (Figure 79). However, ***HH-03*** demonstrated low catalytic activity. Ribozymes ***HH-01, 2, 3, 4 and 5*** are also extremely resistant to degradation by human serum nucleases.

Oligonucleotides with 2'-Deoxy-2'-Alkylnucleotide

This invention uses 2'-deoxy-2'-alkylnucleotides in oligonucleotides, which are particularly useful for enzymatic cleavage of RNA or single-stranded DNA, and also as antisense oligonucleotides. As the term is used in this application, 2'-deoxy-2'-alkyinucleotide-containing enzymatic nucleic acids are catalytic nucleic molecules that contain 2'-deoxy-2'-alkylnucleotide components replacing, but not limited to, double stranded stems, single stranded "catalytic core" sequences, single-stranded loops or single-stranded recognition sequences. These molecules are able to cleave (preferably, repeatedly cleave) separate RNA or DNA molecules in a nucleotide base sequence specific manner. Such catalytic nucleic acids can also act to cleave intramolecularly if that is desired. Such enzymatic molecules can be targeted to virtually any RNA transcript.

Also within the invention are 2'-deoxy-2'-alkylnucleotides which may be present in enzymatic nucleic acid or even in antisense oligonucleotides. Contrary to the findings of De Mesmaeker *et al.* applicant has found that such nucleotides are useful since they enhance the stability of the antisense or enzymatic molecule, and can be used in locations which do not affect the desired activity of the molecule. That is, while the presence of the 2'-alkyl group may reduce binding affinity of the oligonucleotide containing this modification, if that moiety is not in an essential base pair forming region then the enhanced stability that it provides to the molecule is advantageous. In addition, while the reduced binding may reduce enzymatic activity, the enhanced stability may make the loss of activity of less consequence. Thus, for example, if a 2'-deoxy-2'-alkyl-containing molecule has 10% the activity of the unmodified molecule, but has 10-fold higher stability *in vivo* then it has utility in the present invention. The same analysis is true for antisense oligonucleotides containing such modifications. The invention also relates to novel intermediates useful in the synthesis of such nucleotides and oligonucleotides (examples of which are shown in the Figures), and to methods for their synthesis.

Thus, in one aspect, the invention features 2'-deoxy-2'-alkylnucleotides, that is a nucleotide base having at the 2'-position on the sugar molecule an alkyl moiety and in preferred embodiments features those where the nucleotide is not uridine or thymidine. That is, the invention preferably includes all those nucleotides useful for making enzymatic nucleic acids or antisense molecules that are not described by the art discussed above.

Examples of various alkyl groups useful in this invention are shown in Figure 81, where each R group is any alkyl. The term "alkyl" does not include alkoxy groups which have an "-*O*-alkyl" group, where "alkyl" is defined as described above, where the O is adjacent the 2'-position of the sugar molecule.

In other aspects, also related to those discussed above, the invention features oligonucleotides having one or more 2'-deoxy-2'-alkylnucleotides (preferably not a 2'-alkyl- uridine or thymidine); *e.g*. enzymatic nucleic acids having a 2'-deoxy-2'-alkylnucleotide; and a method for producing an enzymatic nucleic acid molecule having enhanced activity to cleave an RNA or single-stranded DNA molecule, by forming the enzymatic molecule with at least one nucleotide having at its 2'-position an alkyl group. In other related aspects, the invention features 2'-deoxy-2'-alkylnucleotide triphosphates. These triphosphates can be used in standard protocols to form useful oligonucleotides of this invention.

The 2'-alkyl derivatives of this invention provide enhanced stability to the oligonulceotides containing them. While they may also reduce absolute activity in an *in vitro* assay they will provide enhanced overall activity *in vivo.* Below are provided assays to determine which such molecules are useful. Those in the art will recognize that equivalent assays can be readily devised.

In another aspect, the invention features hammerhead motifs having enzymatic activity having ribonucleotides at locations shown in Figure 80 at 5, 6, 8, 12, and 15.1, and having substituted ribonucleotides at other positions in

the core and in the substrate binding arms if desired. (The term "core" refers to positions between bases 3 and 14 in Figure 80, and the binding arms correspond to the bases from the 3'-end to base 15.1, and from the 5'-end to base 2). Applicant has found that use of ribonucleotides at these five locations in the core provide a molecule having sufficient enzymatic activity even when modified nucleotides are present at other sites in the motif. Other such combinations of useful ribonucleotides can be determined as described by Usman *et al. supra.*

Figure 80 shows base numbering of a hammerhead motif in which the numbering of various nucleotides in a hammerhead ribozyme is provided. This is not to be taken as an indication that the Figure is prior art to the pending claims, or that the art discussed is prior art to those claims. Referring to Figure 80 the preferred sequence of a hammerhead ribozyme in a 5'- to 3'-direction of the catalytic core is CUGANGAG[base paired with]CGAAA. In this invention, the use of 2'-*C*-alkyl substituted nucleotides that maintain or enhance the catalytic activity and or nuclease resistance of the hammerhead ribozyme is described. Although substitutions of any nucleotide with any of the modified nucleotides shown in Figure 81 are possible, and were indeed synthesized, the basic structure composed of promarily 2'-*O*-Me nucleotides weth selected substitutions was chosen to maintain maximal catalytic activity (Yang *et al. Biochemistry* **1992**, 31, 5005-5009 and Paolella *et al., EMBO J*. **1992**, 11, 1913-1919) and ease of synthesis, but is not limiting to this invention.

Ribozymes from Figure 80 and Table 45 were synthesized and assayed for catalytic activity and nuclease resistance. With the exception of entries **8** and **17**, all of the modified ribozymes retained at lease 1/10 of the wild-type catalytic activity. From Table 45, all 2'-modified ribozymes showed very large and significant increases in stability in human serum (shown) and in the other fluids described below (Example 55, data not shown). The order of most agressive nuclease activity was fetal bovine serum, > human serum >human plasma > human synovial fluid. As an overall measure of the effect of these 2'-substitutions on stability and activity, a ratio $\beta$ was calculated (Table 45). This $\beta$ value indicated that all modified ribozymes tested had significant, >100 - >1700 fold, increases in overall stability and activity. These increases in $\beta$ indicate that the lifetime of these modified ribozymes *in vivo* are significantly increased which should lead to a more pronounced biological effect.

More general substitutions of the 2'-modified nucleotides from Figure 81 also increased the $t_{1/2}$ of the resulting modified ribozymes. However the catalytic activity of these ribozymes was decreased > 10-fold.

In Figure 86 compound **37** may be used as a general intermediate to prepare derivatized 2'*C*-alkyl phosphoramidites, where X is $CH_3$, or an alkyl, or other group described above.

The following are non-limiting examples showing the synthesis of nucleic acids using 2'-*C*-alkyl substituted phosphoramidites, the syntheses of the amidites, their testing for enzymatic activity and nuclease resistance.

## Example 53: Synthesis of Hammerhead Ribozymes Containing 2'-Deoxy-2'-Alkylnucleotides & Other 2'-Modified Nucleotides

The method of synthesis used generally follows the procedure for normal RNA synthesis as described in Usman,N.; Ogilvie,K.K.; Jiang, M.-Y.; Cedergren,R.J. *J. Am. Chem. Soc.* **1987**, *109*, 7845-7854 and in Scaringe,S.A.; Franklyn, C.; Usman,N. *Nucleic Acids Res.* **1990**, *18*, 5433-5441 and makes use of common nucleic acid protecting and coupling groups, such as dimethoxytrityl at the 5'-end, and phosphoramidites at the 3'-end (compounds **10, 12, 17, 22, 31, 18, 26, 32, 36** and **38**). Other 2'-modified phosphoramidites were prepared according to: **3** & **4**, Eckstein *et al. International Publication* No. WO 92/07065; and **5** Kois *et al. Nucleosides & Nucleotides* **1993**, *12*, 1093-1109. The average stepwise coupling yields were ~98%. The 2'-substituted phosphoramidites were incorporated into hammerhead ribozymes as shown in Figure 80. However, these 2'-alkyl substituted phosphoramidites may be incorporated not only into hammerhead ribozymes, but also into hairpin, hepatitis delta virus, Group I or Group II intron catalytic nucleic acids, or into antisense oligonucleotides. They are, therefore, of general use in any nucleic acid structure.

## Example 54: Ribozyme Activity Assay

Purified 5'-end labeled RNA substrates (15-25-mers) and purified 5'-end labeled ribozymes (~36-mers) were both heated to 95 °C, quenched on ice and equilibrated at 37 °C, separately. Ribozyme stock solutions were 1 mM, 200 nM, 40 nM or 8 nM and the final substrate RNA concentrations were ~ 1 nM. Total reaction volumes were 50 mL. The assay buffer was 50 mM Tris-Cl, pH 7.5 and 10 mM $MgCl_2$. Reactions were initiated by mixing substrate and ribozyme solutions at t = 0. Aliquots of 5 mL were removed at time points of 1, 5, 15, 30, 60 and 120 m. Each time point was quenched in formamide loading buffer and loaded onto a 15% denaturing polyacrylamide gel for analysis. Quantitative analyses were performed using a phosphorimager (Molecular Dynamics).

## Example 55: Stability Assay

500 pmol of gel-purified 5'-end-labeled ribozymes were precipitated in ethanol and pelleted by centrifugation.

Each pellet was resuspended in 20 mL of appropriate fluid (human serum, human plasma, human synovial fluid or fetal bovine serum) by vortexing for 20 s at room temperature. The samples were placed into a 37 °C incubator and 2 mL aliquots were withdrawn after incubation for 0, 15, 30, 45, 60, 120, 240 and 480 m. Aliquots were added to 20 mL of a solution containing 95% formamide and 0.5X TBE (50 mM Tris, 50 mM borate, 1 mM EDTA) to quench further nuclease activity and the samples were frozen until loading onto gels. Ribozymes were size-fractionated by electrophoresis in 20% acrylamide/8M urea gels. The amount of intact ribozyme at each time point was quantified by scanning the bands with a phosphorimager (Molecular Dynamics) and the half-life of each ribozyme in the fluids was determined by plotting the percent intact ribozyme *vs* the time of incubation and extrapolation from the graph.

Example 56: 3',5'-*O*-(Tetraisopropyl-disiloxane-1,3-diyl)-2'-*O*-Phenoxythio-carbonyl-Uridine (**7**)

To a stirred solution of 3',5'-*O*-(tetraisopropyl-disiloxane-1,3-diyl)-uridine, **6**, (15.1 g, 31 mmol, synthesized according to *Nucleic Acid Chemistry,* ed. Leroy Townsend, **1986** pp. 229-231) and dimethylaminopyridine (7.57 g, 62 mmol) a solution of phenylchlorothionoformate (5.15 mL, 37.2 mmol) in 50 mL of acetonitrile was added dropwise and the reaction stirred for 8 h. TLC (EtOAc:hexanes / 1:1) showed disappearance of the starting material. The reaction mixture was evaporated, the residue dissolved in chloroform, washed with water and brine, the organic layer was dried over sodium sulfate, filtered and evaporated to dryness. The residue was purified by flash chromatography on silica gel with EtOAc:hexanes / 2:1 as eluent to give 16.44 g (85%) of **7**.

Example 57: 3',5'-*O*-(Tetraisopropyl-disiloxane-1,3-diyl)-2'-*C*-Allyl -Uridine (**8**)

To a refluxing, under argon, solution of 3',5'-*O*-(tetraisopropyl-disiloxane-1,3-diyl)-2'-*O*-phenoxythiocarbonyl-uridine, 7, (5 g, 8.03 mmol) and allyltributyltin (12.3 mL, 40.15 mmol) in dry toluene, benzoyl peroxide (0.5 g) was added portionwise during 1 h. The resulting mixture was allowed to reflux under argon for an additional 7-8 h. The reaction was then evaporated and the product 8 purified by flash chromatography on silica gel with EtOAc:hexanes / 1:3 as eluent. Yield 2.82 g (68.7%).

Example 58: 5'-*O*-Dimethoxytrityl-2'-*C*-Allyl-Uridine (**9**)

A solution of **8** (1.25 g, 2.45 mmol) in 10 mL of dry tetrahydrofuran (THF) was treated with a 1 M solution of tetrabutylammoniumfluoride in THF (3.7 mL) for 10 m at room temperature. The resulting mixture was evaporated, the residue was loaded onto a silica gel column, washed with 1 L of chloroform, and the desired deprotected compound was eluted with chloroform:methanol / 9:1. Appropriate fractions were combined, solvents removed by evaporation, and the residue was dried by coevaporation with dry pyridine. The oily residue was redissolved in dry pyridine, dimethoxytritylchloride (1.2 eq) was added and the reaction mixture was left under anhydrous conditions overnight. The reaction was quenched with methanol (20 mL), evaporated, dissolved in chloroform, washed with 5% aq. sodium bicarbonate and brine. The organic layer was dried over sodium sulfate and evaporated. The residue was purified by flash chromatography on silica gel, EtOAc:hexanes / 1:1 as eluent, to give 0.85 g (57%) of **9** as a white foam.

Example 59: 5'-*O*-Dimethoxytrityl-2'-*C*-Allyl-Uridine 3'-(2-Cyanoethyl *N,N*-diisopropylphosphoramidite) (**10**)

5'-*O*-Dimethoxytrityl-2'-*C*-allyl-uridine (0.64 g, 1.12 mmol) was dissolved in dry dichloromethane under dry argon. *N,N*-Diisopropylethyl-amine (0.39 mL, 2.24 mmol) was added and the solution was ice-cooled; 2-Cyanoethyl *N,N*-diisopropylchlorophosphoramidite (0.35 mL, 1.57 mmol) was added dropwise to the stirred reaction solution and stirring was continued for 2 h at RT. The reaction mixture was then ice-cooled and quenched with 12 mL of dry methanol. After stirring for 5 m, the mixture was concentrated *in vacuo* (40 °C) and purified by flash chromatography on silica gel using a gradient of 10-60% EtOAc in hexanes containing 1% triethylamine mixture as eluent. Yield: 0.78 g (90%), white foam.

Example 60: 3',5'-*O*-(Tetraisopropyl-disiloxane-1,3-diyl)-2'-*C*-Allyl-$N^4$-Acetyl-Cytidine (**11**)

Triethylamine (6.35 mL, 45.55 mmol) was added dropwise to a stirred ice-cooled mixture of 1,2,4-triazole (5.66 g, 81.99 mmol) and phosphorous oxychloride (0.86 mL, 9.11 mmol) in 50 mL of anhydrous acetonitrile. To the resulting suspension a solution of 3',5'-0-(tetraisopropyl-disiloxane-1,3-diyl)-2'-*C*-allyl uridine (2.32 g, 4.55 mmol) in 30 mL of acetonitrile was added dropwise and the reaction mixture was stirred for 4 h at room temperature. The reaction was concentrated *in vacuo* to a minimal volume (not to dryness). The residue was dissolved in chloroform and washed with water, saturated aq. sodium bicarbonate and brine. The organic layer was dried over sodium sulfate and the solvent was removed *in vacuo.* The resulting foam was dissolved in 50 mL of 1,4-dioxane and treated with 29% aq.

$NH_4OH$ overnight at room temperature. TLC (chloroform:methanol /9:1) showed complete conversion of the starting material. The solution was evaporated, dried by coevaporation with anhydrous pyridine and acetylated with acetic anhydride (0.52 mL, 5.46 mmol) in pyridine overnight. The reaction mixture was quenched with methanol, evaporated, the residue was dissolved in chloroform, washed with sodium bicarbonate and brine. The organic layer was dried over sodium sulfate, evaporated to dryness and purified by flash chromatography on silica gel (3% MeOH in chloroform). Yield 2.3 g (90%) as a white foam.

Example 61: 5'-*O*-Dimethoxytrityl-2'-*C*-Allyl-*N*$^4$-Acetyl-Cytidine

This compound was obtained analogously to the uridine derivative 9 in 55% yield.

Example 62: 5'-*O*-Dimethoxytrityl-2'-*C*-allyl-*N*$^4$-Acetyl-Cytidine 3'-(2-Cyanoethyl *N,N*-diisopropylphosphoramidite) (**12**)

2'-*O*-Dimethoxytrityl-2'-*C*-allyl-*N*$^4$-acetyl cytidine (0.8 g, 1.31 mmol) was dissolved in dry dichloromethane under argon. *N,N*-Diisopropylethyl-amine (0.46 mL, 2.62 mmol) was added and the solution was ice-cooled. 2-Cyanoethyl *N,N*-diisopropylchlorophosphoramidite (0.38 mL, 1.7 mmol) was added dropwise to a stirred reaction solution and stirring was continued for 2 h at room temperature. The reaction mixture was then ice-cooled and quenched with 12 mL of dry methanol. After stirring for 5 m, the mixture was concentrated *in vacuo* (40 °C) and purified by flash chromatography on silica gel using chloroform:ethanol / 98:2 with 2% triethylamine mixture as eluent. Yield: 0.91 g (85%), white foam.

Example 63: 2'-Deoxy-2'-Methylene-Uridine

2'-Deoxy-2'-methylene-3',5'-*O*-(tetraisopropyldisiloxane-1,3-diyl)-uridine **14** (Hansske,F.; Madej,D.; Robins, M. J. *Tetrahedron* **1984**, *40*, 125 and Matsuda,A.; Takenuki,K.; Tanaka,S.; Sasaki,T.; Ueda,T. *J. Med. Chem.* **1991**, *34,* 812) (2.2 g, 4.55 mmol ) dissolved in THF (20 mL) was treated with 1 M TBAF in THF (10 mL) for 20 m and concentrated *in vacuo*. The residue was triturated with petroleum ether and chromatographed on a silica gel column. 2'-Deoxy-2'-methylene-uridine (1.0 g, 3.3 mmol, 72.5%) was eluted with 20% MeOH in $CH_2Cl_2$.

Example 64: 5'-*O*-DMT-2'-Deoxy-2'-Methylene-Uridine (**15**)

2'-Deoxy-2'-methylene-uridine (0.91 g, 3.79 mmol) was dissolved in pyridine (10 mL) and a solution of DMT-Cl in pyridine (10 mL) was added dropwise over 15 m. The resulting mixture was stirred at RT for 12 h and MeOH (2 mL) was added to quench the reaction. The mixture was concentrated *in vacuo* and the residue taken up in $CH_2Cl_2$ (100 mL) and washed with sat. $NaHCO_3$, water and brine. The organic extracts were dried over $MgSO_4$, concentrated *in vacuo* and purified over a silica gel column using EtOAc:hexanes as eluant to yield **15** (0.43 g, 0.79 mmol, 22%).

Example 65: 5'-*O*-DMT-2'-Deoxy-2'-Methylene-Uridine 3'-(2-Cyanoethyl *N,N*-diisopropylphosphoramidite) (**17**)

1-(2'-Deoxy-2'-methylene-5'-*O*-dimethoxytrityl-β-D-ribofuranosyl)-uracil (0.43 g, 0.8 mmol) dissolved in dry $CH_2Cl_2$ (15 mL) was placed in a round-bottom flask under Ar. Diisopropylethylamine (0.28 mL, 1.6 mmol) was added, followed by the dropwise addition of 2-cyanoethyl *N,N*-diiso-propylchlorophosphoramidite (0.25 mL, 1.12 mmol). The reaction mixture was stirred 2 h at RT and quenched with ethanol (1 mL). After 10 m the mixture evaporated to a syrup *in vacuo* (40 °C). The product (0.3 g, 0.4 mmol, 50%) was purified by flash column chromatography over silica gel using a 25-70% EtOAc gradient in hexanes, containing 1% triethylamine, as eluant. $R_f$ 0.42 ($CH_2Cl_2$: MeOH / 15:1)

Example 66: 2'-Deoxy-2'-Difluoromethylene-3'.5'-*O*-(Tetraisopropyldisilox -ane-1,3-diyl)-Uridine

2'-Keto-3',5'-*O*-(tetraisopropyldisiloxane-1,3-diyl)uridine **14** (1.92 g, 12.6 mmol) and triphenylphosphine (2.5 g, 9.25 mmol) were dissolved in diglyme (20 mL), and heated to a bath temperature of 160 °C. A warm (60 °C) solution of sodium chlorodifluoroacetate in diglyme (50 mL) was added (dropwise from an equilibrating dropping funnel) over a period of ~1 h. The resulting mixture was further stirred for 2 h and concentrated *in vacuo.* The residue was dissolved in $CH_2Cl_2$ and chromatographed over silica gel. 2'-Deoxy-2'-difluoromethylene-3',5'-O-(tetraisopropyldlsiloxane-1,3-diyl)-uridine (3.1 g, 5.9 mmol, 70%) eluted with 25% hexanes in EtOAc.

Example 67: 2'-Deoxy-2'-Difluoromethylene-Uridine

2'-Deoxy-2'-methylene-3',5'-*O*-(tetraisopropyldisiloxane-1,3-diyl)-uridine (3.1 g, 5.9 mmol) dissolved in THF

(20 mL) was treated with 1 M TBAF in THF (10 mL) for 20 m and concentrated *in vacuo*. The residue was triturated with petroleum ether and chromatographed on silica gel column. 2'-Deoxy-2'-difluoromethylene-uridine (1.1 g, 4.0 mmol, 68%) was eluted with 20% MeOH in $CH_2Cl_2$.

Example 68: 5'-*O*-DMT-2'-Deoxy-2'-Difluoromethylene-Uridine (**16**)

2'-Deoxy-2'-difluoromethylene-uridine (1.1 g, 4.0 mmol) was dissolved in pyridine (10 mL) and a solution of DMT-Cl (1.42 g, 4.18 mmol) in pyridine (10 mL) was added dropwise over 15 m. The resulting mixture was stirred at RT for 12 h and MeOH (2 mL) was added to quench the reaction. The mixture was concentrated *in vacuo* and the residue taken up in $CH_2Cl_2$ (100 mL) and washed with sat. $NaHCO_3$, water and brine. The organic extracts were dried over $MgSO_4$, concentrated *in vacuo* and purified over a silica gel column using 40% EtOAc:hexanes as eluant to yield 5'-*O*-DMT-2'-deoxy-2'-difluoromethylene-uridine **16** (1.05 g, 1.8 mmol, 45%).

Example 69: 5'-*O*-DMT-2'-Deoxy-2'-Difluoromethylene-Uridine 3'-(2 -Cyanoethyl *N,N*-diisopropylphosphoramidite) (**18**)

1-(2'-Deoxy-2'-difluoromethylene-5'-*O*-dimethoxytrityl-β-D-ribofurano-syl)-uracil (0.577 g, 1 mmol) dissolved in dry $CH_2Cl_2$ (15 mL) was placed in a round-bottom flask under Ar. Diisopropylethylamine (0.36 mL, 2 mmol) was added, followed by the dropwise addition of 2-cyanoethyl *N,N*-diiso-propylchlorophosphoramidite (0.44 mL, 1.4 mmol). The reaction mixture was stirred for 2 h at RT and quenched with ethanol (1 mL). After 10 m the mixture evaporated to a syrup *in vacuo (40* °C). The product (0.404 g, 0.52 mmol, 52%) was purified by flash chromatography over silica gel using 20-50% EtOAc gradient in hexanes, containing 1% triethylamine, as eluant. $R_f$ 0.48 ($CH_2Cl_2$: MeOH / 15:1).

Example 70: 2'-Deoxy-2'-Methylene-3'.5'-*O*-(Tetraisopropyldisiloxane-1,3-diyl)-4-*N*-Acetyl-Cytidine **20**

Triethylamine (4.8 mL, 34 mmol) was added to a solution of $POCl_3$ (0.65 mL, 6.8 mmol) and 1,2,4-triazole (2.1 g, 30.6 mmol) in acetonitrile (20 mL) at 0 °C. A solution of 2'-deoxy-2'-methylene-3',5'-*0*-(tetraisopropyldi-siloxane-1,3-diyl) uridine **19** (1.65 g, 3.4 mmol) in acetonitrile (20 mL) was added dropwise to the above reaction mixture and left to stir at room temperature for 4 h. The mixture was concentrated *in vacuo,* dissolved in $CH_2Cl_2$ (2 x 100 mL) and washed with 5% $NaHCO_3$ (1 x 100 mL). The organic extracts were dried over $Na_2SO_4$ concentrated *in vacuo*, dissolved in dioxane (10 mL) and aq. ammonia (20 mL). The mixture was stirred for 12 h and concentrated *in vacuo*. The residue was azeotroped with anhydrous pyridine (2 x 20 mL). Acetic anhydride (3 mL) was added to the residue dissolved in pyridine, stirred at RT for 4 h and quenched with sat. $NaHCO_3$ (5 mL). The mixture was concentrated *in vacuo*, dissolved in $CH_2Cl_2$ (2 x 100 mL) and washed with 5% $NaHCO_3$ (1 x 100 mL). The organic extracts were dried over $Na_2SO_4$, concentrated *in vacuo* and the residue chromatographed over silica gel. 2'-Deoxy-2'-methylene-3',5'-*O*-(tetraisopropyldisiloxane-1,3-diyl)-4-*N*-acetyl-cytidine **20** (1.3 g, 2.5 mmol, 73%) was eluted with 20% EtOAc in hexanes.

Example 71: 1-(2'-Deoxy-2'-Methvlene-5'-*O*-Dimethoxytrityl-β-D-ribo-furanosyl)-4-*N*-Acetyl-Cytosine **21**

2'-Deoxy-2'-methylene-3',5'-*O*-(tetraisopropyldisiloxane-1,3-diyl)-4-*N*-acetyl-cytidine **20** (1.3 g, 2.5 mmol) dissolved in THF (20 mL) was treated with 1 M TBAF in THF (3 mL) for 20 m and concentrated *in vacuo*. The residue was triturated with petroleum ether and chromatographed on silica gel column. 2'-Deoxy-2'-methylene-4-*N*-acetyl-cytidine (0.56 g, 1.99 mmol, 80%) was eluted with 10% MeOH in $CH_2Cl_2$. 2'-Deoxy-2'-methylene-4-*N*-acetyl-cytidine (0.56 g, 1.99 mmol) was dissolved in pyridine (10 mL) and a solution of DMT-Cl (0.81 g, 2.4 mmol) in pyridine (10 mL) was added dropwise over 15 m. The resulting mixture was stirred at RT for 12 h and MeOH (2 mL) was added to quench the reaction. The mixture was concentrated *in vacuo* and the residue taken up in $CH_2Cl_2$ (100 mL) and washed with sat. $NaHCO_3$ (50 mL), water (50 mL) and brine (50 mL). The organic extracts were dried over $MgSO_4$, concentrated *in vacuo* and purified over a silica gel column using EtOAc:hexanes / 60:40 as eluant to yield **21** (0.88 g, 1.5 mmol, 75%).

Example 72: 1-(2'-Deoxy-2'-Methylene-5'-*O*-Dimethoxytrityl-β-D-ribo-furanosyl)-4-*N*-Acetyl-Cytosine 3'-(2-Cyanoethyl-*N,N*-diisopropylphosphor-amidite) (**22**)

1-(2'-Deoxy-2'-methylene-5'-*O*-dimethoxytrityl-β-D-ribofuranosyl)-4-*N*-acetyl-cytosine **21** (0.88 g, 1.5 mmol) dissolved in dry $CH_2Cl_2$ (10 mL) was placed in a round-bottom flask under Ar. Diisopropylethylamine (0.8 mL, 4.5 mmol) was added, followed by the dropwise addition of 2-cyanoethyl *N,N*-diisopropylchlorophosphoramidite (0.4 mL, 1.8 mmol). The reaction mixture was stirred 2 h at room temperature and quenched with ethanol (1 mL). After 10 m the mixture evaporated to a syrup *in vacuo* (40 °C). The product **22** (0.82 g, 1.04 mmol, 69%) was purified by flash chromatography over silica gel using 50-70% EtOAc gradient in hexanes, containing 1% triethylamine, as eluant. Rf

0.36 ($CH_2Cl_2$:MeOH / 20:1).

Example 73: 2'-Deoxy-2'-Difluoromethylene-3',5'-*O*-(Tetraisopropyl disiloxane-1,3-diyl)-4-*N*-Acetyl-Cytidine (**24**)

$Et_3N$ (6.9 mL, 50 mmol) was added to a solution of $POCl_3$ (0.94 mL, 10 mmol) and 1,2,4-triazole (3.1 g, 45 mmol) in acetonitrile (20 mL) at 0 °C. A solution of 2'-deoxy-2'-difluoromethylene-3',5'-*O*-(tetraisopropyldisilox-ane-1,3-diyl)uridine **23** ([described in example 14] 2.6 g, 5 mmol) in acetonitrile (20 mL) was added dropwise to the above reaction mixture and left to stir at RT for 4 h. The mixture was concentrated *in vacuo*, dissolved in $CH_2Cl_2$ (2 x 100 mL) and washed with 5% $NaHCO_3$ (1 x 100 mL). The organic extracts were dried over $Na_2SO_4$ concentrated *in vacuo*, dissolved in dioxane (20 mL) and aq. ammonia (30 mL). The mixture was stirred for 12 h and concentrated *in vacuo*. The residue was azeotroped with anhydrous pyridine (2 x 20 mL). Acetic anhydride (5 mL) was added to the residue dissolved in pyridine, stirred at RT for 4 h and quenched with sat. $NaHCO_3$ (5mL). The mixture was concentrated *in vacuo*, dissolved in $CH_2Cl_2$ (2 x 100 mL) and washed with 5% $NaHCO_3$ (1 x 100 mL). The organic extracts were dried over $Na_2SO_4$, concentrated *in vacuo* and the residue chromatographed over silica gel. 2'-Deoxy-2'-difluoromethylene-3',5'-*O*-(tetraisopropyldisiloxane-1,3-diyl)-4-*N*-acetyl-cytidine **24** (2.2 g, 3.9 mmol, 78%) was eluted with 20% EtOAc in hexanes.

Example 74: 1-(2'-Deoxy-2'-Difluoromethylene-5'-*O*-Dimethoxytrityl-β-D-ribofuranosyl)-4-*N*-Acetyl-Cytosine (**25**)

2'-Deoxy-2'-difluoromethylene-3',5'-*O*-(tetraisopropyldisiloxane-1,3-diyl)-4-*N*-acetyl-cytidine **24** (2.2 g, 3.9 mmol) dissolved in THF (20 mL) was treated with 1 M TBAF in THF (3 mL) for 20 m and concentrated *in vacuo.* The residue was triturated with petroleum ether and chromatographed on a silica gel column. 2'-Deoxy-2'-difluoromethyl-ene-4-*N*-acetyl-cytidine (0.89 g, 2.8 mmol, 72%) was eluted with 10% MeOH in $CH_2Cl_2$. 2'-Deoxy-2'-difluoromethylene-4-*N*-acetyl-cytidine (0.89 g, 2.8 mmol) was dissolved in pyridine (10 mL) and a solution of DMT-Cl (1.03 g, 3.1 mmol) in pyridine (10 mL) was added dropwise over 15 m. The resulting mixture was stirred at RT for 12 h and MeOH (2 mL) was added to quench the reaction. The mixture was concentrated *in vacuo* and the residue taken up in $CH_2Cl_2$ (100 mL) and washed with sat. $NaHCO_3$ (50 mL), water (50 mL) and brine (50 mL). The organic extracts were dried over $MgSO_4$, concentrated *in vacuo* and purified over a silica gel column using EtOAc:hexanes / 60:40 as eluant to yield **25** (1.2 g, 1.9 mmol, 68%).

Example 75: 1-(2'-Deoxy-2'-Difluoromethylene-5'-*O*-Dimethoxytrityl-β-D-ribofuranosyl)-4-*N*-Acetylcytosine 3'-(2-cyanoethyl-N,N-diisopropylphos-phoramidite) (**26**)

1-(2'-Deoxy-2'-difluoromethylene-5'-*O*-dimethoxytrityl-β-D-ribofurano-syl)-4-*N*-acetylcytosine **25** (0.6 g, 0.97 mmol) dissolved in dry $CH_2Cl_2$ (10 mL) was placed in a round-bottom flask under Ar. Diisopropylethylamine (0.5 mL, 2.9 mmol) was added, followed by the dropwise addition of 2-cyanoethyl *N,N*-diisopropylchlorophosphoramidite (0.4 mL, 1.8 mmol). The reaction mixture was stirred 2 h at RT and quenched with ethanol (1 mL). After 10 m the mixture was evaporated to a syrup *in vacuo* (40 °C). The product **26**, a white foam (0.52 g, 0.63 mmol, 65%) was purified by flash chromatography over silica gel using 30-70% EtOAc gradient in hexanes, containing 1% triethylamine, as eluant. $R_f$ 0.48 ($CH_2Cl_2$:MeOH / 20:1).

Example 76: 2'-Keto-3',5'-*O*-(Tetraisopropyldisiloxane-1,3-diyl)-6-*N*-(4-*t*-Butylbenzoyl)-Adenosine (**28**)

Acetic anhydride (4.6 mL) was added to a solution of 3',5'-*O*-(tetraiso-propyldisiloxane-1,3-diyl)-6-*N*-(4-*t*-butyl-benzoyl)-adenosine (Brown,*J.*; Christodolou, C.; Jones,S.; Modak,A.; Reese,C.; Sibanda,S.; Ubasawa A. J. *Chem . Soc. Perkin Trans.* /**1989**, 1735) (6.2 g, 9.2 mmol) in DMSO (37 mL) and the resulting mixture was stirred at room temperature for 24 h. The mixture was concentrated *in vacuo*. The residue was taken up in EtOAc and washed with water. The organic layer was dried over $MgSO_4$ and concentrated *in vacuo*. The residue was purified on a silica gel column to yield 2'-keto-3',5'-*O*-(tetraisopropyldisiloxane-1,3-diyl)-6-*N*-(4-*t*-butylben-zoyl)-adenosine **28** (4.8 g, 7.2 mmol, 78%).

Example 77: 2'-Deoxy-2'-methylene-3',5'-*O*-(Tetraisopropyldisiloxane-1,3-diyl)-6-*N*-(4-*t*-Butylbenzoyl)-Adenosine (**29**)

Under a pressure of argon, sec-butyllithium in hexanes (11.2 mL, 14.6 mmol) was added to a suspension of triphenylmethylphosphonium iodide (7.07 g, 17.5 mmol) in THF (25 mL) cooled at -78 °C. The homogeneous orange solution was allowed to warm to -30 °C and a solution of 2'-keto-3',5'-*O*-(tetraisopropyldisiloxane-1,3-diyl)-6-*N*-(4-*t*-butylbenzoyl)-adenosine **28** (4.87 g, 7.3 mmol) in THF (25 mL) was transferred to this mixture under argon pressure. After warming to RT, stirring was continued for 24 h. THF was evaporated and replaced by $CH_2Cl_2$ (250 mL), water

was added (20 mL), and the solution was neutralized with a cooled solution of 2% HCl. The organic layer was washed with $H_2O$ (20 mL), 5% aqueous $NaHCO_3$ (20 mL), $H_2O$ to neutrality, and brine (10 mL). After drying ($Na_2SO_4$), the solvent was evaporated *in vacuo* to give the crude compound, which, was chromatographed on a silica gel column. Elution with light petroleum ether:EtOAc / 7:3 afforded pure 2'-deoxy-2'-methylene-3',5'-*O*-(tetraiso-propyldisiloxane-1,3-diyl)-6-*N*-(4-*t*-butylbenzoyl)-adenosine **29** (3.86 g, 5.8 mmol, 79%).

Example 78: 2'-Deoxy-2'-Methylene-6-*N*-(4-*t*-Butylbenzoyl)-Adenosine

2'-Deoxy-2'-methylene-3',5'-*O*-(tetraisopropyldisiloxane-1,3-diyl)-6-*N*-(4-*t*-butylbenzoyl)-adenosine (3.86 g, 5.8 mmol) dissolved in THF (30 mL) was treated with 1 M TBAF in THF (15 mL) for 20 m and concentrated *in vacuo*. The residue was triturated with petroleum ether and chromatographed on a silica gel column. 2'-Deoxy-2'-methylene-6-*N*-(4-*t*-butylbenzoyl)-adenosine (1.8 g, 4.3 mmol, 74%) was eluted with 10% MeOH in $CH_2Cl_2$.

Example 79: 5'-*O*-DMT-2'-Deoxy-2'-Methylene-6-*N*-(4-*t*-Butylbenzoyl)-Adenosine (**29**)

2'-Deoxy-2'-methylene-6-*N*-(4-*t*-butylbenzoyl)-adenosine (0.75 g, 1.77 mmol) was dissolved in pyridine (10 mL) and a solution of DMT-Cl (0.66 g, 1.98 mmol) in pyridine (10 mL) was added dropwise over 15 m. The resulting mixture was stirred at RT for 12 h and MeOH (2 mL) was added to quench the reaction. The mixture was concentrated *in vacuo* and the residue taken up in $CH_2Cl_2$ (100 mL) and washed with sat. $NaHCO_3$, water and brine. The organic extracts were dried over $MgSO_4$, concentrated *in vacuo* and purified over a silica gel column using 50% EtOAc:hexanes as an eluant to yield **29** (0.81 g, 1.1 mmol, 62%).

Example 80: 5'-*O*-DMT-2'-Deoxy-2'-Methylene-6-*N*-(4-*t*-Butylbenzoyl)-Adenosine 3'-(2-Cyanoethyl *N,N*-diisopropylphosphoramidite) (**31**)

1-(2'-Deoxy-2'-methylene-5'-*O*-dimethoxytrityl-β-D-ribofuranosyl)-6-*N*-(4-*t*-butylbenzoyl)-adenine **29** dissolved in dry $CH_2Cl_2$ (15 mL) was placed in a round bottom flask under Ar. Diisopropylethylamine was added, followed by the dropwise addition of 2-cyanoethyl *N,N*-diisopropylchlorophosphoramidite. The reaction mixture was stirred 2 h at RT and quenched with ethanol (1 mL). After 10 m the mixture was evaporated to a syrup *in vacuo* (40 °C). The product was purified by flash chromatography over silica gel using 30-50% EtOAc gradient in hexanes, containing 1% triethylamine, as eluant (0.7 g, 0.76 mmol, 68%). $R_f$ 0.45 ($CH_2Cl_2$: MeOH / 20:1)

Example 81: 2'-Deoxy-2'-Difluoromethylene-3',5'-*O*-(Tetraisopropyldisilox-ane-1,3-diyl)-6-*N*-(4-*t*-Butylbenzoyl) -Adenosine

2'-Keto-3',5'-O-(tetraisopropyldlsiloxane-1,3-diyl)-6-*N*-(4-*t*-butyl-benzoyl)-adenosine **28** (6.7 g, 10 mmol) and triphenylphosphine (2.9 g, 11 mmol ) were dissolved in diglyme (20 mL), and heated to a bath temperature of 160 °C. A warm (60 °C) solution of sodium chlorodifluoroacetate (2.3 g, 15 mmol) in diglyme (50 mL) was added (dropwise from an equilibrating dropping funnel) over a period of ~1 h. The resulting mixture was further stirred for 2 h and concentrated *in vacuo*. The residue was dissolved in $CH_2Cl_2$ and chromatographed over silica gel. 2'-Deoxy-2'-difluoromethylene-3',5'-*O*-(tetraisopropyldisiloxane-1,3-diyl)-6-*N*-(4-*t*-butylbenzoyl)-adenosine (4.1g, 6.4 mmol, 64%) eluted with 15% hexanes in EtOAc.

Example 82: 2'-Deoxy-2'-Difluoromethylene-6-*N*-(4-*t*-Butylbenzoyl) -Adenosine

2'-Deoxy-2'-difluoromethylene-3',5'-*O*-(tetraisopropyldisiloxane-1,3-diyl)-6-*N*-(4-*t*-butylbenzoyl)-adenosine (4.1 g, 6.4 mmol) dissolved in THF (20 mL) was treated with 1 M TBAF in THF (10 mL) for 20 m and concentrated *in vacuo*. The residue was triturated with petroleum ether and chromatographed on a silica gel column. 2'-Deoxy-2'-difluoromethyl-ene-6-*N*-(4-*t*-butylbenzoyl)-adenosine (2.3 g, 4.9 mmol, 77%) was eluted with 20% MeOH in $CH_2Cl_2$.

Example 83: 5'-*O*-DMT-2'-Deoxy-2'-Difluoromethylene-6-*N*-(4-*t*-Butyl-benzoyl)-Adenosine (**30**)

2'-Deoxy-2'-difluoromethylene-6-*N*-(4-*t*-butylbenzoyl)-adenosine (2.3 g, 4.9 mmol) was dissolved in pyridine (10 mL) and a solution of DMT-Cl in pyridine (10 mL) was added dropwise over 15 m. The resulting mixture was stirred at RT for 12 h and MeOH (2 mL) was added to quench the reaction. The mixture was concentrated *in vacuo* and the residue taken up in $CH_2Cl_2$ (100 mL) and washed with sat. $NaHCO_3$, water and brine. The organic extracts were dried over $MgSO_4$, concentrated *in vacuo* and purified over a silica gel column using 50% EtOAc:hexanes as eluant to yield 30 (2.6 g, 3.41 mmol, 69%).

Example 84: 5'-O-DMT-2'-Deoxy-2'-Difluoromethylene-6-*N*-(4-*t*-Butyl-benzoyl)-Adenosine 3'-(2-Cyanoethyl *N,N*-diisopropylphosphoramidite) (**32**)

1-(2'-Deoxy-2'-difluoromethylene-5'-*O*-dimethoxytrityl-β-D-ribofurano-syl)-6-*N*-(4-*t*-butylbenzoyl)-adenine 30 (2.6 g, 3.4 mmol) dissolved in dry $CH_2Cl_2$ (25 mL) was placed in a round bottom flask under Ar. Diisopropylethylamine (1.2 mL, 6.8 mmol) was added, followed by the dropwise addition of 2-cyanoethyl *N,N*-diisopropylchlorophosphoramidite (1.06 mL, 4.76 mmol). The reaction mixture was stirred 2 h at RT and quenched with ethanol (1 mL). After 10 m the mixture evaporated to a syrup *in vacuo* (40 °C). **32** (2.3 g, 2.4 mmol, 70%) was purified by flash column chromatography over silica gel using 20-50% EtOAc gradient in hexanes, containing 1% triethylamine, as eluant. Rf 0.52 ($CH_2Cl_2$: MeOH /15:1).

Example 85: 2'-Deoxy-2'-Methoxycarbonylmethylidine-3',5'-*O*-(Tetraiso-propyldisiloxane-1,3-diyl)-Uridine (**33**)

Methyl(triphenylphosphoranylidine)acetate (5.4 g, 16 mmol) was added to a solution of 2'-keto-3',5'-*O*-(tetraisopropyl disiloxane-1,3-diyl)-uridine **14** in $CH_2Cl_2$ under argon. The mixture was left to stir at RT for 30 h. $CH_2Cl_2$ (100 mL) and water were added (20 mL), and the solution was neutralized with a cooled solution of 2% HCl. The organic layer was washed with $H_2O$ (20 mL), 5% aq. $NaHCO_3$ (20 mL), $H_2O$ to neutrality, and brine (10 mL). After drying ($Na_2SO_4$), the solvent was evaporated *in vacuo* to give crude product, that was chromatographed on a silica gel column. Elution with light petroleum ether:EtOAc / 7:3 afforded pure 2'-deoxy-2'-methoxycarbonylmethylidine-3',5'-*O*-(tetraisopropyldisiloxane-1,3-diyl)-uridine **33** (5.8 g, 10.8 mmol, 67.5%).

Example 86: 2'-Deoxy-2'-Methoxycarbonylmethylidine-Uridine (**34**)

$Et_3N$•3 HF (3 mL) was added to a solution of 2'-deoxy-2'-methoxy-carboxylmethylidine-3',5'-*O*-(tetraisopropyldisiloxane-1,3-diyl)-uridine **33** (5 g, 9.3 mmol) dissolved in $CH_2Cl_2$ (20 mL) and $Et_3N$ (15 mL). The resulting mixture was evaporated in *vacuo* after 1 h and chromatographed on a silica gel column eluting 2'-deoxy-2'-methoxycarbonylmethylidine-uridine **34** (2.4 g, 8 mmol, 86%) with THF:$CH_2Cl_2$ / 4:1.

Example 87: 5'-*O*-DMT-2'-Deoxy-2'-Methoxycarbonylmethylidine-Uridine (**35**)

2'-Deoxy-2'-methoxycarbonylmethylidine-uridine **34** (1.2 g, 4.02 mmol) was dissolved in pyridine (20 mL). A solution of DMT-Cl (1.5 g, 4.42 mmol) in pyridine (10 mL) was added dropwise over 15 m. The resulting mixture was stirred at RT for 12 h and MeOH (2 mL) was added to quench the reaction. The mixture was concentrated *in vacuo* and the residue taken up in $CH_2Cl_2$ (100 mL) and washed with sat. $NaHCO_3$, water and brine. The organic extracts were dried over $MgSO_4$, concentrated *in vacuo* and purified over a silica gel column using 2-5% MeOH in $CH_2Cl_2$ as an eluant to yield 5'-*0*-DMT-2'-deoxy-2'-methoxycarbonylmethylidine-uridine **35** (2.03 g, 3.46 mmol, 86%).

Example 88: 5'-*O*-DMT-2'-Deoxy-2'-Methoxvcarbonylmethylidine-Uridine 3'-(2-cyanoethyl-*N,N*-diisopropylphosphoramidite) (**36**)

1-(2'-Deoxy-2'-2'-methoxycarbonylmethylidine-5'-*O*-dimethoxytrityl-β-D-ribofuranosyl)-uridine **35** (2.0 g, 3.4 mmol) dissolved in dry $CH_2Cl_2$ (10 mL) was placed in a round-bottom flask under Ar. Diisopropylethylamine (1.2 mL, 6.8 mmol) was added, followed by the dropwise addition of 2-cyanoethyl *N,N*-diisopropylchlorophosphoramidite (0.91 mL, 4.08 mmol). The reaction mixture was stirred 2 h at RT and quenched with ethanol (1 mL). After 10 m the mixture was evaporated to a syrup *in vacuo* (40 °C). 5'-*O*-DMT-2'-deoxy-2'-methoxycarbonylmethylidine-uridine 3'-(2-cyanoethyl-*N,N*-diisopropylphosphoramidite) **36** (1.8 g, 2.3 mmol, 67%) was purified by flash column chromatography over silica gel using a 30-60% EtOAc gradient in hexanes, containing 1% triethylamine, as eluant. $R_f$ 0.44 ($CH_2Cl_2$:MeOH / 9.5:0.5).

Example 89: 2'-Deoxy-2'-Carboxymethylidine-3',5'-*O*-(Tetraisopropyldi-siloxane-1,3-diyl)-Uridine **37**

2'-Deoxy-2'-methoxycarbonylmethylidine-3',5'-*O*-(tetraisopropyldi-siloxane-1,3-diyl)-uridine **33** (5.0 g, 10.8 mmol) was dissolved in MeOH (50 mL) and 1 N NaOH solution (50 mL) Was added to the stirred solution at RT. The mixture was stirred for 2 h and MeOH removed *in vacuo.* The pH of the aqueous layer was adjusted to 4.5 with 1N HCl solution, extracted with EtOAc (2 x 100 mL), washed with brine, dried over $MgSO_4$ and concentrated *in vacuo* to yield the crude acid. 2'-Deoxy-2'-carboxymethylidine-3',5'-*O*-(tetraisopropyldisiloxane-1,3-diyl)-uridine **37** (4.2 g, 7.8 mmol, 73%) was purified on a silica gel column using a gradient of 10-15% MeOH in $CH_2Cl_2$.

The alkyl substituted nucleotides of this invention can be used to form stable oligonucleotides as discussed

above for use in enzymatic cleavage or antisense situations. Such oligonucleotides can be formed enzymatically using triphosphate forms by standard procedure. Administration of such oligonucleotides is by standard procedure. See Sullivan *et al. PCT WO* 94/02595.

Oligonucleotides with 3' and/or 5' Dihalophosphonate

This invention synthesis and uses 3' and/or 5' dihalophosphonate-, e.g., 3' or 5'-$CF_2$-phosphonate-, substituted nucleotides that maintain or enhance the catalytic activity and/or nuclease resistance of an enzymatic or antisense molecule.

As the term is used in this application, 5'- and/or 3'-dihalophosphonate nucleotide containing ribozymes, deoxyribozymes (see Usman et al., PCT/US94/11649, incorporated by reference herein), and chimeras of nucleotides, are catalytic nucleic molecules that contain 5'-and/or 3'-dihalophosphonate nucleotide components replacing, but not limited to, double-stranded stems, single-stranded "catalytic core" sequences, single-stranded loops or single-stranded recognition sequences. These molecules are able to cleave (preferably, repeatedly cleave) separate RNA or DNA molecules in a nucleotide base sequence specific manner. Such catalytic nucleic acids can also act to cleave intramolecularly if that is desired. Such enzymatic molecules can be targeted to virtually any RNA or DNA transcript. This invention concerns nucleic acids formed of standard nucleotides or modified nucleotides, which also contain at least one 5'-dihalophosphonate and/or one 3'-dihalophosphonate group.

The synthesis of 1-*O*-Ac-2,3-di-*O*-Bz-D-ribofuranose 5-d-5+dihalomethylphosphonate in three steps from 1-*O*-methyl-2,3-*O*-isopropylidene-β-D-ribofuranose 5-deoxy-5-dihalomethylphosphonate is described (e.g., for the difluoro, in Figure 87). Condensation of this suitably derivatized sugar with silylated pyrimidines and purines affords novel nucleoside 5'-deoxy-5'-dihalomethylphosphonates. These intermediates may be incorporated into catalytic or antisense nucleic acids by either chemical (conversion of the nucleoside 5'-deoxy-5'-dihalomethylphosphonates into suitably protected phosphoramidites **12a** or solid supports **12b**, e.g., Figure 88) or enzymatic means (conversion of the nucleoside 5'-deoxy-5'-dihalomethylphosphonates into their triphosphates, e.g., **14** Figure 89, for T7 transcription).

Thus, in one aspect the invention features 5' and/or 3'-dihalonucleotides and nucleic acids containing such 5' and/or 3'-dihalonucleotides. The general structure of such molecules is shown below.

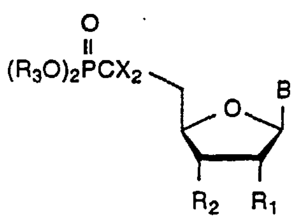
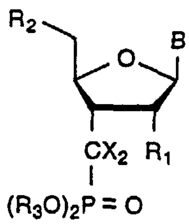
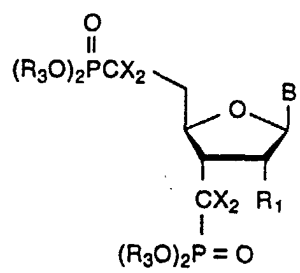

where $R_1$ is H, OH, or R, where R is a hydroxyl protecting group, e.g., acyl, alkysilyl, or carbonate; each $R_2$ is separately H, OH, or R; each $R_3$ is separately a phosphate protecting group, e.g., methyl, ethyl, cyanoethyl, p-nitrophenyl, or chlorophenyl; each X is separately any halogen; and each B is any nucleotide base.

The invention in particular features nucleic acid molecules having such modified nucleotides and enzymatic activity. In a related aspect the invention features a method for synthesis of such nucleoside 5'-deoxy-5'-dihalo and/or 3'-deoxy-3'-dihalophosphonates by condensing a dihalophosphonate-containing sugar with a pyrimidine or a purine under conditions suitable to form a nucleoside 5'-deoxy-5'-dihalophosphonate and/or a 3'-deoxy-3'-dihalophosphonate.

Phosphonic acids may exhibit important biological properties because of their similarity to phosphates (Engel, *Chem. Rev.* 1977, 77, 349-367). Blackburn and Kent (*J. Chem. Soc., Perkin Trans.* 1986, 913-917) indicate that based on electronic and steric considerations _-fluoro and _,_-difluoromethylphosphonates might mimic phosphate esters better than the corresponding phosphonates. Analogues of pyro- and triphosphates **1**, where the bridging oxygen atoms are replaced by a difluoromethylene group, have been employed as substrates in enzymatic processes (Blackburn *et al., Nucleosides & Nucleotides* 1985, 4, 165-167; Blackburn *et al., Chem. Scr.* 1986, 26, 21-24). 9-(5,5-Difluoro-5-phosphonopentyl)guanine (**2**) has been utilized as a multisubstrate analogue inhibitor of purine nucleoside phosphorylase (Halazy *et al., J. Am. Chem. Soc.* 1991, 113, 315-317). Oligonucleotides containing methylene groups in place of phosphodiester 5'-oxygens are resistant toward nucleases that cleave phosphodiester linkages between phosphorus and the 5'-oxygen (Breaker *et al., Biochemistry **1993**,* 32, 9125-9128), but can still form stable complexes with complementary sequences. Heinemann *et al. (Nucleic Acids Res.* 1991, 19, 427-433) found that a single 3'-methylenephosphonate linkage had a minor influence on the conformation of a DNA octamer double helix.

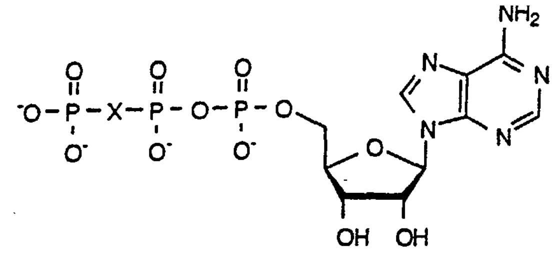

**1**

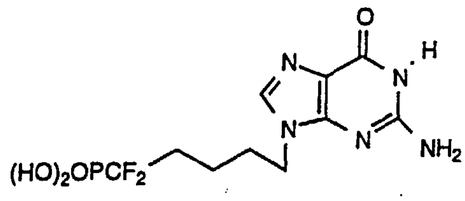

**2**

$(ETCO)_2POCF_2Li$

One common synthetic approach to α,α-difluoro-alkylphosphonates features the displacement of a leaving group from a suitable reactive substrate by diethyl (lithiodifluoromethyl)phosphonate (**3**) (Obayashi *et al., Tetrahedron Lett.* 1982, 23, 2323-2326). However, our attempts to synthesize nucleoside 5'-deoxy-5'-difluoro-methylphosphonates from 5'-deoxy-5'-iodonucleosides using **3** were unsuccessful, *i.e.* starting compounds were quantitatively recovered. The reaction of nucleoside 5'-aldehydes with **3**, according to the procedure of Martin *et al.* (Martin *et al.*, *Tetrahedron Lett.* 1992, 33, 1839-1842), led to a complex mixture of products. Recently, the synthesis of sugar α,α-difluoroalkylphosphonates from primary sugar triflates using **3** was described (Berkowitz *et al., J. Org. Chem.* 1993, 58, 6174-6176). Unfortunately, our experience is that nucleoside 5'-triflates are too unstable to be used in these syntheses.

The following are non-limiting examples showing the synthesis of nucleoside 5'-deoxy-5'-difluoromethyl-phosphonates. Those in the art will recognize that equivalent methods can be readily devised based upon these examples. These examples demonstrate that it is possible to achieve synthesis of 5'-deoxy-5'-difluoro derivatives in good yield and thus guide those in the art to such equivalent methods. The examples also indicate utility of such synthesis to provide useful oligonucleotides as described above.

Those in the art will recognize that useful modified enzymatic nucleic acids can now be designed, much as described by Draper et al., PCT/US94/13129 hereby incorporated by reference herein (including drawings).

Example 90: Synthesis of Nucleoside 5'-Deoxy-5'-difluoromethylphosphonates

Referring to Fig. 87, we synthesized a suitable glycosylating agent from the known D-ribose α,α-difluoromethylphosphonate (4) (Martin *et al.*, *Tetrahedron Lett.* 1992, 33, 1839-1842) which served as a key intermediate for the synthesis of nucleoside 5'-difluoromethylphosphonates.

Methyl 2,3-*O*-isopropylidene-β-D-ribofuranose a,a-difluoromethylphosphonate (**4**) was synthesized from the 5-aldehyde according to the procedure of Martin *et al.* *(Tetrahedron Lett.* **1992**, 33, 1839-1842) (Figure 87). Removal of the isopropylidene group was accomplished under mild conditions ($I_2$-MeOH, reflux, 18 h (Szarek *et al., Tetrahedron Lett.* **1986**, 27, 3827) or Dowex 50 WX8 ($H^+$), MeOH, RT (about 20-25°C), 3 days) in 72% yield. The anomeric mixture thus obtained was benzoylated with benzoyl chloride/pyridine to afford the 2,3-di-*O*-benzoyl derivative, which was subjected to mild acetolysis conditions (Walczak *et al., Synthesis,* 1993, 790-792) ($Ac_2O$, AcOH, $H_2SO_4$, EtOAc, 0°C.

The desired 1-*O*-acetyl-2,3-di-*O*-benzoyl-D-ribofuranose difluoromethylphosphonate **(5)** was obtained in quantitative yield as an anomeric mixture. These derivatives were used for selective glycosylation of silylated uracil and $N^4$-acetylcytosine under Vorbrüggen conditions (Vorbruggen, *Nucleoside Analogs. Chemistry, Biology and Medical Applications, NATO ASI Series A,* 26, Plenum Press, New York, London, 1980; pp. 35-69. The use of $F_3CSO_2OSi(CH_3)_3$ as a glycosylation catalyst is precluded because it is expected to lead to the undesired 1-ethyluracil or 9-ethyladenine byproducts: Podyukova, *et al., Tetrahedron Lett*. 1987, 28, 3623-3626 and references cited therein) ($SnCl_4$ as a catalyst, boiling acetonitrile) to yield β-nucleosides (62% **6a,** 75% **6b**). Glycosylation of silylated $N^6$-benzoyladenine under the same conditions yielded a mixture of N-9 isomer **6c** and N-7 isomer **7** in 34% and 15% yield, respectively. The above nucleotides were successfully deprotected using trimethylsilylbromide for the cleavage of the ethyl groups, followed by treatment with ammonia-methanol to remove the acyl protecting groups. Nucleoside 5'-deoxy-5'-difluoromethylphosphonates **8** were finally purified on a DEAE Sephadex A-25 ($HCO_3^-$) column using a 0.01-0.25 M TEAB gradient for elution and obtained as their sodium salts (82% **8a**; 87% **8b**; 82% **8c**).

Selected analytical data: $^{31}$P-NMR ($^{31}$P) and $^1$H-NMR ($^1$H) were recorded on a Varian Gemini 400. Chemical shifts in ppm refer to $H_3PO_4$ and TMS, respectively. Solvent was $CDCl_3$ unless otherwise noted. **5**: $^1$H δ 8.07-7.28 (m, Bz), 6.66 (d, $J_{1,2}$ 4.5, αH1), 6.42 (s, βH1), 5.74 (d, $J_{2,3}$ 4.9, βH2), 5.67 (dd, $J_{3,2}$ 4.9, $J_{3,4}$ 6.6, βH3), 5.63 (dd, $J_{3,2}$ 6.7, $J_{3,4}$ 3.6, αH3), 5.57 (dd, $J_{2.1}$ 4.5, $J_{2,3}$ 6.7, αH2), 4.91 (m, H4), 4.30 (m, $CH_2CH_3$), 2.64 (m, $CH_2CF_2$), 2.18 (s, βAc), 2.12 (s, αAc), 1.39 (m, $CH_2CH_3$). $^{31}$P δ 7.82 (t, $J_{P,F}$ 105.2), 7.67 (t, $J_{P,F}$ 106.5). **6a:** $^1$H δ 9.11 (s, 1H, NH), 8.01 (m, 11H, Bz, H6), 5.94 (d, $J_{1',2'}$ 4.1, 1H, H1'), 5.83 (dd, $J_{5,6}$ 8.1, 1H, H5), 5.79 (dd, $J_{2',1'}$ 4.1, $J_{2',3'}$ 6.5, 1H, H2'), 5.71 (dd, $J_{3',2'}$ 6.5, $J_{3',4'}$ 6.4, 1H, H3'), 4.79 (dd, $J_{4',3'}$ 6.4, $J_{4',F}$ 11.6, 1H, H4'), 4.31 (m, 4H, $CH_2CH_3$), 2.75 (tq, $J_{H,F}$ 19.6, 2H, $CH_2CF_2$), 1.40 (m, 6H, $CH_2CH_3$). $^{31}$P δ 7.77 (t, $J_{P,F}$ 104.0). **8c**: $^{31}$P (vs DSS) ($D_2O$) δ 5.71 (t, $J_{P,F}$ 87.9).

Compound **7** was deacylated with methanolic ammonia yielding the product that showed $\lambda_{max}$ ($H_2O$) 271 nm and $\lambda_{min}$ 233 nm, confirming that the site of glycosylation was N-7.

Example 91:Synthesis of Nucleic Acids Containing Modified Nucleotide Containing Cores

The method of synthesis used follows the procedure for normal RNA synthesis as described in Usman *et al., J. Am. Chem. Soc.* **1987**, *109,* 7845-7854 and in Scaringe *et al., Nucleic Acids Res*. **1990**, *18*, 5433-5441 and makes use of common nucleic acid protecting and coupling groups, such as dimethoxytrityl at the 5'-end, and phosphoramidites at the 3'-end (Figure 88 and Janda *et al., Science* **1989,** 244:437-440.). These nucleoside 5'-deoxy-5'-difluoromethylphosphonates may be incorporated not only into hammerhead ribozymes, but also into hairpin, hepatitis delta virus, Group 1 or Group 2 introns, or into antisense oligonucleotides. They are, therefore, of general use in any nucleic acid structure.

Example 92: Synthesis of Modified Triphosphate

The triphosphate derivatives of the above nucleotides can be formed as shown in Fig. 89, according to known procedures. *Nucleic Acid Chem.,* Leroy B. Townsend, John Wiley & Sons, New York 1991, pp. 337-340; *Nucleotide Analogs,* Karl Heinz Scheit; John Wiley & Sons New York 1980, pp. 211-218.

Equivalent synthetic schemes for 3' dihalophosphonates are shown in Figures 90 and 91 using art recognized nomenclature. The conditions can be optimized by standard procedures.

The nucleoside dihalophosphonates described herein are advantageous as modified nucleotides in any nucleic acid structure, e.g., catalytic or antisense, since they are resistant to exo- and endonucleases that normally degrade unmodified nucleic acids *in vivo.* They also do not perturb the normal structure of the nucleic acid in which they are incorporated thereby maintaining any activity associated with that structure. These compounds may also be of use as monomers as antiviral and/or antitumor drugs.

Oligonucleotides with Amido or Peptido Modification

This invention replaces 2'-hydroxyl group of a ribonucleotide moiety with a 2'-amido or 2'-peptido moiety. In other embodiments, the 3' and 5' portions of the sugar of a nucleotide may be substituted, or the phosphate group may be substituted with amido or peptido moieties. Generally, such a nucleotide has the general structure shown in Formula I below:

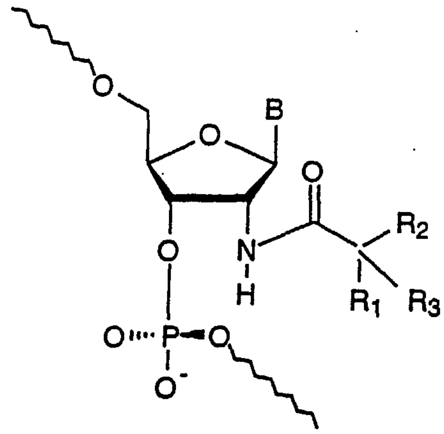

FORMULA I

The base (B) is any one of the standard bases or is a modified nucleotide base known to those in the art, or can be a hydrogen group. In addition, either $R_1$ or $R_2$ is H or an alkyl, alkene or alkyne group containing between 2 and 10 carbon atoms, or hydrogen, an amine (primary, secondary or tertiary, e.g., $R_3NR_4$ where each $R_3$ and $R_4$ independently is hydrogen or an alkyl, alkene or alkyne having between 2 and 10 carbon atoms, or is a residue of an amino acid, i.e., an amide), an alkyl group, or an amino acid (D or L forms) or peptide containing between 2 and 5 amino acids. The zigzag lines represent hydrogen, or a bond to another base or other chemical moiety known in the art. Preferably, one of $R_1$, R2 and $R_3$ is an H, and the other is an amino acid or peptide.

Applicant has recognized that RNA can assume a much more complex structural form than DNA because of the presence of the 2'-hydroxyl group in RNA. This group is able to provide additional hydrogen bonding with other hydrogen donors, acceptors and metal ions within the RNA molecule. Applicant now provides molecules which have a modified amine group at the 2' position, such that significantly more complex structures can be formed by the modified oligonucleotide. Such modification with a 2'-amido or peptido group leads to expansion and enrichment of the side-chain hydrogen bonding network. The amide and peptide moieties are responsible for complex structural formation of the oligonucleotide and can form strong complexes with other bases, and interfere with standard base pairing interactions. Such interference will allow the formation of a complex nucleic acid and protein conglomerate.

Oligonucleotides of this invention are significantly more stable than existing oligonucleotides and can potentially form biologically active bioconjugates not previously possible for oligonucleotides. They may also be used for *in vitro* selection of unique aptamers, that is, randomly generated oligonucleotides which can be folded into an effective ligand for a target protein, nucleic acid or polysaccharide.

Thus, in one aspect, the invention features an oligonucleotide containing the modified base shown in Formula I, above.

In other aspects, the oligonucleotide may include a 3' or 5' nucleotide having a 3' or 5' located amino acid or aminoacyl group. In all these aspects, as well as the 2'-modified nucleotide, it will be evident that various standard modifications can be made. For example, an "O" may be replaced with an S, the sugar may lack a base (i.e., abasic) and the phosphate moiety may be modified to include other substitutions (see Sproat, supra).

Example 93: General procedure for the preparation of 2'-aminoacyl-2'-deoxy-2'-aminonucleoside conjugates.

Referring to Fig. 92, to the solution of 2'-deoxy-2'-amino nucleoside (1 mmol) and N-Fmoc L- (or D-) amino acid (1 mmol) in methanol [dimethylformamide (DMF) and tetrahydrofuran (THF) can also be used], 1-ethoxycarbonyl-2-ethoxy-1,2-dihydroquinoline (EEDQ) [or 1-isobutyloxycarbonyl-2-isobutyloxy-1,2-dihydroquinoline (IIDQ)] (2 mmol) is added and the reaction mixture is stirred at room temperature or up to 50 °C from 3-48 hours. Solvents are removed under reduced pressure and the residual syrup is chromatographed on the column of silica-gel using 1-10 % methanol in dichloromethane. Fractions containing the product are concentrated yielding a white foam with yields ranging from 85 to 95 %. Structures are confirmed by $^1$H NMR spectra of conjugates which show correct chemical shifts for nucleoside and aminoacyl part of the molecule. Further proofs of the structures are obtained by cleaving the aminoacyl protecting groups under appropriate conditions and assigning $^1$H NMR resonances for the fully deprotected conjugate.

Partially protected conjugates described above are converted into their 5'-O-dimethoxytrityl derivatives and into 3'-phosphoramidites using standard procedures (Oligonucleotide Synthesis: A Practical Approach, M.J. Gait ed.;

IRL Press, Oxford, 1984). Incorporation of these phosphoramidites into RNA was performed using standard protocols (Usman *et al.,* 1987 *supra).*

A general deprotection protocol for oligonucleotides of the present invention is described in Fig. 93.

The scheme shows synthesis of conjugate of 2'-d-2'-aminouridine. This is meant to be a non-limiting example, and those skilled in the art will recognize that, variations to the synthesis protocol can be readily generated to synthesize other nucelotides (e.g., adenosine, cytidine, guanosine) and/or abasic moieties.

Example 94: RNA cleavage by hammerhead ribozymes containing 2'-aminoacyl modifications.

Hammerhead ribozymes targeted to site N (see Fig. 94) are synthesized using solid-phase synthesis, as described above. U4 and U7 positions are modified, individually or in combination, with either 2'-NH-alanine or 2'-NH-lysine.

RNA cleavage assay *in vitro*: Substrate RNA is 5' end-labeled using [$\gamma$-$^{32}$P] ATP and T4 polynucleotide kinase (US Biochemicals). Cleavage reactions were carried out under ribozyme "excess" conditions. Trace amount ($\leq$ 1 nM) of 5' end-labeled substrate and 40 nM unlabeled ribozyme are denatured and renatured separately by heating to 90°C for 2 min and snap-cooling on ice for 10-15 min. The ribozyme and substrate are incubated, separately, at 37°C for 10 min in a buffer containing 50 mM Tris-HCl and 10 mM $MgCl_2$. The reaction is initiated by mixing the ribozyme and substrate solutions and incubating at 37°C. Aliquots of 5 μl are taken at regular intervals of time and the reaction is quenched by mixing with equal volume of 2X formamide stop mix. The samples are resolved on 20 % denaturing polyacrylamide gels. The results are quantified and percentage of target RNA cleaved is plotted as a function of time.

Referring to Fig. 95, hammerhead ribozymes containing 2'-NH-alanine or 2'-NH-lysine modifications at U4 and U7 positions cleave the target RNA efficiently.

Sequences listed in Figure 94 and the modifications described in Figure 95 are meant to be non-limiting examples. Those skilled in the art will recognize that variants (base-substitutions, deletions, insertions, mutations, chemical modifications) of the ribozyme and RNA containing other 2'-hydroxyl group modifications, including but not limited to amino acids, peptides and cholesterol, can be readily generated using techniques known in the art, and are within the scope of the present invention.

Example 95: Aminoacylation of 3'-ends of RNA

I. Referring to Fig. 96, 3'-OH group of the nucleotide is converted to succinate as described by Gait, *supra*. This can be linked with amino-alkyl solid support (for example: CpG). Zig-zag line indicates linkage of 3'OH group with the solid support.

II. Preparation of aminoacyl-derivatized solid support

A) Synthesis of O-Dimethoxytrityl (O-DMT) amino acids

Referring to Fig. 97, to a solution of L- (or D-) serine, tyrosine or threonine (2 mmol) in dry pyridine (15 ml) 4,4'-dimethoxytrityl chloride (3 mmol) is added and the reaction mixture is stirred at RT (about 20-25°C) for 16 h. Methanol (10 ml) is then added and the solution evaporated under reduced pressure. The residual syrup was partitioned between 5% aq. '$NaHCO_3$ and dichloromethane, organic layer was washed with brine, dried ($Na_2SO_4$) and concentrated *in vacuo*. The residue is purified by flash silicagel column chromatography using 2-10% methanol in dichloromethane (containing 0.5 % pyridine). Fractions containing product are combined and concentrated *in vacuo* to yield white foam (75-85 % yield).

B) Preparation of the solid support and its derivatization with amino acids

Referring to Fig. 97, the modified solid support (has an OH group instead of the standard $NH_2$ end group) was prepared according to Haralambidis et al., Tetrahedron Lett. 1987, 28, 5199, (P denotes aminopropyl CPG or polystyrene type support). O-DMT or NH-monomethoxytrityl (NH-MMT amino acid was attached to the above solid support using standard procedures for derivatization of the solid support (Gait, 1984, supra) creating a base-labile ester bond between amino acids and the support. This support is suitable for the construction of RNA/DNA chain using suitably protected nucleoside phosphoramidites.

Example 96: Aminoacylation of 5'-ends of RNA

I. Referring to Fig. 98, 5'-amino-containing sugar moiety was synthesized as described (Mag and Engels, 1989 *Nucleic Acids Res.* 17, 5973). Aminoacylation of the 5'-end of the monomer was achieved as described above and RNA phosphoramidite of the 5'-aminoacylated monomer was prepared as described by Usman *et al*., 1987 *supra*. The phosphoramidite was then incorporated at the 5'-end of the oligonucleotide using standard solid-phase synthesis protocols described above.

II. Referring to Fig. 99, aminoacyl group(s) is attached to the phosphate group at the 5'-end of the RNA using standard procedures described above.

**VII. Reversing Genetic Mutations**

Modification of existing nucleic acid sequences can be achieved by homologous recombination. In this process a transfected sequence recombines with homologous chromosomal sequences and can replace the endogenous cellular sequence. Boggs, 8 *International J. Cell Cloning* 80, 1990, describes targeted gene modification. It reviews the use of homologous DNA recombination to correct genetic defects. Banga and Boyd, 89 *Proc. Natl. Acad. Sci. U.S.A.* 1735, 1992, describe a specific' example of *in vivo* site-directed mutagenesis using a 50 base oligonucleotide. In this methodology a gene or gene segment is essentially replaced by the oligonucleotide used.

This invention uses a complementary oligonucleotide to position a nucleotide base changing activity at a particular site on a gene (RNA or genomic DNA), such that the nucleotide modifying activity will change (or revert) a mutation to wild-type, or its equivalent. By reversion or change of a mutation, we refer to reversion in a broad sense, such as when a mutation at a second site which leads to functional reversion to a wild type phenotype. Also, due to the degeneracy of the genetic code, a revertant may be achieved by changing any one of the three codon positions. Additionally, creation of a stop codon in a deleterious gene (or transcript) is defined here as reverting a mutant phenotype to wild-type. An example of this type of reversion is creating a stop codon in a critical HIV proviral gene in a human.

Referring to Figures 100 and 101, broadly there are two approaches to causing a site directed change in order to revert a mutation to wild-type. In one (Fig. 100) the oligonucleotide is used to target RNA specifically. RNA is provided with a complementary (Watson-crick) oligonucleotide sequence to that in the target molecule. In this case the sequence modifying oligonucleotide would (analogously to an antisense oligonucleotide or ribozyme) have to be continuously present to revert the RNA as it is made by the cell. Such a reversion would be transient and would potentially require continuous addition of more sequence modifying oligonucleotide. The transient nature of this approach is an advantage, in that treatment could be stopped by simply removing the sequence modifying oligonucleotide (as with a traditional drug).

A second approach targets DNA (Fig. 101) and has the advantage that changes may be permanently encoded in the target cell's genetic code. Thus, a single course (or several courses) of treatment may lead to permanent reversion of the genetic disease. If inadvertent chromosomal mutations are introduced this may cause cancer, mutate other genes, or cause genetic changes in the germ-line (in patients of reproductive age). However, if the base changing activity is a specific methylation that may modulate gene expression it would not necessarily lead to germ-line transmission. See Lewin, Genes. 1983 John Wilely & Sons, Inc. NY pp 493-496.

Complementary base pairing to single-stranded DNA or RNA is one method of directing an oligonucleotide to a particular site of DNA. This could occur by a strand displacement mechanism or by targeting DNA when it is single-stranded (such as during replication, or transcription). Another method is using triple-strand binding (triplex formation) to double-stranded DNA, which is an established technique for binding poly-pyrimidine tracts, and can be extended to recognize all 4 nucleotides. See Povsic, T., Strobel, S., & Dervan, P. (1992). Sequence-specific double-strand alkylation and cleavage of DNA mediated by triple-helix formation. J. Am. Chem. Soc. **114**, 5934-5944 (1992). Knorre, D.G., Valentin, V.V., Valentina, F.Z., Lebedev, A.V. & Federova, O.S. *Design and targeted reactions of oligonucleotide derivatives* 1-366 (CRC Press, Novosibirsk, 1993) describe conjugation of reactive groups or enzyme to oligonucleotides and can be used in the methods described herein.

Recently, antisense oligonucleotides have been used to redirect an incorrect splice into order to obtain correct splicing of a splice mutant globin gene *in vitro.* Dominski Z; Kole R (1993) Restoration of correct splicing in thalassemia pre-mRNA by antisense oligonucleotides. Proc Natl Acad Sci USA 90:8673-7. Analogously, in one preferred embodiment of this invention a complementary oligomer is used to correct an existiing mutant RNA, instead of the traditional approach of inhibiting that RNA by antisense.

In either the RNA or DNA mode, after binding to a particular site on the RNA or DNA the oligonucleotide will

modify the nucleic acid sequence. This can be accomplished by activating an endogenous enzyme (see Figure 102), by appropriate positioning of an enzyme (or ribozyme) conjugated (or activated by the duplex) to the oligonucleotide, or by appropriate positioning of a chemical mutagen. Specific mutagens, such as nitrous acid which deaminates C to U, are most useful, but others can also be used if inactivation of a harmful RNA is desired.

RNA editing is an naturally occurring event in mammalian cells in which a sequence modifying activity edits a RNA to its proper sequence post-transcriptionally. Higuchi, M.,, Single, F., Kohler, M., Sommer, B., and Seeburg, P. (1993) RNA Editing of AMPA Receptor Subunit GluR-B: A base-paired intron-exon structure determines position and efficiency Cell 75:1361-1370. The machinery involved in RNA editing can be co-opted by a suitable oligonucleotide in order to promote chemical modification.

The changes in the base created by the methods of this invention cause a change in the nucleotide sequence, either directly, or after DNA repair by normal cellular mechanisms. These changes functionally correct a genetic defect or introduce a stop codon. Thus, the invention is distinct from techniques in which an active chemical group (*e.g.*, an alkylator) is attached to an antisense or triple strand oligonucleotide in order to chemically inactivate the target RNA or DNA.

Thus, this invention creates an alteration to an existing base in a nucleic acid molecule so that the base is read *in vivo* as a different base. This includes correcting a sequence instead of inactivating a gene but can also include inactivating a deleterious gene.

Thus, in one aspect, the invention features a method for altering in vivo the nucleotide base sequence of a naturally occurring mutant nucleic acid molecule. The method includes contacting the nucleic acid molecule in vivo with an oligonucleotide or peptide nucleic acid or other sequence specific binding molecules able to form a duplex or triplex molecule with the nucleic acid molecule. After formation of the duplex or triplex molecule a base modifying activity chemically or enzymatically alters the targeted base directly, or after nucleic acid repair *in vivo.* This results in the functional alteration of the nucleic acid sequence.

By "alter", as it is used in this context, is meant that one or more chemical moieties in a targeted base, or bases, is altered so that the mutant nucleic acid will be functionally different. Thus, this is distinct from prior methods of correcting defects in DNA, such as homologous recombination, in which an entire segment of the targeted sequence is replaced with a segment of DNA from the transfected nucleic acid. This is also distinct from other methods that use reactive groups to inactivate a RNA or DNA target, in that this method functionally corrects the sequence of the target, instead of merely damaging it, by causing it to be read by a polymerase as a different base from the original base. As noted above, the naturally occurring enzymes in a cell can be utilized to cause the chemical alteration, examples of which are provided below.

By "functionally alter" is meant that the ability of the target nucleic acid to perform its normal function (*i.e.*., transcription or translation control) is changed. For example, an RNA molecule may be altered so that it can cause production of a desired protein, or a DNA molecule can be altered so that upon DNA repair, the DNA sequence is changed.

By "mutant" it is meant a nucleic acid molecule which is altered in some way compared to equivalent molecules present in a normal individual. Such mutants may be well known in the art, and include, molecules present in individuals with known genetic deficiencies, such as muscular dystrophy, or diabetes and the like. It also includes individuals with diseases or conditions characterized by abnormal expression of a gene, such as cancer, thalassemia's and sickle cell anemia, and cystic fibrosis. It allows modulation of lipid metabolism to reduce artery disease, treatment of integrated AIDS genomes, and AIDs RNA, and Alzeimer's disease. Thus, this invention concems alteration of a base in a mutant to provide a "wild type" phenotype and/or genotype. For deleterious conditions this involves altering a base to allow expression or prevent expression as is necassary. When treating an infection, such as HIV, it concerns inactivation of a gene in the HIV RNA by mutation of the mutant (*i.e.*, non-human gene) to a wild type (*i.e.*, no production of a non-human protein). Such modification is performed *in trans* rather than *in cis* as in prior methods.

In preferred embodiments, the oligonucleotide is of a length (at least 12 bases, preferably 17 - 22) sufficient to activate dsRNA deaminase in vivo to cause conversion of an adenine base to inosine; the oligonucleotide is an enzymatic nucleic acid molecule that is active to chemically modify a base (see below); the nucleic acid molecule is DNA or RNA; the oligonucleotide includes a chemical mutagen, e.g., the mutagen is nitrous acid; and the oligonucleotide causes deamination of 5-methylcytosine to thymidine, cytosine to uracil, or adenine to inosine, or methtylation of cytosine to 5-methylcytosine.

In a most preferred embodiment, the invention features correction of a mutation, rather than inactivation of a target by causing a mutation.

Using *in vitro* directed evolution, it is possible to screen for ribozymes with catalytic activities different than RNA cleavage. Bartel, D. and Szostak, J. (1993) Isolation of new ribozymes from a large pool of random sequences. Science 261:1411-1418. Using these methods of *in vitro* directed evolution, an enzymatic nucleic acid molecule, or ribozyme that mutates bases, instead of cleaving the phosphodiester backbone can be selected. This is a convenient method of obtaining an enzyme with the appropriate base sequence modifying activities for use in the present invention.

Sequence modifying activities can change one nucleotide to another (or modify a nucleotide so that it will be repaired by the cellular machinery to another nucleotide). Sequence modifying activities could also delete or add one or more nucleotides to a sequence. A specific embodiment of adding sequences is described by Sullenger and Cech, PCT/US94/12976 hereby incorporated by reference herein), in which entire exons with wild-type sequence are spliced into a mutant transcript. The present invention features only the addition of a few bases (1 - 3).

Thus, in another aspect, the invention features ribozymes or enzymatic nucleic acid molecules active to change the chemical structure of an existing base in a separate nucleic acid molecule. Applicant is the first to determine that such molecules would be useful, and to provide a description of how such molecules might be isolated.

Molecules used to achieve *in situ* reversion can be delivered using the existing means employed for delivering antisense molecules and ribozymes, including liposomes and cationic lipid complexes. If the *in situ* reverting molecule is composed only of RNA, then expression vectors can be used in a gene therapy protocol to produce the reverting molecules endogenously, analogously to antisense or ribozymes expression vectors. There are several advantages of using such an expression vector, rather than simply replacing the gene through standard gene therapy. Firstly, this approach would limit the production of the corrected gene to cells that already express that gene. Furthermore, the corrected gene would be properly regulated by its natural transcriptional promoter. Lastly, reversion can be used when the mutant RNA creates a dominant gain of function protein (*e.g.*, in sickle cell anemia), where correction of the mutant RNA is necessary to stop the production of the deleterious mutant protein, and allow production of the corrected protein.

Endogenous Mammalian RNA Editing System

It was observed in the mid-1980s that the sequence of certain cellular RNAs were different from the DNA sequence that encodes them. By a process called RNA editing, cellular RNA are post-transcriptionally modified to a) create a translation initiation and termination codons, b) enable tRNA and rRNA to fold into a functional conformation (for a review see Bass, B. L. (1993) In The RNA World, R. Gesteland, R. and Atkins, J. eds. (Cold Spring Harbor, New York; CSH Lab. Press) pp. 383-418). The process of RNA editing includes base modification, deletion and insertion of nucleotides.

Although, the RNA editing process is widespread among lower eukaryotes, very few RNAs (four) have been reported to undergo editing in mammals (Bass, *supra*). The predominant mode of RNA editing in mammalian system is base modification (C $\rightarrow$ U and A $\rightarrow$ G). The mechanism of RNA editing in the mammalian system is postulated to be that C$\rightarrow$U conversion is catalyzed by cytidine deaminase. The mechanism of conversion of A$\rightarrow$G has recently been reported for glutamate receptor B subunit (gluR-B) in rat PC12 cells (Higuchi, M. et al. (1993) Cell 75, 1361-1370). According to Higuchi gluR-B mRNA precursor attains a structure such that intron 11 and exon 11 can form a stable stem-loop structure. This stem-loop structure is a substrate for a nuclear double strand-specific adenosine deaminase enzyme. The deamination will result in the conversion of A$\rightarrow$I. Reverse transcription followed by double strand synthesis will result in the incorporation of G in place of A.

In the present invention, the endogenous deaminase activity or other such activities can be utilized to achieve targeted base modification.

The following are examples of the invention to illustrate different methods by which *in vivo* conversion of a base can be achieved. These are provided only to clarify specific embodiments of the invention and are not limiting to the invention. Those in the art will recognize that equivalent methods can be readily devised within the scope of the claims.

Example 97: Exploiting cellular dsRNA dependent Adenine to Inosine converter:

An endogenous activity in most mammalian cells and Xenopus oocytes converts about 50% of adenines to inosines in double stranded RNA. (Bass, B. L., & Weintraub, H. (1988). An unwinding activity that covalently modifies it double-stranded RNA substrate. Cell, 55, 1089-1098.). This activity can be used to cause an *in situ* reversion of a mutation at the RNA level. Referring to Figures 102 and 104, for demonstration purposes a stop codon is incorporated into the coding region of dystrophin, which is fused to the reporter gene luciferase. This stop codon can be reverted by targeting an antisense RNA which is long enough to activate the dsRNA deaminase, which converts Adenines to Inosines. The A to I transition will be read by the ribosome as an A to G transition in some cases and will thereby functionally revert the stop codon. While other A's in this region may be converted to I's and read as G. converting an A to I (G) cannot create a stop codon. The A to I transitions in the region surrounding the target mutation will create some point mutations, however, the function of the dystrophin protein is rarely inactivated by point mutations.

The reverted mRNA was then translated in a cell lysate and assayed for luciferase activity. As evidenced by the dramatic increase in luciferase counts in the graph in figure 103, the A to I transition was read by the ribosome as an A to G transition and the stop codon has successfully been reverted with the lysate treated complex. As a control, an irrelevant non-complementary RNA oligonucleotide was added to the dystrophin/luciferase mRNA. As expected, in

this case no translation (luciferase activity) is observed because of the stop codon. As an additional control, the hybrid was not treated with extract, and again no translation (luciferase activity) is observed (Figure 103).

While other A's in the targeted region may have been converted to I's and read as G, converting an A to I (G) cannot create a stop codon, so the ribosome will still read through the region. Dystrophin is not generally sensitive to point mutations if the open reading frame is maintained, so a dystrophin protein made from an mRNA reverted by this method should retain full activity.

The following detail specifics of the methodology: RNA oligonucleotides were synthesized on a 394 (ABI) synthesizer using phosphoramidite chemistry. The sequence of the synthetic complementary RNA that binds to the mutant dystrophin sequence is as follows (5' to 3'):

CCCGCGGTAGATCTTTCTGGAGGCTTACAGTTTTCTACAAACCTCC
CTTCAAA (Seq. ID No. 1)

Referring to Figure 104. fifty-nine base pairs of a human dystrophin mutant sequence containing a stop codon was fused in frame to the luciferase coding region using standard cloning technology, into the *Hind* III and *Not I* sites of pRC-CMV (Invitrogen, San Diego, CA). The AUG of luciferase was deleted. The sequences of the insert from the *Hind* III site to the start of the luciferase coding region is (5' to 3'):

GCCCCTGAGGAGCGATGGAGGCCTTGAAGGGAGGTTTGTGGAAAA

CTGTAAGCCTCCAGAAAGATCTACCGCGG (Seq ID No. 2)

This corresponds to base pairs 3649-3708 of normal dystrophin (Entrez ID # 311627) with a *Sac* II site at the 3' end. This plasmid was used as a template for *in vitro* transcription of mRNA using T7 polymerase with the manufacturers protocol (Promega, Madison, WI).

*Xenopus* nuclear extracts were prepared in 0.5X TGKED buffer (0.5X= 25mM Tris (pH 7.9), 12.5% glycerol, 25 mM KCl, 0.25mM DTT and 0.05mM EDTA), by vortexing nuclei and resuspended in a volume of 0.5X TGKED equal to total cytoplasm volume of the oocytes. Bass, B.L. & Weintraub, H. *Cell 55,* 1089-1098 (1988).

The target mRNA at 500ng/ul was pre-annealed to 1 micromolar complementary or irrelevant RNA oligonucleotide by heating to 70°C, and allowing it to slowly cool to 37°C over 30 minutes. Fifty nanograms of mRNA pre-annealed to the RNA oligonucleotides was added to 7ul of nuclear extracts containing 1mM ATP, 15mM EDTA, 1600un/ml RNasin and 12.5mM Tris pH 8 to a total volume of 12ul. Bass, B.L. & Weintraub, H. *supra*. This mixture, which contains the dsRNA deaminase activity, was incubated for 30 minutes at 25°C. Next, 1.5ul of this mixture was added to a rabbit reticulocyte lysate *in vitro* translation mixture and translated for two hours according to the manufacturers protocol (Life Technologies, Gaithersberg, MD), except that an additional 1.3 mM magnesium acetate was added to compensate for the EDTA carried through from the nuclear extract mixture. Luciferase assays were performed on 15ul of extract with the Promega luciferase assay system (Promega, Madison, WI), and luminescence was detected with a 96 well luminometer, and the results are displayed in the graph in figure 102.

Example 98: Base changing activities

The chemical synthesis of antisense and triple-strand forming oligomers conjugated to reactive groups is well studied and characterized (Knorre, D.G., Valentin, V.V., Valentina, F.Z., Lebedev, A.V. & Federova, O.S. *Design and targeted reactions of oligonucleotide derivatives* 1-366 (CRC Press, Novosibirsk, 1993) and Povsic, T., Strobel, S. & Dervan, P. Sequence-specific double-strand alkylation and cleavage of DNA mediated by triple-helix formation *J. Am. Chem. Soc.* **114**, 5934-5944 (1992). Reactive groups such as alkylators that can modify nucleotide bases in targeted RNA or DNA have been conjugated to oligonucleotides. Additionally enzymes that modify nucleic acids have been conjugated to oligonucleotides. (Knorre, D.G., Valentin, V.V., Valentina, F.Z., Lebedev, A.V. & Federova, O.S. *Design and targeted reactions of oligonucleotide derivatives* 1-366 (CRC Press, Novosibirsk, 1993). In the past these conjugated chemical groups or enzymes have been used to inactivate DNA or RNA that is specifically targeted by antisense or triple-strand interactions. Below is a list of useful base changing activities that could be used to change the sequence

of DNA or RNA targeted by antisense or triple strand interactions, in order to achieve *in situ* reversion of mutations, as described herein (see figure 100-104).

1. Deamination of 5-methylcytosine to create thymidine (performed by the enzyme cytidine deaminase (Bass, B. L. in *The RNA World* (Cold Spring Harbor Laboratory Press, Cold Spring Harbor, 1993). Also, nitrous acid or related compounds promote oxidative deamination of C to be read at T(Microbial Genetics, David Freifelder, Jones and Bartlett Publishers, Inc., Boston,1987, PP.226-230.). Additionally hydroxylamine or related compounds can transform C to be read at T (Microbial Genetics, David Freifelder, Jones and Bartlett Publishers, Inc., Boston,1987, PP. 226-230.)

2. Deamination of cytosine to create uracil (performed by the enzyme cytidine deaminase (Bass, B.L. in *The RNA World* (Cold Spring Harbor Laboratory Press, Cold Spring Harbor, 1993) or by chemical groups similar to nitrous acid that promote oxidative deamination (Microbial Genetics, David Freifelder, Jones and Bartlett Publishers, Inc., Boston,1987, PP.226-230.)

3. Deamination of Adenine to be read like G (Inosine) (as done by the adenosine deaminase, AMP deaminase or the dsRNA deaminating activity ( Bass, B.L. in *The RNA World* (Cold Spring Harbor Laboratory Press, Cold Spring Harbor, 1993).

4. Methylation of cytosine to 5-methylcytosine

5. Transforming thymidine (or uracil) to $O^2$-methyl thymidine (or $O^2$-methyl uracil), to be read as cytosine by alkynitrosoureas (Xu, and Swann, Tetrahedron Letters 35:303-306 (1994)).

6. Transforming guanine to 6-*O*-methyl (or other alkyls) to be read as adenine (Mehta and Ludlum, Biochimica et Biophysica Acta, 521:770-778 (1978) which can be done with the mutagen ethyl methane sulfonate (EMS) Microbial Genetics, David Freifelder, Jones and Bartlett Publishers, Inc., Boston, 1987, PP.226-230.

7. Amination of uracil to cytosine (as performed by the cellular enzyme CTP synthetase (Bass, B.L. in *The RNA World* (Cold Spring Harbor Laboratory Press, Cold Spring Harbor, 1993).

The following are examples of useful chemical modifications that can be utilized in the present invention. There are a few preferred straightforward chemical modifications that can change one base to another base. Appropriate mutagenic chemicals are placed on the targetting oligonucleotide, *e.g.*, nitrous acid, or a suitable protein with such activity. Such chemicals and proteins can be attatched by standard procedures. These include molecules which introduce fundamental chemical changes, that would be useful independent of the particular technical approach. See Lewin, Genes, 1983 John Wilely & Sons, Inc. NY pp 42-48.

The following matrix shows that the chemical modifications noted can cause transversion reversions (pyrimidine to pyrimidine, or purine to purine) in RNA or DNA. The transversions (pyrimidine to purine, or purine to pyrimidine) are not preferred because these are more difficult chemical transformations. The footnotes refer to the specific desired chemical transformations. The bold footnotes refer to the reaction on the opposite DNA strand. For example, if one desires to change an A to a G, this can be accomplished at the DNA level by using reaction #5 to change a T to a C in the opposing strand. In this example an A/T base pair goes to A/C, then when the DNA is replicated, or mismatch repair occurs this can become G/C, thus the original A has been converted to a G.

## ISR matrix

### Reverted Base

| Mutant base | A | T(U) | C | G |
|---|---|---|---|---|

| | | | | |
|---|---|---|---|---|
| A | - | Transversion | Transversion | $DNA^{5,3}/RNA^{3}$ |
| T(U) | Transversion | - | $DNA^{5}/RNA^{7}$ | Transversion |
| C | Transversion | $RNA^{2}/DNA^{6}$ | - | Transversion |
| G | $DNA^{6}/RNA^{6}$ | Transversion | Transversion | - |

1 Deamination of 5-methylcytosine to create thymidine.

2 Deamination of cytosine to create uracil.

3 Deamination of Adenine to be read like G (Inosine).

4 Methylation of cytosine to 5-methylcytosine.

5 Transforming thymidine (or uracil) to $O^2$-methyl thymidine (or $O^2$-methyl uracil), to be read as cytosine (Xu, and Swann, Tetrahedron Letters 35:303-306 (1994)).

6 Transforming guanine to 6-*O*-methyl (or other alkyls) to be read as adenine (Mehta and Ludlum, Biochimica et Biophysica Acta, 521:770-778 (1978)).

7. Amination of uracil to cytosine. Bass *supra*. fig. 6c.

*In Vitro* Selection Strategy

Referring to Figure 105, there is provided a schematic describing an approach to selecting for a ribozyme with such base changing activity. An RNA is designed that folds back on itself (this is similar to approaches already used to select for RNA ligases, Bartel, D. and Szostak, J. (1993) Isolation of new ribozymes from a large pool of random sequences. Science 261:1411-1418). A degenerate loop opposing the base to be modified provides for diversity. After incubating this library of molecules in a buffer, the RNA is reverse transcribed into DNA (that is, using standard *in vitro* evolution protocol. Tuerk and Gold, 249 Science 505, 1990), and then the DNA is selected for having a base change. A restriction enzyme cleavage and size selection or its equivalent is used to isolate the fraction of DNAs with the appropriate base change. The cycle could then be repeated many times.

The *in vitro* selection (evolution) strategy is similar to approaches developed by Joyce (Beaudry, A. A. and Joyce, G.F. (1992) Science 257, 635-641; Joyce, G. F. (1992) Scientific American 267, 90-97) and Szostak (Bartel, D. and Szostak, J. (1993) Science 261:1411-1418; Szostak, J. W. (1993) TIBS 17, 89-93). Briefly, a random pool of nucleic acids is synthesized wherein, each member contains two domains: a) one domain consists of a region with defined (known) nucleotide sequence; b) the second domain consists of a region with degenerate (random) sequence. The known nucleotide sequence domain enables: 1) the nucleic acid to bind to its target (the region flanking the mutant nucleotide), 2) complimentary DNA (cDNA) synthesis and PCR amplification of molecules selected for their base modifying activity, 3) introduction of restriction endonuclease site for the purpose of cloning. The degenerate domain can be created to be completely random (each of the four nucleotides represented at every position within the random region) or the degeneracy can be partial (Beaudry, A. A. and Joyce, G.F. (1992) Science 257, 635-641). In this invention, the degenerate domain is flanked by regions containing known sequences (see Figure 105), such that the degenerate domain is placed across from the mutant base (the base that is targeted for modification). This random library of nucleic acids is incubated under conditions that ensure folding of the nucleic acids into conformations that facilitate the catalysis of base modification (the reaction protocol may also include certain cofactors like ATP or GTP or an S-adenosylmethionine (if methylation is desired) in order to make the selection more stringent). Following incubation, nucleic acids are converted into complimentary DNA (if the starting pool of nucleic acids is RNA). Nucleic acids with base modification (at the mutant base position) can be separated from rest of the population of nucleic acids by using a variety of methods. For example, a restriction endonuclease cleavage site can either be created or abolished as a result of base modification. If a restriction endonuclease site is created as a result of base modification, then the library can be digested

with the restriction endonuclease (RE). The fraction of the population that is cleaved by the RE is the population that has been able to catalyze the base modification reaction (active pool). A new piece of DNA (containing oligonucleotide primer binding sites for PCR and RE sites for cloning) is ligated to the termini of the active pool to facilitate PCR amplification and subsequent cycles (if necessary) of selection. The final pool of nucleic acids with the best base modifying activity is cloned in to a plasmid vector and transformed into bacterial hosts. Recombinant plasmids can then be isolated from transformed bacteria and the identity of clones can be determined using DNA sequencing techniques.

Base modifying enzymatic nucleic acids (identified via in vitro selection) can be used to cause the chemical modification *in vivo*.

In addition, the ribozyme could be evolved to specifically bind a protein having an enzymatic base changing acitivity.

Such ribozymes can be used to cause the above chemical modifications *in vivo*. The ribozymes or above noted antisense-type molecules can be administered by methods discussed in the above referenced art.

## VIII. Administration of Nucleic Acids

Applicant has determined that double-stranded nucleic acid lacking a transcription termination signal can be used for continuous expression of the encoded RNA. This is achieved by use of an R-loop, *i.e.,* an RNA molecule non-covalently associated with the double-stranded nucleic acid and which causes localized denaturation ("bubble" formation) within the double stranded nucleic acid (Thomas et al., 1976 Proc. Natl. Acad. Sci. USA 73, 2294). In addition, applicant has determined that that the RNA portion of the R-loop can be used to target the whole R-loop complex to a desirable intracellular or cellular site, and aid in cellular uptake of the complex. Further, applicant indicates that expression of enzymatically active RNA or ribozymes can be significantly enhanced by use of such R-loop complexes.

Thus, in one aspect, the invention features a method for introduction of enzymatic nucleic acid into a cell or tissue. A complex of a first nucleic acid encoding the enzymatic nucleic acid and a second nucleic acid molecule is provided. The second nucleic acid molecule has sufficient complementarity with the first nucleic acid to be able to form an R-loop base pair structure under physiological conditions. The R-loop is formed in a region of the first nucleic acid molecule which promotes expression of RNA from the first nucleic acid under physiological conditions. The method further includes contacting the complex with a cell or tissue under conditions in which the enzymatic nucleic acid is produced within the cell or tissue.

By "complex" is simply meant that the two nucleic acid molecules interact by intermolecular bond formation (such as by hydrogen bonding) between two complementary base-paired sequences. The complex will generally be stable under physiological condition such that it is able to cause initiation of transcription from the first nucleic acid molecule.

The first and second nucleic acid molecules may be formed from any desired nucleotide bases, either those naturally occurring (such as adenine, guanine, thymine and cytosine), or other bases well known in the art, or may have modifications at the sugar or phosphate moieties to allow greater stability or greater complex formation to be achieved. In addition, such molecules may contain non-nucleotides in place of nucleotides. Such modifications are well known in the art, see *e.g.,* Eckstein *et al.,* International Publication No. WO 92/07065; Perrault *et al.,* 1990 Nature 344, 565; Pieken *et al.,* 1991 Science, 253, 314; Usman and Cedergren, 1992 Trends in Biochem. Sci. 17, 334; Usman *et al.,* International Publication No. WO 93/15187; and Rossi *et al.,* International Publication No. WO 91/03162, as well as Sproat,B. *European Patent Application 92110298.4* which describe various chemical modifications that can be made to the sugar moieties of enzymatic RNA molecules. All these publications are hereby incorporated by reference herein.

By "sufficient complementarity" is meant that sufficient base pairing occurs so that the R-loop base pair structure can be formed under the appropriate conditions to cause transcription of the enzymatic nucleic acid. Those in the art will recognize routine tests by which such sufficient base pairs can be determined. In general, between about 15 - 80 bases is sufficient in this invention.

By "physiological condition" is meant the condition in the cell or tissue to be targeted by the first nucleic acid molecule, although the R-loop complex may be formed under many other conditions. One example is use of a standard physiological saline at 37°C, but it is simply desirable in this invention that the R-loop structure exists to some extent at the site of action so that the expression of the desired nucleic acid will be achieved at that site of action. While it is preferred that the R-loop structure be stable under those conditions, even a minimal amount of formation of the R-loop structure to cause expression will be sufficient. Those in the art will recognize that measurement of such expression is readily achieved, especially in the absence of any promoter or leader sequence on the first nucleic acid molecule (Daube and von Hippel, 1992 Science 258, 1320). Such expression can thus only be achieved if an R-loop structure is truly formed with the second nucleic acid. If a promoter of leader sequence is provided, then it is preferred that the R-loop be formed at a site distant from those regions so that transcription is enhanced.

In a related aspect, the invention features a method for introduction of ribonucleic acid within a cell or tissue

by forming an R-loop base-paired structure (as described above) with the first nucleic acid molecule lacking any promoter region or transcription termination signal such that once expression is initiated it will continue until the first nucleic acid is degraded.

In another related aspect, the invention features a method in which the second nucleic acid is provided with a localization factor, such as a protein, e.g., an antibody, transferin, a nuclear localization peptide, or folate, or other such compounds well known in the art, which will aid in targeting the R-loop complex to a desired cell or tissue.

In preferred embodiments, the first nucleic acid is a plasmid, *e.g.*, one without a promoter or a transcription termination signal ; the second nucleic acid is of length between about 40-200 bases and is formed of ribonucleotides at a majority of positions; and the second nucleic is covalently bonded with a ligand such as a nucleic acid, protein, peptide, lipid, carbohydrate, cellular receptor, nuclear localization factor, or is attached to maleimide or a thiol group: the first nucleic acid is an expression plasmid lacking a promoter able to express a desired gene, e.g., it is a double-stranded molecule formed with a majority of deoxyribonucleic acids; the R-loop complex is a RNA/DNA heteroduplex; no promoter or leader region is provided in the first nucleic acid; and the R-loop is adapted to prevent nucleosome assembly and is designed to aid recruitment of cellular transcription machinery.

In other preferred embodiments, the first nucleic acid encodes one or more enzymatic nucleic acids, *e.g.*, it is formed with a plurality of intramolecular and intermolecular cleaving enzymatic nucleic acids to allow release of therapeutic enzymatic nucleic acid *in vivo.*

In a further related aspect, the invention features a complex of the above first nucleic acid molecules and second nucleic acid molecules. R-loop complex

An R-loop complex is designed to provide a non-integrating plasmid so that, when an RNA polymerase binds to the plasmid, transcription is continuous until the plasmid is degraded. This is achieved by hybridizing an RNA molecule, 40 to 200 nucleotides in length, to a DNA expression plasmid resulting in an R-loop structure (see figure 106). This RNA, when conjugated with a ligand that binds to a cell surface receptor, triggers internalization of the plasmid/RNA-ligand complex. Formation of R-loops in general is described by DeWet, 1987 Methods in Enzymol. 145, 235; Neuwald et al., 1977 J. Virol. 21,1019; and Meyer et al., 1986 J. Ult. Mol. Str. Res. 96, 187. Thus, those in the art can readily design complexes of this invention following the teachings of the art.

Promoters placed in retroviral genomes have not always behaved as planned in that the additional promoter will serve as a stop signal or reverses the direction of the polymerase. Applicant was told that creation of an R-loop between the promoter and the reporter gene increased the transfection efficiency. Incubation of an RNA molecule with a double-stranded DNA molecule, containing a region of complementarity with the RNA will result in the formation of a stable RNA-DNA hetroduplex and the DNA strand that has a sequence identical to the RNA will be displaced into a loop-like structure called the R-loop. This displacement of DNA strand occurs because an RNA-DNA duplex is more stable compared to a DNA-DNA duplex. Applicant was also told that an 80 nt long RNA was used to generate a R-loop structure in a plasmid encoding the β-galactosidase gene. The R-loop was initiated either in the promoter region or in the leader sequence. Plasmids containing an R-loop structure were microinjected into the cytoplasm of COS cells and the gene expression was assayed. R-loop formation in the promoter region of the plasmid inhibited expression of the gene. RNA that hybridized to the leader sequence between the promoter and the gene, or directly to the first 80 nucleotides of the mRNA increased the expression levels 8-10 fold. The proposed mechanism is that R-loop formation prevents nucleosome assembly, thus making the DNA more accessible for transcription. Alternatively, the R-loop may resemble a RNA primer promoting either DNA replication or transcription (Daube and von Hippel, 1992, supra).

One of the salient features of this invention is to generate R-loops in expression vectors of choice and introduce them into cells to achieve enhanced expression from the expression vector. The presence of an R-loop may aid in the recruitment of cellular transcription machinery. Once an RNA polymerase binds to the plasmid and initiates transcription, the process will continue until a termination signal is reached, or the plasmid is degraded.

This invention will increase the expression of ribozymes inside a cell. The idea is to construct a plasmid with no transcription termination signal, such that a transcript-containing multiple ribozyme units can be generated. In order to liberate unit length ribozymes, self-processing ribozymes can be cloned downstream of each therapeutic ribozyme (see figure 107) as described by Draper *supra.*

## Ligand Targeting

Another salient feature of this invention is that the RNA used to generate R-loop structures can be covalently linked to a ligand (nucleic acid, proteins, peptides, lipids, carbohydrates, *etc.*). Specific ligands can be chosen such that the ligand can bind selectively to a desired cell surface receptor. This ligand-receptor interaction will help internalize a plasmid containing an R-loop. Thus, RNA is used to attach the ligand to the DNA such that localization of the gene to certain regions of the cell is achieved. One of several methods can be used to attach a ligand to RNA. This includes the incorporation of deoxythymidine containing a 6 carbon spacer having a terminal primary amine into the RNA (see figure 108). This amino group can be directly derivatized with the ligand, such as folate (Lee and Low, 1994 J. Biol.

Chem. 269, 3198-3204). The RNA containing a 6 carbon spacer with a terminal amine group is mixed with folate and the mixture is reacted with activators like 1-(3-Dimethylaminopropyl)-3-ethylcarbodiimide hydrochloride (EDC). This reaction should be carried out in the presence of 1-Hydroxybenzotriazole hydrate (HOBT) to prevent any undesirable side reactions.

The RNA can also be derivatized with a heterobifuctional crosslinking agent (or linker) like succinimidyl 4-(p-maleimidophenyl)butyrate (SMPB). The SMPB introduces a maleimide into the RNA. This maleimide can then react with a thiol moiety either in a peptide or in a protein. Thiols can also be introduced into proteins or peptides that lack naturally occurring thiols using succinylacetylthioacetate. The amino linker can be attached at the 5' end or 3' end of the RNA. The RNA can also contain a series of nucleotides that do not hybridize to the DNA and extend the linker away from the RNA/DNA complex, thus increasing the accessibility of the ligand for its receptor and not interfering with the hybridization. These techniques can be used to link peptides such as nuclear localization signal (NLS) peptides (Lanford et al., 1984 Cell 37, 801-813; Kalderon et al., 1984 Cell 39, 499-509; Goldfarb et al., 1986 Nature 322, 641-644) and/or proteins like the transferrin (Curiel et al., 1991 Proc. Natl. Acad. Sci, USA 88, 8850-8854; Wagner et al., 1992 Proc. Natl. Acad. Sci. USA 89, 6099-6103; Giulio et al., 1994 Cell. Signal. 6, 83-90) to the ends of R-loop forming RNA in order to facilitate the uptake and localization of the R-loop-DNA complex. To link a protein to the ends of R-loop forming RNA, an intrinsic thiol can be used to react with the maleimide or the thiols can be introduced into the protein itself using either iminothiolate or succinimidyl acetyl thioacetate (SATA; Duncan et al., 1983 Anal. Biochem 132, 68). The SATA requires an additional deprotection step using 0.5 M hydroxylamine.

In addition liposomes can be used to cause an R-loop complex to be delivered to an appropriate intracellular cite by techniques well known in the art. For example, pH-sensitive liposomes (Connor and Huang, 1986 Cancer Res. 46, 3431-3435) can be used to facilitate DNA transfection.

Calcium phosphate mediated or electroporation-mediated delivery of the R-loop complex in to desired cells can also be readily acomplished.

## *In vitro Selection*

*In vitro* selection strategies can be used to select nucleic acids that a) can form stable R-loops b) selectively bind to specific cell surface receptors. These nucleic acids can then be covalently linked to each other. This will help internalize the R-loop-containing plasmid efficiently using receptor-mediated endocytosis. The *in vitro* selection (evolution) strategy is similar to approaches developed by Joyce (Beaudry and Joyce, 1992 Science 257, 635-641; Joyce, 1992 Scientific American 267, 90-97) and Szostak (Bartel and Szostak, 1993 Science 261:1411-1418; Szostak, 1993 TIBS 17, 89-93). Briefly, a random pool of nucleic acids is synthesized wherein each member contains two domains: a) one domain consists of a region with defined (known) nucleotide sequence; b) the second domain consists of a region with degenerate (random) sequence. The known nucleotide sequence domain enables: 1) the nucleic acid to bind to its target (a specific region of the double strand DNA), 2) complimentary DNA (cDNA) synthesis and PCR amplification of molecules selected for their affinity to form R-loop and/or their ability to bind to a specific receptor, 3) introduction of a restriction endonuclease site for the purpose of cloning. The degenerate domain can be created to be completely random (each of the four nucleotides represented at every position within the random region) or the degeneracy can be partial (Beaudry and Joyce, 1992 Science 257, 635-641). In this invention, the degenerate domain is flanked by regions containing known sequences. This random library of nucleic acids is incubated under conditions that ensure equilibrium binding to either double-stranded DNA or cell surface receptor. Following incubation, nucleic acids are converted into complementary DNA (if the starting pool of nucleic acids is RNA). Nucleic acids with desired characteristics can be separated from the rest of the population of nucleic acids by using a variety of methods (Joyce, 1992 supra). The desired pool of nucleic acids can then be carried through subsequent rounds of selection to enrich the population with the most desired traits. These molecules are then cloned in to appropriate vectors. Recombinant plasmids can then be isolated from transformed bacteria and the identity of clones can be determined using DNA sequencing techniques.

Other embodiments are within the following claims.

TABLE I

Characteristics of Ribozymes

**Group I Introns**
Size: ~200 to >1000 nucleotides.
Requires a U in the target sequence immediately 5' of the cleavage site.
Binds 4-6 nucleotides at 5' side of cleavage site.
Over 75 known members of this class. Found in *Tetrahymena thermophila* rRNA, fungal mitochondria, chloroplasts, phage T4, blue-green algae, and others.

**RNAseP RNA (M1 RNA)**
Size: ~290 to 400 nucleotides.
RNA portion of a ribonucleoprotein enzyme. Cleaves tRNA precursors to form mature tRNA.
Roughly 10 known members of this group all are bacterial in origin.

**Hammerhead Ribozyme**
Size: ~13 to 40 nucleotides.
Requires the target sequence UH immediately 5' of the cleavage site.
Binds a variable number nucleotides on both sides of the cleavage site.
14 known members of this class. Found in a number of plant pathogens (virusoids) that use RNA as the infectious agent (Figures 1 and 2)

**Hairpin Ribozyme**
Size: ~50 nucleotides.
Requires the target sequence GUC immediately 3' of the cleavage site.
Binds 4-6 nucleotides at 5' side of the cleavage site and a variable number to the 3' side of the cleavage site.
Only 3 known member of this class. Found in three plant pathogen (satellite RNAs of the tobacco ringspot virus, **arabis mosaic virus and chicory yellow mottle virus**) which uses RNA as the infectious agent (Figure 3).

**Hepatitis Delta Virus (HDV) Ribozyme**
Size: 50 - 60 nucleotides (at present).
Cleavage of target RNAs recently demonstrated.
Sequence requirements not fully determined.
Binding sites and structural requirements not fully determined, although no sequences 5' of cleavage site are required.
Only 1 known member of this class. Found in human HDV (Figure 4).

***Neurospora* VS RNA Ribozyme**

Size: ~144 nucleotides (at present)

Cleavage of target RNAs recently demonstrated.
Sequence requirements not fully determined.
Binding sites and structural requirements not fully determined. Only 1 known member of this class. Found in *Neurospora* VS RNA (Figure 5).

Table 2
Human ICAM HH Target sequence

| nt. Position | Target Sequences | nt. Position | Target Sequences |
|---|---|---|---|
| 11 | CCCCAGU C GACGCUG | 386 | ACCGUGU A CUGGACU |
| 23 | CUGAGCU C CUCUGCU | 394 | CUGGACU C CAGAACG |
| 26 | AGCUCCU C UGCUACU | 420 | CACCCCU C CCCUCUU |
| 31 | CUCUGCU A CUCAGAG | 425 | CUCCCCU C UUGGCAG |
| 34 | UGCUACU C AGAGUUG | 427 | CCCCUCU U GGCAGCC |
| 40 | UCAGAGU U GCAACCU | 450 | AGAACCU U ACCCUAC |
| 48 | GCAACCU C AGCCUCG | 451 | GAACCUU A CCCUACG |
| 54 | UCAGCCU C GCUAUGG | 456 | UUACCCU A CGCUGCC |
| 58 | CCUCGCU A UGGCUCC | 495 | CCAACCU C ACCGUGG |
| 64 | UAUGGCU C CCAGCAG | 510 | UGCUGCU C CGUGGGG |
| 96 | CCGCACU C CUGGUCC | 564 | CUGAGGU C ACGACCA |
| 102 | UCCUGGU C CUGCUCG | 592 | GAGAGAU C ACCAUGG |
| 108 | UCCUGCU C GGGGCUC | 607 | AGCCAAU U UCUCGUG |
| 115 | CGGGGCU C UGUUCCC | 608 | GCCAAUU U CUCGUGC |
| 119 | GCUCUGU U CCCAGGA | 609 | CCAAUUU C UCGUGCC |
| 120 | CUCUGUU C CCAGGAC | 611 | AAUUUCU C GUGCCGC |
| 146 | CAGACAU C UGUGUCC | 656 | GAGCUGU U UGAGAAC |
| 152 | UCUGUGU C CCCCUCA | 657 | AGCUGUU U GAGAACA |
| 158 | UCCCCCU C AAAAGUC | 668 | AACACCU C GGCCCCC |
| 165 | CAAAAGU C AUCCUGC | 677 | GCCCCCU A CCAGCUC |
| 168 | AAGUCAU C CUGCCCC | 684 | ACCAGCU C CAGACCU |
| 185 | GGAGGCU C CGUGCUG | 692 | CAGACCU U UGUCCUG |
| 209 | AGCACCU C CUGUGAC | 693 | AGACCUU U GUCCUGC |
| 227 | CCCAAGU U GUUGGGC | 696 | CCUUUGU C CUGCCAG |
| 230 | AAGUUGU U GGGCAUA | 709 | AGCGACU C CCCCACA |
| 237 | UGGGCAU A GAGACCC | 720 | CACAACU U GUCAGCC |
| 248 | ACCCCGU U GCCUAAA | 723 | AACUUGU C AGCCCCC |
| 253 | GUUGCCU A AAAAGGA | 735 | CCCGGGU C CUAGAGG |
| 263 | AAGGAGU U GCUCCUG | 738 | GGGUCCU A GAGGUGG |
| 267 | AGUUGCU C CUGCCUG | 765 | CCGUGGU C UGUUCCC |
| 293 | AAGGUGU A UGAACUG | 769 | GGUCUGU U CCCUGGA |
| 319 | AGAAGAU A GCCAACC | 770 | GUCUGUU C CCUGGAC |
| 335 | AUGUGCU A UUCAAAC | 785 | GGGCUGU U CCCAGUC |
| 337 | GUGCUAU U CAAACUG | 786 | GGCUGUU C CCAGUCU |
| 338 | UGCUAUU C AAACUGC | 792 | UCCCAGU C UCGGAGG |
| 359 | GGGCAGU C AACAGCU | 794 | CCAGUCU C GGAGGCC |
| 367 | AACAGCU A AAACCUU | 807 | CCCAGGU C CACCUGG |
| 374 | AAAACCU U CCUCACC | 833 | CAGAGGU U GAACCCC |
| 375 | AAACCUU C CUCACCG | 846 | CCACAGU C ACCUAUG |
| 378 | CCUUCCU C ACCGUGU | 851 | GUCACCU A UGGCAAC |

| | | | |
|---|---|---|---|
| 863 | AACGACU C CUUCUCG | 1408 | UCGAGAU C UUGAGGG |
| 866 | GACUCCU U CUCGGCC | 1410 | GAGAUCU U GAGGGCA |
| 867 | ACUCCUU C UCGGCCA | 1421 | GGCACCU A CCUCUGU |
| 869 | UCCUUCU C GGCCAAG | 1425 | CCUACCU C UGUCGGG |
| 881 | AAGGCCU C AGUCAGU | 1429 | CCUCUGU C GGGCCAG |
| 885 | CCUCAGU C AGUGUGA | 1444 | GAGCACU C AAGGGGA |
| 933 | GUGCAGU A AUACUGG | 1455 | GGGAGGU C ACCCGCG |
| 936 | CAGUAAU A CUGGGGA | 1482 | AUGUGCU C UCCCCCC |
| 978 | UGACCAU C UACAGCU | 1484 | GUGCUCU C CCCCCGG |
| 980 | ACCAUCU A CAGCUUU | 1493 | CCCCGGU A UGAGAUU |
| 986 | UACAGCU U UCCGGCG | 1500 | AUGAGAU U GUCAUCA |
| 987 | ACAGCUU U CCGGCGC | 1503 | AGAUUGU C AUCAUCA |
| 988 | CAGCUUU C CGGCGCC | 1506 | UUGUCAU C AUCACUG |
| 1005 | ACGUGAU U CUGACGA | 1509 | UCAUCAU C ACUGUGG |
| 1006 | CGUGAUU C UGACGAA | 1518 | CUGUGGU A GCAGCCG |
| 1023 | CAGAGGU C UCAGAAG | 1530 | CCGCAGU C AUAAUGG |
| 1025 | GAGGUCU C AGAAGGG | 1533 | CAGUCAU A AUGGGCA |
| 1066 | CCACCCU A GAGCCAA | 1551 | CAGGCCU C AGCACGU |
| 1092 | AUGGGGU U CCAGCCC | 1559 | AGCACGU A CCUCUAU |
| 1093 | UGGGGUU C CAGCCCA | 1563 | CGUACCU C UAUAACC |
| 1125 | CCCAGCU C CUGCUGA | 1565 | UACCUCU A UAACCGC |
| 1163 | CGCAGCU U CUCCUGC | 1567 | CCUCUAU A ACCGCCA |
| 1164 | GCAGCUU C UCCUGCU | 1584 | GGAAGAU C AAGAAAU |
| 1166 | AGCUUCU C CUGCUCU | 1592 | AAGAAAU A CAGACUA |
| 1172 | UCCUGCU C UGCAACC | 1599 | ACAGACU A CAACAGG |
| 1200 | GCCAGCU U AUACACA | 1651 | CACGCCU C CCUGAAC |
| 1201 | CCAGCUU A UACACAA | 1661 | UGAACCU A UCCCGGG |
| 1203 | AGCUUAU A CACAAGA | 1663 | AACCUAU C CCGGGAC |
| 1227 | GGGAGCU U CGUGUCC | 1678 | AGGGCCU C UUCCUCG |
| 1228 | GGAGCUU C GUGUCCU | 1680 | GGCCUCU U CCUCGGC |
| 1233 | UUCGUGU C CUGUAUG | 1681 | GCCUCUU C CUCGGCC |
| 1238 | GUCCUGU A UGGCCCC | 1684 | UCUUCCU C GGCCUUC |
| 1264 | GAGGGAU U GUCCGGG | 1690 | UCGGCCU U CCCAUAU |
| 1267 | GGAUUGU C CGGGAAA | 1691 | CGGCCUU C CCAUAUU |
| 1294 | AGAAAAU U CCCAGCA | 1696 | UUCCCAU A UUGGUGG |
| 1295 | GAAAAUU C CCAGCAG | 1698 | CCCAUAU U GGUGGCA |
| 1306 | GCAGACU C CAAUGUG | 1737 | AAGACAU A UGCCAUG |
| 1321 | CCAGGCU U GGGGGAA | 1750 | UGCAGCU A CACCUAC |
| 1334 | AACCCAU U GCCCGAG | 1756 | UACACCU A CCGGCCC |
| 1344 | CCGAGCU C AAGUGUC | 1787 | AGGGCAU U GUCCUCA |
| 1351 | CAAGUGU C UAAAGGA | 1790 | GCAUUGU C CUCAGUC |
| 1353 | AGUGUCU A AAGGAUG | 1793 | UUGUCCU C AGUCAGA |
| 1366 | UGGCACU U UCCCACU | 1797 | CCUCAGU C AGAUACA |
| 1367 | GGCACUU U CCCACUG | 1802 | GUCAGAU A CAACAGC |
| 1368 | GCACUUU C CCACUGC | 1812 | ACAGCAU U UGGGGCC |
| 1380 | UGCCCAU C GGGGAAU | 1813 | CAGCAUU U GGGGCCA |
| 1388 | GGGGAAU C AGUGACU | 1825 | CCAUGGU A CCUGCAC |
| 1398 | UGACUGU C ACUCGAG | 1837 | CACACCU A AAACACU |
| 1402 | UGUCACU C GAGAUCU | 1845 | AAACACU A GGCCACG |

| | | | |
|---|---|---|---|
| 1856 | CACGCAU C UGAUCUG | 2189 | UAUUUAU U GAGUGUC |
| 1861 | AUCUGAU C UGUAGUC | 2196 | UGAGUGU C UUUUAUG |
| 1865 | GAUCUGU A GUCACAU | 2198 | AGUGUCU U UUAUGUA |
| 1868 | CUGUAGU C ACAUGAC | 2199 | GUGUCUU U UAUGUAG |
| 1877 | CAUGACU A AGCCAAG | 2200 | UGUCUUU U AUGUAGG |
| 1901 | CAAGACU C AAGACAU | 2201 | GUCUUUU A UGUAGGC |
| 1912 | ACAUGAU U GAUGGAU | 2205 | UUUAUGU A GGCUAAA |
| 1922 | UGGAUGU U AAAGUCU | 2210 | GUAGGCU A AAUGAAC |
| 1923 | GGAUGUU A AAGUCUA | 2220 | UGAACAU A GGCCUCU |
| 1928 | UUAAAGU C UAGCCUG | 2224 | CAUAGGU C UCUGGCC |
| 1930 | AAAGUCU A GCCUGAU | 2226 | UAGGUCU C UGGCCUC |
| 1964 | GAGACAU A GCCCCAC | 2233 | CUGGCCU C ACGGAGC |
| 1983 | AGGACAU A CAACUGG | 2242 | CGGAGCU C CCAGUCC |
| 1996 | GGGAAAU A CUGAAAC | 2248 | UCCCAGU C CAUGUCA |
| 2005 | UGAAACU U GCUGCCU | 2254 | UCCAUGU C ACAUUCA |
| 2013 | GCUGCCU A UUGGGUA | 2259 | GUCACAU U CAAGGUC |
| 2015 | UGCCUAU U GGGUAUG | 2260 | UCACAUU C AAGGUCA |
| 2020 | AUUGGGU A UGCUGAG | 2266 | UCAAGGU C ACCAGGU |
| 2039 | ACAGACU U ACAGAAG | 2274 | ACCAGGU A CAGUUGU |
| 2040 | CAGACUU A CAGAAGA | 2279 | GUACAGU U GUACAGG |
| 2057 | UGGCCCU C CAUAGAC | 2282 | CAGUUGU A CAGGUUG |
| 2061 | CCUCCAU A GACAUGU | 2288 | UACAGGU U GUACACU |
| 2071 | CAUGUGU A GCAUCAA | 2291 | AGGUUGU A CACUGCA |
| 2076 | GUAGCAU C AAAACAC | 2321 | AAAAGAU C AAAUGGG |
| 2097 | CCACACU U CCUGACG | 2338 | UGGGACU U CUCAUUG |
| 2098 | CACACUU C CUGACGG | 2339 | GGGACUU C UCAUUGG |
| 2115 | GCCAGCU U GGGCACU | 2341 | GACUUCU C AUUGGCC |
| 2128 | CUGCUGU C UACUGAC | 2344 | UUCUCAU U GGCCAAC |
| 2130 | GCUGUCU A CUGACCC | 2358 | CCUGCCU U UCCCCAG |
| 2145 | CAACCCU U GAUGAUA | 2359 | CUGCCUU U CCCCAGA |
| 2152 | UGAUGAU A UGUAUUU | 2360 | UGCCUUU C CCCAGAA |
| 2156 | GAUAUGU A UUUAUUC | 2376 | GAGUGAU U UUUCUAU |
| 2158 | UAUGUAU U UAUUCAU | 2377 | AGUGAUU U UUCUAUC |
| 2159 | AUGUAUU U AUUCAUU | 2378 | GUGAUUU U UCUAUCG |
| 2160 | UGUAUUU A UUCAUUU | 2379 | UGAUUUU U CUAUCGG |
| 2162 | UAUUUAU U CAUUUGU | 2380 | GAUUUUU C UAUCGGC |
| 2163 | AUUUAUU C AUUUGUU | 2382 | UUUUUCU A UCGGCAC |
| 2166 | UAUUCAU U UGUUAUU | 2384 | UUUCUAU C GGCACAA |
| 2167 | AUUCAUU U GUUAUUU | 2399 | AAGCACU A UAUGGAC |
| 2170 | CAUUUGU U AUUUUAC | 2401 | GCACUAU A UGGACUG |
| 2171 | AUUUGUU A UUUUACC | 2411 | GACUGGU A AUGGUUC |
| 2173 | UUGUUAU U UUACCAG | 2417 | UAAUGGU U CACAGGU |
| 2174 | UGUUAUU U UACCAGC | 2418 | AAUGGUU C ACAGGUU |
| 2175 | GUUAUUU U ACCAGCU | 2425 | CACAGGU U CAGAGAU |
| 2176 | UUAUUUU A CCAGCUA | 2426 | ACAGGUU C AGAGAUU |
| 2183 | ACCAGCU A UUUAUUG | 2433 | CAGAGAU U ACCCAGU |
| 2185 | CAGCUAU U UAUUGAG | 2434 | AGAGAUU A CCCAGUG |
| 2186 | AGCUAUU U AUUGAGU | 2448 | GAGGCCU U AUUCCUC |
| 2187 | GCUAUUU A UUGAGUG | 2449 | AGGCCUU A UUCCUCC |

| | | | |
|---|---|---|---|
| 2451 | GCCUUAU U CCUCCCU | 2750 | UAUGUGU A GACAAGC |
| 2452 | CCUUAUU C CUCCCUU | 2759 | ACAAGCU C UCGCUCU |
| 2455 | UAUUCCU C CCUUCCC | 2761 | AAGCUCU C GCUCUGU |
| 2459 | CCUCCCU U CCCCCCA | 2765 | UCUCGCU C UGUCACC |
| 2460 | CUCCCUU C CCCCCAA | 2769 | GCUCUGU C ACCCAGG |
| 2479 | GACACCU U UGUUAGC | 2797 | GUGCAAU C AUGGUUC |
| 2480 | ACACCUU U GUUAGCC | 2803 | UCAUGGU U CACUGCA |
| 2483 | CCUUUGU U AGCCACC | 2804 | CAUGGUU C ACUGCAG |
| 2484 | CUUUGUU A GCCACCU | 2813 | CUGCAGU C UUGACCU |
| 2492 | GCCACCU C CCCACCC | 2815 | GCAGUCU U GACCUUU |
| 2504 | CCCACAU A CAUUUCU | 2821 | UUGACCU U UUGGGCU |
| 2508 | CAUACAU U UCUGCCA | 2822 | UGACCUU U UGGGCUC |
| 2509 | AUACAUU U CUGCCAG | 2823 | GACCUUU U GGGCUCA |
| 2510 | UACAUUU C UGCCAGU | 2829 | UUGGGCU C AAGUGAU |
| 2520 | CCAGUGU U CACAAUG | 2837 | AAGUGAU C CUCCCAC |
| 2521 | CAGUGUU C ACAAUGA | 2840 | UGAUCCU C CCACCUC |
| 2533 | UGACACU C AGCGGUC | 2847 | CCCACCU C AGCCUCC |
| 2540 | CAGCGGU C AUGUCUG | 2853 | UCAGCCU C CUGAGUA |
| 2545 | GUCAUGU C UGGACAU | 2860 | CCUGAGU A GCUGGGA |
| 2568 | AGGGAAU A UGCCCAA | 2872 | GGACCAU A GGCUCAC |
| 2579 | CCAAGCU A UGCCUUG | 2877 | AUAGGCU C ACAACAC |
| 2585 | UAUGCCU U GUCCUCU | 2899 | GGCAAAU U UGAUUUU |
| 2588 | GCCUUGU C CUCUUGU | 2900 | GCAAAUU U GAUUUUU |
| 2591 | UUGUCCU C UUGUCCU | 2904 | AUUUGAU U UUUUUUU |
| 2593 | GUCCUCU U GUCCUGU | 2905 | UUUGAUU U UUUUUUU |
| 2596 | CUCUUGU C CUGUUUG | 2906 | UUGAUUU U UUUUUUU |
| 2601 | GUCCUGU U UGCAUUU | 2907 | UGAUUUU U UUUUUUU |
| 2602 | UCCUGUU U GCAUUUC | 2908 | GAUUUUU U UUUUUUU |
| 2607 | UUUGCAU U UCACUGG | 2909 | AUUUUUU U UUUUUUU |
| 2608 | UUGCAUU U CACUGGG | 2910 | UUUUUUU U UUUUUUU |
| 2609 | UGCAUUU C ACUGGGA | 2911 | UUUUUUU U UUUUUUU |
| 2620 | GGGAGCU U GCACUAU | 2912 | UUUUUUU U UUUUUUC |
| 2626 | UUGCACU A UUGCAGC | 2913 | UUUUUUU U UUUUUCA |
| 2628 | GCACUAU U GCAGCUC | 2914 | UUUUUUU U UUUUCAG |
| 2635 | UGCAGCU C CAGUUUC | 2915 | UUUUUUU U UUUCAGA |
| 2640 | CUCCAGU U UCCUGCA | 2916 | UUUUUUU U UUCAGAG |
| 2641 | UCCAGUU U CCUGCAG | 2917 | UUUUUUU U UCAGAGA |
| 2642 | CCAGUUU C CUGCAGU | 2918 | UUUUUUU U CAGAGAC |
| 2653 | CAGUGAU C AGGGUCC | 2919 | UUUUUUU C AGAGACG |
| 2659 | UCAGGGU C CUGCAAG | 2931 | ACGGGGU C UCGCAAC |
| 2689 | CCAAGGU A UUGGAGG | 2933 | GGGGUCU C GCAACAU |
| 2691 | AAGGUAU U GGAGGAC | 2941 | GCAACAU U GCCCAGA |
| 2700 | GAGGACU C CCUCCCA | 2951 | CCAGACU U CCUUUGU |
| 2704 | ACUCCCU C CCAGCUU | 2952 | CAGACUU C CUUUGUG |
| 2711 | CCCAGCU U UGGAAGG | 2955 | ACUUCCU U UGUGUUA |
| 2712 | CCAGCUU U GGAAGGG | 2956 | CUUCCUU U GUGUUAG |
| 2721 | GAAGGGU C AUCCGCG | 2961 | UUUGUGU U AGUUAAU |
| 2724 | GGGUCAU C CGCGUGU | 2962 | UUGUGUU A GUUAAUA |
| 2744 | UGUGUGU A UGUGUAG | 2965 | UGUUAGU U AAUAAAG |

| | |
|---|---|
| 2966 | GUUAGUU A AUAAAGC |
| 2969 | AGUUAAU A AAGCUUU |
| 2975 | UAAAGCU U UCUCAAC |
| 2976 | AAAGCUU U CUCAACU |
| 2977 | AAGCUUU C UCAACUG |
| 2979 | GCUUUCU C AACUGCC |

## Table 3

Mouse ICAM HH Target Sequence

| nt. Position | Target Sequence | nt. Position | Target Sequence |
|---|---|---|---|
| 11 | CCCugGU C acCGuUG | 367 | AAugGCU u cAACCcg |
| 23 | CaGuGgU u CUCUGCU | 374 | gAAgCCU U CCUgcCC |
| 26 | uGgUuCU C UGCUcCU | 375 | AAgCCUU C CUgcCCc |
| 31 | CUCUGCU c CUCcaca | 378 | CuacCaU C ACCGUGU |
| 34 | UuCUcaU a AGgGUcG | 386 | ACCGUGU A uUcGuuU |
| 40 | gCAcAcU U GuAgCCU | 394 | CcGGACU u ucGAuCu |
| 48 | aggACCU C AGCCUgG | 420 | CACaCuU C CCCcCcg |
| 54 | UggGCCU C GugAUGG | 425 | CaCCCCU C ccaGCAG |
| 58 | CaUgcCU u UaGCUCC | 427 | CagCUCU c aGCAGug |
| 64 | cAcccCU C CCAGCAG | 450 | AGgACCU c ACCCUgC |
| 96 | CucugCU C CUGGcCC | 451 | GAAaCcU u uCCUuuG |
| 102 | UgCcaGU a CUGCUgG | 456 | UUACCCU c aGCcaCu |
| 108 | cuCUGCU C cuGGCcC | 495 | CuAcCaU C ACCGUGu |
| 115 | uGGuuCU C UGcUCCu | 510 | UGCUGCU C CGUGGGG |
| 119 | GgaaUGU c aCCAGGA | 564 | CUcAGGU a uCcAuCc |
| 120 | CUCUGcU C CugGccC | 592 | GAaAGAU C ACaugGG |
| 146 | CAGuCgU C cGcuUCC | 607 | AGCCAAU U UCUCaUG |
| 152 | UCUGUGU C agCCaCu | 608 | GCCAAUU U CUCaUGC |
| 158 | UCCuguU u AAAAacC | 609 | CCAAUUU C UCaUGCC |
| 165 | CAgAAGU u gUuuUGC | 611 | AAUUUCU C aUGCCGC |
| 168 | AAGcCuU C CUGCCCC | 656 | aAGCUGU U UGAGcug |
| 185 | GGuGGgU C CGUGCaG | 657 | AGCUGUU U GAGcugA |
| 209 | gcCACuU C CUcUGgC | 668 | cgagCCU a GGCCaCC |
| 227 | CagAAGU U GUUuuGC | 677 | GaCCuCU A CCAGCcu |
| 230 | AAGUUGU U uuGCucc | 684 | uuCAGCU C CgGuCCU |
| 237 | UGuGCuU u GAGAaCu | 692 | CgGACuU U cGauCUu |
| 248 | AaCCCaU c uCCUAAA | 693 | AGgaCcU c acCCUGC |
| 253 | ccUGCCU A AggAaGA | 696 | CCUgUuU C CUGCCuc |
| 263 | AgGGuuU c uCUaCUG | 709 | gGCGgCU C CaCCuCA |
| 267 | AGggGCU C CUGCCUa | 720 | uACAACU U uUCAGCu |
| 293 | AAGcUGU u UGAgCUG | 723 | AACUUuU C AGCuCCg |
| 319 | AGgAGAU A cugAgCC | 735 | aCCaGaU C CUgGAGa |
| 335 | cUGUGCU u UgagAAC | 738 | uGGgCCU c GuGaUGG |
| 337 | GUcCaAU U CAcACUG | 765 | CaGUcGU C cGcUuCC |
| 338 | aGCUgUU u gAgCUGa | 769 | GGcCUGU U uCCUGcc |
| 359 | GuGCAGU C guCcGCU | 770 | uUuUGcU C CCUGGAa |
| 785 | GGcCUGU U uCCuGcC | 1353 | AGUGggU c gAaGgUG |
| 786 | GcCUGUU u CCuGcCU | 1366 | UaaCAgU c UaCaACU |
| 792 | UggagGU C UCGGAaG | 1367 | aGCACcU c CCCACcu |
| 794 | CugGgCU u GGAGaCu | 1368 | GuACUgU a CCACUcu |
| 807 | CuCgGaU a uACCUGG | 1380 | UGCCCAU C GGGGugg |
| 833 | CAaAGcU c GAcaCCC | 1388 | GGaGAcU C AGUGgCU |
| 846 | CCcugGU C ACCguUG | 1398 | UGgCUGU C ACagaAc |
| 851 | GagACCU c UacCAgC | 1402 | UGUgcuU u GAGAaCU |

| | |
|---|---|
| 863 | AgCcACU u CcUCUgG |
| 866 | GAagCCU U CcuGcCC |
| 867 | AuUCgUU u cCGGagA |
| 869 | UCuUcCU C augCAAG |
| 881 | AuGGCuU C AacCcGU |
| 885 | CCUugGU a gagGUGA |
| 933 | cUauAaU c AUuCUGG |
| 936 | uAaUcAU u CUGGuGc |
| 978 | UaACagU C UACAaCU |
| 980 | ACagUCU A CAaCUUU |
| 986 | UACAaCU U UuCaGCu |
| 987 | ACAaCUU U uCaGCuC |
| 988 | CAaCUUU u CaGCuCC |
| 1005 | ACcaGAU c CUGgaGA |
| 1006 | uGaGAgU C UGggGAA |
| 1023 | ugGAGGU C UCgGAAG |
| 1025 | GAGGUCU C gGAAGGG |
| 1066 | CCACuCU c aAaauAA |
| 1092 | AcuGGaU c uCAGgCC |
| 1093 | UGGaccU u CAGCCaA |
| 1125 | CCCAaCU C uUcuUGA |
| 1163 | CGaAGCU U CUuuUGC |
| 1164 | GaAGCUU C UuuUGCU |
| 1166 | AGCUUCU u uUGCUCU |
| 1172 | UCCUGuU u aaaAACC |
| 1200 | cuCuGCU c cUcCACA |
| 1201 | gCuGCUU u UgaACAg |
| 1203 | AcuUUuU u CACcAGu |
| 1227 | GGuAcaU a CGUGUgC |
| 1228 | GaAGCUU C uUuUgCU |
| 1233 | UUCGUuU C CgGagaG |
| 1238 | GUgCUGU A UGGuCCu |
| 1264 | GAaGGgU c GUgCaaG |
| 1267 | uGAgaGU C uGGGgAA |
| 1294 | AGgAgAU a CugAGCc |
| 1295 | GAggggU C uCAGCAG |
| 1306 | GCAGACU C ugAaaUG |
| 1321 | gaAGGCU c aGGaGgA |
| 1334 | AACCCAU c uCCuaAa |
| 1344 | auGAGCU C gAGaGUg |
| 1351 | ugAaUGU a UAAguuA |
| 1793 | UgGUCCU C gGcugGA |
| 1797 | CacCAGU C AcAUAaA |
| 1802 | acCAGAU c CuggAGa |
| 1812 | ACuGgAU c UcaGGCC |
| 1813 | CAGCAUU U acccuCA |
| 1825 | CCAcGcU A CCUcugC |
| 1837 | CAugCCU u uAgCuCc |
| 1845 | cgAgcCU A GGCCACc |

| | |
|---|---|
| 1408 | gCGAGAU C ggGgaGG |
| 1410 | GAGgUCU c GgaaGgg |
| 1421 | ccCACCU A CuUuUGU |
| 1425 | aCUgCCU u gGUaGaG |
| 1429 | uCUCUaU u GcCCCuG |
| 1444 | GAaggCU C AgGaGGA |
| 1455 | GGaAuGU C ACCaGga |
| 1482 | AguUGuU u UgCuCCC |
| 1484 | cUGuUCU u CCuCauG |
| 1493 | CuguGcU u UGAGAac |
| 1500 | AUGAaAU c aUggUCc |
| 1503 | gGAcUaU a AUCAUuc |
| 1506 | UUaUguU u AUaACcG |
| 1509 | cuAcCAU C ACcGUGu |
| 1518 | ucaUGGU c cCAGgCG |
| 1530 | CuauAaU C AUucUGG |
| 1533 | ugGUCAU u gUGGGCc |
| 1551 | CAuGCCU u AGCAgcU |
| 1559 | AGCACcU c CCcaccU |
| 1563 | CuUAugU u UAUAACC |
| 1565 | UAugUuU A UAACCGC |
| 1567 | ugUuUAU A ACCGCCA |
| 1584 | GaAAGAU C AgGAuAU |
| 1592 | AgGAuAU A CAaguUA |
| 1599 | ACAaguU A CAgaAGG |
| 1651 | CcCaCCU C CCUGAgC |
| 1661 | gaAACCU u UCCuuuG |
| 1663 | AACCUuU C CuuuGAa |
| 1678 | AGGaCCU C agCCUgG |
| 1680 | aGCCaCU U CCUCuGg |
| 1681 | GCCaCUU C CUCuGgC |
| 1684 | aCUUCCU C uGgCUgu |
| 1690 | cCGGaCU U uCgAUcU |
| 1691 | CGGaCUU u CgAUcUU |
| 1696 | UgCCCAU c ggGGUGG |
| 1698 | CggAUAU a ccUGGag |
| 1737 | gAGACcU c UaCCAgc |
| 1750 | gGCgGCU c CACCUca |
| 1756 | gAagCCU u CCuGCCC |
| 1787 | gaGaCAU U GUCCcCA |
| 1790 | GCAUUGU u CUCuaau |
| 2173 | UUagagU U UUACCAG |
| 2174 | UagagUU U UACCAGC |
| 2175 | agagUUU U ACCAGCU |
| 2176 | gagUUUU A CCAGCUA |
| 2183 | ACCAGCU A UUUAUUG |
| 2185 | CAGCUAU U UAUUGAG |
| 2186 | AGCUAUU U AUUGAGU |
| 2187 | GCUAUUU A UUGAGUa |

| | | | |
|---|---|---|---|
| 1856 | CggaCuU u cGAUCUu | 2189 | UAUUUAU U GAGUacC |
| 1861 | AcaUGAU a UccAGUa | 2196 | caAcUcU u cUUgAUG |
| 1865 | cAcuUGU A GcCuCAg | 2198 | gcaGcCU c UUAUGUu |
| 1868 | CaccAGU C ACAUaAa | 2199 | GccUCUU a UgUuUAu |
| 1877 | CAUGcCU u AGCagcu | 2200 | UcUuccU c AUGcAaG |
| 1901 | uAAaACU C AAGggAc | 2201 | aagUUUU A UGUcGGC |
| 1912 | AuAUagU a GAUcagU | 2205 | UUUAUGU c GGCcugA |
| 1922 | UGaAUGU a uAAGUua | 2210 | GgAGaCU c AgUGgcu |
| 1923 | uGAUGcU c AgGUaUc | 2220 | cuggCAU u GuUCUCU |
| 1928 | UUAgAGU u UuaCCaG | 2224 | CucAGGU a UCcauCC |
| 1930 | AgAGUuU u aCCaGcU | 2226 | UgGaUCU C aGGCCgC |
| 1964 | GAGACAU u GuCCCca | 2233 | CUGaCCU C cuGGAGg |
| 1983 | AGGAuAU A CAAgUua | 2242 | uGGAGCU a gCgGaCC |
| 1996 | aGGAgAU A CUGAgcC | 2248 | UauCcaU C CAUccCA |
| 2005 | UGgAgCU a GCgGaCc | 2254 | UCCAauU C ACAcUgA |
| 2013 | GCUauuU A UUGaGUA | 2259 | aUCACAU U CAcGGUg |
| 2015 | UGCCcAU c GGGgugG | 2260 | UCACAUU C AcGGUgc |
| 2020 | ggUGGuU c UuCUGAG | 2266 | ggAAuGU C ACCAGGa |
| 2039 | gCuGgCU a gCAGAgG | 2274 | ACCAGaU c CuGgaGa |
| 2040 | CuGACcU c CuGgAGg | 2279 | GaAggGU c GUgCAaG |
| 2057 | UGcuCCU C CAcAucC | 2282 | aAGcUGU u ugaGcUG |
| 2061 | CuaCCAU c acCgUGU | 2288 | UAuAaGU U aUggcCU |
| 2071 | CAcuUGU A GCcUCAg | 2291 | caGUgGU u CuCUGCu |
| 2076 | GUAGCcU C AgAgCua | 2321 | gAAAGAU C AcAUGGG |
| 2097 | CaACuCU U CuUGAuG | 2338 | UGaGACU c CUgccUG |
| 2098 | CACACUU C CcccCcG | 2339 | GaaACcU u UCcUUuG |
| 2115 | GCCAGCU c GGaggaU | 2341 | GACcUCU a ccaGcCu |
| 2128 | CaGCUaU u UAuUGAg | 2344 | UUucgAU c uuCCAgC |
| 2130 | cCUGUuU c CUGcCuC | 2358 | CCcagCU c UCagCAG |
| 2145 | CAACuCU U cuUGAUg | 2359 | CUGCuUU U gaaCAGA |
| 2152 | UauUaAU u UagAgUU | 2360 | aaCCUUU C CuuuGAA |
| 2156 | uugAUGU A UUUAUUa | 2376 | agGUGgU U cUUCUga |
| 2158 | gAUGUAU U UAUUaAU | 2377 | gGUGgUU c UUCUgag |
| 2159 | AUGUAUU U AUUaAUU | 2378 | agGgUUU c UCUAcuG |
| 2160 | UGUAUUU A UUaAUUU | 2379 | UGcUUUU c ucAUaaG |
| 2162 | UAUUUAU U aAUUUag | 2380 | aAgUUUU a UgUCGGC |
| 2163 | AUgUAUU u AUUaaUU | 2382 | aUUcUCU A UuGcCcC |
| 2166 | acUUCAU U cucUAUU | 2384 | aUcCagU a GaCACAA |
| 2167 | AUguAUU U aUUAaUU | 2399 | AAaCACU A UgUGGAC |
| 2170 | uAUUUaU U AaUUUAg | 2401 | aagCUgU u UGagCUG |
| 2171 | AgUUGUU u UgcUcCC | 2411 | uACUGGU c AgGaUgC |
| 2417 | gAAUGGU a CAuAcGU | 2691 | AAuGUcU c cGAGGcC |
| 2418 | AcUGGaU C uCAGGcc | 2700 | GAaGcCU u CCUgCCc |
| 2425 | CAugGGU c gAGgGuU | 2704 | gacCuCU a CCAGCcU |
| 2426 | AuuaaUU u AGAGuUU | 2711 | CCCAGCU c UcagcaG |
| 2433 | uAGAGuU U uaCCAGc | 2712 | gagGucU c GGAAGGG |
| 2434 | AGAGuUU u aCCAGcu | 2721 | GAAGGGU C gUgCaaG |
| 2448 | GAaGCCU U ccUgCcC | 2724 | GGuaCAU a CGuGUGc |
| 2449 | AaGCCUU c cUgCcCC | 2744 | gGUGgGU c cGUGcAG |

| | |
|---|---|
| 2451 | GCCUguU U CCUgCCU |
| 2452 | CCUguUU C CUgCCUc |
| 2455 | gAagCCU u CCUgCCC |
| 2459 | CCaCaCU U CCCCCCc |
| 2460 | CaCaCUU C CCCCCcg |
| 2479 | GAgACCU c UaccAGC |
| 2480 | uCACCgU U GUgAuCC |
| 2483 | CCaaUGU c AGCCACC |
| 2484 | CUUUuUU c aCCAguc |
| 2492 | agCACCU C CCCACCu |
| 2504 | CCCACcU A CuUUUgU |
| 2508 | uAUcCAU c caUcCCA |
| 2509 | uUAgAgU U uUaCCAG |
| 2510 | UAgAgUU u UaCCAGc |
| 2520 | CuuuUGU U CcCAAUG |
| 2521 | CAGcaUU u ACccUcA |
| 2533 | UGAugCU C AGguaUC |
| 2540 | CAGCaGU C cgcUgUG |
| 2545 | GUgcUGU a UGGuCcU |
| 2568 | guGaAgU c UGuCaAA |
| 2579 | auAAGuU A UGgCcUG |
| 2585 | cugGCaU U GUuCUCU |
| 2588 | GCaUUGU u CUCUaaU |
| 2591 | UgGUuCU C UgcUCCU |
| 2593 | cUuCUuU U GcuCUGc |
| 2596 | CUuUUGU u CccaaUG |
| 2601 | acCgUGU a UuCgUUU |
| 2602 | UCCaGcU a cCAUccC |
| 2607 | cUcGgAU a UacCUGG |
| 2608 | caGCAgU c CgCUGuG |
| 2609 | gGaAUgU C ACcaGGA |
| 2620 | aGGAcCU c aCcCUgc |
| 2626 | UUuCgaU c UUcCAGC |
| 2628 | GCACacU U GuAGCcu |
| 2635 | UuCAGCU C CgGUccu |
| 2640 | ggCCuGU U UCCUGCc |
| 2641 | cCCAGcU c uCaGCAG |
| 2642 | CCuGUUU C CUGCcuc |
| 2653 | uAcUGgU C AGGaUgC |
| 2659 | gaAGGGU C gUGCAAG |
| 2689 | CuAAuGU c UccGAGG |
| 2941 | GagACAU U GuCCccA |
| 2951 | CCAcgCU a CCUcUGc |
| 2952 | CAGcagU C CgcUGUG |
| 2955 | AgUgaCU c UGUGUcA |
| 2956 | uUUCCUU U GaaUcAa |
| 2961 | UcUGUGU c AGccAcU |
| 2962 | aUGUaUU u aUUAAUu |
| 2965 | UuUgAaU c AAUAAAG |
| 2750 | UAUuUaU u GAguAcC |
| 2759 | cCggaCU u UCGaUCU |
| 2761 | AgGacCU C aCcCUGc |
| 2765 | UuUuGCU C UGcCgCu |
| 2769 | agUCUGU C AaaCAGG |
| 2797 | aUGaAAU C AUGGUcC |
| 2803 | UCAUGGU c CcagGCg |
| 2804 | ggUGGgU C cgUGCAG |
| 2813 | CUcCgGU C cUGACCc |
| 2815 | aCAGUCU a cAaCUUU |
| 2821 | cUGACCU c cUGGagg |
| 2822 | gGAgCcU c cGGaCUu |
| 2823 | ugCCUUU a GcuCcCA |
| 2829 | cUGGaCU a uAaUcAU |
| 2837 | AgGUGgU u CUuCuga |
| 2840 | UGAgaCU C CugCCUg |
| 2847 | CCaAugU C AGCCaCC |
| 2853 | gCAGCCU C uUauGUu |
| 2860 | gCcaAGU A aCUGuGA |
| 2872 | GGACCuU c aGCcaAg |
| 2877 | uUccGCU a cCAuCAC |
| 2899 | cGgAcuU U cGAUcUU |
| 2900 | uuAAuUU a GAgUUUU |
| 2904 | AcUUcAU U cUcUaUU |
| 2905 | cUUcAUU c UcUaUUg |
| 2906 | UUGAUgU a UUUaUUa |
| 2907 | UGuaUUU a UUaaUUU |
| 2908 | GAagcUU c UUUUgcU |
| 2909 | AgcUUcU U UUgcUcU |
| 2910 | UgUaUUU a UUaaUUU |
| 2911 | UgUaUUU a UUaaUUU |
| 2912 | UUgUUcU c UaaUgUC |
| 2913 | UUUcUcU a cUggUCA |
| 2914 | UgcUUUU c UcaUaAG |
| 2915 | aUUUaUU a aUUuAGA |
| 2916 | UaUUcgU U UcCgGAG |
| 2917 | aUUcgUU U cCgGAGA |
| 2918 | UUcgUUU c CgGAGAg |
| 2919 | UUcUcaU a AGgGuCG |
| 2931 | ugGaGGU C UCGgAAg |
| 2933 | GaGGUCU C GgAAggg |

| | |
|---|---|
| 2966 | GcUgGcU A gcAgAGg |
| 2969 | AaUcAAU A AAGuUUU |
| 2975 | UAgAGuU U UacCAgC |
| 2976 | gAgGgUU U CUCuACU |
| 2977 | AAGCUgU u UgAgCUG |
| 2979 | uCaUUCU C uAuUGCC |

Table 4
Human ICAM HH Ribozyme Sequences

| nt. Position | Ribozyme Sequence |
|---|---|
| 11 | CAGCGUC CUGAUGAGGCCGAAAGGCCGAA ACUGGGG |
| 23 | AGCAGAG CUGAUGAGGCCGAAAGGCCGAA AGCUCAG |
| 26 | AGUAGCA CUGAUGAGGCCGAAAGGCCGAA AGGAGCU |
| 31 | CUCUGAG CUGAUGAGGCCGAAAGGCCGAA AGCAGAG |
| 34 | CAACUCU CUGAUGAGGCCGAAAGGCCGAA AGUAGCA |
| 40 | AGGUUGC CUGAUGAGGCCGAAAGGCCGAA ACUCUGA |
| 48 | CGAGGCU CUGAUGAGGCCGAAAGGCCGAA AGGUUGC |
| 54 | CCAUAGC CUGAUGAGGCCGAAAGGCCGAA AGGCUGA |
| 58 | GGAGCCA CUGAUGAGGCCGAAAGGCCGAA AGCGAGG |
| 64 | CUGCUGG CUGAUGAGGCCGAAAGGCCGAA AGCCAUA |
| 96 | GGACCAG CUGAUGAGGCCGAAAGGCCGAA AGUGCGG |
| 102 | CGAGCAG CUGAUGAGGCCGAAAGGCCGAA ACCAGGA |
| 108 | GAGCCCC CUGAUGAGGCCGAAAGGCCGAA AGCAGGA |
| 115 | GGGAACA CUGAUGAGGCCGAAAGGCCGAA AGCCCCG |
| 119 | UCCUGGG CUGAUGAGGCCGAAAGGCCGAA ACAGAGC |
| 120 | GUCCUGG CUGAUGAGGCCGAAAGGCCGAA AACAGAG |
| 146 | GGACACA CUGAUGAGGCCGAAAGGCCGAA AUGUCUG |
| 152 | UGAGGGG CUGAUGAGGCCGAAAGGCCGAA ACACAGA |
| 158 | GACUUUU CUGAUGAGGCCGAAAGGCCGAA AGGGGGA |
| 165 | GCAGGAU CUGAUGAGGCCGAAAGGCCGAA ACUUUUG |
| 168 | GGGGCAG CUGAUGAGGCCGAAAGGCCGAA AUGACUU |
| 185 | CAGCACG CUGAUGAGGCCGAAAGGCCGAA AGCCUCC |
| 209 | GUCACAG CUGAUGAGGCCGAAAGGCCGAA AGGUGCU |
| 227 | GCCCAAC CUGAUGAGGCCGAAAGGCCGAA ACUUGGG |
| 230 | UAUGCCC CUGAUGAGGCCGAAAGGCCGAA ACAACUU |
| 237 | GGGUCUC CUGAUGAGGCCGAAAGGCCGAA AUGCCCA |
| 248 | UUUAGGC CUGAUGAGGCCGAAAGGCCGAA ACGGGGU |
| 253 | UCCUUUU CUGAUGAGGCCGAAAGGCCGAA AGGCAAC |
| 263 | CAGGAGC CUGAUGAGGCCGAAAGGCCGAA ACUCCUU |
| 267 | CAGGCAG CUGAUGAGGCCGAAAGGCCGAA AGCAACU |
| 293 | CAGUUCA CUGAUGAGGCCGAAAGGCCGAA ACACCUU |
| 319 | GGUUGGC CUGAUGAGGCCGAAAGGCCGAA AUCUUCU |
| 335 | GUUUGAA CUGAUGAGGCCGAAAGGCCGAA AGCACAU |
| 337 | CAGUUUG CUGAUGAGGCCGAAAGGCCGAA AUAGCAC |
| 338 | GCAGUUU CUGAUGAGGCCGAAAGGCCGAA AAUAGCA |
| 359 | AGCUGUU CUGAUGAGGCCGAAAGGCCGAA ACUGCCC |
| 367 | AAGGUUU CUGAUGAGGCCGAAAGGCCGAA AGCUGUU |
| 374 | GGUGAGG CUGAUGAGGCCGAAAGGCCGAA AGGUUUU |
| 375 | CGGUGAG CUGAUGAGGCCGAAAGGCCGAA AAGGUUU |
| 378 | ACACGGU CUGAUGAGGCCGAAAGGCCGAA AGGAAGG |
| 386 | AGUCCAG CUGAUGAGGCCGAAAGGCCGAA ACACGGU |
| 394 | CGUUCUG CUGAUGAGGCCGAAAGGCCGAA AGUCCAG |
| 420 | AAGAGGG CUGAUGAGGCCGAAAGGCCGAA AGGGGUG |
| 425 | CUGCCAA CUGAUGAGGCCGAAAGGCCGAA AGGGGAG |

| | |
|---|---|
| 427 | GGCUGCC CUGAUGAGGCCGAAAGGCCGAA AGAGGGG |
| 450 | GUAGGGU CUGAUGAGGCCGAAAGGCCGAA AGGUUCU |
| 451 | CGUAGGG CUGAUGAGGCCGAAAGGCCGAA AAGGUUC |
| 456 | GGCAGCG CUGAUGAGGCCGAAAGGCCGAA AGGGUAA |
| 495 | CCACGGU CUGAUGAGGCCGAAAGGCCGAA AGGUUGG |
| 510 | CCCCACG CUGAUGAGGCCGAAAGGCCGAA AGCAGCA |
| 564 | UGGUCGU CUGAUGAGGCCGAAAGGCCGAA ACCUCAG |
| 592 | CCAUGGU CUGAUGAGGCCGAAAGGCCGAA AUCUCUC |
| 607 | CACGAGA CUGAUGAGGCCGAAAGGCCGAA AUUGGCU |
| 608 | GCACGAG CUGAUGAGGCCGAAAGGCCGAA AAUUGGC |
| 609 | GGCACGA CUGAUGAGGCCGAAAGGCCGAA AAAUUGG |
| 611 | GCGGCAC CUGAUGAGGCCGAAAGGCCGAA AGAAAUU |
| 656 | GUUCUCA CUGAUGAGGCCGAAAGGCCGAA ACAGCUC |
| 657 | UGUUCUC CUGAUGAGGCCGAAAGGCCGAA AACAGCU |
| 668 | GGGGGCC CUGAUGAGGCCGAAAGGCCGAA AGGUGUU |
| 677 | GAGCUGG CUGAUGAGGCCGAAAGGCCGAA AGGGGGC |
| 684 | AGGUCUG CUGAUGAGGCCGAAAGGCCGAA AGCUGGU |
| 692 | CAGGACA CUGAUGAGGCCGAAAGGCCGAA AGGUCUG |
| 693 | GCAGGAC CUGAUGAGGCCGAAAGGCCGAA AAGGUCU |
| 696 | CUGGCAG CUGAUGAGGCCGAAAGGCCGAA ACAAAGG |
| 709 | UGUGGGG CUGAUGAGGCCGAAAGGCCGAA AGUCGCU |
| 720 | GGCUGAC CUGAUGAGGCCGAAAGGCCGAA AGUUGUG |
| 723 | GGGGGCU CUGAUGAGGCCGAAAGGCCGAA ACAAGUU |
| 735 | CCUCUAG CUGAUGAGGCCGAAAGGCCGAA ACCCGGG |
| 738 | CCACCUC CUGAUGAGGCCGAAAGGCCGAA AGGACCC |
| 765 | GGGAACA CUGAUGAGGCCGAAAGGCCGAA ACCACGG |
| 769 | UCCAGGG CUGAUGAGGCCGAAAGGCCGAA ACAGACC |
| 770 | GUCCAGG CUGAUGAGGCCGAAAGGCCGAA AACAGAC |
| 785 | GACUGGG CUGAUGAGGCCGAAAGGCCGAA ACAGCCC |
| 786 | AGACUGG CUGAUGAGGCCGAAAGGCCGAA AACAGCC |
| 792 | CCUCCGA CUGAUGAGGCCGAAAGGCCGAA ACUGGGA |
| 794 | GGCCUCC CUGAUGAGGCCGAAAGGCCGAA AGACUGG |
| 807 | CCAGGUG CUGAUGAGGCCGAAAGGCCGAA ACCUGGG |
| 833 | GGGGUUC CUGAUGAGGCCGAAAGGCCGAA ACCUCUG |
| 846 | CAUAGGU CUGAUGAGGCCGAAAGGCCGAA ACUGUGG |
| 851 | GUUGCCA CUGAUGAGGCCGAAAGGCCGAA AGGUGAC |
| 863 | CGAGAAG CUGAUGAGGCCGAAAGGCCGAA AGUCGUU |
| 866 | GGCCGAG CUGAUGAGGCCGAAAGGCCGAA AGGAGUC |
| 867 | UGGCCGA CUGAUGAGGCCGAAAGGCCGAA AAGGAGU |
| 869 | CUUGGCC CUGAUGAGGCCGAAAGGCCGAA AGAAGGA |
| 881 | ACUGACU CUGAUGAGGCCGAAAGGCCGAA AGGCCUU |
| 885 | UCACACU CUGAUGAGGCCGAAAGGCCGAA ACUGAGG |
| 933 | CCAGUAU CUGAUGAGGCCGAAAGGCCGAA ACUGCAC |
| 936 | UCCCCAG CUGAUGAGGCCGAAAGGCCGAA AUUACUG |
| 978 | AGCUGUA CUGAUGAGGCCGAAAGGCCGAA AUGGUCA |
| 980 | AAAGCUG CUGAUGAGGCCGAAAGGCCGAA AGAUGGU |
| 986 | CGCCGGA CUGAUGAGGCCGAAAGGCCGAA AGCUGUA |
| 987 | GCGCCGG CUGAUGAGGCCGAAAGGCCGAA AAGCUGU |
| 988 | GGCGCCG CUGAUGAGGCCGAAAGGCCGAA AAAGCUG |

| | |
|---|---|
| 1005 | UCGUCAG CUGAUGAGGCCGAAAGGCCGAA AUCACGU |
| 1006 | UUCGUCA CUGAUGAGGCCGAAAGGCCGAA AAUCACG |
| 1023 | CUUCUGA CUGAUGAGGCCGAAAGGCCGAA ACCUCUG |
| 1025 | CCCUUCU CUGAUGAGGCCGAAAGGCCGAA AGACCUC |
| 1066 | UUGGCUC CUGAUGAGGCCGAAAGGCCGAA AGGGUGG |
| 1092 | GGGCUGG CUGAUGAGGCCGAAAGGCCGAA ACCCCAU |
| 1093 | UGGGCUG CUGAUGAGGCCGAAAGGCCGAA AACCCCA |
| 1125 | UCAGCAG CUGAUGAGGCCGAAAGGCCGAA AGCUGGG |
| 1163 | GCAGGAG CUGAUGAGGCCGAAAGGCCGAA AGCUGCG |
| 1164 | AGCAGGA CUGAUGAGGCCGAAAGGCCGAA AAGCUGC |
| 1166 | AGAGCAG CUGAUGAGGCCGAAAGGCCGAA AGAAGCU |
| 1172 | GGUUGCA CUGAUGAGGCCGAAAGGCCGAA AGCAGGA |
| 1200 | UGUGUAU CUGAUGAGGCCGAAAGGCCGAA AGCUGGC |
| 1201 | UUGUGUA CUGAUGAGGCCGAAAGGCCGAA AAGCUGG |
| 1203 | UCUUGUG CUGAUGAGGCCGAAAGGCCGAA AUAAGCU |
| 1227 | GGACACG CUGAUGAGGCCGAAAGGCCGAA AGCUCCC |
| 1228 | AGGACAC CUGAUGAGGCCGAAAGGCCGAA AAGCUCC |
| 1233 | CAUACAG CUGAUGAGGCCGAAAGGCCGAA ACACGAA |
| 1238 | GGGGCCA CUGAUGAGGCCGAAAGGCCGAA ACAGGAC |
| 1264 | CCCGGAC CUGAUGAGGCCGAAAGGCCGAA AUCCCUC |
| 1267 | UUUCCCG CUGAUGAGGCCGAAAGGCCGAA ACAAUCC |
| 1294 | UGCUGGG CUGAUGAGGCCGAAAGGCCGAA AUUUUCU |
| 1295 | CUGCUGG CUGAUGAGGCCGAAAGGCCGAA AAUUUUC |
| 1306 | CACAUUG CUGAUGAGGCCGAAAGGCCGAA AGUCUGC |
| 1321 | UUCCCCC CUGAUGAGGCCGAAAGGCCGAA AGCCUGG |
| 1334 | CUCGGGC CUGAUGAGGCCGAAAGGCCGAA AUGGGUU |
| 1344 | GACACUU CUGAUGAGGCCGAAAGGCCGAA AGCUCGG |
| 1351 | UCCUUUA CUGAUGAGGCCGAAAGGCCGAA ACACUUG |
| 1353 | CAUCCUU CUGAUGAGGCCGAAAGGCCGAA AGACACU |
| 1366 | AGUGGGA CUGAUGAGGCCGAAAGGCCGAA AGUGCCA |
| 1367 | CAGUGGG CUGAUGAGGCCGAAAGGCCGAA AAGUGCC |
| 1368 | GCAGUGG CUGAUGAGGCCGAAAGGCCGAA AAAGUGC |
| 1380 | AUUCCCC CUGAUGAGGCCGAAAGGCCGAA AUGGGCA |
| 1388 | AGUCACU CUGAUGAGGCCGAAAGGCCGAA AUUCCCC |
| 1398 | CUCGAGU CUGAUGAGGCCGAAAGGCCGAA ACAGUCA |
| 1402 | AGAUCUC CUGAUGAGGCCGAAAGGCCGAA AGUGACA |
| 1408 | CCCUCAA CUGAUGAGGCCGAAAGGCCGAA AUCUCGA |
| 1410 | UGCCCUC CUGAUGAGGCCGAAAGGCCGAA AGAUCUC |
| 1421 | ACAGAGG CUGAUGAGGCCGAAAGGCCGAA AGGUGCC |
| 1425 | CCCGACA CUGAUGAGGCCGAAAGGCCGAA AGGUAGG |
| 1429 | CUGGCCC CUGAUGAGGCCGAAAGGCCGAA ACAGAGG |
| 1444 | UCCCCUU CUGAUGAGGCCGAAAGGCCGAA AGUGCUC |
| 1455 | CGCGGGU CUGAUGAGGCCGAAAGGCCGAA ACCUCCC |
| 1482 | GGGGGGA CUGAUGAGGCCGAAAGGCCGAA AGCACAU |
| 1484 | CCGGGGG CUGAUGAGGCCGAAAGGCCGAA AGAGCAC |
| 1493 | AAUCUCA CUGAUGAGGCCGAAAGGCCGAA ACCGGGG |
| 1500 | UGAUGAC CUGAUGAGGCCGAAAGGCCGAA AUCUCAU |
| 1503 | UGAUGAU CUGAUGAGGCCGAAAGGCCGAA ACAAUCU |
| 1506 | CAGUGAU CUGAUGAGGCCGAAAGGCCGAA AUGACAA |

| | | | |
|---|---|---|---|
| 1509 | CCACAGU | CUGAUGAGGCCGAAAGGCCGAA | AUGAUGA |
| 1518 | CGGCUGC | CUGAUGAGGCCGAAAGGCCGAA | ACCACAG |
| 1530 | CCAUUAU | CUGAUGAGGCCGAAAGGCCGAA | ACUGCGG |
| 1533 | UGCCCAU | CUGAUGAGGCCGAAAGGCCGAA | AUGACUG |
| 1551 | ACGUGCU | CUGAUGAGGCCGAAAGGCCGAA | AGGCCUG |
| 1559 | AUAGAGG | CUGAUGAGGCCGAAAGGCCGAA | ACGUGCU |
| 1563 | GGUUAUA | CUGAUGAGGCCGAAAGGCCGAA | AGGUACG |
| 1565 | GCGGUUA | CUGAUGAGGCCGAAAGGCCGAA | AGAGGUA |
| 1567 | UGGCGGU | CUGAUGAGGCCGAAAGGCCGAA | AUAGAGG |
| 1584 | AUUUCUU | CUGAUGAGGCCGAAAGGCCGAA | AUCUUCC |
| 1592 | UAGUCUG | CUGAUGAGGCCGAAAGGCCGAA | AUUUCUU |
| 1599 | CCUGUUG | CUGAUGAGGCCGAAAGGCCGAA | AGUCUGU |
| 1651 | GUUCAGG | CUGAUGAGGCCGAAAGGCCGAA | AGGCGUG |
| 1661 | CCCGGGA | CUGAUGAGGCCGAAAGGCCGAA | AGGUUCA |
| 1663 | GUCCCGG | CUGAUGAGGCCGAAAGGCCGAA | AUAGGUU |
| 1678 | CGAGGAA | CUGAUGAGGCCGAAAGGCCGAA | AGGCCCU |
| 1680 | GCCGAGG | CUGAUGAGGCCGAAAGGCCGAA | AGAGGCC |
| 1681 | GGCCGAG | CUGAUGAGGCCGAAAGGCCGAA | AAGAGGC |
| 1684 | GAAGGCC | CUGAUGAGGCCGAAAGGCCGAA | AGGAAGA |
| 1690 | AUAUGGG | CUGAUGAGGCCGAAAGGCCGAA | AGGCCGA |
| 1691 | AAUAUGG | CUGAUGAGGCCGAAAGGCCGAA | AAGGCCG |
| 1696 | CCACCAA | CUGAUGAGGCCGAAAGGCCGAA | AUGGGAA |
| 1698 | UGCCACC | CUGAUGAGGCCGAAAGGCCGAA | AUAUGGG |
| 1737 | CAUGGCA | CUGAUGAGGCCGAAAGGCCGAA | AUGUCUU |
| 1750 | GUAGGUG | CUGAUGAGGCCGAAAGGCCGAA | AGCUGCA |
| 1756 | GGGCCGG | CUGAUGAGGCCGAAAGGCCGAA | AGGUGUA |
| 1787 | UGAGGAC | CUGAUGAGGCCGAAAGGCCGAA | AUGCCCU |
| 1790 | GACUGAG | CUGAUGAGGCCGAAAGGCCGAA | ACAAUGC |
| 1793 | UCUGACU | CUGAUGAGGCCGAAAGGCCGAA | AGGACAA |
| 1797 | UGUAUCU | CUGAUGAGGCCGAAAGGCCGAA | ACUGAGG |
| 1802 | GCUGUUG | CUGAUGAGGCCGAAAGGCCGAA | AUCUGAC |
| 1812 | GGCCCCA | CUGAUGAGGCCGAAAGGCCGAA | AUGCUGU |
| 1813 | UGGCCCC | CUGAUGAGGCCGAAAGGCCGAA | AAUGCUG |
| 1825 | GUGCAGG | CUGAUGAGGCCGAAAGGCCGAA | ACCAUGG |
| 1837 | AGUGUUU | CUGAUGAGGCCGAAAGGCCGAA | AGGUGUG |
| 1845 | CGUGGCC | CUGAUGAGGCCGAAAGGCCGAA | AGUGUUU |
| 1856 | CAGAUCA | CUGAUGAGGCCGAAAGGCCGAA | AUGCGUG |
| 1861 | GACUACA | CUGAUGAGGCCGAAAGGCCGAA | AUCAGAU |
| 1865 | AUGUGAC | CUGAUGAGGCCGAAAGGCCGAA | ACAGAUC |
| 1868 | GUCAUGU | CUGAUGAGGCCGAAAGGCCGAA | ACUACAG |
| 1877 | CUUGGCU | CUGAUGAGGCCGAAAGGCCGAA | AGUCAUG |
| 1901 | AUGUCUU | CUGAUGAGGCCGAAAGGCCGAA | AGUCUUG |
| 1912 | AUCCAUC | CUGAUGAGGCCGAAAGGCCGAA | AUCAUGU |
| 1922 | AGACUUU | CUGAUGAGGCCGAAAGGCCGAA | ACAUCCA |
| 1923 | UAGACUU | CUGAUGAGGCCGAAAGGCCGAA | AACAUCC |
| 1928 | CAGGCUA | CUGAUGAGGCCGAAAGGCCGAA | ACUUUAA |
| 1930 | AUCAGGC | CUGAUGAGGCCGAAAGGCCGAA | AGACUUU |
| 1964 | GUGGGGC | CUGAUGAGGCCGAAAGGCCGAA | AUGUCUC |
| 1983 | CCAGUUG | CUGAUGAGGCCGAAAGGCCGAA | AUGUCCU |

| | | | |
|---|---|---|---|
| 1996 | GUUUCAG | CUGAUGAGGCCGAAAGGCCGAA | AUUUCCC |
| 2005 | AGGCAGC | CUGAUGAGGCCGAAAGGCCGAA | AGUUUCA |
| 2013 | UACCCAA | CUGAUGAGGCCGAAAGGCCGAA | AGGCAGC |
| 2015 | CAUACCC | CUGAUGAGGCCGAAAGGCCGAA | AUAGGCA |
| 2020 | CUCAGCA | CUGAUGAGGCCGAAAGGCCGAA | ACCCAAU |
| 2039 | CUUCUGU | CUGAUGAGGCCGAAAGGCCGAA | AGUCUGU |
| 2040 | UCUUCUG | CUGAUGAGGCCGAAAGGCCGAA | AAGUCUG |
| 2057 | GUCUAUG | CUGAUGAGGCCGAAAGGCCGAA | AGGGCCA |
| 2061 | ACAUGUC | CUGAUGAGGCCGAAAGGCCGAA | AUGGAGG |
| 2071 | UUGAUGC | CUGAUGAGGCCGAAAGGCCGAA | ACACAUG |
| 2076 | GUGUUUU | CUGAUGAGGCCGAAAGGCCGAA | AUGCUAC |
| 2097 | CGUCAGG | CUGAUGAGGCCGAAAGGCCGAA | AGUGUGG |
| 2098 | CCGUCAG | CUGAUGAGGCCGAAAGGCCGAA | AAGUGUG |
| 2115 | AGUGCCC | CUGAUGAGGCCGAAAGGCCGAA | AGCUGGC |
| 2128 | GUCAGUA | CUGAUGAGGCCGAAAGGCCGAA | ACAGCAG |
| 2130 | GGGUCAG | CUGAUGAGGCCGAAAGGCCGAA | AGACAGC |
| 2145 | UAUCAUC | CUGAUGAGGCCGAAAGGCCGAA | AGGGUUG |
| 2152 | AAAUACA | CUGAUGAGGCCGAAAGGCCGAA | AUCAUCA |
| 2156 | GAAUAAA | CUGAUGAGGCCGAAAGGCCGAA | ACAUAUC |
| 2158 | AUGAAUA | CUGAUGAGGCCGAAAGGCCGAA | AUACAUA |
| 2159 | AAUGAAU | CUGAUGAGGCCGAAAGGCCGAA | AAUACAU |
| 2160 | AAAUGAA | CUGAUGAGGCCGAAAGGCCGAA | AAAUACA |
| 2162 | ACAAAUG | CUGAUGAGGCCGAAAGGCCGAA | AUAAAUA |
| 2163 | AACAAAU | CUGAUGAGGCCGAAAGGCCGAA | AAUAAAU |
| 2166 | AAUAACA | CUGAUGAGGCCGAAAGGCCGAA | AUGAAUA |
| 2167 | AAAUAAC | CUGAUGAGGCCGAAAGGCCGAA | AAUGAAU |
| 2170 | GUAAAAU | CUGAUGAGGCCGAAAGGCCGAA | ACAAAUG |
| 2171 | GGUAAAA | CUGAUGAGGCCGAAAGGCCGAA | AACAAAU |
| 2173 | CUGGUAA | CUGAUGAGGCCGAAAGGCCGAA | AUAACAA |
| 2174 | GCUGGUA | CUGAUGAGGCCGAAAGGCCGAA | AAUAACA |
| 2175 | AGCUGGU | CUGAUGAGGCCGAAAGGCCGAA | AAAUAAC |
| 2176 | UAGCUGG | CUGAUGAGGCCGAAAGGCCGAA | AAAAUAA |
| 2183 | CAAUAAA | CUGAUGAGGCCGAAAGGCCGAA | AGCUGGU |
| 2185 | CUCAAUA | CUGAUGAGGCCGAAAGGCCGAA | AUAGCUG |
| 2186 | ACUCAAU | CUGAUGAGGCCGAAAGGCCGAA | AAUAGCU |
| 2187 | CACUCAA | CUGAUGAGGCCGAAAGGCCGAA | AAAUAGC |
| 2189 | GACACUC | CUGAUGAGGCCGAAAGGCCGAA | AUAAAUA |
| 2196 | CAUAAAA | CUGAUGAGGCCGAAAGGCCGAA | ACACUCA |
| 2198 | UACAUAA | CUGAUGAGGCCGAAAGGCCGAA | AGACACU |
| 2199 | CUACAUA | CUGAUGAGGCCGAAAGGCCGAA | AAGACAC |
| 2200 | CCUACAU | CUGAUGAGGCCGAAAGGCCGAA | AAAGACA |
| 2201 | GCCUACA | CUGAUGAGGCCGAAAGGCCGAA | AAAAGAC |
| 2205 | UUUAGCC | CUGAUGAGGCCGAAAGGCCGAA | ACAUAAA |
| 2210 | GUUCAUU | CUGAUGAGGCCGAAAGGCCGAA | AGCCUAC |
| 2220 | AGAGACC | CUGAUGAGGCCGAAAGGCCGAA | AUGUUCA |
| 2224 | GGCCAGA | CUGAUGAGGCCGAAAGGCCGAA | ACCUAUG |
| 2226 | GAGGCCA | CUGAUGAGGCCGAAAGGCCGAA | AGACCUA |
| 2233 | GCUCCGU | CUGAUGAGGCCGAAAGGCCGAA | AGGCCAG |
| 2242 | GGACUGG | CUGAUGAGGCCGAAAGGCCGAA | AGCUCCG |

| | | | |
|---|---|---|---|
| 2248 | UGACAUG | CUGAUGAGGCCGAAAGGCCGAA | ACUGGGA |
| 2254 | UGAAUGU | CUGAUGAGGCCGAAAGGCCGAA | ACAUGGA |
| 2259 | GACCUUG | CUGAUGAGGCCGAAAGGCCGAA | AUGUGAC |
| 2260 | UGACCUU | CUGAUGAGGCCGAAAGGCCGAA | AAUGUGA |
| 2266 | ACCUGGU | CUGAUGAGGCCGAAAGGCCGAA | ACCUUGA |
| 2274 | ACAACUG | CUGAUGAGGCCGAAAGGCCGAA | ACCUGGU |
| 2279 | CCUGUAC | CUGAUGAGGCCGAAAGGCCGAA | ACUGUAC |
| 2282 | CAACCUG | CUGAUGAGGCCGAAAGGCCGAA | ACAACUG |
| 2288 | AGUGUAC | CUGAUGAGGCCGAAAGGCCGAA | ACCUGUA |
| 2291 | UGCAGUG | CUGAUGAGGCCGAAAGGCCGAA | ACAACCU |
| 2321 | CCCAUUU | CUGAUGAGGCCGAAAGGCCGAA | AUCUUUU |
| 2338 | CAAUGAG | CUGAUGAGGCCGAAAGGCCGAA | AGUCCCA |
| 2339 | CCAAUGA | CUGAUGAGGCCGAAAGGCCGAA | AAGUCCC |
| 2341 | GGCCAAU | CUGAUGAGGCCGAAAGGCCGAA | AGAAGUC |
| 2344 | GUUGGCC | CUGAUGAGGCCGAAAGGCCGAA | AUGAGAA |
| 2358 | CUGGGGA | CUGAUGAGGCCGAAAGGCCGAA | AGGCAGG |
| 2359 | UCUGGGG | CUGAUGAGGCCGAAAGGCCGAA | AAGGCAG |
| 2360 | UUCUGGG | CUGAUGAGGCCGAAAGGCCGAA | AAAGGCA |
| 2376 | AUAGAAA | CUGAUGAGGCCGAAAGGCCGAA | AUCACUC |
| 2377 | GAUAGAA | CUGAUGAGGCCGAAAGGCCGAA | AAUCACU |
| 2378 | CGAUAGA | CUGAUGAGGCCGAAAGGCCGAA | AAAUCAC |
| 2379 | CCGAUAG | CUGAUGAGGCCGAAAGGCCGAA | AAAAUCA |
| 2380 | GCCGAUA | CUGAUGAGGCCGAAAGGCCGAA | AAAAAUC |
| 2382 | GUGCCGA | CUGAUGAGGCCGAAAGGCCGAA | AGAAAAA |
| 2384 | UUGUGCC | CUGAUGAGGCCGAAAGGCCGAA | AUAGAAA |
| 2399 | GUCCAUA | CUGAUGAGGCCGAAAGGCCGAA | AGUGCUU |
| 2401 | CAGUCCA | CUGAUGAGGCCGAAAGGCCGAA | AUAGUGC |
| 2411 | GAACCAU | CUGAUGAGGCCGAAAGGCCGAA | ACCAGUC |
| 2417 | ACCUGUG | CUGAUGAGGCCGAAAGGCCGAA | ACCAUUA |
| 2418 | AACCUGU | CUGAUGAGGCCGAAAGGCCGAA | AACCAUU |
| 2425 | AUCUCUG | CUGAUGAGGCCGAAAGGCCGAA | ACCUGUG |
| 2426 | AAUCUCU | CUGAUGAGGCCGAAAGGCCGAA | AACCUGU |
| 2433 | ACUGGGU | CUGAUGAGGCCGAAAGGCCGAA | AUCUCUG |
| 2434 | CACUGGG | CUGAUGAGGCCGAAAGGCCGAA | AAUCUCU |
| 2448 | GAGGAAU | CUGAUGAGGCCGAAAGGCCGAA | AGGCCUC |
| 2449 | GGAGGAA | CUGAUGAGGCCGAAAGGCCGAA | AAGGCCU |
| 2451 | AGGGAGG | CUGAUGAGGCCGAAAGGCCGAA | AUAAGGC |
| 2452 | AAGGGAG | CUGAUGAGGCCGAAAGGCCGAA | AAUAAGG |
| 2455 | GGGAAGG | CUGAUGAGGCCGAAAGGCCGAA | AGGAAUA |
| 2459 | UGGGGGG | CUGAUGAGGCCGAAAGGCCGAA | AGGGAGG |
| 2460 | UUGGGGG | CUGAUGAGGCCGAAAGGCCGAA | AAGGGAG |
| 2479 | GCUAACA | CUGAUGAGGCCGAAAGGCCGAA | AGGUGUC |
| 2480 | GGCUAAC | CUGAUGAGGCCGAAAGGCCGAA | AAGGUGU |
| 2483 | GGUGGCU | CUGAUGAGGCCGAAAGGCCGAA | ACAAAGG |
| 2484 | AGGUGGC | CUGAUGAGGCCGAAAGGCCGAA | AACAAAG |
| 2492 | GGGUGGG | CUGAUGAGGCCGAAAGGCCGAA | AGGUGGC |
| 2504 | AGAAAUG | CUGAUGAGGCCGAAAGGCCGAA | AUGUGGG |
| 2508 | UGGCAGA | CUGAUGAGGCCGAAAGGCCGAA | AUGUAUG |
| 2509 | CUGGCAG | CUGAUGAGGCCGAAAGGCCGAA | AAUGUAU |

| | |
|---|---|
| 2510 | ACUGGCA CUGAUGAGGCCGAAAGGCCGAA AAAUGUA |
| 2520 | CAUUGUG CUGAUGAGGCCGAAAGGCCGAA ACACUGG |
| 2521 | UCAUUGU CUGAUGAGGCCGAAAGGCCGAA AACACUG |
| 2533 | GACCGCU CUGAUGAGGCCGAAAGGCCGAA AGUGUCA |
| 2540 | CAGACAU CUGAUGAGGCCGAAAGGCCGAA ACCGCUG |
| 2545 | AUGUCCA CUGAUGAGGCCGAAAGGCCGAA ACAUGAC |
| 2568 | UUGGGCA CUGAUGAGGCCGAAAGGCCGAA AUUCCCU |
| 2579 | CAAGGCA CUGAUGAGGCCGAAAGGCCGAA AGCUUGG |
| 2585 | AGAGGAC CUGAUGAGGCCGAAAGGCCGAA AGGCAUA |
| 2588 | ACAAGAG CUGAUGAGGCCGAAAGGCCGAA ACAAGGC |
| 2591 | AGGACAA CUGAUGAGGCCGAAAGGCCGAA AGGACAA |
| 2593 | ACAGGAC CUGAUGAGGCCGAAAGGCCGAA AGAGGAC |
| 2596 | CAAACAG CUGAUGAGGCCGAAAGGCCGAA ACAAGAG |
| 2601 | AAAUGCA CUGAUGAGGCCGAAAGGCCGAA ACAGGAC |
| 2602 | GAAAUGC CUGAUGAGGCCGAAAGGCCGAA AACAGGA |
| 2607 | CCAGUGA CUGAUGAGGCCGAAAGGCCGAA AUGCAAA |
| 2608 | CCCAGUG CUGAUGAGGCCGAAAGGCCGAA AAUGCAA |
| 2609 | UCCCAGU CUGAUGAGGCCGAAAGGCCGAA AAAUGCA |
| 2620 | AUAGUGC CUGAUGAGGCCGAAAGGCCGAA AGCUCCC |
| 2626 | GCUGCAA CUGAUGAGGCCGAAAGGCCGAA AGUGCAA |
| 2628 | GAGCUGC CUGAUGAGGCCGAAAGGCCGAA AUAGUGC |
| 2635 | GAAACUG CUGAUGAGGCCGAAAGGCCGAA AGCUGCA |
| 2640 | UGCAGGA CUGAUGAGGCCGAAAGGCCGAA ACUGGAG |
| 2641 | CUGCAGG CUGAUGAGGCCGAAAGGCCGAA AACUGGA |
| 2642 | ACUGCAG CUGAUGAGGCCGAAAGGCCGAA AAACUGG |
| 2653 | GGACCCU CUGAUGAGGCCGAAAGGCCGAA AUCACUG |
| 2659 | CUUGCAG CUGAUGAGGCCGAAAGGCCGAA ACCCUGA |
| 2689 | CCUCCAA CUGAUGAGGCCGAAAGGCCGAA ACCUUGG |
| 2691 | GUCCUCC CUGAUGAGGCCGAAAGGCCGAA AUACCUU |
| 2700 | UGGGAGG CUGAUGAGGCCGAAAGGCCGAA AGUCCUC |
| 2704 | AAGCUGG CUGAUGAGGCCGAAAGGCCGAA AGGGAGU |
| 2711 | CCUUCCA CUGAUGAGGCCGAAAGGCCGAA AGCUGGG |
| 2712 | CCCUUCC CUGAUGAGGCCGAAAGGCCGAA AAGCUGG |
| 2721 | CGCGGAU CUGAUGAGGCCGAAAGGCCGAA ACCCUUC |
| 2724 | ACACGCG CUGAUGAGGCCGAAAGGCCGAA AUGACCC |
| 2744 | CUACACA CUGAUGAGGCCGAAAGGCCGAA ACACACA |
| 2750 | GCUUGUC CUGAUGAGGCCGAAAGGCCGAA ACACAUA |
| 2759 | AGAGCGA CUGAUGAGGCCGAAAGGCCGAA AGCUUGU |
| 2761 | ACAGAGC CUGAUGAGGCCGAAAGGCCGAA AGAGCUU |
| 2765 | GGUGACA CUGAUGAGGCCGAAAGGCCGAA AGCGAGA |
| 2769 | CCUGGGU CUGAUGAGGCCGAAAGGCCGAA ACAGAGC |
| 2797 | GAACCAU CUGAUGAGGCCGAAAGGCCGAA AUUGCAC |
| 2803 | UGCAGUG CUGAUGAGGCCGAAAGGCCGAA ACCAUGA |
| 2804 | CUGCAGU CUGAUGAGGCCGAAAGGCCGAA AACCAUG |
| 2813 | AGGUCAA CUGAUGAGGCCGAAAGGCCGAA ACUGCAG |
| 2815 | AAAGGUC CUGAUGAGGCCGAAAGGCCGAA AGACUGC |
| 2821 | AGCCCAA CUGAUGAGGCCGAAAGGCCGAA AGGUCAA |
| 2822 | GAGCCCA CUGAUGAGGCCGAAAGGCCGAA AAGGUCA |
| 2823 | UGAGCCC CUGAUGAGGCCGAAAGGCCGAA AAAGGUC |

| | | | |
|---|---|---|---|
| 2829 | AUCACUU | CUGAUGAGGCCGAAAGGCCGAA | AGCCCAA |
| 2837 | GUGGGAG | CUGAUGAGGCCGAAAGGCCGAA | AUCACUU |
| 2840 | GAGGUGG | CUGAUGAGGCCGAAAGGCCGAA | AGGAUCA |
| 2847 | GGAGGCU | CUGAUGAGGCCGAAAGGCCGAA | AGGUGGG |
| 2853 | UACUCAG | CUGAUGAGGCCGAAAGGCCGAA | AGGCUGA |
| 2860 | UCCCAGC | CUGAUGAGGCCGAAAGGCCGAA | ACUCAGG |
| 2872 | GUGAGCC | CUGAUGAGGCCGAAAGGCCGAA | AUGGUCC |
| 2877 | GUGUUGU | CUGAUGAGGCCGAAAGGCCGAA | AGCCUAU |
| 2899 | AAAAUCA | CUGAUGAGGCCGAAAGGCCGAA | AUUUGCC |
| 2900 | AAAAAUC | CUGAUGAGGCCGAAAGGCCGAA | AAUUUGC |
| 2904 | AAAAAAA | CUGAUGAGGCCGAAAGGCCGAA | AUCAAAU |
| 2905 | AAAAAAA | CUGAUGAGGCCGAAAGGCCGAA | AAUCAAA |
| 2906 | AAAAAAA | CUGAUGAGGCCGAAAGGCCGAA | AAAUCAA |
| 2907 | AAAAAAA | CUGAUGAGGCCGAAAGGCCGAA | AAAAUCA |
| 2908 | AAAAAAA | CUGAUGAGGCCGAAAGGCCGAA | AAAAAUC |
| 2909 | AAAAAAA | CUGAUGAGGCCGAAAGGCCGAA | AAAAAAU |
| 2910 | AAAAAAA | CUGAUGAGGCCGAAAGGCCGAA | AAAAAAA |
| 2911 | AAAAAAA | CUGAUGAGGCCGAAAGGCCGAA | AAAAAAA |
| 2912 | GAAAAAA | CUGAUGAGGCCGAAAGGCCGAA | AAAAAAA |
| 2913 | UGAAAAA | CUGAUGAGGCCGAAAGGCCGAA | AAAAAAA |
| 2914 | CUGAAAA | CUGAUGAGGCCGAAAGGCCGAA | AAAAAAA |
| 2915 | UCUGAAA | CUGAUGAGGCCGAAAGGCCGAA | AAAAAAA |
| 2916 | CUCUGAA | CUGAUGAGGCCGAAAGGCCGAA | AAAAAAA |
| 2917 | UCUCUGA | CUGAUGAGGCCGAAAGGCCGAA | AAAAAAA |
| 2918 | GUCUCUG | CUGAUGAGGCCGAAAGGCCGAA | AAAAAAA |
| 2919 | CGUCUCU | CUGAUGAGGCCGAAAGGCCGAA | AAAAAAA |
| 2931 | GUUGCGA | CUGAUGAGGCCGAAAGGCCGAA | ACCCCGU |
| 2933 | AUGUUGC | CUGAUGAGGCCGAAAGGCCGAA | AGACCCC |
| 2941 | UCUGGGC | CUGAUGAGGCCGAAAGGCCGAA | AUGUUGC |
| 2951 | ACAAAGG | CUGAUGAGGCCGAAAGGCCGAA | AGUCUGG |
| 2952 | CACAAAG | CUGAUGAGGCCGAAAGGCCGAA | AAGUCUG |
| 2955 | UAACACA | CUGAUGAGGCCGAAAGGCCGAA | AGGAAGU |
| 2956 | CUAACAC | CUGAUGAGGCCGAAAGGCCGAA | AAGGAAG |
| 2961 | AUUAACU | CUGAUGAGGCCGAAAGGCCGAA | ACACAAA |
| 2962 | UAUUAAC | CUGAUGAGGCCGAAAGGCCGAA | AACACAA |
| 2965 | CUUUAUU | CUGAUGAGGCCGAAAGGCCGAA | ACUAACA |
| 2966 | GCUUUAU | CUGAUGAGGCCGAAAGGCCGAA | AACUAAC |
| 2969 | AAAGCUU | CUGAUGAGGCCGAAAGGCCGAA | AUUAACU |
| 2975 | GUUGAGA | CUGAUGAGGCCGAAAGGCCGAA | AGCUUUA |
| 2976 | AGUUGAG | CUGAUGAGGCCGAAAGGCCGAA | AAGCUUU |
| 2977 | CAGUUGA | CUGAUGAGGCCGAAAGGCCGAA | AAAGCUU |
| 2979 | GGCAGUU | CUGAUGAGGCCGAAAGGCCGAA | AGAAAGC |

Table 5

Mouse ICAM HH Ribozyme Sequence

| nt. Position | Ribozyme Sequence |
|---|---|
| 11 | CAACGGU CUGAUGAGGCCGAAAGGCCGAA ACCAGGG |
| 23 | AGCAGAG CUGAUGAGGCCGAAAGGCCGAA ACCACUG |
| 26 | AGGAGCA CUGAUGAGGCCGAAAGGCCGAA AGAACCA |
| 31 | UGUGGAG CUGAUGAGGCCGAAAGGCCGAA AGCAGAG |
| 34 | CGACCCU CUGAUGAGGCCGAAAGGCCGAA AUGAGAA |
| 40 | AGGCUAC CUGAUGAGGCCGAAAGGCCGAA AGUGUGC |
| 48 | CCAGGCU CUGAUGAGGCCGAAAGGCCGAA AGGUCCU |
| 54 | CCAUCAC CUGAUGAGGCCGAAAGGCCGAA AGGCCCA |
| 58 | GGAGCUA CUGAUGAGGCCGAAAGGCCGAA AGGCAUG |
| 64 | CUGCUGG CUGAUGAGGCCGAAAGGCCGAA AGGGGUG |
| 96 | GGGCCAG CUGAUGAGGCCGAAAGGCCGAA AGCAGAG |
| 102 | CCAGCAG CUGAUGAGGCCGAAAGGCCGAA ACUGGCA |
| 108 | GGGCCAG CUGAUGAGGCCGAAAGGCCGAA AGCAGAG |
| 115 | AGGAGCA CUGAUGAGGCCGAAAGGCCGAA AGAACCA |
| 119 | UCCUGGU CUGAUGAGGCCGAAAGGCCGAA ACAUUCC |
| 120 | GGGCCAG CUGAUGAGGCCGAAAGGCCGAA AGCAGAG |
| 146 | GGAAGCG CUGAUGAGGCCGAAAGGCCGAA ACGACUG |
| 152 | AGUGGCU CUGAUGAGGCCGAAAGGCCGAA ACACAGA |
| 158 | GGUUUUU CUGAUGAGGCCGAAAGGCCGAA AACAGGA |
| 165 | GCAAAAC CUGAUGAGGCCGAAAGGCCGAA ACUUCUG |
| 168 | GGGGCAG CUGAUGAGGCCGAAAGGCCGAA AAGGCUU |
| 185 | CUGCACG CUGAUGAGGCCGAAAGGCCGAA ACCCACC |
| 209 | GCCAGAG CUGAUGAGGCCGAAAGGCCGAA AAGUGGC |
| 227 | GCAAAAC CUGAUGAGGCCGAAAGGCCGAA ACUUCUG |
| 230 | GGAGCAA CUGAUGAGGCCGAAAGGCCGAA ACAACUU |
| 237 | AGUUCUC CUGAUGAGGCCGAAAGGCCGAA AAGCACA |
| 248 | UUUAGGA CUGAUGAGGCCGAAAGGCCGAA AUGGGUU |
| 253 | UCUUCCU CUGAUGAGGCCGAAAGGCCGAA AGGCAGG |
| 263 | CAGUAGA CUGAUGAGGCCGAAAGGCCGAA AAACCCU |
| 267 | UAGGCAG CUGAUGAGGCCGAAAGGCCGAA AGCCCCU |
| 293 | CAGCUCA CUGAUGAGGCCGAAAGGCCGAA ACAGCUU |
| 319 | GGCUCAG CUGAUGAGGCCGAAAGGCCGAA AUCUCCU |
| 335 | GUUCUCA CUGAUGAGGCCGAAAGGCCGAA AGCACAG |
| 337 | CAGUGUG CUGAUGAGGCCGAAAGGCCGAA AUUGGAC |
| 338 | UCAGCUC CUGAUGAGGCCGAAAGGCCGAA AACAGCU |
| 359 | AGCGGAC CUGAUGAGGCCGAAAGGCCGAA ACUGCAC |
| 367 | CGGGUUG CUGAUGAGGCCGAAAGGCCGAA AGCCAUU |
| 374 | GGGCAGG CUGAUGAGGCCGAAAGGCCGAA AGGCUUC |
| 375 | GGGGCAG CUGAUGAGGCCGAAAGGCCGAA AAGGCUU |
| 378 | ACACGGU CUGAUGAGGCCGAAAGGCCGAA AUGGUAG |
| 386 | AAACGAA CUGAUGAGGCCGAAAGGCCGAA ACACGGU |
| 394 | AGAUCGA CUGAUGAGGCCGAAAGGCCGAA AGUCCGG |
| 420 | CGGGGGG CUGAUGAGGCCGAAAGGCCGAA AAGUGUG |
| 425 | CUGCUGG CUGAUGAGGCCGAAAGGCCGAA AGGGGUG |

| | | | |
|---|---|---|---|
| 427 | CACUGCU | CUGAUGAGGCCGAAAGGCCGAA | AGAGCUG |
| 450 | GCAGGGU | CUGAUGAGGCCGAAAGGCCGAA | AGGUCCU |
| 451 | CAAAGGA | CUGAUGAGGCCGAAAGGCCGAA | AGGUUUC |
| 456 | AGUGGCU | CUGAUGAGGCCGAAAGGCCGAA | AGGGUAA |
| 495 | ACACGGU | CUGAUGAGGCCGAAAGGCCGAA | AUGGUAG |
| 510 | CCCCACG | CUGAUGAGGCCGAAAGGCCGAA | AGCAGCA |
| 564 | GGAUGGA | CUGAUGAGGCCGAAAGGCCGAA | ACCUGAG |
| 592 | CCCAUGU | CUGAUGAGGCCGAAAGGCCGAA | AUCUUUC |
| 607 | CAUGAGA | CUGAUGAGGCCGAAAGGCCGAA | AUUGGCU |
| 608 | GCAUGAG | CUGAUGAGGCCGAAAGGCCGAA | AAUUGGC |
| 609 | GGCAUGA | CUGAUGAGGCCGAAAGGCCGAA | AAAUUGG |
| 611 | GCGGCAU | CUGAUGAGGCCGAAAGGCCGAA | AGAAAUU |
| 656 | CAGCUCA | CUGAUGAGGCCGAAAGGCCGAA | ACAGCUU |
| 657 | UCAGCUC | CUGAUGAGGCCGAAAGGCCGAA | AACAGCU |
| 668 | GGUGGCC | CUGAUGAGGCCGAAAGGCCGAA | AGGCUCG |
| 677 | AGGCUGG | CUGAUGAGGCCGAAAGGCCGAA | AGAGGUC |
| 684 | AGGACCG | CUGAUGAGGCCGAAAGGCCGAA | AGCUGAA |
| 692 | AAGAUCG | CUGAUGAGGCCGAAAGGCCGAA | AAGUCCG |
| 693 | GCAGGGU | CUGAUGAGGCCGAAAGGCCGAA | AGGUCCU |
| 696 | GAGGCAG | CUGAUGAGGCCGAAAGGCCGAA | AAACAGG |
| 709 | UGAGGUG | CUGAUGAGGCCGAAAGGCCGAA | AGCCGCC |
| 720 | AGCUGAA | CUGAUGAGGCCGAAAGGCCGAA | AGUUGUA |
| 723 | CGGAGCU | CUGAUGAGGCCGAAAGGCCGAA | AAAAGUU |
| 735 | UCUCCAG | CUGAUGAGGCCGAAAGGCCGAA | AUCUGGU |
| 738 | CCAUCAC | CUGAUGAGGCCGAAAGGCCGAA | AGGCCCA |
| 765 | GGAAGCG | CUGAUGAGGCCGAAAGGCCGAA | ACGACUG |
| 769 | GGCAGGA | CUGAUGAGGCCGAAAGGCCGAA | ACAGGCC |
| 770 | UUCCAGG | CUGAUGAGGCCGAAAGGCCGAA | AGCAAAA |
| 785 | GGCAGGA | CUGAUGAGGCCGAAAGGCCGAA | ACAGGCC |
| 786 | AGGCAGG | CUGAUGAGGCCGAAAGGCCGAA | AACAGGC |
| 792 | CUUCCGA | CUGAUGAGGCCGAAAGGCCGAA | ACCUCCA |
| 794 | AGUCUCC | CUGAUGAGGCCGAAAGGCCGAA | AGCCCAG |
| 807 | CCAGGUA | CUGAUGAGGCCGAAAGGCCGAA | AUCCGAG |
| 833 | GGGUGUC | CUGAUGAGGCCGAAAGGCCGAA | AGCUUUG |
| 846 | CAACGGU | CUGAUGAGGCCGAAAGGCCGAA | ACCAGGG |
| 851 | GCUGGUA | CUGAUGAGGCCGAAAGGCCGAA | AGGUCUC |
| 863 | CCAGAGG | CUGAUGAGGCCGAAAGGCCGAA | AGUGGCU |
| 866 | GGGCAGG | CUGAUGAGGCCGAAAGGCCGAA | AGGCUUC |
| 867 | UCUCCGG | CUGAUGAGGCCGAAAGGCCGAA | AACGAAU |
| 869 | CUUGCAU | CUGAUGAGGCCGAAAGGCCGAA | AGGAAGA |
| 881 | ACGGGUU | CUGAUGAGGCCGAAAGGCCGAA | AAGCCAU |
| 885 | UCACCUC | CUGAUGAGGCCGAAAGGCCGAA | ACCAAGG |
| 933 | CCAGAAU | CUGAUGAGGCCGAAAGGCCGAA | AUUAUAG |
| 936 | GCACCAG | CUGAUGAGGCCGAAAGGCCGAA | AUGAUUA |
| 978 | AGUUGUA | CUGAUGAGGCCGAAAGGCCGAA | ACUGUUA |
| 980 | AAAGUUG | CUGAUGAGGCCGAAAGGCCGAA | AGACUGU |
| 986 | AGCUGAA | CUGAUGAGGCCGAAAGGCCGAA | AGUUGUA |
| 987 | GAGCUGA | CUGAUGAGGCCGAAAGGCCGAA | AAGUUGU |
| 988 | GGAGCUG | CUGAUGAGGCCGAAAGGCCGAA | AAAGUUG |

| | | | |
|---|---|---|---|
| 1005 | UCUCCAG | CUGAUGAGGCCGAAAGGCCGAA | AUCUGGU |
| 1006 | UUCCCCA | CUGAUGAGGCCGAAAGGCCGAA | ACUCUCA |
| 1023 | CUUCCGA | CUGAUGAGGCCGAAAGGCCGAA | ACCUCCA |
| 1025 | CCCUUCC | CUGAUGAGGCCGAAAGGCCGAA | AGACCUC |
| 1066 | UUAUUUU | CUGAUGAGGCCGAAAGGCCGAA | AGAGUGG |
| 1092 | GGCCUGA | CUGAUGAGGCCGAAAGGCCGAA | AUCCAGU |
| 1093 | UUGGCUG | CUGAUGAGGCCGAAAGGCCGAA | AGGUCCA |
| 1125 | UCAAGAA | CUGAUGAGGCCGAAAGGCCGAA | AGUUGGG |
| 1163 | GCAAAAG | CUGAUGAGGCCGAAAGGCCGAA | AGCUUCG |
| 1164 | AGCAAAA | CUGAUGAGGCCGAAAGGCCGAA | AAGCUUC |
| 1166 | AGAGCAA | CUGAUGAGGCCGAAAGGCCGAA | AGAAGCU |
| 1172 | GGUUUUU | CUGAUGAGGCCGAAAGGCCGAA | AACAGGA |
| 1200 | UGUGGAG | CUGAUGAGGCCGAAAGGCCGAA | AGCAGAG |
| 1201 | CUGUUCA | CUGAUGAGGCCGAAAGGCCGAA | AAGCAGC |
| 1203 | ACUGGUG | CUGAUGAGGCCGAAAGGCCGAA | AAAAAGU |
| 1227 | GCACACG | CUGAUGAGGCCGAAAGGCCGAA | AUGUACC |
| 1228 | AGCAAAA | CUGAUGAGGCCGAAAGGCCGAA | AAGCUUC |
| 1233 | CUCUCCG | CUGAUGAGGCCGAAAGGCCGAA | AAACGAA |
| 1238 | AGGACCA | CUGAUGAGGCCGAAAGGCCGAA | ACAGCAC |
| 1264 | CUUGCAC | CUGAUGAGGCCGAAAGGCCGAA | ACCCUUC |
| 1267 | UUCCCCA | CUGAUGAGGCCGAAAGGCCGAA | ACUCUCA |
| 1294 | GGCUCAG | CUGAUGAGGCCGAAAGGCCGAA | AUCUCCU |
| 1295 | CUGCUGA | CUGAUGAGGCCGAAAGGCCGAA | ACCCCUC |
| 1306 | CAUUUCA | CUGAUGAGGCCGAAAGGCCGAA | AGUCUGC |
| 1321 | UCCUCCU | CUGAUGAGGCCGAAAGGCCGAA | AGCCUUC |
| 1334 | UUUAGGA | CUGAUGAGGCCGAAAGGCCGAA | AUGGGUU |
| 1344 | CACUCUC | CUGAUGAGGCCGAAAGGCCGAA | AGCUCAU |
| 1351 | UAACUUA | CUGAUGAGGCCGAAAGGCCGAA | ACAUUCA |
| 1353 | CACCUUC | CUGAUGAGGCCGAAAGGCCGAA | ACCCACU |
| 1366 | AGUUGUA | CUGAUGAGGCCGAAAGGCCGAA | ACUGUUA |
| 1367 | AGGUGGG | CUGAUGAGGCCGAAAGGCCGAA | AGGUGCU |
| 1368 | AGAGUGG | CUGAUGAGGCCGAAAGGCCGAA | ACAGUAC |
| 1380 | CCACCCC | CUGAUGAGGCCGAAAGGCCGAA | AUGGGCA |
| 1388 | AGCCACU | CUGAUGAGGCCGAAAGGCCGAA | AGUCUCC |
| 1398 | GUUCUGU | CUGAUGAGGCCGAAAGGCCGAA | ACAGCCA |
| 1402 | AGUUCUC | CUGAUGAGGCCGAAAGGCCGAA | AAGCACA |
| 1408 | CCUCCCC | CUGAUGAGGCCGAAAGGCCGAA | AUCUCGC |
| 1410 | CCCUUCC | CUGAUGAGGCCGAAAGGCCGAA | AGACCUC |
| 1421 | ACAAAAG | CUGAUGAGGCCGAAAGGCCGAA | AGGUGGG |
| 1425 | CUCUACC | CUGAUGAGGCCGAAAGGCCGAA | AGGCAGU |
| 1429 | CAGGGGC | CUGAUGAGGCCGAAAGGCCGAA | AUAGAGA |
| 1444 | UCCUCCU | CUGAUGAGGCCGAAAGGCCGAA | AGCCUUC |
| 1455 | UCCUGGU | CUGAUGAGGCCGAAAGGCCGAA | ACAUUCC |
| 1482 | GGGAGCA | CUGAUGAGGCCGAAAGGCCGAA | AACAACU |
| 1484 | CAUGAGG | CUGAUGAGGCCGAAAGGCCGAA | AGAACAG |
| 1493 | GUUCUCA | CUGAUGAGGCCGAAAGGCCGAA | AGCACAG |
| 1500 | GGACCAU | CUGAUGAGGCCGAAAGGCCGAA | AUUUCAU |
| 1503 | GAAUGAU | CUGAUGAGGCCGAAAGGCCGAA | AUAGUCC |
| 1506 | CGGUUAU | CUGAUGAGGCCGAAAGGCCGAA | AACAUAA |

| | | |
|---|---|---|
| 1509 | ACACGGU | CUGAUGAGGCCGAAAGGCCGAA AUGGUAG |
| 1518 | CGCCUGG | CUGAUGAGGCCGAAAGGCCGAA ACCAUGA |
| 1530 | CCAGAAU | CUGAUGAGGCCGAAAGGCCGAA AUUAUAG |
| 1533 | GGCCCAC | CUGAUGAGGCCGAAAGGCCGAA AUGACCA |
| 1551 | AGCUGCU | CUGAUGAGGCCGAAAGGCCGAA AGGCAUG |
| 1559 | AGGUGGG | CUGAUGAGGCCGAAAGGCCGAA AGGUGCU |
| 1563 | GGUUAUA | CUGAUGAGGCCGAAAGGCCGAA ACAUAAG |
| 1565 | GCGGUUA | CUGAUGAGGCCGAAAGGCCGAA AAACAUA |
| 1567 | UGGCGGU | CUGAUGAGGCCGAAAGGCCGAA AUAAACA |
| 1584 | AUAUCCU | CUGAUGAGGCCGAAAGGCCGAA AUCUUUC |
| 1592 | UAACUUG | CUGAUGAGGCCGAAAGGCCGAA AUAUCCU |
| 1599 | CCUUCUG | CUGAUGAGGCCGAAAGGCCGAA AACUUGU |
| 1651 | GCUCAGG | CUGAUGAGGCCGAAAGGCCGAA AGGUGGG |
| 1661 | CAAAGGA | CUGAUGAGGCCGAAAGGCCGAA AGGUUUC |
| 1663 | UUCAAAG | CUGAUGAGGCCGAAAGGCCGAA AAAGGUU |
| 1678 | CCAGGCU | CUGAUGAGGCCGAAAGGCCGAA AGGUCCU |
| 1680 | CCAGAGG | CUGAUGAGGCCGAAAGGCCGAA AGUGGCU |
| 1681 | GCCAGAG | CUGAUGAGGCCGAAAGGCCGAA AAGUGGC |
| 1684 | ACAGCCA | CUGAUGAGGCCGAAAGGCCGAA AGGAAGU |
| 1690 | AGAUCGA | CUGAUGAGGCCGAAAGGCCGAA AGUCCGG |
| 1691 | AAGAUCG | CUGAUGAGGCCGAAAGGCCGAA AAGUCCG |
| 1696 | CCACCCC | CUGAUGAGGCCGAAAGGCCGAA AUGGGCA |
| 1698 | CUCCAGG | CUGAUGAGGCCGAAAGGCCGAA AUAUCCG |
| 1737 | GCUGGUA | CUGAUGAGGCCGAAAGGCCGAA AGGUCUC |
| 1750 | UGAGGUG | CUGAUGAGGCCGAAAGGCCGAA AGCCGCC |
| 1756 | GGGCAGG | CUGAUGAGGCCGAAAGGCCGAA AGGCUUC |
| 1787 | UGGGGAC | CUGAUGAGGCCGAAAGGCCGAA AUGUCUC |
| 1790 | AUUAGAG | CUGAUGAGGCCGAAAGGCCGAA ACAAUGC |
| 1793 | UCCAGCC | CUGAUGAGGCCGAAAGGCCGAA AGGACCA |
| 1797 | UUUAUGU | CUGAUGAGGCCGAAAGGCCGAA ACUGGUG |
| 1802 | UCUCCAG | CUGAUGAGGCCGAAAGGCCGAA AUCUGGU |
| 1812 | GGCCUGA | CUGAUGAGGCCGAAAGGCCGAA AUCCAGU |
| 1813 | UGAGGGU | CUGAUGAGGCCGAAAGGCCGAA AAUGCUG |
| 1825 | GCAGAGG | CUGAUGAGGCCGAAAGGCCGAA AGCGUGG |
| 1837 | GGAGCUA | CUGAUGAGGCCGAAAGGCCGAA AGGCAUG |
| 1845 | GGUGGCC | CUGAUGAGGCCGAAAGGCCGAA AGGCUCG |
| 1856 | AAGAUCG | CUGAUGAGGCCGAAAGGCCGAA AAGUCCG |
| 1861 | UACUGGA | CUGAUGAGGCCGAAAGGCCGAA AUCAUGU |
| 1865 | CUGAGGC | CUGAUGAGGCCGAAAGGCCGAA ACAAGUG |
| 1868 | UUUAUGU | CUGAUGAGGCCGAAAGGCCGAA ACUGGUG |
| 1877 | AGCUGCU | CUGAUGAGGCCGAAAGGCCGAA AGGCAUG |
| 1901 | GUCCCUU | CUGAUGAGGCCGAAAGGCCGAA AGUUUUA |
| 1912 | ACUGAUC | CUGAUGAGGCCGAAAGGCCGAA ACUAUAU |
| 1922 | UAACUUA | CUGAUGAGGCCGAAAGGCCGAA ACAUUCA |
| 1923 | GAUACCU | CUGAUGAGGCCGAAAGGCCGAA AGCAUCA |
| 1928 | CUGGUAA | CUGAUGAGGCCGAAAGGCCGAA ACUCUAA |
| 1930 | AGCUGGU | CUGAUGAGGCCGAAAGGCCGAA AAACUCU |
| 1964 | UGGGGAC | CUGAUGAGGCCGAAAGGCCGAA AUGUCUC |
| 1983 | UAACUUG | CUGAUGAGGCCGAAAGGCCGAA AUAUCCU |

| | | | |
|---|---|---|---|
| 1996 | GGCUCAG | CUGAUGAGGCCGAAAGGCCGAA | AUCUCCU |
| 2005 | GGUCCGC | CUGAUGAGGCCGAAAGGCCGAA | AGCUCCA |
| 2013 | UACUCAA | CUGAUGAGGCCGAAAGGCCGAA | AAAUAGC |
| 2015 | CCACCCC | CUGAUGAGGCCGAAAGGCCGAA | AUGGGCA |
| 2020 | CUCAGAA | CUGAUGAGGCCGAAAGGCCGAA | AACCACC |
| 2039 | CCUCUGC | CUGAUGAGGCCGAAAGGCCGAA | AGCCAGC |
| 2040 | CCUCCAG | CUGAUGAGGCCGAAAGGCCGAA | AGGUCAG |
| 2057 | GGAUGUG | CUGAUGAGGCCGAAAGGCCGAA | AGGAGCA |
| 2061 | ACACGGU | CUGAUGAGGCCGAAAGGCCGAA | AUGGUAG |
| 2071 | CUGAGGC | CUGAUGAGGCCGAAAGGCCGAA | ACAAGUG |
| 2076 | UAGCUCU | CUGAUGAGGCCGAAAGGCCGAA | AGGCUAC |
| 2097 | CAUCAAG | CUGAUGAGGCCGAAAGGCCGAA | AGAGUUG |
| 2098 | CGGGGGG | CUGAUGAGGCCGAAAGGCCGAA | AAGUGUG |
| 2115 | AUCCUCC | CUGAUGAGGCCGAAAGGCCGAA | AGCUGGC |
| 2128 | CUCAAUA | CUGAUGAGGCCGAAAGGCCGAA | AUAGCUG |
| 2130 | GAGGCAG | CUGAUGAGGCCGAAAGGCCGAA | AAACAGG |
| 2145 | CAUCAAG | CUGAUGAGGCCGAAAGGCCGAA | AGAGUUG |
| 2152 | AACUCUA | CUGAUGAGGCCGAAAGGCCGAA | AUUAAUA |
| 2156 | UAAUAAA | CUGAUGAGGCCGAAAGGCCGAA | ACAUCAA |
| 2158 | AUUAAUA | CUGAUGAGGCCGAAAGGCCGAA | AUACAUC |
| 2159 | AAUUAAU | CUGAUGAGGCCGAAAGGCCGAA | AAUACAU |
| 2160 | AAAUUAA | CUGAUGAGGCCGAAAGGCCGAA | AAAUACA |
| 2162 | CUAAAUU | CUGAUGAGGCCGAAAGGCCGAA | AUAAAUA |
| 2163 | AAUUAAU | CUGAUGAGGCCGAAAGGCCGAA | AAUACAU |
| 2166 | AAUAGAG | CUGAUGAGGCCGAAAGGCCGAA | AUGAAGU |
| 2167 | AAUUAAU | CUGAUGAGGCCGAAAGGCCGAA | AAUACAU |
| 2170 | CUAAAUU | CUGAUGAGGCCGAAAGGCCGAA | AUAAAUA |
| 2171 | GGGAGCA | CUGAUGAGGCCGAAAGGCCGAA | AACAACU |
| 2173 | CUGGUAA | CUGAUGAGGCCGAAAGGCCGAA | ACUCUAA |
| 2174 | GCUGGUA | CUGAUGAGGCCGAAAGGCCGAA | AACUCUA |
| 2175 | AGCUGGU | CUGAUGAGGCCGAAAGGCCGAA | AAACUCU |
| 2176 | UAGCUGG | CUGAUGAGGCCGAAAGGCCGAA | AAAACUC |
| 2183 | CAAUAAA | CUGAUGAGGCCGAAAGGCCGAA | AGCUGGU |
| 2185 | CUCAAUA | CUGAUGAGGCCGAAAGGCCGAA | AUAGCUG |
| 2186 | ACUCAAU | CUGAUGAGGCCGAAAGGCCGAA | AAUAGCU |
| 2187 | UACUCAA | CUGAUGAGGCCGAAAGGCCGAA | AAAUAGC |
| 2189 | GGUACUC | CUGAUGAGGCCGAAAGGCCGAA | AUAAAUA |
| 2196 | CAUCAAG | CUGAUGAGGCCGAAAGGCCGAA | AGAGUUG |
| 2198 | AACAUAA | CUGAUGAGGCCGAAAGGCCGAA | AGGCUGC |
| 2199 | AUAAACA | CUGAUGAGGCCGAAAGGCCGAA | AAGAGGC |
| 2200 | CUUGCAU | CUGAUGAGGCCGAAAGGCCGAA | AGGAAGA |
| 2201 | GCCGACA | CUGAUGAGGCCGAAAGGCCGAA | AAAACUU |
| 2205 | UCAGGCC | CUGAUGAGGCCGAAAGGCCGAA | ACAUAAA |
| 2210 | AGCCACU | CUGAUGAGGCCGAAAGGCCGAA | AGUCUCC |
| 2220 | AGAGAAC | CUGAUGAGGCCGAAAGGCCGAA | AUGCCAG |
| 2224 | GGAUGGA | CUGAUGAGGCCGAAAGGCCGAA | ACCUGAG |
| 2226 | GCGGCCU | CUGAUGAGGCCGAAAGGCCGAA | AGAUCCA |
| 2233 | CCUCCAG | CUGAUGAGGCCGAAAGGCCGAA | AGGUCAG |
| 2242 | GGUCCGC | CUGAUGAGGCCGAAAGGCCGAA | AGCUCCA |

| | | | |
|---|---|---|---|
| 2248 | UGGGAUG | CUGAUGAGGCCGAAAGGCCGAA | AUGGAUA |
| 2254 | UCAGUGU | CUGAUGAGGCCGAAAGGCCGAA | AAUUGGA |
| 2259 | CACCGUG | CUGAUGAGGCCGAAAGGCCGAA | AUGUGAU |
| 2260 | GCACCGU | CUGAUGAGGCCGAAAGGCCGAA | AAUGUGA |
| 2266 | UCCUGGU | CUGAUGAGGCCGAAAGGCCGAA | ACAUUCC |
| 2274 | UCUCCAG | CUGAUGAGGCCGAAAGGCCGAA | AUCUGGU |
| 2279 | CUUGCAC | CUGAUGAGGCCGAAAGGCCGAA | ACCCUUC |
| 2282 | CAGCUCA | CUGAUGAGGCCGAAAGGCCGAA | ACAGCUU |
| 2288 | AGGCCAU | CUGAUGAGGCCGAAAGGCCGAA | ACUUAUA |
| 2291 | AGCAGAG | CUGAUGAGGCCGAAAGGCCGAA | ACCACUG |
| 2321 | CCCAUGU | CUGAUGAGGCCGAAAGGCCGAA | AUCUUUC |
| 2338 | CAGGCAG | CUGAUGAGGCCGAAAGGCCGAA | AGUCUCA |
| 2339 | CAAAGGA | CUGAUGAGGCCGAAAGGCCGAA | AGGUUUC |
| 2341 | AGGCUGG | CUGAUGAGGCCGAAAGGCCGAA | AGAGGUC |
| 2344 | GCUGGAA | CUGAUGAGGCCGAAAGGCCGAA | AUCGAAA |
| 2358 | CUGCUGA | CUGAUGAGGCCGAAAGGCCGAA | AGCUGGG |
| 2359 | UCUGUUC | CUGAUGAGGCCGAAAGGCCGAA | AAAGCAG |
| 2360 | UUCAAAG | CUGAUGAGGCCGAAAGGCCGAA | AAAGGUU |
| 2376 | UCAGAAG | CUGAUGAGGCCGAAAGGCCGAA | ACCACCU |
| 2377 | CUCAGAA | CUGAUGAGGCCGAAAGGCCGAA | AACCACC |
| 2378 | CAGUAGA | CUGAUGAGGCCGAAAGGCCGAA | AAACCCU |
| 2379 | CUUAUGA | CUGAUGAGGCCGAAAGGCCGAA | AAAAGCA |
| 2380 | GCCGACA | CUGAUGAGGCCGAAAGGCCGAA | AAAACUU |
| 2382 | GGGGCAA | CUGAUGAGGCCGAAAGGCCGAA | AGAGAAU |
| 2384 | UUGUGUC | CUGAUGAGGCCGAAAGGCCGAA | ACUGGAU |
| 2399 | GUCCACA | CUGAUGAGGCCGAAAGGCCGAA | AGUGUUU |
| 2401 | CAGCUCA | CUGAUGAGGCCGAAAGGCCGAA | ACAGCUU |
| 2411 | GCAUCCU | CUGAUGAGGCCGAAAGGCCGAA | ACCAGUA |
| 2417 | ACGUAUG | CUGAUGAGGCCGAAAGGCCGAA | ACCAUUC |
| 2418 | GGCCUGA | CUGAUGAGGCCGAAAGGCCGAA | AUCCAGU |
| 2425 | AACCCUC | CUGAUGAGGCCGAAAGGCCGAA | ACCCAUG |
| 2426 | AAACUCU | CUGAUGAGGCCGAAAGGCCGAA | AAUUAAU |
| 2433 | GCUGGUA | CUGAUGAGGCCGAAAGGCCGAA | AACUCUA |
| 2434 | AGCUGGU | CUGAUGAGGCCGAAAGGCCGAA | AAACUCU |
| 2448 | GGGCAGG | CUGAUGAGGCCGAAAGGCCGAA | AGGCUUC |
| 2449 | GGGGCAG | CUGAUGAGGCCGAAAGGCCGAA | AAGGCUU |
| 2451 | AGGCAGG | CUGAUGAGGCCGAAAGGCCGAA | AACAGGC |
| 2452 | GAGGCAG | CUGAUGAGGCCGAAAGGCCGAA | AAACAGG |
| 2455 | GGGCAGG | CUGAUGAGGCCGAAAGGCCGAA | AGGCUUC |
| 2459 | GGGGGGG | CUGAUGAGGCCGAAAGGCCGAA | AGUGUGG |
| 2460 | CGGGGGG | CUGAUGAGGCCGAAAGGCCGAA | AAGUGUG |
| 2479 | GCUGGUA | CUGAUGAGGCCGAAAGGCCGAA | AGGUCUC |
| 2480 | GGAUCAC | CUGAUGAGGCCGAAAGGCCGAA | ACGGUGA |
| 2483 | GGUGGCU | CUGAUGAGGCCGAAAGGCCGAA | ACAUUGG |
| 2484 | GACUGGU | CUGAUGAGGCCGAAAGGCCGAA | AAAAAAG |
| 2492 | AGGUGGG | CUGAUGAGGCCGAAAGGCCGAA | AGGUGCU |
| 2504 | ACAAAAG | CUGAUGAGGCCGAAAGGCCGAA | AGGUGGG |
| 2508 | UGGGAUG | CUGAUGAGGCCGAAAGGCCGAA | AUGGAUA |
| 2509 | CUGGUAA | CUGAUGAGGCCGAAAGGCCGAA | ACUCUAA |

| | | |
|---|---|---|
| 2510 | GCUGGUA | CUGAUGAGGCCGAAAGGCCGAA AACUCUA |
| 2520 | CAUUGGG | CUGAUGAGGCCGAAAGGCCGAA ACAAAAG |
| 2521 | UGAGGGU | CUGAUGAGGCCGAAAGGCCGAA AAUGCUG |
| 2533 | GAUACCU | CUGAUGAGGCCGAAAGGCCGAA AGCAUCA |
| 2540 | CACAGCG | CUGAUGAGGCCGAAAGGCCGAA ACUGCUG |
| 2545 | AGGACCA | CUGAUGAGGCCGAAAGGCCGAA ACAGCAC |
| 2568 | UUUGACA | CUGAUGAGGCCGAAAGGCCGAA ACUUCAC |
| 2579 | CAGGCCA | CUGAUGAGGCCGAAAGGCCGAA AACUUAU |
| 2585 | AGAGAAC | CUGAUGAGGCCGAAAGGCCGAA AUGCCAG |
| 2588 | AUUAGAG | CUGAUGAGGCCGAAAGGCCGAA ACAAUGC |
| 2591 | AGGAGCA | CUGAUGAGGCCGAAAGGCCGAA AGAACCA |
| 2593 | GCAGAGC | CUGAUGAGGCCGAAAGGCCGAA AAAGAAG |
| 2596 | CAUUGGG | CUGAUGAGGCCGAAAGGCCGAA ACAAAAG |
| 2601 | AAACGAA | CUGAUGAGGCCGAAAGGCCGAA ACACGGU |
| 2602 | GGGAUGG | CUGAUGAGGCCGAAAGGCCGAA AGCUGGA |
| 2607 | CCAGGUA | CUGAUGAGGCCGAAAGGCCGAA AUCCGAG |
| 2608 | CACAGCG | CUGAUGAGGCCGAAAGGCCGAA ACUGCUG |
| 2609 | UCCUGGU | CUGAUGAGGCCGAAAGGCCGAA ACAUUCC |
| 2620 | GCAGGGU | CUGAUGAGGCCGAAAGGCCGAA AGGUCCU |
| 2626 | GCUGGAA | CUGAUGAGGCCGAAAGGCCGAA AUCGAAA |
| 2628 | AGGCUAC | CUGAUGAGGCCGAAAGGCCGAA AGUGUGC |
| 2635 | AGGACCG | CUGAUGAGGCCGAAAGGCCGAA AGCUGAA |
| 2640 | GGCAGGA | CUGAUGAGGCCGAAAGGCCGAA ACAGGCC |
| 2641 | CUGCUGA | CUGAUGAGGCCGAAAGGCCGAA AGCUGGG |
| 2642 | GAGGCAG | CUGAUGAGGCCGAAAGGCCGAA AAACAGG |
| 2653 | GCAUCCU | CUGAUGAGGCCGAAAGGCCGAA ACCAGUA |
| 2659 | CUUGCAC | CUGAUGAGGCCGAAAGGCCGAA ACCCUUC |
| 2689 | CCUCGGA | CUGAUGAGGCCGAAAGGCCGAA ACAUUAG |
| 2691 | GGCCUCG | CUGAUGAGGCCGAAAGGCCGAA AGACAUU |
| 2700 | GGGCAGG | CUGAUGAGGCCGAAAGGCCGAA AGGCUUC |
| 2704 | AGGCUGG | CUGAUGAGGCCGAAAGGCCGAA AGAGGUC |
| 2711 | CUGCUGA | CUGAUGAGGCCGAAAGGCCGAA AGCUGGG |
| 2712 | CCCUUCC | CUGAUGAGGCCGAAAGGCCGAA AGACCUC |
| 2721 | CUUGCAC | CUGAUGAGGCCGAAAGGCCGAA ACCCUUC |
| 2724 | GCACACG | CUGAUGAGGCCGAAAGGCCGAA AUGUACC |
| 2744 | CUGCACG | CUGAUGAGGCCGAAAGGCCGAA ACCCACC |
| 2750 | GGUACUC | CUGAUGAGGCCGAAAGGCCGAA AUAAAUA |
| 2759 | AGAUCGA | CUGAUGAGGCCGAAAGGCCGAA AGUCCGG |
| 2761 | GCAGGGU | CUGAUGAGGCCGAAAGGCCGAA AGGUCCU |
| 2765 | AGCGGCA | CUGAUGAGGCCGAAAGGCCGAA AGCAAAA |
| 2769 | CCUGUUU | CUGAUGAGGCCGAAAGGCCGAA ACAGACU |
| 2797 | GGACCAU | CUGAUGAGGCCGAAAGGCCGAA AUUUCAU |
| 2803 | CGCCUGG | CUGAUGAGGCCGAAAGGCCGAA ACCAUGA |
| 2804 | CUGCACG | CUGAUGAGGCCGAAAGGCCGAA ACCCACC |
| 2813 | GGGUCAG | CUGAUGAGGCCGAAAGGCCGAA ACCGGAG |
| 2815 | AAAGUUG | CUGAUGAGGCCGAAAGGCCGAA AGACUGU |
| 2821 | CCUCCAG | CUGAUGAGGCCGAAAGGCCGAA AGGUCAG |
| 2822 | AAGUCCG | CUGAUGAGGCCGAAAGGCCGAA AGGCUCC |
| 2823 | UGGGAGC | CUGAUGAGGCCGAAAGGCCGAA AAAGGCA |

| | | | |
|---|---|---|---|
| 2829 | AUGAUUA | CUGAUGAGGCCGAAAGGCCGAA | AGUCCAG |
| 2837 | UCAGAAG | CUGAUGAGGCCGAAAGGCCGAA | ACCACCU |
| 2840 | CAGGCAG | CUGAUGAGGCCGAAAGGCCGAA | AGUCUCA |
| 2847 | GGUGGCU | CUGAUGAGGCCGAAAGGCCGAA | ACAUUGG |
| 2853 | AACAUAA | CUGAUGAGGCCGAAAGGCCGAA | AGGCUGC |
| 2860 | UCACAGU | CUGAUGAGGCCGAAAGGCCGAA | ACUUGGC |
| 2872 | CUUGGCU | CUGAUGAGGCCGAAAGGCCGAA | AAGGUCC |
| 2877 | GUGAUGG | CUGAUGAGGCCGAAAGGCCGAA | AGCGGAA |
| 2899 | AAGAUCG | CUGAUGAGGCCGAAAGGCCGAA | AAGUCCG |
| 2900 | AAAACUC | CUGAUGAGGCCGAAAGGCCGAA | AAAUUAA |
| 2904 | AAUAGAG | CUGAUGAGGCCGAAAGGCCGAA | AUGAAGU |
| 2905 | CAAUAGA | CUGAUGAGGCCGAAAGGCCGAA | AAUGAAG |
| 2906 | UAAUAAA | CUGAUGAGGCCGAAAGGCCGAA | ACAUCAA |
| 2907 | AAAUUAA | CUGAUGAGGCCGAAAGGCCGAA | AAAUACA |
| 2908 | AGCAAAA | CUGAUGAGGCCGAAAGGCCGAA | AAGCUUC |
| 2909 | AGAGCAA | CUGAUGAGGCCGAAAGGCCGAA | AGAAGCU |
| 2910 | AAAUUAA | CUGAUGAGGCCGAAAGGCCGAA | AAAUACA |
| 2911 | AAAUUAA | CUGAUGAGGCCGAAAGGCCGAA | AAAUACA |
| 2912 | GACAUUA | CUGAUGAGGCCGAAAGGCCGAA | AGAACAA |
| 2913 | UGACCAG | CUGAUGAGGCCGAAAGGCCGAA | AGAGAAA |
| 2914 | CUUAUGA | CUGAUGAGGCCGAAAGGCCGAA | AAAAGCA |
| 2915 | UCUAAAU | CUGAUGAGGCCGAAAGGCCGAA | AAUAAAU |
| 2916 | CUCCGGA | CUGAUGAGGCCGAAAGGCCGAA | ACGAAUA |
| 2917 | UCUCCGG | CUGAUGAGGCCGAAAGGCCGAA | AACGAAU |
| 2918 | CUCUCCG | CUGAUGAGGCCGAAAGGCCGAA | AAACGAA |
| 2919 | CGACCCU | CUGAUGAGGCCGAAAGGCCGAA | AUGAGAA |
| 2931 | CUUCCGA | CUGAUGAGGCCGAAAGGCCGAA | ACCUCCA |
| 2933 | CCCUUCC | CUGAUGAGGCCGAAAGGCCGAA | AGACCUC |
| 2941 | UGGGGAC | CUGAUGAGGCCGAAAGGCCGAA | AUGUCUC |
| 2951 | GCAGAGG | CUGAUGAGGCCGAAAGGCCGAA | AGCGUGG |
| 2952 | CACAGCG | CUGAUGAGGCCGAAAGGCCGAA | ACUGCUG |
| 2955 | UGACACA | CUGAUGAGGCCGAAAGGCCGAA | AGUCACU |
| 2956 | UUGAUUC | CUGAUGAGGCCGAAAGGCCGAA | AAGGAAA |
| 2961 | AGUGGCU | CUGAUGAGGCCGAAAGGCCGAA | ACACAGA |
| 2962 | AAUUAAU | CUGAUGAGGCCGAAAGGCCGAA | AAUACAU |
| 2965 | CUUUAUU | CUGAUGAGGCCGAAAGGCCGAA | AUUCAAA |
| 2966 | CCUCUGC | CUGAUGAGGCCGAAAGGCCGAA | AGCCAGC |
| 2969 | AAAACUU | CUGAUGAGGCCGAAAGGCCGAA | AUUGAUU |
| 2975 | GCUGGUA | CUGAUGAGGCCGAAAGGCCGAA | AACUCUA |
| 2976 | AGUAGAG | CUGAUGAGGCCGAAAGGCCGAA | AACCCUC |
| 2977 | CAGCUCA | CUGAUGAGGCCGAAAGGCCGAA | ACAGCUU |
| 2979 | GGCAAUA | CUGAUGAGGCCGAAAGGCCGAA | AGAAUGA |

Table 6
Human ICAM Hairpin Ribozyme/Substrate Sequences

| nt. Position | Hairpin Ribozyme Sequence | Substrate |
|---|---|---|
| 70 | GGGCCGGG AGAA GCUG ACCAGAGAAACACACGUUGUGGUACAUUACCUGGUA | CAGCA GCC CCCGGCCC |
| 86 | GGAGUGCG AGAA GCGC ACCAGAGAAACACACGUUGUGGUACAUUACCUGGUA | GCGCU GCC CGCACUCC |
| 343 | CCCAUCAG AGAA GUUU ACCAGAGAAACACACGUUGUGGUACAUUACCUGGUA | AAACU GCC CUGAUGGG |
| 635 | GCCCUUGG AGAA GCAG ACCAGAGAAACACACGUUGUGGUACAUUACCUGGUA | CUGCG GCC CCAAGGGC |
| 653 | UGUUCUCA AGAA GCUC ACCAGAGAAACACACGUUGUGGUACAUUACCUGGUA | GAGCU GUU UGAGAACA |
| 782 | AGACUGGG AGAA GCCC ACCAGAGAAACACACGUUGUGGUACAUUACCUGGUA | GGGCU GUU CCCAGUCU |
| 920 | CUGCACAC AGAA GCCG ACCAGAGAAACACACGUUGUGGUACAUUACCUGGUA | CGGCU GAC GUGUGCAG |
| 1301 | ACAUUGGA AGAA GCUG ACCAGAGAAACACACGUUGUGGUACAUUACCUGGUA | CAGCA GAC UCCAAUGU |
| 1373 | CCCCGAUG AGAA GUGG ACCAGAGAAACACACGUUGUGGUACAUUACCUGGUA | CCACU GCC CAUCGGGG |
| 1521 | AUGACUGC AGAA GCUA ACCAGAGAAACACACGUUGUGGUACAUUACCUGGUA | UAGCA GCC GCAGUCAU |
| 1594 | CUGUUGUA AGAA GUAU ACCAGAGAAACACACGUUGUGGUACAUUACCUGGUA | AUACA GAC UACAACAG |
| 2008 | ACCCAAUA AGAA GCAA ACCAGAGAAACACACGUUGUGGUACAUUACCUGGUA | UUGCU GCC UAUUGGGU |
| 2034 | UUCUGUAA AGAA GUGG ACCAGAGAAACACACGUUGUGGUACAUUACCUGGUA | CCACA GAC UUACAGAA |
| 2125 | GGUCAGUA AGAA GCAG ACCAGAGAAACACACGUUGUGGUACAUUACCUGGUA | CUGCU GUC UACUGACC |
| 2132 | GGGUUGGG AGAA GUAG ACCAGAGAAACACACGUUGUGGUACAUUACCUGGUA | CUACU GAC CCCAACCC |
| 2276 | ACCUGUAC AGAA GUAC ACCAGAGAAACACACGUUGUGGUACAUUACCUGGUA | GUACA GUU GUACAGGU |
| 2810 | AAGGUCAA AGAA GCAG ACCAGAGAAACACACGUUGUGGUACAUUACCUGGUA | CUGCA GUC UUGACCUU |

Table 7

Mouse ICAM Hairpin Ribozyme/Substrate Sequences

| nt. Position | Hairpin Ribozyme Sequence | Substrate |
|---|---|---|
| 76 | GGGAUCAC AGAA GUGA ACCAGAGAAACACACGUUGUGGUACAUUACCUGGUA | UCACC GUU GUGAUCCC |
| 164 | UGAGGAAG AGAA GUUC ACCAGAGAAACACACGUUGUGGUACAUUACCUGGUA | GAACU GUU CUUCCUCA |
| 252 | UCAGCUCA AGAA GCUU ACCAGAGAAACACACGUUGUGGUACAUUACCUGGUA | AAGCU GUU UGAGCUGA |
| 284 | GCACAGCG AGAA GCUG ACCAGAGAAACACACGUUGUGGUACAUUACCUGGUA | CAGCA GUC CGCUGUGC |
| 318 | AAGCGGAC AGAA GCAC ACCAGAGAAACACACGUUGUGGUACAUUACCUGGUA | GUGCA GUC GUCCGCUU |
| 447 | AGAGCUGG AGAA GCGG ACCAGAGAAACACACGUUGUGGUACAUUACCUGGUA | CCGCG GAC CCAGCUCU |
| 804 | UCUCCUGG AGAA GCAU ACCAGAGAAACACACGUUGUGGUACAUUACCUGGUA | AUGCC GAC CCAGGAGA |
| 847 | UCUACCAA AGAA GUGG ACCAGAGAAACACACGUUGUGGUACAUUACCUGGUA | CCACU GCC UUGGUAGA |
| 913 | AGGAUCUG AGAA GCUA ACCAGAGAAACACACGUUGUGGUACAUUACCUGGUA | UAGCG GAC CAGAUCCU |
| 946 | AAGUUGUA AGAA GUUA ACCAGAGAAACACACGUUGUGGUACAUUACCUGGUA | UAACA GUC UACAACUU |
| 1234 | CCCAAGCA AGAA GUCU ACCAGAGAAACACACGUUGUGGUACAUUACCUGGUA | AGACG GAC UGCUUGGG |
| 1275 | AUUUCAGA AGAA GCUG ACCAGAGAAACACACGUUGUGGUACAUUACCUGGUA | CAGCA GAC UCUGAAAU |
| 1325 | UGCCUUCC AGAA GCAG ACCAGAGAAACACACGUUGUGGUACAUUACCUGGUA | CUGCA GAC GGAAGGCA |
| 1350 | CCCCGAUG AGAA GCAG ACCAGAGAAACACACGUUGUGGUACAUUACCUGGUA | CUGCU GCC CAUCGGGG |
| 1534 | ACAUAAGA AGAA GCCA ACCAGAGAAACACACGUUGUGGUACAUUACCUGGUA | UGGCA GCC UCUUAUGU |
| 1851 | GUCCACCG AGAA GUAG ACCAGAGAAACACACGUUGUGGUACAUUACCUGGUA | CUACA GCC CGGUGGAC |
| 1880 | AGAAUGAA AGAA GCGU ACCAGAGAAACACACGUUGUGGUACAUUACCUGGUA | ACGCU GAC UUCAUUCU |

Table 8
Rat ICAM Hairpin Ribozyme/Substrate Sequences

| nt. Position | Hairpin Ribozyme Sequence | Substrate |
|---|---|---|
| 5 | AAAGUGCA AGAA GCAG ACCAGAGAAACACACGUUGUGGUACAUUACCUGGUA | CUGCU GCC UGCACUUU |
| 59 | GGAGCAGA AGAA GCAU ACCAGAGAAACACACGUUGUGGUACAUUACCUGGUA | AUGCU GCC UCUGCUCC |
| 84 | GGGAUCAC AGAA GCGA ACCAGAGAAACACACGUUGUGGUACAUUACCUGGUA | UCGCC GUU GUGAUCCC |
| 295 | GCACAGUG AGAA GCUG ACCAGAGAAACACACGUUGUGGUACAUUACCUGGUA | CAGCA GAC CACUGUGC |
| 329 | AAGCCGAG AGAA GCGU ACCAGAGAAACACACGUUGUGGUACAUUACCUGGUA | ACGCA GUC CUCGGCUU |
| 433 | UUCCACCA AGAA GCGC ACCAGAGAAACACACGUUGUGGUACAUUACCUGGUA | GCGCU GCC UGGUGGAA |
| 626 | CAUUCUUG AGAA GUGA ACCAGAGAAACACACGUUGUGGUACAUUACCUGGUA | UCACU GUU CAAGAAUG |
| 806 | UCUCCAGG AGAA GCAU ACCAGAGAAACACACGUUGUGGUACAUUACCUGGUA | AUGCU GAC CCUGGAGA |
| 849 | UCCACUGA AGAA GUGG ACCAGAGAAACACACGUUGUGGUACAUUACCUGGUA | CCACU GCC UCAGUGGA |
| 915 | AGGGUCUG AGAA GCCA ACCAGAGAAACACACGUUGUGGUACAUUACCUGGUA | UGGCG GAC CAGACCCU |
| 1182 | ACCUCCAA AGAA GCAG ACCAGAGAAACACACGUUGUGGUACAUUACCUGGUA | CUGCG GCC UUGGAGGU |
| 1307 | AUGUAAGA AGAA GCUG ACCAGAGAAACACACGUUGUGGUACAUUACCUGGUA | CAGCA GAC UCUUACAU |
| 1357 | UGCUUUCC AGAA GCAG ACCAGAGAAACACACGUUGUGGUACAUUACCUGGUA | CUGCA GCC GGAAAGCA |
| 1382 | UCCCGAUA AGAA GCGG ACCAGAGAAACACACGUUGUGGUACAUUACCUGGUA | CCGCU GCC UAUCGGGA |
| 1858 | GCCCACCA AGAA GUAG ACCAGAGAAACACACGUUGUGGUACAUUACCUGGUA | CUACA GCC UGGUGGGC |
| 1887 | AGAAGGAA AGAA GCCU ACCAGAGAAACACACGUUGUGGUACAUUACCUGGUA | AGGCU GAC UUCCUUCU |
| 2012 | GAGUUGGG AGAA GUGU ACCAGAGAAACACACGUUGUGGUACAUUACCUGGUA | ACACU GUC CCCAACUC |
| 2303 | AGACUCCA AGAA GUGG ACCAGAGAAACACACGUUGUGGUACAUUACCUGGUA | CCACA GCC UGGAGUCU |
| 2539 | CCUCCCAC AGAA GCUU ACCAGAGAAACACACGUUGUGGUACAUUACCUGGUA | AAGCU GUU GUGGGAGG |

Table 9: Rat ICAM HH Ribozyme Target Sequence

| nt. Position | HH Target sequence | nt. Position | HH Target Sequence |
|---|---|---|---|
| 11 | GAUCCAAU U CACACUGA | 394 | GUGGUGCU U CUGAACAG |
| 23 | GCUGACUU C CUUCUCUA | 420 | GCACCCCU C CCAGCGCA |
| 26 | GAACUGCU C UUCCUCUU | 425 | CCUCGGCU U CUGCCACC |
| 31 | CCUCUGCU C CUGGUCCU | 427 | UCCCUGUU U AAAAACCA |
| 34 | CUGAAGCU C AGAUAUAC | 450 | AAGAACCU C AUCCUGCG |
| 40 | CUCAAGGU A CAAGCCCC | 451 | GGGUACUU C CCCCAGGC |
| 48 | GAGAACCU C GGCCUGGG | 456 | CUCGGCUU C UGCCACCA |
| 54 | CCCCGCCU C CCUGAGCC | 495 | GCCACCAU C ACUGUGUA |
| 58 | CCGUGCCU U UAGCUCCC | 510 | GUGCUGCU C CGUGGGAA |
| 64 | CAAUGGCU U CAACCCGU | 564 | GAAAAUGU U CCAACCAC |
| 96 | CCUCUGCU C CUGGUCCU | 592 | GGGAGUAU C ACCAGGGA |
| 102 | CUCCUGGU C CUGGUCGC | 607 | GAGCCAAU U UCUCAUGC |
| 108 | GGACUGCU U GGGGAACU | 608 | AGCCAAUU U CUCAUGCU |
| 115 | UCCUACCU U UGUUCCCA | 609 | GCCAAUUU C UCAUGCUU |
| 119 | GACACUGU C CCCAACUC | 611 | CAAUUUCU C AUGCUUCA |
| 120 | GUUGUGAU C CCCGGGCC | 656 | GUCACUGU U CAAGAAUG |
| 146 | CCAGACCU U GGAACUCC | 657 | UCACUGUU C AAGAAUGU |
| 152 | ACCCGGCU C CACCUCAA | 668 | GAACUGCU C UUCCUCUU |
| 158 | AUUUCUUU C ACGAGUCA | 677 | GCACCCCU C CCAGCGCA |
| 165 | UGAACAGU A CUUCCCCC | 684 | AGGCAGCU C CGGACUUU |
| 168 | GAAGCCUU C CUGCCUCG | 692 | CCAGACCU U GGAACUCC |
| 185 | GGGUGGAU C CGUGCAGG | 693 | CGGACUUU C GAUCUUCC |
| 209 | CAGCCCCU A AUCUGACC | 696 | GCCUGUUU C CUGCCUCU |
| 227 | GACCAAGU A ACUGUGAA | 709 | CAGCAUUU A CCCCUCAC |
| 230 | CAAGCUGU U GUGGGAGG | 720 | CUACAACU U UUCAGCUC |
| 237 | CUGAAGCU C GACACCCC | 723 | CAACUUUU C AGCUCCCA |
| 248 | GGCCCCCU A CCUUAGGA | 735 | CUCCUGGU C CUGGUCGC |
| 253 | CACUGCCU C AGUGGAGG | 738 | UCCUGCCU C GGGGUGGA |
| 263 | GAGCCAAU U UCUCAUGC | 765 | ACUGUGCU U UGAGAACU |
| 267 | GAAGCCUU C CUGCCUCG | 769 | UCUUGUGU U CCCUGGAA |
| 293 | GAAGCUCU U CAAGCUGA | 770 | CUUGUGUU C CCUGGAAG |
| 319 | CGGAGGAU C ACAAACGA | 785 | AGGCCUGU U UCCUGCCU |
| 335 | ACUGUGCU U UGAGAACU | 786 | GGCCUGUU U CCUGCCUC |
| 337 | UGUGCUAU A UGGUCCUC | 792 | CUCCUGGU C CUGGUCGC |
| 338 | AAGCUCUU C AAGCUGAG | 794 | UCCUGCCU C UGAAGCUC |
| 359 | CACGCAGU C CUCGGCUU | 807 | GCUCAGAU A UACCUGGA |
| 367 | CAAUGGCU U CAACCCGU | 833 | CCUGGGGU U GGAGACUA |
| 374 | UUACCCCU C ACCCACCU | 846 | CUGACAGU U AUUUAUUG |
| 375 | AGAAGCCU U CCUGCCUC | 851 | GCUCACCU U UAGCAGCU |
| 378 | ACCCACCU C ACAGGGUA | 863 | CAAUGGCU U CAACCCGU |
| 386 | CGCUGUGU U UUGGAGCU | 866 | CCAUGCUU C CUCUGACA |

| | | | | | | | |
|---|---|---|---|---|---|---|---|
| 867 | GACCACCU | C | CCCACCUA | 1421 | GGGUACUU | C | CCCCAGGC |
| 869 | CUCUUCCU | C | UUGCGAAG | 1425 | ACCCACCU | C | CUCUGGCU |
| 881 | AAUGGCUU | C | AACCCGUG | 1429 | AUACUUGU | A | GCCUCAGG |
| 885 | GACCAAGU | A | ACUGUGAA | 1444 | AGAAGGCU | C | AGGAGGAG |
| 933 | UGUGUAUU | C | GUUCCCAG | 1455 | GGGAGUAU | C | ACCAGGGA |
| 936 | GCAGAGAU | U | UUGUGUCA | 1482 | AGGGUACU | U | CCCCCAGG |
| 978 | UUGAGAAU | C | UACAACUU | 1484 | ACUGCUCU | U | CCUCUUGC |
| 980 | GAGAAUCU | A | CAACUUUU | 1493 | CCUGGGGU | U | GGAGACUA |
| 986 | CUACAACU | U | UUCAGCUC | 1500 | CGUGAAAU | U | AUGGUCAA |
| 987 | UACAACUU | U | UCAGCUCC | 1503 | GAAAAUGU | U | CCAACCAC |
| 988 | ACAACUUU | U | CAGCUCCC | 1506 | UGGGUCAU | A | AUUGUUGG |
| 1005 | UUCGUGAU | C | GUGGCGUC | 1509 | GCCACCAU | C | ACUGUGUA |
| 1006 | GUGGGAGU | A | UCACCAGG | 1518 | GUCCUGGU | C | GCCGUUGU |
| 1023 | CCGGAGGU | C | UCAGAAGG | 1530 | ACCUGGGU | C | AUAAUUGU |
| 1025 | GGAGGUCU | C | AGAAGGGG | 1533 | CUGAUCAU | U | GCGGGCUU |
| 1066 | CCUACCUU | U | GUUCCCAA | 1551 | GUGGCCCU | C | UGCUCGUA |
| 1092 | AGAGGGGU | C | UCAGCAGA | 1559 | UGGGAAGU | C | CCUGUUUA |
| 1093 | AGGGGAAU | C | CAGCCCCU | 1563 | UCCUACCU | U | UGUUCCCA |
| 1125 | CCCCAACU | C | UUGUUGAU | 1565 | UUACACCU | A | UUACCGCC |
| 1163 | ACGACGCU | U | CUUUUGCU | 1567 | ACACCUAU | U | ACCGCCAG |
| 1164 | CGACGCUU | C | UUUUGCUC | 1584 | AGGAAGAU | C | AGGAUAUA |
| 1166 | ACGCUUCU | U | UUGCUCUG | 1592 | CAGGAUAU | A | CAAGUUAC |
| 1172 | CUUUUGCU | C | UGCGGCCU | 1599 | UACAAGUU | A | CAGAAGGC |
| 1200 | AUCCAAUU | C | ACACUGAA | 1651 | CCCCGCCU | C | CCUGAGCC |
| 1201 | UUGGGCUU | C | UCCACAGG | 1661 | CUGCACUU | U | GCCCUGGU |
| 1203 | GGGCUUCU | C | CACAGGUC | 1663 | GAACAGAU | C | AAUGGACA |
| 1227 | UUGGAACU | C | CAUGUGCU | 1678 | GAGAACCU | C | GGCCUGGG |
| 1228 | GCGGGCUU | C | GUGAUCGU | 1680 | GGGCUUCU | C | CACAGGUC |
| 1233 | CUCCUGGU | C | CUGGUCGC | 1681 | GGCCUGUU | U | CCUGCCUC |
| 1238 | UGUGCUAU | A | UGGUCCUC | 1684 | CUGCUCGU | A | GACCUCUC |
| 1264 | GGAAAGAU | C | AUACGGGU | 1690 | CCCCACCU | A | CAUACAUU |
| 1267 | GUCACUGU | U | CAAGAAUG | 1691 | CCGGACUU | U | CGAUCUUC |
| 1294 | CAGAGAUU | U | UGUGUCAG | 1696 | CUCCUGGU | C | CUGGUCGC |
| 1295 | AGAGGGGU | C | UCAGCAGA | 1698 | UCAGAUAU | A | CCUGGAGA |
| 1306 | AGCAGACU | C | UUACAUGC | 1737 | GAUCACAU | U | CACGGUGC |
| 1321 | AACAGAGU | C | UGGGGAAA | 1750 | GUCCAUUU | A | CACCUAUU |
| 1334 | GUAUUCGU | U | CCCAGAGC | 1756 | CCUCUGCU | C | CUGGUCCU |
| 1344 | UCGGUGCU | C | AGGUAUCC | 1787 | GAGAACCU | C | GGCCUGGG |
| 1351 | UCAGGCCU | A | AGAGGACU | 1790 | GACACUGU | C | CCCAACUC |
| 1353 | UAGCAGCU | C | AACAAUGG | 1793 | AUGGUCCU | C | ACCUGGAC |
| 1366 | AGGGUACU | U | CCCCCAGG | 1797 | UCCCUGUU | U | AAAAACCA |
| 1367 | GGGUACUU | C | CCCCAGGC | 1802 | GCUCAGAU | A | UACCUGGA |
| 1368 | GAUGGUGU | C | CCGCUGCC | 1812 | AACAGAGU | C | UGGGGAAA |
| 1380 | CUGCCUAU | C | GGGAUGGU | 1813 | GCGGGCUU | C | GUGAUCGU |
| 1388 | UGGAGACU | A | ACUGGAUG | 1825 | GCCACCAU | C | ACUGUGUA |
| 1398 | CUGGCUGU | C | ACAGGACA | 1837 | ACCCACCU | C | ACAGGGUA |
| 1402 | CUGUGCUU | U | GAGAACUG | 1845 | AGAGGACU | C | GGAGGGGC |
| 1408 | UUCGUGAU | C | GUGGCGUC | 1856 | CCCCUAAU | C | UGACCUGC |
| 1410 | CGAACUAU | C | GAGUGGAC | 1861 | CAUGUGCU | A | UAUGGUCC |

| | | | |
|---|---|---|---|
| 1865 | UAUCCGGU A GACACAAG | 2198 | GAAUGUCU C CGAGGUCA |
| 1868 | UCACGAGU C AUAUAAAU | 2199 | AGACUCUU A CAUGCCAG |
| 1877 | ACAGUACU U CCCCCAGG | 2200 | GGGUACUU C CCCCAGGC |
| 1901 | CUAAAACU C AAGGUACA | 2201 | GGGCUUCU C CACAGGUC |
| 1912 | GAACAGAU C AAUGGACA | 2205 | UUUUGUGU C AGCCACUG |
| 1922 | AUGUAAGU U AUUGCCUA | 2210 | UGGAGACU A ACUGGAUG |
| 1923 | UGGACGCU C ACCUUUAG | 2220 | GAGAACCU C GGCCUGGG |
| 1928 | GCUCAGAU A UACCUGGA | 2224 | ACAUACAU U CCUACCUU |
| 1930 | UGGAGACU A ACUGGAUG | 2226 | CUGGACCU C AGGCCACA |
| 1964 | AGAGAUUU U GUGUCAGC | 2233 | UCAUGCUU C ACAGAACU |
| 1983 | GAGAACCU C GGCCUGGG | 2242 | ACACAGCU C UCAGUAGU |
| 1996 | UGGAAGCU C UUCAAGCU | 2248 | CUCCUGGU C CUGGUCGC |
| 2005 | AUGUAAGU U AUUGCCUA | 2254 | AUCCAAUU C ACACUGAA |
| 2013 | CGCUGCCU A UCGGGAUG | 2259 | GAUCACAU U CACGGUGC |
| 2015 | CUGCCUAU C GGGAUGGU | 2260 | AUCACAUU C ACGGUGCU |
| 2020 | UAUUGAGU A CCCUGUAC | 2266 | AUCAGGAU A UACAAGUU |
| 2039 | CGGAGGAU C ACAAACGA | 2274 | GAGCAGGU U AACAUGUA |
| 2040 | CCUGACCU C CUGGAGGU | 2279 | GGAAAGAU C AUACGGGU |
| 2057 | CUGGUCCU C CAAUGGCU | 2282 | ACAGUUAU U UAUUGAGU |
| 2061 | GCGUCCAU U UACACCUA | 2288 | GCCCUGGU C CUCCAAUG |
| 2071 | AUACUUGU A GCCUCAGG | 2291 | CAGGAUAU A CAAGUUAC |
| 2076 | UGUAGCCU C AGGCCUAA | 2321 | GGAAAGAU C AUACGGGU |
| 2097 | CCAACUCU U GUUGAUGU | 2338 | UUGGGCUU C UCCACAGG |
| 2098 | CCUGACCU C CUGGAGGU | 2339 | GGGUACUU C CCCCAGGC |
| 2115 | UUCCGACU A GGGUCCUG | 2341 | GGGCCUGU C GGUGCUCA |
| 2128 | AGUGCUGU A CCAUGAUC | 2344 | CUGCUCGU A GACCUCUC |
| 2130 | GCCUGUUU C CUGCCUCU | 2358 | CCCUGCCU C CUCCCACA |
| 2145 | CCAACUCU U GUUGAUGU | 2359 | CCAUCCAU C CCACAGAA |
| 2152 | UUGAGAAU C UACAACUU | 2360 | CUUGUGUU C CCUGGAAG |
| 2156 | UGACAGUU A UUUAUUGA | 2376 | GAACUGCU C UUCCUCUU |
| 2158 | UGAUGUAU U UAUUAAUU | 2377 | GACUUCCU U CUCUAUUA |
| 2159 | GAUGUAUU U AUUAAUUC | 2378 | GCUGAUUU C UUUCACGA |
| 2160 | AUGUAUUU A UUAAUUCA | 2379 | CUGCUCUU C CUCUUGCG |
| 2162 | ACAUUCCU A CCUUUGUU | 2380 | UGAUUUCU U UCACGAGU |
| 2163 | UAUUUAUU A AUUCAGAG | 2382 | AUUUCUUU C ACGAGUCA |
| 2166 | UGAUGUAU U UAUUAAUU | 2384 | UAUCCGGU A GACACAAG |
| 2167 | GAUGUAUU U AUUAAUUC | 2399 | UAAAUACU A UGUGGACG |
| 2170 | GUAUUUAU U AAUUCAGA | 2401 | UGUGCUAU A UGGUCCUC |
| 2171 | CAGUUAUU U AUUGAGUA | 2411 | CAAUUUCU C AUGCUUCA |
| 2173 | UGUGCUAU A UGGUCCUC | 2417 | AUCAGGAU A UACAAGUU |
| 2174 | UCUCUAUU A CCCCUGCU | 2418 | UCAUGCUU C ACAGAACU |
| 2175 | AUUUCUUU C ACGAGUCA | 2425 | UUAUUAAU U CAGAGUUC |
| 2176 | GAAAAUGU U CCAACCAC | 2426 | CCUGGGGU U GGAGACUA |
| 2183 | UGACAGUU A UUUAUUGA | 2433 | UCAGAGUU C UGACAGUU |
| 2185 | ACAGUUAU U UAUUGAGU | 2434 | CGGAGGAU C ACAAACGA |
| 2186 | CAGUUAUU U AUUGAGUA | 2448 | UGAACAGU A CUUCCCCC |
| 2187 | AGUUAUUU A UUGAGUAC | 2449 | GAAGCCUU C CUGCCUCG |
| 2189 | UUAUUUAU U GAGUACCC | 2451 | GGCCUGUU U CCUGCCUC |
| 2196 | CUGACAGU U AUUUAUUG | 2452 | GCCUGUUU C CUGCCUCU |

| | | | |
|---|---|---|---|
| 2455 | ACAUUCCU A CCUUUGUU | 2761 | CGGACUUU C GAUCUUCC |
| 2459 | CCCUGCCU C CUCCCACA | 2765 | CUUUUGCU C UGCGGCCU |
| 2460 | CCUACCUU U GUUCCCAA | 2769 | UUCUCUAU U ACCCCUGC |
| 2479 | UUACACCU A UUACCGCC | 2797 | CGUGAAAU U AUGGUCAA |
| 2480 | GUCGCCGU U GUGAUCCC | 2803 | CUCAUGCU U CACAGAAC |
| 2483 | ACCUUUGU U CCCAAUGU | 2804 | UCAUGCUU C ACAGAACU |
| 2484 | CCUUUGUU C CCAAUGUC | 2813 | GCUCCCAU C CUGACCCU |
| 2492 | GACCACCU C CCCACCUA | 2815 | CGGACUUU C GAUCUUCC |
| 2504 | ACCUACAU A CAUUCCUA | 2821 | CCUGACCU C CUGGAGGU |
| 2508 | ACAUACAU U CCUACCUU | 2822 | UACAACUU U UCAGCUCC |
| 2509 | CAUACAUU C CUACCUUU | 2823 | CAACUUUU C AGCUCCCA |
| 2510 | GUCCAUUU A CACCUAUU | 2829 | UCGGUGCU C AGGUAUCC |
| 2520 | ACCUUUGU U CCCAAUGU | 2837 | CACAGGGU A CUUCCCCC |
| 2521 | CCUUUGUU C CCAAUGUC | 2840 | GCACCCCU C CCAGCGCA |
| 2533 | ACAGCAUU U ACCCCUCA | 2847 | UUACCCCU C ACCCACCU |
| 2540 | UCGGUGCU C AGGUAUCC | 2853 | UUCGAUCU U CCGACUAG |
| 2545 | AGGCAGCU C CGGACUUU | 2860 | UCUUGUGU U CCCUGGAA |
| 2568 | CAGAGAUU U UGUGUCAG | 2872 | GGGCCUGU C GGUGCUCA |
| 2579 | CCUGCACU U UGCCCUGG | 2877 | UGGAGUCU C CCAGCACC |
| 2585 | CUGCUCGU A GACCUCUC | 2899 | AGGCAGCU C CGGACUUU |
| 2588 | UGCCUCCU C CCACAGCC | 2900 | GGCUGACU U CCUUCUCU |
| 2591 | CUCUUCCU C UUGCGAAG | 2904 | GAACUGCU C UUCCUCUU |
| 2593 | UCUCUAUU A CCCCUGCU | 2905 | GGCUGACU U CCUUCUCU |
| 2596 | CUCCUGGU C CUGGUCGC | 2906 | GUUGAUGU A UUUAUUAA |
| 2601 | UGUGCUAU A UGGUCCUC | 2907 | CUGCUCUU C CUCUUGCG |
| 2602 | GUCCUGGU C GCCGUUGU | 2908 | UGAUGUAU U UAUUAAUU |
| 2607 | GUGGGAGU A UCACCAGG | 2909 | GAACUGCU C UUCCUCUU |
| 2608 | CUUUAGCU C CCGUGGGA | 2910 | ACUUCCUU C UCUAUUAC |
| 2609 | UGGAGACU A ACUGGAUG | 2911 | UUCCUUCU C UAUUACCC |
| 2620 | UCAGAGUU C UGACAGUU | 2912 | AUGUAUUU A UUAAUUCA |
| 2626 | CUCUCAGU A GUGCUGCU | 2913 | UGUGUAUU C GUUCCCAG |
| 2628 | UACAACUU U UCAGCUCC | 2914 | GUAUUUAU U AAUUCAGA |
| 2635 | UCACAGAU C CAAUUCAC | 2915 | UAUUUAUU A AUUCAGAG |
| 2640 | GCUCAGGU A UCCAUCCA | 2916 | CUCUUCCU C UUGCGAAG |
| 2641 | CCCCACCU A CAUACAUU | 2917 | CUUCCUCU U GCGAAGAC |
| 2642 | GCCUGUUU C CUGCCUCU | 2918 | AUUUCUUU C ACGAGUCA |
| 2653 | CCACAGGU C AGGGUGCU | 2919 | UUUUGUGU C AGCCACUG |
| 2659 | AGAAGGGU C CUGCAAGC | 2931 | GAUGGUGU C CCGCUGCC |
| 2689 | ACUAGGGU C CUGAAGCU | 2933 | UGGAGUCU C CCAGCACC |
| 2691 | UCAGGCCU A AGAGGACU | 2941 | CAGUACUU C CCCCAGGC |
| 2700 | AGGGUACU U CCCCCAGG | 2951 | ACCAUGCU U CCUCUGAC |
| 2704 | GACCACCU C CCCACCUA | 2952 | CCGGACUU U CGAUCUUC |
| 2711 | CCCUACCU U AGGAAGGU | 2955 | UGCUUCCU C UGACAUGG |
| 2712 | CCUACCUU A GGAAGGUG | 2956 | CUUUCCUU U GAAUCAAU |
| 2721 | GGAAAGAU C AUACGGGU | 2961 | UUUUGUGU C AGCCACUG |
| 2724 | AAGAUCAU A CGGGUUUG | 2962 | UGUGUAUU C GUUCCCAG |
| 2744 | GGGUGGAU C CGUGCAGG | 2965 | CUUUGAAU C AAUAAAGU |
| 2750 | GUCCCUGU U UAAAAACC | 2966 | UGGAAGCU C UUCAAGCU |
| 2759 | GACGAACU A UCGAGUGG | 2969 | GAAUCAAU A AAGUUUUA |

| | |
|---|---|
| 2975 | UGGAAGCU C UUCAAGCU |
| 2976 | UAUAUGGU C CUCACCUG |
| 2977 | GAAGCUCU U CAAGCUGA |

# Table 10: Rat ICAM HH Ribozyme Sequences

| nt. Position | Rat HH Ribozyme Sequence |
|---|---|
| 11 | UCAGUGUG CUGAUGAGGCCGAAAGGCCGAA AUUGGAUC |
| 23 | UAGAGAAG CUGAUGAGGCCGAAAGGCCGAA AAGUCAGC |
| 26 | AAGAGGAA CUGAUGAGGCCGAAAGGCCGAA AGCAGUUC |
| 31 | AGGACCAG CUGAUGAGGCCGAAAGGCCGAA AGCAGAGG |
| 34 | GUAUAUCU CUGAUGAGGCCGAAAGGCCGAA AGCUUCAG |
| 40 | GGGGCUUG CUGAUGAGGCCGAAAGGCCGAA ACCUUGAG |
| 48 | CCCAGGCC CUGAUGAGGCCGAAAGGCCGAA AGGUUCUC |
| 54 | GGCUCAGG CUGAUGAGGCCGAAAGGCCGAA AGGCGGGG |
| 58 | GGGAGCUA CUGAUGAGGCCGAAAGGCCGAA AGGCACGG |
| 64 | ACGGGUUG CUGAUGAGGCCGAAAGGCCGAA AGCCAUUG |
| 96 | AGGACCAG CUGAUGAGGCCGAAAGGCCGAA AGCAGAGG |
| 102 | GCGACCAG CUGAUGAGGCCGAAAGGCCGAA ACCAGGAG |
| 108 | AGUUCCCC CUGAUGAGGCCGAAAGGCCGAA AGCAGUCC |
| 115 | UGGGAACA CUGAUGAGGCCGAAAGGCCGAA AGGUAGGA |
| 119 | GAGUUGGG CUGAUGAGGCCGAAAGGCCGAA ACAGUGUC |
| 120 | GGCCCGGG CUGAUGAGGCCGAAAGGCCGAA AUCACAAC |
| 146 | GGAGUUCC CUGAUGAGGCCGAAAGGCCGAA AGGUCUGG |
| 152 | UUGAGGUG CUGAUGAGGCCGAAAGGCCGAA AGCCGGGU |
| 158 | UGACUCGU CUGAUGAGGCCGAAAGGCCGAA AAAGAAAU |
| 165 | GGGGGAAG CUGAUGAGGCCGAAAGGCCGAA ACUGUUCA |
| 168 | CGAGGCAG CUGAUGAGGCCGAAAGGCCGAA AAGGCUUC |
| 185 | CCUGCACG CUGAUGAGGCCGAAAGGCCGAA AUCCACCC |
| 209 | GGUCAGAU CUGAUGAGGCCGAAAGGCCGAA AGGGGCUG |
| 227 | UUCACAGU CUGAUGAGGCCGAAAGGCCGAA ACUUGGUC |
| 230 | CCUCCCAC CUGAUGAGGCCGAAAGGCCGAA ACAGCUUG |
| 237 | GGGGUGUC CUGAUGAGGCCGAAAGGCCGAA AGCUUCAG |
| 248 | UCCUAAGG CUGAUGAGGCCGAAAGGCCGAA AGGGGGCC |
| 253 | CCUCCACU CUGAUGAGGCCGAAAGGCCGAA AGGCAGUG |
| 263 | GCAUGAGA CUGAUGAGGCCGAAAGGCCGAA AUUGGCUC |
| 267 | CGAGGCAG CUGAUGAGGCCGAAAGGCCGAA AAGGCUUC |
| 293 | UCAGCUUG CUGAUGAGGCCGAAAGGCCGAA AGAGCUUC |
| 319 | UCGUUUGU CUGAUGAGGCCGAAAGGCCGAA AUCCUCCG |
| 335 | AGUUCUCA CUGAUGAGGCCGAAAGGCCGAA AGCACAGU |
| 337 | GAGGACCA CUGAUGAGGCCGAAAGGCCGAA AUAGCACA |
| 338 | CUCAGCUU CUGAUGAGGCCGAAAGGCCGAA AAGAGCUU |
| 359 | AAGCCGAG CUGAUGAGGCCGAAAGGCCGAA ACUGCGUG |
| 367 | ACGGGUUG CUGAUGAGGCCGAAAGGCCGAA AGCCAUUG |
| 374 | AGGUGGGU CUGAUGAGGCCGAAAGGCCGAA AGGGGUAA |
| 375 | GAGGCAGG CUGAUGAGGCCGAAAGGCCGAA AGGCUUCU |
| 378 | UACCCUGU CUGAUGAGGCCGAAAGGCCGAA AGGUGGGU |
| 386 | AGCUCCAA CUGAUGAGGCCGAAAGGCCGAA ACACAGCG |

| | | | |
|---|---|---|---|
| 394 | CUGUUCAG | CUGAUGAGGCCGAAAGGCCGAA | AGCACCAC |
| 420 | UGCGCUGG | CUGAUGAGGCCGAAAGGCCGAA | AGGGGUGC |
| 425 | GGUGGCAG | CUGAUGAGGCCGAAAGGCCGAA | AGCCGAGG |
| 427 | UGGUUUUU | CUGAUGAGGCCGAAAGGCCGAA | AACAGGGA |
| 450 | CGCAGGAU | CUGAUGAGGCCGAAAGGCCGAA | AGGUUCUU |
| 451 | GCCUGGGG | CUGAUGAGGCCGAAAGGCCGAA | AAGUACCC |
| 456 | UGGUGGCA | CUGAUGAGGCCGAAAGGCCGAA | AAGCCGAG |
| 495 | UACACAGU | CUGAUGAGGCCGAAAGGCCGAA | AUGGUGGC |
| 510 | UUCCCACG | CUGAUGAGGCCGAAAGGCCGAA | AGCAGCAC |
| 564 | GUGGUUGG | CUGAUGAGGCCGAAAGGCCGAA | ACAUUUUC |
| 592 | UCCCUGGU | CUGAUGAGGCCGAAAGGCCGAA | AUACUCCC |
| 607 | GCAUGAGA | CUGAUGAGGCCGAAAGGCCGAA | AUUGGCUC |
| 608 | AGCAUGAG | CUGAUGAGGCCGAAAGGCCGAA | AAUUGGCU |
| 609 | AAGCAUGA | CUGAUGAGGCCGAAAGGCCGAA | AAAUUGGC |
| 611 | UGAAGCAU | CUGAUGAGGCCGAAAGGCCGAA | AGAAAUUG |
| 656 | CAUUCUUG | CUGAUGAGGCCGAAAGGCCGAA | ACAGUGAC |
| 657 | ACAUUCUU | CUGAUGAGGCCGAAAGGCCGAA | AACAGUGA |
| 668 | AAGAGGAA | CUGAUGAGGCCGAAAGGCCGAA | AGCAGUUC |
| 677 | UGCGCUGG | CUGAUGAGGCCGAAAGGCCGAA | AGGGGUGC |
| 684 | AAAGUCCG | CUGAUGAGGCCGAAAGGCCGAA | AGCUGCCU |
| 692 | GGAGUUCC | CUGAUGAGGCCGAAAGGCCGAA | AGGUCUGG |
| 693 | GGAAGAUC | CUGAUGAGGCCGAAAGGCCGAA | AAAGUCCG |
| 696 | AGAGGCAG | CUGAUGAGGCCGAAAGGCCGAA | AAACAGGC |
| 709 | GUGAGGGG | CUGAUGAGGCCGAAAGGCCGAA | AAAUGCUG |
| 720 | GAGCUGAA | CUGAUGAGGCCGAAAGGCCGAA | AGUUGUAG |
| 723 | UGGGAGCU | CUGAUGAGGCCGAAAGGCCGAA | AAAAGUUG |
| 735 | GCGACCAG | CUGAUGAGGCCGAAAGGCCGAA | ACCAGGAG |
| 738 | UCCACCCC | CUGAUGAGGCCGAAAGGCCGAA | AGGCAGGA |
| 765 | AGUUCUCA | CUGAUGAGGCCGAAAGGCCGAA | AGCACAGU |
| 769 | UUCCAGGG | CUGAUGAGGCCGAAAGGCCGAA | ACACAAGA |
| 770 | CUUCCAGG | CUGAUGAGGCCGAAAGGCCGAA | AACACAAG |
| 785 | AGGCAGGA | CUGAUGAGGCCGAAAGGCCGAA | ACAGGCCU |
| 786 | GAGGCAGG | CUGAUGAGGCCGAAAGGCCGAA | AACAGGCC |
| 792 | GCGACCAG | CUGAUGAGGCCGAAAGGCCGAA | ACCAGGAG |
| 794 | GAGCUUCA | CUGAUGAGGCCGAAAGGCCGAA | AGGCAGGA |
| 807 | UCCAGGUA | CUGAUGAGGCCGAAAGGCCGAA | AUCUGAGC |
| 833 | UAGUCUCC | CUGAUGAGGCCGAAAGGCCGAA | ACCCCAGG |
| 846 | CAAUAAAU | CUGAUGAGGCCGAAAGGCCGAA | ACUGUCAG |
| 851 | AGCUGCUA | CUGAUGAGGCCGAAAGGCCGAA | AGGUGAGC |
| 863 | ACGGGUUG | CUGAUGAGGCCGAAAGGCCGAA | AGCCAUUG |
| 866 | UGUCAGAG | CUGAUGAGGCCGAAAGGCCGAA | AAGCAUGG |
| 867 | UAGGUGGG | CUGAUGAGGCCGAAAGGCCGAA | AGGUGGUC |
| 869 | CUUCGCAA | CUGAUGAGGCCGAAAGGCCGAA | AGGAAGAG |
| 881 | CACGGGUU | CUGAUGAGGCCGAAAGGCCGAA | AAGCCAUU |
| 885 | UUCACAGU | CUGAUGAGGCCGAAAGGCCGAA | ACUUGGUC |
| 933 | CUGGGAAC | CUGAUGAGGCCGAAAGGCCGAA | AAUACACA |
| 936 | UGACACAA | CUGAUGAGGCCGAAAGGCCGAA | AUCUCUGC |
| 978 | AAGUUGUA | CUGAUGAGGCCGAAAGGCCGAA | AUUCUCAA |
| 980 | AAAAGUUG | CUGAUGAGGCCGAAAGGCCGAA | AGAUUCUC |

| | | | |
|---|---|---|---|
| 986 | GAGCUGAA | CUGAUGAGGCCGAAAGGCCGAA | AGUUGUAG |
| 987 | GGAGCUGA | CUGAUGAGGCCGAAAGGCCGAA | AAGUUGUA |
| 988 | GGGAGCUG | CUGAUGAGGCCGAAAGGCCGAA | AAAGUUGU |
| 1005 | GACGCCAC | CUGAUGAGGCCGAAAGGCCGAA | AUCACGAA |
| 1006 | CCUGGUGA | CUGAUGAGGCCGAAAGGCCGAA | ACUCCCAC |
| 1023 | CCUUCUGA | CUGAUGAGGCCGAAAGGCCGAA | ACCUCCGG |
| 1025 | CCCCUUCU | CUGAUGAGGCCGAAAGGCCGAA | AGACCUCC |
| 1066 | UUGGGAAC | CUGAUGAGGCCGAAAGGCCGAA | AAGGUAGG |
| 1092 | UCUGCUGA | CUGAUGAGGCCGAAAGGCCGAA | ACCCCUCU |
| 1093 | AGGGGCUG | CUGAUGAGGCCGAAAGGCCGAA | AUUCCCCU |
| 1125 | AUCAACAA | CUGAUGAGGCCGAAAGGCCGAA | AGUUGGGG |
| 1163 | AGCAAAAG | CUGAUGAGGCCGAAAGGCCGAA | AGCGUCGU |
| 1164 | GAGCAAAA | CUGAUGAGGCCGAAAGGCCGAA | AAGCGUCG |
| 1166 | CAGAGCAA | CUGAUGAGGCCGAAAGGCCGAA | AGAAGCGU |
| 1172 | AGGCCGCA | CUGAUGAGGCCGAAAGGCCGAA | AGCAAAAG |
| 1200 | UUCAGUGU | CUGAUGAGGCCGAAAGGCCGAA | AAUUGGAU |
| 1201 | CCUGUGGA | CUGAUGAGGCCGAAAGGCCGAA | AAGCCCAA |
| 1203 | GACCUGUG | CUGAUGAGGCCGAAAGGCCGAA | AGAAGCCC |
| 1227 | AGCACAUG | CUGAUGAGGCCGAAAGGCCGAA | AGUUCCAA |
| 1228 | ACGAUCAC | CUGAUGAGGCCGAAAGGCCGAA | AAGCCCGC |
| 1233 | GCGACCAG | CUGAUGAGGCCGAAAGGCCGAA | ACCAGGAG |
| 1238 | GAGGACCA | CUGAUGAGGCCGAAAGGCCGAA | AUAGCACA |
| 1264 | ACCCGUAU | CUGAUGAGGCCGAAAGGCCGAA | AUCUUUCC |
| 1267 | CAUUCUUG | CUGAUGAGGCCGAAAGGCCGAA | ACAGUGAC |
| 1294 | CUGACACA | CUGAUGAGGCCGAAAGGCCGAA | AAUCUCUG |
| 1295 | UCUGCUGA | CUGAUGAGGCCGAAAGGCCGAA | ACCCCUCU |
| 1306 | GCAUGUAA | CUGAUGAGGCCGAAAGGCCGAA | AGUCUGCU |
| 1321 | UUUCCCCA | CUGAUGAGGCCGAAAGGCCGAA | ACUCUGUU |
| 1334 | GCUCUGGG | CUGAUGAGGCCGAAAGGCCGAA | ACGAAUAC |
| 1344 | GGAUACCU | CUGAUGAGGCCGAAAGGCCGAA | AGCACCGA |
| 1351 | AGUCCUCU | CUGAUGAGGCCGAAAGGCCGAA | AGGCCUGA |
| 1353 | CCAUUGUU | CUGAUGAGGCCGAAAGGCCGAA | AGCUGCUA |
| 1366 | CCUGGGGG | CUGAUGAGGCCGAAAGGCCGAA | AGUACCCU |
| 1367 | GCCUGGGG | CUGAUGAGGCCGAAAGGCCGAA | AAGUACCC |
| 1368 | GGCAGCGG | CUGAUGAGGCCGAAAGGCCGAA | ACACCAUC |
| 1380 | ACCAUCCC | CUGAUGAGGCCGAAAGGCCGAA | AUAGGCAG |
| 1388 | CAUCCAGU | CUGAUGAGGCCGAAAGGCCGAA | AGUCUCCA |
| 1398 | UGUCCUGU | CUGAUGAGGCCGAAAGGCCGAA | ACAGCCAG |
| 1402 | CAGUUCUC | CUGAUGAGGCCGAAAGGCCGAA | AAGCACAG |
| 1408 | GACGCCAC | CUGAUGAGGCCGAAAGGCCGAA | AUCACGAA |
| 1410 | GUCCACUC | CUGAUGAGGCCGAAAGGCCGAA | AUAGUUCG |
| 1421 | GCCUGGGG | CUGAUGAGGCCGAAAGGCCGAA | AAGUACCC |
| 1425 | AGCCAGAG | CUGAUGAGGCCGAAAGGCCGAA | AGGUGGGU |
| 1429 | CCUGAGGC | CUGAUGAGGCCGAAAGGCCGAA | ACAAGUAU |
| 1444 | CUCCUCCU | CUGAUGAGGCCGAAAGGCCGAA | AGCCUUCU |
| 1455 | UCCCUGGU | CUGAUGAGGCCGAAAGGCCGAA | AUACUCCC |
| 1482 | CCUGGGGG | CUGAUGAGGCCGAAAGGCCGAA | AGUACCCU |
| 1484 | GCAAGAGG | CUGAUGAGGCCGAAAGGCCGAA | AGAGCAGU |
| 1493 | UAGUCUCC | CUGAUGAGGCCGAAAGGCCGAA | ACCCCAGG |

| | | | |
|---|---|---|---|
| 1500 | UUGACCAU | CUGAUGAGGCCGAAAGGCCGAA | AUUUCACG |
| 1503 | GUGGUUGG | CUGAUGAGGCCGAAAGGCCGAA | ACAUUUUC |
| 1506 | CCAACAAU | CUGAUGAGGCCGAAAGGCCGAA | AUGACCCA |
| 1509 | UACACAGU | CUGAUGAGGCCGAAAGGCCGAA | AUGGUGGC |
| 1518 | ACAACGGC | CUGAUGAGGCCGAAAGGCCGAA | ACCAGGAC |
| 1530 | ACAAUUAU | CUGAUGAGGCCGAAAGGCCGAA | ACCCAGGU |
| 1533 | AAGCCCGC | CUGAUGAGGCCGAAAGGCCGAA | AUGAUCAG |
| 1551 | UACGAGCA | CUGAUGAGGCCGAAAGGCCGAA | AGGGCCAC |
| 1559 | UAAACAGG | CUGAUGAGGCCGAAAGGCCGAA | ACUUCCCA |
| 1563 | UGGGAACA | CUGAUGAGGCCGAAAGGCCGAA | AGGUAGGA |
| 1565 | GGCGGUAA | CUGAUGAGGCCGAAAGGCCGAA | AGGUGUAA |
| 1567 | CUGGCGGU | CUGAUGAGGCCGAAAGGCCGAA | AUAGGUGU |
| 1584 | UAUAUCCU | CUGAUGAGGCCGAAAGGCCGAA | AUCUUCCU |
| 1592 | GUAACUUG | CUGAUGAGGCCGAAAGGCCGAA | AUAUCCUG |
| 1599 | GCCUUCUG | CUGAUGAGGCCGAAAGGCCGAA | AACUUGUA |
| 1651 | GGCUCAGG | CUGAUGAGGCCGAAAGGCCGAA | AGGCGGGG |
| 1661 | ACCAGGGC | CUGAUGAGGCCGAAAGGCCGAA | AAGUGCAG |
| 1663 | UGUCCAUU | CUGAUGAGGCCGAAAGGCCGAA | AUCUGUUC |
| 1678 | CCCAGGCC | CUGAUGAGGCCGAAAGGCCGAA | AGGUUCUC |
| 1680 | GACCUGUG | CUGAUGAGGCCGAAAGGCCGAA | AGAAGCCC |
| 1681 | GAGGCAGG | CUGAUGAGGCCGAAAGGCCGAA | AACAGGCC |
| 1684 | GAGAGGUC | CUGAUGAGGCCGAAAGGCCGAA | ACGAGCAG |
| 1690 | AAUGUAUG | CUGAUGAGGCCGAAAGGCCGAA | AGGUGGGG |
| 1691 | GAAGAUCG | CUGAUGAGGCCGAAAGGCCGAA | AAGUCCGG |
| 1696 | GCGACCAG | CUGAUGAGGCCGAAAGGCCGAA | ACCAGGAG |
| 1698 | UCUCCAGG | CUGAUGAGGCCGAAAGGCCGAA | AUAUCUGA |
| 1737 | GCACCGUG | CUGAUGAGGCCGAAAGGCCGAA | AUGUGAUC |
| 1750 | AAUAGGUG | CUGAUGAGGCCGAAAGGCCGAA | AAAUGGAC |
| 1756 | AGGACCAG | CUGAUGAGGCCGAAAGGCCGAA | AGCAGAGG |
| 1787 | CCCAGGCC | CUGAUGAGGCCGAAAGGCCGAA | AGGUUCUC |
| 1790 | GAGUUGGG | CUGAUGAGGCCGAAAGGCCGAA | ACAGUGUC |
| 1793 | GUCCAGGU | CUGAUGAGGCCGAAAGGCCGAA | AGGACCAU |
| 1797 | UGGUUUUU | CUGAUGAGGCCGAAAGGCCGAA | AACAGGGA |
| 1802 | UCCAGGUA | CUGAUGAGGCCGAAAGGCCGAA | AUCUGAGC |
| 1812 | UUUCCCCA | CUGAUGAGGCCGAAAGGCCGAA | ACUCUGUU |
| 1813 | ACGAUCAC | CUGAUGAGGCCGAAAGGCCGAA | AAGCCCGC |
| 1825 | UACACAGU | CUGAUGAGGCCGAAAGGCCGAA | AUGGUGGC |
| 1837 | UACCCUGU | CUGAUGAGGCCGAAAGGCCGAA | AGGUGGGU |
| 1845 | GCCCCUCC | CUGAUGAGGCCGAAAGGCCGAA | AGUCCUCU |
| 1856 | GCAGGUCA | CUGAUGAGGCCGAAAGGCCGAA | AUUAGGGG |
| 1861 | GGACCAUA | CUGAUGAGGCCGAAAGGCCGAA | AGCACAUG |
| 1865 | CUUGUGUC | CUGAUGAGGCCGAAAGGCCGAA | ACCGGAUA |
| 1868 | AUUUAUAU | CUGAUGAGGCCGAAAGGCCGAA | ACUCGUGA |
| 1877 | CCUGGGGG | CUGAUGAGGCCGAAAGGCCGAA | AGUACUGU |
| 1901 | UGUACCUU | CUGAUGAGGCCGAAAGGCCGAA | AGUUUUAG |
| 1912 | UGUCCAUU | CUGAUGAGGCCGAAAGGCCGAA | AUCUGUUC |
| 1922 | UAGGCAAU | CUGAUGAGGCCGAAAGGCCGAA | ACUUACAU |
| 1923 | CUAAAGGU | CUGAUGAGGCCGAAAGGCCGAA | AGCGUCCA |
| 1928 | UCCAGGUA | CUGAUGAGGCCGAAAGGCCGAA | AUCUGAGC |

| | | | |
|---|---|---|---|
| 1930 | CAUCCAGU | CUGAUGAGGCCGAAAGGCCGAA | AGUCUCCA |
| 1964 | GCUGACAC | CUGAUGAGGCCGAAAGGCCGAA | AAAUCUCU |
| 1983 | CCCAGGCC | CUGAUGAGGCCGAAAGGCCGAA | AGGUUCUC |
| 1996 | AGCUUGAA | CUGAUGAGGCCGAAAGGCCGAA | AGCUUCCA |
| 2005 | UAGGCAAU | CUGAUGAGGCCGAAAGGCCGAA | ACUUACAU |
| 2013 | CAUCCCGA | CUGAUGAGGCCGAAAGGCCGAA | AGGCAGCG |
| 2015 | ACCAUCCC | CUGAUGAGGCCGAAAGGCCGAA | AUAGGCAG |
| 2020 | GUACAGGG | CUGAUGAGGCCGAAAGGCCGAA | ACUCAAUA |
| 2039 | UCGUUUGU | CUGAUGAGGCCGAAAGGCCGAA | AUCCUCCG |
| 2040 | ACCUCCAG | CUGAUGAGGCCGAAAGGCCGAA | AGGUCAGG |
| 2057 | AGCCAUUG | CUGAUGAGGCCGAAAGGCCGAA | AGGACCAG |
| 2061 | UAGGUGUA | CUGAUGAGGCCGAAAGGCCGAA | AUGGACGC |
| 2071 | CCUGAGGC | CUGAUGAGGCCGAAAGGCCGAA | ACAAGUAU |
| 2076 | UUAGGCCU | CUGAUGAGGCCGAAAGGCCGAA | AGGCUACA |
| 2097 | ACAUCAAC | CUGAUGAGGCCGAAAGGCCGAA | AGAGUUGG |
| 2098 | ACCUCCAG | CUGAUGAGGCCGAAAGGCCGAA | AGGUCAGG |
| 2115 | CAGGACCC | CUGAUGAGGCCGAAAGGCCGAA | AGUCGGAA |
| 2128 | GAUCAUGG | CUGAUGAGGCCGAAAGGCCGAA | ACAGCACU |
| 2130 | AGAGGCAG | CUGAUGAGGCCGAAAGGCCGAA | AAACAGGC |
| 2145 | ACAUCAAC | CUGAUGAGGCCGAAAGGCCGAA | AGAGUUGG |
| 2152 | AAGUUGUA | CUGAUGAGGCCGAAAGGCCGAA | AUUCUCAA |
| 2156 | UCAAUAAA | CUGAUGAGGCCGAAAGGCCGAA | AACUGUCA |
| 2158 | AAUUAAUA | CUGAUGAGGCCGAAAGGCCGAA | AUACAUCA |
| 2159 | GAAUUAAU | CUGAUGAGGCCGAAAGGCCGAA | AAUACAUC |
| 2160 | UGAAUUAA | CUGAUGAGGCCGAAAGGCCGAA | AAAUACAU |
| 2162 | AACAAAGG | CUGAUGAGGCCGAAAGGCCGAA | AGGAAUGU |
| 2163 | CUCUGAAU | CUGAUGAGGCCGAAAGGCCGAA | AAUAAAUA |
| 2166 | AAUUAAUA | CUGAUGAGGCCGAAAGGCCGAA | AUACAUCA |
| 2167 | GAAUUAAU | CUGAUGAGGCCGAAAGGCCGAA | AAUACAUC |
| 2170 | UCUGAAUU | CUGAUGAGGCCGAAAGGCCGAA | AUAAAUAC |
| 2171 | UACUCAAU | CUGAUGAGGCCGAAAGGCCGAA | AAUAACUG |
| 2173 | GAGGACCA | CUGAUGAGGCCGAAAGGCCGAA | AUAGCACA |
| 2174 | AGCAGGGG | CUGAUGAGGCCGAAAGGCCGAA | AAUAGAGA |
| 2175 | UGACUCGU | CUGAUGAGGCCGAAAGGCCGAA | AAAGAAAU |
| 2176 | GUGGUUGG | CUGAUGAGGCCGAAAGGCCGAA | ACAUUUUC |
| 2183 | UCAAUAAA | CUGAUGAGGCCGAAAGGCCGAA | AACUGUCA |
| 2185 | ACUCAAUA | CUGAUGAGGCCGAAAGGCCGAA | AUAACUGU |
| 2186 | UACUCAAU | CUGAUGAGGCCGAAAGGCCGAA | AAUAACUG |
| 2187 | GUACUCAA | CUGAUGAGGCCGAAAGGCCGAA | AAAUAACU |
| 2189 | GGGUACUC | CUGAUGAGGCCGAAAGGCCGAA | AUAAAUAA |
| 2196 | CAAUAAAU | CUGAUGAGGCCGAAAGGCCGAA | ACUGUCAG |
| 2198 | UGACCUCG | CUGAUGAGGCCGAAAGGCCGAA | AGACAUUC |
| 2199 | CUGGCAUG | CUGAUGAGGCCGAAAGGCCGAA | AAGAGUCU |
| 2200 | GCCUGGGG | CUGAUGAGGCCGAAAGGCCGAA | AAGUACCC |
| 2201 | GACCUGUG | CUGAUGAGGCCGAAAGGCCGAA | AGAAGCCC |
| 2205 | CAGUGGCU | CUGAUGAGGCCGAAAGGCCGAA | ACACAAAA |
| 2210 | CAUCCAGU | CUGAUGAGGCCGAAAGGCCGAA | AGUCUCCA |
| 2220 | CCCAGGCC | CUGAUGAGGCCGAAAGGCCGAA | AGGUUCUC |
| 2224 | AAGGUAGG | CUGAUGAGGCCGAAAGGCCGAA | AUGUAUGU |

| | | | |
|---|---|---|---|
| 2226 | UGUGGCCU | CUGAUGAGGCCGAAAGGCCGAA | AGGUCCAG |
| 2233 | AGUUCUGU | CUGAUGAGGCCGAAAGGCCGAA | AAGCAUGA |
| 2242 | ACUACUGA | CUGAUGAGGCCGAAAGGCCGAA | AGCUGUGU |
| 2248 | GCGACCAG | CUGAUGAGGCCGAAAGGCCGAA | ACCAGGAG |
| 2254 | UUCAGUGU | CUGAUGAGGCCGAAAGGCCGAA | AAUUGGAU |
| 2259 | GCACCGUG | CUGAUGAGGCCGAAAGGCCGAA | AUGUGAUC |
| 2260 | AGCACCGU | CUGAUGAGGCCGAAAGGCCGAA | AAUGUGAU |
| 2266 | AACUUGUA | CUGAUGAGGCCGAAAGGCCGAA | AUCCUGAU |
| 2274 | UACAUGUU | CUGAUGAGGCCGAAAGGCCGAA | ACCUGCUC |
| 2279 | ACCCGUAU | CUGAUGAGGCCGAAAGGCCGAA | AUCUUUCC |
| 2282 | ACUCAAUA | CUGAUGAGGCCGAAAGGCCGAA | AUAACUGU |
| 2288 | CAUUGGAG | CUGAUGAGGCCGAAAGGCCGAA | ACCAGGGC |
| 2291 | GUAACUUG | CUGAUGAGGCCGAAAGGCCGAA | AUAUCCUG |
| 2321 | ACCCGUAU | CUGAUGAGGCCGAAAGGCCGAA | AUCUUUCC |
| 2338 | CCUGUGGA | CUGAUGAGGCCGAAAGGCCGAA | AAGCCCAA |
| 2339 | GCCUGGGG | CUGAUGAGGCCGAAAGGCCGAA | AAGUACCC |
| 2341 | UGAGCACC | CUGAUGAGGCCGAAAGGCCGAA | ACAGGCCC |
| 2344 | GAGAGGUC | CUGAUGAGGCCGAAAGGCCGAA | ACGAGCAG |
| 2358 | UGUGGGAG | CUGAUGAGGCCGAAAGGCCGAA | AGGCAGGG |
| 2359 | UUCUGUGG | CUGAUGAGGCCGAAAGGCCGAA | AUGGAUGG |
| 2360 | CUUCCAGG | CUGAUGAGGCCGAAAGGCCGAA | AACACAAG |
| 2376 | AAGAGGAA | CUGAUGAGGCCGAAAGGCCGAA | AGCAGUUC |
| 2377 | UAAUAGAG | CUGAUGAGGCCGAAAGGCCGAA | AGGAAGUC |
| 2378 | UCGUGAAA | CUGAUGAGGCCGAAAGGCCGAA | AAAUCAGC |
| 2379 | CGCAAGAG | CUGAUGAGGCCGAAAGGCCGAA | AAGAGCAG |
| 2380 | ACUCGUGA | CUGAUGAGGCCGAAAGGCCGAA | AGAAAUCA |
| 2382 | UGACUCGU | CUGAUGAGGCCGAAAGGCCGAA | AAAGAAAU |
| 2384 | CUUGUGUC | CUGAUGAGGCCGAAAGGCCGAA | ACCGGAUA |
| 2399 | CGUCCACA | CUGAUGAGGCCGAAAGGCCGAA | AGUAUUUA |
| 2401 | GAGGACCA | CUGAUGAGGCCGAAAGGCCGAA | AUAGCACA |
| 2411 | UGAAGCAU | CUGAUGAGGCCGAAAGGCCGAA | AGAAAUUG |
| 2417 | AACUUGUA | CUGAUGAGGCCGAAAGGCCGAA | AUCCUGAU |
| 2418 | AGUUCUGU | CUGAUGAGGCCGAAAGGCCGAA | AAGCAUGA |
| 2425 | GAACUCUG | CUGAUGAGGCCGAAAGGCCGAA | AUUAAUAA |
| 2426 | UAGUCUCC | CUGAUGAGGCCGAAAGGCCGAA | ACCCCAGG |
| 2433 | AACUGUCA | CUGAUGAGGCCGAAAGGCCGAA | AACUCUGA |
| 2434 | UCGUUUGU | CUGAUGAGGCCGAAAGGCCGAA | AUCCUCCG |
| 2448 | GGGGGAAG | CUGAUGAGGCCGAAAGGCCGAA | ACUGUUCA |
| 2449 | CGAGGCAG | CUGAUGAGGCCGAAAGGCCGAA | AAGGCUUC |
| 2451 | GAGGCAGG | CUGAUGAGGCCGAAAGGCCGAA | AACAGGCC |
| 2452 | AGAGGCAG | CUGAUGAGGCCGAAAGGCCGAA | AAACAGGC |
| 2455 | AACAAAGG | CUGAUGAGGCCGAAAGGCCGAA | AGGAAUGU |
| 2459 | UGUGGGAG | CUGAUGAGGCCGAAAGGCCGAA | AGGCAGGG |
| 2460 | UUGGGAAC | CUGAUGAGGCCGAAAGGCCGAA | AAGGUAGG |
| 2479 | GGCGGUAA | CUGAUGAGGCCGAAAGGCCGAA | AGGUGUAA |
| 2480 | GGGAUCAC | CUGAUGAGGCCGAAAGGCCGAA | ACGGCGAC |
| 2483 | ACAUUGGG | CUGAUGAGGCCGAAAGGCCGAA | ACAAAGGU |
| 2484 | GACAUUGG | CUGAUGAGGCCGAAAGGCCGAA | AACAAAGG |
| 2492 | UAGGUGGG | CUGAUGAGGCCGAAAGGCCGAA | AGGUGGUC |

| | | |
|---|---|---|
| 2504 | UAGGAAUG CUGAUGAGGCCGAAAGGCCGAA AUGUAGGU | |
| 2508 | AAGGUAGG CUGAUGAGGCCGAAAGGCCGAA AUGUAUGU | |
| 2509 | AAAGGUAG CUGAUGAGGCCGAAAGGCCGAA AAUGUAUG | |
| 2510 | AAUAGGUG CUGAUGAGGCCGAAAGGCCGAA AAAUGGAC | |
| 2520 | ACAUUGGG CUGAUGAGGCCGAAAGGCCGAA ACAAAGGU | |
| 2521 | GACAUUGG CUGAUGAGGCCGAAAGGCCGAA AACAAAGG | |
| 2533 | UGAGGGGU CUGAUGAGGCCGAAAGGCCGAA AAUGCUGU | |
| 2540 | GGAUACCU CUGAUGAGGCCGAAAGGCCGAA AGCACCGA | |
| 2545 | AAAGUCCG CUGAUGAGGCCGAAAGGCCGAA AGCUGCCU | |
| 2568 | CUGACACA CUGAUGAGGCCGAAAGGCCGAA AAUCUCUG | |
| 2579 | CCAGGGCA CUGAUGAGGCCGAAAGGCCGAA AGUGCAGG | |
| 2585 | GAGAGGUC CUGAUGAGGCCGAAAGGCCGAA ACGAGCAG | |
| 2588 | GGCUGUGG CUGAUGAGGCCGAAAGGCCGAA AGGAGGCA | |
| 2591 | CUUCGCAA CUGAUGAGGCCGAAAGGCCGAA AGGAAGAG | |
| 2593 | AGCAGGGG CUGAUGAGGCCGAAAGGCCGAA AAUAGAGA | |
| 2596 | GCGACCAG CUGAUGAGGCCGAAAGGCCGAA ACCAGGAG | |
| 2601 | GAGGACCA CUGAUGAGGCCGAAAGGCCGAA AUAGCACA | |
| 2602 | ACAACGGC CUGAUGAGGCCGAAAGGCCGAA ACCAGGAC | |
| 2607 | CCUGGUGA CUGAUGAGGCCGAAAGGCCGAA ACUCCCAC | |
| 2608 | UCCCACGG CUGAUGAGGCCGAAAGGCCGAA AGCUAAAG | |
| 2609 | CAUCCAGU CUGAUGAGGCCGAAAGGCCGAA AGUCUCCA | |
| 2620 | AACUGUCA CUGAUGAGGCCGAAAGGCCGAA AACUCUGA | |
| 2626 | AGCAGCAC CUGAUGAGGCCGAAAGGCCGAA ACUGAGAG | |
| 2628 | GGAGCUGA CUGAUGAGGCCGAAAGGCCGAA AAGUUGUA | |
| 2635 | GUGAAUUG CUGAUGAGGCCGAAAGGCCGAA AUCUGUGA | |
| 2640 | UGGAUGGA CUGAUGAGGCCGAAAGGCCGAA ACCUGAGC | |
| 2641 | AAUGUAUG CUGAUGAGGCCGAAAGGCCGAA AGGUGGGG | |
| 2642 | AGAGGCAG CUGAUGAGGCCGAAAGGCCGAA AAACAGGC | |
| 2653 | AGCACCCU CUGAUGAGGCCGAAAGGCCGAA ACCUGUGG | |
| 2659 | GCUUGCAG CUGAUGAGGCCGAAAGGCCGAA ACCCUUCU | |
| 2689 | AGCUUCAG CUGAUGAGGCCGAAAGGCCGAA ACCCUAGU | |
| 2691 | AGUCCUCU CUGAUGAGGCCGAAAGGCCGAA AGGCCUGA | |
| 2700 | CCUGGGGG CUGAUGAGGCCGAAAGGCCGAA AGUACCCU | |
| 2704 | UAGGUGGG CUGAUGAGGCCGAAAGGCCGAA AGGUGGUC | |
| 2711 | ACCUUCCU CUGAUGAGGCCGAAAGGCCGAA AGGUAGGG | |
| 2712 | CACCUUCC CUGAUGAGGCCGAAAGGCCGAA AAGGUAGG | |
| 2721 | ACCCGUAU CUGAUGAGGCCGAAAGGCCGAA AUCUUUCC | |
| 2724 | CAAACCCG CUGAUGAGGCCGAAAGGCCGAA AUGAUCUU | |
| 2744 | CCUGCACG CUGAUGAGGCCGAAAGGCCGAA AUCCACCC | |
| 2750 | GGUUUUUA CUGAUGAGGCCGAAAGGCCGAA ACAGGGAC | |
| 2759 | CCACUCGA CUGAUGAGGCCGAAAGGCCGAA AGUUCGUC | |
| 2761 | GGAAGAUC CUGAUGAGGCCGAAAGGCCGAA AAAGUCCG | |
| 2765 | AGGCCGCA CUGAUGAGGCCGAAAGGCCGAA AGCAAAAG | |
| 2769 | GCAGGGGU CUGAUGAGGCCGAAAGGCCGAA AUAGAGAA | |
| 2797 | UUGACCAU CUGAUGAGGCCGAAAGGCCGAA AUUUCACG | |
| 2803 | GUUCUGUG CUGAUGAGGCCGAAAGGCCGAA AGCAUGAG | |
| 2804 | AGUUCUGU CUGAUGAGGCCGAAAGGCCGAA AAGCAUGA | |
| 2813 | AGGGUCAG CUGAUGAGGCCGAAAGGCCGAA AUGGGAGC | |
| 2815 | GGAAGAUC CUGAUGAGGCCGAAAGGCCGAA AAAGUCCG | |

| | | | |
|---|---|---|---|
| 2821 | ACCUCCAG | CUGAUGAGGCCGAAAGGCCGAA | AGGUCAGG |
| 2822 | GGAGCUGA | CUGAUGAGGCCGAAAGGCCGAA | AAGUUGUA |
| 2823 | UGGGAGCU | CUGAUGAGGCCGAAAGGCCGAA | AAAAGUUG |
| 2829 | GGAUACCU | CUGAUGAGGCCGAAAGGCCGAA | AGCACCGA |
| 2837 | GGGGGAAG | CUGAUGAGGCCGAAAGGCCGAA | ACCCUGUG |
| 2840 | UGCGCUGG | CUGAUGAGGCCGAAAGGCCGAA | AGGGGUGC |
| 2847 | AGGUGGGU | CUGAUGAGGCCGAAAGGCCGAA | AGGGGUAA |
| 2853 | CUAGUCGG | CUGAUGAGGCCGAAAGGCCGAA | AGAUCGAA |
| 2860 | UUCCAGGG | CUGAUGAGGCCGAAAGGCCGAA | ACACAAGA |
| 2872 | UGAGCACC | CUGAUGAGGCCGAAAGGCCGAA | ACAGGCCC |
| 2877 | GGUGCUGG | CUGAUGAGGCCGAAAGGCCGAA | AGACUCCA |
| 2899 | AAAGUCCG | CUGAUGAGGCCGAAAGGCCGAA | AGCUGCCU |
| 2900 | AGAGAAGG | CUGAUGAGGCCGAAAGGCCGAA | AGUCAGCC |
| 2904 | AAGAGGAA | CUGAUGAGGCCGAAAGGCCGAA | AGCAGUUC |
| 2905 | AGAGAAGG | CUGAUGAGGCCGAAAGGCCGAA | AGUCAGCC |
| 2906 | UUAAUAAA | CUGAUGAGGCCGAAAGGCCGAA | ACAUCAAC |
| 2907 | CGCAAGAG | CUGAUGAGGCCGAAAGGCCGAA | AAGAGCAG |
| 2908 | AAUUAAUA | CUGAUGAGGCCGAAAGGCCGAA | AUACAUCA |
| 2909 | AAGAGGAA | CUGAUGAGGCCGAAAGGCCGAA | AGCAGUUC |
| 2910 | GUAAUAGA | CUGAUGAGGCCGAAAGGCCGAA | AAGGAAGU |
| 2911 | GGGUAAUA | CUGAUGAGGCCGAAAGGCCGAA | AGAAGGAA |
| 2912 | UGAAUUAA | CUGAUGAGGCCGAAAGGCCGAA | AAAUACAU |
| 2913 | CUGGGAAC | CUGAUGAGGCCGAAAGGCCGAA | AAUACACA |
| 2914 | UCUGAAUU | CUGAUGAGGCCGAAAGGCCGAA | AUAAAUAC |
| 2915 | CUCUGAAU | CUGAUGAGGCCGAAAGGCCGAA | AAUAAAUA |
| 2916 | CUUCGCAA | CUGAUGAGGCCGAAAGGCCGAA | AGGAAGAG |
| 2917 | GUCUUCGC | CUGAUGAGGCCGAAAGGCCGAA | AGAGGAAG |
| 2918 | UGACUCGU | CUGAUGAGGCCGAAAGGCCGAA | AAAGAAAU |
| 2919 | CAGUGGCU | CUGAUGAGGCCGAAAGGCCGAA | ACACAAAA |
| 2931 | GGCAGCGG | CUGAUGAGGCCGAAAGGCCGAA | ACACCAUC |
| 2933 | GGUGCUGG | CUGAUGAGGCCGAAAGGCCGAA | AGACUCCA |
| 2941 | GCCUGGGG | CUGAUGAGGCCGAAAGGCCGAA | AAGUACUG |
| 2951 | GUCAGAGG | CUGAUGAGGCCGAAAGGCCGAA | AGCAUGGU |
| 2952 | GAAGAUCG | CUGAUGAGGCCGAAAGGCCGAA | AAGUCCGG |
| 2955 | CCAUGUCA | CUGAUGAGGCCGAAAGGCCGAA | AGGAAGCA |
| 2956 | AUUGAUUC | CUGAUGAGGCCGAAAGGCCGAA | AAGGAAAG |
| 2961 | CAGUGGCU | CUGAUGAGGCCGAAAGGCCGAA | ACACAAAA |
| 2962 | CUGGGAAC | CUGAUGAGGCCGAAAGGCCGAA | AAUACACA |
| 2965 | ACUUUAUU | CUGAUGAGGCCGAAAGGCCGAA | AUUCAAAG |
| 2966 | AGCUUGAA | CUGAUGAGGCCGAAAGGCCGAA | AGCUUCCA |
| 2969 | UAAAACUU | CUGAUGAGGCCGAAAGGCCGAA | AUUGAUUC |
| 2975 | AGCUUGAA | CUGAUGAGGCCGAAAGGCCGAA | AGCUUCCA |
| 2976 | CAGGUGAG | CUGAUGAGGCCGAAAGGCCGAA | ACCAUAUA |
| 2977 | UCAGCUUG | CUGAUGAGGCCGAAAGGCCGAA | AGAGCUUC |

## Table 11: Human IL-5 HH Target Sequence

| nt. Position | HH Target Sequence | nt. Position | HH Target Sequence |
|---|---|---|---|
| 8 | AUGCACU U UCUUUGC | 245 | AAGAAAU C UUUCAGG |
| 9 | UGCACUU U CUUUGCC | 247 | GAAAUCU U UCAGGGA |
| 10 | GCACUUU C UUUGCCA | 248 | AAAUCUU U CAGGGAA |
| 12 | ACUUUCU U UGCCAAA | 249 | AAUCUUU C AGGGAAU |
| 13 | CUUUCUU U GCCAAAG | 257 | AGGGAAU A GGCACAC |
| 36 | AGAACGU U UCAGAGC | 273 | GGAGAGU C AAACUGU |
| 37 | GAACGUU U CAGAGCC | 291 | AGGGGGU A CUGUGGA |
| 38 | AACGUUU C AGAGCCA | 305 | AAAGACU A UUCAAAA |
| 56 | GGAUGCU U CUGCAUU | 307 | AGACUAU U CAAAAAC |
| 57 | GAUGCUU C UGCAUUU | 308 | GACUAUU C AAAAACU |
| 63 | UCUGCAU U UGAGUUU | 316 | AAAAACU U GUCCUUA |
| 64 | CUGCAUU U GAGUUUG | 319 | AACUUGU C CUUAAUA |
| 69 | UUUGAGU U UGCUAGC | 322 | UUGUCCU U AAUAAAG |
| 70 | UUGAGUU U GCUAGCU | 323 | UGUCCUU A AUAAAGA |
| 74 | GUUUGCU A GCUCUUG | 326 | CCUUAAU A AAGAAAU |
| 78 | GCUAGCU C UUGGAGC | 334 | AAGAAAU A CAUUGAC |
| 80 | UAGCUCU U GGAGCUG | 338 | AAUACAU U GACGGCC |
| 91 | GCUGCCU A CGUGUAU | 380 | GGAGAGU A AACCAAU |
| 97 | UACGUGU A UGCCAUC | 388 | AACCAAU U CCUAGAC |
| 104 | AUGCCAU C CCCACAG | 389 | ACCAAUU C CUAGACU |
| 116 | CAGAAAU U CCCACAA | 392 | AAUUCCU A GACUACC |
| 117 | AGAAAUU C CCACAAG | 397 | CUAGACU A CCUGCAA |
| 130 | AGUGCAU U GGUGAAA | 409 | CAAGAGU U UCUUGGU |
| 145 | GAGACCU U GGCACUG | 410 | AAGAGUU U CUUGGUG |
| 155 | CACUGCU U UCUACUC | 411 | AGAGUUU C UUGGUGU |
| 156 | ACUGCUU U CUACUCA | 413 | AGUUUCU U GGUGUAA |
| 157 | CUGCUUU C UACUCAU | 419 | UUGGUGU A AUGAACA |
| 159 | GCUUUCU A CUCAUCG | 437 | AGUGGAU A AUAGAAA |
| 162 | UUCUACU C AUCGAAC | 440 | GGAUAAU A GAAAGUU |
| 165 | UACUCAU C GAACUCU | 447 | AGAAAGU U GAGACUA |
| 171 | UCGAACU C UGCUGAU | 454 | UGAGACU A AACUGGU |
| 179 | UGCUGAU A GCCAAUG | 462 | AACUGGU U UGUUGCA |
| 192 | UGAGACU C UGAGGAU | 463 | ACUGGUU U GUUGCAG |
| 200 | UGAGGAU U CCUGUUC | 466 | GGUUUGU U GCAGCCA |
| 201 | GAGGAUU C CUGUUCC | 479 | CAAAGAU U UUGGAGG |
| 206 | UUCCUGU U CCUGUAC | 480 | AAAGAUU U UGGAGGA |
| 207 | UCCUGUU C CUGUACA | 481 | AAGAUUU U GGAGGAG |
| 212 | UUCCUGU A CAUAAAA | 497 | AGGACAU U UUACUGC |
| 216 | UGUACAU A AAAAUCA | 498 | GGACAUU U UACUGCA |
| 222 | UAAAAAU C ACCAACU | 499 | GACAUUU U ACUGCAG |

| | | | |
|---|---|---|---|
| 500 | ACAUUUU A CUGCAGU | 684 | UACUUUU U UCUUAUU |
| 531 | AAAGAGU C AGGCCUU | 685 | ACUUUUU U CUUAUUU |
| 538 | CAGGCCU U AAUUUUC | 686 | CUUUUUU C UUAUUUA |
| 539 | AGGCCUU A AUUUUCA | 688 | UUUUUCU U AUUUAAC |
| 542 | CCUUAAU U UUCAAUA | 689 | UUUUCUU A UUUAACU |
| 543 | CUUAAUU U UCAAUAU | 691 | UUCUUAU U UAACUUA |
| 544 | UUAAUUU U CAAUAUA | 692 | UCUUAUU U AACUUAA |
| 545 | UAAUUUU C AAUAUAA | 693 | CUUAUUU A ACUUAAC |
| 549 | UUUCAAU A UAAUUUA | 697 | UUUAACU U AACAUUC |
| 551 | UCAAUAU A AUUUAAC | 698 | UUAACUU A ACAUUCU |
| 554 | AUAUAAU U UAACUUC | 703 | UUAACAU U CUGUAAA |
| 555 | UAUAAUU U AACUUCA | 704 | UAACAUU C UGUAAAA |
| 556 | AUAAUUU A ACUUCAG | 708 | AUUCUGU A AAAUGUC |
| 560 | UUUAACU U CAGAGGG | 715 | AAAAUGU C UGUUAAC |
| 561 | UUAACUU C AGAGGGA | 719 | UGUCUGU U AACUUAA |
| 573 | GGAAAGU A AAUAUUU | 720 | GUCUGUU A ACUUAAU |
| 577 | AGUAAAU A UUUCAGG | 724 | GUUAACU U AAUAGUA |
| 579 | UAAAUAU U UCAGGCA | 725 | UUAACUU A AUAGUAU |
| 580 | AAAUAUU U CAGGCAU | 728 | ACUUAAU A GUAUUUA |
| 581 | AAUAUUU C AGGCAUA | 731 | UAAUAGU A UUUAUGA |
| 588 | CAGGCAU A CUGACAC | 733 | AUAGUAU U UAUGAAA |
| 597 | UGACACU U UGCCAGA | 734 | UAGUAUU U AUGAAAU |
| 598 | GACACUU U GCCAGAA | 735 | AGUAUUU A UGAAAUG |
| 611 | AAAGCAU A AAAUUCU | 745 | AAAUGGU U AAGAAUU |
| 616 | AUAAAAU U CUUAAAA | 746 | AAUGGUU A AGAAUUU |
| 617 | UAAAAUU C UUAAAAU | 752 | UAAGAAU U UGGUAAA |
| 619 | AAAUUCU U AAAAUAU | 753 | AAGAAUU U GGUAAAU |
| 620 | AAUUCUU A AAAUAUA | 757 | AUUUGGU A AAUUAGU |
| 625 | UUAAAAU A UAUUUCA | 761 | GGUAAAU U AGUAUUU |
| 627 | AAAAUAU A UUUCAGA | 762 | GUAAAUU A GUAUUUA |
| 629 | AAUAUAU U UCAGAUA | 765 | AAUUAGU A UUUAUUU |
| 630 | AUAUAUU U CAGAUAU | 767 | UUAGUAU U UAUUUAA |
| 631 | UAUAUUU C AGAUAUC | 768 | UAGUAUU U AUUUAAU |
| 636 | UUCAGAU A UCAGAAU | 769 | AGUAUUU A UUUAAUG |
| 638 | CAGAUAU C AGAAUCA | 771 | UAUUUAU U UAAUGUU |
| 644 | UCAGAAU C AUUGAAG | 772 | AUUUAUU U AAUGUUA |
| 647 | GAAUCAU U GAAGUAU | 773 | UUUAUUU A AUGUUAU |
| 653 | UUGAAGU A UUUUCCU | 778 | UUAAUGU U AUGUUGU |
| 655 | GAAGUAU U UUCCUCC | 779 | UAAUGUU A UGUUGUG |
| 656 | AAGUAUU U UCCUCCA | 783 | GUUAUGU U GUGUUCU |
| 657 | AGUAUUU U CCUCCAG | 788 | GUUGUGU U CUAAUAA |
| 658 | GUAUUUU C CUCCAGG | 789 | UUGUGUU C UAAUAAA |
| 661 | UUUUCCU C CAGGCAA | 791 | GUGUUCU A AUAAAAC |
| 672 | GCAAAAU U GAUAUAC | 794 | UUCUAAU A AAACAAA |
| 676 | AAUUGAU A UACUUUU | 805 | CAAAAAU A GACAACU |
| 678 | UUGAUAU A CUUUUUU | | |
| 681 | AUAUACU U UUUUCUU | | |
| 682 | UAUACUU U UUUCUUA | | |

# Table 12: Human IL-5 HH Ribozyme Sequences

| nt. Position | HH Ribozyme Sequence |
|---|---|
| 8 | GCAAAGA CUGAUGAGGCCGAAAGGCCGAA AGUGCAU |
| 9 | GGCAAAG CUGAUGAGGCCGAAAGGCCGAA AAGUGCA |
| 10 | UGGCAAA CUGAUGAGGCCGAAAGGCCGAA AAAGUGC |
| 12 | UUUGGCA CUGAUGAGGCCGAAAGGCCGAA AGAAAGU |
| 13 | CUUUGGC CUGAUGAGGCCGAAAGGCCGAA AAGAAAG |
| 36 | GCUCUGA CUGAUGAGGCCGAAAGGCCGAA ACGUUCU |
| 37 | GGCUCUG CUGAUGAGGCCGAAAGGCCGAA AACGUUC |
| 38 | UGGCUCU CUGAUGAGGCCGAAAGGCCGAA AAACGUU |
| 56 | AAUGCAG CUGAUGAGGCCGAAAGGCCGAA AGCAUCC |
| 57 | AAAUGCA CUGAUGAGGCCGAAAGGCCGAA AAGCAUC |
| 63 | AAACUCA CUGAUGAGGCCGAAAGGCCGAA AUGCAGA |
| 64 | CAAACUC CUGAUGAGGCCGAAAGGCCGAA AAUGCAG |
| 69 | GCUAGCA CUGAUGAGGCCGAAAGGCCGAA ACUCAAA |
| 70 | AGCUAGC CUGAUGAGGCCGAAAGGCCGAA AACUCAA |
| 74 | CAAGAGC CUGAUGAGGCCGAAAGGCCGAA AGCAAAC |
| 78 | GCUCCAA CUGAUGAGGCCGAAAGGCCGAA AGCUAGC |
| 80 | CAGCUCC CUGAUGAGGCCGAAAGGCCGAA AGAGCUA |
| 91 | AUACACG CUGAUGAGGCCGAAAGGCCGAA AGGCAGC |
| 97 | GAUGGCA CUGAUGAGGCCGAAAGGCCGAA ACACGUA |
| 104 | CUGUGGG CUGAUGAGGCCGAAAGGCCGAA AUGGCAU |
| 116 | UUGUGGG CUGAUGAGGCCGAAAGGCCGAA AUUUCUG |
| 117 | CUUGUGG CUGAUGAGGCCGAAAGGCCGAA AAUUUCU |
| 130 | UUUCACC CUGAUGAGGCCGAAAGGCCGAA AUGCACU |
| 145 | CAGUGCC CUGAUGAGGCCGAAAGGCCGAA AGGUCUC |
| 155 | GAGUAGA CUGAUGAGGCCGAAAGGCCGAA AGCAGUG |
| 156 | UGAGUAG CUGAUGAGGCCGAAAGGCCGAA AAGCAGU |
| 157 | AUGAGUA CUGAUGAGGCCGAAAGGCCGAA AAAGCAG |
| 159 | CGAUGAG CUGAUGAGGCCGAAAGGCCGAA AGAAAGC |
| 162 | GUUCGAU CUGAUGAGGCCGAAAGGCCGAA AGUAGAA |
| 165 | AGAGUUC CUGAUGAGGCCGAAAGGCCGAA AUGAGUA |
| 171 | AUCAGCA CUGAUGAGGCCGAAAGGCCGAA AGUUCGA |
| 179 | CAUUGGC CUGAUGAGGCCGAAAGGCCGAA AUCAGCA |
| 192 | AUCCUCA CUGAUGAGGCCGAAAGGCCGAA AGUCUCA |
| 200 | GAACAGG CUGAUGAGGCCGAAAGGCCGAA AUCCUCA |
| 201 | GGAACAG CUGAUGAGGCCGAAAGGCCGAA AAUCCUC |
| 206 | GUACAGG CUGAUGAGGCCGAAAGGCCGAA ACAGGAA |
| 207 | UGUACAG CUGAUGAGGCCGAAAGGCCGAA AACAGGA |
| 212 | UUUUAUG CUGAUGAGGCCGAAAGGCCGAA ACAGGAA |
| 216 | UGAUUUU CUGAUGAGGCCGAAAGGCCGAA AUGUACA |
| 222 | AGUUGGU CUGAUGAGGCCGAAAGGCCGAA AUUUUUA |
| 245 | CCUGAAA CUGAUGAGGCCGAAAGGCCGAA AUUUCUU |

| | | | |
|---|---|---|---|
| 247 | UCCCUGA | CUGAUGAGGCCGAAAGGCCGAA | AGAUUUC |
| 248 | UUCCCUG | CUGAUGAGGCCGAAAGGCCGAA | AAGAUUU |
| 249 | AUUCCCU | CUGAUGAGGCCGAAAGGCCGAA | AAAGAUU |
| 257 | GUGUGCC | CUGAUGAGGCCGAAAGGCCGAA | AUUCCCU |
| 273 | ACAGUUU | CUGAUGAGGCCGAAAGGCCGAA | ACUCUCC |
| 291 | UCCACAG | CUGAUGAGGCCGAAAGGCCGAA | ACCCCCU |
| 305 | UUUUGAA | CUGAUGAGGCCGAAAGGCCGAA | AGUCUUU |
| 307 | GUUUUUG | CUGAUGAGGCCGAAAGGCCGAA | AUAGUCU |
| 308 | AGUUUUU | CUGAUGAGGCCGAAAGGCCGAA | AAUAGUC |
| 316 | UAAGGAC | CUGAUGAGGCCGAAAGGCCGAA | AGUUUUU |
| 319 | UAUUAAG | CUGAUGAGGCCGAAAGGCCGAA | ACAAGUU |
| 322 | CUUUAUU | CUGAUGAGGCCGAAAGGCCGAA | AGGACAA |
| 323 | UCUUUAU | CUGAUGAGGCCGAAAGGCCGAA | AAGGACA |
| 326 | AUUUCUU | CUGAUGAGGCCGAAAGGCCGAA | AUUAAGG |
| 334 | GUCAAUG | CUGAUGAGGCCGAAAGGCCGAA | AUUUCUU |
| 338 | GGCCGUC | CUGAUGAGGCCGAAAGGCCGAA | AUGUAUU |
| 380 | AUUGGUU | CUGAUGAGGCCGAAAGGCCGAA | ACUCUCC |
| 388 | GUCUAGG | CUGAUGAGGCCGAAAGGCCGAA | AUUGGUU |
| 389 | AGUCUAG | CUGAUGAGGCCGAAAGGCCGAA | AAUUGGU |
| 392 | GGUAGUC | CUGAUGAGGCCGAAAGGCCGAA | AGGAAUU |
| 397 | UUGCAGG | CUGAUGAGGCCGAAAGGCCGAA | AGUCUAG |
| 409 | ACCAAGA | CUGAUGAGGCCGAAAGGCCGAA | ACUCUUG |
| 410 | CACCAAG | CUGAUGAGGCCGAAAGGCCGAA | AACUCUU |
| 411 | ACACCAA | CUGAUGAGGCCGAAAGGCCGAA | AAACUCU |
| 413 | UUACACC | CUGAUGAGGCCGAAAGGCCGAA | AGAAACU |
| 419 | UGUUCAU | CUGAUGAGGCCGAAAGGCCGAA | ACACCAA |
| 437 | UUUCUAU | CUGAUGAGGCCGAAAGGCCGAA | AUCCACU |
| 440 | AACUUUC | CUGAUGAGGCCGAAAGGCCGAA | AUUAUCC |
| 447 | UAGUCUC | CUGAUGAGGCCGAAAGGCCGAA | ACUUUCU |
| 454 | ACCAGUU | CUGAUGAGGCCGAAAGGCCGAA | AGUCUCA |
| 462 | UGCAACA | CUGAUGAGGCCGAAAGGCCGAA | ACCAGUU |
| 463 | CUGCAAC | CUGAUGAGGCCGAAAGGCCGAA | AACCAGU |
| 466 | UGGCUGC | CUGAUGAGGCCGAAAGGCCGAA | ACAAACC |
| 479 | CCUCCAA | CUGAUGAGGCCGAAAGGCCGAA | AUCUUUG |
| 480 | UCCUCCA | CUGAUGAGGCCGAAAGGCCGAA | AAUCUUU |
| 481 | CUCCUCC | CUGAUGAGGCCGAAAGGCCGAA | AAAUCUU |
| 497 | GCAGUAA | CUGAUGAGGCCGAAAGGCCGAA | AUGUCCU |
| 498 | UGCAGUA | CUGAUGAGGCCGAAAGGCCGAA | AAUGUCC |
| 499 | CUGCAGU | CUGAUGAGGCCGAAAGGCCGAA | AAAUGUC |
| 500 | ACUGCAG | CUGAUGAGGCCGAAAGGCCGAA | AAAAUGU |
| 531 | AAGGCCU | CUGAUGAGGCCGAAAGGCCGAA | ACUCUUU |
| 538 | GAAAAUU | CUGAUGAGGCCGAAAGGCCGAA | AGGCCUG |
| 539 | UGAAAAU | CUGAUGAGGCCGAAAGGCCGAA | AAGGCCU |
| 542 | UAUUGAA | CUGAUGAGGCCGAAAGGCCGAA | AUUAAGG |
| 543 | AUAUUGA | CUGAUGAGGCCGAAAGGCCGAA | AAUUAAG |
| 544 | UAUAUUG | CUGAUGAGGCCGAAAGGCCGAA | AAAUUAA |
| 545 | UUAUAUU | CUGAUGAGGCCGAAAGGCCGAA | AAAAUUA |
| 549 | UAAAUUA | CUGAUGAGGCCGAAAGGCCGAA | AUUGAAA |
| 551 | GUUAAAU | CUGAUGAGGCCGAAAGGCCGAA | AUAUUGA |

| | | | |
|---|---|---|---|
| 554 | GAAGUUA | CUGAUGAGGCCGAAAGGCCGAA | AUUAUAU |
| 555 | UGAAGUU | CUGAUGAGGCCGAAAGGCCGAA | AAUUAUA |
| 556 | CUGAAGU | CUGAUGAGGCCGAAAGGCCGAA | AAAUUAU |
| 560 | CCCUCUG | CUGAUGAGGCCGAAAGGCCGAA | AGUUAAA |
| 561 | UCCCUCU | CUGAUGAGGCCGAAAGGCCGAA | AAGUUAA |
| 573 | AAAUAUU | CUGAUGAGGCCGAAAGGCCGAA | ACUUUCC |
| 577 | CCUGAAA | CUGAUGAGGCCGAAAGGCCGAA | AUUUACU |
| 579 | UGCCUGA | CUGAUGAGGCCGAAAGGCCGAA | AUAUUUA |
| 580 | AUGCCUG | CUGAUGAGGCCGAAAGGCCGAA | AAUAUUU |
| 581 | UAUGCCU | CUGAUGAGGCCGAAAGGCCGAA | AAAUAUU |
| 588 | GUGUCAG | CUGAUGAGGCCGAAAGGCCGAA | AUGCCUG |
| 597 | UCUGGCA | CUGAUGAGGCCGAAAGGCCGAA | AGUGUCA |
| 598 | UUCUGGC | CUGAUGAGGCCGAAAGGCCGAA | AAGUGUC |
| 611 | AGAAUUU | CUGAUGAGGCCGAAAGGCCGAA | AUGCUUU |
| 616 | UUUUAAG | CUGAUGAGGCCGAAAGGCCGAA | AUUUUAU |
| 617 | AUUUUAA | CUGAUGAGGCCGAAAGGCCGAA | AAUUUUA |
| 619 | AUAUUUU | CUGAUGAGGCCGAAAGGCCGAA | AGAAUUU |
| 620 | UAUAUUU | CUGAUGAGGCCGAAAGGCCGAA | AAGAAUU |
| 625 | UGAAAUA | CUGAUGAGGCCGAAAGGCCGAA | AUUUUAA |
| 627 | UCUGAAA | CUGAUGAGGCCGAAAGGCCGAA | AUAUUUU |
| 629 | UAUCUGA | CUGAUGAGGCCGAAAGGCCGAA | AUAUAUU |
| 630 | AUAUCUG | CUGAUGAGGCCGAAAGGCCGAA | AAUAUAU |
| 631 | GAUAUCU | CUGAUGAGGCCGAAAGGCCGAA | AAAUAUA |
| 636 | AUUCUGA | CUGAUGAGGCCGAAAGGCCGAA | AUCUGAA |
| 638 | UGAUUCU | CUGAUGAGGCCGAAAGGCCGAA | AUAUCUG |
| 644 | CUUCAAU | CUGAUGAGGCCGAAAGGCCGAA | AUUCUGA |
| 647 | AUACUUC | CUGAUGAGGCCGAAAGGCCGAA | AUGAUUC |
| 653 | AGGAAAA | CUGAUGAGGCCGAAAGGCCGAA | ACUUCAA |
| 655 | GGAGGAA | CUGAUGAGGCCGAAAGGCCGAA | AUACUUC |
| 656 | UGGAGGA | CUGAUGAGGCCGAAAGGCCGAA | AAUACUU |
| 657 | CUGGAGG | CUGAUGAGGCCGAAAGGCCGAA | AAAUACU |
| 658 | CCUGGAG | CUGAUGAGGCCGAAAGGCCGAA | AAAAUAC |
| 661 | UUGCCUG | CUGAUGAGGCCGAAAGGCCGAA | AGGAAAA |
| 672 | GUAUAUC | CUGAUGAGGCCGAAAGGCCGAA | AUUUUGC |
| 676 | AAAAGUA | CUGAUGAGGCCGAAAGGCCGAA | AUCAAUU |
| 678 | AAAAAAG | CUGAUGAGGCCGAAAGGCCGAA | AUAUCAA |
| 681 | AAGAAAA | CUGAUGAGGCCGAAAGGCCGAA | AGUAUAU |
| 682 | UAAGAAA | CUGAUGAGGCCGAAAGGCCGAA | AAGUAUA |
| 683 | AUAAGAA | CUGAUGAGGCCGAAAGGCCGAA | AAAGUAU |
| 684 | AAUAAGA | CUGAUGAGGCCGAAAGGCCGAA | AAAAGUA |
| 685 | AAAUAAG | CUGAUGAGGCCGAAAGGCCGAA | AAAAAGU |
| 686 | UAAAUAA | CUGAUGAGGCCGAAAGGCCGAA | AAAAAAG |
| 688 | GUUAAAU | CUGAUGAGGCCGAAAGGCCGAA | AGAAAAA |
| 689 | AGUUAAA | CUGAUGAGGCCGAAAGGCCGAA | AAGAAAA |
| 691 | UAAGUUA | CUGAUGAGGCCGAAAGGCCGAA | AUAAGAA |
| 692 | UUAAGUU | CUGAUGAGGCCGAAAGGCCGAA | AAUAAGA |
| 693 | GUUAAGU | CUGAUGAGGCCGAAAGGCCGAA | AAAUAAG |
| 697 | GAAUGUU | CUGAUGAGGCCGAAAGGCCGAA | AGUUAAA |
| 698 | AGAAUGU | CUGAUGAGGCCGAAAGGCCGAA | AAGUUAA |

| | | | |
|---|---|---|---|
| 703 | UUUACAG | CUGAUGAGGCCGAAAGGCCGAA | AUGUUAA |
| 704 | UUUUACA | CUGAUGAGGCCGAAAGGCCGAA | AAUGUUA |
| 708 | GACAUUU | CUGAUGAGGCCGAAAGGCCGAA | ACAGAAU |
| 715 | GUUAACA | CUGAUGAGGCCGAAAGGCCGAA | ACAUUUU |
| 719 | UUAAGUU | CUGAUGAGGCCGAAAGGCCGAA | ACAGACA |
| 720 | AUUAAGU | CUGAUGAGGCCGAAAGGCCGAA | AACAGAC |
| 724 | UACUAUU | CUGAUGAGGCCGAAAGGCCGAA | AGUUAAC |
| 725 | AUACUAU | CUGAUGAGGCCGAAAGGCCGAA | AAGUUAA |
| 728 | UAAAUAC | CUGAUGAGGCCGAAAGGCCGAA | AUUAAGU |
| 731 | UCAUAAA | CUGAUGAGGCCGAAAGGCCGAA | ACUAUUA |
| 733 | UUUCAUA | CUGAUGAGGCCGAAAGGCCGAA | AUACUAU |
| 734 | AUUUCAU | CUGAUGAGGCCGAAAGGCCGAA | AAUACUA |
| 735 | CAUUUCA | CUGAUGAGGCCGAAAGGCCGAA | AAAUACU |
| 745 | AAUUCUU | CUGAUGAGGCCGAAAGGCCGAA | ACCAUUU |
| 746 | AAAUUCU | CUGAUGAGGCCGAAAGGCCGAA | AACCAUU |
| 752 | UUUACCA | CUGAUGAGGCCGAAAGGCCGAA | AUUCUUA |
| 753 | AUUUACC | CUGAUGAGGCCGAAAGGCCGAA | AAUUCUU |
| 757 | ACUAAUU | CUGAUGAGGCCGAAAGGCCGAA | ACCAAAU |
| 761 | AAAUACU | CUGAUGAGGCCGAAAGGCCGAA | AUUUACC |
| 762 | UAAAUAC | CUGAUGAGGCCGAAAGGCCGAA | AAUUUAC |
| 765 | AAAUAAA | CUGAUGAGGCCGAAAGGCCGAA | ACUAAUU |
| 767 | UUAAAUA | CUGAUGAGGCCGAAAGGCCGAA | AUACUAA |
| 768 | AUUAAAU | CUGAUGAGGCCGAAAGGCCGAA | AAUACUA |
| 769 | CAUUAAA | CUGAUGAGGCCGAAAGGCCGAA | AAAUACU |
| 771 | AACAUUA | CUGAUGAGGCCGAAAGGCCGAA | AUAAAUA |
| 772 | UAACAUU | CUGAUGAGGCCGAAAGGCCGAA | AAUAAAU |
| 773 | AUAACAU | CUGAUGAGGCCGAAAGGCCGAA | AAAUAAA |
| 778 | ACAACAU | CUGAUGAGGCCGAAAGGCCGAA | ACAUUAA |
| 779 | CACAACA | CUGAUGAGGCCGAAAGGCCGAA | AACAUUA |
| 783 | AGAACAC | CUGAUGAGGCCGAAAGGCCGAA | ACAUAAC |
| 788 | UUAUUAG | CUGAUGAGGCCGAAAGGCCGAA | ACACAAC |
| 789 | UUUAUUA | CUGAUGAGGCCGAAAGGCCGAA | AACACAA |
| 791 | GUUUUAU | CUGAUGAGGCCGAAAGGCCGAA | AGAACAC |
| 794 | UUUGUUU | CUGAUGAGGCCGAAAGGCCGAA | AUUAGAA |
| 805 | AGUUGUC | CUGAUGAGGCCGAAAGGCCGAA | AUUUUUG |

# Table 13: Mouse IL-5 HH Ribozyme Target Sequence

| nt. Position | HH Target Sequence | nt. Position | HH Target Sequence |
|---|---|---|---|
| 8 | cGCuCUU c CUUUGCu | 253 | AGGGgcU A GaCAuAC |
| 11 | uCUUcCU U UGCugAA | 259 | UagACAU a CUGaAgA |
| 12 | CUUcCUU U GCugAAG | 269 | GaAGAaU C AAACUGU |
| 36 | GAAgacU U CAGAGuC | 269 | GaAGAaU c AAaCugU |
| 36 | GaAgAcU u cAgAGUc | 269 | GAAgaAU c aAAcUgU |
| 37 | AAgacUU C AGAGuCA | 287 | uGGGGGU A CUGUGGA |
| 43 | UcaGaGU c AUGAgaA | 301 | AAAugCU A UUCcAAA |
| 58 | GGAUGCU U CUGCAcU | 301 | AAAugCU a uUCCaaA |
| 59 | GAUGCUU C UGCAcUU | 303 | AUGCuAU u CCaAaAc |
| 59 | gAUGcUU c uGcAcUU | 303 | AugCUAU U CcAAAAC |
| 66 | CUGCAcU U GAGUgUu | 304 | ugCUAUU C cAAAACc |
| 82 | UgAcucU c aGcUGUG | 315 | AACcUGU C aUUAAUA |
| 91 | GcUgUGU c uggGCCA | 318 | cUGUCaU U AAUAAAG |
| 112 | ugGAgAU U CCCAugA | 319 | UGUCaUU A AUAAAGA |
| 113 | gGAgAUU C CCAugAG | 322 | CaUUAAU A AAGAAAU |
| 141 | GAGACCU U GaCACaG | 330 | AAGAAAU A CAUUGAC |
| 141 | GAgACCcU U GaCAcAg | 334 | AAUACAU U GACcGCC |
| 158 | gUCcgCU C AcCGAgC | 334 | AAUaCaU u GACcgCC |
| 167 | cCGAgCU C UGuUGAc | 384 | AggCAgU U CCUgGAu |
| 196 | UGAGGcU U CCUGUcC | 385 | ggCAgUU C CUgGAuU |
| 197 | GAGGcUU C CUGUcCC | 393 | CUgGAuU A CCUGCAA |
| 197 | gAGGCuU c CUGuCcC | 405 | CAAGAGU U cCUUGGU |
| 202 | UUCCUGU c CCUacuC | 406 | AAGAGUU c CUUGGUG |
| 202 | UUCCUGU c CcUAcuc | 409 | AGUUcCU U GGUGUgA |
| 206 | UGUCccU a cuCaUAA | 481 | UcaCAAU u UAAgUUA |
| 212 | UACUCAU a aAAaUCa | 482 | cAcAAUU U AAgUUaA |
| 212 | UacuCAU A AAAAUCA | 483 | AcAAUUU A AgUUaAa |
| 218 | UaaAaaU c aCcAGCU | 483 | AcAAUuU a aGUUAAa |
| 218 | UAAAAAU C ACCAgCU | 495 | AAAUUgU c AAcAgAU |
| 218 | uAAAAAU c acCAgCU | 553 | GCUGuuU c CaUuUAU |
| 232 | uaUGGAU U GGaGAAA | 557 | UuUcCAU U UauaUUU |
| 241 | gAGAAAU C UUUCAGG | 564 | UUauAuU u aUgUCCU |
| 241 | gAgAaAU c UUucAGG | 564 | UUAuaUU u AugUcCU |
| 241 | gagAAAU c UUUCAGG | 565 | uaUAUUU a ugUCCuG |
| 241 | gAgAaAU c UUUCAGg | 565 | UAUAuUU a UgUCcUg |
| 243 | gaAAucU U UCAGgGg | 569 | UUuAUGU c cUGUaGU |
| 243 | GAAAUCU U UCAGGGg | 569 | uUUAUGU c cUGUagU |
| 244 | AAAUCUU U CAGGGgc | 613 | AAAGuGU u uaaCCUU |
| 245 | AAUCUUU C AGGGgcU | 614 | AAgUGuU u aACcUUU |

| | |
|---|---|
| 620 | UUAACcU u uUuGUAU |
| 793 | caAGgCU u UGuGcAU |
| 816 | CUGagUU a UACUCcc |
| 818 | GAguUAU a cUCCcuC |
| 825 | ACUcCcU c CccCUCA |
| 825 | aCUccCU c CcCcUCa |
| 839 | AuCcucU U cGUUGCA |
| 840 | uCcucUU c GUUGCAu |
| 863 | cAAgUAU U cCAGGCu |
| 864 | AAgUAUU c CAGGCug |
| 864 | AAGUAUU c caggCug |
| 913 | gAaCUCU U GGuCcaG |
| 917 | UcUuggU c CAGAuGG |
| 957 | UUagcAU c CUUUcUc |
| 960 | GCAuccU u UcUcCuA |
| 960 | GcaUcCU u uCUCcUa |
| 962 | AUcCuuU c UCcUaGC |
| 975 | gccccUU u AgAUAgA |
| 987 | aGaUGAU A cuuAAUG |
| 990 | UGAuACU u AAugacU |
| 1000 | UGACuCU c UugCuGA |
| 1027 | CgggGCU U cCUgCUC |
| 1034 | UCCUGcU C CUaUcuA |
| 1037 | UgcUCcU A UcUAACU |
| 1039 | cUccuAU c UAACUUC |
| 1039 | cUCcUAU c UAACUUc |
| 1041 | CcUAUcU A ACUUcAa |
| 1051 | UUcAAuU U AAuAccC |
| 1148 | uGAcUUU u cUuaUGU |
| 1213 | GCUgGaU u UUGGAaa |
| 1213 | gcUGGAU u uUgGAAA |
| 1214 | cugGAUU U UGGAaaA |
| 1215 | ugGAUUU U GGAaaAG |
| 1234 | gGGACAU c UccuUGC |
| 1236 | GACAUcU c cuUGCAG |
| 1275 | ugGGCCU U AcUUcUC |
| 1276 | gGGCCUU A cUUcUCc |
| 1280 | CUUAcUU c UCcgUgU |
| 1298 | UgAACUU a AGAaGcA |
| 1310 | gcAAAGU a aAuACcA |
| 1310 | GCAAAgU a aAUAcca |
| 1310 | GcaAAgU a AAUAccA |
| 1350 | AAAGCAU A AAAUggU |
| 1358 | AAAUGGU U ggGAugU |
| 1370 | UgUuaUU C AGgUAUC |
| 1375 | UUCAGgU A UCAGggU |
| 1377 | CAGgUAU C AGggUCA |
| 1383 | UCAGggU C AcUGGaG |
| 1405 | cccCAgU U UACUcCA |

| | |
|---|---|
| 1407 | cCAgUUU A CUcCAGg |
| 1407 | ccAgUUU a CUCCAGG |
| 1410 | gUUUaCU C CAGGaAA |
| 1434 | AUgCUUU U aUuUaAU |
| 1434 | aUgcUuU U AUUUAAu |
| 1434 | aUgcuUU u AuUUAAU |
| 1435 | UgCUUUU a UuUaAUU |
| 1435 | ugcUUUU a uUUAaUU |
| 1438 | UuUUAUU U AAuUcug |
| 1438 | uUUUAUU U AAUucUg |
| 1439 | UUUAUUU A AUucUgU |
| 1443 | UUUaAuU c UGuaAGa |
| 1447 | AUUCUGU A AgAUGUu |
| 1458 | ugUUcaU a UUAUUUA |
| 1458 | ugUUcAU A uUAUUUA |
| 1460 | UucAUAU u AUUUAug |
| 1461 | UcAUAuU A UUUAUGA |
| 1463 | AUAuUAU U UAUGAug |
| 1475 | AuGgAUU c aGUAAgU |
| 1479 | AUUcaGU A AgUUAaU |
| 1483 | aGuAAGU u AAUAUUU |
| 1483 | aGUAAgU U AaUAUUU |
| 1484 | GUAAgUU A aUAUUUA |
| 1487 | agUUAAU a UUuAuUA |
| 1487 | AgUUAaU A UUUAUUa |
| 1489 | UUAAUaU U uAuUAcA |
| 1489 | UUAAuAU u UAUUaCA |
| 1489 | UUAaUAU U UAUUacA |
| 1490 | UAAUaUU u AuUAcAc |
| 1490 | UAaUAUU U AUuAcAc |
| 1490 | UAaUAUU U AUUacAc |
| 1491 | AAUAUUU a uuaCAcg |
| 1491 | AAUAUuU a UuAcAcg |
| 1491 | AaUAUUU A UuAcAcG |
| 1491 | AaUAUUU A UUacAcG |
| 1494 | AUuUAUU a CAcgUAU |
| 1502 | cACGUaU A UaauAUu |
| 1502 | cAcgUAU a UAAUaUU |
| 1507 | AUAUAaU a UUcUaaU |
| 1509 | AUAAuAU U CUaAuAA |
| 1509 | aUaaUaU U CUAAUAA |
| 1510 | UAAuAUU C UaAuAAa |
| 1510 | UAAuAUU C UaauAAA |
| 1510 | UAAuAuU c UaaUAAA |
| 1510 | UaaUaUU C UAAUAAA |
| 1512 | aUaUUCU A AUAAAgC |
| 1515 | UUCUAAU A AAgCAgA |

Table 14: Human IL-5 Hairpin Ribozyme Sequences

| nt. Position | Hairpin Ribozyme Sequence | Substrate |
|---|---|---|
| 86 | UACACGUA AGAA GCUCCA ACCAGAGAAACACACGUUGUGGUACAUUACCUGGUA | UGGAGCU GCC UACGUGUA |
| 151 | GAGUAGAA AGAA GUGCCA ACCAGAGAAACACACGUUGUGGUACAUUACCUGGUA | UGGCACU GCU UUCUACUC |
| 172 | UGGCUAUC AGAA GAGUUC ACCAGAGAAACACACGUUGUGGUACAUUACCUGGUA | GAACUCU GCU GAUAGCCA |
| 203 | UGUACAGG AGAA GGAAUC ACCAGAGAAACACACGUUGUGGUACAUUACCUGGUA | GAUUCCU GUU CCUGUACA |

## Table 15: Mouse IL-5 Hairpin Ribozyme Sequences

| nt. Position | Hairpin Ribozyme Sequence | Substrate |
|---|---|---|
| 75 | AGCUGAGA AGAA GAACAC ACCAGAGAAACACACGUUGUGGUACAUUACCUGGUA | GUGUUCU GAC UCUCAGCU |
| 83 | CCAGACAC AGAA GAGAGU ACCAGAGAAACACACGUUGUGGUACAUUACCUGGUA | ACUCUCA GCU GUGUCUGG |
| 147 | GAGCGGAC AGAA GUGUCA ACCAGAGAAACACACGUUGUGGUACAUUACCUGGUA | UGACACA GCU GUCCGCUC |
| 150 | GGUGAGCG AGAA GCUGUG ACCAGAGAAACACACGUUGUGGUACAUUACCUGGUA | CACAGCU GUC CGCUCACC |
| 154 | GCUCGGUG AGAA GACAGC ACCAGAGAAACACACGUUGUGGUACAUUACCUGGUA | GCUGUCC GCU CACCGAGC |
| 168 | UGCUUGUC AGAA GAGCUC ACCAGAGAAACACACGUUGUGGUACAUUACCUGGUA | GAGCUCU GUU GACAAGCA |
| 199 | UGAGUAGG AGAA GGAAGC ACCAGAGAAACACACGUUGUGGUACAUUACCUGGUA | GCUUCCU GUC CCUACUCA |
| 274 | CCCCCACG AGAA GUUUGA ACCAGAGAAACACACGUUGUGGUACAUUACCUGGUA | UCAAACU GUC CGUGGGGG |
| 381 | AAUCCAGG AGAA GCCUCG ACCAGAGAAACACACGUUGUGGUACAUUACCUGGUA | CGAGGCA GUU CCUGGAUU |
| 454 | CACCAUGG AGAA GCUCAG ACCAGAGAAACACACGUUGUGGUACAUUACCUGGUA | CUGAGCU GCU CCAUGGUG |
| 499 | GUUUUUGC AGAA GUUGAC ACCAGAGAAACACACGUUGUGGUACAUUACCUGGUA | GUCAACA GAU GCAAAAAC |
| 548 | UAAAUGGA AGAA GCAUAU ACCAGAGAAACACACGUUGUGGUACAUUACCUGGUA | AUAUGCU GUU UCCAUUUA |
| 701 | GCAGGAGG AGAA GAAAUU ACCAGAGAAACACACGUUGUGGUACAUUACCUGGUA | AAUUUCU GAU CCUCCUGC |
| 710 | GAAGAGGA AGAA GGAGGA ACCAGAGAAACACACGUUGUGGUACAUUACCUGGUA | UCCUCCU GCC UCCUCUUC |
| 870 | AGUUCAAA AGAA GCCUGG ACCAGAGAAACACACGUUGUGGUACAUUACCUGGUA | CCAGGCU GAC UUUGAACU |
| 919 | CUGCGUCC AGAA GGACCA ACCAGAGAAACACACGUUGUGGUACAUUACCUGGUA | UGGUCCA GAU GGACGCAG |
| 1030 | UAGAUAGG AGAA GGAAGC ACCAGAGAAACACACGUUGUGGUACAUUACCUGGUA | GCUUCCU GCU CCUAUCUA |
| 1170 | AUGGCACA AGAA GAUUCA ACCAGAGAAACACACGUUGUGGUACAUUACCUGGUA | UGAAUCA GAC UGUGCCAU |
| 1205 | CAAAAUCC AGAA GCUCCA ACCAGAGAAACACACGUUGUGGUACAUUACCUGGUA | UGGAGCA GCU GGAUUUUG |
| 1402 | CUGGAGUA AGAA GGGGGA ACCAGAGAAACACACGUUGUGGUACAUUACCUGGUA | UCCCCCA GUU UACUCCAG |
| 1421 | AAGCAUAC AGAA GUUUUU ACCAGAGAAACACACGUUGUGGUACAUUACCUGGUA | AAAAACA GAU GUAUGCUU |

## Table 16 : Mouse IL-5 Hairpin Ribozyme Sequences

| nt. Position | Hairpin Ribozyme Sequence | Substrate |
|---|---|---|
| 75 | AGCUGAGA AGAA GAACAC ACCAGAGAAACACACGUUGUGGUACAUUACCUGGUA | GUGUUCU GAC UCUCAGCU |
| 83 | CCAGACAC AGAA GAGAGU ACCAGAGAAACACACGUUGUGGUACAUUACCUGGUA | ACUCUCA GCU GUGUCUGG |
| 147 | GAGCGGAC AGAA GUGUCA ACCAGAGAAACACACGUUGUGGUACAUUACCUGGUA | UGACACA GCU GUCCGCUC |
| 150 | GGUGAGCG AGAA GCUGUG ACCAGAGAAACACACGUUGUGGUACAUUACCUGGUA | CACAGCU GUC CGCUCACC |
| 154 | GCUCGGUG AGAA GACAGC ACCAGAGAAACACACGUUGUGGUACAUUACCUGGUA | GCUGUCC GCU CACCGAGC |
| 168 | UGCUUGUC AGAA GAGCUC ACCAGAGAAACACACGUUGUGGUACAUUACCUGGUA | GAGCUCU GUU GACAAGCA |
| 199 | UGAGUAGG AGAA GGAAGC ACCAGAGAAACACACGUUGUGGUACAUUACCUGGUA | GCUUCCU GUC CCUACUCA |
| 274 | CCCCCACG AGAA GUUUGA ACCAGAGAAACACACGUUGUGGUACAUUACCUGGUA | UCAAACU GUC CGUGGGGG |
| 381 | AAUCCAGG AGAA GCCUCG ACCAGAGAAACACACGUUGUGGUACAUUACCUGGUA | CGAGGCA GUU CCUGGAUU |
| 454 | CACCAUGG AGAA GCUCAG ACCAGAGAAACACACGUUGUGGUACAUUACCUGGUA | CUGAGCU GCU CCAUGGUG |
| 499 | GUUUUUGC AGAA GUUGAC ACCAGAGAAACACACGUUGUGGUACAUUACCUGGUA | GUCAACA GAU GCAAAAAC |
| 548 | UAAAUGGA AGAA GCAUAU ACCAGAGAAACACACGUUGUGGUACAUUACCUGGUA | AUAUGCU GUU UCCAUUUA |
| 701 | GCAGGAGG AGAA GAAAUU ACCAGAGAAACACACGUUGUGGUACAUUACCUGGUA | AAUUUCU GAU CCUCCUGC |
| 710 | GAAGAGGA AGAA GGAGGA ACCAGAGAAACACACGUUGUGGUACAUUACCUGGUA | UCCUCCU GCC UCCUCUUC |
| 870 | AGUUCAAA AGAA GCCUGG ACCAGAGAAACACACGUUGUGGUACAUUACCUGGUA | CCAGGCU GAC UUUGAACU |
| 919 | CUGCGUCC AGAA GGACCA ACCAGAGAAACACACGUUGUGGUACAUUACCUGGUA | UGGUCCA GAU GGACGCAG |
| 1030 | UAGAUAGG AGAA GGAAGC ACCAGAGAAACACACGUUGUGGUACAUUACCUGGUA | GCUUCCU GCU CCUAUCUA |
| 1170 | AUGGCACA AGAA GAUUCA ACCAGAGAAACACACGUUGUGGUACAUUACCUGGUA | UGAAUCA GAC UGUGCCAU |
| 1205 | CAAAAUCC AGAA GCUCCA ACCAGAGAAACACACGUUGUGGUACAUUACCUGGUA | UGGAGCA GCU GGAUUUUG |
| 1402 | CUGGAGUA AGAA GGGGGA ACCAGAGAAACACACGUUGUGGUACAUUACCUGGUA | UCCCCCA GUU UACUCCAG |
| 1421 | AAGCAUAC AGAA GUUUUU ACCAGAGAAACACACGUUGUGGUACAUUACCUGGUA | AAAAACA GAU GUAUGCUU |

Table 17
Mouse *rel A* HH Target sequence

| nt. Position | HH Target Sequence | nt. Position | HH Target Sequence |
|---|---|---|---|
| 19 | AAUGGCU a caCaGgA | 467 | cCAGGCU c cuguUCg |
| 22 | aGCUCcU a cGUgGUG | 469 | AaGCcAU u AGcCAGC |
| 26 | CcUCcaU u GcGgACa | 473 | UuUgAGU C AGauCAg |
| 93 | GAuCUGU U uCCCCUC | 481 | AGCGaAU C CAGACCA |
| 94 | AuCUGUU u CCCCUCA | 501 | AACCCCU U uCAcGUU |
| 100 | UuCCCCU C AUCUUuC | 502 | ACCCCUU u CAcGUUC |
| 103 | CCCUCAU C UUuCCcu | 508 | UuCAcGU U CCUAUAG |
| 105 | CUCAUCU U uCCcuCA | 509 | uCAcGUU C CUAUAGA |
| 106 | UCAUCUU u CCcuCAG | 512 | cGUUCCU A UAGAgGA |
| 129 | CAGGCuU C UGGgCCu | 514 | UUCCUAU A GAgGAGC |
| 138 | GGgCCuU A UGUGGAG | 534 | GGGACU A uGACuUG |
| 148 | UGGAGAU C AUcGAaC | 556 | UGCGcCU C UGCUUCC |
| 151 | AGAUCAU c GAaCAGC | 561 | CUCUGCU U CCAGGUG |
| 180 | AUGCGaU U CCGCUAu | 562 | UCUGCUU C CAGGUGA |
| 181 | UGCGaUU C CGCUAuA | 585 | aAgCCAU u AGcCAGc |
| 186 | UUCCGCU A uAAaUGC | 598 | GGCCCCU C CuCCUGa |
| 204 | GGGCGCU C aGCGGGC | 613 | CcCCUGU C CUcuCaC |
| 217 | GCAGuAU u CCuGGCG | 616 | CUGUCCU c uCaCAUC |
| 239 | CACAGAU A CCACCAA | 617 | gucCCUU C CUCAgCC |
| 262 | CCACCAU C AAGAUCA | 620 | CCUUCCU C AgCCaug |
| 268 | UCAAGAU C AAUGGCU | 623 | UCCUgcU u CCAUCUc |
| 276 | AAUGGCU A CACAGGA | 628 | AUCCgAU u UUUGAuA |
| 301 | UuCGaAU C UCCCUGG | 630 | CCgAUuU U UGAuAAc |
| 303 | CGaAUCU C CCUGGUC | 631 | CgAUuUU U GAuAAcC |
| 310 | CCCUGGU C ACCAAGG | 638 | UGgCcAU u GUGuuCC |
| 323 | GGcCCCU C CUCcuga | 661 | CCGAGCU C AAGAUCU |
| 326 | uCCaCCU C ACCGGCC | 667 | UCAAGAU C UGCCGAG |
| 335 | CCGGCCU C AuCCaCA | 687 | CGgAACU C UGGgAGC |
| 349 | AuGAaCU U GUgGGgA | 700 | GCUGCCU C GGUGGGG |
| 352 | AGaUcaU c GaAcAGc | 715 | AUGAGAU C UUCuUgC |
| 375 | GAUGGCU a CUAUGAG | 717 | GAGAUCU U CuUgCUG |
| 376 | AUGGucU C UccGgaG | 718 | AGAUCUU C uUgCUGU |
| 378 | GGCUaCU A UGAGGCU | 721 | UucUCCU c CauUGcG |
| 391 | CUGAcCU C UGCCCaG | 751 | AaGACAU U GAGGUGU |
| 409 | GCaGuAU C CAuAGcU | 759 | GAGGUGU A UUUCACG |
| 416 | CCgCAGU a UCCAuAg | 761 | GGUGUAU U UCACGGG |
| 417 | CAuAGcU U CCAGAAC | 762 | GUGUAUU U CACGGGA |
| 418 | AuAGcUU C CAGAACC | 763 | UGUAUUU C ACGGGAC |
| 433 | UGGGgAU C CAGUGUG | 792 | CGAGGCU C CUUUUCu |
| 795 | GGCUCCU U UUCuCAA | 1167 | GAUGAGU U UuCCcCC |
| 796 | GCUCCUU U UCuCAAG | 1168 | AUGAGUU U uCCcCCA |
| 797 | CUCCUUU U CuCAAGC | 1169 | UGAGUUU u CCcCCAU |
| 798 | UCCUUUU C uCAAGCU | 1182 | AUGcUGU U aCCaUCa |
| 829 | UGGCCAU U GUGUUCC | 1183 | UGcUGUU a CCaUCaG |

| | | | |
|---|---|---|---|
| 834 | AUUGUGU | U | CCGGACu |
| 835 | UUGUGUU | C | CGGACuC |
| 845 | GACuCCU | C | CgUACGC |
| 849 | CCUCCgU | A | CGCcGAC |
| 872 | cCAGGCU | C | CUGUuCG |
| 883 | UuCGaGU | C | UCCAUGC |
| 885 | CGaGUCU | C | CAUGCAG |
| 905 | GCGGCCU | U | CuGAuCG |
| 906 | CGGCCUU | C | uGAuCGc |
| 919 | GcGAGCU | C | AGUGAGC |
| 936 | AUGGAgU | U | CCAGUAC |
| 937 | UGGAgUU | C | CAGUACu |
| 942 | UUCCAGU | A | CuUGCCA |
| 953 | GCCucAU | c | CAcAuGA |
| 962 | AGAuGAU | C | GcCACCG |
| 965 | CagUacU | u | gCCaGAc |
| 973 | ACCGGAU | U | GAaGAGA |
| 986 | GAgACcU | u | cAAGagu |
| 996 | AGGACcU | A | UGAGACC |
| 1005 | GAGACCU | U | CAAGAGu |
| 1006 | AGACCUU | C | AAGAGuA |
| 1015 | AGAGuAU | C | AUGAAGA |
| 1028 | GAAGAGU | C | CUUUCAa |
| 1031 | GAGUCCU | U | UCAauGG |
| 1032 | AGUCCUU | U | CAauGGA |
| 1033 | GUCCUUU | C | AauGGAC |
| 1058 | CCGGCCU | C | CAaCcCG |
| 1064 | UaCACCU | u | GAucCAa |
| 1072 | GgCGuAU | U | GCUGUGC |
| 1082 | UGUGCCU | a | CCCGaAa |
| 1083 | aaGCCUU | C | CCGaAGu |
| 1092 | CGaAaCU | C | AaCUUCU |
| 1097 | CUCAaCU | U | CUGUCCC |
| 1098 | UCAaCUU | C | UGUCCCC |
| 1102 | CUUCUGU | C | CCCAAGC |
| 1125 | CAGCCCU | A | caCCUUc |
| 1127 | GCCaUAU | a | gCcUUAC |
| 1131 | cAUCCCU | c | agCacCA |
| 1132 | AcaCCUU | c | cCagCAU |
| 1133 | UCCaUcU | c | CagCuUC |
| 1137 | UUUACuU | u | AgCgCgc |
| 1140 | cCagCAU | C | CCUcAGC |
| 1153 | GCACCAU | C | AACUuUG |
| 1158 | AUCAACU | u | UGAUGAG |
| 1680 | GAAGACU | U | CUCCUCC |
| 1681 | AAGACUU | C | UCCUCCA |
| 1683 | GACUUCU | C | CUCCAUU |
| 1686 | UUCUCCU | C | CAUUGCG |
| 1690 | CCUCCAU | U | GCGGACA |
| 1184 | GGccccU | C | CUcCUGa |
| 1187 | GUccCuU | c | CUcaGCc |
| 1188 | UUaCCaU | C | aGGGCAG |
| 1198 | GGgAGuU | u | AGuCuGa |
| 1209 | CAGcCCU | a | caCCUUc |
| 1215 | cuGGCCU | U | aGCaCCG |
| 1229 | GGuCCCU | u | CCucAGc |
| 1237 | CCCAgcU | C | CUGCCCC |
| 1250 | CCAGcCU | C | CAGgCuC |
| 1268 | CCCaGCU | C | CuGCCcc |
| 1279 | CCAUGGU | c | cCuuCcu |
| 1281 | gUGGgcU | C | AGCUgcG |
| 1286 | AUgAGuU | u | UccCCCA |
| 1309 | CuCCUGU | u | CgAGUCu |
| 1315 | cCCCAGU | u | CUAaCCC |
| 1318 | CAGUuCU | A | aCCCCgG |
| 1331 | gGGuCCU | C | CcCAGuC |
| 1334 | CuuUuCU | C | AaGCUGa |
| 1389 | ACGCUGU | C | gGAaGCC |
| 1413 | CUGCAGU | U | UGAUGcU |
| 1414 | UGCAGUU | U | GAUGcUG |
| 1437 | GGGGCCU | U | GCUUGGC |
| 1441 | CCUUGCU | U | GGCAACA |
| 1467 | GgaGUGU | U | CACAGAC |
| 1468 | gaGUGUU | C | ACAGACC |
| 1482 | CUGGCAU | C | uGUgGAC |
| 1486 | CuUCgGU | a | GggAACU |
| 1494 | GACAACU | C | aGAGUUU |
| 1500 | UCaGAGU | U | UCAGCAG |
| 1501 | CaGAGUU | U | CAGCAGC |
| 1502 | aGAGUUU | C | AGCAGCU |
| 1525 | gGuGCAU | c | CCUGUGu |
| 1566 | AUGGAGU | A | CCCUGAa |
| 1577 | UGAaGCU | A | UAACUCG |
| 1579 | AaGCUAU | A | ACUCGCC |
| 1583 | UAUAACU | C | GCCUgGU |
| 1588 | CUCuCCU | A | GaGAggG |
| 1622 | CCCAGCU | C | CUGCcCC |
| 1628 | UCCUGCU | u | CggUaGG |
| 1648 | CGGGGCU | u | CCCAAUG |
| 1660 | cUGaCCU | C | ugccCAG |
| 1663 | cuCUgCU | U | cCAGGuG |
| 1664 | uCUgCUU | c | CAGGuGA |
| 1665 | CUCgcUU | u | cGGAGgU |

| | |
|---|---|
| 1704 | AUGGACU U CUCuGCu |
| 1705 | UGGACUU C UCuGCuC |
| 1707 | GACUUCU C uGCuCUu |
| 1721 | uuUGAGU C AGAUCAG |
| 1726 | GUCAGAU C AGCUCCU |
| 1731 | AUCAGCU C CUAAGGu |
| 1734 | AGCUCCU A AGGuGcU |
| 1754 | CaGugCU C CCaAGAG |

Table 18
Human *rel A* HH Target Sequences

| nt. Position | HH Target Sequence | nt. Position | HH Target Sequence |
|---|---|---|---|
| 19 | AAUGGCU C GUCUGUA | 467 | GCAGGCU A UCAGUCA |
| 22 | GGCUCGU C UGUAGUG | 469 | AGGCUAU C AGUCAGC |
| 26 | CGUCUGU A GUGCACG | 473 | UAUCAGU C AGCGCAU |
| 93 | GAACUGU U CCCCCUC | 481 | AGCGCAU C CAGACCA |
| 94 | AACUGUU C CCCCUCA | 501 | AACCCCU U CCAAGUU |
| 100 | UCCCCCU C AUCUUCC | 502 | ACCCCUU C CAAGUUC |
| 103 | CCCUCAU C UUCCCGG | 508 | UCCAAGU U CCUAUAG |
| 105 | CUCAUCU U CCCGGCA | 509 | CCAAGUU C CUAUAGA |
| 106 | UCAUCUU C CCGGCAG | 512 | AGUUCCU A UAGAAGA |
| 129 | CAGGCCU C UGGCCCC | 514 | UUCCUAU A GAAGAGC |
| 138 | GGCCCCU A UGUGGAG | 534 | GGGGACU A CGACCUG |
| 148 | UGGAGAU C AUUGAGC | 556 | UGCGGCU C UGCUUCC |
| 151 | AGAUCAU U GAGCAGC | 561 | CUCUGCU U CCAGGUG |
| 180 | AUGCGCU U CCGCUAC | 562 | UCUGCUU C CAGGUGA |
| 181 | UGCGCUU C CGCUACA | 585 | GACCCAU C AGGCAGG |
| 186 | UUCCGCU A CAAGUGC | 598 | GGCCCCU C CGCCUGC |
| 204 | GGGCGCU C CGCGGGC | 613 | CGCCUGU C CUUCCUC |
| 217 | GCAGCAU C CCAGGCG | 616 | CUGUCCU U CCUCAUC |
| 239 | CACAGAU A CCACCAA | 617 | UGUCCUU C CUCAUCC |
| 262 | CCACCAU C AAGAUCA | 620 | CCUUCCU C AUCCCAU |
| 268 | UCAAGAU C AAUGGCU | 623 | UCCUCAU C CCAUCUU |
| 276 | AAUGGCU A CACAGGA | 628 | AUCCCAU C UUUGACA |
| 301 | UGCGCAU C UCCCUGG | 630 | CCCAUCU U UGACAAU |
| 303 | CGCAUCU C CCUGGUC | 631 | CCAUCUU U GACAAUC |
| 310 | CCCUGGU C ACCAAGG | 638 | UGACAAU C GUGCCCC |
| 323 | GGACCCU C CUCACCG | 661 | CCGAGCU C AAGAUCU |
| 326 | CCCUCCU C ACCGGCC | 667 | UCAAGAU C UGCCGAG |
| 335 | CCGGCCU C ACCCCCA | 687 | CGAAACU C UGGCAGC |
| 349 | ACGAGCU U GUAGGAA | 700 | GCUGCCU C GGUGGGG |
| 352 | AGCUUGU A GGAAAGG | 715 | AUGAGAU C UUCCUAC |
| 375 | GAUGGCU U CUAUGAG | 717 | GAGAUCU U CCUACUG |
| 376 | AUGGCUU C UAUGAGG | 718 | AGAUCUU C CUACUGU |
| 378 | GGCUUCU A UGAGGCU | 721 | UCUUCCU A CUGUGUG |
| 391 | CUGAGCU C UGCCCGG | 751 | AGGACAU U GAGGUGU |
| 409 | GCUGCAU C CACAGUU | 759 | GAGGUGU A UUUCACG |
| 416 | CCACAGU U UCCAGAA | 761 | GGUGUAU U UCACGGG |
| 417 | CACAGUU U CCAGAAC | 762 | GUGUAUU U CACGGGA |
| 418 | ACAGUUU C CAGAACC | 763 | UGUAUUU C ACGGGAC |
| 433 | UGGGAAU C CAGUGUG | 792 | CGAGGCU C CUUUUCG |
| 795 | GGCUCCU U UUCGCAA | 1167 | GAUGAGU U UCCCACC |
| 796 | GCUCCUU U UCGCAAG | 1168 | AUGAGUU U CCCACCA |
| 797 | CUCCUUU U CGCAAGC | 1169 | UGAGUUU C CCACCAU |
| 798 | UCCUUUU C GCAAGCU | 1182 | AUGGUGU U UCCUUCU |
| 829 | UGGCCAU U GUGUUCC | 1183 | UGGUGUU U CCUUCUG |
| 834 | AUUGUGU U CCGGACC | 1184 | GGUGUUU C CUUCUGG |

| | | | |
|---|---|---|---|
| 835 | UUGUGUU C CGGACCC | 1187 | GUUUCCU U CUGGGCA |
| 845 | GACCCCU C CCUACGC | 1188 | UUUCCUU C UGGGCAG |
| 849 | CCUCCCU A CGCAGAC | 1198 | GGCAGAU C AGCCAGG |
| 872 | GCAGGCU C CUGUGCG | 1209 | CAGGCCU C GGCCUUG |
| 883 | UGCGUGU C UCCAUGC | 1215 | UCGGCCU U GGCCCCG |
| 885 | CGUGUCU C CAUGCAG | 1229 | GGCCCCU C CCCAAGU |
| 905 | GCGGCCU U CCGACCG | 1237 | CCCAAGU C CUGCCCC |
| 906 | CGGCCUU C CGACCGG | 1250 | CCAGGCU C CAGCCCC |
| 919 | GGGAGCU C AGUGAGC | 1268 | CCCUGCU C CAGCCAU |
| 936 | AUGGAAU U CCAGUAC | 1279 | CCAUGGU A UCAGCUC |
| 937 | UGGAAUU C CAGUACC | 1281 | AUGGUAU C AGCUCUG |
| 942 | UUCCAGU A CCUGCCA | 1286 | AUCAGCU C UGGCCCA |
| 953 | GCCAGAU A CAGACGA | 1309 | CCCCUGU C CCAGUCC |
| 962 | AGACGAU C GUCACCG | 1315 | UCCCAGU C CUAGCCC |
| 965 | CGAUCGU C ACCGGAU | 1318 | CAGUCCU A GCCCCAG |
| 973 | ACCGGAU U GAGGAGA | 1331 | AGGCCCU C CUCAGGC |
| 986 | GAAACGU A AAAGGAC | 1334 | CCCUCCU C AGGCUGU |
| 996 | AGGACAU A UGAGACC | 1389 | ACGCUGU C AGAGGCC |
| 1005 | GAGACCU U CAAGAGC | 1413 | CUGCAGU U UGAUGAU |
| 1006 | AGACCUU C AAGAGCA | 1414 | UGCAGUU U GAUGAUG |
| 1015 | AGAGCAU C AUGAAGA | 1437 | GGGGCCU U GCUUGGC |
| 1028 | GAAGAGU C CUUUCAG | 1441 | CCUUGCU U GGCAACA |
| 1031 | GAGUCCU U UCAGCGG | 1467 | GCUGUGU U CACAGAC |
| 1032 | AGUCCUU U CAGCGGA | 1468 | CUGUGUU C ACAGACC |
| 1033 | GUCCUUU C AGCGGAC | 1482 | CUGGCAU C CGUCGAC |
| 1058 | CCGGCCU C CACCUCG | 1486 | CAUCCGU C GACAACU |
| 1064 | UCCACCU C GACGCAU | 1494 | GACAACU C CGAGUUU |
| 1072 | GACGCAU U GCUGUGC | 1500 | UCCGAGU U UCAGCAG |
| 1082 | UGUGCCU U CCCGCAG | 1501 | CCGAGUU U CAGCAGC |
| 1083 | GUGCCUU C CCGCAGC | 1502 | CGAGUUU C AGCAGCU |
| 1092 | CGCAGCU C AGCUUCU | 1525 | AGGGCAU A CCUGUGG |
| 1097 | CUCAGCU U CUGUCCC | 1566 | AUGGAGU A CCCUGAG |
| 1098 | UCAGCUU C UGUCCCC | 1577 | UGAGGCU A UAACUCG |
| 1102 | CUUCUGU C CCCAAGC | 1579 | AGGCUAU A ACUCGCC |
| 1125 | CAGCCCU A UCCCUUU | 1583 | UAUAACU C GCCUAGU |
| 1127 | GCCCUAU C CCUUUAC | 1588 | CUCGCCU A GUGACAG |
| 1131 | UAUCCCU U UACGUCA | 1622 | CCCAGCU C CUGCUCC |
| 1132 | AUCCCUU U ACGUCAU | 1628 | UCCUGCU C CACUGGG |
| 1133 | UCCCUUU A CGUCAUC | 1648 | CGGGGCU C CCCAAUG |
| 1137 | UUUACGU C AUCCCUG | 1660 | AUGGCCU C CUUUCAG |
| 1140 | ACGUCAU C CCUGAGC | 1663 | GCCUCCU U UCAGGAG |
| 1153 | GCACCAU C AACUAUG | 1664 | CCUCCUU U CAGGAGA |
| 1158 | AUCAACU A UGAUGAG | 1665 | CUCCUUU C AGGAGAU |
| 1680 | GAAGACU U CUCCUCC | | |
| 1681 | AAGACUU C UCCUCCA | | |
| 1683 | GACUUCU C CUCCAUU | | |
| 1686 | UUCUCCU C CAUUGCG | | |
| 1690 | CCUCCAU U GCGGACA | | |
| 1704 | AUGGACU U CUCAGCC | | |

| | |
|---|---|
| 1705 | UGGACUU C UCAGCCC |
| 1707 | GACUUCU C AGCCCUG |
| 1721 | GCUGAGU C AGAUCAG |
| 1726 | GUCAGAU C AGCUCCU |
| 1731 | AUCAGCU C CUAAGGG |
| 1734 | AGCUCCU A AGGGGGU |
| 1754 | CUGCCCU C CCCAGAG |

Table 19
Mouse *rel A* HH Ribozyme Sequences

| nt. Sequence | HH Ribozyme Sequence |
|---|---|
| 19 | UCCUGUG CUGAUGAGGCCGAAAGGCCGAA AGCCAUU |
| 22 | CACCACG CUGAUGAGGCCGAAAGGCCGAA AGGAGCU |
| 26 | UGUCCGC CUGAUGAGGCCGAAAGGCCGAA AUGGAGG |
| 93 | GAGGGGA CUGAUGAGGCCGAAAGGCCGAA ACAGAUC |
| 94 | UGAGGGG CUGAUGAGGCCGAAAGGCCGAA AACAGAU |
| 100 | GAAAGAU CUGAUGAGGCCGAAAGGCCGAA AGGGGAA |
| 103 | AGGGAAA CUGAUGAGGCCGAAAGGCCGAA AUGAGGG |
| 105 | UGAGGGA CUGAUGAGGCCGAAAGGCCGAA AGAUGAG |
| 106 | CUGAGGG CUGAUGAGGCCGAAAGGCCGAA AAGAUGA |
| 129 | AGGCCCA CUGAUGAGGCCGAAAGGCCGAA AAGCCUG |
| 138 | CUCCACA CUGAUGAGGCCGAAAGGCCGAA AAGGCCC |
| 148 | GUUCGAU CUGAUGAGGCCGAAAGGCCGAA AUCUCCA |
| 151 | GCUGUUC CUGAUGAGGCCGAAAGGCCGAA AUGAUCU |
| 180 | AUAGCGG CUGAUGAGGCCGAAAGGCCGAA AUCGCAU |
| 181 | UAUAGCG CUGAUGAGGCCGAAAGGCCGAA AAUCGCA |
| 186 | GCAUUUA CUGAUGAGGCCGAAAGGCCGAA AGCUGAA |
| 204 | GCCCGCU CUGAUGAGGCCGAAAGGCCGAA AGCGCCC |
| 217 | CGCCAGG CUGAUGAGGCCGAAAGGCCGAA AUACUGC |
| 239 | UUGGUGG CUGAUGAGGCCGAAAGGCCGAA AUCUGUG |
| 262 | UGAUCUU CUGAUGAGGCCGAAAGGCCGAA AUGGUGG |
| 268 | AGCCAUU CUGAUGAGGCCGAAAGGCCGAA AUCUUGA |
| 276 | UCCUGUG CUGAUGAGGCCGAAAGGCCGAA AGCCAUU |
| 301 | CCAGGGA CUGAUGAGGCCGAAAGGCCGAA AUUCGAA |
| 303 | GACCAGG CUGAUGAGGCCGAAAGGCCGAA AGAUUCG |
| 310 | CCUUGGU CUGAUGAGGCCGAAAGGCCGAA ACCAGGG |
| 323 | UCAGGAG CUGAUGAGGCCGAAAGGCCGAA AGGGGCC |
| 326 | GGCCGGU CUGAUGAGGCCGAAAGGCCGAA AGGUGGA |
| 335 | UGUGGAU CUGAUGAGGCCGAAAGGCCGAA AGGCCGG |
| 349 | UCCCCAC CUGAUGAGGCCGAAAGGCCGAA AGUUCAU |
| 352 | GCUGUUC CUGAUGAGGCCGAAAGGCCGAA AUGAUCU |
| 375 | CUCAUAG CUGAUGAGGCCGAAAGGCCGAA AGCCAUC |
| 376 | CUCCGGA CUGAUGAGGCCGAAAGGCCGAA AGACCAU |
| 378 | AGCCUCA CUGAUGAGGCCGAAAGGCCGAA AGUAGCC |
| 391 | CUGGGCA CUGAUGAGGCCGAAAGGCCGAA AGGUCAG |
| 409 | AGCUAUG CUGAUGAGGCCGAAAGGCCGAA AUACUGC |
| 416 | CUAUGGA CUGAUGAGGCCGAAAGGCCGAA ACUGCGG |
| 417 | GUUCUGG CUGAUGAGGCCGAAAGGCCGAA AGCUAUG |
| 418 | GGUUCUG CUGAUGAGGCCGAAAGGCCGAA AAGCUAU |
| 433 | CACACUG CUGAUGAGGCCGAAAGGCCGAA AUCCCCA |
| 467 | CGAACAG CUGAUGAGGCCGAAAGGCCGAA AGCCUGG |
| 469 | GCUGGCU CUGAUGAGGCCGAAAGGCCGAA AUGGCUU |
| 473 | CUGAUCU CUGAUGAGGCCGAAAGGCCGAA ACUCAAA |
| 481 | UGGUCUG CUGAUGAGGCCGAAAGGCCGAA AUUCGCU |

| | | | | |
|---|---|---|---|---|
| 501 | AACGUGA | CUGAUGAGGCCGAAAGGCCGAA | AGGGGUU |
| 502 | GAACGUG | CUGAUGAGGCCGAAAGGCCGAA | AAGGGGU |
| 508 | CUAUAGG | CUGAUGAGGCCGAAAGGCCGAA | ACGUGAA |
| 509 | UCUAUAG | CUGAUGAGGCCGAAAGGCCGAA | AACGUGA |
| 512 | UCCUCUA | CUGAUGAGGCCGAAAGGCCGAA | AGGAACG |
| 514 | GCUCCUC | CUGAUGAGGCCGAAAGGCCGAA | AUAGGAA |
| 534 | CAAGUCA | CUGAUGAGGCCGAAAGGCCGAA | AGUCCCC |
| 556 | GGAAGCA | CUGAUGAGGCCGAAAGGCCGAA | AGGCGCA |
| 561 | CACCUGG | CUGAUGAGGCCGAAAGGCCGAA | AGCAGAG |
| 562 | UCACCUG | CUGAUGAGGCCGAAAGGCCGAA | AAGCAGA |
| 585 | GCUGGCU | CUGAUGAGGCCGAAAGGCCGAA | AUGGCUU |
| 598 | UCAGGAG | CUGAUGAGGCCGAAAGGCCGAA | AGGGGCC |
| 613 | GUGAGAG | CUGAUGAGGCCGAAAGGCCGAA | ACAGGGG |
| 616 | GAUGUGA | CUGAUGAGGCCGAAAGGCCGAA | AGGACAG |
| 617 | GGCUGAG | CUGAUGAGGCCGAAAGGCCGAA | AAGGGAC |
| 620 | CAUGGCU | CUGAUGAGGCCGAAAGGCCGAA | AGGAAGG |
| 623 | GAGAUGG | CUGAUGAGGCCGAAAGGCCGAA | AGCAGGA |
| 628 | UAUCAAA | CUGAUGAGGCCGAAAGGCCGAA | AUCGGAU |
| 630 | GUUAUCA | CUGAUGAGGCCGAAAGGCCGAA | AAAUCGG |
| 631 | GGUUAUC | CUGAUGAGGCCGAAAGGCCGAA | AAAAUCG |
| 638 | GGAACAC | CUGAUGAGGCCGAAAGGCCGAA | AUGGCCA |
| 661 | AGAUCUU | CUGAUGAGGCCGAAAGGCCGAA | AGCUCGG |
| 667 | CUCGGCA | CUGAUGAGGCCGAAAGGCCGAA | AUCUUGA |
| 687 | GCUCCCA | CUGAUGAGGCCGAAAGGCCGAA | AGUUCCG |
| 700 | CCCCACC | CUGAUGAGGCCGAAAGGCCGAA | AGGCAGC |
| 715 | GCAAGAA | CUGAUGAGGCCGAAAGGCCGAA | AUCUCAU |
| 717 | CAGCAAG | CUGAUGAGGCCGAAAGGCCGAA | AGAUCUC |
| 718 | ACAGCAA | CUGAUGAGGCCGAAAGGCCGAA | AAGAUCU |
| 721 | CGCAAUG | CUGAUGAGGCCGAAAGGCCGAA | AGGAGAA |
| 751 | ACACCUC | CUGAUGAGGCCGAAAGGCCGAA | AUGUCUU |
| 759 | CGUGAAA | CUGAUGAGGCCGAAAGGCCGAA | ACACCUC |
| 761 | CCCGUGA | CUGAUGAGGCCGAAAGGCCGAA | AUACACC |
| 762 | UCCCGUG | CUGAUGAGGCCGAAAGGCCGAA | AAUACAC |
| 763 | GUCCCGU | CUGAUGAGGCCGAAAGGCCGAA | AAAUACA |
| 792 | AGAAAAG | CUGAUGAGGCCGAAAGGCCGAA | AGCCUCG |
| 795 | UUGAGAA | CUGAUGAGGCCGAAAGGCCGAA | AGGAGCC |
| 796 | CUUGAGA | CUGAUGAGGCCGAAAGGCCGAA | AAGGAGC |
| 797 | GCUUGAG | CUGAUGAGGCCGAAAGGCCGAA | AAAGGAG |
| 798 | AGCUUGA | CUGAUGAGGCCGAAAGGCCGAA | AAAAGGA |
| 829 | GGAACAC | CUGAUGAGGCCGAAAGGCCGAA | AUGGCCA |
| 834 | AGUCCGG | CUGAUGAGGCCGAAAGGCCGAA | ACACAAU |
| 835 | GAGUCCG | CUGAUGAGGCCGAAAGGCCGAA | AACACAA |
| 845 | GCGUACG | CUGAUGAGGCCGAAAGGCCGAA | AGGAGUC |
| 849 | GUCGGCG | CUGAUGAGGCCGAAAGGCCGAA | ACGGAGG |
| 872 | CGAACAG | CUGAUGAGGCCGAAAGGCCGAA | AGCCUGG |
| 883 | GCAUGGA | CUGAUGAGGCCGAAAGGCCGAA | ACUCGAA |
| 885 | CUGCAUG | CUGAUGAGGCCGAAAGGCCGAA | AGACUCG |
| 905 | CGAUCAG | CUGAUGAGGCCGAAAGGCCGAA | AGGCCGC |
| 906 | GCGAUCA | CUGAUGAGGCCGAAAGGCCGAA | AAGGCCG |

| | |
|---|---|
| 919 | GCUCACU CUGAUGAGGCCGAAAGGCCGAA AGCUCGC |
| 936 | GUACUGG CUGAUGAGGCCGAAAGGCCGAA ACUCCAU |
| 937 | AGUACUG CUGAUGAGGCCGAAAGGCCGAA AACUCCA |
| 942 | UGGCAAG CUGAUGAGGCCGAAAGGCCGAA ACUGGAA |
| 953 | UCAUGUG CUGAUGAGGCCGAAAGGCCGAA AUGAGGC |
| 962 | CGGUGGC CUGAUGAGGCCGAAAGGCCGAA AUCAUCU |
| 965 | GUCUGGC CUGAUGAGGCCGAAAGGCCGAA AGUACUG |
| 973 | UCUCUUC CUGAUGAGGCCGAAAGGCCGAA AUCCGGU |
| 986 | ACUCUUG CUGAUGAGGCCGAAAGGCCGAA AGGUCUC |
| 996 | GGUCUCA CUGAUGAGGCCGAAAGGCCGAA AGGUCCU |
| 1005 | ACUCUUG CUGAUGAGGCCGAAAGGCCGAA AGGUCUC |
| 1006 | UACUCUU CUGAUGAGGCCGAAAGGCCGAA AAGGUCU |
| 1015 | UCUUCAU CUGAUGAGGCCGAAAGGCCGAA AUACUCU |
| 1028 | UUGAAAG CUGAUGAGGCCGAAAGGCCGAA ACUCUUC |
| 1031 | CCAUUGA CUGAUGAGGCCGAAAGGCCGAA AGGACUC |
| 1032 | UCCAUUG CUGAUGAGGCCGAAAGGCCGAA AAGGACU |
| 1033 | GUCCAUU CUGAUGAGGCCGAAAGGCCGAA AAAGGAC |
| 1058 | CGGGUUG CUGAUGAGGCCGAAAGGCCGAA AGGCCGG |
| 1064 | UUGGAUC CUGAUGAGGCCGAAAGGCCGAA AGGUGUA |
| 1072 | GCACAGC CUGAUGAGGCCGAAAGGCCGAA AUACGCC |
| 1082 | UUUCGGG CUGAUGAGGCCGAAAGGCCGAA AGGCACA |
| 1083 | ACUUCGG CUGAUGAGGCCGAAAGGCCGAA AAGGCUU |
| 1092 | AGAAGUU CUGAUGAGGCCGAAAGGCCGAA AGUUUCG |
| 1097 | GGGACAG CUGAUGAGGCCGAAAGGCCGAA AGUUGAG |
| 1098 | GGGGACA CUGAUGAGGCCGAAAGGCCGAA AAGUUGA |
| 1102 | GCUUGGG CUGAUGAGGCCGAAAGGCCGAA ACAGAAG |
| 1125 | GAAGGUG CUGAUGAGGCCGAAAGGCCGAA AGGGCUG |
| 1127 | GUAAGGC CUGAUGAGGCCGAAAGGCCGAA AUAUGGC |
| 1131 | UGGUGCU CUGAUGAGGCCGAAAGGCCGAA AGGGAUG |
| 1132 | AUGCUGG CUGAUGAGGCCGAAAGGCCGAA AAGGUGU |
| 1133 | GAAGCUG CUGAUGAGGCCGAAAGGCCGAA AGAUGGA |
| 1137 | GCGCGCU CUGAUGAGGCCGAAAGGCCGAA AAGUAAA |
| 1140 | GCUGAGG CUGAUGAGGCCGAAAGGCCGAA AUGCUGG |
| 1153 | CAAAGUU CUGAUGAGGCCGAAAGGCCGAA AUGGUGC |
| 1158 | CUCAUCA CUGAUGAGGCCGAAAGGCCGAA AGUUGAU |
| 1167 | GGGGGAA CUGAUGAGGCCGAAAGGCCGAA ACUCAUC |
| 1168 | UGGGGGA CUGAUGAGGCCGAAAGGCCGAA AACUCAU |
| 1169 | AUGGGGG CUGAUGAGGCCGAAAGGCCGAA AAACUCA |
| 1182 | UGAUGGU CUGAUGAGGCCGAAAGGCCGAA ACAGCAU |
| 1183 | CUGAUGG CUGAUGAGGCCGAAAGGCCGAA AACAGCA |
| 1184 | UCAGGAG CUGAUGAGGCCGAAAGGCCGAA AGGGGCC |
| 1187 | GGCUGAG CUGAUGAGGCCGAAAGGCCGAA AAGGGAC |
| 1188 | CUGCCCU CUGAUGAGGCCGAAAGGCCGAA AUGGUAA |
| 1198 | UCAGACU CUGAUGAGGCCGAAAGGCCGAA AACUCCC |
| 1209 | GAAGGUG CUGAUGAGGCCGAAAGGCCGAA AGGGCUG |
| 1215 | CGGUGCU CUGAUGAGGCCGAAAGGCCGAA AGGCCAG |
| 1229 | GCUGAGG CUGAUGAGGCCGAAAGGCCGAA AGGGACC |
| 1237 | GGGGCAG CUGAUGAGGCCGAAAGGCCGAA AGCUGGG |
| 1250 | GAGCCUG CUGAUGAGGCCGAAAGGCCGAA AGGCUGG |

| | | |
|---|---|---|
| 1268 | GGGGCAG CUGAUGAGGCCGAAAGGCCGAA AGCUGGG | |
| 1279 | AGGAAGG CUGAUGAGGCCGAAAGGCCGAA ACCAUGG | |
| 1281 | CGCAGCU CUGAUGAGGCCGAAAGGCCGAA AGCCCAC | |
| 1286 | UGGGGGA CUGAUGAGGCCGAAAGGCCGAA AACUCAU | |
| 1309 | AGACUCG CUGAUGAGGCCGAAAGGCCGAA ACAGGAG | |
| 1315 | GGGUUAG CUGAUGAGGCCGAAAGGCCGAA ACUGGGG | |
| 1318 | CCGGGGU CUGAUGAGGCCGAAAGGCCGAA AGAACUG | |
| 1331 | GACUGGG CUGAUGAGGCCGAAAGGCCGAA AGGACCC | |
| 1334 | UCAGCUU CUGAUGAGGCCGAAAGGCCGAA AGAAAAG | |
| 1389 | GGCUUCC CUGAUGAGGCCGAAAGGCCGAA ACAGCGU | |
| 1413 | AGCAUCA CUGAUGAGGCCGAAAGGCCGAA ACUGCAG | |
| 1414 | CAGCAUC CUGAUGAGGCCGAAAGGCCGAA AACUGCA | |
| 1437 | GCCAAGC CUGAUGAGGCCGAAAGGCCGAA AGGCCCC | |
| 1441 | UGUUGCC CUGAUGAGGCCGAAAGGCCGAA AGCAAGG | |
| 1467 | GUCUGUG CUGAUGAGGCCGAAAGGCCGAA ACACUCC | |
| 1468 | GGUCUGU CUGAUGAGGCCGAAAGGCCGAA AACACUC | |
| 1482 | GUCCACA CUGAUGAGGCCGAAAGGCCGAA AUGCCAG | |
| 1486 | AGUUCCC CUGAUGAGGCCGAAAGGCCGAA ACCGAAG | |
| 1494 | AAACUCU CUGAUGAGGCCGAAAGGCCGAA AGUUGUC | |
| 1500 | CUGCUGA CUGAUGAGGCCGAAAGGCCGAA ACUCUGA | |
| 1501 | GCUGCUG CUGAUGAGGCCGAAAGGCCGAA AACUCUG | |
| 1502 | AGCUGCU CUGAUGAGGCCGAAAGGCCGAA AAACUCU | |
| 1525 | ACACAGG CUGAUGAGGCCGAAAGGCCGAA AUGCACC | |
| 1566 | UUCAGGG CUGAUGAGGCCGAAAGGCCGAA ACUCCAU | |
| 1577 | CGAGUUA CUGAUGAGGCCGAAAGGCCGAA AGCUUCA | |
| 1579 | GGCGAGU CUGAUGAGGCCGAAAGGCCGAA AUAGCUU | |
| 1583 | ACCAGGC CUGAUGAGGCCGAAAGGCCGAA AGUUAUA | |
| 1588 | CCCUCUC CUGAUGAGGCCGAAAGGCCGAA AGGAGAG | |
| 1622 | GGGGCAG CUGAUGAGGCCGAAAGGCCGAA AGCUGGG | |
| 1628 | CCUACCG CUGAUGAGGCCGAAAGGCCGAA AGCAGGA | |
| 1648 | CAUUGGG CUGAUGAGGCCGAAAGGCCGAA AGCCCCG | |
| 1660 | CUGGGCA CUGAUGAGGCCGAAAGGCCGAA AGGUCAG | |
| 1663 | CACCUGG CUGAUGAGGCCGAAAGGCCGAA AGCAGAG | |
| 1664 | UCACCUG CUGAUGAGGCCGAAAGGCCGAA AAGCAGA | |
| 1665 | ACCUCCG CUGAUGAGGCCGAAAGGCCGAA AAGCGAG | |
| 1680 | GGAGGAG CUGAUGAGGCCGAAAGGCCGAA AGUCUUC | |
| 1681 | UGGAGGA CUGAUGAGGCCGAAAGGCCGAA AAGUCUU | |
| 1683 | AAUGGAG CUGAUGAGGCCGAAAGGCCGAA AGAAGUC | |
| 1686 | CGCAAUG CUGAUGAGGCCGAAAGGCCGAA AGGAGAA | |
| 1690 | UGUCCGC CUGAUGAGGCCGAAAGGCCGAA AUGGAGG | |
| 1704 | AGCAGAG CUGAUGAGGCCGAAAGGCCGAA AGUCCAU | |
| 1705 | GAGCAGA CUGAUGAGGCCGAAAGGCCGAA AAGUCCA | |
| 1707 | AAGAGCA CUGAUGAGGCCGAAAGGCCGAA AGAAGUC | |
| 1721 | CUGAUCU CUGAUGAGGCCGAAAGGCCGAA ACUCAAA | |
| 1726 | AGGAGCU CUGAUGAGGCCGAAAGGCCGAA AUCUGAC | |
| 1731 | ACCUUAG CUGAUGAGGCCGAAAGGCCGAA AGCUGAU | |
| 1734 | AGCACCU CUGAUGAGGCCGAAAGGCCGAA AGGAGCU | |
| 1754 | CUCUUGG CUGAUGAGGCCGAAAGGCCGAA AGCACUG | |

Table 20
Human *rel A* HH Ribozyme Sequences

| nt. Position | HH Ribozyme Sequences |
|---|---|
| 19 | UACAGAC CUGAUGAGGCCGAAAGGCCGAA AGCCAUU |
| 22 | CACUACA CUGAUGAGGCCGAAAGGCCGAA ACGAGCC |
| 26 | CGUGCAC CUGAUGAGGCCGAAAGGCCGAA ACAGACG |
| 93 | GAGGGGG CUGAUGAGGCCGAAAGGCCGAA ACAGUUC |
| 94 | UGAGGGG CUGAUGAGGCCGAAAGGCCGAA AACAGUU |
| 100 | GGAAGAU CUGAUGAGGCCGAAAGGCCGAA AGGGGGA |
| 103 | CCGGGAA CUGAUGAGGCCGAAAGGCCGAA AUGAGGG |
| 105 | UGCCGGG CUGAUGAGGCCGAAAGGCCGAA AGAUGAG |
| 106 | CUGCCGG CUGAUGAGGCCGAAAGGCCGAA AAGAUGA |
| 129 | GGGGCCA CUGAUGAGGCCGAAAGGCCGAA AGGCCUG |
| 138 | CUCCACA CUGAUGAGGCCGAAAGGCCGAA AGGGGCC |
| 148 | GCUCAAU CUGAUGAGGCCGAAAGGCCGAA AUCUCCA |
| 151 | GCUGCUC CUGAUGAGGCCGAAAGGCCGAA AUGAUCU |
| 180 | GUAGCGG CUGAUGAGGCCGAAAGGCCGAA AGCGCAU |
| 181 | UGUAGCG CUGAUGAGGCCGAAAGGCCGAA AAGCGCA |
| 186 | GCACUUG CUGAUGAGGCCGAAAGGCCGAA AGCGGAA |
| 204 | GCCCGCG CUGAUGAGGCCGAAAGGCCGAA AGCGCCC |
| 217 | CGCCUGG CUGAUGAGGCCGAAAGGCCGAA AUGCUGC |
| 239 | UUGGUGG CUGAUGAGGCCGAAAGGCCGAA AUCUGUG |
| 262 | UGAUCUU CUGAUGAGGCCGAAAGGCCGAA AUGGUGG |
| 268 | AGCCAUU CUGAUGAGGCCGAAAGGCCGAA AUCUUGA |
| 276 | UCCUGUG CUGAUGAGGCCGAAAGGCCGAA AGCCAUU |
| 301 | CCAGGGA CUGAUGAGGCCGAAAGGCCGAA AUGCGCA |
| 303 | GACCAGG CUGAUGAGGCCGAAAGGCCGAA AGAUGCG |
| 310 | CCUUGGU CUGAUGAGGCCGAAAGGCCGAA ACCAGGG |
| 323 | CGGUGAG CUGAUGAGGCCGAAAGGCCGAA AGGGUCC |
| 326 | GGCCGGU CUGAUGAGGCCGAAAGGCCGAA AGGAGGG |
| 335 | UGGGGGU CUGAUGAGGCCGAAAGGCCGAA AGGCCGG |
| 349 | UUCCUAC CUGAUGAGGCCGAAAGGCCGAA AGCUCGU |
| 352 | CCUUUCC CUGAUGAGGCCGAAAGGCCGAA ACAAGCU |
| 375 | CUCAUAG CUGAUGAGGCCGAAAGGCCGAA AGCCAUC |
| 376 | CCUCAUA CUGAUGAGGCCGAAAGGCCGAA AAGCCAU |
| 378 | AGCCUCA CUGAUGAGGCCGAAAGGCCGAA AGAAGCC |
| 391 | CCGGGCA CUGAUGAGGCCGAAAGGCCGAA AGCUCAG |
| 409 | AACUGUG CUGAUGAGGCCGAAAGGCCGAA AUGCAGC |
| 416 | UUCUGGA CUGAUGAGGCCGAAAGGCCGAA ACUGUGG |
| 417 | GUUCUGG CUGAUGAGGCCGAAAGGCCGAA AACUGUG |
| 418 | GGUUCUG CUGAUGAGGCCGAAAGGCCGAA AAACUGU |
| 433 | CACACUG CUGAUGAGGCCGAAAGGCCGAA AUUCCCA |
| 467 | UGACUGA CUGAUGAGGCCGAAAGGCCGAA AGCCUGC |
| 469 | GCUGACU CUGAUGAGGCCGAAAGGCCGAA AUAGCCU |
| 473 | AUGCGCU CUGAUGAGGCCGAAAGGCCGAA ACUGAUA |
| 481 | UGGUCUG CUGAUGAGGCCGAAAGGCCGAA AUGCGCU |
| 501 | AACUUGG CUGAUGAGGCCGAAAGGCCGAA AGGGGUU |

| | | | |
|---|---|---|---|
| 502 | GAACUUG | CUGAUGAGGCCGAAAGGCCGAA | AAGGGGU |
| 508 | CUAUAGG | CUGAUGAGGCCGAAAGGCCGAA | ACUUGGA |
| 509 | UCUAUAG | CUGAUGAGGCCGAAAGGCCGAA | AACUUGG |
| 512 | UCUUCUA | CUGAUGAGGCCGAAAGGCCGAA | AGGAACU |
| 514 | GCUCUUC | CUGAUGAGGCCGAAAGGCCGAA | AUAGGAA |
| 534 | CAGGUCG | CUGAUGAGGCCGAAAGGCCGAA | AGUCCCC |
| 556 | GGAAGCA | CUGAUGAGGCCGAAAGGCCGAA | AGCCGCA |
| 561 | CACCUGG | CUGAUGAGGCCGAAAGGCCGAA | AGCAGAG |
| 562 | UCACCUG | CUGAUGAGGCCGAAAGGCCGAA | AAGCAGA |
| 585 | CCUGCCU | CUGAUGAGGCCGAAAGGCCGAA | AUGGGUC |
| 598 | GCAGGCG | CUGAUGAGGCCGAAAGGCCGAA | AGGGGCC |
| 613 | GAGGAAG | CUGAUGAGGCCGAAAGGCCGAA | ACAGGCG |
| 616 | GAUGAGG | CUGAUGAGGCCGAAAGGCCGAA | AGGACAG |
| 617 | GGAUGAG | CUGAUGAGGCCGAAAGGCCGAA | AAGGACA |
| 620 | AUGGGAU | CUGAUGAGGCCGAAAGGCCGAA | AGGAAGG |
| 623 | AAGAUGG | CUGAUGAGGCCGAAAGGCCGAA | AUGAGGA |
| 628 | UGUCAAA | CUGAUGAGGCCGAAAGGCCGAA | AUGGGAU |
| 630 | AUUGUCA | CUGAUGAGGCCGAAAGGCCGAA | AGAUGGG |
| 631 | GAUUGUC | CUGAUGAGGCCGAAAGGCCGAA | AAGAUGG |
| 638 | GGGGCAC | CUGAUGAGGCCGAAAGGCCGAA | AUUGUCA |
| 661 | AGAUCUU | CUGAUGAGGCCGAAAGGCCGAA | AGCUCGG |
| 667 | CUCGGCA | CUGAUGAGGCCGAAAGGCCGAA | AUCUUGA |
| 687 | GCUGCCA | CUGAUGAGGCCGAAAGGCCGAA | AGUUUCG |
| 700 | CCCCACC | CUGAUGAGGCCGAAAGGCCGAA | AGGCAGC |
| 715 | GUAGGAA | CUGAUGAGGCCGAAAGGCCGAA | AUCUCAU |
| 717 | CAGUAGG | CUGAUGAGGCCGAAAGGCCGAA | AGAUCUC |
| 718 | ACAGUAG | CUGAUGAGGCCGAAAGGCCGAA | AAGAUCU |
| 721 | CACACAG | CUGAUGAGGCCGAAAGGCCGAA | AGGAAGA |
| 751 | ACACCUC | CUGAUGAGGCCGAAAGGCCGAA | AUGUCCU |
| 759 | CGUGAAA | CUGAUGAGGCCGAAAGGCCGAA | ACACCUC |
| 761 | CCCGUGA | CUGAUGAGGCCGAAAGGCCGAA | AUACACC |
| 762 | UCCCGUG | CUGAUGAGGCCGAAAGGCCGAA | AAUACAC |
| 763 | GUCCCGU | CUGAUGAGGCCGAAAGGCCGAA | AAAUACA |
| 792 | CGAAAAG | CUGAUGAGGCCGAAAGGCCGAA | AGCCUCG |
| 795 | UUGCGAA | CUGAUGAGGCCGAAAGGCCGAA | AGGAGCC |
| 796 | CUUGCGA | CUGAUGAGGCCGAAAGGCCGAA | AAGGAGC |
| 797 | GCUUGCG | CUGAUGAGGCCGAAAGGCCGAA | AAAGGAG |
| 798 | AGCUUGC | CUGAUGAGGCCGAAAGGCCGAA | AAAAGGA |
| 829 | GGAACAC | CUGAUGAGGCCGAAAGGCCGAA | AUGGCCA |
| 834 | GGUCCGG | CUGAUGAGGCCGAAAGGCCGAA | ACACAAU |
| 835 | GGGUCCG | CUGAUGAGGCCGAAAGGCCGAA | AACACAA |
| 845 | GCGUAGG | CUGAUGAGGCCGAAAGGCCGAA | AGGGGUC |
| 849 | GUCUGCG | CUGAUGAGGCCGAAAGGCCGAA | AGGGAGG |
| 872 | CGCACAG | CUGAUGAGGCCGAAAGGCCGAA | AGCCUGC |
| 883 | GCAUGGA | CUGAUGAGGCCGAAAGGCCGAA | ACACGCA |
| 885 | CUGCAUG | CUGAUGAGGCCGAAAGGCCGAA | AGACACG |
| 905 | CGGUCGG | CUGAUGAGGCCGAAAGGCCGAA | AGGCCGC |
| 906 | CCGGUCG | CUGAUGAGGCCGAAAGGCCGAA | AAGGCCG |
| 919 | GCUCACU | CUGAUGAGGCCGAAAGGCCGAA | AGCUCCC |

| | | | |
|---|---|---|---|
| 936 | GUACUGG | CUGAUGAGGCCGAAAGGCCGAA | AUUCCAU |
| 937 | GGUACUG | CUGAUGAGGCCGAAAGGCCGAA | AAUUCCA |
| 942 | UGGCAGG | CUGAUGAGGCCGAAAGGCCGAA | ACUGGAA |
| 953 | UCGUCUG | CUGAUGAGGCCGAAAGGCCGAA | AUCUGGC |
| 962 | CGGUGAC | CUGAUGAGGCCGAAAGGCCGAA | AUCGUCU |
| 965 | AUCCGGU | CUGAUGAGGCCGAAAGGCCGAA | ACGAUCG |
| 973 | UCUCCUC | CUGAUGAGGCCGAAAGGCCGAA | AUCCGGU |
| 986 | GUCCUUU | CUGAUGAGGCCGAAAGGCCGAA | ACGUUUC |
| 996 | GGUCUCA | CUGAUGAGGCCGAAAGGCCGAA | AUGUCCU |
| 1005 | GCUCUUG | CUGAUGAGGCCGAAAGGCCGAA | AGGUCUC |
| 1006 | UGCUCUU | CUGAUGAGGCCGAAAGGCCGAA | AAGGUCU |
| 1015 | UCUUCAU | CUGAUGAGGCCGAAAGGCCGAA | AUGCUCU |
| 1028 | CUGAAAG | CUGAUGAGGCCGAAAGGCCGAA | ACUCUUC |
| 1031 | CCGCUGA | CUGAUGAGGCCGAAAGGCCGAA | AGGACUC |
| 1032 | UCCGCUG | CUGAUGAGGCCGAAAGGCCGAA | AAGGACU |
| 1033 | GUCCGCU | CUGAUGAGGCCGAAAGGCCGAA | AAAGGAC |
| 1058 | CGAGGUG | CUGAUGAGGCCGAAAGGCCGAA | AGGCCGG |
| 1064 | AUGCGUC | CUGAUGAGGCCGAAAGGCCGAA | AGGUGGA |
| 1072 | GCACAGC | CUGAUGAGGCCGAAAGGCCGAA | AUGCGUC |
| 1082 | CUGCGGG | CUGAUGAGGCCGAAAGGCCGAA | AGGCACA |
| 1083 | GCUGCGG | CUGAUGAGGCCGAAAGGCCGAA | AAGGCAC |
| 1092 | AGAAGCU | CUGAUGAGGCCGAAAGGCCGAA | AGCUGCG |
| 1097 | GGGACAG | CUGAUGAGGCCGAAAGGCCGAA | AGCUGAG |
| 1098 | GGGGACA | CUGAUGAGGCCGAAAGGCCGAA | AAGCUGA |
| 1102 | GCUUGGG | CUGAUGAGGCCGAAAGGCCGAA | ACAGAAG |
| 1125 | AAAGGGA | CUGAUGAGGCCGAAAGGCCGAA | AGGGCUG |
| 1127 | GUAAAGG | CUGAUGAGGCCGAAAGGCCGAA | AUAGGGC |
| 1131 | UGACGUA | CUGAUGAGGCCGAAAGGCCGAA | AGGGAUA |
| 1132 | AUGACGU | CUGAUGAGGCCGAAAGGCCGAA | AAGGGAU |
| 1133 | GAUGACG | CUGAUGAGGCCGAAAGGCCGAA | AAAGGGA |
| 1137 | CAGGGAU | CUGAUGAGGCCGAAAGGCCGAA | ACGUAAA |
| 1140 | GCUCAGG | CUGAUGAGGCCGAAAGGCCGAA | AUGACGU |
| 1153 | CAUAGUU | CUGAUGAGGCCGAAAGGCCGAA | AUGGUGC |
| 1158 | CUCAUCA | CUGAUGAGGCCGAAAGGCCGAA | AGUUGAU |
| 1167 | GGUGGGA | CUGAUGAGGCCGAAAGGCCGAA | ACUCAUC |
| 1168 | UGGUGGG | CUGAUGAGGCCGAAAGGCCGAA | AACUCAU |
| 1169 | AUGGUGG | CUGAUGAGGCCGAAAGGCCGAA | AAACUCA |
| 1182 | AGAAGGA | CUGAUGAGGCCGAAAGGCCGAA | ACACCAU |
| 1183 | CAGAAGG | CUGAUGAGGCCGAAAGGCCGAA | AACACCA |
| 1184 | CCAGAAG | CUGAUGAGGCCGAAAGGCCGAA | AAACACC |
| 1187 | UGCCCAG | CUGAUGAGGCCGAAAGGCCGAA | AGGAAAC |
| 1188 | CUGCCCA | CUGAUGAGGCCGAAAGGCCGAA | AAGGAAA |
| 1198 | CCUGGCU | CUGAUGAGGCCGAAAGGCCGAA | AUCUGCC |
| 1209 | CAAGGCC | CUGAUGAGGCCGAAAGGCCGAA | AGGCCUG |
| 1215 | CGGGGCC | CUGAUGAGGCCGAAAGGCCGAA | AGGCCGA |
| 1229 | ACUUGGG | CUGAUGAGGCCGAAAGGCCGAA | AGGGGCC |
| 1237 | GGGGCAG | CUGAUGAGGCCGAAAGGCCGAA | ACUUGGG |
| 1250 | GGGGCUG | CUGAUGAGGCCGAAAGGCCGAA | AGCCUGG |
| 1268 | AUGGCUG | CUGAUGAGGCCGAAAGGCCGAA | AGCAGGG |

| | |
|---|---|
| 1279 | GAGCUGA CUGAUGAGGCCGAAAGGCCGAA ACCAUGG |
| 1281 | CAGAGCU CUGAUGAGGCCGAAAGGCCGAA AUACCAU |
| 1286 | UGGGCCA CUGAUGAGGCCGAAAGGCCGAA AGCUGAU |
| 1309 | GGACUGG CUGAUGAGGCCGAAAGGCCGAA ACAGGGG |
| 1315 | GGGCUAG CUGAUGAGGCCGAAAGGCCGAA ACUGGGA |
| 1318 | CUGGGGC CUGAUGAGGCCGAAAGGCCGAA AGGACUG |
| 1331 | GCCUGAG CUGAUGAGGCCGAAAGGCCGAA AGGGCCU |
| 1334 | ACAGCCU CUGAUGAGGCCGAAAGGCCGAA AGGAGGG |
| 1389 | GGCCUCU CUGAUGAGGCCGAAAGGCCGAA ACAGCGU |
| 1413 | AUCAUCA CUGAUGAGGCCGAAAGGCCGAA ACUGCAG |
| 1414 | CAUCAUC CUGAUGAGGCCGAAAGGCCGAA AACUGCA |
| 1437 | GCCAAGC CUGAUGAGGCCGAAAGGCCGAA AGGCCCC |
| 1441 | UGUUGCC CUGAUGAGGCCGAAAGGCCGAA AGCAAGG |
| 1467 | GUCUGUG CUGAUGAGGCCGAAAGGCCGAA ACACAGC |
| 1468 | GGUCUGU CUGAUGAGGCCGAAAGGCCGAA AACACAG |
| 1482 | GUCGACG CUGAUGAGGCCGAAAGGCCGAA AUGCCAG |
| 1486 | AGUUGUC CUGAUGAGGCCGAAAGGCCGAA ACGGAUG |
| 1494 | AAACUCG CUGAUGAGGCCGAAAGGCCGAA AGUUGUC |
| 1500 | CUGCUGA CUGAUGAGGCCGAAAGGCCGAA ACUCGGA |
| 1501 | GCUGCUG CUGAUGAGGCCGAAAGGCCGAA AACUCGG |
| 1502 | AGCUGCU CUGAUGAGGCCGAAAGGCCGAA AAACUCG |
| 1525 | CCACAGG CUGAUGAGGCCGAAAGGCCGAA AUGCCCU |
| 1566 | CUCAGGG CUGAUGAGGCCGAAAGGCCGAA ACUCCAU |
| 1577 | CGAGUUA CUGAUGAGGCCGAAAGGCCGAA AGCCUCA |
| 1579 | GGCGAGU CUGAUGAGGCCGAAAGGCCGAA AUAGCCU |
| 1583 | ACUAGGC CUGAUGAGGCCGAAAGGCCGAA AGUUAUA |
| 1588 | CUGUCAC CUGAUGAGGCCGAAAGGCCGAA AGGCGAG |
| 1622 | GGAGCAG CUGAUGAGGCCGAAAGGCCGAA AGCUGGG |
| 1628 | CCCAGUG CUGAUGAGGCCGAAAGGCCGAA AGCAGGA |
| 1648 | CAUUGGG CUGAUGAGGCCGAAAGGCCGAA AGCCCCG |
| 1660 | CUGAAAG CUGAUGAGGCCGAAAGGCCGAA AGGCCAU |
| 1663 | CUCCUGA CUGAUGAGGCCGAAAGGCCGAA AGGAGGC |
| 1664 | UCUCCUG CUGAUGAGGCCGAAAGGCCGAA AAGGAGG |
| 1665 | AUCUCCU CUGAUGAGGCCGAAAGGCCGAA AAAGGAG |
| 1680 | GGAGGAG CUGAUGAGGCCGAAAGGCCGAA AGUCUUC |
| 1681 | UGGAGGA CUGAUGAGGCCGAAAGGCCGAA AAGUCUU |
| 1683 | AAUGGAG CUGAUGAGGCCGAAAGGCCGAA AGAAGUC |
| 1686 | CGCAAUG CUGAUGAGGCCGAAAGGCCGAA AGGAGAA |
| 1690 | UGUCCGC CUGAUGAGGCCGAAAGGCCGAA AUGGAGG |
| 1704 | GGCUGAG CUGAUGAGGCCGAAAGGCCGAA AGUCCAU |
| 1705 | GGGCUGA CUGAUGAGGCCGAAAGGCCGAA AAGUCCA |
| 1707 | CAGGGCU CUGAUGAGGCCGAAAGGCCGAA AGAAGUC |
| 1721 | CUGAUCU CUGAUGAGGCCGAAAGGCCGAA ACUCAGC |
| 1726 | AGGAGCU CUGAUGAGGCCGAAAGGCCGAA AUCUGAC |
| 1731 | CCCUUAG CUGAUGAGGCCGAAAGGCCGAA AGCUGAU |
| 1734 | ACCCCCU CUGAUGAGGCCGAAAGGCCGAA AGGAGCU |
| 1754 | CUCUGGG CUGAUGAGGCCGAAAGGCCGAA AGGGCAG |

Table 21
Human *rel A* Hairpin Ribozyme/Target Sequences

| nt. Position | Hairpin Ribozyme sequence | Substrate |
|---|---|---|
| 90 | UGAGGGGG AGAA GUUC ACCAGAGAAACACACGUUGUGGUACAUUACCUGGUA | GAACU GUU CCCCCUCA |
| 156 | GCUGCUUG AGAA GCUC ACCAGAGAAACACACGUUGUGGUACAUUACCUGGUA | GAGCA GCC CAAGCAGC |
| 362 | GCCAUCCC AGAA GUCC ACCAGAGAAACACACGUUGUGGUACAUUACCUGGUA | GGACU GCC GGGAUGGC |
| 413 | GUUCUGGA AGAA GUGG ACCAGAGAAACACACGUUGUGGUACAUUACCUGGUA | CCACA GUU UCCAGAAC |
| 606 | GAAGGACA AGAA GCAG ACCAGAGAAACACACGUUGUGGUACAUUACCUGGUA | CUGCC GCC UGUCCUUC |
| 652 | UUGAGCUC AGAA GUGU ACCAGAGAAACACACGUUGUGGUACAUUACCUGGUA | ACACU GCC GAGCUCAA |
| 695 | CCCACCGA AGAA GCUG ACCAGAGAAACACACGUUGUGGUACAUUACCUGGUA | CAGCU GCC UCGGUGGG |
| 853 | AGGCUGGG AGAA GCGU ACCAGAGAAACACACGUUGUGGUACAUUACCUGGUA | ACGCA GAC CCCAGCCU |
| 900 | GGUCGGAA AGAA GCCG ACCAGAGAAACACACGUUGUGGUACAUUACCUGGUA | CGGCG GCC UUCCGACC |
| 955 | UGACGAUC AGAA GUAU ACCAGAGAAACACACGUUGUGGUACAUUACCUGGUA | AUACA GAC GAUCGUCA |
| 1037 | GUCGGUGG AGAA GCUG ACCAGAGAAACACACGUUGUGGUACAUUACCUGGUA | CAGCG GAC CCACCGAC |
| 1045 | GGCCGGGG AGAA GUGG ACCAGAGAAACACACGUUGUGGUACAUUACCUGGUA | CCACC GAC CCCCGGCC |
| 1410 | CAUCAUCA AGAA GCAG ACCAGAGAAACACACGUUGUGGUACAUUACCUGGUA | CUGCA GUU UGAUGAUG |
| 1453 | ACAGCUGG AGAA GUGC ACCAGAGAAACACACGUUGUGGUACAUUACCUGGUA | GCACA GAC CCAGCUGU |
| 1471 | GAUGCCAG AGAA GUGA ACCAGAGAAACACACGUUGUGGUACAUUACCUGGUA | UCACA GAC CUGGCAUC |

Table 22

Mouse *rel A* Hairpin Ribozyme/Target Sequences

| nt. Position | Hairpin Ribozyme sequence | Substrate |
|---|---|---|
| 137 | GUUGCUUC AGAA GUUC ACCAGAGAAACACACGUUGUGGUACAUUACCUGGUA | GAACA GCC GAAGCAAC |
| 273 | GAGAUUCG AGAA GUUC ACCAGAGAAACACACGUUGUGGUACAUUACCUGGUA | GAACA GUU CGAAUCUC |
| 343 | GCCAUCCC AGAA GUCC ACCAGAGAAACACACGUUGUGGUACAUUACCUGGUA | GGACU GCC GGGAUGGC |
| 366 | GGGCAGAG AGAA GCCU ACCAGAGAAACACACGUUGUGGUACAUUACCUGGUA | AGGCU GAC CUCUGCCC |
| 633 | UUGAGCUC AGAA GUGU ACCAGAGAAACACACGUUGUGGUACAUUACCUGGUA | ACACU GCC GAGCUCAA |
| 676 | CCCACCGA AGAA GCUC ACCAGAGAAACACACGUUGUGGUACAUUACCUGGUA | GAGCU GCC UCGGUGGG |
| 834 | AGGCUGGG AGAA GCGU ACCAGAGAAACACACGUUGUGGUACAUUACCUGGUA | ACGCC GAC CCCAGCCU |
| 881 | GAUCAGAA AGAA GCCG ACCAGAGAAACACACGUUGUGGUACAUUACCUGGUA | CGGCG GCC UUCUGAUC |
| 1100 | AGGUGUAG AGAA GCGG ACCAGAGAAACACACGUUGUGGUACAUUACCUGGUA | CCGCA GCC CUACACCU |
| 1205 | GGGCAGAG AGAA GUGC ACCAGAGAAACACACGUUGUGGUACAUUACCUGGUA | GCACC GUC CUCUGCCC |
| 1361 | GGGCUUCC AGAA GCGU ACCAGAGAAACACACGUUGUGGUACAUUACCUGGUA | ACGCU GUC GGAAGCCC |
| 1385 | CAGCAUCA AGAA GCAG ACCAGAGAAACACACGUUGUGGUACAUUACCUGGUA | CUGCA GUU UGAUGCUG |
| 1431 | ACUCCUGG AGAA GUGC ACCAGAGAAACACACGUUGUGGUACAUUACCUGGUA | GCACA GAC CCAGGAGU |
| 1449 | GAUGCCAG AGAA GUGA ACCAGAGAAACACACGUUGUGGUACAUUACCUGGUA | UCACA GAC CUGGCAUC |
| 1802 | AAGUCGGG AGAA GCUG ACCAGAGAAACACACGUUGUGGUACAUUACCUGGUA | CAGCU GCC CCCGACUU |
| 2009 | UGGCUCCA AGAA GUCC ACCAGAGAAACACACGUUGUGGUACAUUACCUGGUA | GGACA GAC UGGAGCCA |
| 2124 | UGGUGUCG AGAA GCAC ACCAGAGAAACACACGUUGUGGUACAUUACCUGGUA | GUGCU GCC CGACACCA |
| 2233 | AUUCUGAA AGAA GCCA ACCAGAGAAACACACGUUGUGGUACAUUACCUGGUA | UGGCC GCC UUCAGAAU |
| 2354 | UCAGUAAA AGAA GUCU ACCAGAGAAACACACGUUGUGGUACAUUACCUGGUA | AGACA GCC UUUACUGA |

Table 23: Human TNF-α HH Ribozyme Target Sequence

| nt. Position | HH Target Sequence | nt. Position | HH Target Sequence |
|---|---|---|---|
| 28 | GGCAGGU U CUCUUCC | 321 | GUCAGAU C AUCUUCU |
| 29 | GCAGGUU C UCUUCCU | 324 | AGAUCAU C UUCUCGA |
| 31 | AGGUUCU C UUCCUCU | 326 | AUCAUCU U CUCGAAC |
| 33 | GUUCUCU U CCUCUCA | 327 | UCAUCUU C UCGAACC |
| 34 | UUCUCUU C CUCUCAC | 329 | AUCUUCU C GAACCCC |
| 37 | UCUUCCU C UCACAUA | 352 | AGCCUGU A GCCCAUG |
| 39 | UUCCUCU C ACAUACU | 361 | CCCAUGU U GUAGCAA |
| 44 | CUCACAU A CUGACCC | 364 | AUGUUGU A GCAAACC |
| 58 | CACGGCU C CACCCUC | 374 | AAACCCU C AAGCUGA |
| 65 | CCACCCU C UCUCCCC | 391 | GGCAGCU C CAGUGGC |
| 67 | ACCCUCU C UCCCCUG | 421 | AUGCCCU C CUGGCCA |
| 69 | CCUCUCU C CCCUGGA | 449 | GAGAGAU A ACCAGCU |
| 106 | GCAUGAU C CGGGACG | 468 | GUGCCAU C AGAGGGC |
| 136 | AGGCGCU C CCCAAGA | 480 | GGCCUGU A CCUCAUC |
| 165 | CAGGGCU C CAGGCGG | 484 | UGUACCU C AUCUACU |
| 177 | CGGUGCU U GUUCCUC | 487 | ACCUCAU C UACUCCC |
| 180 | UGCUUGU U CCUCAGC | 489 | CUCAUCU A CUCCCAG |
| 181 | GCUUGUU C CUCAGCC | 492 | AUCUACU C CCAGGUC |
| 184 | UGUUCCU C AGCCUCU | 499 | CCCAGGU C CUCUUCA |
| 190 | UCAGCCU C UUCUCCU | 502 | AGGUCCU C UUCAAGG |
| 192 | AGCCUCU U CUCCUUC | 504 | GUCCUCU U CAAGGGC |
| 193 | GCCUCUU C UCCUUCC | 505 | UCCUCUU C AAGGGCC |
| 195 | CUCUUCU C CUUCCUG | 525 | UGCCCCU C CACCCAU |
| 198 | UUCUCCU U CCUGAUC | 538 | AUGUGCU C CUCACCC |
| 199 | UCUCCUU C CUGAUCG | 541 | UGCUCCU C ACCCACA |
| 205 | UCCUGAU C GUGGCAG | 553 | ACACCAU C AGCCGCA |
| 226 | CCACGCU C UUCUGCC | 562 | GCCGCAU C GCCGUCU |
| 228 | ACGCUCU U CUGCCUG | 568 | UCGCCGU C UCCUACC |
| 229 | CGCUCUU C UGCCUGC | 570 | GCCGUCU C CUACCAG |
| 243 | CUGCACU U UGGAGUG | 573 | GUCUCCU A CCAGACC |
| 244 | UGCACUU U GGAGUGA | 586 | CCAAGGU C AACCUCC |
| 253 | GAGUGAU C GGCCCCC | 592 | UCAACCU C CUCUCUG |
| 273 | GAAGAGU C CCCCAGG | 595 | ACCUCCU C UCUGCCA |
| 286 | GGGACCU C UCUCUAA | 597 | CUCCUCU C UGCCAUC |
| 288 | GACCUCU C UCUAAUC | 604 | CUGCCAU C AAGAGCC |
| 290 | CCUCUCU C UAAUCAG | 657 | CCCUGGU A UGAGCCC |
| 292 | UCUCUCU A AUCAGCC | 667 | AGCCCAU C UAUCUGG |
| 295 | CUCUAAU C AGCCCUC | 669 | CCCAUCU A UCUGGGA |
| 302 | CAGCCCU C UGGCCCA | | |

| | | | |
|---|---|---|---|
| 671 | CAUCUAU C UGGGAGG | 960 | UGGGAUU C AGGAAUG |
| 682 | GAGGGGU C UUCCAGC | 1001 | AACCACU A AGAAUUC |
| 684 | GGGGUCU U CCAGCUG | 1007 | UAAGAAU U CAAACUG |
| 685 | GGGUCUU C CAGCUGG | 1008 | AAGAAUU C AAACUGG |
| 709 | ACCGACU C AGCGCUG | 1021 | GGGGCCU C CAGAACU |
| 721 | CUGAGAU C AAUCGGC | 1029 | CAGAACU C ACUGGGG |
| 725 | GAUCAAU C GGCCCGA | 1040 | GGGGCCU A CAGCUUU |
| 735 | CCCGACU A UCUCGAC | 1046 | UACAGCU U UGAUCCC |
| 737 | CGACUAU C UCGACUU | 1047 | ACAGCUU U GAUCCCU |
| 739 | ACUAUCU C GACUUUG | 1051 | CUUUGAU C CCUGACA |
| 744 | CUCGACU U UGCCGAG | 1060 | CUGACAU C UGGAAUC |
| 745 | UCGACUU U GCCGAGU | 1067 | CUGGAAU C UGGAGAC |
| 753 | GCCGAGU C UGGGCAG | 1085 | GGAGCCU U UGGUUCU |
| 763 | GGCAGGU C UACUUUG | 1086 | GAGCCUU U GGUUCUG |
| 765 | CAGGUCU A CUUUGGG | 1090 | CUUUGGU U CUGGCCA |
| 768 | GUCUACU U UGGGAUC | 1091 | UUUGGUU C UGGCCAG |
| 769 | UCUACUU U GGGAUCA | 1113 | CAGGACU U GAGAAGA |
| 775 | UUGGGAU C AUUGCCC | 1124 | AAGACCU C ACCUAGA |
| 778 | GGAUCAU U GCCCUGU | 1129 | CUCACCU A GAAAUUG |
| 801 | CGAACAU C CAACCUU | 1135 | UAGAAAU U GACACAA |
| 808 | CCAACCU U CCCAAAC | 1151 | UGGACCU U AGGCCUU |
| 809 | CAACCUU C CCAAACG | 1152 | GGACCUU A GGCCUUC |
| 820 | AACGCCU C CCCUGCC | 1158 | UAGGCCU U CCUCUCU |
| 833 | CCCCAAU C CCUUUAU | 1159 | AGGCCUU C CUCUCUC |
| 837 | AAUCCCU U UAUUACC | 1162 | CCUUCCU C UCUCCAG |
| 838 | AUCCCUU U AUUACCC | 1164 | UUCCUCU C UCCAGAU |
| 839 | UCCCUUU A UUACCCC | 1166 | CCUCUCU C CAGAUGU |
| 841 | CCUUUAU U ACCCCCU | 1174 | CAGAUGU U UCCAGAC |
| 842 | CUUUAUU A CCCCCUC | 1175 | AGAUGUU U CCAGACU |
| 849 | ACCCCCU C CUUCAGA | 1176 | GAUGUUU C CAGACUU |
| 852 | CCCUCCU U CAGACAC | 1183 | CCAGACU U CCUUGAG |
| 853 | CCUCCUU C AGACACC | 1184 | CAGACUU C CUUGAGA |
| 863 | ACACCCU C AACCUCU | 1187 | ACUUCCU U GAGACAC |
| 869 | UCAACCU C UUCUGGC | 1208 | CAGCCCU C CCCAUGG |
| 871 | AACCUCU U CUGGCUC | 1224 | GCCAGCU C CCUCUAU |
| 872 | ACCUCUU C UGGCUCA | 1228 | GCUCCCU C UAUUUAU |
| 878 | UCUGGCU C AAAAAGA | 1230 | UCCCUCU A UUUAUGU |
| 890 | AGAGAAU U GGGGGCU | 1232 | CCUCUAU U UAUGUUU |
| 898 | GGGGGCU U AGGGUCG | 1233 | CUCUAUU U AUGUUUG |
| 899 | GGGGCUU A GGGUCGG | 1234 | UCUAUUU A UGUUUGC |
| 904 | UUAGGGU C GGAACCC | 1238 | UUUAUGU U UGCACUU |
| 917 | CCAAGCU U AGAACUU | 1239 | UUAUGUU U GCACUUG |
| 918 | CAAGCUU A GAACUUU | 1245 | UUGCACU U GUGAUUA |
| 924 | UAGAACU U UAAGCAA | 1251 | UUGUGAU U AUUUAUU |
| 925 | AGAACUU U AAGCAAC | 1252 | UGUGAUU A UUUAUUA |
| 926 | GAACUUU A AGCAACA | 1254 | UGAUUAU U UAUUAUU |
| 945 | CACCACU U CGAAACC | 1255 | GAUUAUU U AUUAUUU |
| 946 | ACCACUU C GAAACCU | 1256 | AUUAUUU A UUAUUUA |
| 959 | CUGGGAU U CAGGAAU | 1258 | UAUUUAU U AUUUAUU |

| | |
|---|---|
| 1259 | AUUUAUU A UUUAUUU |
| 1261 | UUAUUAU U UAUUUAU |
| 1262 | UAUUAUU U AUUUAUU |
| 1263 | AUUAUUU A UUUAUUA |
| 1265 | UAUUUAU U UAUUAUU |
| 1266 | AUUUAUU U AUUAUUU |
| 1267 | UUUAUUU A UUAUUUA |
| 1269 | UAUUUAU U AUUUAUU |
| 1270 | AUUUAUU A UUUAUUU |
| 1272 | UUAUUAU U UAUUUAU |
| 1273 | UAUUAUU U AUUUAUU |
| 1274 | AUUAUUU A UUUAUUU |
| 1276 | UAUUUAU U UAUUUAC |
| 1277 | AUUUAUU U AUUUACA |
| 1278 | UUUAUUU A UUUACAG |
| 1280 | UAUUUAU U UACAGAU |
| 1281 | AUUUAUU U ACAGAUG |
| 1282 | UUUAUUU A CAGAUGA |
| 1294 | UGAAUGU A UUUAUUU |
| 1296 | AAUGUAU U UAUUUGG |
| 1297 | AUGUAUU U AUUUGGG |
| 1298 | UGUAUUU A UUUGGGA |
| 1300 | UAUUUAU U UGGGAGA |
| 1301 | AUUUAUU U GGGAGAC |
| 1315 | CCGGGGU A UCCUGGG |
| 1317 | GGGGUAU C CUGGGGG |
| 1334 | CCAAUGU A GGAGCUG |
| 1345 | GCUGCCU U GGCUCAG |
| 1350 | CUUGGCU C AGACAUG |
| 1359 | GACAUGU U UUCCGUG |
| 1360 | ACAUGUU U UCCGUGA |
| 1361 | CAUGUUU U CCGUGAA |
| 1362 | AUGUUUU C CGUGAAA |
| 1386 | GAACAAU A GGCUGUU |
| 1393 | AGGCUGU U CCCAUGU |
| 1394 | GGCUGUU C CCAUGUA |
| 1401 | CCCAUGU A GCCCCCU |
| 1414 | CUGGCCU C UGUGCCU |
| 1422 | UGUGCCU U CUUUUGA |
| 1423 | GUGCCUU C UUUUGAU |
| 1425 | GCCUUCU U UUGAUUA |
| 1426 | CCUUCUU U UGAUUAU |
| 1427 | CUUCUUU U GAUUAUG |
| 1431 | UUUUGAU U AUGUUUU |
| 1432 | UUUGAUU A UGUUUUU |
| 1436 | AUUAUGU U UUUUAAA |
| 1437 | UUAUGUU U UUUAAAA |
| 1438 | UAUGUUU U UUAAAAU |
| 1440 | UGUUUUU U AAAAUAU |
| 1441 | GUUUUUU A AAAUAUU |
| 1446 | UUAAAAU A UUAUCUG |
| 1448 | AAAAUAU U AUCUGAU |
| 1449 | AAAUAUU A UCUGAUU |
| 1451 | AUAUUAU C UGAUUAA |
| 1456 | AUCUGAU U AAGUUGU |
| 1457 | UCUGAUU A AGUUGUC |
| 1461 | AUUAAGU U GUCUAAA |
| 1464 | AAGUUGU C UAAACAA |
| 1466 | GUUGUCU A AACAAUG |
| 1479 | UGCUGAU U UGGUGAC |
| 1480 | GCUGAUU U GGUGACC |
| 1494 | CAACUGU C ACUCAUU |
| 1498 | UGUCACU C AUUGCUG |
| 1501 | CACUCAU U GCUGAGG |
| 1512 | GAGGCCU C UGCUCCC |
| 1517 | CUCUGCU C CCCAGGG |
| 1528 | AGGGAGU U GUGCUG |
| 1533 | GUUGUGU C UGUAAUC |
| 1537 | UGUCUGU A AUCGGCC |
| 1540 | CUGUAAU C GGCCUAC |
| 1546 | UCGGCCU A CUAUUCA |
| 1549 | GCCUACU A UUCAGUG |
| 1551 | CUACUAU U CAGUGGC |
| 1552 | UACUAUU C AGUGGCG |
| 1566 | GAGAAAU A AAGGUUG |
| 1572 | UAAAGGU U GCUUAGG |
| 1576 | GGUUGCU U AGGAAAG |
| 1577 | GUUGCUU A GGAAAGA |

# Table 24: Human TNF-α Hammerhead Ribozyme Sequences

| nt. Position | HH Ribozyme Sequence |
|---|---|
| 28 | GGAAGAG CUGAUGAGGCCGAAAGGCCGAA ACCUGCC |
| 29 | AGGAAGA CUGAUGAGGCCGAAAGGCCGAA AACCUGC |
| 31 | AGAGGAA CUGAUGAGGCCGAAAGGCCGAA AGAACCU |
| 33 | UGAGAGG CUGAUGAGGCCGAAAGGCCGAA AGAGAAC |
| 34 | GUGAGAG CUGAUGAGGCCGAAAGGCCGAA AAGAGAA |
| 37 | UAUGUGA CUGAUGAGGCCGAAAGGCCGAA AGGAAGA |
| 39 | AGUAUGU CUGAUGAGGCCGAAAGGCCGAA AGAGGAA |
| 44 | GGGUCAG CUGAUGAGGCCGAAAGGCCGAA AUGUGAG |
| 58 | GAGGGUG CUGAUGAGGCCGAAAGGCCGAA AGCCGUG |
| 65 | GGGGAGA CUGAUGAGGCCGAAAGGCCGAA AGGGUGG |
| 67 | CAGGGGA CUGAUGAGGCCGAAAGGCCGAA AGAGGGU |
| 69 | UCCAGGG CUGAUGAGGCCGAAAGGCCGAA AGAGAGG |
| 106 | CGUCCCG CUGAUGAGGCCGAAAGGCCGAA AUCAUGC |
| 136 | UCUUGGG CUGAUGAGGCCGAAAGGCCGAA AGCGCCU |
| 165 | CCGCCUG CUGAUGAGGCCGAAAGGCCGAA AGCCCUG |
| 177 | GAGGAAC CUGAUGAGGCCGAAAGGCCGAA AGCACCG |
| 180 | GCUGAGG CUGAUGAGGCCGAAAGGCCGAA ACAAGCA |
| 181 | GGCUGAG CUGAUGAGGCCGAAAGGCCGAA AACAAGC |
| 184 | AGAGGCU CUGAUGAGGCCGAAAGGCCGAA AGGAACA |
| 190 | AGGAGAA CUGAUGAGGCCGAAAGGCCGAA AGGCUGA |
| 192 | GAAGGAG CUGAUGAGGCCGAAAGGCCGAA AGAGGCU |
| 193 | GGAAGGA CUGAUGAGGCCGAAAGGCCGAA AAGAGGC |
| 195 | CAGGAAG CUGAUGAGGCCGAAAGGCCGAA AGAAGAG |
| 198 | GAUCAGG CUGAUGAGGCCGAAAGGCCGAA AGGAGAA |
| 199 | CGAUCAG CUGAUGAGGCCGAAAGGCCGAA AAGGAGA |
| 205 | CUGCCAC CUGAUGAGGCCGAAAGGCCGAA AUCAGGA |
| 226 | GGCAGAA CUGAUGAGGCCGAAAGGCCGAA AGCGUGG |
| 228 | CAGGCAG CUGAUGAGGCCGAAAGGCCGAA AGAGCGU |
| 229 | GCAGGCA CUGAUGAGGCCGAAAGGCCGAA AAGAGCG |
| 243 | CACUCCA CUGAUGAGGCCGAAAGGCCGAA AGUGCAG |
| 244 | UCACUCC CUGAUGAGGCCGAAAGGCCGAA AAGUGCA |
| 253 | GGGGGCC CUGAUGAGGCCGAAAGGCCGAA AUCACUC |
| 273 | CCUGGGG CUGAUGAGGCCGAAAGGCCGAA ACUCUUC |
| 286 | UUAGAGA CUGAUGAGGCCGAAAGGCCGAA AGGUCCC |
| 288 | GAUUAGA CUGAUGAGGCCGAAAGGCCGAA AGAGGUC |
| 290 | CUGAUUA CUGAUGAGGCCGAAAGGCCGAA AGAGAGG |
| 292 | GGCUGAU CUGAUGAGGCCGAAAGGCCGAA AGAGAGA |
| 295 | GAGGGCU CUGAUGAGGCCGAAAGGCCGAA AUUAGAG |
| 302 | UGGGCCA CUGAUGAGGCCGAAAGGCCGAA AGGGCUG |

| | |
|---|---|
| 321 | AGAAGAU CUGAUGAGGCCGAAAGGCCGAA AUCUGAC |
| 324 | UCGAGAA CUGAUGAGGCCGAAAGGCCGAA AUGAUCU |
| 326 | GUUCGAG CUGAUGAGGCCGAAAGGCCGAA AGAUGAU |
| 327 | GGUUCGA CUGAUGAGGCCGAAAGGCCGAA AAGAUGA |
| 329 | GGGGUUC CUGAUGAGGCCGAAAGGCCGAA AGAAGAU |
| 352 | CAUGGGC CUGAUGAGGCCGAAAGGCCGAA ACAGGCU |
| 361 | UUGCUAC CUGAUGAGGCCGAAAGGCCGAA ACAUGGG |
| 364 | GGUUUGC CUGAUGAGGCCGAAAGGCCGAA ACAACAU |
| 374 | UCAGCUU CUGAUGAGGCCGAAAGGCCGAA AGGGUUU |
| 391 | GCCACUG CUGAUGAGGCCGAAAGGCCGAA AGCUGCC |
| 421 | UGGCCAG CUGAUGAGGCCGAAAGGCCGAA AGGGCAU |
| 449 | AGCUGGU CUGAUGAGGCCGAAAGGCCGAA AUCUCUC |
| 468 | GCCCUCU CUGAUGAGGCCGAAAGGCCGAA AUGGCAC |
| 480 | GAUGAGG CUGAUGAGGCCGAAAGGCCGAA ACAGGCC |
| 484 | AGUAGAU CUGAUGAGGCCGAAAGGCCGAA AGGUACA |
| 487 | GGGAGUA CUGAUGAGGCCGAAAGGCCGAA AUGAGGU |
| 489 | CUGGGAG CUGAUGAGGCCGAAAGGCCGAA AGAUGAG |
| 492 | GACCUGG CUGAUGAGGCCGAAAGGCCGAA AGUAGAU |
| 499 | UGAAGAG CUGAUGAGGCCGAAAGGCCGAA ACCUGGG |
| 502 | CCUUGAA CUGAUGAGGCCGAAAGGCCGAA AGGACCU |
| 504 | GCCCUUG CUGAUGAGGCCGAAAGGCCGAA AGAGGAC |
| 505 | GGCCCUU CUGAUGAGGCCGAAAGGCCGAA AAGAGGA |
| 525 | AUGGGUG CUGAUGAGGCCGAAAGGCCGAA AGGGGCA |
| 538 | GGGUGAG CUGAUGAGGCCGAAAGGCCGAA AGCACAU |
| 541 | UGUGGGU CUGAUGAGGCCGAAAGGCCGAA AGGAGCA |
| 553 | UGCGGCU CUGAUGAGGCCGAAAGGCCGAA AUGGUGU |
| 562 | AGACGGC CUGAUGAGGCCGAAAGGCCGAA AUGCGGC |
| 568 | GGUAGGA CUGAUGAGGCCGAAAGGCCGAA ACGGCGA |
| 570 | CUGGUAG CUGAUGAGGCCGAAAGGCCGAA AGACGGC |
| 573 | GGUCUGG CUGAUGAGGCCGAAAGGCCGAA AGGAGAC |
| 586 | GGAGGUU CUGAUGAGGCCGAAAGGCCGAA ACCUUGG |
| 592 | CAGAGAG CUGAUGAGGCCGAAAGGCCGAA AGGUUGA |
| 595 | UGGCAGA CUGAUGAGGCCGAAAGGCCGAA AGGAGGU |
| 597 | GAUGGCA CUGAUGAGGCCGAAAGGCCGAA AGAGGAG |
| 604 | GGCUCUU CUGAUGAGGCCGAAAGGCCGAA AUGGCAG |
| 657 | GGGCUCA CUGAUGAGGCCGAAAGGCCGAA ACCAGGG |
| 667 | CCAGAUA CUGAUGAGGCCGAAAGGCCGAA AUGGGCU |
| 669 | UCCCAGA CUGAUGAGGCCGAAAGGCCGAA AGAUGGG |
| 671 | CCUCCCA CUGAUGAGGCCGAAAGGCCGAA AUAGAUG |
| 682 | GCUGGAA CUGAUGAGGCCGAAAGGCCGAA ACCCCUC |
| 684 | CAGCUGG CUGAUGAGGCCGAAAGGCCGAA AGACCCC |
| 685 | CCAGCUG CUGAUGAGGCCGAAAGGCCGAA AAGACCC |
| 709 | CAGCGCU CUGAUGAGGCCGAAAGGCCGAA AGUCGGU |
| 721 | GCCGAUU CUGAUGAGGCCGAAAGGCCGAA AUCUCAG |
| 725 | UCGGGCC CUGAUGAGGCCGAAAGGCCGAA AUUGAUC |
| 735 | GUCGAGA CUGAUGAGGCCGAAAGGCCGAA AGUCGGG |
| 737 | AAGUCGA CUGAUGAGGCCGAAAGGCCGAA AUAGUCG |
| 739 | CAAAGUC CUGAUGAGGCCGAAAGGCCGAA AGAUAGU |
| 744 | CUCGGCA CUGAUGAGGCCGAAAGGCCGAA AGUCGAG |

| | |
|---|---|
| 745 | ACUCGGC CUGAUGAGGCCGAAAGGCCGAA AAGUCGA |
| 753 | CUGCCCA CUGAUGAGGCCGAAAGGCCGAA ACUCGGC |
| 763 | CAAAGUA CUGAUGAGGCCGAAAGGCCGAA ACCUGCC |
| 765 | CCCAAAG CUGAUGAGGCCGAAAGGCCGAA AGACCUG |
| 768 | GAUCCCA CUGAUGAGGCCGAAAGGCCGAA AGUAGAC |
| 769 | UGAUCCC CUGAUGAGGCCGAAAGGCCGAA AAGUAGA |
| 775 | GGGCAAU CUGAUGAGGCCGAAAGGCCGAA AUCCCAA |
| 778 | ACAGGGC CUGAUGAGGCCGAAAGGCCGAA AUGAUCC |
| 801 | AAGGUUG CUGAUGAGGCCGAAAGGCCGAA AUGUUCG |
| 808 | GUUUGGG CUGAUGAGGCCGAAAGGCCGAA AGGUUGG |
| 809 | CGUUUGG CUGAUGAGGCCGAAAGGCCGAA AAGGUUG |
| 820 | GGCAGGG CUGAUGAGGCCGAAAGGCCGAA AGGCGUU |
| 833 | AUAAAGG CUGAUGAGGCCGAAAGGCCGAA AUUGGGG |
| 837 | GGUAAUA CUGAUGAGGCCGAAAGGCCGAA AGGGAUU |
| 838 | GGGUAAU CUGAUGAGGCCGAAAGGCCGAA AAGGGAU |
| 839 | GGGGUAA CUGAUGAGGCCGAAAGGCCGAA AAAGGGA |
| 841 | AGGGGGU CUGAUGAGGCCGAAAGGCCGAA AUAAAGG |
| 842 | GAGGGGG CUGAUGAGGCCGAAAGGCCGAA AAUAAAG |
| 849 | UCUGAAG CUGAUGAGGCCGAAAGGCCGAA AGGGGGU |
| 852 | GUGUCUG CUGAUGAGGCCGAAAGGCCGAA AGGAGGG |
| 853 | GGUGUCU CUGAUGAGGCCGAAAGGCCGAA AAGGAGG |
| 863 | AGAGGUU CUGAUGAGGCCGAAAGGCCGAA AGGGUGU |
| 869 | GCCAGAA CUGAUGAGGCCGAAAGGCCGAA AGGUUGA |
| 871 | GAGCCAG CUGAUGAGGCCGAAAGGCCGAA AGAGGUU |
| 872 | UGAGCCA CUGAUGAGGCCGAAAGGCCGAA AAGAGGU |
| 878 | UCUUUUU CUGAUGAGGCCGAAAGGCCGAA AGCCAGA |
| 890 | AGCCCCC CUGAUGAGGCCGAAAGGCCGAA AUUCUCU |
| 898 | CGACCCU CUGAUGAGGCCGAAAGGCCGAA AGCCCCC |
| 899 | CCGACCC CUGAUGAGGCCGAAAGGCCGAA AAGCCCC |
| 904 | GGGUUCC CUGAUGAGGCCGAAAGGCCGAA ACCCUAA |
| 917 | AAGUUCU CUGAUGAGGCCGAAAGGCCGAA AGCUUGG |
| 918 | AAAGUUC CUGAUGAGGCCGAAAGGCCGAA AAGCUUG |
| 924 | UUGCUUA CUGAUGAGGCCGAAAGGCCGAA AGUUCUA |
| 925 | GUUGCUU CUGAUGAGGCCGAAAGGCCGAA AAGUUCU |
| 926 | UGUUGCU CUGAUGAGGCCGAAAGGCCGAA AAAGUUC |
| 945 | GGUUUCG CUGAUGAGGCCGAAAGGCCGAA AGUGGUG |
| 946 | AGGUUUC CUGAUGAGGCCGAAAGGCCGAA AAGUGGU |
| 959 | AUUCCUG CUGAUGAGGCCGAAAGGCCGAA AUCCCAG |
| 960 | CAUUCCU CUGAUGAGGCCGAAAGGCCGAA AAUCCCA |
| 1001 | GAAUUCU CUGAUGAGGCCGAAAGGCCGAA AGUGGUU |
| 1007 | CAGUUUG CUGAUGAGGCCGAAAGGCCGAA AUUCUUA |
| 1008 | CCAGUUU CUGAUGAGGCCGAAAGGCCGAA AAUUCUU |
| 1021 | AGUUCUG CUGAUGAGGCCGAAAGGCCGAA AGGCCCC |
| 1029 | CCCCAGU CUGAUGAGGCCGAAAGGCCGAA AGUUCUG |
| 1040 | AAAGCUG CUGAUGAGGCCGAAAGGCCGAA AGGCCCC |
| 1046 | GGGAUCA CUGAUGAGGCCGAAAGGCCGAA AGCUGUA |
| 1047 | AGGGAUC CUGAUGAGGCCGAAAGGCCGAA AAGCUGU |
| 1051 | UGUCAGG CUGAUGAGGCCGAAAGGCCGAA AUCAAAG |
| 1060 | GAUUCCA CUGAUGAGGCCGAAAGGCCGAA AUGUCAG |

| | | | | |
|---|---|---|---|---|
| 1067 | GUCUCCA | CUGAUGAGGCCGAAAGGCCGAA | AUUCCAG |
| 1085 | AGAACCA | CUGAUGAGGCCGAAAGGCCGAA | AGGCUCC |
| 1086 | CAGAACC | CUGAUGAGGCCGAAAGGCCGAA | AAGGCUC |
| 1090 | UGGCCAG | CUGAUGAGGCCGAAAGGCCGAA | ACCAAAG |
| 1091 | CUGGCCA | CUGAUGAGGCCGAAAGGCCGAA | AACCAAA |
| 1113 | UCUUCUC | CUGAUGAGGCCGAAAGGCCGAA | AGUCCUG |
| 1124 | UCUAGGU | CUGAUGAGGCCGAAAGGCCGAA | AGGUCUU |
| 1129 | CAAUUUC | CUGAUGAGGCCGAAAGGCCGAA | AGGUGAG |
| 1135 | UUGUGUC | CUGAUGAGGCCGAAAGGCCGAA | AUUUCUA |
| 1151 | AAGGCCU | CUGAUGAGGCCGAAAGGCCGAA | AGGUCCA |
| 1152 | GAAGGCC | CUGAUGAGGCCGAAAGGCCGAA | AAGGUCC |
| 1158 | AGAGAGG | CUGAUGAGGCCGAAAGGCCGAA | AGGCCUA |
| 1159 | GAGAGAG | CUGAUGAGGCCGAAAGGCCGAA | AAGGCCU |
| 1162 | CUGGAGA | CUGAUGAGGCCGAAAGGCCGAA | AGGAAGG |
| 1164 | AUCUGGA | CUGAUGAGGCCGAAAGGCCGAA | AGAGGAA |
| 1166 | ACAUCUG | CUGAUGAGGCCGAAAGGCCGAA | AGAGAGG |
| 1174 | GUCUGGA | CUGAUGAGGCCGAAAGGCCGAA | ACAUCUG |
| 1175 | AGUCUGG | CUGAUGAGGCCGAAAGGCCGAA | AACAUCU |
| 1176 | AAGUCUG | CUGAUGAGGCCGAAAGGCCGAA | AAACAUC |
| 1183 | CUCAAGG | CUGAUGAGGCCGAAAGGCCGAA | AGUCUGG |
| 1184 | UCUCAAG | CUGAUGAGGCCGAAAGGCCGAA | AAGUCUG |
| 1187 | GUGUCUC | CUGAUGAGGCCGAAAGGCCGAA | AGGAAGU |
| 1208 | CCAUGGG | CUGAUGAGGCCGAAAGGCCGAA | AGGGCUG |
| 1224 | AUAGAGG | CUGAUGAGGCCGAAAGGCCGAA | AGCUGGC |
| 1228 | AUAAAUA | CUGAUGAGGCCGAAAGGCCGAA | AGGGAGC |
| 1230 | ACAUAAA | CUGAUGAGGCCGAAAGGCCGAA | AGAGGGA |
| 1232 | AAACAUA | CUGAUGAGGCCGAAAGGCCGAA | AUAGAGG |
| 1233 | CAAACAU | CUGAUGAGGCCGAAAGGCCGAA | AAUAGAG |
| 1234 | GCAAACA | CUGAUGAGGCCGAAAGGCCGAA | AAAUAGA |
| 1238 | AAGUGCA | CUGAUGAGGCCGAAAGGCCGAA | ACAUAAA |
| 1239 | CAAGUGC | CUGAUGAGGCCGAAAGGCCGAA | AACAUAA |
| 1245 | UAAUCAC | CUGAUGAGGCCGAAAGGCCGAA | AGUGCAA |
| 1251 | AAUAAAU | CUGAUGAGGCCGAAAGGCCGAA | AUCACAA |
| 1252 | UAAUAAA | CUGAUGAGGCCGAAAGGCCGAA | AAUCACA |
| 1254 | AAUAAUA | CUGAUGAGGCCGAAAGGCCGAA | AUAAUCA |
| 1255 | AAAUAAU | CUGAUGAGGCCGAAAGGCCGAA | AAUAAUC |
| 1256 | UAAAUAA | CUGAUGAGGCCGAAAGGCCGAA | AAAUAAU |
| 1258 | AAUAAAU | CUGAUGAGGCCGAAAGGCCGAA | AUAAAUA |
| 1259 | AAAUAAA | CUGAUGAGGCCGAAAGGCCGAA | AAUAAAU |
| 1261 | AUAAAUA | CUGAUGAGGCCGAAAGGCCGAA | AUAAUAA |
| 1262 | AAUAAAU | CUGAUGAGGCCGAAAGGCCGAA | AAUAAUA |
| 1263 | UAAUAAA | CUGAUGAGGCCGAAAGGCCGAA | AAAUAAU |
| 1265 | AAUAAUA | CUGAUGAGGCCGAAAGGCCGAA | AUAAAUA |
| 1266 | AAAUAAU | CUGAUGAGGCCGAAAGGCCGAA | AAUAAAU |
| 1267 | UAAAUAA | CUGAUGAGGCCGAAAGGCCGAA | AAAUAAA |
| 1269 | AAUAAAU | CUGAUGAGGCCGAAAGGCCGAA | AUAAAUA |
| 1270 | AAAUAAA | CUGAUGAGGCCGAAAGGCCGAA | AAUAAAU |
| 1272 | AUAAAUA | CUGAUGAGGCCGAAAGGCCGAA | AUAAUAA |
| 1273 | AAUAAAU | CUGAUGAGGCCGAAAGGCCGAA | AAUAAUA |

| | | | |
|---|---|---|---|
| 1274 | AAAUAAA | CUGAUGAGGCCGAAAGGCCGAA | AAAUAAU |
| 1276 | GUAAAUA | CUGAUGAGGCCGAAAGGCCGAA | AUAAAUA |
| 1277 | UGUAAAU | CUGAUGAGGCCGAAAGGCCGAA | AAUAAAU |
| 1278 | CUGUAAA | CUGAUGAGGCCGAAAGGCCGAA | AAAUAAA |
| 1280 | AUCUGUA | CUGAUGAGGCCGAAAGGCCGAA | AUAAAUA |
| 1281 | CAUCUGU | CUGAUGAGGCCGAAAGGCCGAA | AAUAAAU |
| 1282 | UCAUCUG | CUGAUGAGGCCGAAAGGCCGAA | AAAUAAA |
| 1294 | AAAUAAA | CUGAUGAGGCCGAAAGGCCGAA | ACAUUCA |
| 1296 | CCAAAUA | CUGAUGAGGCCGAAAGGCCGAA | AUACAUU |
| 1297 | CCCAAAU | CUGAUGAGGCCGAAAGGCCGAA | AAUACAU |
| 1298 | UCCCAAA | CUGAUGAGGCCGAAAGGCCGAA | AAAUACA |
| 1300 | UCUCCCA | CUGAUGAGGCCGAAAGGCCGAA | AUAAAUA |
| 1301 | GUCUCCC | CUGAUGAGGCCGAAAGGCCGAA | AAUAAAU |
| 1315 | CCCAGGA | CUGAUGAGGCCGAAAGGCCGAA | ACCCCGG |
| 1317 | CCCCCAG | CUGAUGAGGCCGAAAGGCCGAA | AUACCCC |
| 1334 | CAGCUCC | CUGAUGAGGCCGAAAGGCCGAA | ACAUUGG |
| 1345 | CUGAGCC | CUGAUGAGGCCGAAAGGCCGAA | AGGCAGC |
| 1350 | CAUGUCU | CUGAUGAGGCCGAAAGGCCGAA | AGCCAAG |
| 1359 | CACGGAA | CUGAUGAGGCCGAAAGGCCGAA | ACAUGUC |
| 1360 | UCACGGA | CUGAUGAGGCCGAAAGGCCGAA | AACAUGU |
| 1361 | UUCACGG | CUGAUGAGGCCGAAAGGCCGAA | AAACAUG |
| 1362 | UUUCACG | CUGAUGAGGCCGAAAGGCCGAA | AAAACAU |
| 1386 | AACAGCC | CUGAUGAGGCCGAAAGGCCGAA | AUUGUUC |
| 1393 | ACAUGGG | CUGAUGAGGCCGAAAGGCCGAA | ACAGCCU |
| 1394 | UACAUGG | CUGAUGAGGCCGAAAGGCCGAA | AACAGCC |
| 1401 | AGGGGGC | CUGAUGAGGCCGAAAGGCCGAA | ACAUGGG |
| 1414 | AGGCACA | CUGAUGAGGCCGAAAGGCCGAA | AGGCCAG |
| 1422 | UCAAAAG | CUGAUGAGGCCGAAAGGCCGAA | AGGCACA |
| 1423 | AUCAAAA | CUGAUGAGGCCGAAAGGCCGAA | AAGGCAC |
| 1425 | UAAUCAA | CUGAUGAGGCCGAAAGGCCGAA | AGAAGGC |
| 1426 | AUAAUCA | CUGAUGAGGCCGAAAGGCCGAA | AAGAAGG |
| 1427 | CAUAAUC | CUGAUGAGGCCGAAAGGCCGAA | AAAGAAG |
| 1431 | AAAACAU | CUGAUGAGGCCGAAAGGCCGAA | AUCAAAA |
| 1432 | AAAAACA | CUGAUGAGGCCGAAAGGCCGAA | AAUCAAA |
| 1436 | UUUAAAA | CUGAUGAGGCCGAAAGGCCGAA | ACAUAAU |
| 1437 | UUUUAAA | CUGAUGAGGCCGAAAGGCCGAA | AACAUAA |
| 1438 | AUUUUAA | CUGAUGAGGCCGAAAGGCCGAA | AAACAUA |
| 1439 | UAUUUUA | CUGAUGAGGCCGAAAGGCCGAA | AAAACAU |
| 1440 | AUAUUUU | CUGAUGAGGCCGAAAGGCCGAA | AAAAACA |
| 1441 | AAUAUUU | CUGAUGAGGCCGAAAGGCCGAA | AAAAAAC |
| 1446 | CAGAUAA | CUGAUGAGGCCGAAAGGCCGAA | AUUUUAA |
| 1448 | AUCAGAU | CUGAUGAGGCCGAAAGGCCGAA | AUAUUUU |
| 1449 | AAUCAGA | CUGAUGAGGCCGAAAGGCCGAA | AAUAUUU |
| 1451 | UUAAUCA | CUGAUGAGGCCGAAAGGCCGAA | AUAAUAU |
| 1456 | ACAACUU | CUGAUGAGGCCGAAAGGCCGAA | AUCAGAU |
| 1457 | GACAACU | CUGAUGAGGCCGAAAGGCCGAA | AAUCAGA |
| 1461 | UUUAGAC | CUGAUGAGGCCGAAAGGCCGAA | ACUUAAU |
| 1464 | UUGUUUA | CUGAUGAGGCCGAAAGGCCGAA | ACAACUU |
| 1466 | CAUUGUU | CUGAUGAGGCCGAAAGGCCGAA | AGACAAC |

| | |
|---|---|
| 1479 | GUCACCA CUGAUGAGGCCGAAAGGCCGAA AUCAGCA |
| 1480 | GGUCACC CUGAUGAGGCCGAAAGGCCGAA AAUCAGC |
| 1494 | AAUGAGU CUGAUGAGGCCGAAAGGCCGAA ACAGUUG |
| 1498 | CAGCAAU CUGAUGAGGCCGAAAGGCCGAA AGUGACA |
| 1501 | CCUCAGC CUGAUGAGGCCGAAAGGCCGAA AUGAGUG |
| 1512 | GGGAGCA CUGAUGAGGCCGAAAGGCCGAA AGGCCUC |
| 1517 | CCCUGGG CUGAUGAGGCCGAAAGGCCGAA AGCAGAG |
| 1528 | CAGACAC CUGAUGAGGCCGAAAGGCCGAA ACUCCCU |
| 1533 | GAUUACA CUGAUGAGGCCGAAAGGCCGAA ACACAAC |
| 1537 | GGCCGAU CUGAUGAGGCCGAAAGGCCGAA ACAGACA |
| 1540 | GUAGGCC CUGAUGAGGCCGAAAGGCCGAA AUUACAG |
| 1546 | UGAAUAG CUGAUGAGGCCGAAAGGCCGAA AGGCCGA |
| 1549 | CACUGAA CUGAUGAGGCCGAAAGGCCGAA AGUAGGC |
| 1551 | GCCACUG CUGAUGAGGCCGAAAGGCCGAA AUAGUAG |
| 1552 | CGCCACU CUGAUGAGGCCGAAAGGCCGAA AAUAGUA |
| 1566 | CAACCUU CUGAUGAGGCCGAAAGGCCGAA AUUUCUC |
| 1572 | CCUAAGC CUGAUGAGGCCGAAAGGCCGAA ACCUUUA |
| 1576 | CUUUCCU CUGAUGAGGCCGAAAGGCCGAA AGCAACC |
| 1577 | UCUUUCC CUGAUGAGGCCGAAAGGCCGAA AAGCAAC |

# Table 25: Mouse TNF-a HH Target Sequences

| nt. Position | HH Target Sequence | nt. Position | HH Target Sequence |
|---|---|---|---|
| 66 | UgGAAAU a GcucCcA | 324 | GgGUGAU C GGuCCCC |
| 101 | GGCAGGU U CUgUcCC | 347 | GAGAagU u cCCAaaU |
| 101 | GGCAGgU u CuGUccC | 364 | CCUCcCU C UcAUCAG |
| 102 | GCAGGUU C UgUcCCU | 366 | UCcCUCU c AUCAGuu |
| 102 | gCAGgUU c ugUCCCU | 366 | UcCCUCU C auCAGuU |
| 106 | GUUCUgU c CCUuUCA | 369 | CUCUcAU C AGuuCUa |
| 110 | UgUcCCU u UCACucA | 376 | CAGuuCU a UGGCCCA |
| 111 | gUCcCUU u CaCUCAC | 390 | AgACCCU C AcaCUcA |
| 111 | guCCCuU u CACuCAc | 396 | ucaCAcU C AGAUCAU |
| 112 | UcCCUuU C ACucACU | 401 | cUCAGAU C AUCUUCU |
| 116 | UuUCACU C AcUGgcc | 404 | AGAUCAU C UUCUCaA |
| 137 | GCCaCAU C uCCcUCc | 406 | AUCAUCU U CUCaAAa |
| 139 | caCAuCU C CCUCcAg | 406 | AUcAUcU U cUcaAAA |
| 177 | GCAUGAU C CGcGACG | 407 | UCAUCUU C UCaAAau |
| 207 | AGGCaCU C CCCcAaA | 409 | AUCUUCU C aAAauuC |
| 228 | GGGGCuU C CAGAACU | 409 | AuCuuCU c AaAAUUC |
| 228 | GGGGCuU c CAGaacU | 409 | aUcUUcU c AAAauUc |
| 236 | CAGaaCU C CAGGCGG | 432 | AGCCUGU A GCCCAcG |
| 236 | CAGaACU c cAGgcGg | | |
| 249 | GGugCCU a UgUCUcA | | |
| 249 | GGuGCCU a UGucUCa | 444 | AcGUcGU A GCAAACC |
| | | 501 | AcGCCCU C CUGGCCA |
| 261 | UCAGCCU C UUCUCaU | 560 | gGgUUGU a CCUuguC |
| 261 | UCAgCCU C UUCUcau | 560 | GGguUGU A CCUugUC |
| 263 | AGCCUCU U CUCaUUC | 564 | UGUACCU u gUCUACU |
| 263 | AgCCUCU U CUcauUC | 567 | ACCUugU C UACUCCC |
| 264 | GCCUCUU C UCaUUCC | 569 | CUugUCU A CUCCCAG |
| 264 | gCCUCUU C UcauUCc | 572 | gUCUACU C CCAGGUu |
| 266 | CUCUUCU C aUUCCUG | 572 | GUCUaCU c CCAGguu |
| 269 | UUCUCaU U CCUGcUu | 572 | GuCUacU C CCAgGUu |
| 270 | UCUCaUU C CUGcUuG | 579 | CCCAGGU u CUCUUCA |
| 276 | UCCUGcU u GUGGCAG | 580 | CCAGguU c uCUUcAa |
| 297 | CCACGCU C UUCUGuC | 580 | CCaGGuU c UCuUcaa |
| 299 | ACGCUCU U CUGuCUa | 582 | AGGUUCU C UUCaagg |
| 300 | CGCUCUU C UGuCUaC | 582 | AGGUuCU C UUCAAGG |
| 304 | CUuCUgU c uAcUGaa | 584 | GUuCUCU U CAAGGGa |
| 306 | UcUGUcU a cUgAAcU | 585 | UuCUCUU C AAGGGaC |
| 314 | CUGaACU U cGGgGUG | 608 | CcCGaCU a CgugCUC |
| 315 | UGaACUU c GGgGUGA | 615 | aCgUGcU C CUCAcCC |
| 315 | uGaaCUU c GGGguGa | 615 | AcGUGCU C CUCACCC |
| 324 | gGGUGaU c GgUCCcC | 618 | UGCUCCU C ACCCACA |

| | |
|---|---|
| 630 | ACACCgU C AGCCGau |
| 630 | ACACCgU C AgCCgaU |
| 638 | agcCgAU u uGCUaUc |
| 643 | aUUUGcU a uCUcAuA |
| 645 | UuGCuaU C UCaUACC |
| 647 | GCuaUCU C aUACCAG |
| 663 | agAAaGU C AACCUCC |
| 669 | UCAACCU C CUCUCUG |
| 669 | UcAAccU c cUcUCUG |
| 672 | ACCUCCU C UCUGCCg |
| 674 | CUCCUCU C UGCCgUC |
| 681 | cUGCCgU C AagaGcC |
| 681 | CUGCCgU C AAGAGCC |
| 681 | CUGcCgU C aaGAgcC |
| 734 | CCCUGGU A UGAGCCC |
| 734 | CccUGGU a ugaGCCc |
| 744 | AGCCCAU a UAcCUGG |
| 746 | CCCAUaU A cCUGGGA |
| 759 | GAgGAGU C uuCCAGc |
| 759 | GAGGaGU C UUCCAGC |
| 761 | GGaGUCU U CCAGCUG |
| 762 | GaGUCUU C CAGCUGG |
| 786 | ACCaACU C AGCGCUG |
| 798 | CUGAGgU C AAUCuGC |
| 802 | GgUCAAU C uGCCCaA |
| 812 | CCCaAgU A cuUaGAC |
| 816 | AgUAcuU a GACUUUG |
| 821 | uUaGACU U UGCgGAG |
| 822 | UaGACUU U GCgGAGU |
| 830 | GCgGAGU C cGGGCAG |
| 840 | GGCAGGU C UACUUUG |
| 842 | CAGGUCU A CUUUGGa |
| 842 | CAGgucU a CUUugGA |
| 842 | cagGuCU a CUUUgGA |
| 845 | GUCUACU U UGGagUC |
| 846 | UCUACUU U GGagUCA |
| 852 | UUGGagU C AUUGCuC |
| 855 | GagUCAU U GCuCUGU |
| 887 | AUCCaUU c ucUACCC |
| 891 | AuucuCU a CCCaGCC |
| 905 | CCcCaCU C UgaCCCC |
| 905 | cCCCacU c UgACCCC |
| 905 | CcCCACU c uGAccCC |
| 914 | GAcCCcU U uacUCUG |
| 915 | ACCCCuU u acUCuGA |
| 919 | CUUUAcU c ugaCCcC |
| 928 | GACCcCU u UaUugUC |
| 928 | gAcCCCU U UAUUguC |
| 932 | CCUUUAU U guCuaCU |

| | |
|---|---|
| 940 | GuCUACU c cUCAGaG |
| 943 | UACUccU C AGaGcCc |
| 972 | UCUaaCU u AgAAAGg |
| 972 | ucUaaCU u AGAaAgG |
| 973 | CUaACuU A GAAAggG |
| 984 | AGgGgAU U auGGcuc |
| 984 | AGGGgaU U aUGgCUc |
| 985 | GGGGauU a uGGcUCa |
| 997 | UcAGAgU c CAAcucu |
| 1010 | CuguGCU c AGAgCUU |
| 1017 | cAGAgCU U UcAaCAA |
| 1018 | AGAgCUU U cAaCAAC |
| 1019 | GAgCUUU c AaCAACu |
| 1073 | UgGGCCU c ucAUgCA |
| 1096 | AAGgAcU C AAAugGG |
| 1106 | aUGGGcU U uccGAAU |
| 1107 | UGGGcUU u ccGAAUu |
| 1108 | GGgCuUU c cGaaUUC |
| 1115 | CcGAAuU C ACUGGaG |
| 1133 | CGAAugU C CAuuCcU |
| 1164 | gagUGgU c AgGUUGc |
| 1180 | UcUgUcU c agaAUGA |
| 1203 | aaGAuCU c AGGCCUU |
| 1210 | cAGGCCU U CCUacCU |
| 1211 | AGGCCUU C CUacCUu |
| 1214 | CCUUCCU a cCUuCAG |
| 1218 | CcuACcU u CaGACCu |
| 1218 | CCuaCCU U CAGACcu |
| 1218 | cCuACcU u cAgACCU |
| 1218 | CCUacCU u CAGAccU |
| 1219 | CuaCCUU C AGACcuu |
| 1219 | CuAcCUU c agACcUU |
| 1226 | CagACCU U uCCAgAC |
| 1226 | CAGAccU U UCCAGAC |
| 1227 | agACCUU u CCAgACu |
| 1227 | AGAccUU U CCAGACU |
| 1228 | GAccUUU C CAGACUc |
| 1238 | gACUCuU c cCUGAGG |
| 1262 | CAGCCuU C CuCAcaG |
| 1283 | CCCCccU C uaUUUAU |
| 1283 | cCcCCCU C UAUUUAU |
| 1285 | cCCCUCU A UUUAUaU |
| 1287 | CcuCUAU u UauAuUU |
| 1287 | CCUCUAU U UAUaUUU |
| 1288 | CUCUAUU U AUaUUUG |
| 1289 | UCUAUUU A UaUUUGC |
| 1293 | UUUAUaU U UGCACUU |
| 1293 | uUUaUaU u UGcAcUu |
| 1294 | UUAUaUU U GCACUUa |

| | |
|---|---|
| 1300 | UUGCACU U aUuAUUu |
| 1303 | CAcuUaU u AuUuAUU |
| 1304 | acUuAUU A UUUAUUA |
| 1306 | UuAUUAU U UAUUAUU |
| 1307 | uAUUAUU U AUUAUUU |
| 1307 | UaUUaUU U AuuAUuU |
| 1308 | AUUAUUU A UUAUUUA |
| 1310 | UauUuAU U AUUUAUU |
| 1310 | UAUUUAU U AUUUAUU |
| 1310 | UAUUUAU U AUUUAUU |
| 1311 | AUUUAUU A UUUAUUU |
| 1311 | AUUUAUU A UUUAUUU |
| 1311 | AuuUAUU A UuUauUU |
| 1313 | UUAUUAU U UAUUUAU |
| 1313 | UUAUUAU U UAUUUAU |
| 1313 | uUAUUAU u UauUUAu |
| 1314 | UAUUAUU U AUUUAUU |
| 1314 | UAUUAUU U AUUUAUU |
| 1315 | AUUAUUU A UUUAUUA |
| 1317 | UAUUUAU U UAUUAUU |
| 1318 | AUUUAUU U AUUAUUU |
| 1319 | UUUAUUU A UUAUUUA |
| 1326 | AUUAUUU A UUUAUUU |
| 1328 | UAUUUAU U UAUUUgC |
| 1329 | AUUUAUU U AUUUgCu |
| 1330 | UUUAUUU A UUUgCuu |
| 1332 | UAUUUAU U UgCuuAU |
| 1333 | AUUUAUU U gCuuAUG |
| 1337 | auUUGCU U AuGAAuG |
| 1338 | uUUGCUU A uGAAuGu |
| 1346 | UGAAUGU A UUUAUUU |
| 1348 | AAUGUAU U UAUUUGG |
| 1349 | AUGUAUU U AUUUGGa |
| 1350 | UGUAUUU A UUUGGaA |
| 1352 | uAUuUAU u UGGaAGG |
| 1352 | UAUUUAU U UGGaAGg |
| 1353 | AUUUAUU U GGaAGgC |
| 1369 | GGGGUgU C CUGGaGG |
| 1398 | gCUguCU U cAGACAg |
| 1398 | GCUGuCU U cagaCAG |
| 1412 | GACAUGU U UUCuGUG |
| 1413 | ACAUGUU U UCuGUGA |
| 1414 | CAUGUUU U CuGUGAA |
| 1415 | AUGUUUU C uGUGAAA |
| 1415 | AUGUUUU c UgugAaA |
| 1438 | gaGCUGU c CCCAccU |
| 1451 | CUGGCCU C UcUaCCU |
| 1453 | ggCCUCU C UaCCuUG |
| 1462 | aCCuUGU u GCCuCCU |
| 1470 | GccuCcU C UUUUGcU |
| 1472 | cuCcUCU U UUGcUUA |
| 1473 | uCcUCUU U UGcUUAU |
| 1474 | CcUCUUU U GcUUAUG |
| 1478 | UUUUGcU U AUGUUUa |
| 1479 | UUUGcUU a UGuuuAa |
| 1479 | UUUGcUU A UGUUUaa |
| 1484 | UUAUGUU U aaaAcAA |
| 1498 | AAAuauU U AUCUaAc |
| 1511 | AcccAaU U GUCUuAA |
| 1514 | cAaUUGU C UuAAuAA |
| 1516 | aUUGUCU u AAuAAcG |
| 1529 | CgcugAU u UGGuGAC |
| 1529 | cGCUGAU U UGGUGAC |
| 1530 | gCUGAUU u gGUgacC |
| 1530 | GCUGAUU U GGUGACC |
| 1563 | UgaAcCU c UGcUCCC |
| 1563 | ugaaCCU C UGCUCCC |
| 1568 | CUCUGCU C CCCAcGG |
| 1589 | UGaCUGU A AUuGcCC |
| 1592 | CUGUAAU u GcCCUAC |
| 1617 | GAGAAAU A AAGaUcG |
| 1623 | UAAAGaU c GCUUAaa |
| 1633 | UUAaaaU a aaAAaCC |
| 25 | AgGgaCU a gCCagGA |

# Table 26: Mouse TNF-α Hammerhead Ribozyme Sequences

| nt. Position | Mouse HH Ribozyme Sequence |
|---|---|
| 25 | UCCUGGC CUGAUGAGGCCGAAAGGCCGAA AGUCCCU |
| 66 | UGGGAGC CUGAUGAGGCCGAAAGGCCGAA AUUUCCA |
| 101 | GGGACAG CUGAUGAGGCCGAAAGGCCGAA ACCUGCC |
| 101 | GGGACAG CUGAUGAGGCCGAAAGGCCGAA ACCUGCC |
| 102 | AGGGACA CUGAUGAGGCCGAAAGGCCGAA AACCUGC |
| 102 | AGGGACA CUGAUGAGGCCGAAAGGCCGAA AACCUGC |
| 106 | UGAAAGG CUGAUGAGGCCGAAAGGCCGAA ACAGAAC |
| 110 | UGAGUGA CUGAUGAGGCCGAAAGGCCGAA AGGGACA |
| 111 | GUGAGUG CUGAUGAGGCCGAAAGGCCGAA AAGGGAC |
| 111 | GUGAGUG CUGAUGAGGCCGAAAGGCCGAA AAGGGAC |
| 112 | AGUGAGU CUGAUGAGGCCGAAAGGCCGAA AAAGGGA |
| 116 | GGCCAGU CUGAUGAGGCCGAAAGGCCGAA AGUGAAA |
| 137 | GGAGGGA CUGAUGAGGCCGAAAGGCCGAA AUGUGGC |
| 139 | CUGGAGG CUGAUGAGGCCGAAAGGCCGAA AGAUGUG |
| 177 | CGUCGCG CUGAUGAGGCCGAAAGGCCGAA AUCAUGC |
| 207 | UUUGGGG CUGAUGAGGCCGAAAGGCCGAA AGUGCCU |
| 228 | AGUUCUG CUGAUGAGGCCGAAAGGCCGAA AAGCCCC |
| 228 | AGUUCUG CUGAUGAGGCCGAAAGGCCGAA AAGCCCC |
| 236 | CCGCCUG CUGAUGAGGCCGAAAGGCCGAA AGUUCUG |
| 236 | CCGCCUG CUGAUGAGGCCGAAAGGCCGAA AGUUCUG |
| 249 | UGAGACA CUGAUGAGGCCGAAAGGCCGAA AGGCACC |
| 249 | UGAGACA CUGAUGAGGCCGAAAGGCCGAA AGGCACC |
| 261 | AUGAGAA CUGAUGAGGCCGAAAGGCCGAA AGGCUGA |
| 261 | AUGAGAA CUGAUGAGGCCGAAAGGCCGAA AGGCUGA |
| 263 | GAAUGAG CUGAUGAGGCCGAAAGGCCGAA AGAGGCU |
| 263 | GAAUGAG CUGAUGAGGCCGAAAGGCCGAA AGAGGCU |
| 264 | GGAAUGA CUGAUGAGGCCGAAAGGCCGAA AAGAGGC |
| 264 | GGAAUGA CUGAUGAGGCCGAAAGGCCGAA AAGAGGC |
| 266 | CAGGAAU CUGAUGAGGCCGAAAGGCCGAA AGAAGAG |
| 269 | AAGCAGG CUGAUGAGGCCGAAAGGCCGAA AUGAGAA |
| 270 | CAAGCAG CUGAUGAGGCCGAAAGGCCGAA AAUGAGA |
| 276 | CUGCCAC CUGAUGAGGCCGAAAGGCCGAA AGCAGGA |
| 297 | GACAGAA CUGAUGAGGCCGAAAGGCCGAA AGCGUGG |
| 299 | UAGACAG CUGAUGAGGCCGAAAGGCCGAA AGAGCGU |
| 300 | GUAGACA CUGAUGAGGCCGAAAGGCCGAA AAGAGCG |
| 304 | UUCAGUA CUGAUGAGGCCGAAAGGCCGAA ACAGAAG |
| 306 | AGUUCAG CUGAUGAGGCCGAAAGGCCGAA AGACAGA |
| 314 | CACCCCG CUGAUGAGGCCGAAAGGCCGAA AGUUCAG |
| 315 | UCACCCC CUGAUGAGGCCGAAAGGCCGAA AAGUUCA |

| | |
|---|---|
| 315 | UCACCCC CUGAUGAGGCCGAAAGGCCGAA AAGUUCA |
| 324 | GGGGACC CUGAUGAGGCCGAAAGGCCGAA AUCACCC |
| 324 | GGGGACC CUGAUGAGGCCGAAAGGCCGAA AUCACCC |
| 347 | AUUUGGG CUGAUGAGGCCGAAAGGCCGAA ACUUCUC |
| 364 | CUGAUGA CUGAUGAGGCCGAAAGGCCGAA AGGGAGG |
| 366 | AACUGAU CUGAUGAGGCCGAAAGGCCGAA AGAGGGA |
| 366 | AACUGAU CUGAUGAGGCCGAAAGGCCGAA AGAGGGA |
| 369 | UAGAACU CUGAUGAGGCCGAAAGGCCGAA AUGAGAG |
| 376 | UGGGCCA CUGAUGAGGCCGAAAGGCCGAA AGAACUG |
| 390 | UGAGUGU CUGAUGAGGCCGAAAGGCCGAA AGGGUCU |
| 396 | AUGAUCU CUGAUGAGGCCGAAAGGCCGAA AGUGUGA |
| 401 | AGAAGAU CUGAUGAGGCCGAAAGGCCGAA AUCUGAG |
| 404 | UUGAGAA CUGAUGAGGCCGAAAGGCCGAA AUGAUCU |
| 406 | UUUUGAG CUGAUGAGGCCGAAAGGCCGAA AGAUGAU |
| 406 | UUUUGAG CUGAUGAGGCCGAAAGGCCGAA AGAUGAU |
| 407 | AUUUUGA CUGAUGAGGCCGAAAGGCCGAA AAGAUGA |
| 409 | GAAUUUU CUGAUGAGGCCGAAAGGCCGAA AGAAGAU |
| 409 | GAAUUUU CUGAUGAGGCCGAAAGGCCGAA AGAAGAU |
| 409 | GAAUUUU CUGAUGAGGCCGAAAGGCCGAA AGAAGAU |
| 432 | CGUGGGC CUGAUGAGGCCGAAAGGCCGAA ACAGGCU |
| 444 | GGUUUGC CUGAUGAGGCCGAAAGGCCGAA ACGACGU |
| 501 | UGGCCAG CUGAUGAGGCCGAAAGGCCGAA AGGGCGU |
| 560 | GACAAGG CUGAUGAGGCCGAAAGGCCGAA ACAACCC |
| 560 | GACAAGG CUGAUGAGGCCGAAAGGCCGAA ACAACCC |
| 564 | AGUAGAC CUGAUGAGGCCGAAAGGCCGAA AGGUACA |
| 567 | GGGAGUA CUGAUGAGGCCGAAAGGCCGAA ACAAGGU |
| 569 | CUGGGAG CUGAUGAGGCCGAAAGGCCGAA AGACAAG |
| 572 | AACCUGG CUGAUGAGGCCGAAAGGCCGAA AGUAGAC |
| 572 | AACCUGG CUGAUGAGGCCGAAAGGCCGAA AGUAGAC |
| 572 | AACCUGG CUGAUGAGGCCGAAAGGCCGAA AGUAGAC |
| 579 | UGAAGAG CUGAUGAGGCCGAAAGGCCGAA ACCUGGG |
| 580 | UUGAAGA CUGAUGAGGCCGAAAGGCCGAA AACCUGG |
| 580 | UUGAAGA CUGAUGAGGCCGAAAGGCCGAA AACCUGG |
| 582 | CCUUGAA CUGAUGAGGCCGAAAGGCCGAA AGAACCU |
| 582 | CCUUGAA CUGAUGAGGCCGAAAGGCCGAA AGAACCU |
| 584 | UCCCUUG CUGAUGAGGCCGAAAGGCCGAA AGAGAAC |
| 585 | GUCCCUU CUGAUGAGGCCGAAAGGCCGAA AAGAGAA |
| 608 | GAGCACG CUGAUGAGGCCGAAAGGCCGAA AGUCGGG |
| 615 | GGGUGAG CUGAUGAGGCCGAAAGGCCGAA AGCACGU |
| 615 | GGGUGAG CUGAUGAGGCCGAAAGGCCGAA AGCACGU |
| 618 | UGUGGGU CUGAUGAGGCCGAAAGGCCGAA AGGAGCA |
| 630 | AUCGGCU CUGAUGAGGCCGAAAGGCCGAA ACGGUGU |
| 630 | AUCGGCU CUGAUGAGGCCGAAAGGCCGAA ACGGUGU |
| 638 | GAUAGCA CUGAUGAGGCCGAAAGGCCGAA AUCGGCU |
| 643 | UAUGAGA CUGAUGAGGCCGAAAGGCCGAA AGCAAAU |
| 645 | GGUAUGA CUGAUGAGGCCGAAAGGCCGAA AUAGCAA |
| 647 | CUGGUAU CUGAUGAGGCCGAAAGGCCGAA AGAUAGC |

| | |
|---|---|
| 663 | GGAGGUU CUGAUGAGGCCGAAAGGCCGAA ACUUUCCU |
| 669 | CAGAGAG CUGAUGAGGCCGAAAGGCCGAA AGGUUGA |
| 669 | CAGAGAG CUGAUGAGGCCGAAAGGCCGAA AGGUUGA |
| 672 | CGGCAGA CUGAUGAGGCCGAAAGGCCGAA AGGAGGU |
| 674 | GACGGCA CUGAUGAGGCCGAAAGGCCGAA AGAGGAG |
| 681 | GGCUCUU CUGAUGAGGCCGAAAGGCCGAA ACGGCAG |
| 681 | GGCUCUU CUGAUGAGGCCGAAAGGCCGAA ACGGCAG |
| 681 | GGCUCUU CUGAUGAGGCCGAAAGGCCGAA ACGGCAG |
| 734 | GGGCUCA CUGAUGAGGCCGAAAGGCCGAA ACCAGGG |
| 734 | GGGCUCA CUGAUGAGGCCGAAAGGCCGAA ACCAGGG |
| 744 | CCAGGUA CUGAUGAGGCCGAAAGGCCGAA AUGGGCU |
| 746 | UCCCAGG CUGAUGAGGCCGAAAGGCCGAA AUAUGGG |
| 759 | GCUGGAA CUGAUGAGGCCGAAAGGCCGAA ACUCCUC |
| 759 | GCUGGAA CUGAUGAGGCCGAAAGGCCGAA ACUCCUC |
| 761 | CAGCUGG CUGAUGAGGCCGAAAGGCCGAA AGACUCC |
| 762 | CCAGCUG CUGAUGAGGCCGAAAGGCCGAA AAGACUC |
| 786 | CAGCGCU CUGAUGAGGCCGAAAGGCCGAA AGUUGGU |
| 798 | GCAGAUU CUGAUGAGGCCGAAAGGCCGAA ACCUCAG |
| 802 | UUGGGCA CUGAUGAGGCCGAAAGGCCGAA AUUGACC |
| 812 | GUCUAAG CUGAUGAGGCCGAAAGGCCGAA ACUUGGG |
| 816 | CAAAGUC CUGAUGAGGCCGAAAGGCCGAA AAGUACU |
| 821 | CUCCGCA CUGAUGAGGCCGAAAGGCCGAA AGUCUAA |
| 822 | ACUCCGC CUGAUGAGGCCGAAAGGCCGAA AAGUCUA |
| 830 | CUGCCCG CUGAUGAGGCCGAAAGGCCGAA ACUCCGC |
| 840 | CAAAGUA CUGAUGAGGCCGAAAGGCCGAA ACCUGCC |
| 842 | UCCAAAG CUGAUGAGGCCGAAAGGCCGAA AGACCUG |
| 842 | UCCAAAG CUGAUGAGGCCGAAAGGCCGAA AGACCUG |
| 842 | UCCAAAG CUGAUGAGGCCGAAAGGCCGAA AGACCUG |
| 845 | GACUCCA CUGAUGAGGCCGAAAGGCCGAA AGUAGAC |
| 846 | UGACUCC CUGAUGAGGCCGAAAGGCCGAA AAGUAGA |
| 852 | GAGCAAU CUGAUGAGGCCGAAAGGCCGAA ACUCCAA |
| 855 | ACAGAGC CUGAUGAGGCCGAAAGGCCGAA AUGACUC |
| 887 | GGGUAGA CUGAUGAGGCCGAAAGGCCGAA AAUGGAU |
| 891 | GGCUGGG CUGAUGAGGCCGAAAGGCCGAA AGAGAAU |
| 905 | GGGGUCA CUGAUGAGGCCGAAAGGCCGAA AGUGGGG |
| 905 | GGGGUCA CUGAUGAGGCCGAAAGGCCGAA AGUGGGG |
| 905 | GGGGUCA CUGAUGAGGCCGAAAGGCCGAA AGUGGGG |
| 914 | CAGAGUA CUGAUGAGGCCGAAAGGCCGAA AGGGGUC |
| 915 | UCAGAGU CUGAUGAGGCCGAAAGGCCGAA AAGGGGU |
| 919 | GGGGUCA CUGAUGAGGCCGAAAGGCCGAA AGUAAAG |
| 928 | GACAAUA CUGAUGAGGCCGAAAGGCCGAA AGGGGUC |
| 928 | GACAAUA CUGAUGAGGCCGAAAGGCCGAA AGGGGUC |
| 932 | AGUAGAC CUGAUGAGGCCGAAAGGCCGAA AUAAAGG |
| 940 | CUCUGAG CUGAUGAGGCCGAAAGGCCGAA AGUAGAC |
| 943 | GGGCUCU CUGAUGAGGCCGAAAGGCCGAA AGGAGUA |
| 972 | CCUUUCU CUGAUGAGGCCGAAAGGCCGAA AGUUAGA |
| 972 | CCUUUCU CUGAUGAGGCCGAAAGGCCGAA AGUUAGA |
| 973 | CCCUUUC CUGAUGAGGCCGAAAGGCCGAA AAGUUAG |
| 984 | GAGCCAU CUGAUGAGGCCGAAAGGCCGAA AUCCCCU |

| | |
|---|---|
| 984 | GAGCCAU CUGAUGAGGCCGAAAGGCCGAA AUCCCCU |
| 985 | UGAGCCA CUGAUGAGGCCGAAAGGCCGAA AAUCCCC |
| 997 | AGAGUUG CUGAUGAGGCCGAAAGGCCGAA ACUCUGA |
| 1010 | AAGCUCU CUGAUGAGGCCGAAAGGCCGAA AGCACAG |
| 1017 | UUGUUGA CUGAUGAGGCCGAAAGGCCGAA AGCUCUG |
| 1018 | GUUGUUG CUGAUGAGGCCGAAAGGCCGAA AAGCUCU |
| 1019 | AGUUGUU CUGAUGAGGCCGAAAGGCCGAA AAAGCUC |
| 1073 | UGCAUGA CUGAUGAGGCCGAAAGGCCGAA AGGCCCA |
| 1096 | CCCAUUU CUGAUGAGGCCGAAAGGCCGAA AGUCCUU |
| 1106 | AUUCGGA CUGAUGAGGCCGAAAGGCCGAA AGCCCAU |
| 1107 | AAUUCGG CUGAUGAGGCCGAAAGGCCGAA AAGCCCA |
| 1108 | GAAUUCG CUGAUGAGGCCGAAAGGCCGAA AAAGCCC |
| 1115 | CUCCAGU CUGAUGAGGCCGAAAGGCCGAA AAUUCGG |
| 1133 | AGGAAUG CUGAUGAGGCCGAAAGGCCGAA ACAUUCG |
| 1164 | GCAACCU CUGAUGAGGCCGAAAGGCCGAA ACCACUC |
| 1180 | UCAUUCU CUGAUGAGGCCGAAAGGCCGAA AGACAGA |
| 1203 | AAGGCCU CUGAUGAGGCCGAAAGGCCGAA AGAUCUU |
| 1210 | AGGUAGG CUGAUGAGGCCGAAAGGCCGAA AGGCCUG |
| 1211 | AAGGUAG CUGAUGAGGCCGAAAGGCCGAA AAGGCCU |
| 1214 | CUGAAGG CUGAUGAGGCCGAAAGGCCGAA AGGAAGG |
| 1218 | AGGUCUG CUGAUGAGGCCGAAAGGCCGAA AGGUAGG |
| 1218 | AGGUCUG CUGAUGAGGCCGAAAGGCCGAA AGGUAGG |
| 1218 | AGGUCUG CUGAUGAGGCCGAAAGGCCGAA AGGUAGG |
| 1218 | AGGUCUG CUGAUGAGGCCGAAAGGCCGAA AGGUAGG |
| 1219 | AAGGUCU CUGAUGAGGCCGAAAGGCCGAA AAGGUAG |
| 1219 | AAGGUCU CUGAUGAGGCCGAAAGGCCGAA AAGGUAG |
| 1226 | GUCUGGA CUGAUGAGGCCGAAAGGCCGAA AGGUCUG |
| 1226 | GUCUGGA CUGAUGAGGCCGAAAGGCCGAA AGGUCUG |
| 1227 | AGUCUGG CUGAUGAGGCCGAAAGGCCGAA AAGGUCU |
| 1227 | AGUCUGG CUGAUGAGGCCGAAAGGCCGAA AAGGUCU |
| 1228 | GAGUCUG CUGAUGAGGCCGAAAGGCCGAA AAAGGUC |
| 1238 | CCUCAGG CUGAUGAGGCCGAAAGGCCGAA AAGAGUC |
| 1262 | CUGUGAG CUGAUGAGGCCGAAAGGCCGAA AAGGCUG |
| 1283 | AUAAAUA CUGAUGAGGCCGAAAGGCCGAA AGGGGGG |
| 1283 | AUAAAUA CUGAUGAGGCCGAAAGGCCGAA AGGGGGG |
| 1285 | AUAUAAA CUGAUGAGGCCGAAAGGCCGAA AGAGGGG |
| 1287 | AAAUAUA CUGAUGAGGCCGAAAGGCCGAA AUAGAGG |
| 1287 | AAAUAUA CUGAUGAGGCCGAAAGGCCGAA AUAGAGG |
| 1288 | CAAAUAU CUGAUGAGGCCGAAAGGCCGAA AAUAGAG |
| 1289 | GCAAAUA CUGAUGAGGCCGAAAGGCCGAA AAAUAGA |
| 1293 | AAGUGCA CUGAUGAGGCCGAAAGGCCGAA AUAUAAA |
| 1293 | AAGUGCA CUGAUGAGGCCGAAAGGCCGAA AUAUAAA |
| 1294 | UAAGUGC CUGAUGAGGCCGAAAGGCCGAA AAUAUAA |
| 1300 | AAAUAAU CUGAUGAGGCCGAAAGGCCGAA AGUGCAA |
| 1303 | AAUAAAU CUGAUGAGGCCGAAAGGCCGAA AUAAGUG |
| 1304 | UAAUAAA CUGAUGAGGCCGAAAGGCCGAA AAUAAGU |
| 1306 | AAUAAUA CUGAUGAGGCCGAAAGGCCGAA AUAAUAA |
| 1307 | AAAUAAU CUGAUGAGGCCGAAAGGCCGAA AAUAAUA |
| 1307 | AAAUAAU CUGAUGAGGCCGAAAGGCCGAA AAUAAUA |

| | | | |
|---|---|---|---|
| 1308 | UAAAUAA | CUGAUGAGGCCGAAAGGCCGAA | AAAUAAU |
| 1310 | AAUAAAU | CUGAUGAGGCCGAAAGGCCGAA | AUAAAUA |
| 1310 | AAUAAAU | CUGAUGAGGCCGAAAGGCCGAA | AUAAAUA |
| 1310 | AAUAAAU | CUGAUGAGGCCGAAAGGCCGAA | AUAAAUA |
| 1311 | AAAUAAA | CUGAUGAGGCCGAAAGGCCGAA | AAUAAAU |
| 1311 | AAAUAAA | CUGAUGAGGCCGAAAGGCCGAA | AAUAAAU |
| 1311 | AAAUAAA | CUGAUGAGGCCGAAAGGCCGAA | AAUAAAU |
| 1313 | AUAAAUA | CUGAUGAGGCCGAAAGGCCGAA | AUAAUAA |
| 1313 | AUAAAUA | CUGAUGAGGCCGAAAGGCCGAA | AUAAUAA |
| 1313 | AUAAAUA | CUGAUGAGGCCGAAAGGCCGAA | AUAAUAA |
| 1314 | AAUAAAU | CUGAUGAGGCCGAAAGGCCGAA | AAUAAUA |
| 1314 | AAUAAAU | CUGAUGAGGCCGAAAGGCCGAA | AAUAAUA |
| 1315 | UAAUAAA | CUGAUGAGGCCGAAAGGCCGAA | AAAUAAU |
| 1317 | AAUAAUA | CUGAUGAGGCCGAAAGGCCGAA | AUAAAUA |
| 1318 | AAAUAAU | CUGAUGAGGCCGAAAGGCCGAA | AAUAAAU |
| 1319 | UAAAUAA | CUGAUGAGGCCGAAAGGCCGAA | AAAUAAA |
| 1326 | AAAUAAA | CUGAUGAGGCCGAAAGGCCGAA | AAAUAAU |
| 1328 | GCAAAUA | CUGAUGAGGCCGAAAGGCCGAA | AUAAAUA |
| 1329 | AGCAAAU | CUGAUGAGGCCGAAAGGCCGAA | AAUAAAU |
| 1330 | AAGCAAA | CUGAUGAGGCCGAAAGGCCGAA | AAAUAAA |
| 1332 | AUAAGCA | CUGAUGAGGCCGAAAGGCCGAA | AUAAAUA |
| 1333 | CAUAAGC | CUGAUGAGGCCGAAAGGCCGAA | AAUAAAU |
| 1337 | CAUUCAU | CUGAUGAGGCCGAAAGGCCGAA | AGCAAAU |
| 1338 | ACAUUCA | CUGAUGAGGCCGAAAGGCCGAA | AAGCAAA |
| 1346 | AAAUAAA | CUGAUGAGGCCGAAAGGCCGAA | ACAUUCA |
| 1348 | CCAAAUA | CUGAUGAGGCCGAAAGGCCGAA | AUACAUU |
| 1349 | UCCAAAU | CUGAUGAGGCCGAAAGGCCGAA | AAUACAU |
| 1350 | UUCCAAA | CUGAUGAGGCCGAAAGGCCGAA | AAAUACA |
| 1352 | CCUUCCA | CUGAUGAGGCCGAAAGGCCGAA | AUAAAUA |
| 1352 | CCUUCCA | CUGAUGAGGCCGAAAGGCCGAA | AUAAAUA |
| 1353 | GCCUUCC | CUGAUGAGGCCGAAAGGCCGAA | AAUAAAU |
| 1369 | CCUCCAG | CUGAUGAGGCCGAAAGGCCGAA | ACACCCC |
| 1398 | CUGUCUG | CUGAUGAGGCCGAAAGGCCGAA | AGACAGC |
| 1398 | CUGUCUG | CUGAUGAGGCCGAAAGGCCGAA | AGACAGC |
| 1412 | CACAGAA | CUGAUGAGGCCGAAAGGCCGAA | ACAUGUC |
| 1413 | UCACAGA | CUGAUGAGGCCGAAAGGCCGAA | AACAUGU |
| 1414 | UUCACAG | CUGAUGAGGCCGAAAGGCCGAA | AAACAUG |
| 1415 | UUUCACA | CUGAUGAGGCCGAAAGGCCGAA | AAAACAU |
| 1415 | UUUCACA | CUGAUGAGGCCGAAAGGCCGAA | AAAACAU |
| 1438 | AGGUGGG | CUGAUGAGGCCGAAAGGCCGAA | ACAGCUC |
| 1451 | AGGUAGA | CUGAUGAGGCCGAAAGGCCGAA | AGGCCAG |
| 1453 | CAAGGUA | CUGAUGAGGCCGAAAGGCCGAA | AGAGGCC |
| 1455 | AACAAGG | CUGAUGAGGCCGAAAGGCCGAA | AGAGAGG |
| 1462 | AGGAGGC | CUGAUGAGGCCGAAAGGCCGAA | ACAAGGU |
| 1470 | AGCAAAA | CUGAUGAGGCCGAAAGGCCGAA | AGGAGGC |
| 1472 | UAAGCAA | CUGAUGAGGCCGAAAGGCCGAA | AGAGGAG |
| 1473 | AUAAGCA | CUGAUGAGGCCGAAAGGCCGAA | AAGAGGA |
| 1474 | CAUAAGC | CUGAUGAGGCCGAAAGGCCGAA | AAAGAGG |
| 1478 | UAAACAU | CUGAUGAGGCCGAAAGGCCGAA | AGCAAAA |

| | | | |
|---|---|---|---|
| 1479 | UUAAACA | CUGAUGAGGCCGAAAGGCCGAA | AAGCAAA |
| 1479 | UUAAACA | CUGAUGAGGCCGAAAGGCCGAA | AAGCAAA |
| 1484 | UUGUUUU | CUGAUGAGGCCGAAAGGCCGAA | AACAUAA |
| 1498 | GUUAGAU | CUGAUGAGGCCGAAAGGCCGAA | AAUAUUU |
| 1511 | UUAAGAC | CUGAUGAGGCCGAAAGGCCGAA | AUUGGGU |
| 1514 | UUAUUAA | CUGAUGAGGCCGAAAGGCCGAA | ACAAUUG |
| 1516 | CGUUAUU | CUGAUGAGGCCGAAAGGCCGAA | AGACAAU |
| 1529 | GUCACCA | CUGAUGAGGCCGAAAGGCCGAA | AUCAGCG |
| 1529 | GUCACCA | CUGAUGAGGCCGAAAGGCCGAA | AUCAGCG |
| 1530 | GGUCACC | CUGAUGAGGCCGAAAGGCCGAA | AAUCAGC |
| 1530 | GGUCACC | CUGAUGAGGCCGAAAGGCCGAA | AAUCAGC |
| 1563 | GGGAGCA | CUGAUGAGGCCGAAAGGCCGAA | AGGUUCA |
| 1563 | GGGAGCA | CUGAUGAGGCCGAAAGGCCGAA | AGGUUCA |
| 1568 | CCGUGGG | CUGAUGAGGCCGAAAGGCCGAA | AGCAGAG |
| 1589 | GGGCAAU | CUGAUGAGGCCGAAAGGCCGAA | ACAGUCA |
| 1592 | GUAGGGC | CUGAUGAGGCCGAAAGGCCGAA | AUUACAG |
| 1617 | CGAUCUU | CUGAUGAGGCCGAAAGGCCGAA | AUUUCUC |
| 1623 | UUUAAGC | CUGAUGAGGCCGAAAGGCCGAA | AUCUUUA |
| 1633 | GGUUUUU | CUGAUGAGGCCGAAAGGCCGAA | AUUUUAA |

Table 27: Human TNF-α Hairpin Ribozyme Sequences

| nt. Position | Hairpin Ribozyme Sequence | Substrate |
|---|---|---|
| 46 | AGCCGUGG AGAA GUAUGU ACCAGAGAAACACACGUUGUGGUACAUUACCUGGUA | ACAUACU GAC CCACGGCU |
| 54 | GAGGGUGG AGAA GUGGGU ACCAGAGAAACACACGUUGUGGUACAUUACCUGGUA | ACCCACG GCU CCACCCUC |
| 185 | GGAGAAGA AGAA GAGGAA ACCAGAGAAACACACGUUGUGGUACAUUACCUGGUA | UUCCUCA GCC UCUUCUCC |
| 201 | CUGCCACG AGAA GGAAGG ACCAGAGAAACACACGUUGUGGUACAUUACCUGGUA | CCUUCCU GAU CGUGGCAG |
| 230 | GUGCAGCA AGAA GAAGAG ACCAGAGAAACACACGUUGUGGUACAUUACCUGGUA | CUCUUCU GCC UGCUGCAC |
| 234 | CAAAGUGC AGAA GGCAGA ACCAGAGAAACACACGUUGUGGUACAUUACCUGGUA | UCUGCCU GCU GCACUUUG |
| 254 | CCUCUGGG AGAA GAUCAC ACCAGAGAAACACACGUUGUGGUACAUUACCUGGUA | GUGAUCG GCC CCCAGAGG |
| 296 | GGCCAGAG AGAA GAUUAG ACCAGAGAAACACACGUUGUGGUACAUUACCUGGUA | CUAAUCA GCC CUCUGGCC |
| 317 | AGAAGAUG AGAA GACUGC ACCAGAGAAACACACGUUGUGGUACAUUACCUGGUA | GCAGUCA GAU CAUCUUCU |
| 387 | GCCACUGG AGAA GCCCCU ACCAGAGAAACACACGUUGUGGUACAUUACCUGGUA | AGGGGCA GCU CCAGUGGC |
| 404 | AUUGGCCC AGAA GUUCAG ACCAGAGAAACACACGUUGUGGUACAUUACCUGGUA | CUGAACC GCC GGGCCAAU |
| 453 | GCACCACC AGAA GGUUAU ACCAGAGAAACACACGUUGUGGUACAUUACCUGGUA | AUAACCA GCU GGUGGUGC |
| 518 | GGUGGAGG AGAA GCCUUG ACCAGAGAAACACACGUUGUGGUACAUUACCUGGUA | CAAGGCU GCC CCUCCACC |
| 554 | GGCGAUGC AGAA GAUGGU ACCAGAGAAACACACGUUGUGGUACAUUACCUGGUA | ACCAUCA GCC GCAUCGCC |
| 565 | UGGUAGGA AGAA GCGAUG ACCAGAGAAACACACGUUGUGGUACAUUACCUGGUA | CAUCGCC GUC UCCUACCA |
| 576 | UGACCUUG AGAA GGUAGG ACCAGAGAAACACACGUUGUGGUACAUUACCUGGUA | CCUACCA GAC CAAGGUCA |
| 687 | CCUUCUCC AGAA GGAAGA ACCAGAGAAACACACGUUGUGGUACAUUACCUGGUA | UCUUCCA GCU GGAGAAGG |
| 704 | AGCGCUGA AGAA GUCACC ACCAGAGAAACACACGUUGUGGUACAUUACCUGGUA | GGUGACC GAC UCAGCGCU |
| 726 | GAUAGUCG AGAA GAUUGA ACCAGAGAAACACACGUUGUGGUACAUUACCUGGUA | UCAAUCG GCC CGACUAUC |
| 730 | UCGAGAUA AGAA GGCCGA ACCAGAGAAACACACGUUGUGGUACAUUACCUGGUA | UCGGCCC GAC UAUCUCGA |
| 824 | GGGAUUGG AGAA GGGGAG ACCAGAGAAACACACGUUGUGGUACAUUACCUGGUA | CUCCCCU GCC CCAAUCCC |
| 1042 | GGGAUCAA AGAA GUAGGC ACCAGAGAAACACACGUUGUGGUACAUUACCUGGUA | GCCUACA GCU UUGAUCCC |
| 1168 | CUGGAAAC AGAA GGAGAG ACCAGAGAAACACACGUUGUGGUACAUUACCUGGUA | CUCUCCA GAU GUUUCCAG |
| 1178 | UCAAGGAA AGAA GGAAAC ACCAGAGAAACACACGUUGUGGUACAUUACCUGGUA | GUUUCCA GAC UUCCUUGA |
| 1202 | AUGGGGAG AGAA GGGCUC ACCAGAGAAACACACGUUGUGGUACAUUACCUGGUA | GAGCCCA GCC CUCCCCAU |
| 1220 | AUAGAGGG AGAA GGCUCC ACCAGAGAAACACACGUUGUGGUACAUUACCUGGUA | GGAGCCA GCU CCCUCUAU |
| 1284 | AUACAUUC AGAA GUAAAU ACCAGAGAAACACACGUUGUGGUACAUUACCUGGUA | AUUUACA GAU GAAUGUAU |
| 1340 | UGAGCCAA AGAA GCUCCU ACCAGAGAAACACACGUUGUGGUACAUUACCUGGUA | AGGAGCU GCC UUGGCUCA |
| 1390 | UACAUGGG AGAA GCCUAU ACCAGAGAAACACACGUUGUGGUACAUUACCUGGUA | AUAGGCU GUU CCCAUGUA |

| | | | | | | | |
|---|---|---|---|---|---|---|---|
| 1452 | ACAACUUA | AGAA | GAUAAU | ACCAGAGAAACACACGUUGUGGUACAUUACCUGGUA | AUUAUCU | GAU | UAAGUUGU |
| 1475 | GUCACCAA | AGAA | GCAUUG | ACCAGAGAAACACACGUUGUGGUACAUUACCUGGUA | CAAUGCU | GAU | UUGGUGAC |
| 1513 | CCCUGGGG | AGAA | GAGGCC | ACCAGAGAAACACACGUUGUGGUACAUUACCUGGUA | GGCCUCU | GCU | CCCCAGGG |
| 1541 | GAAUAGUA | AGAA | GAUUAC | ACCAGAGAAACACACGUUGUGGUACAUUACCUGGUA | GUAAUCG | GCC | UACUAUUC |

**Table 28: Mouse TNF-α Hairpin Ribozyme Sequences**

| nt. Position | Hairpin Ribozyme Sequence | Substrate |
|---|---|---|
| 103 | GUGAAAGG AGAA GAACCU ACCAGAGAAACACACGUUGUGGUACAUUACCUGGUA | AGGUUCU GUC CCUUUCAC |
| 256 | UGAGAAGA AGAA GAGACA ACCAGAGAAACACACGUUGUGGUACAUUACCUGGUA | UGUCUCA GCC UCUUCUCA |
| 272 | CUGCCACA AGAA GGAAUG ACCAGAGAAACACACGUUGUGGUACAUUACCUGGUA | CAUUCCU GCU UGUGGCAG |
| 301 | GUUCAGUA AGAA GAAGAG ACCAGAGAAACACACGUUGUGGUACAUUACCUGGUA | CUCUUCU GUC UACUGAAC |
| 325 | CCUUUGGG AGAA GAUCAC ACCAGAGAAACACACGUUGUGGUACAUUACCUGGUA | GUGAUCG GUC CCCAAAGG |
| 370 | GGCCAUAG AGAA GAUGAG ACCAGAGAAACACACGUUGUGGUACAUUACCUGGUA | CUCAUCA GUU CUAUGGCC |
| 383 | GUGUGAGG AGAA GGGCCA ACCAGAGAAACACACGUUGUGGUACAUUACCUGGUA | UGGCCCA GAC CCUCACAC |
| 397 | AGAAGAUG AGAA GAGUGU ACCAGAGAAACACACGUUGUGGUACAUUACCUGGUA | ACACUCA GAU CAUCUUCU |
| 467 | GCCACUCC AGAA GCUCCU ACCAGAGAAACACACGUUGUGGUACAUUACCUGGUA | AGGAGCA GCU GGAGUGGC |
| 546 | AACCCAUC AGAA GGCACC ACCAGAGAAACACACGUUGUGGUACAUUACCUGGUA | GGUGCCA GCC GAUGGGUU |
| 549 | UACAACCC AGAA GCUGGC ACCAGAGAAACACACGUUGUGGUACAUUACCUGGUA | GCCAGCC GAU GGGUUGUA |
| 598 | GUAGUCGG AGAA GCCUUG ACCAGAGAAACACACGUUGUGGUACAUUACCUGGUA | CAAGGCU GCC CCGACUAC |
| 603 | AGCACGUA AGAA GGGCAG ACCAGAGAAACACACGUUGUGGUACAUUACCUGGUA | CUGCCCC GAC UACGUGCU |
| 631 | AGCAAAUC AGAA GACGGU ACCAGAGAAACACACGUUGUGGUACAUUACCUGGUA | ACCGUCA GCC GAUUUGCU |
| 634 | GAUAGCAA AGAA GCUGAC ACCAGAGAAACACACGUUGUGGUACAUUACCUGGUA | GUCAGCC GAU UUGCUAUC |
| 675 | CUCUUGAC AGAA GAGAGG ACCAGAGAAACACACGUUGUGGUACAUUACCUGGUA | CCUCUCU GCC GUCAAGAG |
| 691 | GUCCUUGG AGAA GGGGCU ACCAGAGAAACACACGUUGUGGUACAUUACCUGGUA | AGCCCCU GCC CCAAGGAC |
| 764 | CCUUCUCC AGAA GGAAGA ACCAGAGAAACACACGUUGUGGUACAUUACCUGGUA | UCUUCCA GCU GGAGAAGG |
| 803 | AGUACUUG AGAA GAUUGA ACCAGAGAAACACACGUUGUGGUACAUUACCUGGUA | UCAAUCU GCC CAAGUACU |
| 895 | AGAGUGGG AGAA GGGUAG ACCAGAGAAACACACGUUGUGGUACAUUACCUGGUA | CUACCCA GCC CCCACUCU |
| 906 | GUAAAGGG AGAA GAGUGG ACCAGAGAAACACACGUUGUGGUACAUUACCUGGUA | CCACUCU GAC CCCUUUAC |
| 920 | AUAAAGGG AGAA GAGUAA ACCAGAGAAACACACGUUGUGGUACAUUACCUGGUA | UUACUCU GAC CCCUUUAU |
| 953 | AGGACACA AGAA GGGGGC ACCAGAGAAACACACGUUGUGGUACAUUACCUGGUA | GCCCCCA GUC UGUGUCCU |
| 1175 | CAUUCUGA AGAA GAGGCA ACCAGAGAAACACACGUUGUGGUACAUUACCUGGUA | UGCCUCU GUC UCAGAAUG |
| 1220 | CUGGAAAG AGAA GAAGGU ACCAGAGAAACACACGUUGUGGUACAUUACCUGGUA | ACCUUCA GAC CUUUCCAG |
| 1230 | AGGGAAGA AGAA GGAAAG ACCAGAGAAACACACGUUGUGGUACAUUACCUGGUA | CUUUCCA GAC UCUUCCCU |
| 1256 | GUGAGGAA AGAA GUGCAU ACCAGAGAAACACACGUUGUGGUACAUUACCUGGUA | AUGCACA GCC UUCCUCAC |
| 1274 | UAGAGGGG AGAA GGCUCU ACCAGAGAAACACACGUUGUGGUACAUUACCUGGUA | AGAGCCA GCC CCCCUCUA |

| | | | | | | | |
|---|---|---|---|---|---|---|---|
| 1393 | UGUCUGAA | AGAA | GCUUCC | ACCAGAGAAACACACGUUGUGGUACAUUACCUGGUA | GGAAGCU | GUC | UUCAGACA |
| 1435 | CAGGUGGG | AGAA | GCUCAG | ACCAGAGAAACACACGUUGUGGUACAUUACCUGGUA | CUGAGCU | GUC | CCCACCUG |
| 1525 | GUCACCAA | AGAA | GCGUUA | ACCAGAGAAACACACGUUGUGGUACAUUACCUGGUA | UAACGCU | GAU | UUGGUGAC |
| 1542 | GAUGUAGC | AGAA | GCCUGG | ACCAGAGAAACACACGUUGUGGUACAUUACCUGGUA | CCAGGCU | GUC | GCUACAUC |
| 1564 | CCGUGGGG | AGAA | GAGGUU | ACCAGAGAAACACACGUUGUGGUACAUUACCUGGUA | AACCUCU | GCU | CCCCACGG |

Table 29: Human *bcr/abl* HH Target Sequence

| Sequence ID No. | HH Target Sequence |
|---|---|
| ***b2-a2 Junction*** | |
| 20 | UGACCAUCA AUA AGGAAGAAGCC |
| 21 | GAAGAAGCC CUU CAGCGGCCAGU |
| 22 | AAGAAGCCC UUC AGCGGCCAGUA |
| ***b3-a2 Junction*** | |
| 23 | UAAGCAGAG UUC AAAAGCCCUUC |
| 24 | UCAAAAGCC CUU CAGCGGCCAGU |
| 25 | CAAAAGCCC UUC AGCGGCCAGUA |

Table 30: Human *bcr-abl* HH Ribozyme Sequences

| Sequence ID No. | HH Ribozyme Sequence |
|---|---|
| 26 | GGCUUCUUCCU CUGAUGAGGCCGAAAGGCCGAA AUUGAUGGUCA |
| 27 | ACUGGCCGCUG CUGAUGAGGCCGAAAGGCCGAA AGGGCUUCUUC |
| 28 | UACUGGCCGCU CUGAUGAGGCCGAAAGGCCGAA AAGGGCUUCUU |
| 29 | GAAGGGCUUUU CUGAUGAGGCCGAAAGGCCGAA AACUCUGCUUA |
| 30 | ACUGGCCGCUG CUGAUGAGGCCGAAAGGCCGAA AGGGCUUUUGA |
| 31 | UACUGGCCGCU CUGAUGAGGCCGAAAGGCCGAA AAGGGCUUUUG |

Table 31: RSV (1B) HH Target Sequence

| nt. Position | HH Target Sequence | nt. Position | HH Target Sequence |
|---|---|---|---|
| 10 | GGCAAAU A AAUCAAU | 276 | AAAAUAU A CUGAAUA |
| 14 | AAUAAAU C AAUUCAG | 283 | ACUGAAU A CAACACA |
| 18 | AAUCAAU U CAGCCAA | 295 | ACAAAAU A UGGCACU |
| 19 | AUCAAUU C AGCCAAC | 303 | UGGCACU U UCCCUAU |
| 54 | CAAUGAU A AUACACC | 304 | GGCACUU U CCCUAUG |
| 57 | UGAUAAU A CACCACA | 305 | GCACUUU C CCUAUGC |
| 77 | UGAUGAU C ACAGACA | 309 | UUUCCCU A UGCCAAU |
| 94 | AGACCGU U GUCACUU | 317 | UGCCAAU A UUCAUCA |
| 97 | CCGUUGU C ACUUGAG | 319 | CCAAUAU U CAUCAAU |
| 101 | UGUCACU U GAGACCA | 320 | CAAUAUU C AUCAAUC |
| 110 | AGACCAU A AUAACAU | 323 | UAUUCAU C AAUCAUG |
| 113 | CCAUAAU A ACAUCAC | 327 | CAUCAAU C AUGAUGG |
| 118 | AUAACAU C ACUAACC | 337 | GAUGGGU U CUUAGAA |
| 122 | CAUCACU A ACCAGAG | 338 | AUGGGUU C UUAGAAU |
| 134 | GAGACAU C AUAACAC | 340 | GGGUUCU U AGAAUGC |
| 137 | ACAUCAU A ACACACA | 341 | GGUUCUU A GAAUGCA |
| 148 | CACAAAU U UAUAUAC | 350 | AAUGCAU U GGCAUUA |
| 149 | ACAAAUU U AUAUACU | 356 | UUGGCAU U AAGCCUA |
| 150 | CAAAUUU A UAUACUU | 357 | UGGCAUU A AGCCUAC |
| 152 | AAUUUAU A UACUUGA | 363 | UAAGCCU A CAAAGCA |
| 154 | UUUAUAU A CUUGAUA | 372 | AAAGCAU A CUCCCAU |
| 157 | AUAUACU U GAUAAAU | 375 | GCAUACU C CCAUAAU |
| 161 | ACUUGAU A AAUCAUG | 380 | CUCCCAU A AUAUACA |
| 165 | GAUAAAU C AUGAAUG | 383 | CCAUAAU A UACAAGU |
| 176 | AAUGCAU A GUGAGAA | 385 | AUAAUAU A CAAGUAU |
| 188 | GAAAACU U GAUGAAA | 391 | UACAAGU A UGAUCUC |
| 208 | GCCACAU U UACAUUC | 396 | GUAUGAU C UCAAUCC |
| 209 | CCACAUU U ACAUUCC | 398 | AUGAUCU C AAUCCAU |
| 210 | CACAUUU A CAUUCCU | 402 | UCUCAAU C CAUAAAU |
| 214 | UUUACAU U CCUGGUC | 406 | AAUCCAU A AAUUUCA |
| 215 | UUACAUU C CUGGUCA | 410 | CAUAAAU U UCAACAC |
| 221 | UCCUGGU C AACUAUG | 411 | AUAAAUU U CAACACA |
| 226 | GUCAACU A UGAAAUG | 412 | UAAAUUU C AACACAA |
| 239 | UGAAACU A UUACACA | 421 | ACACAAU A UUCACAC |
| 241 | AAACUAU U ACACAAA | 423 | ACAAUAU U CACACAA |
| 242 | AACUAUU A CACAAAG | 424 | CAAUAUU C ACACAAU |
| 251 | ACAAAGU A GGAAGCA | 432 | ACACAAU C UAAAACA |
| 261 | AAGCACU A AAUAUAA | 434 | ACAAUCU A AAACAAC |
| 265 | ACUAAAU A UAAAAAA | 446 | AACAACU C UAUGCAU |
| 267 | UAAAUAU A AAAAAUA | 448 | CAACUCU A UGCAUAA |
| 274 | AAAAAAU A UACUGAA | 454 | UAUGCAU A ACUAUAC |

| | |
|---|---|
| 458 | CAUAACU A UACUCCA |
| 460 | UAACUAU A CUCCAUA |
| 463 | CUAUACU C CAUAGUC |
| 467 | ACUCCAU A GUCCAGA |
| 470 | CCAUAGU C CAGAUGG |
| 489 | UGAAAAU U AUAGUAA |
| 490 | GAAAAUU A UAGUAAU |
| 492 | AAAUUAU A GUAAUUU |
| 495 | UUAUAGU A AUUUAAA |

## Table 32: RSV (1B) HH Ribozyme Sequence

| nt. Position | HH Ribozyme Sequence |
|---|---|
| 10 | AUUGAUU CUGAUGAGGCCGAAAGGCCGAA AUUUGCC |
| 14 | CUGAAUU CUGAUGAGGCCGAAAGGCCGAA AUUUAUU |
| 18 | UUGGCUG CUGAUGAGGCCGAAAGGCCGAA AUUGAUU |
| 19 | GUUGGCU CUGAUGAGGCCGAAAGGCCGAA AAUUGAU |
| 54 | GGUGUAU CUGAUGAGGCCGAAAGGCCGAA AUCAUUG |
| 57 | UGUGGUG CUGAUGAGGCCGAAAGGCCGAA AUUAUCA |
| 77 | UGUCUGU CUGAUGAGGCCGAAAGGCCGAA AUCAUCA |
| 94 | AAGUGAC CUGAUGAGGCCGAAAGGCCGAA ACGGUCU |
| 97 | CUCAAGU CUGAUGAGGCCGAAAGGCCGAA ACAACGG |
| 101 | UGGUCUC CUGAUGAGGCCGAAAGGCCGAA AGUGACA |
| 110 | AUGUUAU CUGAUGAGGCCGAAAGGCCGAA AUGGUCU |
| 113 | GUGAUGU CUGAUGAGGCCGAAAGGCCGAA AUUAUGG |
| 118 | GGUUAGU CUGAUGAGGCCGAAAGGCCGAA AUGUUAU |
| 122 | CUCUGGU CUGAUGAGGCCGAAAGGCCGAA AGUGAUG |
| 134 | GUGUUAU CUGAUGAGGCCGAAAGGCCGAA AUGUCUC |
| 137 | UGUGUGU CUGAUGAGGCCGAAAGGCCGAA AUGAUGU |
| 148 | GUAUAUA CUGAUGAGGCCGAAAGGCCGAA AUUUGUG |
| 149 | AGUAUAU CUGAUGAGGCCGAAAGGCCGAA AAUUUGU |
| 150 | AAGUAUA CUGAUGAGGCCGAAAGGCCGAA AAAUUUG |
| 152 | UCAAGUA CUGAUGAGGCCGAAAGGCCGAA AUAAAUU |
| 154 | UAUCAAG CUGAUGAGGCCGAAAGGCCGAA AUAUAAA |
| 157 | AUUUAUC CUGAUGAGGCCGAAAGGCCGAA AGUAUAU |
| 161 | CAUGAUU CUGAUGAGGCCGAAAGGCCGAA AUCAAGU |
| 165 | CAUUCAU CUGAUGAGGCCGAAAGGCCGAA AUUUAUC |
| 176 | UUCUCAC CUGAUGAGGCCGAAAGGCCGAA AUGCAUU |
| 188 | UUUCAUC CUGAUGAGGCCGAAAGGCCGAA AGUUUUC |
| 208 | GAAUGUA CUGAUGAGGCCGAAAGGCCGAA AUGUGGC |
| 209 | GGAAUGU CUGAUGAGGCCGAAAGGCCGAA AAUGUGG |
| 210 | AGGAAUG CUGAUGAGGCCGAAAGGCCGAA AAAUGUG |
| 214 | GACCAGG CUGAUGAGGCCGAAAGGCCGAA AUGUAAA |
| 215 | UGACCAG CUGAUGAGGCCGAAAGGCCGAA AAUGUAA |
| 221 | CAUAGUU CUGAUGAGGCCGAAAGGCCGAA ACCAGGA |
| 226 | CAUUUCA CUGAUGAGGCCGAAAGGCCGAA AGUUGAC |
| 239 | UGUGUAA CUGAUGAGGCCGAAAGGCCGAA AGUUUCA |
| 241 | UUUGUGU CUGAUGAGGCCGAAAGGCCGAA AUAGUUU |
| 242 | CUUUGUG CUGAUGAGGCCGAAAGGCCGAA AAUAGUU |
| 251 | UGCUUCC CUGAUGAGGCCGAAAGGCCGAA ACUUUGU |
| 261 | UUAUAUU CUGAUGAGGCCGAAAGGCCGAA AGUGCUU |
| 265 | UUUUUUA CUGAUGAGGCCGAAAGGCCGAA AUUUAGU |
| 267 | UAUUUUU CUGAUGAGGCCGAAAGGCCGAA AUAUUUA |
| 274 | UUCAGUA CUGAUGAGGCCGAAAGGCCGAA AUUUUUU |
| 276 | UAUUCAG CUGAUGAGGCCGAAAGGCCGAA AUAUUUU |

| | |
|---|---|
| 283 | UGUGUUG CUGAUGAGGCCGAAAGGCCGAA AUUCAGU |
| 295 | AGUGCCA CUGAUGAGGCCGAAAGGCCGAA AUUUUGU |
| 303 | AUAGGGA CUGAUGAGGCCGAAAGGCCGAA AGUGCCA |
| 304 | CAUAGGG CUGAUGAGGCCGAAAGGCCGAA AAGUGCC |
| 305 | GCAUAGG CUGAUGAGGCCGAAAGGCCGAA AAAGUGC |
| 309 | AUUGGCA CUGAUGAGGCCGAAAGGCCGAA AGGGAAA |
| 317 | UGAUGAA CUGAUGAGGCCGAAAGGCCGAA AUUGGCA |
| 319 | AUUGAUG CUGAUGAGGCCGAAAGGCCGAA AUAUUGG |
| 320 | GAUUGAU CUGAUGAGGCCGAAAGGCCGAA AAUAUUG |
| 323 | CAUGAUU CUGAUGAGGCCGAAAGGCCGAA AUGAAUA |
| 327 | CCAUCAU CUGAUGAGGCCGAAAGGCCGAA AUUGAUG |
| 337 | UUCUAAG CUGAUGAGGCCGAAAGGCCGAA ACCCAUC |
| 338 | AUUCUAA CUGAUGAGGCCGAAAGGCCGAA AACCCAU |
| 340 | GCAUUCU CUGAUGAGGCCGAAAGGCCGAA AGAACCC |
| 341 | UGCAUUC CUGAUGAGGCCGAAAGGCCGAA AAGAACC |
| 350 | UAAUGCC CUGAUGAGGCCGAAAGGCCGAA AUGCAUU |
| 356 | UAGGCUU CUGAUGAGGCCGAAAGGCCGAA AUGCCAA |
| 357 | GUAGGCU CUGAUGAGGCCGAAAGGCCGAA AAUGCCA |
| 363 | UGCUUUG CUGAUGAGGCCGAAAGGCCGAA AGGCUUA |
| 372 | AUGGGAG CUGAUGAGGCCGAAAGGCCGAA AUGCUUU |
| 375 | AUUAUGG CUGAUGAGGCCGAAAGGCCGAA AGUAUGC |
| 380 | UGUAUAU CUGAUGAGGCCGAAAGGCCGAA AUGGGAG |
| 383 | ACUUGUA CUGAUGAGGCCGAAAGGCCGAA AUUAUGG |
| 385 | AUACUUG CUGAUGAGGCCGAAAGGCCGAA AUAUUAU |
| 391 | GAGAUCA CUGAUGAGGCCGAAAGGCCGAA ACUUGUA |
| 396 | GGAUUGA CUGAUGAGGCCGAAAGGCCGAA AUCAUAC |
| 398 | AUGGAUU CUGAUGAGGCCGAAAGGCCGAA AGAUCAU |
| 402 | AUUUAUG CUGAUGAGGCCGAAAGGCCGAA AUUGAGA |
| 406 | UGAAAUU CUGAUGAGGCCGAAAGGCCGAA AUGGAUU |
| 410 | GUGUUGA CUGAUGAGGCCGAAAGGCCGAA AUUUAUG |
| 411 | UGUGUUG CUGAUGAGGCCGAAAGGCCGAA AAUUUAU |
| 412 | UUGUGUU CUGAUGAGGCCGAAAGGCCGAA AAAUUUA |
| 421 | GUGUGAA CUGAUGAGGCCGAAAGGCCGAA AUUGUGU |
| 423 | UUGUGUG CUGAUGAGGCCGAAAGGCCGAA AUAUUGU |
| 424 | AUUGUGU CUGAUGAGGCCGAAAGGCCGAA AAUAUUG |
| 432 | UGUUUUA CUGAUGAGGCCGAAAGGCCGAA AUUGUGU |
| 434 | GUUGUUU CUGAUGAGGCCGAAAGGCCGAA AGAUUGU |
| 446 | AUGCAUA CUGAUGAGGCCGAAAGGCCGAA AGUUGUU |
| 448 | UUAUGCA CUGAUGAGGCCGAAAGGCCGAA AGAGUUG |
| 454 | GUAUAGU CUGAUGAGGCCGAAAGGCCGAA AUGCAUA |
| 458 | UGGAGUA CUGAUGAGGCCGAAAGGCCGAA AGUUAUG |
| 460 | UAUGGAG CUGAUGAGGCCGAAAGGCCGAA AUAGUUA |
| 463 | GACUAUG CUGAUGAGGCCGAAAGGCCGAA AGUAUAG |
| 467 | UCUGGAC CUGAUGAGGCCGAAAGGCCGAA AUGGAGU |
| 470 | CCAUCUG CUGAUGAGGCCGAAAGGCCGAA ACUAUGG |
| 489 | UUACUAU CUGAUGAGGCCGAAAGGCCGAA AUUUUCA |
| 490 | AUUACUA CUGAUGAGGCCGAAAGGCCGAA AAUUUUC |
| 492 | AAAUUAC CUGAUGAGGCCGAAAGGCCGAA AUAAUUU |
| 495 | UUUAAAU CUGAUGAGGCCGAAAGGCCGAA ACUAUAA |

Table 33 : RSV (1C) HH target Sequence

| nt. Position | Target Sequence | nt. Position | Target Sequence |
|---|---|---|---|
| 10 | GGCAAAU A AGAAUUU | 165 | UACAUUU A ACUAACG |
| 16 | UAAGAAU U UGAUAAG | 169 | UUUAACU A ACGCUUU |
| 17 | AAGAAUU U GAUAAGU | 175 | UAACGCU U UGGCUAA |
| 21 | AUUUGAU A AGUACCA | 176 | AACGCUU U GGCUAAG |
| 25 | GAUAAGU A CCACUUA | 181 | UUUGGCU A AGGCAGU |
| 31 | UACCACU U AAAUUUA | 192 | CAGUGAU A CAUACAA |
| 32 | ACCACUU A AAUUUAA | 196 | GAUACAU A CAAUCAA |
| 36 | CUUAAAU U UAACUCC | 201 | AUACAAU C AAAUUGA |
| 37 | UUAAAUU U AACUCCC | 206 | AUCAAAU U GAAUGGC |
| 38. | UAAAUUU A ACUCCCU | 216 | AUGGCAU U GUGUUUG |
| 42 | UUUAACU C CCUUGGU | 221 | AUUGUGU U UGUGCAU |
| 46 | ACUCCCU U GGUUAGA | 222 | UUGUGUU U GUGCAUG |
| 50 | CCUUGGU U AGAGAUG | 231 | UGCAUGU U AUUACAA |
| 51 | CUUGGUU A GAGAUGG | 232 | GCAUGUU A UUACAAG |
| 67 | CAGCAAU U CAUUGAG | 234 | AUGUUAU U ACAAGUA |
| 68 | AGCAAUU C AUUGAGU | 235 | UGUUAUU A CAAGUAG |
| 71 | AAUUCAU U GAGUAUG | 241 | UACAAGU A GUGAUAU |
| 76 | AUUGAGU A UGAUAAA | 247 | UAGUGAU A UUUGCCC |
| 81 | GUAUGAU A AAAGUUA | 249 | GUGAUAU U UGCCCUA |
| 87 | UAAAAGU U AGAUUAC | 250 | UGAUAUU U GCCCUAA |
| 88 | AAAAGUU A GAUUACA | 256 | UUGCCCU A AUAAUAA |
| 92 | GUUAGAU U ACAAAAU | 259 | CCCUAAU A AUAAUAU |
| 93 | UUAGAUU A CAAAAUU | 262 | UAAUAAU A AUAUUGU |
| 100 | ACAAAAU U UGUUUGA | 265 | UAAUAAU A UUGUAGU |
| 101 | CAAAAUU U GUUUGAC | 267 | AUAAUAU U GUAGUAA |
| 104 | AAUUUGU U UGACAAU | 270 | AUAUUGU A GUAAAAU |
| 105 | AUUUGUU U GACAAUG | 273 | UUGUAGU A AAAUCCA |
| 120 | AUGAAGU A GCAUUGU | 278 | GUAAAAU C CAAUUUC |
| 125 | GUAGCAU U GUUAAAA | 283 | AUCCAAU U UCACAAC |
| 128 | GCAUUGU U AAAAAUA | 284 | UCCAAUU U CACAACA |
| 129 | CAUUGUU A AAAAUAA | 285 | CCAAUUU C ACAACAA |
| 135 | UAAAAAU A ACAUGCU | 300 | UGCCAGU A CUACAAA |
| 143 | ACAUGCU A UACUGAU | 303 | CAGUACU A CAAAAUG |
| 145 | AUGCUAU A CUGAUAA | 316 | UGGAGGU U AUAUAUG |
| 151 | UACUGAU A AAUUAAU | 317 | GGAGGUU A UAUAUGG |
| 155 | GAUAAAU U AAUACAU | 319 | AGGUUAU A UAUGGGA |
| 156 | AUAAAUU A AUACAUU | 321 | GUUAUAU A UGGGAAA |
| 159 | AAUUAAU A CAUUUAA | 338 | AUGGAAU U AACACAU |
| 163 | AAUACAU U UAACUAA | 339 | UGGAAUU A ACACAUU |
| 164 | AUACAUU U AACUAAC | 346 | AACACAU U GCUCUCA |

| | | | |
|---|---|---|---|
| 350 | CAUUGCU | C | UCAACCU |
| 352 | UUGCUCU | C | AACCUAA |
| 358 | UCAACCU | A | AUGGUCU |
| 364 | UAAUGGU | C | UACUAGA |
| 366 | AUGGUCU | A | CUAGAUG |
| 369 | GUCUACU | A | GAUGACA |
| 379 | UGACAAU | U | GUGAAAU |
| 387 | GUGAAAU | U | AAAUUCU |
| 388 | UGAAAUU | A | AAUUCUC |
| 392 | AUUAAAU | U | CUCCAAA |
| 393 | UUAAAUU | C | UCCAAAA |
| 395 | AAAUUCU | C | CAAAAAA |
| 405 | AAAAACU | A | AGUGAUU |
| 412 | AAGUGAU | U | CAACAAU |
| 413 | AGUGAUU | C | AACAAUG |
| 427 | GACCAAU | U | AUAUGAA |
| 428 | ACCAAUU | A | UAUGAAU |
| 430 | CAAUUAU | A | UGAAUCA |
| 436 | UAUGAAU | C | AAUUAUC |
| 440 | AAUCAAU | U | AUCUGAA |
| 441 | AUCAAUU | A | UCUGAAU |
| 443 | CAAUUAU | C | UGAAUUA |
| 449 | UCUGAAU | U | ACUUGGA |
| 450 | CUGAAUU | A | CUUGGAU |
| 453 | AAUUACU | U | GGAUUUG |
| 458 | CUUGGAU | U | UGAUCUU |
| 459 | UUGGAUU | U | GAUCUUA |
| 463 | AUUUGAU | C | UUAAUCC |
| 465 | UUGAUCU | U | AAUCCAU |
| 466 | UGAUCUU | A | AUCCAUA |
| 469 | UCUUAAU | C | CAUAAAU |
| 473 | AAUCCAU | A | AAUUAUA |
| 477 | CAUAAAU | U | AUAAUUA |
| 478 | AUAAAUU | A | UAAUUAA |
| 480 | AAAUUAU | A | AUUAAUA |
| 483 | UUAUAAU | U | AAUAUCA |
| 484 | UAUAAUU | A | AUAUCAA |
| 487 | AAUUAAU | A | UCAACUA |
| 489 | UUAAUAU | C | AACUAGC |
| 494 | AUCAACU | A | GCAAAUC |
| 501 | AGCAAAU | C | AAUGUCA |
| 507 | UCAAUGU | C | ACUAACA |
| 511 | UGUCACU | A | ACACCAU |
| 519 | ACACCAU | U | AGUUAAU |
| 520 | CACCAUU | A | GUUAAUA |
| 523 | CAUUAGU | U | AAUAUAA |
| 524 | AUUAGUU | A | AUAUAAA |

## Table 34: RSV (1C) HH Ribozyme Sequence

| nt. Position | HH Ribozyme Sequence |
|---|---|
| 10 | AAAUUCU CUGAUGAGGCCGAAAGGCCGAA AUUUGCC |
| 16 | CUUAUCA CUGAUGAGGCCGAAAGGCCGAA AUUCUUA |
| 17 | ACUUAUC CUGAUGAGGCCGAAAGGCCGAA AAUUCUU |
| 21 | UGGUACU CUGAUGAGGCCGAAAGGCCGAA AUCAAAU |
| 25 | UAAGUGG CUGAUGAGGCCGAAAGGCCGAA ACUUAUC |
| 31 | UAAAUUU CUGAUGAGGCCGAAAGGCCGAA AGUGGUA |
| 32 | UUAAAUU CUGAUGAGGCCGAAAGGCCGAA AAGUGGU |
| 36 | GGAGUUA CUGAUGAGGCCGAAAGGCCGAA AUUUAAG |
| 37 | GGGAGUU CUGAUGAGGCCGAAAGGCCGAA AAUUUAA |
| 38 | AGGGAGU CUGAUGAGGCCGAAAGGCCGAA AAAUUUA |
| 42 | ACCAAGG CUGAUGAGGCCGAAAGGCCGAA AGUUAAA |
| 46 | UCUAACC CUGAUGAGGCCGAAAGGCCGAA AGGGAGU |
| 50 | CAUCUCU CUGAUGAGGCCGAAAGGCCGAA ACCAAGG |
| 51 | CCAUCUC CUGAUGAGGCCGAAAGGCCGAA AACCAAG |
| 67 | CUCAAUG CUGAUGAGGCCGAAAGGCCGAA AUUGCUG |
| 68 | ACUCAAU CUGAUGAGGCCGAAAGGCCGAA AAUUGCU |
| 71 | CAUACUC CUGAUGAGGCCGAAAGGCCGAA AUGAAUU |
| 76 | UUUAUCA CUGAUGAGGCCGAAAGGCCGAA ACUCAAU |
| 81 | UAACUUU CUGAUGAGGCCGAAAGGCCGAA AUCAUAC |
| 87 | GUAAUCU CUGAUGAGGCCGAAAGGCCGAA ACUUUUA |
| 88 | UGUAAUC CUGAUGAGGCCGAAAGGCCGAA AACUUUU |
| 92 | AUUUUGU CUGAUGAGGCCGAAAGGCCGAA AUCUAAC |
| 93 | AAUUUUG CUGAUGAGGCCGAAAGGCCGAA AAUCUAA |
| 100 | UCAAACA CUGAUGAGGCCGAAAGGCCGAA AUUUUGU |
| 101 | GUCAAAC CUGAUGAGGCCGAAAGGCCGAA AAUUUUG |
| 104 | AUUGUCA CUGAUGAGGCCGAAAGGCCGAA ACAAAUU |
| 105 | CAUUGUC CUGAUGAGGCCGAAAGGCCGAA AACAAAU |
| 120 | ACAAUGC CUGAUGAGGCCGAAAGGCCGAA ACUUCAU |
| 125 | UUUUAAC CUGAUGAGGCCGAAAGGCCGAA AUGCUAC |
| 128 | UAUUUUU CUGAUGAGGCCGAAAGGCCGAA ACAAUGC |
| 129 | UUAUUUU CUGAUGAGGCCGAAAGGCCGAA AACAAUG |
| 135 | AGCAUGU CUGAUGAGGCCGAAAGGCCGAA AUUUUUA |
| 143 | AUCAGUA CUGAUGAGGCCGAAAGGCCGAA AGCAUGU |
| 145 | UUAUCAG CUGAUGAGGCCGAAAGGCCGAA AUAGCAU |
| 151 | AUUAAUU CUGAUGAGGCCGAAAGGCCGAA AUCAGUA |
| 155 | AUGUAUU CUGAUGAGGCCGAAAGGCCGAA AUUUAUC |
| 156 | AAUGUAU CUGAUGAGGCCGAAAGGCCGAA AAUUUAU |
| 159 | UUAAAUG CUGAUGAGGCCGAAAGGCCGAA AUUAAUU |
| 163 | UUAGUUA CUGAUGAGGCCGAAAGGCCGAA AUGUAUU |
| 164 | GUUAGUU CUGAUGAGGCCGAAAGGCCGAA AAUGUAU |
| 165 | CGUUAGU CUGAUGAGGCCGAAAGGCCGAA AAAUGUA |

| | | | |
|---|---|---|---|
| 169 | AAAGCGU | CUGAUGAGGCCGAAAGGCCGAA | AGUUAAA |
| 175 | UUAGCCA | CUGAUGAGGCCGAAAGGCCGAA | AGCGUUA |
| 176 | CUUAGCC | CUGAUGAGGCCGAAAGGCCGAA | AAGCGUU |
| 181 | ACUGCCU | CUGAUGAGGCCGAAAGGCCGAA | AGCCAAA |
| 192 | UUGUAUG | CUGAUGAGGCCGAAAGGCCGAA | AUCACUG |
| 196 | UUGAUUG | CUGAUGAGGCCGAAAGGCCGAA | AUGUAUC |
| 201 | UCAAUUU | CUGAUGAGGCCGAAAGGCCGAA | AUUGUAU |
| 206 | GCCAUUC | CUGAUGAGGCCGAAAGGCCGAA | AUUUGAU |
| 216 | CAAACAC | CUGAUGAGGCCGAAAGGCCGAA | AUGCCAU |
| 221 | AUGCACA | CUGAUGAGGCCGAAAGGCCGAA | ACACAAU |
| 222 | CAUGCAC | CUGAUGAGGCCGAAAGGCCGAA | AACACAA |
| 231 | UUGUAAU | CUGAUGAGGCCGAAAGGCCGAA | ACAUGCA |
| 232 | CUUGUAA | CUGAUGAGGCCGAAAGGCCGAA | AACAUGC |
| 234 | UACUUGU | CUGAUGAGGCCGAAAGGCCGAA | AUAACAU |
| 235 | CUACUUG | CUGAUGAGGCCGAAAGGCCGAA | AAUAACA |
| 241 | AUAUCAC | CUGAUGAGGCCGAAAGGCCGAA | ACUUGUA |
| 247 | GGGCAAA | CUGAUGAGGCCGAAAGGCCGAA | AUCACUA |
| 249 | UAGGGCA | CUGAUGAGGCCGAAAGGCCGAA | AUAUCAC |
| 250 | UUAGGGC | CUGAUGAGGCCGAAAGGCCGAA | AAUAUCA |
| 256 | UUAUUAU | CUGAUGAGGCCGAAAGGCCGAA | AGGGCAA |
| 259 | AUAUUAU | CUGAUGAGGCCGAAAGGCCGAA | AUUAGGG |
| 262 | ACAAUAU | CUGAUGAGGCCGAAAGGCCGAA | AUUAUUA |
| 265 | ACUACAA | CUGAUGAGGCCGAAAGGCCGAA | AUUAUUA |
| 267 | UUACUAC | CUGAUGAGGCCGAAAGGCCGAA | AUAUUAU |
| 270 | AUUUUAC | CUGAUGAGGCCGAAAGGCCGAA | ACAAUAU |
| 273 | UGGAUUU | CUGAUGAGGCCGAAAGGCCGAA | ACUACAA |
| 278 | GAAAUUG | CUGAUGAGGCCGAAAGGCCGAA | AUUUUAC |
| 283 | GUUGUGA | CUGAUGAGGCCGAAAGGCCGAA | AUUGGAU |
| 284 | UGUUGUG | CUGAUGAGGCCGAAAGGCCGAA | AAUUGGA |
| 285 | UUGUUGU | CUGAUGAGGCCGAAAGGCCGAA | AAAUUGG |
| 300 | UUUGUAG | CUGAUGAGGCCGAAAGGCCGAA | ACUGGCA |
| 303 | CAUUUUG | CUGAUGAGGCCGAAAGGCCGAA | AGUACUG |
| 316 | CAUAUAU | CUGAUGAGGCCGAAAGGCCGAA | ACCUCCA |
| 317 | CCAUAUA | CUGAUGAGGCCGAAAGGCCGAA | AACCUCC |
| 319 | UCCCAUA | CUGAUGAGGCCGAAAGGCCGAA | AUAACCU |
| 321 | UUUCCCA | CUGAUGAGGCCGAAAGGCCGAA | AUAUAAC |
| 338 | AUGUGUU | CUGAUGAGGCCGAAAGGCCGAA | AUUCCAU |
| 339 | AAUGUGU | CUGAUGAGGCCGAAAGGCCGAA | AAUUCCA |
| 346 | UGAGAGC | CUGAUGAGGCCGAAAGGCCGAA | AUGUGUU |
| 350 | AGGUUGA | CUGAUGAGGCCGAAAGGCCGAA | AGCAAUG |
| 352 | UUAGGUU | CUGAUGAGGCCGAAAGGCCGAA | AGAGCAA |
| 358 | AGACCAU | CUGAUGAGGCCGAAAGGCCGAA | AGGUUGA |
| 364 | UCUAGUA | CUGAUGAGGCCGAAAGGCCGAA | ACCAUUA |
| 366 | CAUCUAG | CUGAUGAGGCCGAAAGGCCGAA | AGACCAU |
| 369 | UGUCAUC | CUGAUGAGGCCGAAAGGCCGAA | AGUAGAC |
| 379 | AUUUCAC | CUGAUGAGGCCGAAAGGCCGAA | AUUGUCA |
| 387 | AGAAUUU | CUGAUGAGGCCGAAAGGCCGAA | AUUUCAC |
| 388 | GAGAAUU | CUGAUGAGGCCGAAAGGCCGAA | AAUUUCA |
| 392 | UUUGGAG | CUGAUGAGGCCGAAAGGCCGAA | AUUUAAU |

| | |
|---|---|
| 393 | UUUUGGA CUGAUGAGGCCGAAAGGCCGAA AAUUUAA |
| 395 | UUUUUUG CUGAUGAGGCCGAAAGGCCGAA AGAAUUU |
| 405 | AAUCACU CUGAUGAGGCCGAAAGGCCGAA AGUUUUU |
| 412 | AUUGUUG CUGAUGAGGCCGAAAGGCCGAA AUCACUU |
| 413 | CAUUGUU CUGAUGAGGCCGAAAGGCCGAA AAUCACU |
| 427 | UUCAUAU CUGAUGAGGCCGAAAGGCCGAA AUUGGUC |
| 428 | AUUCAUA CUGAUGAGGCCGAAAGGCCGAA AAUUGGU |
| 430 | UGAUUCA CUGAUGAGGCCGAAAGGCCGAA AUAAUUG |
| 436 | GAUAAUU CUGAUGAGGCCGAAAGGCCGAA AUUCAUA |
| 440 | UUCAGAU CUGAUGAGGCCGAAAGGCCGAA AUUGAUU |
| 441 | AUUCAGA CUGAUGAGGCCGAAAGGCCGAA AAUUGAU |
| 443 | UAAUUCA CUGAUGAGGCCGAAAGGCCGAA AUAAUUG |
| 449 | UCCAAGU CUGAUGAGGCCGAAAGGCCGAA AUUCAGA |
| 450 | AUCCAAG CUGAUGAGGCCGAAAGGCCGAA AAUUCAG |
| 453 | CAAAUCC CUGAUGAGGCCGAAAGGCCGAA AGUAAUU |
| 458 | AAGAUCA CUGAUGAGGCCGAAAGGCCGAA AUCCAAG |
| 459 | UAAGAUC CUGAUGAGGCCGAAAGGCCGAA AAUCCAA |
| 463 | GGAUUAA CUGAUGAGGCCGAAAGGCCGAA AUCAAAU |
| 465 | AUGGAUU CUGAUGAGGCCGAAAGGCCGAA AGAUCAA |
| 466 | UAUGGAU CUGAUGAGGCCGAAAGGCCGAA AAGAUCA |
| 469 | AUUUAUG CUGAUGAGGCCGAAAGGCCGAA AUUAAGA |
| 473 | UAUAAUU CUGAUGAGGCCGAAAGGCCGAA AUGGAUU |
| 477 | UAAUUAU CUGAUGAGGCCGAAAGGCCGAA AUUUAUG |
| 478 | UUAAUUA CUGAUGAGGCCGAAAGGCCGAA AAUUUAU |
| 480 | UAUUAAU CUGAUGAGGCCGAAAGGCCGAA AUAAUUU |
| 483 | UGAUAUU CUGAUGAGGCCGAAAGGCCGAA AUUAUAA |
| 484 | UUGAUAU CUGAUGAGGCCGAAAGGCCGAA AAUUAUA |
| 487 | UAGUUGA CUGAUGAGGCCGAAAGGCCGAA AUUAAUU |
| 489 | GCUAGUU CUGAUGAGGCCGAAAGGCCGAA AUAUUAA |
| 494 | GAUUUGC CUGAUGAGGCCGAAAGGCCGAA AGUUGAU |
| 501 | UGACAUU CUGAUGAGGCCGAAAGGCCGAA AUUUGCU |
| 507 | UGUUAGU CUGAUGAGGCCGAAAGGCCGAA ACAUUGA |
| 511 | AUGGUGU CUGAUGAGGCCGAAAGGCCGAA AGUGACA |
| 519 | AUUAACU CUGAUGAGGCCGAAAGGCCGAA AUGGUGU |
| 520 | UAUUAAC CUGAUGAGGCCGAAAGGCCGAA AAUGGUG |
| 523 | UUAUAUU CUGAUGAGGCCGAAAGGCCGAA ACUAAUG |
| 524 | UUUAUAU CUGAUGAGGCCGAAAGGCCGAA AACUAAU |

Table 35: RSV (N) HH Target Sequence

| nt. Position | HH Target Sequence | nt. Position | HH Target Sequence |
|---|---|---|---|
| 9 | GGCAAAU A CAAAGAU | 217 | GGUAUGU U AUAUGCG |
| 21 | GAUGGCU C UUAGCAA | 218 | GUAUGUU A UAUGCGA |
| 23 | UGGCUCU U AGCAAAG | 220 | AUGUUAU A UGCGAUG |
| 24 | GGCUCUU A GCAAAGU | 229 | GCGAUGU C UAGGUUA |
| 32 | GCAAAGU C AAGUUGA | 231 | GAUGUCU A GGUUAGG |
| 37 | GUCAAGU U GAAUGAU | 235 | UCUAGGU U AGGAAGA |
| 45 | GAAUGAU A CACUCAA | 236 | CUAGGUU A GGAAGAG |
| 50 | AUACACU C AACAAAG | 254 | ACACCAU A AAAAUAC |
| 60 | CAAAGAU C AACUUCU | 260 | UAAAAAU A CUCAGAG |
| 65 | AUCAACU U CUGUCAU | 263 | AAAUACU C AGAGAUG |
| 66 | UCAACUU C UGUCAUC | 277 | GCGGGAU A UCAUGUA |
| 70 | CUUCUGU C AUCCAGC | 279 | GGGAUAU C AUGUAAA |
| 73 | CUGUCAU C CAGCAAA | 284 | AUCAUGU A AAAGCAA |
| 82 | AGCAAAU A CACCAUC | 299 | AUGGAGU A GAUGUAA |
| 89 | ACACCAU C CAACGGA | 305 | UAGAUGU A ACAACAC |
| 108 | AGGAGAU A GUAUUGA | 315 | AACACAU C GUCAAGA |
| 111 | AGAUAGU A UUGAUAC | 318 | ACAUCGU C AAGACAU |
| 113 | AUAGUAU U GAUACUC | 326 | AAGACAU U AAUGGAA |
| 117 | UAUUGAU A CUCCUAA | 327 | AGACAUU A AUGGAAA |
| 120 | UGAUACU C CUAAUUA | 346 | AUGAAAU U UGAAGUG |
| 123 | UACUCCU A AUUAUGA | 347 | UGAAAUU U GAAGUGU |
| 126 | UCCUAAU U AUGAUGU | 355 | GAAGUGU U AACAUUG |
| 127 | CCUAAUU A UGAUGUG | 356 | AAGUGUU A ACAUUGG |
| 146 | AACACAU C AAUAAGU | 361 | UUAACAU U GGCAAGC |
| 150 | CAUCAAU A AGUUAUG | 370 | GCAAGCU U AACAACU |
| 154 | AAUAAGU U AUGUGGC | 371 | CAAGCUU A ACAACUG |
| 155 | AUAAGUU A UGUGGCA | 383 | CUGAAAU U CAAAUCA |
| 166 | GGCAUGU U AUUAAUC | 384 | UGAAAUU C AAAUCAA |
| 167 | GCAUGUU A UUAAUCA | 389 | UUCAAAU C AACAUUG |
| 169 | AUGUUAU U AAUCACA | 395 | UCAACAU U GAGAUAG |
| 170 | UGUUAUU A AUCACAG | 401 | UUGAGAU A GAAUCUA |
| 173 | UAUUAAU C ACAGAAG | 406 | AUAGAAU C UAGAAAA |
| 186 | AGAUGCU A AUCAUAA | 408 | AGAAUCU A GAAAAUC |
| 189 | UGCUAAU C AUAAAUU | 415 | AGAAAAU C CUACAAA |
| 192 | UAAUCAU A AAUUCAC | 418 | AAAUCCU A CAAAAAA |
| 196 | CAUAAAU U CACUGGG | 431 | AAAUGCU A AAAGAAA |
| 197 | AUAAAUU C ACUGGGU | 449 | GAGAGGU A GCUCCAG |
| 205 | ACUGGGU U AAUAGGU | 453 | GGUAGCU C CAGAAUA |
| 206 | CUGGGUU A AUAGGUA | 460 | CCAGAAU A CAGGCAU |
| 209 | GGUUAAU A GGUAUGU | 472 | CAUGACU C UCCUGAU |
| 213 | AAUAGGU A UGUUAUA | 474 | UGACUCU C CUGAUUG |

| | | | |
|---|---|---|---|
| 480 | UCCUGAU U GUGGGAU | 696 | UUUUGGU A UAGCACA |
| 491 | GGAUGAU A AUAUUAU | 698 | UUGGUAU A GCACAAU |
| 494 | UGAUAAU A UUAUGUA | 706 | GCACAAU C UUCUACC |
| 496 | AUAAUAU U AUGUAUA | 708 | ACAAUCU U CUACCAG |
| 497 | UAAUAUU A UGUAUAG | 709 | CAAUCUU C UACCAGA |
| 501 | AUUAUGU A UAGCAGC | 711 | AUCUUCU A CCAGAGG |
| 503 | UAUGUAU A GCAGCAU | 726 | UGGCAGU A GAGUUGA |
| 511 | GCAGCAU U AGUAAUA | 731 | GUAGAGU U GAAGGGA |
| 512 | CAGCAUU A GUAAUAA | 740 | AAGGGAU U UUUGCAG |
| 515 | CAUUAGU A AUAACUA | 741 | AGGGAUU U UUGCAGG |
| 518 | UAGUAAU A ACUAAAU | 742 | GGGAUUU U UGCAGGA |
| 522 | AAUAACU A AAUUAGC | 743 | GGAUUUU U GCAGGAU |
| 526 | ACUAAAU U AGCAGCA | 751 | GCAGGAU U GUUUAUG |
| 527 | CUAAAUU A GCAGCAG | 754 | GGAUUGU U UAUGAAU |
| 544 | GACAGAU C UGGUCUU | 755 | GAUUGUU U AUGAAUG |
| 549 | AUCUGGU C UUACAGC | 756 | AUUGUUU A UGAAUGC |
| 551 | CUGGUCU U ACAGCCG | 766 | AAUGCCU A UGGUGCA |
| 552 | UGGUCUU A CAGCCGU | 787 | GUGAUGU U ACGGUGG |
| 563 | CCGUGAU U AGGAGAG | 788 | UGAUGUU A CGGUGGG |
| 564 | CGUGAUU A GGAGAGC | 800 | GGGGAGU C UUAGCAA |
| 573 | GAGAGCU A AUAAUGU | 802 | GGAGUCU U AGCAAAA |
| 576 | AGCUAAU A AUGUCCU | 803 | GAGUCUU A GCAAAAU |
| 581 | AUAAUGU C CUAAAAA | 811 | GCAAAAU C AGUUAAA |
| 584 | AUGUCCU A AAAAAUG | 815 | AAUCAGU U AAAAAUA |
| 603 | GAAACGU U ACAAAGG | 816 | AUCAGUU A AAAAUAU |
| 604 | AAACGUU A CAAAGGC | 822 | UAAAAAU A UUAUGUU |
| 613 | AAAGGCU U ACUACCC | 824 | AAAAUAU U AUGUUAG |
| 614 | AAGGCUU A CUACCCA | 825 | AAAUAUU A UGUUAGG |
| 617 | GCUUACU A CCCAAGG | 829 | AUUAUGU U AGGACAU |
| 629 | AGGACAU A GCCAACA | 830 | UUAUGUU A GGACAUG |
| 640 | AACAGCU U CUAUGAA | 840 | ACAUGCU A GUGUGCA |
| 641 | ACAGCUU C UAUGAAG | 866 | AACAAGU U GUUGAGG |
| 643 | AGCUUCU A UGAAGUG | 869 | AAGUUGU U GAGGUUU |
| 652 | GAAGUGU U UGAAAAA | 875 | UUGAGGU U UAUGAAU |
| 653 | AAGUGUU U GAAAAAC | 876 | UGAGGUU U AUGAAUA |
| 663 | AAAACAU C CCCACUU | 877 | GAGGUUU A UGAAUAU |
| 670 | CCCCACU U UAUAGAU | 883 | UAUGAAU A UGCCCAA |
| 671 | CCCACUU U AUAGAUG | 895 | CAAAAAU U GGGUGGU |
| 672 | CCACUUU A UAGAUGU | 913 | GCAGGAU U CUACCAU |
| 674 | ACUUUAU A GAUGUUU | 914 | CAGGAUU C UACCAUA |
| 680 | UAGAUGU U UUUGUUC | 916 | GGAUUCU A CCAUAUA |
| 681 | AGAUGUU U UUGUUCA | 921 | CUACCAU A UAUUGAA |
| 682 | GAUGUUU U UGUUCAU | 923 | ACCAUAU A UUGAACA |
| 683 | AUGUUUU U GUUCAUU | 925 | CAUAUAU U GAACAAC |
| 686 | UUUUUGU U CAUUUUG | 943 | AAAGCAU C AUUAUUA |
| 687 | UUUUGUU C AUUUUGG | 946 | GCAUCAU U AUUAUCU |
| 690 | UGUUCAU U UUGGUAU | 947 | CAUCAUU A UUAUCUU |
| 691 | GUUCAUU U UGGUAUA | 949 | UCAUUAU U AUCUUUG |
| 692 | UUCAUUU U GGUAUAG | 950 | CAUUAUU A UCUUUGA |

| | |
|---|---|
| 952 | UUAUUAU C UUUGACU |
| 954 | AUUAUCU U UGACUCA |
| 955 | UUAUCUU U GACUCAA |
| 960 | UUUGACU C AAUUUCC |
| 964 | ACUCAAU U UCCUCAC |
| 965 | CUCAAUU U CCUCACU |
| 966 | UCAAUUU C CUCACUU |
| 969 | AUUUCCU C ACUUCUC |
| 973 | CCUCACU U CUCCAGU |
| 974 | CUCACUU C UCCAGUG |
| 976 | CACUUCU C CAGUGUA |
| 983 | CCAGUGU A GUAUUAG |
| 986 | GUGUAGU A UUAGGCA |
| 988 | GUAGUAU U AGGCAAU |
| 989 | UAGUAUU A GGCAAUG |
| 1007 | CUGGCCU A GGCAUAA |
| 1013 | UAGGCAU A AUGGGAG |
| 1024 | GGAGAGU A CAGAGGU |
| 1032 | CAGAGGU A CACCGAG |
| 1044 | GAGGAAU C AAGAUCU |
| 1050 | UCAAGAU C UAUAUGA |
| 1052 | AAGAUCU A UAUGAUG |
| 1054 | GAUCUAU A UGAUGCA |
| 1072 | AAGGCAU A UGCUGAA |
| 1085 | AACAACU C AAAGAAA |
| 1103 | GUGUGAU U AACUACA |
| 1104 | UGUGAUU A ACUACAG |
| 1108 | AUUAACU A CAGUGUA |
| 1115 | ACAGUGU A CUAGACU |
| 1118 | GUGUACU A GACUUGA |
| 1123 | CUAGACU U GACAGCA |
| 1139 | AAGAACU A GAGGCUA |
| 1146 | AGAGGCU A UCAAACA |
| 1148 | AGGCUAU C AAACAUC |
| 1155 | CAAACAU C AGCUUAA |
| 1160 | AUCAGCU U AAUCCAA |
| 1161 | UCAGCUU A AUCCAAA |
| 1164 | GCUUAAU C CAAAAGA |
| 1173 | AAAAGAU A AUGAUGU |
| 1181 | AUGAUGU A GAGCUUU |
| 1187 | UAGAGCU U UGAGUUA |
| 1188 | AGAGCUU U GAGUUAA |
| 1193 | UUUGAGU U AAUAAAA |
| 1194 | UUGAGUU A AUAAAAA |

Table 36: RSV (N) HH Ribozyme Sequence

| nt. Position | HH Ribozyme Sequence |
|---|---|
| 9 | AUCUUUG CUGAUGAGGCCGAAAGGCCGAA AUUUGCC |
| 21 | UUGCUAA CUGAUGAGGCCGAAAGGCCGAA AGCCAUC |
| 23 | CUUUGCU CUGAUGAGGCCGAAAGGCCGAA AGAGCCA |
| 24 | ACUUUGC CUGAUGAGGCCGAAAGGCCGAA AAGAGCC |
| 32 | UCAACUU CUGAUGAGGCCGAAAGGCCGAA ACUUUGC |
| 37 | AUCAUUC CUGAUGAGGCCGAAAGGCCGAA ACUUGAC |
| 45 | UUGAGUG CUGAUGAGGCCGAAAGGCCGAA AUCAUUC |
| 50 | CUUUGUU CUGAUGAGGCCGAAAGGCCGAA AGUGUAU |
| 60 | AGAAGUU CUGAUGAGGCCGAAAGGCCGAA AUCUUUG |
| 65 | AUGACAG CUGAUGAGGCCGAAAGGCCGAA AGUUGAU |
| 66 | GAUGACA CUGAUGAGGCCGAAAGGCCGAA AAGUUGA |
| 70 | GCUGGAU CUGAUGAGGCCGAAAGGCCGAA ACAGAAG |
| 73 | UUUGCUG CUGAUGAGGCCGAAAGGCCGAA AUGACAG |
| 82 | GAUGGUG CUGAUGAGGCCGAAAGGCCGAA AUUUGCU |
| 89 | UCCGUUG CUGAUGAGGCCGAAAGGCCGAA AUGGUGU |
| 108 | UCAAUAC CUGAUGAGGCCGAAAGGCCGAA AUCUCCU |
| 111 | GUAUCAA CUGAUGAGGCCGAAAGGCCGAA ACUAUCU |
| 113 | GAGUAUC CUGAUGAGGCCGAAAGGCCGAA AUACUAU |
| 117 | UUAGGAG CUGAUGAGGCCGAAAGGCCGAA AUCAAUA |
| 120 | UAAUUAG CUGAUGAGGCCGAAAGGCCGAA AGUAUCA |
| 123 | UCAUAAU CUGAUGAGGCCGAAAGGCCGAA AGGAGUA |
| 126 | ACAUCAU CUGAUGAGGCCGAAAGGCCGAA AUUAGGA |
| 127 | CACAUCA CUGAUGAGGCCGAAAGGCCGAA AAUUAGG |
| 146 | ACUUAUU CUGAUGAGGCCGAAAGGCCGAA AUGUGUU |
| 150 | CAUAACU CUGAUGAGGCCGAAAGGCCGAA AUUGAUG |
| 154 | GCCACAU CUGAUGAGGCCGAAAGGCCGAA ACUUAUU |
| 155 | UGCCACA CUGAUGAGGCCGAAAGGCCGAA AACUUAU |
| 166 | GAUUAAU CUGAUGAGGCCGAAAGGCCGAA ACAUGCC |
| 167 | UGAUUAA CUGAUGAGGCCGAAAGGCCGAA AACAUGC |
| 169 | UGUGAUU CUGAUGAGGCCGAAAGGCCGAA AUAACAU |
| 170 | CUGUGAU CUGAUGAGGCCGAAAGGCCGAA AAUAACA |
| 173 | CUUCUGU CUGAUGAGGCCGAAAGGCCGAA AUUAAUA |
| 186 | UUAUGAU CUGAUGAGGCCGAAAGGCCGAA AGCAUCU |
| 189 | AAUUUAU CUGAUGAGGCCGAAAGGCCGAA AUUAGCA |
| 192 | GUGAAUU CUGAUGAGGCCGAAAGGCCGAA AUGAUUA |
| 196 | CCCAGUG CUGAUGAGGCCGAAAGGCCGAA AUUUAUG |
| 197 | ACCCAGU CUGAUGAGGCCGAAAGGCCGAA AAUUUAU |
| 205 | ACCUAUU CUGAUGAGGCCGAAAGGCCGAA ACCCAGU |
| 206 | UACCUAU CUGAUGAGGCCGAAAGGCCGAA AACCCAG |
| 209 | ACAUACC CUGAUGAGGCCGAAAGGCCGAA AUUAACC |
| 213 | UAUAACA CUGAUGAGGCCGAAAGGCCGAA ACCUAUU |

| | |
|---|---|
| 217 | CGCAUAU CUGAUGAGGCCGAAAGGCCGAA ACAUACC |
| 218 | UCGCAUA CUGAUGAGGCCGAAAGGCCGAA AACAUAC |
| 220 | CAUCGCA CUGAUGAGGCCGAAAGGCCGAA AUAACAU |
| 229 | UAACCUA CUGAUGAGGCCGAAAGGCCGAA ACAUCGC |
| 231 | CCUAACC CUGAUGAGGCCGAAAGGCCGAA AGACAUC |
| 235 | UCUUCCU CUGAUGAGGCCGAAAGGCCGAA ACCUAGA |
| 236 | CUCUUCC CUGAUGAGGCCGAAAGGCCGAA AACCUAG |
| 254 | GUAUUUU CUGAUGAGGCCGAAAGGCCGAA AUGGUGU |
| 260 | CUCUGAG CUGAUGAGGCCGAAAGGCCGAA AUUUUUA |
| 263 | CAUCUCU CUGAUGAGGCCGAAAGGCCGAA AGUAUUU |
| 277 | UACAUGA CUGAUGAGGCCGAAAGGCCGAA AUCCCGC |
| 279 | UUUACAU CUGAUGAGGCCGAAAGGCCGAA AUAUCCC |
| 284 | UUGCUUU CUGAUGAGGCCGAAAGGCCGAA ACAUGAU |
| 299 | UUACAUC CUGAUGAGGCCGAAAGGCCGAA ACUCCAU |
| 305 | GUGUUGU CUGAUGAGGCCGAAAGGCCGAA ACAUCUA |
| 315 | UCUUGAC CUGAUGAGGCCGAAAGGCCGAA AUGUGUU |
| 318 | AUGUCUU CUGAUGAGGCCGAAAGGCCGAA ACGAUGU |
| 326 | UUCCAUU CUGAUGAGGCCGAAAGGCCGAA AUGUCUU |
| 327 | UUUCCAU CUGAUGAGGCCGAAAGGCCGAA AAUGUCU |
| 346 | CACUUCA CUGAUGAGGCCGAAAGGCCGAA AUUUCAU |
| 347 | ACACUUC CUGAUGAGGCCGAAAGGCCGAA AAUUUCA |
| 355 | CAAUGUU CUGAUGAGGCCGAAAGGCCGAA ACACUUC |
| 356 | CCAAUGU CUGAUGAGGCCGAAAGGCCGAA AACACUU |
| 361 | GCUUGCC CUGAUGAGGCCGAAAGGCCGAA AUGUUAA |
| 370 | AGUUGUU CUGAUGAGGCCGAAAGGCCGAA AGCUUGC |
| 371 | CAGUUGU CUGAUGAGGCCGAAAGGCCGAA AAGCUUG |
| 383 | UGAUUUG CUGAUGAGGCCGAAAGGCCGAA AUUUCAG |
| 384 | UUGAUUU CUGAUGAGGCCGAAAGGCCGAA AAUUUCA |
| 389 | CAAUGUU CUGAUGAGGCCGAAAGGCCGAA AUUUGAA |
| 395 | CUAUCUC CUGAUGAGGCCGAAAGGCCGAA AUGUUGA |
| 401 | UAGAUUC CUGAUGAGGCCGAAAGGCCGAA AUCUCAA |
| 406 | UUUUCUA CUGAUGAGGCCGAAAGGCCGAA AUUCUAU |
| 408 | GAUUUUC CUGAUGAGGCCGAAAGGCCGAA AGAUUCU |
| 415 | UUUGUAG CUGAUGAGGCCGAAAGGCCGAA AUUUUCU |
| 418 | UUUUUUG CUGAUGAGGCCGAAAGGCCGAA AGGAUUU |
| 431 | UUUCUUU CUGAUGAGGCCGAAAGGCCGAA AGCAUUU |
| 449 | CUGGAGC CUGAUGAGGCCGAAAGGCCGAA ACCUCUC |
| 453 | UAUUCUG CUGAUGAGGCCGAAAGGCCGAA AGCUACC |
| 460 | AUGCCUG CUGAUGAGGCCGAAAGGCCGAA AUUCUGG |
| 472 | AUCAGGA CUGAUGAGGCCGAAAGGCCGAA AGUCAUG |
| 474 | CAAUCAG CUGAUGAGGCCGAAAGGCCGAA AGAGUCA |
| 480 | AUCCCAC CUGAUGAGGCCGAAAGGCCGAA AUCAGGA |
| 491 | AUAAUAU CUGAUGAGGCCGAAAGGCCGAA AUCAUCC |
| 494 | UACAUAA CUGAUGAGGCCGAAAGGCCGAA AUUAUCA |
| 496 | UAUACAU CUGAUGAGGCCGAAAGGCCGAA AUAUUAU |
| 497 | CUAUACA CUGAUGAGGCCGAAAGGCCGAA AAUAUUA |
| 501 | GCUGCUA CUGAUGAGGCCGAAAGGCCGAA ACAUAAU |
| 503 | AUGCUGC CUGAUGAGGCCGAAAGGCCGAA AUACAUA |
| 511 | UAUUACU CUGAUGAGGCCGAAAGGCCGAA AUGCUGC |

| | | | |
|---|---|---|---|
| 512 | UUAUUAC | CUGAUGAGGCCGAAAGGCCGAA | AAUGCUG |
| 515 | UAGUUAU | CUGAUGAGGCCGAAAGGCCGAA | ACUAAUG |
| 518 | AUUUAGU | CUGAUGAGGCCGAAAGGCCGAA | AUUACUA |
| 522 | GCUAAUU | CUGAUGAGGCCGAAAGGCCGAA | AGUUAUU |
| 526 | UGCUGCU | CUGAUGAGGCCGAAAGGCCGAA | AUUUAGU |
| 527 | CUGCUGC | CUGAUGAGGCCGAAAGGCCGAA | AAUUUAG |
| 544 | AAGACCA | CUGAUGAGGCCGAAAGGCCGAA | AUCUGUC |
| 549 | GCUGUAA | CUGAUGAGGCCGAAAGGCCGAA | ACCAGAU |
| 551 | CGGCUGU | CUGAUGAGGCCGAAAGGCCGAA | AGACCAG |
| 552 | ACGGCUG | CUGAUGAGGCCGAAAGGCCGAA | AAGACCA |
| 563 | CUCUCCU | CUGAUGAGGCCGAAAGGCCGAA | AUCACGG |
| 564 | GCUCUCC | CUGAUGAGGCCGAAAGGCCGAA | AAUCACG |
| 573 | ACAUUAU | CUGAUGAGGCCGAAAGGCCGAA | AGCUCUC |
| 576 | AGGACAU | CUGAUGAGGCCGAAAGGCCGAA | AUUAGCU |
| 581 | UUUUUAG | CUGAUGAGGCCGAAAGGCCGAA | ACAUUAU |
| 584 | CAUUUUU | CUGAUGAGGCCGAAAGGCCGAA | AGGACAU |
| 603 | CCUUUGU | CUGAUGAGGCCGAAAGGCCGAA | ACGUUUC |
| 604 | GCCUUUG | CUGAUGAGGCCGAAAGGCCGAA | AACGUUU |
| 613 | GGGUAGU | CUGAUGAGGCCGAAAGGCCGAA | AGCCUUU |
| 614 | UGGGUAG | CUGAUGAGGCCGAAAGGCCGAA | AAGCCUU |
| 617 | CCUUGGG | CUGAUGAGGCCGAAAGGCCGAA | AGUAAGC |
| 629 | UGUUGGC | CUGAUGAGGCCGAAAGGCCGAA | AUGUCCU |
| 640 | UUCAUAG | CUGAUGAGGCCGAAAGGCCGAA | AGCUGUU |
| 641 | CUUCAUA | CUGAUGAGGCCGAAAGGCCGAA | AAGCUGU |
| 643 | CACUUCA | CUGAUGAGGCCGAAAGGCCGAA | AGAAGCU |
| 652 | UUUUUCA | CUGAUGAGGCCGAAAGGCCGAA | ACACUUC |
| 653 | GUUUUUC | CUGAUGAGGCCGAAAGGCCGAA | AACACUU |
| 663 | AAGUGGG | CUGAUGAGGCCGAAAGGCCGAA | AUGUUUU |
| 670 | AUCUAUA | CUGAUGAGGCCGAAAGGCCGAA | AGUGGGG |
| 671 | CAUCUAU | CUGAUGAGGCCGAAAGGCCGAA | AAGUGGG |
| 672 | ACAUCUA | CUGAUGAGGCCGAAAGGCCGAA | AAAGUGG |
| 674 | AAACAUC | CUGAUGAGGCCGAAAGGCCGAA | AUAAAGU |
| 680 | GAACAAA | CUGAUGAGGCCGAAAGGCCGAA | ACAUCUA |
| 681 | UGAACAA | CUGAUGAGGCCGAAAGGCCGAA | AACAUCU |
| 682 | AUGAACA | CUGAUGAGGCCGAAAGGCCGAA | AAACAUC |
| 683 | AAUGAAC | CUGAUGAGGCCGAAAGGCCGAA | AAAACAU |
| 686 | CAAAAUG | CUGAUGAGGCCGAAAGGCCGAA | ACAAAAA |
| 687 | CCAAAAU | CUGAUGAGGCCGAAAGGCCGAA | AACAAAA |
| 690 | AUACCAA | CUGAUGAGGCCGAAAGGCCGAA | AUGAACA |
| 691 | UAUACCA | CUGAUGAGGCCGAAAGGCCGAA | AAUGAAC |
| 692 | CUAUACC | CUGAUGAGGCCGAAAGGCCGAA | AAAUGAA |
| 696 | UGUGCUA | CUGAUGAGGCCGAAAGGCCGAA | ACCAAAA |
| 698 | AUUGUGC | CUGAUGAGGCCGAAAGGCCGAA | AUACCAA |
| 706 | GGUAGAA | CUGAUGAGGCCGAAAGGCCGAA | AUUGUGC |
| 708 | CUGGUAG | CUGAUGAGGCCGAAAGGCCGAA | AGAUUGU |
| 709 | UCUGGUA | CUGAUGAGGCCGAAAGGCCGAA | AAGAUUG |
| 711 | CCUCUGG | CUGAUGAGGCCGAAAGGCCGAA | AGAAGAU |
| 726 | UCAACUC | CUGAUGAGGCCGAAAGGCCGAA | ACUGCCA |
| 731 | UCCCUUC | CUGAUGAGGCCGAAAGGCCGAA | ACUCUAC |

| | |
|---|---|
| 740 | CUGCAAA CUGAUGAGGCCGAAAGGCCGAA AUCCCUU |
| 741 | CCUGCAA CUGAUGAGGCCGAAAGGCCGAA AAUCCCU |
| 742 | UCCUGCA CUGAUGAGGCCGAAAGGCCGAA AAAUCCC |
| 743 | AUCCUGC CUGAUGAGGCCGAAAGGCCGAA AAAAUCC |
| 751 | CAUAAAC CUGAUGAGGCCGAAAGGCCGAA AUCCUGC |
| 754 | AUUCAUA CUGAUGAGGCCGAAAGGCCGAA ACAAUCC |
| 755 | CAUUCAU CUGAUGAGGCCGAAAGGCCGAA AACAAUC |
| 756 | GCAUUCA CUGAUGAGGCCGAAAGGCCGAA AAACAAU |
| 766 | UGCACCA CUGAUGAGGCCGAAAGGCCGAA AGGCAUU |
| 787 | CCACCGU CUGAUGAGGCCGAAAGGCCGAA ACAUCAC |
| 788 | CCCACCG CUGAUGAGGCCGAAAGGCCGAA AACAUCA |
| 800 | UUGCUAA CUGAUGAGGCCGAAAGGCCGAA ACUCCCC |
| 802 | UUUUGCU CUGAUGAGGCCGAAAGGCCGAA AGACUCC |
| 803 | AUUUUGC CUGAUGAGGCCGAAAGGCCGAA AAGACUC |
| 811 | UUUAACU CUGAUGAGGCCGAAAGGCCGAA AUUUUGC |
| 815 | UAUUUUU CUGAUGAGGCCGAAAGGCCGAA ACUGAUU |
| 816 | AUAUUUU CUGAUGAGGCCGAAAGGCCGAA AACUGAU |
| 822 | AACAUAA CUGAUGAGGCCGAAAGGCCGAA AUUUUUA |
| 824 | CUAACAU CUGAUGAGGCCGAAAGGCCGAA AUAUUUU |
| 825 | CCUAACA CUGAUGAGGCCGAAAGGCCGAA AAUAUUU |
| 829 | AUGUCCU CUGAUGAGGCCGAAAGGCCGAA ACAUAAU |
| 830 | CAUGUCC CUGAUGAGGCCGAAAGGCCGAA AACAUAA |
| 840 | UGCACAC CUGAUGAGGCCGAAAGGCCGAA AGCAUGU |
| 866 | CCUCAAC CUGAUGAGGCCGAAAGGCCGAA ACUUGUU |
| 869 | AAACCUC CUGAUGAGGCCGAAAGGCCGAA ACAACUU |
| 875 | AUUCAUA CUGAUGAGGCCGAAAGGCCGAA ACCUCAA |
| 876 | UAUUCAU CUGAUGAGGCCGAAAGGCCGAA AACCUCA |
| 877 | AUAUUCA CUGAUGAGGCCGAAAGGCCGAA AAACCUC |
| 883 | UUGGGCA CUGAUGAGGCCGAAAGGCCGAA AUUCAUA |
| 895 | ACCACCC CUGAUGAGGCCGAAAGGCCGAA AUUUUUG |
| 913 | AUGGUAG CUGAUGAGGCCGAAAGGCCGAA AUCCUGC |
| 914 | UAUGGUA CUGAUGAGGCCGAAAGGCCGAA AAUCCUG |
| 916 | UAUAUGG CUGAUGAGGCCGAAAGGCCGAA AGAAUCC |
| 921 | UUCAAUA CUGAUGAGGCCGAAAGGCCGAA AUGGUAG |
| 923 | UGUUCAA CUGAUGAGGCCGAAAGGCCGAA AUAUGGU |
| 925 | GUUGUUC CUGAUGAGGCCGAAAGGCCGAA AUAUAUG |
| 943 | UAAUAAU CUGAUGAGGCCGAAAGGCCGAA AUGCUUU |
| 946 | AGAUAAU CUGAUGAGGCCGAAAGGCCGAA AUGAUGC |
| 947 | AAGAUAA CUGAUGAGGCCGAAAGGCCGAA AAUGAUG |
| 949 | CAAAGAU CUGAUGAGGCCGAAAGGCCGAA AUAAUGA |
| 950 | UCAAAGA CUGAUGAGGCCGAAAGGCCGAA AAUAAUG |
| 952 | AGUCAAA CUGAUGAGGCCGAAAGGCCGAA AUAAUAA |
| 954 | UGAGUCA CUGAUGAGGCCGAAAGGCCGAA AGAUAAU |
| 955 | UUGAGUC CUGAUGAGGCCGAAAGGCCGAA AAGAUAA |
| 960 | GGAAAUU CUGAUGAGGCCGAAAGGCCGAA AGUCAAA |
| 964 | GUGAGGA CUGAUGAGGCCGAAAGGCCGAA AUUGAGU |
| 965 | AGUGAGG CUGAUGAGGCCGAAAGGCCGAA AAUUGAG |
| 966 | AAGUGAG CUGAUGAGGCCGAAAGGCCGAA AAAUUGA |
| 969 | GAGAAGU CUGAUGAGGCCGAAAGGCCGAA AGGAAAU |

| | | | |
|---|---|---|---|
| 973 | ACUGGAG | CUGAUGAGGCCGAAAGGCCGAA | AGUGAGG |
| 974 | CACUGGA | CUGAUGAGGCCGAAAGGCCGAA | AAGUGAG |
| 976 | UACACUG | CUGAUGAGGCCGAAAGGCCGAA | AGAAGUG |
| 983 | CUAAUAC | CUGAUGAGGCCGAAAGGCCGAA | ACACUGG |
| 986 | UGCCUAA | CUGAUGAGGCCGAAAGGCCGAA | ACUACAC |
| 988 | AUUGCCU | CUGAUGAGGCCGAAAGGCCGAA | AUACUAC |
| 989 | CAUUGCC | CUGAUGAGGCCGAAAGGCCGAA | AAUACUA |
| 1007 | UUAUGCC | CUGAUGAGGCCGAAAGGCCGAA | AGGCCAG |
| 1013 | CUCCCAU | CUGAUGAGGCCGAAAGGCCGAA | AUGCCUA |
| 1024 | ACCUCUG | CUGAUGAGGCCGAAAGGCCGAA | ACUCUCC |
| 1032 | CUCGGUG | CUGAUGAGGCCGAAAGGCCGAA | ACCUCUG |
| 1044 | AGAUCUU | CUGAUGAGGCCGAAAGGCCGAA | AUUCCUC |
| 1050 | UCAUAUA | CUGAUGAGGCCGAAAGGCCGAA | AUCUUGA |
| 1052 | CAUCAUA | CUGAUGAGGCCGAAAGGCCGAA | AGAUCUU |
| 1054 | UGCAUCA | CUGAUGAGGCCGAAAGGCCGAA | AUAGAUC |
| 1072 | UUCAGCA | CUGAUGAGGCCGAAAGGCCGAA | AUGCCUU |
| 1085 | UUUCUUU | CUGAUGAGGCCGAAAGGCCGAA | AGUUGUU |
| 1103 | UGUAGUU | CUGAUGAGGCCGAAAGGCCGAA | AUCACAC |
| 1104 | CUGUAGU | CUGAUGAGGCCGAAAGGCCGAA | AAUCACA |
| 1108 | UACACUG | CUGAUGAGGCCGAAAGGCCGAA | AGUUAAU |
| 1115 | AGUCUAG | CUGAUGAGGCCGAAAGGCCGAA | ACACUGU |
| 1118 | UCAAGUC | CUGAUGAGGCCGAAAGGCCGAA | AGUACAC |
| 1123 | UGCUGUC | CUGAUGAGGCCGAAAGGCCGAA | AGUCUAG |
| 1139 | UAGCCUC | CUGAUGAGGCCGAAAGGCCGAA | AGUUCUU |
| 1146 | UGUUUGA | CUGAUGAGGCCGAAAGGCCGAA | AGCCUCU |
| 1148 | GAUGUUU | CUGAUGAGGCCGAAAGGCCGAA | AUAGCCU |
| 1155 | UUAAGCU | CUGAUGAGGCCGAAAGGCCGAA | AUGUUUG |
| 1160 | UUGGAUU | CUGAUGAGGCCGAAAGGCCGAA | AGCUGAU |
| 1161 | UUUGGAU | CUGAUGAGGCCGAAAGGCCGAA | AAGCUGA |
| 1164 | UCUUUUG | CUGAUGAGGCCGAAAGGCCGAA | AUUAAGC |
| 1173 | ACAUCAU | CUGAUGAGGCCGAAAGGCCGAA | AUCUUUU |
| 1181 | AAAGCUC | CUGAUGAGGCCGAAAGGCCGAA | ACAUCAU |
| 1187 | UAACUCA | CUGAUGAGGCCGAAAGGCCGAA | AGCUCUA |
| 1188 | UUAACUC | CUGAUGAGGCCGAAAGGCCGAA | AAGCUCU |
| 1193 | UUUUAUU | CUGAUGAGGCCGAAAGGCCGAA | ACUCAAA |
| 1194 | UUUUUAU | CUGAUGAGGCCGAAAGGCCGAA | AACUCAA |

**Table 37: RSV (1B) HP Ribozyme/Substrate Sequence**

| nt. Position | HP Ribozyme Sequence | Substrate |
|---|---|---|
| 70 | CUGUGAUC AGAA GUCUUU ACCAGAGAAACACACGUUGUGGUACAUUACCUGGUA | AAAGACU GAU GAUCACAG |
| 91 | CAAGUGAC AGAA GUCUCA ACCAGAGAAACACACGUUGUGGUACAUUACCUGGUA | UGAGACC GUU GUCACUUG |
| 472 | CAGGCUCC AGAA GGACUA ACCAGAGAAACACACGUUGUGGUACAUUACCUGGUA | UAGUCCA GAU GGAGCCUG |

Table 38: RSV (N) HP Ribozyme/Substrate Sequence

| nt. Position | Hairpin Ribozyme Sequence | Substrate |
|---|---|---|
| 476 | AUCCCACA AGAA GGAGAG ACCAGAGAAACACACGUUGUGGUACAUUACCUGGUA | CUCUCCU GAU UGUGGGAU |
| 540 | AAGACCAG AGAA GUCCCC ACCAGAGAAACACACGUUGUGGUACAUUACCUGGUA | GGGGACA GAU CUGGUCUU |
| 554 | CUAAUCAC AGAA GUAAGA ACCAGAGAAACACACGUUGUGGUACAUUACCUGGUA | UCUUACA GCC GUGAUUAG |
| 636 | UUCAUAGA AGAA GUUGGC ACCAGAGAAACACACGUUGUGGUACAUUACCUGGUA | GCCAACA GCU UCUAUGAA |
| 998 | CCUAGGCC AGAA GCAUUG ACCAGAGAAACACACGUUGUGGUACAUUACCUGGUA | CAAUGCU GCU GGCCUAGG |
| 1156 | UUGGAUUA AGAA GAUGUU ACCAGAGAAACACACGUUGUGGUACAUUACCUGGUA | AACAUCA GCU UAAUCCAA |

Table 39: Large-Scale Synthesis

| Sequence | Activator [Added/Final] (min) | Amidite [Added/Final] (min) | Time* | % Full Length Product |
|---|---|---|---|---|
| $A_9T$ | T [0.50/0.33] | [0.1/0.02] | 15 m | 85 |
| $A_9T$ | S [0.25/0.17] | [0.1/0.02] | 15 m | 89 |
| $(GGU)_3GGT$ | T [0.50/0.33] | [0.1/0.02] | 15 m | 78 |
| $(GGU)_3GGT$ | S [0.25/0.17] | [0.1/0.02] | 15 m | 81 |
| $C_9T$ | T [0.50/0.33] | [0.1/0.02] | 15 m | 90 |
| $C_9T$ | S [0.25/0.17] | [0.1/0.02] | 15 m | 97 |
| $U_9T$ | T [0.50/0.33] | [0.1/0.02] | 15 m | 80 |
| $U_9T$ | S [0.25/0.17] | [0.1/0.02] | 15 m | 85 |
| A (36-mer) | T [0.50/0.33] | [0.1/0.02] | 15/15m | 21 |
| A (36-mer) | S [0.25/0.17] | [0.1/0.02] | 15/15 m | 25 |
| A (36-mer) | S [0.50/0.24] | [0.1/0.03] | 15/15 m | 25 |
| A (36-mer) | S [0.50/0.18] | [0.1/0.05] | 15/15 m | 38 |
| A (36-mer) | S [0.50/0.18] | [0.1/0.05] | 10/5 m | 42 |

*Where two coupling times are indicated the first refers to RNA coupling and the second to 2'-*O*-methyl coupling. S = 5-*S*-Ethyltetrazole, T = tetrazole activator. A is 5' -ucu ccA UCU GAU GAG GCC GAA AGG CCG AAA Auc ccu -3' where lowerecase represents 2'-*O*-methylnucleotides.

## Table 40: Base Deprotection

| Sequence | Deprotection Reagent | Time (min) | T °C | % Full Length Product |
|---|---|---|---|---|
| iBu(GGU)$_4$ | $NH_4OH$/EtOH | 16 h | 55 | 62.5 |
| | MA | 10 m | 65 | 62.7 |
| | AMA | 10 m | 65 | 74.8 |
| | MA | 10 m | 55 | 75.0 |
| | AMA | 10 m | 55 | 77.2 |
| iPrP(GGU)$_4$ | $NH_4OH$/EtOH | 4 h | 65 | 44.8 |
| | MA | 10 m | 65 | 65.9 |
| | AMA | 10 m | 65 | 59.8 |
| | MA | 10 m | 55 | 61.3 |
| | AMA | 10 m | 55 | 60.1 |
| C$_9$U | $NH_4OH$/EtOH | 4 h | 65 | 75.2 |
| | MA | 10 m | 65 | 79.1 |
| | AMA | 10 m | 65 | 77.1 |
| | MA | 10 m | 55 | 79.8 |
| | AMA | 10 m | 55 | 75.5 |
| A (36-mer) | $NH_4OH$/EtOH | 4 h | 65 | 22.7 |
| | MA | 10 m | 65 | 28.9 |

Table 41: 2'-*O*-Alkylsilyl Deprotection

| Sequence | Deprotection Reagent | Time (min) | T °C | % Full Length Product |
|---|---|---|---|---|
| $A_9T$ | TBAF | 24 h | 20 | 84.5 |
| | 1.4 M HF | 0.5 h | 65 | 81.0 |
| $(GGU)_4$ | TBAF | 24 h | 20 | 60.9 |
| | 1.4 M HF | 0.5 h | 65 | 67.8 |
| $C_{10}$ | TBAF | 24 h | 20 | 86.2 |
| | 1.4 M HF | 0.5 h | 65 | 86.1 |
| $U_{10}$ | TBAF | 24 h | 20 | 84.8 |
| | 1.4 M HF | 0.5 h | 65 | 84.5 |
| B (36-mer) | TBAF | 24 h | 20 | 25.2 |
| | 1.4 M HF | 1.5 h | 65 | 30.6 |
| A (36-mer) | TBAF | 24 h | 20 | 29.7 |
| | 1.4 M HF | 1.5 h | 65 | 30.4 |

B is 5'- UCU CCA UCU GAU GAG GCC GAA AGG CCG AAA AUC CCU -3'.

Table 42 : *NMR Data for UC Dimers containing Phosphorothioate Linkage*

| Synthesis # | Type | Delivery | Eq. | Wait | ASE (%) |
|---|---|---|---|---|---|
| 3524 | ribo | 2 x 3 s | 10.4 | 2 x 100 s | 95.9 |
| 3525 | ribo | 2 x 3 s | 10.4 | 2 x 75 s | 92.6 |
| 3530 | ribo | 2 x 3 s | 10.4 | 2 x 75 s | 92.1 |
| 3526 | ribo | 1 x 5 s | 08.6 | 1 x 300 s | 100.0 |
| 3578 | ribo | 1 x 5 s | 08.6 | 1 x 250 s | 100.0 |
| 3529 | ribo | 1 x 5 s | 08.6 | 1 x 150 s | 73.7 |

Table 43: *NMR Data for 15-mer RNA containing Phosphorothioate Linkages*

| Synthesis # | Type | Delivery | Eq. | Wait | ASE (%) |
|---|---|---|---|---|---|
| 3581 | ribo | 1 x 5 s | 08.6 | 1 x 250 s | 99.6 |
| 3663 | ribo | 2 x 4 s | 13.8 | 2 x 300 s | 100.0 |
| 3582 | 2'-*O*-Me | 1 x 5 s | 08.6 | 1 x 250 s | 99.7 |
| 3668 | 2'-*O*-Me | 2 x 4 s | 13.8 | 2 x 300 s | 99.8 |
| 3682 | 2'-O-Me | 1 x 5 s | 08.6 | 1 x 300 s | 99.8 |

Table 44.

| Kinetics of Self-Processing *In Vitro* | |
|---|---|
| Self-Processing Constructs | k ($min^{-1}$)* |
| HH | 1.16 ± 0.08 |
| HDV | 0.56 ± 0.15 |
| HP(GC) | 0.36 ± 0.06 |
| HP(GU) | 0.054 ± 0.003 |

* k represents the unimolecular rate constant for ribozyme self-cleavage determined from a non-linear, least-squares fit (KaleidaGraph, Synergy Software, Reeding, PA) to the equation:

$$\text{(Fraction Uricleaved Transcript)} = \frac{1}{kt}(1-e^{-kt})$$

The equation describes the extent of ribozyme processing in the presense of ongoing transcription (Long & Uhlenbeck, 1994 Proc. Natl. Acad. Sci. USA 91, 6977) as a function of time (t) and the unimolecular rate constant for cleavage (k). Each value of k represents the average (± range) of values determined from two experiments.

Table 45

| Entry | Modification | $t_{1/2}$ (m) Activity ($t_A$) | $t_{1/2}$ (m) Stability ($t_S$) | $\beta = t_S/t_A \times 10$ |
|---|---|---|---|---|
| 1 | U4 & U7 = U | 1 | 0.1 | 1 |
| 2 | U4 & U7 = 2'-*O*-Me-U | 4 | 260 | 650 |
| 3 | U4 = 2'=$CH_2$-U | 6.5 | 120 | 180 |
| 4 | U7 = 2'=$CH_2$-U | 8 | 280 | 350 |
| 5 | U4 & U7 = 2'=$CH_2$-U | 9.5 | 120 | 130 |
| 6 | U4 = 2'=$CF_2$-U | 5 | 320 | 640 |
| 7 | U7 = 2'=$CF_2$-U | 4 | 220 | 550 |
| 8 | U4 & U7 = 2'=$CF_2$-U | 20 | 320 | 160 |
| 9 | U4 = 2'-F-U | 4 | 320 | 800 |
| 10 | U7 = 2'-F-U | 8 | 400 | 500 |
| 11 | U4 & U7 = 2'-F-U | 4 | 300 | 750 |
| 12 | U4 = 2'-C-Allyl-U | 3 | >500 | >1700 |
| 13 | U7 = 2'-C-Allyl-U | 3 | 220 | 730 |
| 14 | U4 & U7 = 2'-C-Allyl-U | 3 | 120 | 400 |
| 15 | U4 = 2'-araF-U | 5 | >500 | >1000 |
| 16 | U7 = 2'-araF-U | 4 | 350 | 875 |
| 17 | U4 & U7 = 2'-araF-U | 15 | 500 | 330 |
| 18 | U4 = 2'-$NH_2$-U | 10 | 500 | 500 |
| 19 | U7 = 2'-$NH_2$-U | 5 | 500 | 1000 |
| 20 | U4 & U7 = 2'-$NH_2$-U | 2 | 300 | 1500 |
| 21 | U4 = dU | 6 | 100 | 170 |
| 22 | U4 & U7 = dU | 4 | 240 | 600 |

## Claims

1. An enzymatic nucleic acid molecule comprising a nucleotide selected from the group consisting of 5'-C-alkylnucleotide, 2'-deoxy-2'-alkylnucleotide, 5'-deoxy-5'-dihalo-methylnucleotide, 5'-deoxy-5'-difluoro-methylnucleotide, 3'-deoxy-3'-dihalo-methylnucleotide, and 5',3'-dideoxy-5', 3'-bis(dihalo)-methylphosphonate.

2. Method for producing an enzymatic nucleic acid molecule having activity to cleave an RNA or single-stranded DNA molecule, comprising the step of forming said enzymatic molecule with at least one nucleotide having an alkyl group at its 5'-position or 2'-position.

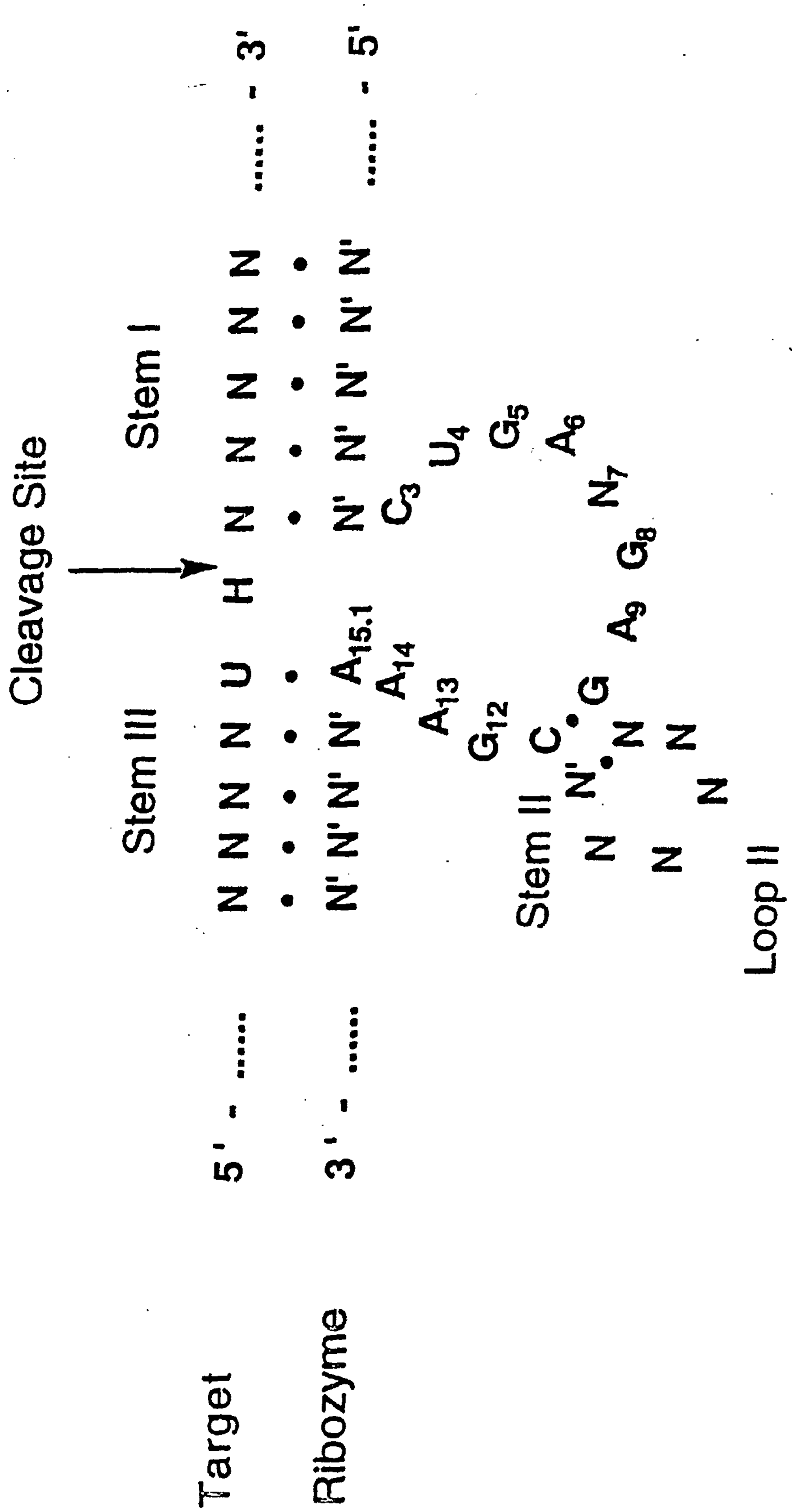
Cleavage Site
Target
Ribozyme
5' - ......
...... - 3'
3' - ......
...... - 5'
Stem I
Stem II
Stem III
Loop II

FIG. 1.

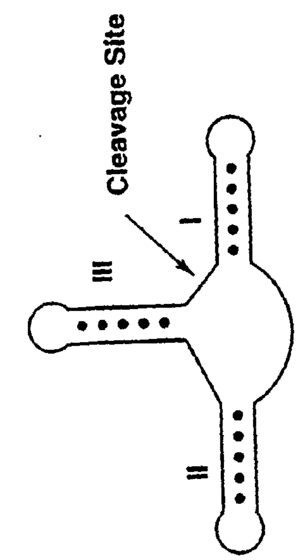


a

FIG. 2a.

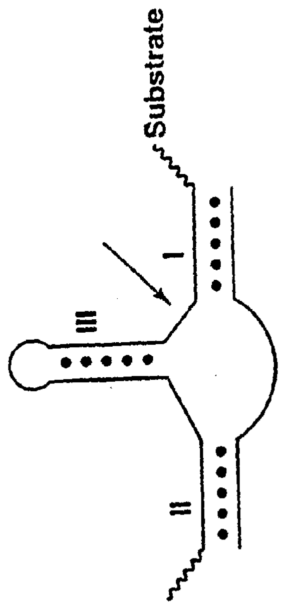


b

FIG. 2b.

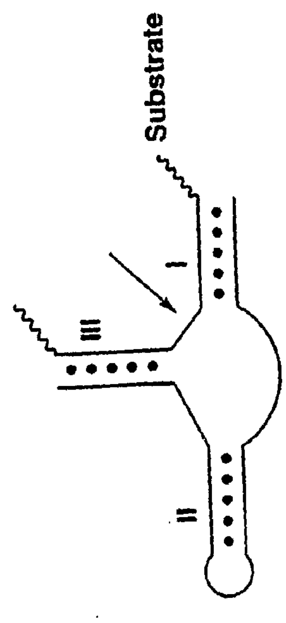


c

FIG. 2c.

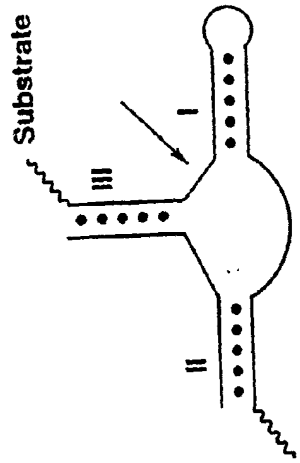


d

FIG. 2d.

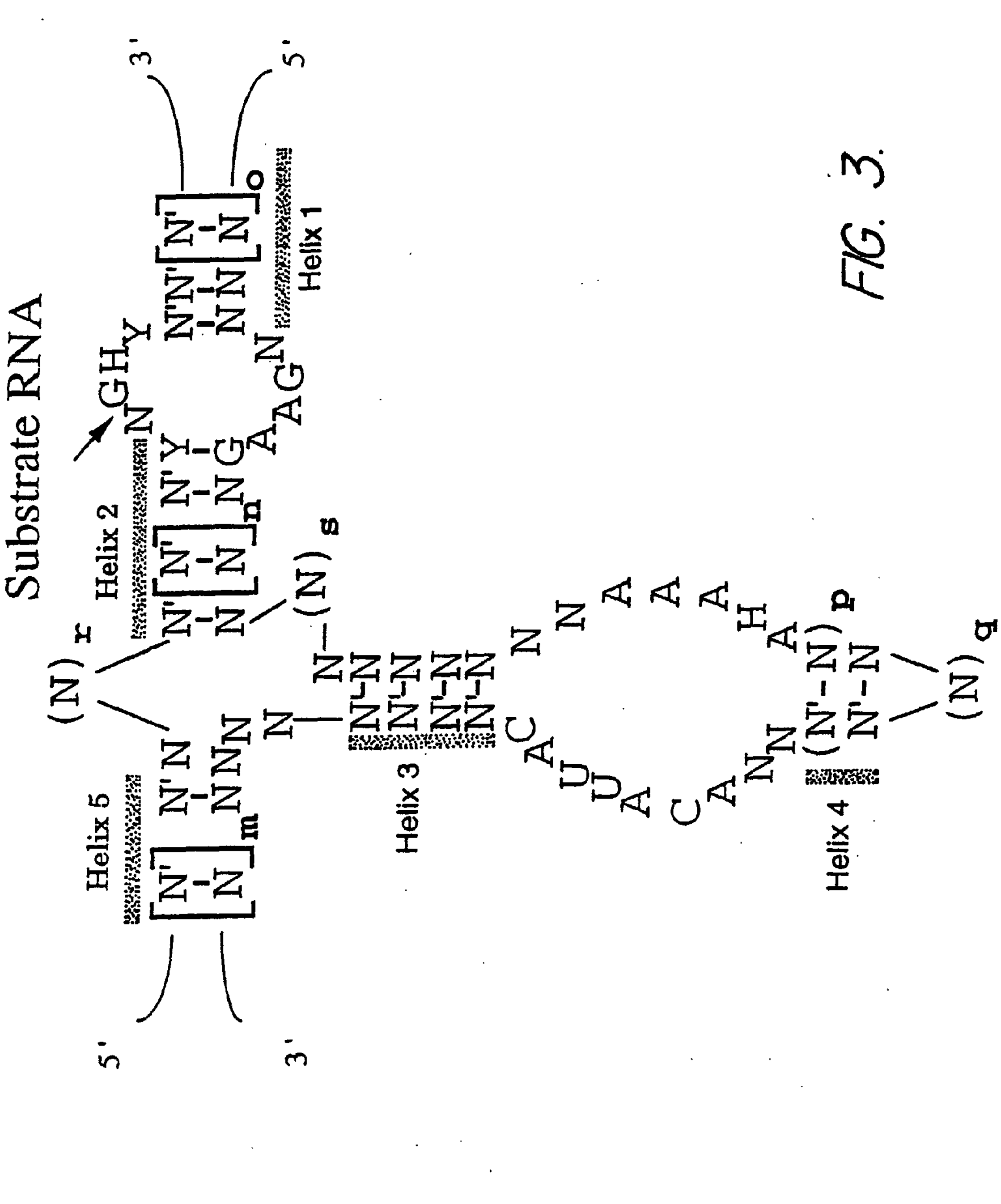


FIG. 3

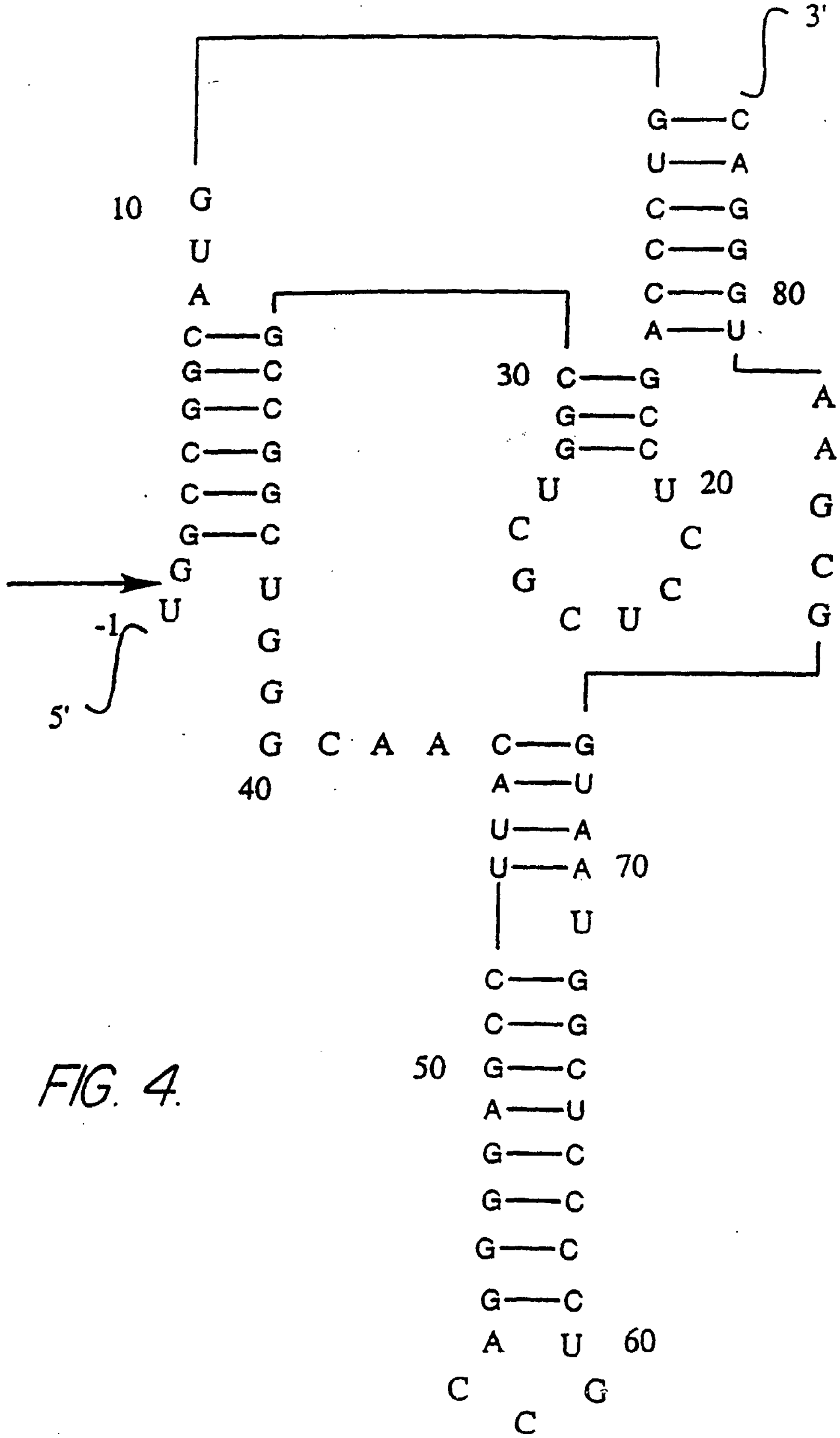


FIG. 4.

FIG. 5

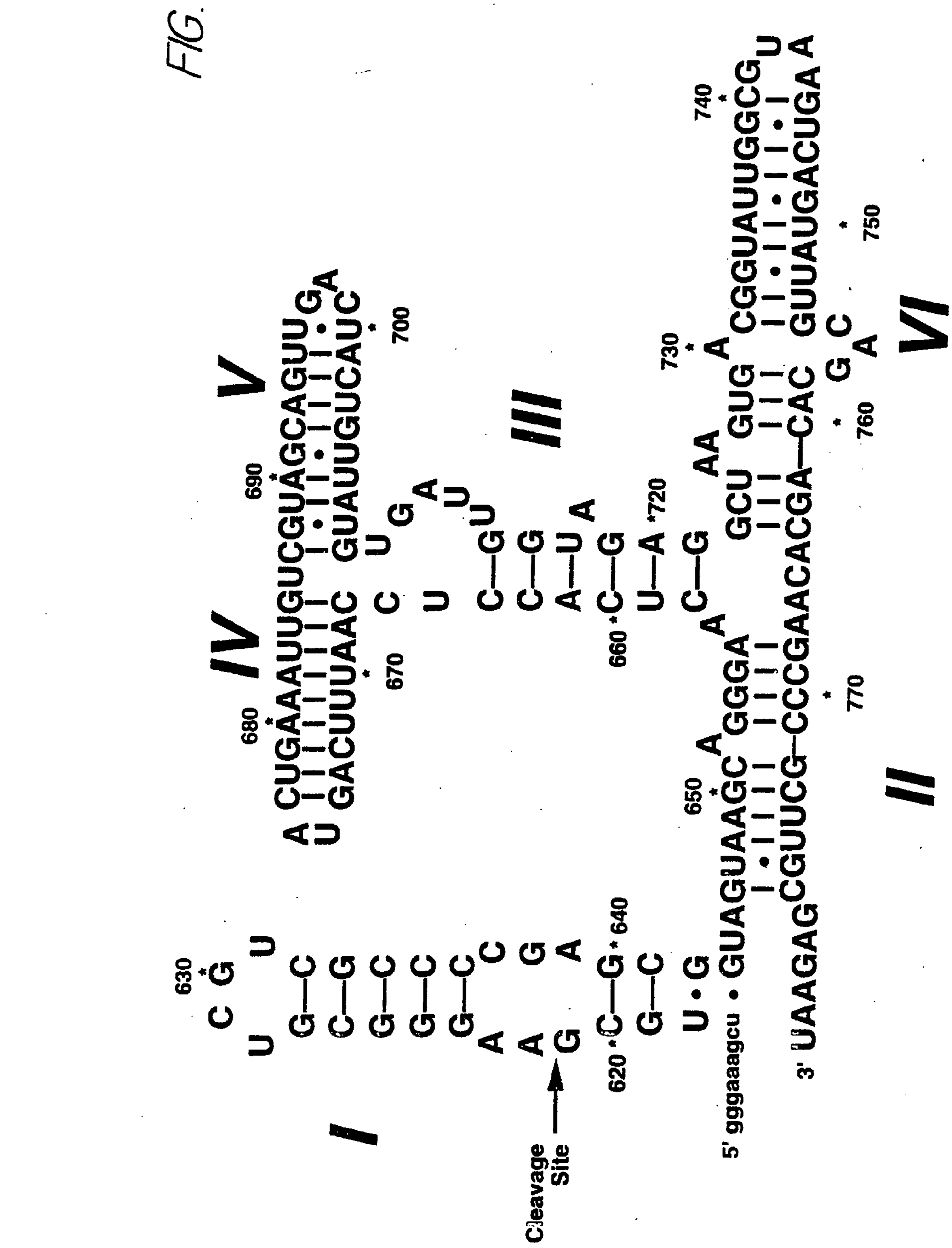

FIG. 6.

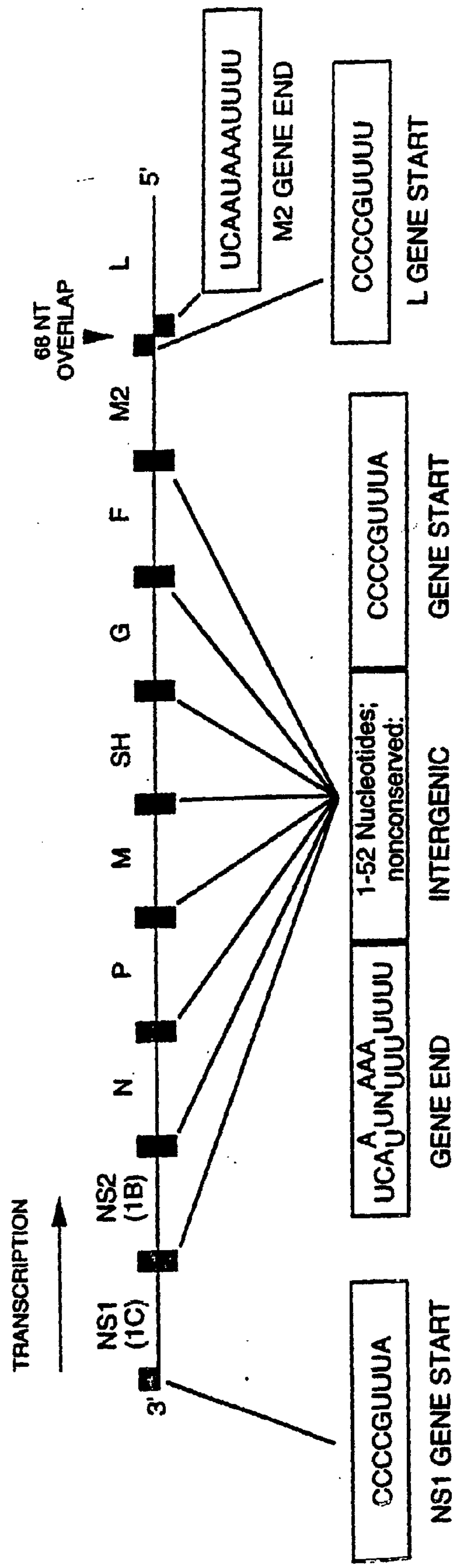


Adapted from Virology, Second Edition, Edited by B.N. Fields, 1990.

FIG. 7.

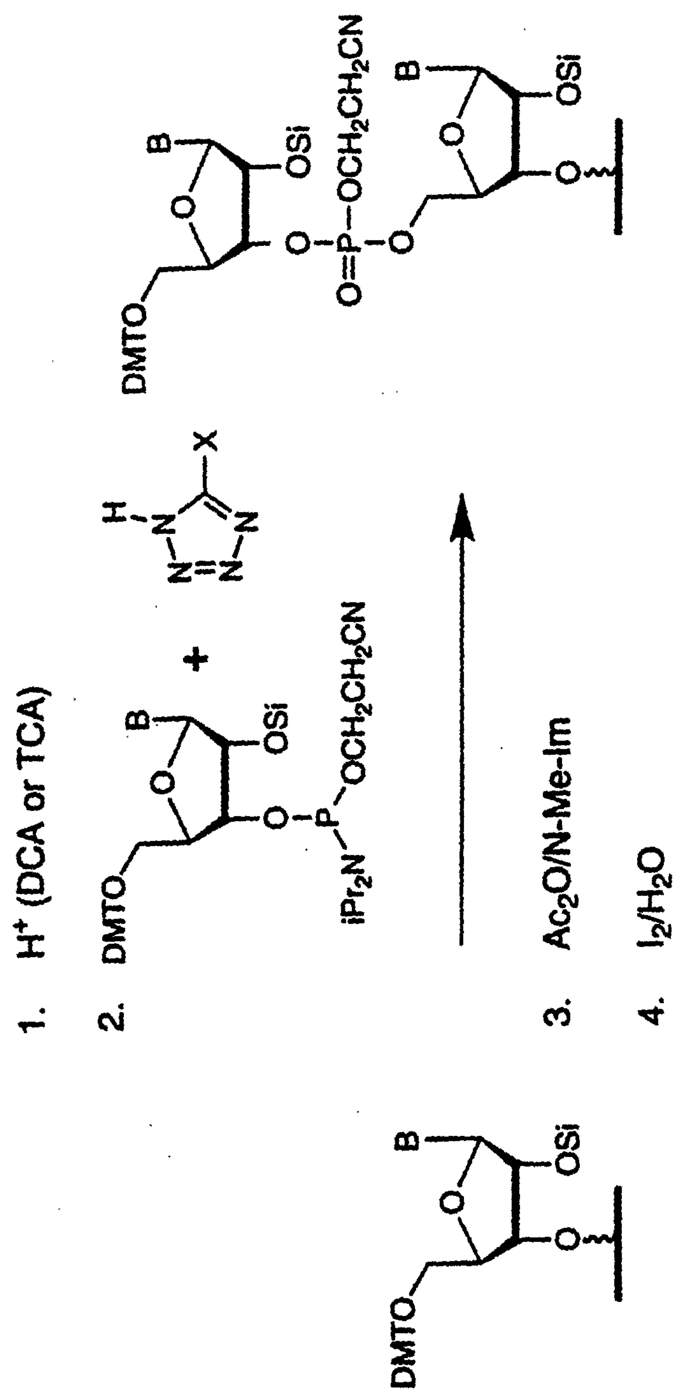

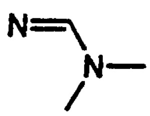

DMF

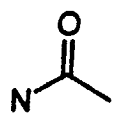

Ac

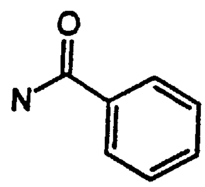

Bz

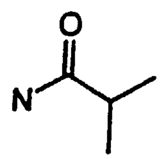

*i*-Bu

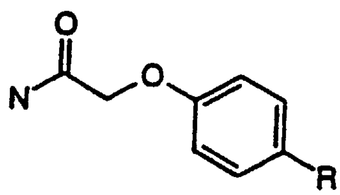

R = H = PAC

R = tBu = TAC

R = iPr = iPPAC

FIG. 8.

FIG. 9.

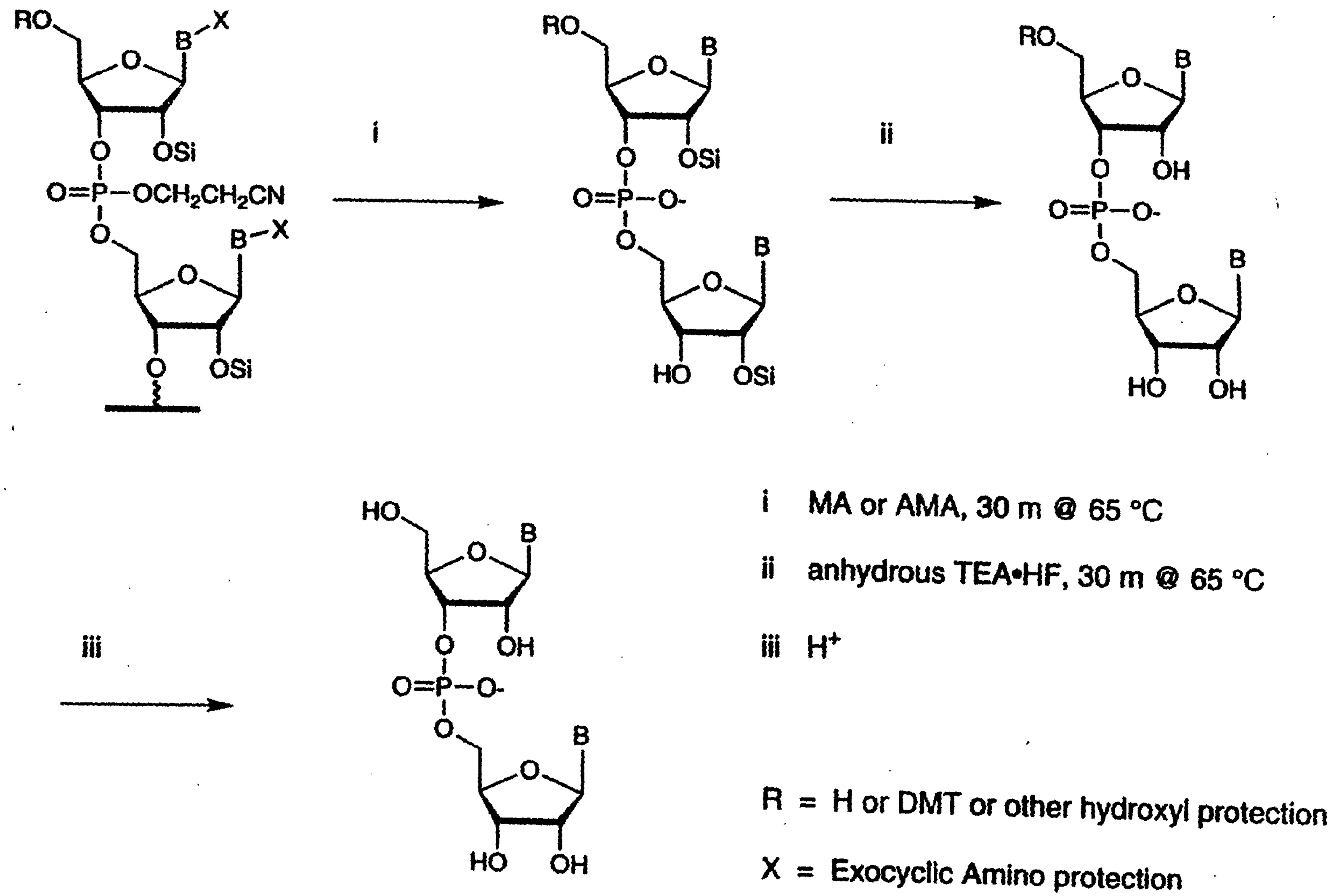
i
ii
iii
i MA or AMA, 30 m @ 65 °C
ii anhydrous TEA•HF, 30 m @ 65 °C
iii H+
R = H or DMT or other hydroxyl protection
X = Exocyclic Amino protection

FIG. 10.

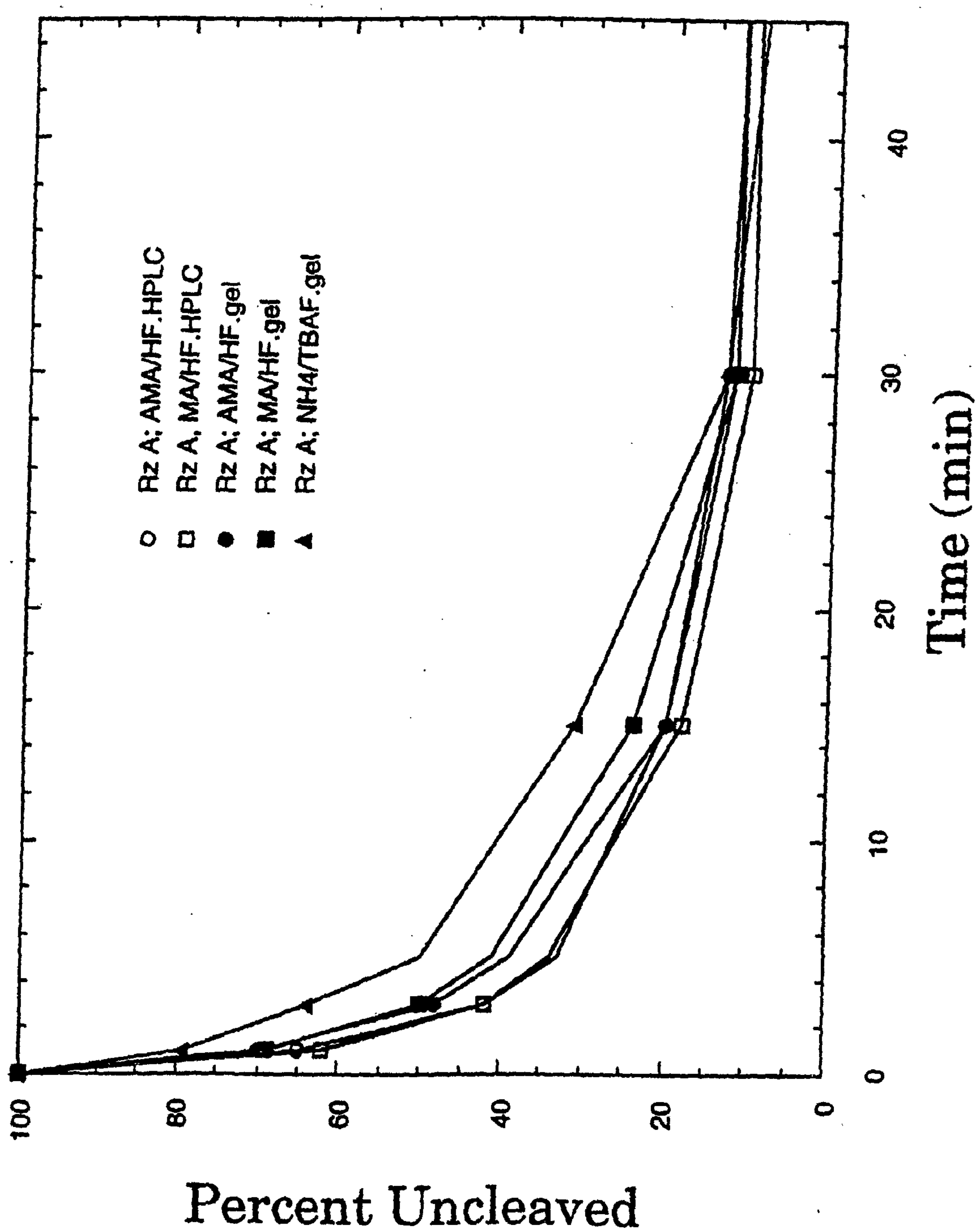
Rz A; AMA/HF.HPLC
Rz A, MA/HF.HPLC
Rz A; AMA/HF.gel
Rz A; MA/HF.gel
Rz A; NH4/TBAF.gel
Percent Uncleaved
100
80
60
40
20
0
Time (min)
0
10
20
30
40

FIG. 11.

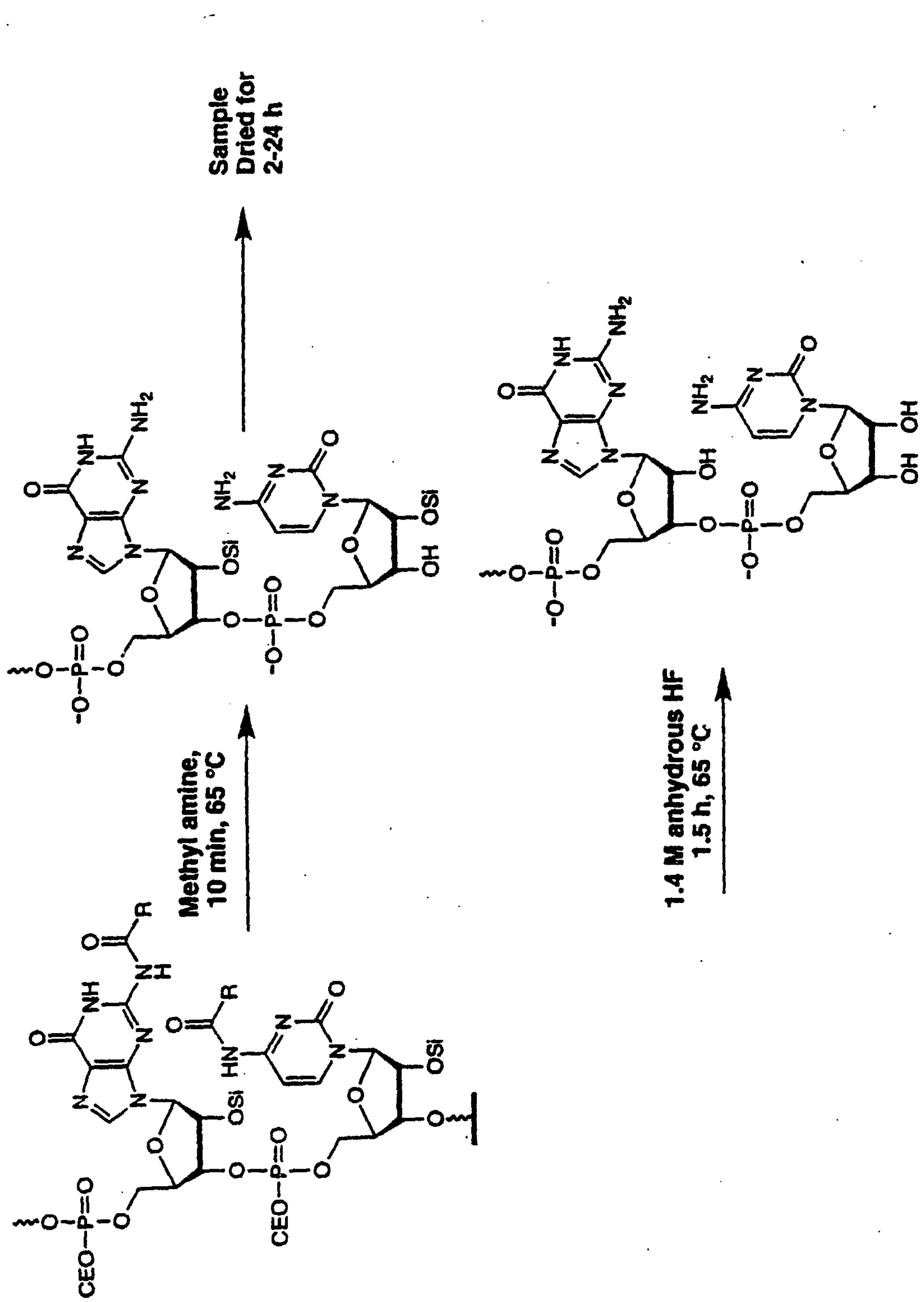
Methyl amine,
10 min, 65 °C
Sample
Dried for
2-24 h
1.4 M anhydrous HF
1.5 h, 65 °C

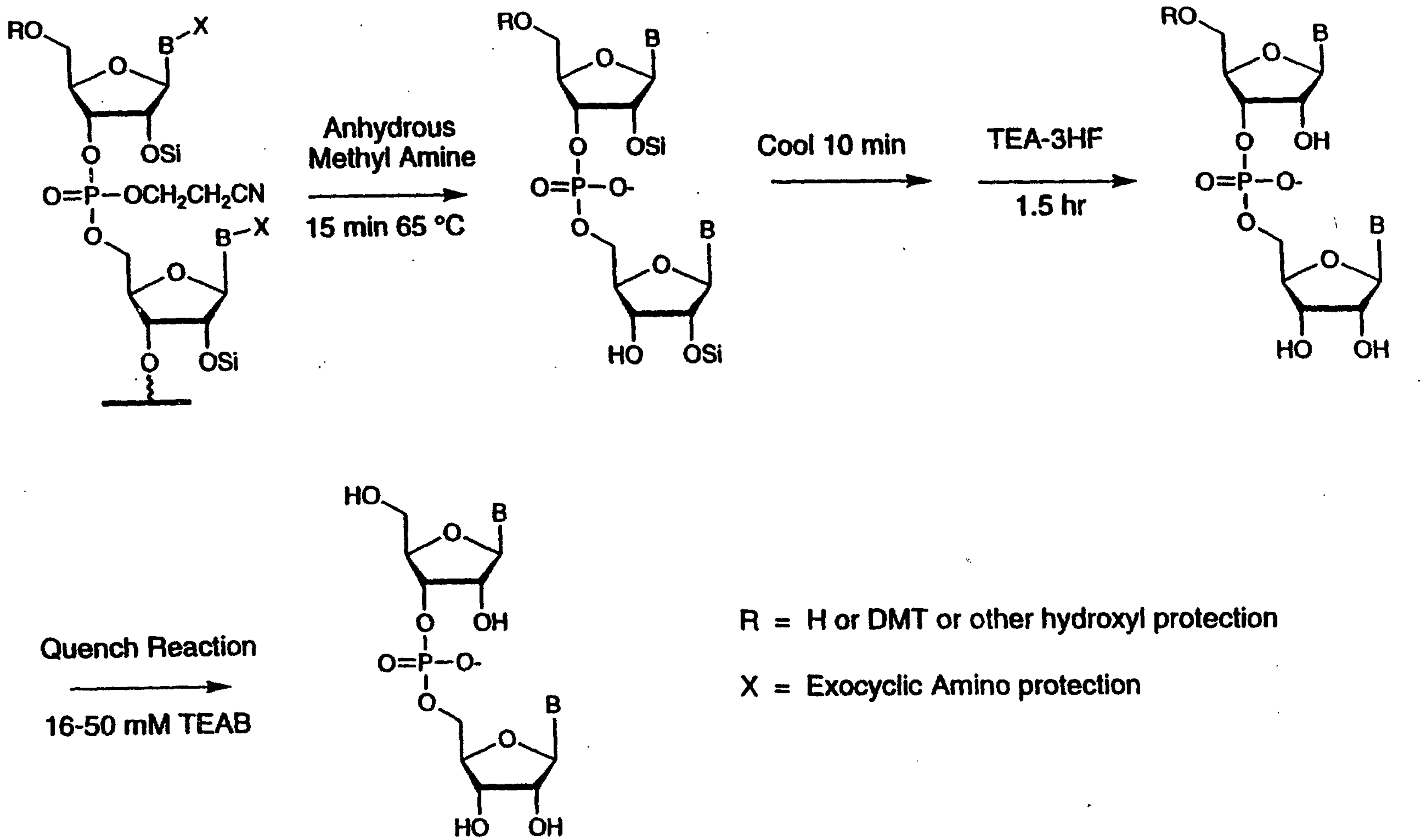


R = H or DMT or other hydroxyl protection

X = Exocyclic Amino protection

FIG. 12.

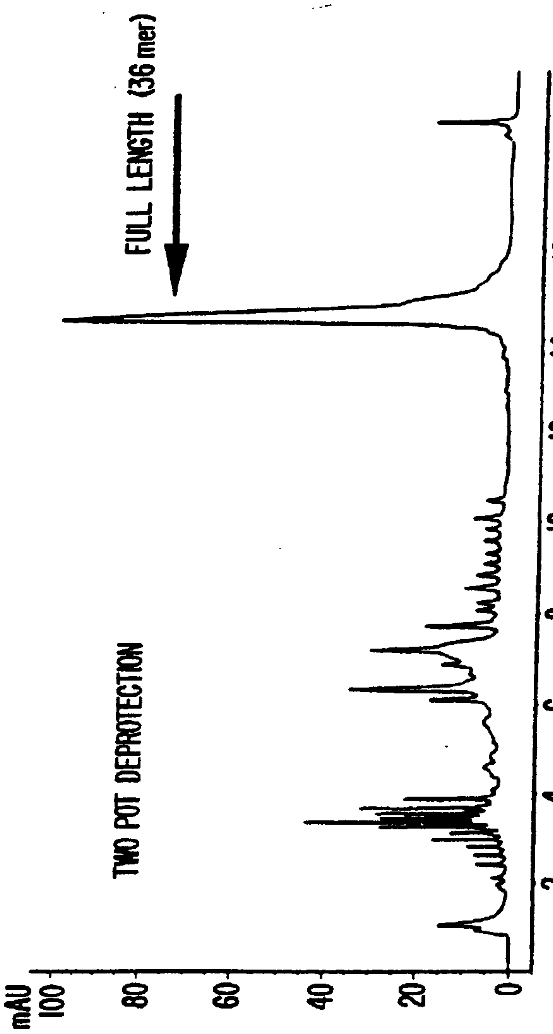


FIG. 13a.

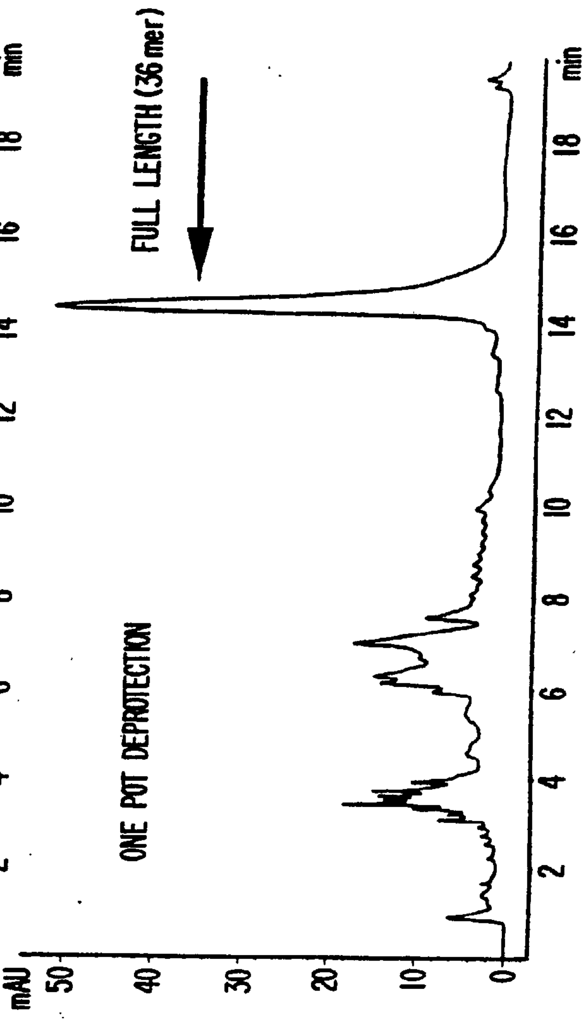


FIG. 13b.

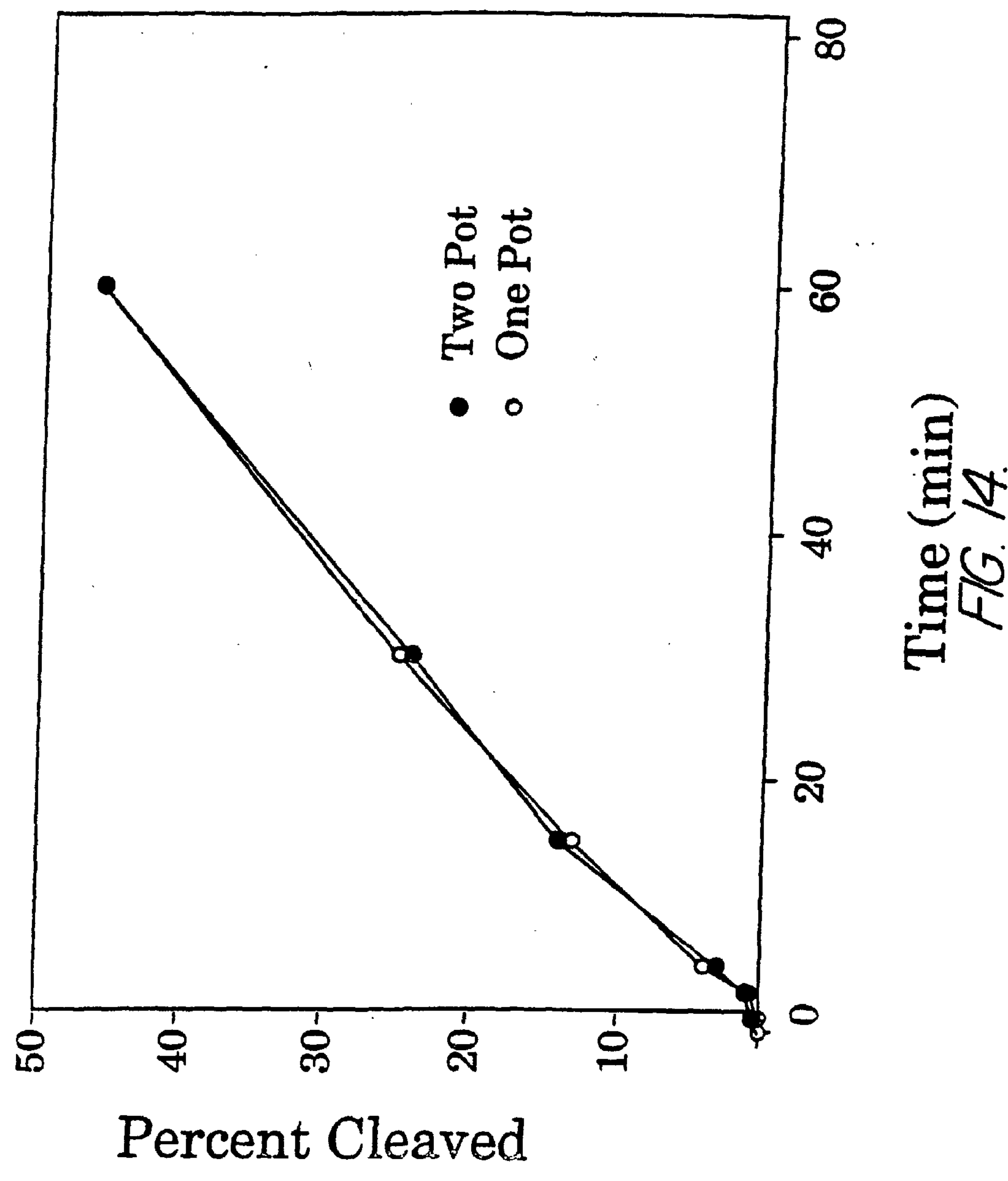
Two Pot
One Pot
Percent Cleaved
50
40
30
20
10
0
20
40
60
80
Time (min)

FIG. 14.

FIG. 15.

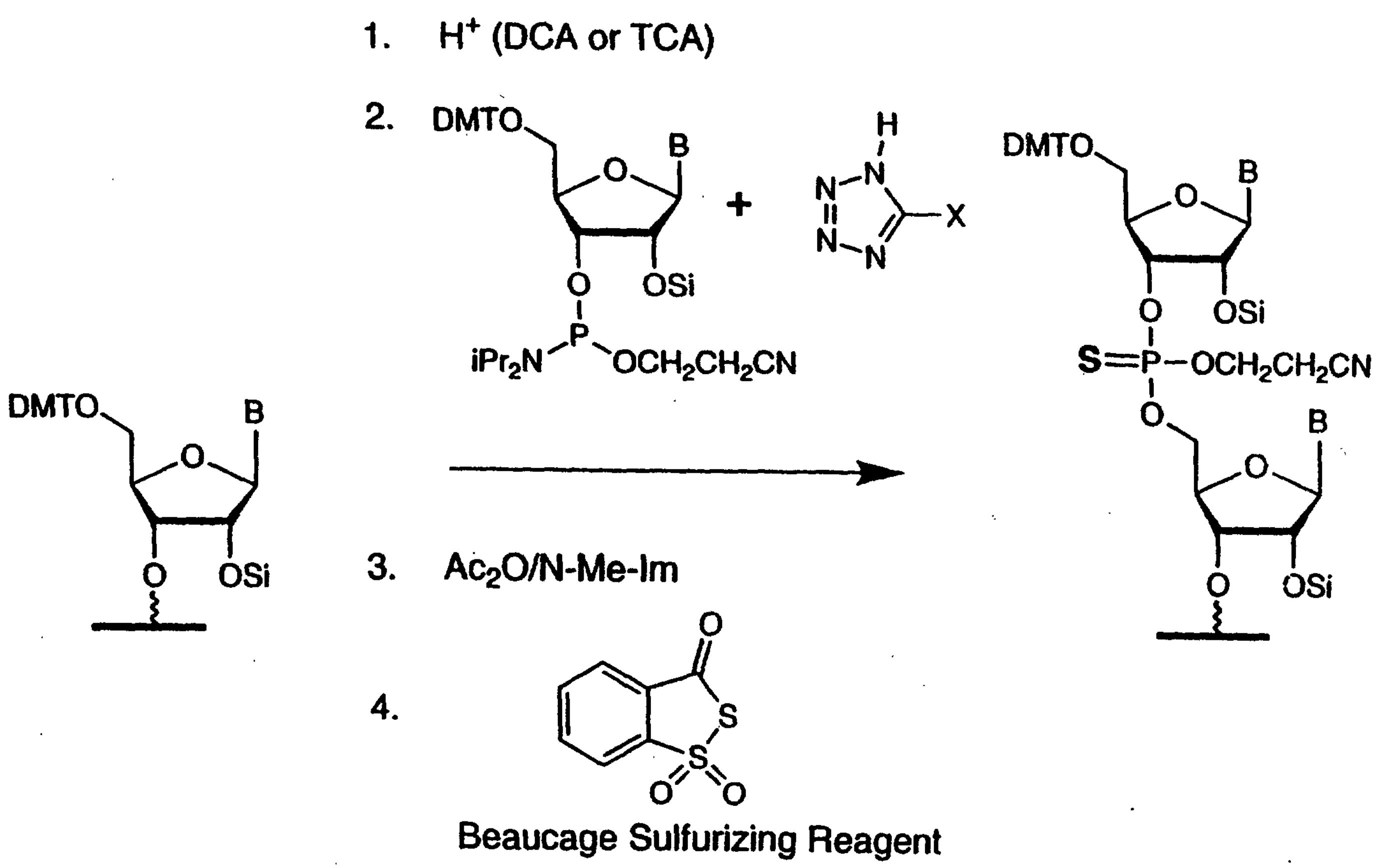

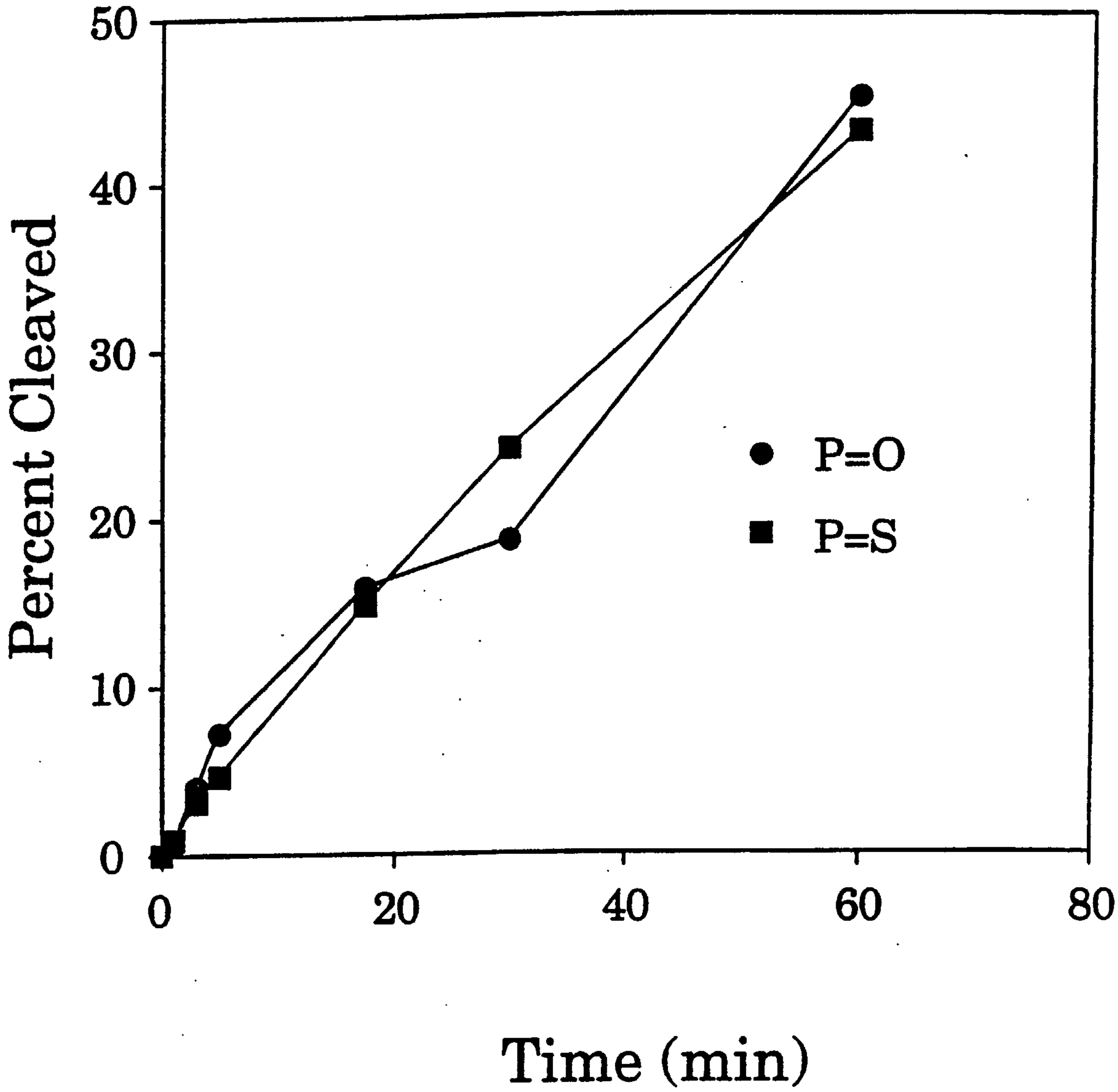


FIG. 16.

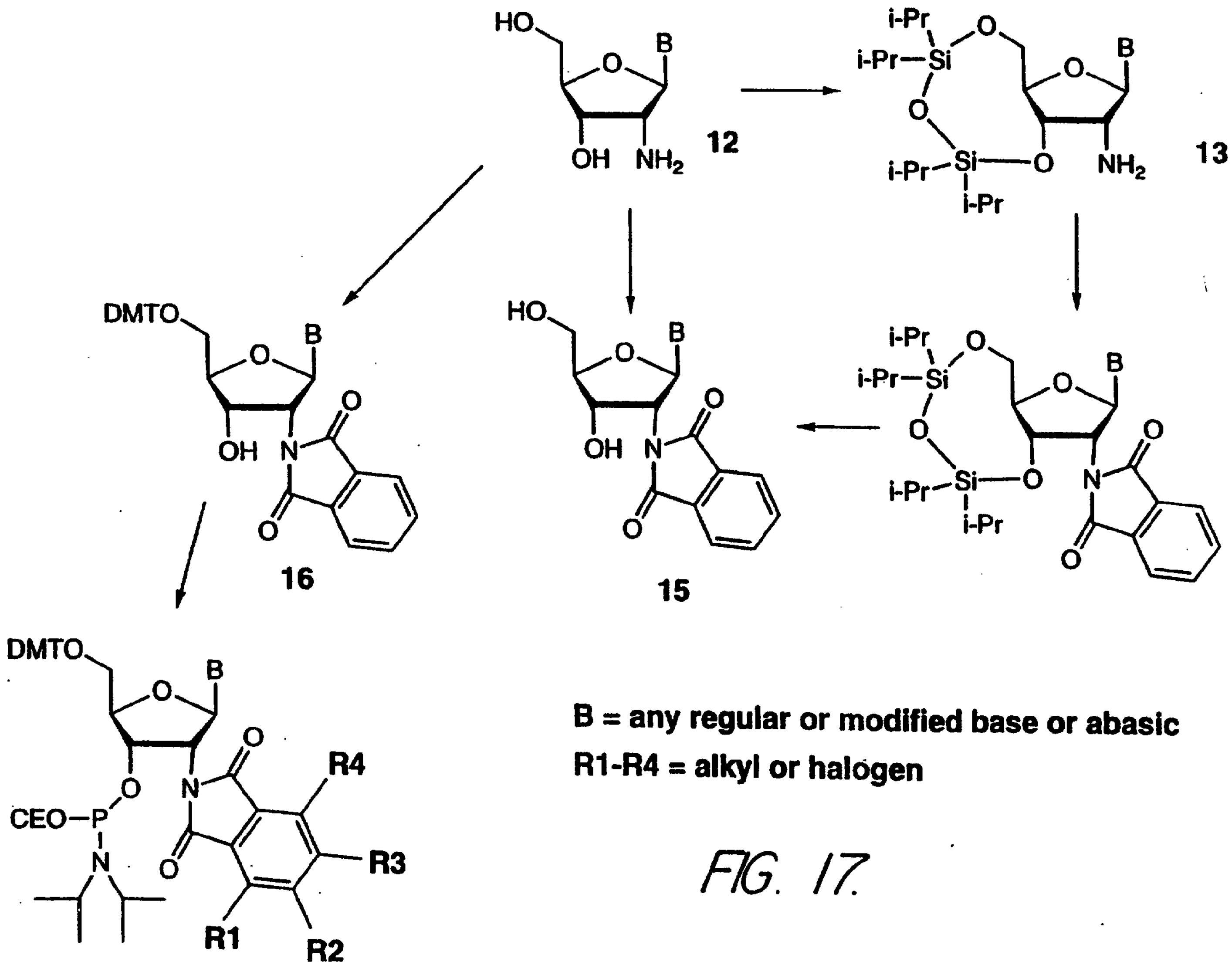

12

13

14

15

16

17

B = any regular or modified base or abasic

R1-R4 = alkyl or halogen

FIG. 17.

FIG. 18.

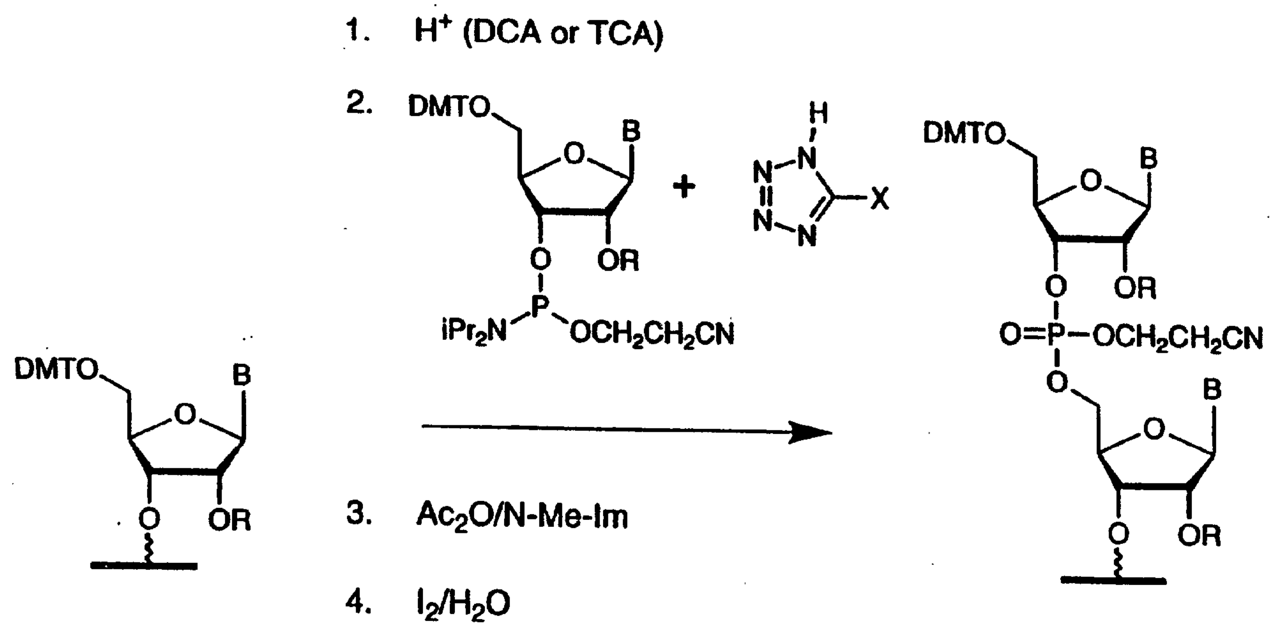


R = Silyl ether (prior art) 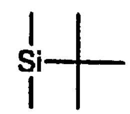

(trimethylsilyl)ethoxymethyl (SEM) 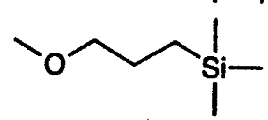

FIG. 19.

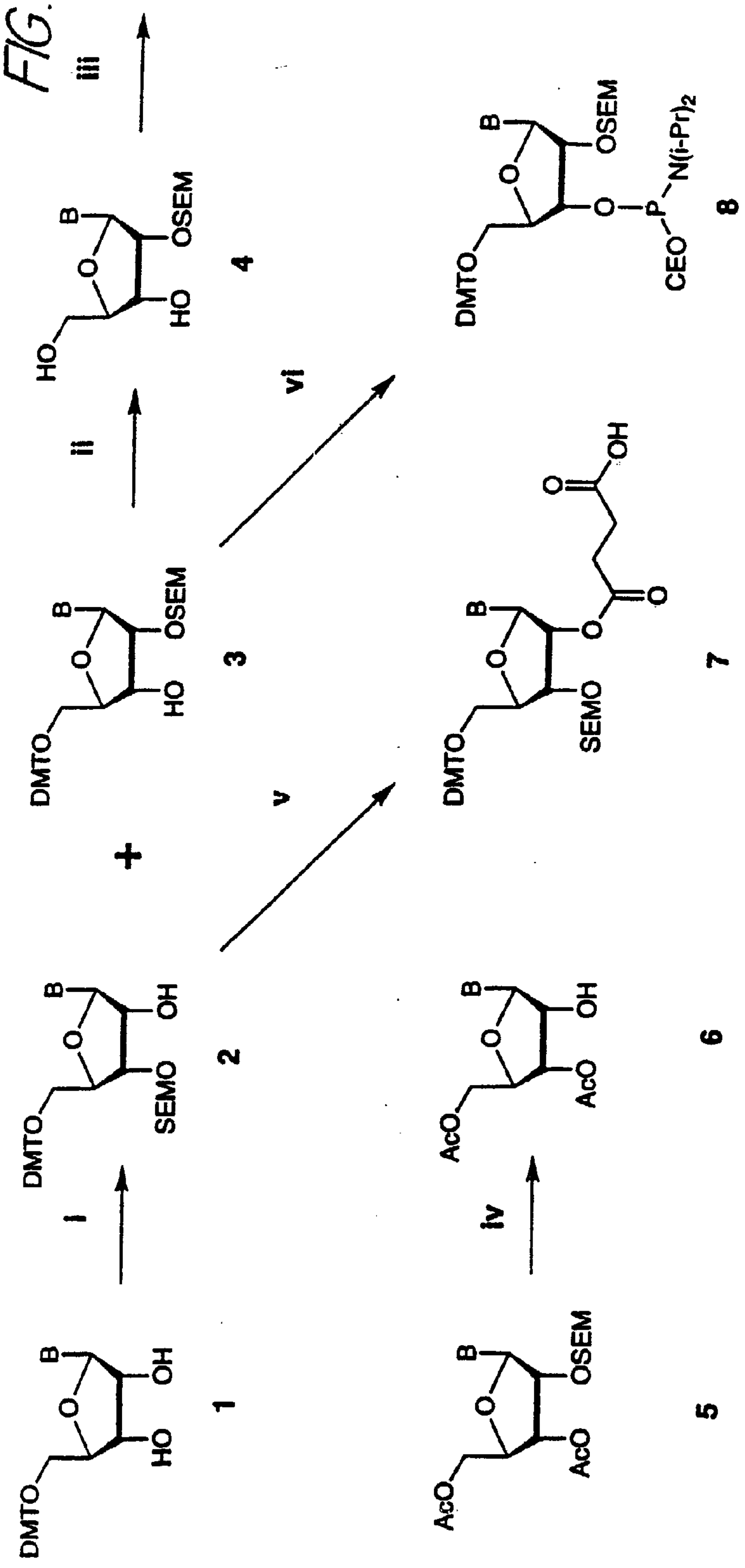


i) = $SnBu_2O$/SEM-Cl
ii) = $H^+$
iii) = $Ac_2O$
iv) = $BF_3 \bullet OEt_2$
v) = Succinic Anhydride
vi) = $P(OCE)(N\text{-}iPr_2)Cl$

B = Protected A, C, G, U, T, 2AP, I, DiAP, P *etc.*

SEM = (trimethylsilyl)ethoxymethyl

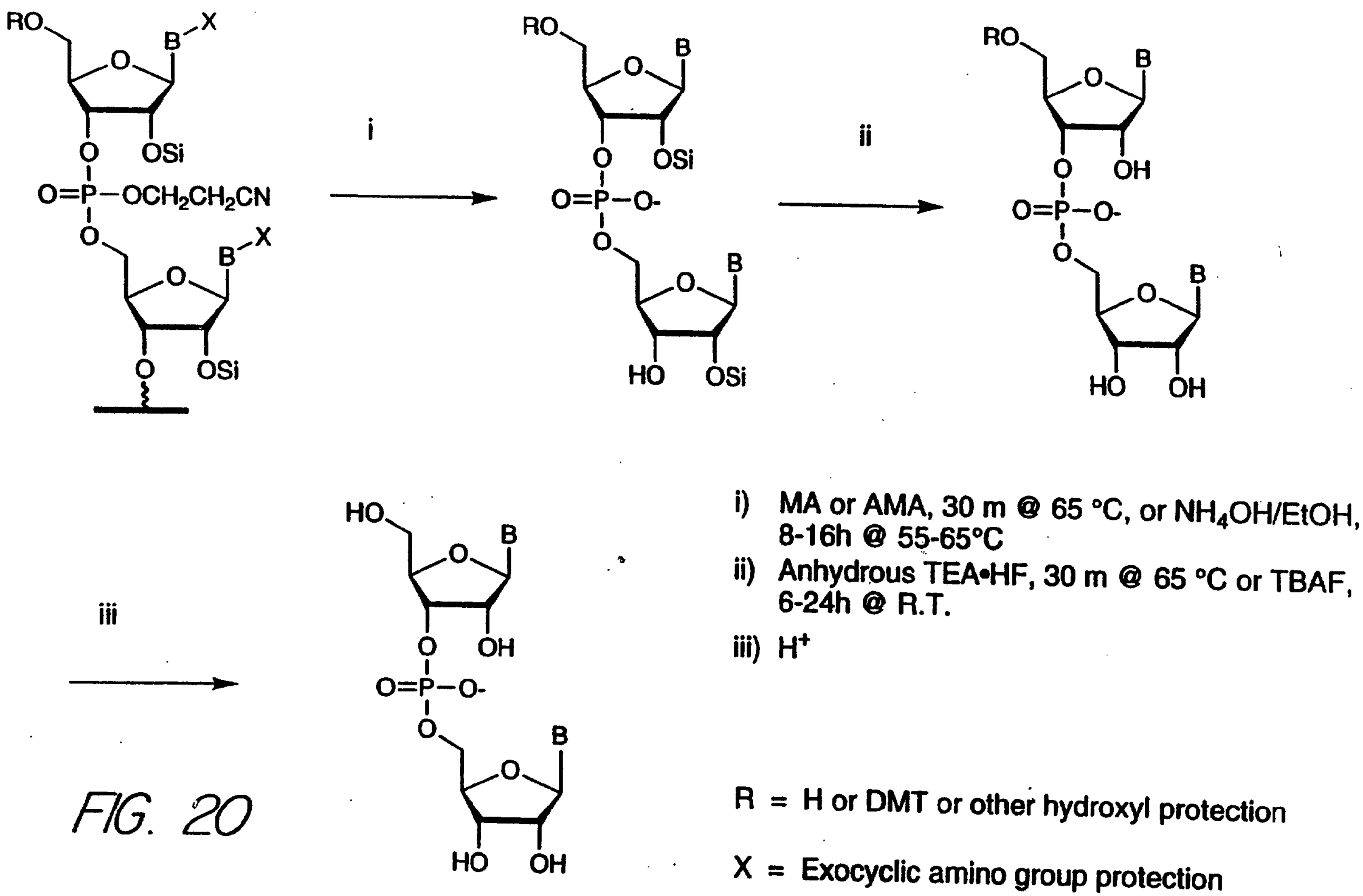


FIG. 20

i) MA or AMA, 30 m @ 65 °C, or $NH_4OH$/EtOH, 8-16h @ 55-65°C
ii) Anhydrous TEA•HF, 30 m @ 65 °C or TBAF, 6-24h @ R.T.
iii) $H^+$

R = H or DMT or other hydroxyl protection

X = Exocyclic amino group protection

FIG. 21.

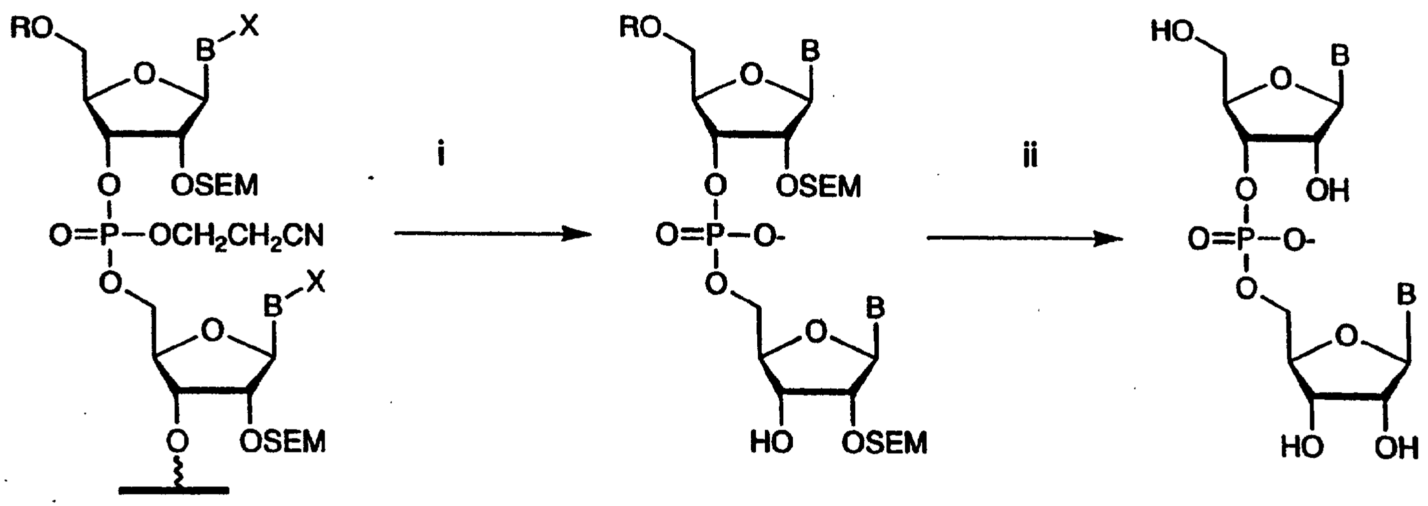


i) MA or AMA, 30 m @ 65 °C or $NH_4OH$ or $NH_4OH$/EtOH, 8-16h @ 55-65°C

ii) $BF_3 \bullet OEt_2$

SEM = (trimethylsilyl)ethoxymethyl

R = H or DMT or other hydroxyl protection

X = Exocyclic amino group protection

FIG. 22.

2'-0-SEM PROTECTED U 10-mer
DEPROTECTED WITH $BF_3 \cdot OEt_2$
30m, 3eq./nucleotide

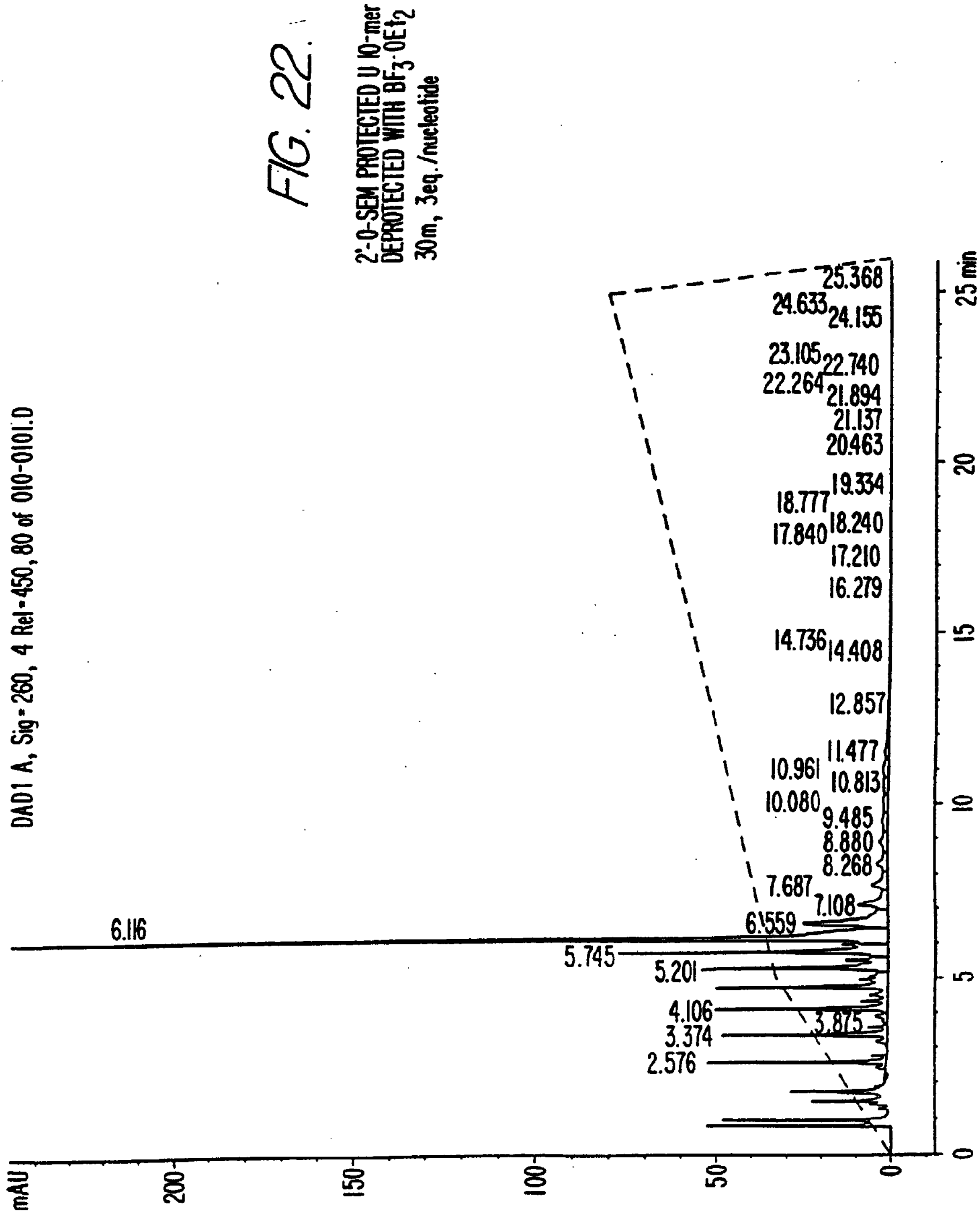

FIG. 23.

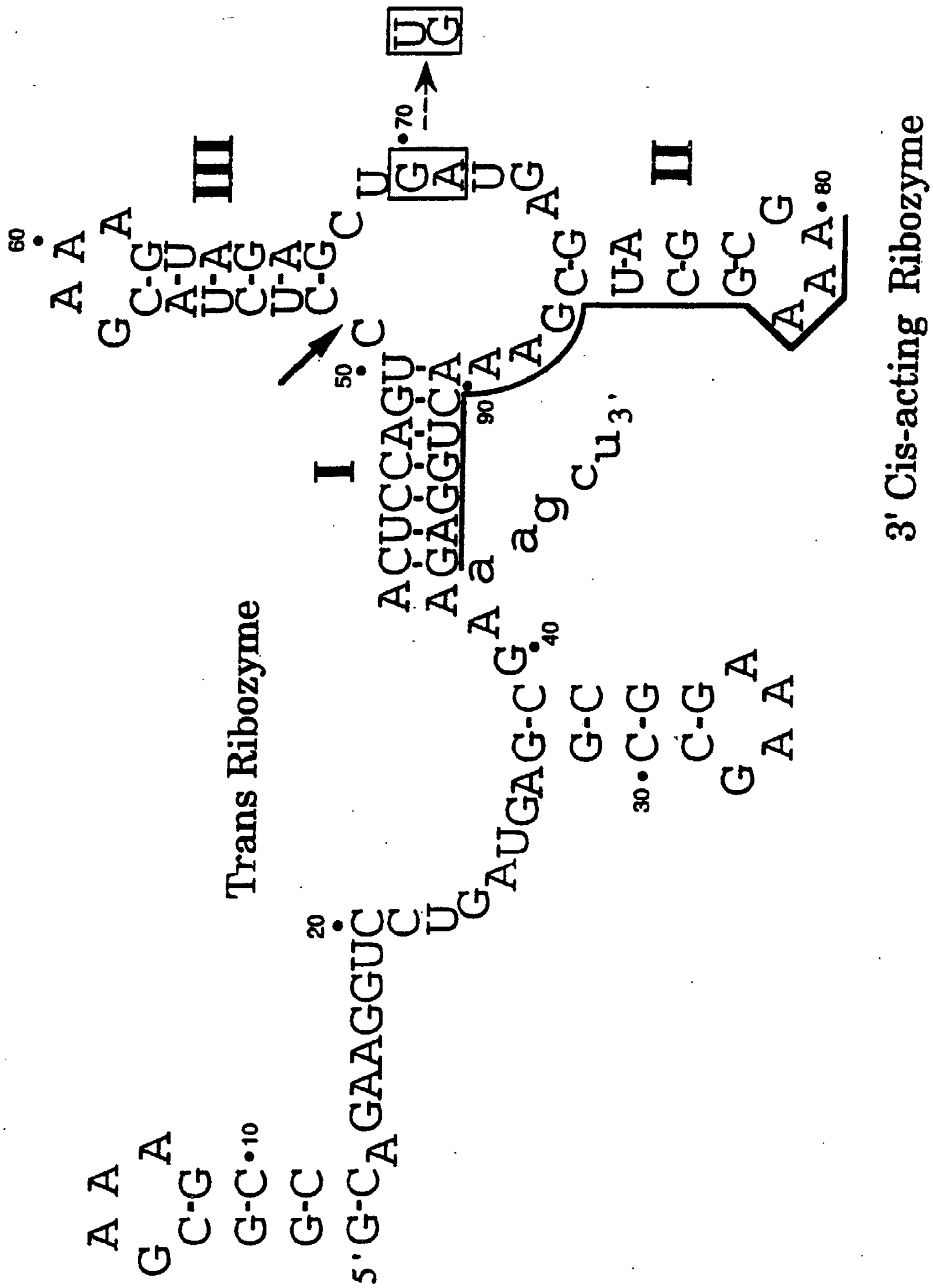

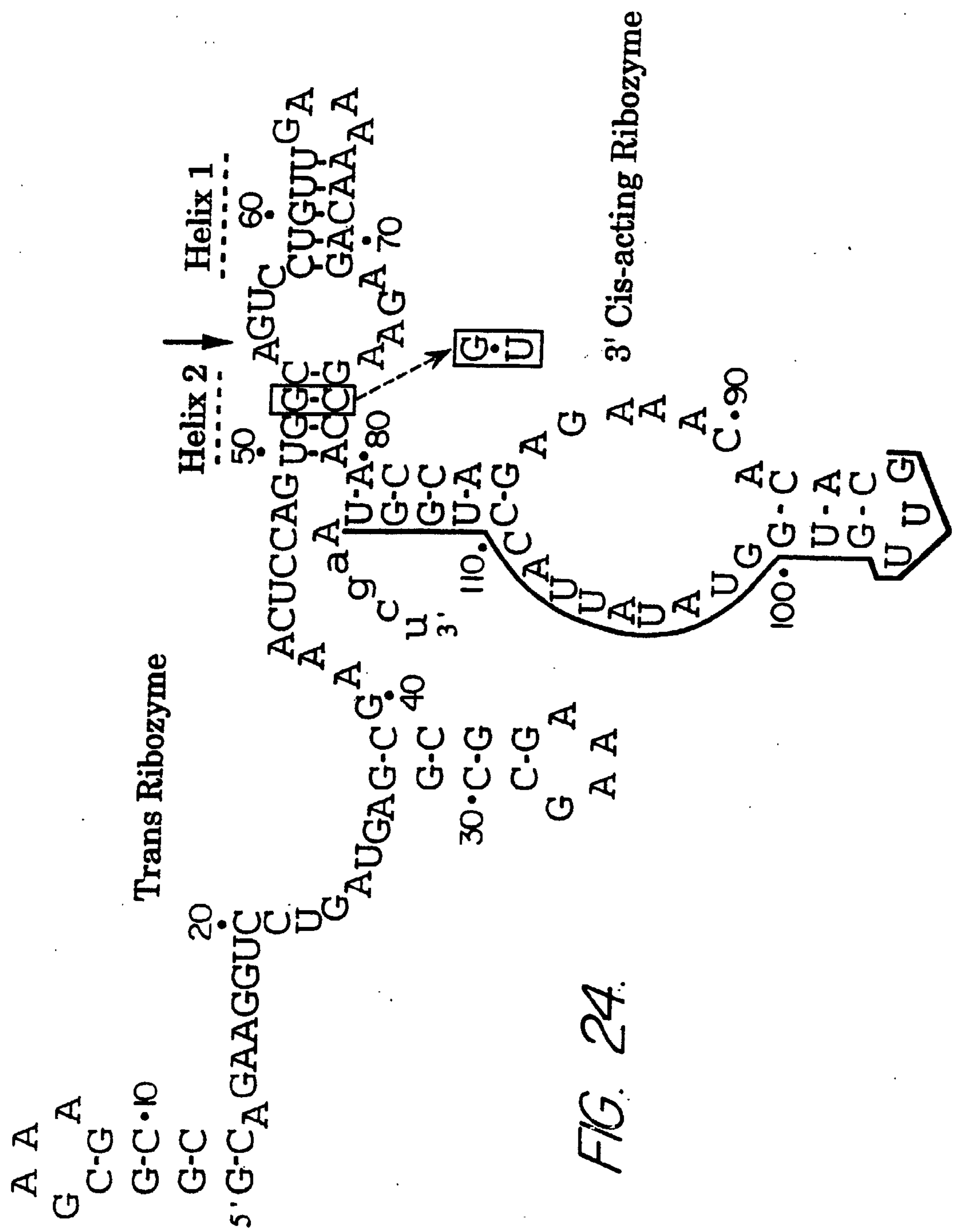


FIG. 24.

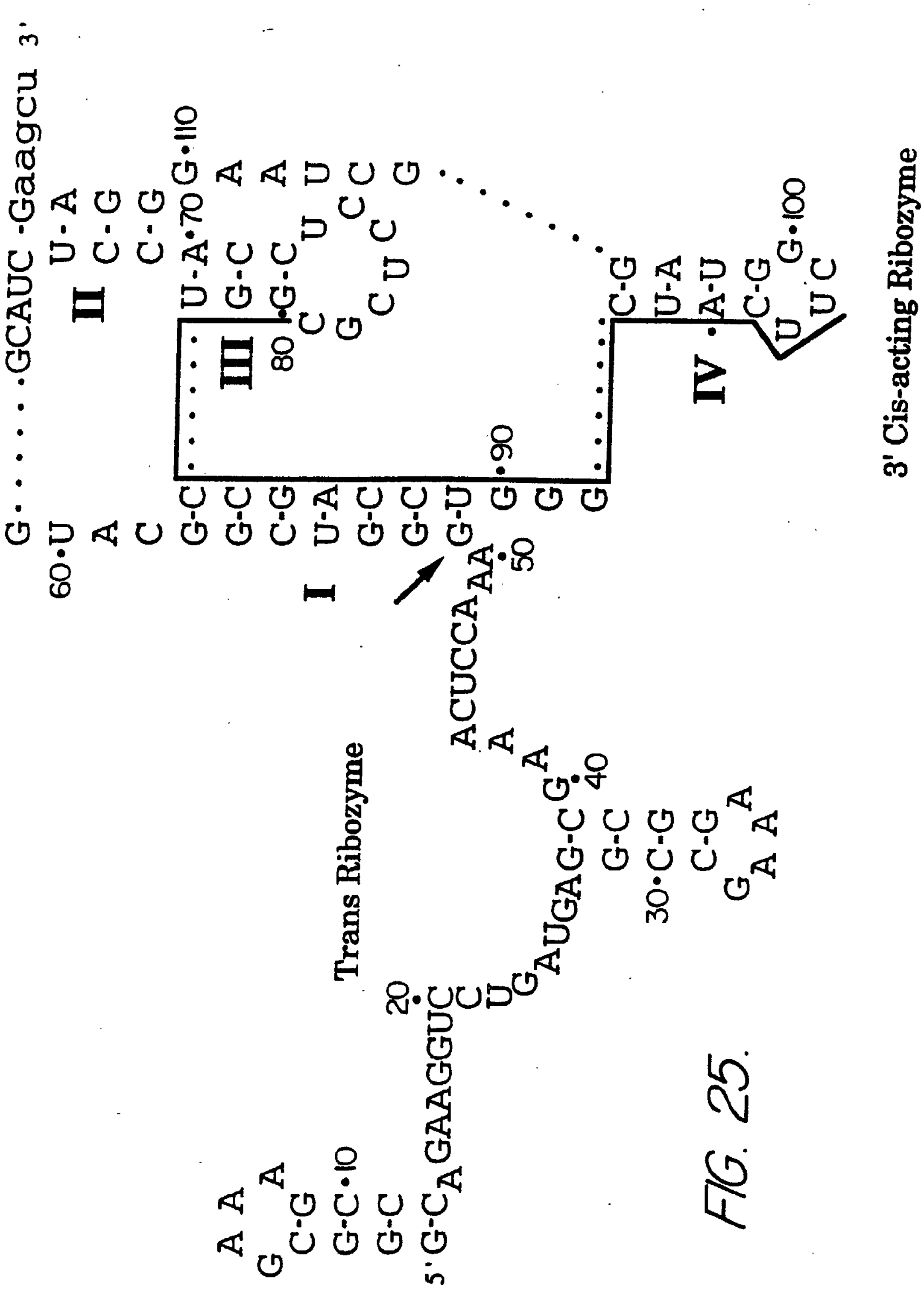


FIG. 25.

FIG. 26.

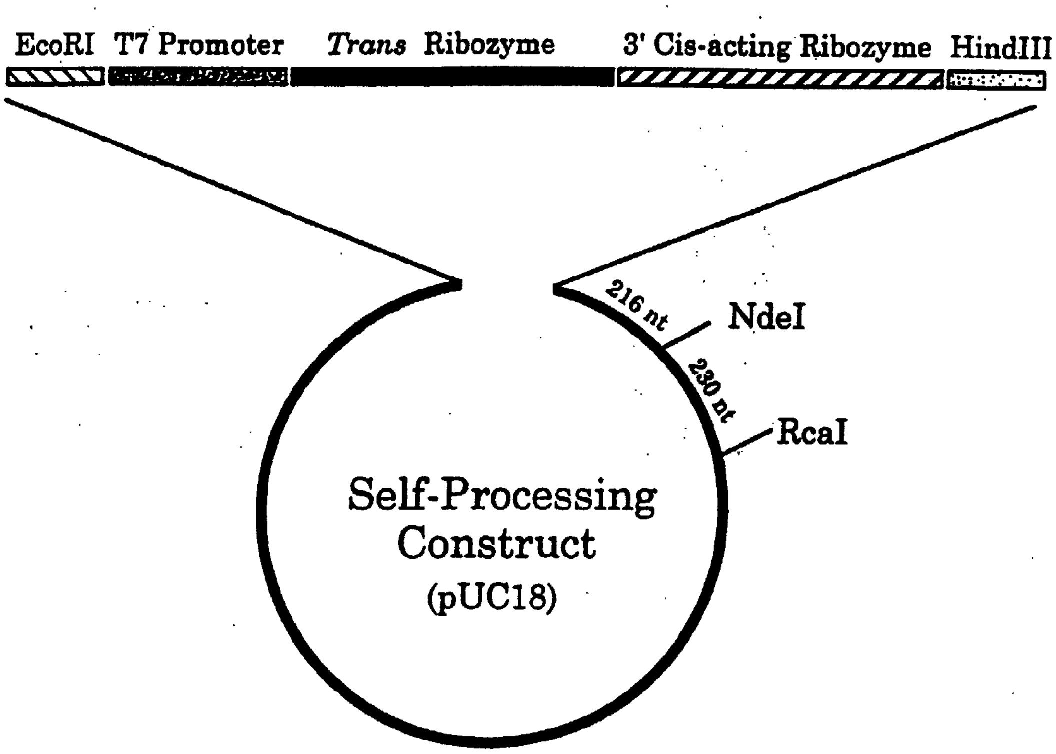
EcoRI
T7 Promoter
Trans Ribozyme
3' Cis-acting Ribozyme
HindIII
216 nt
NdeI
230 nt
RcaI
Self-Processing
Construct
(pUC18)

FIG. 27

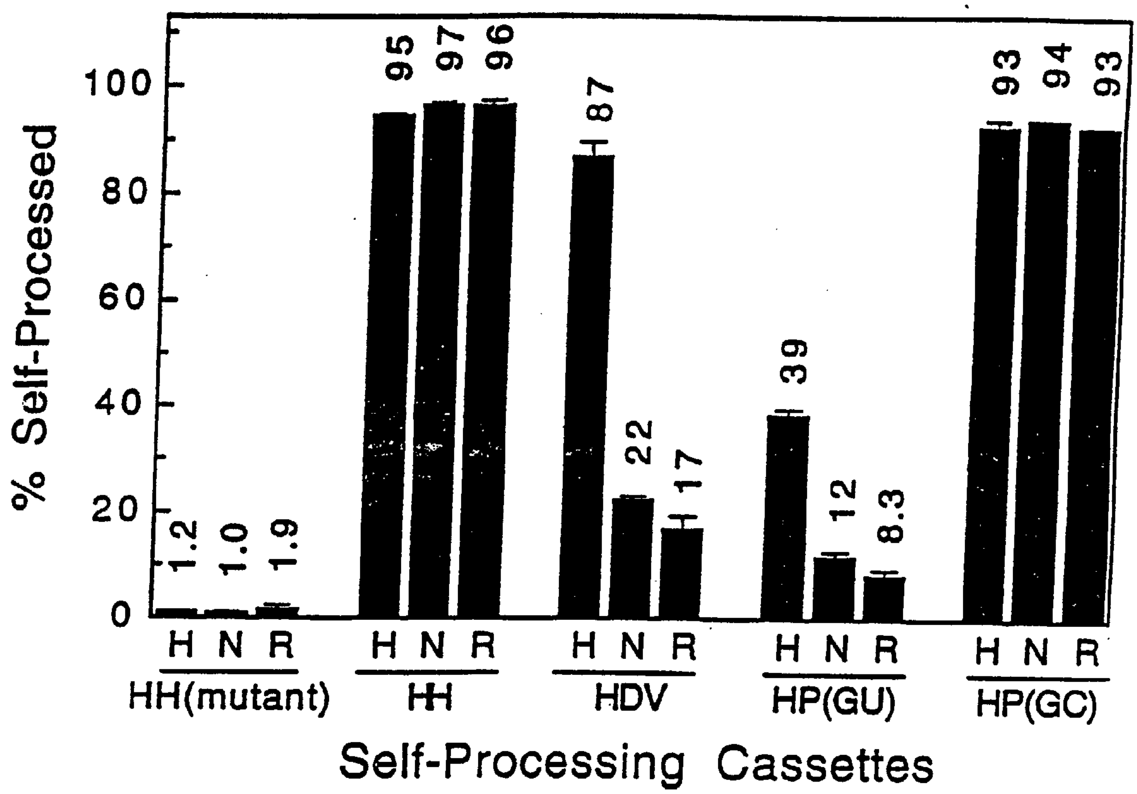
% Self-Processed
100
80
60
40
20
0
1.2
1.0
1.9
95
97
96
87
22
17
39
12
8.3
93
94
93
H N R
HH(mutant)
H N R
HH
H N R
HDV
H N R
HP(GU)
H N R
HP(GC)
Self-Processing Cassettes

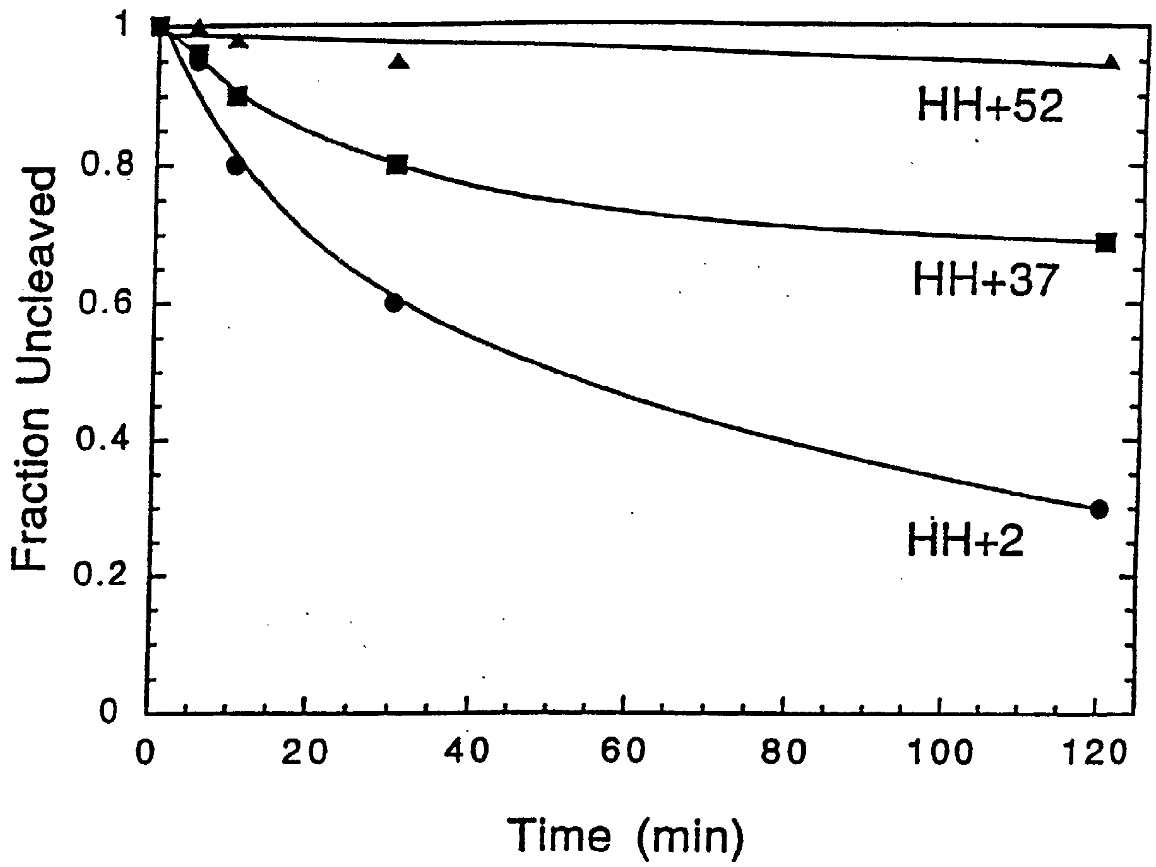

1
0.8
0.6
0.4
0.2
0
Fraction Uncleaved
HH+52
HH+37
HH+2
0
20
40
60
80
100
120
Time (min)


FIG. 28.

FIG. 29.

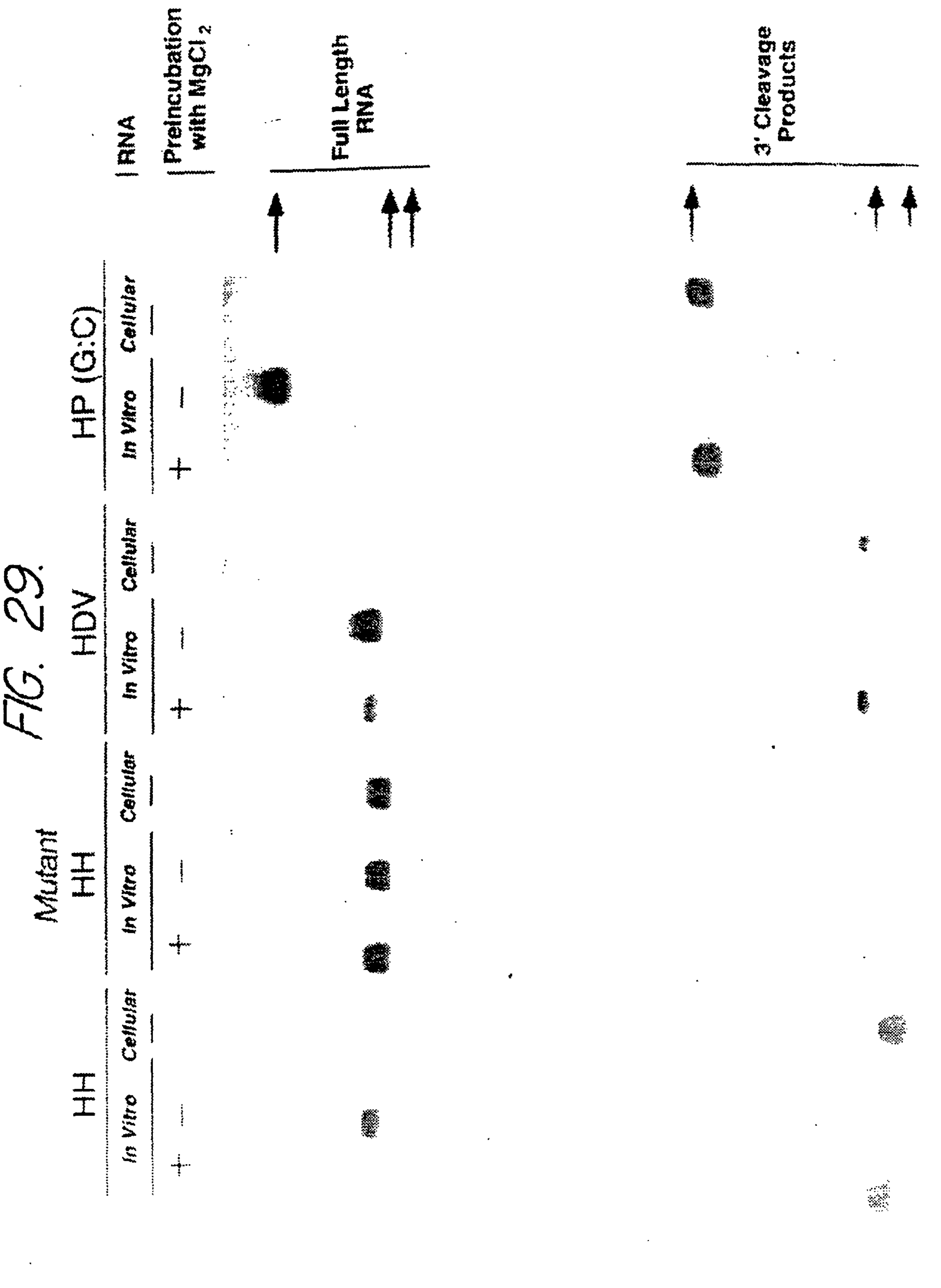
HH
Mutant HH
HDV
HP (G:C)
In Vitro
Cellular
RNA
Preincubation with $MgCl_2$
+
–
Full Length RNA
3' Cleavage Products

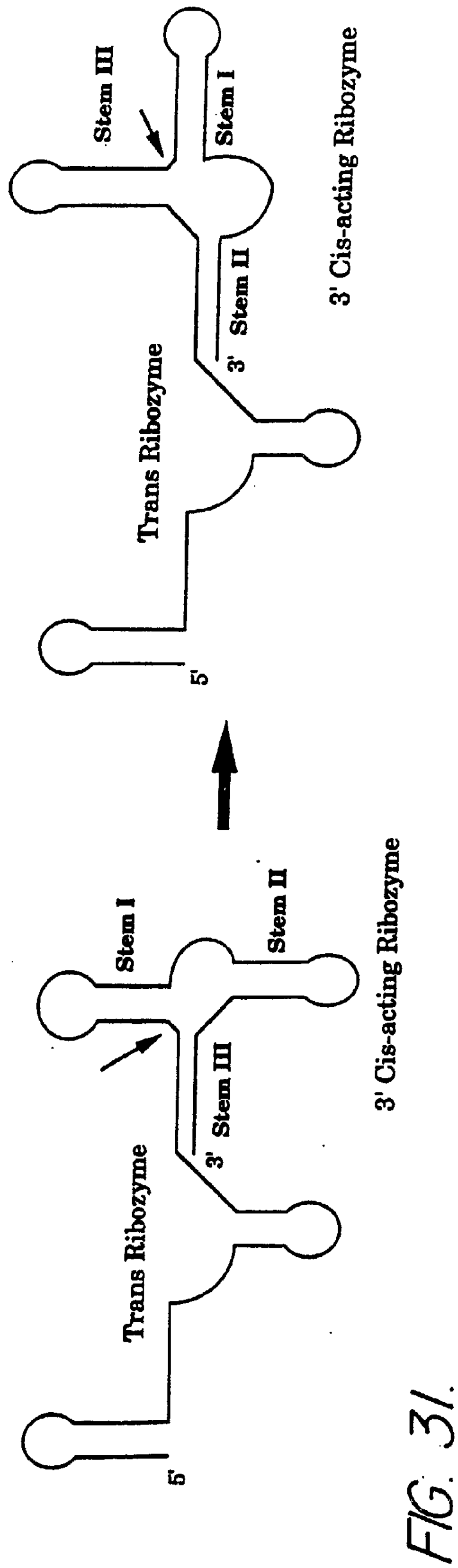

5'
Trans Ribozyme
3'
Stem III
Stem I
Stem II
3' Cis-acting Ribozyme
5'
Trans Ribozyme
3'
Stem II
Stem I
Stem III
3' Cis-acting Ribozyme


FIG. 30

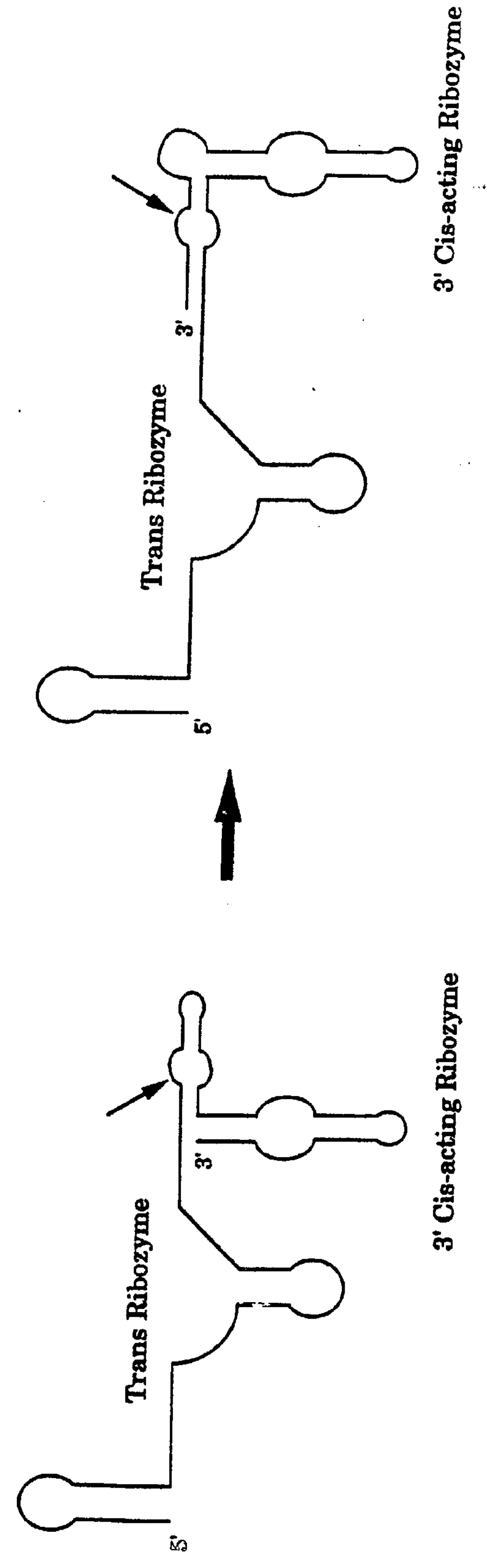

5'
Trans Ribozyme
3'
3' Cis-acting Ribozyme
5'
Trans Ribozyme
3'
3' Cis-acting Ribozyme


FIG. 31.

FIG. 32.

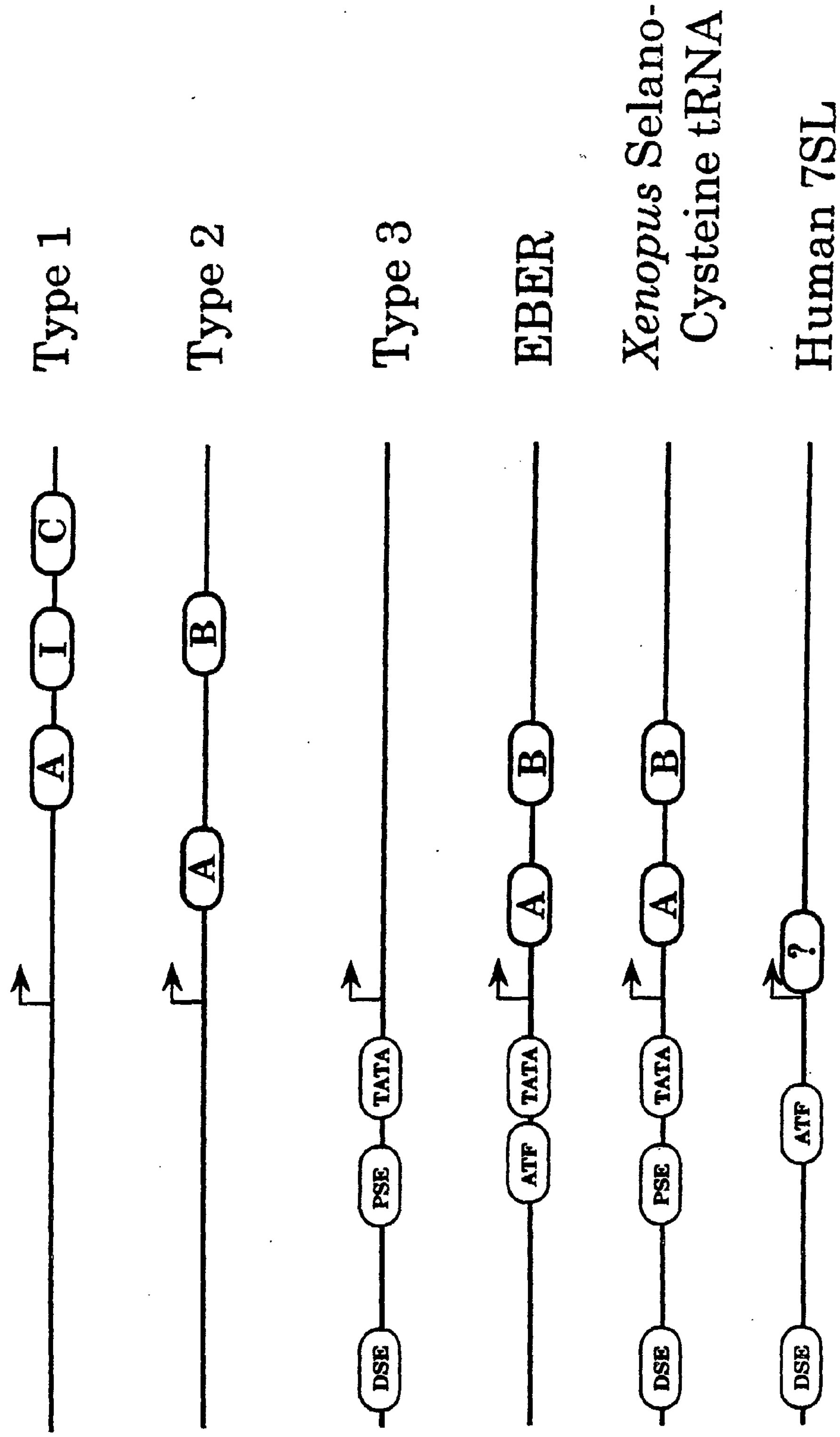
Type 1
A
I
C
Type 2
A
B
Type 3
DSE
PSE
TATA
EBER
ATF
TATA
A
B
Xenopus Selano-
Cysteine tRNA
DSE
PSE
TATA
A
B
Human 7SL
DSE
ATF
?

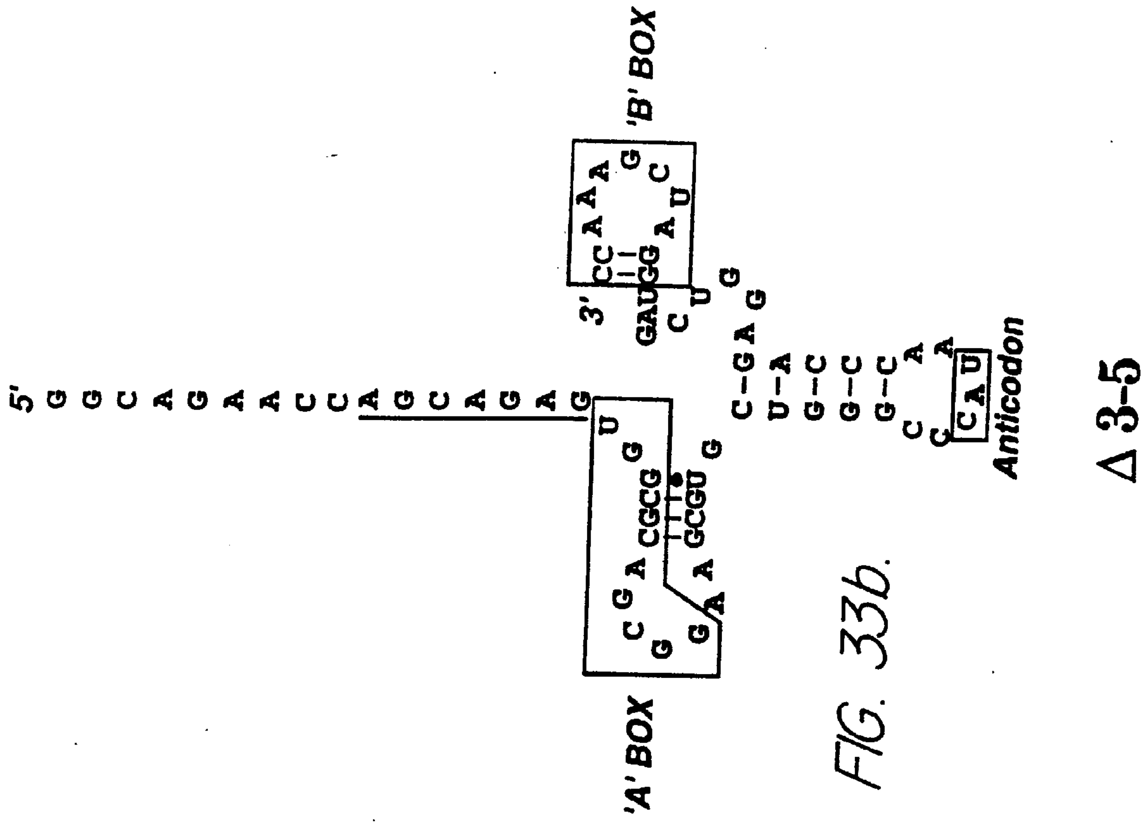


FIG. 33b.

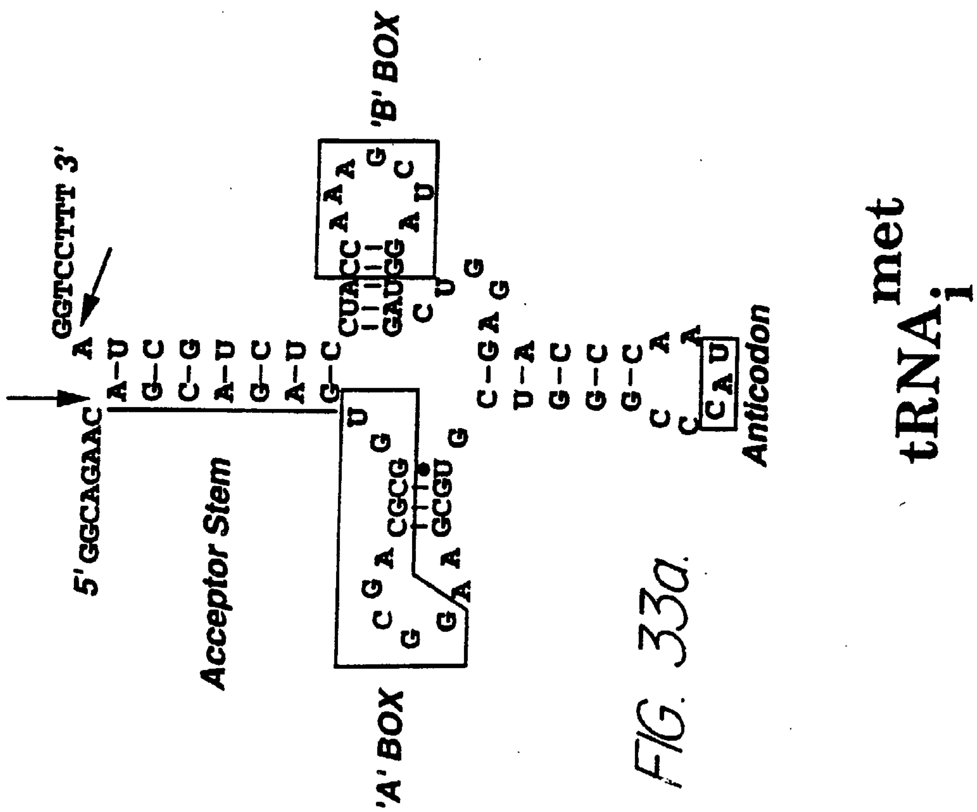


FIG. 33a.

FIG. 34a.

Δ3-5/HHI

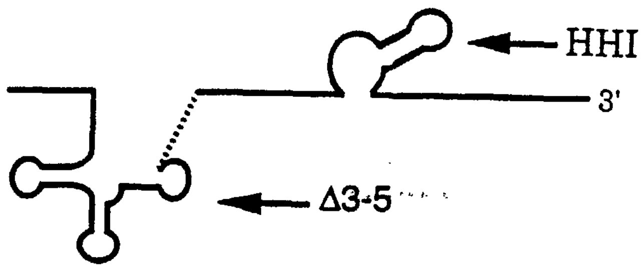


FIG. 34b.

S3

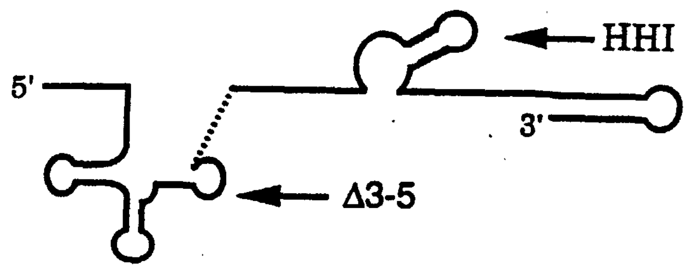


FIG. 34c.

S5

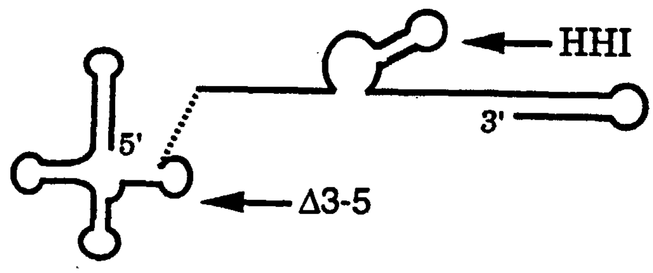


FIG. 34d.

S35

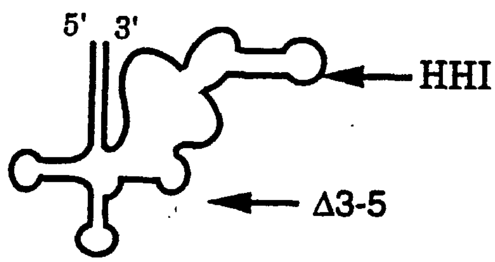


FIG. 34e.

S35Plus

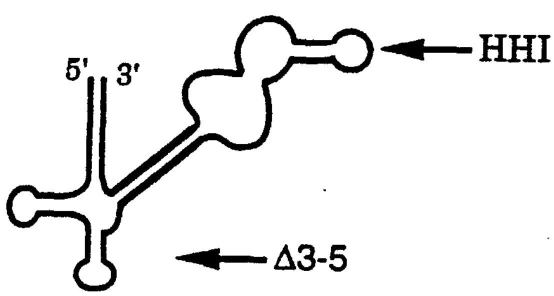

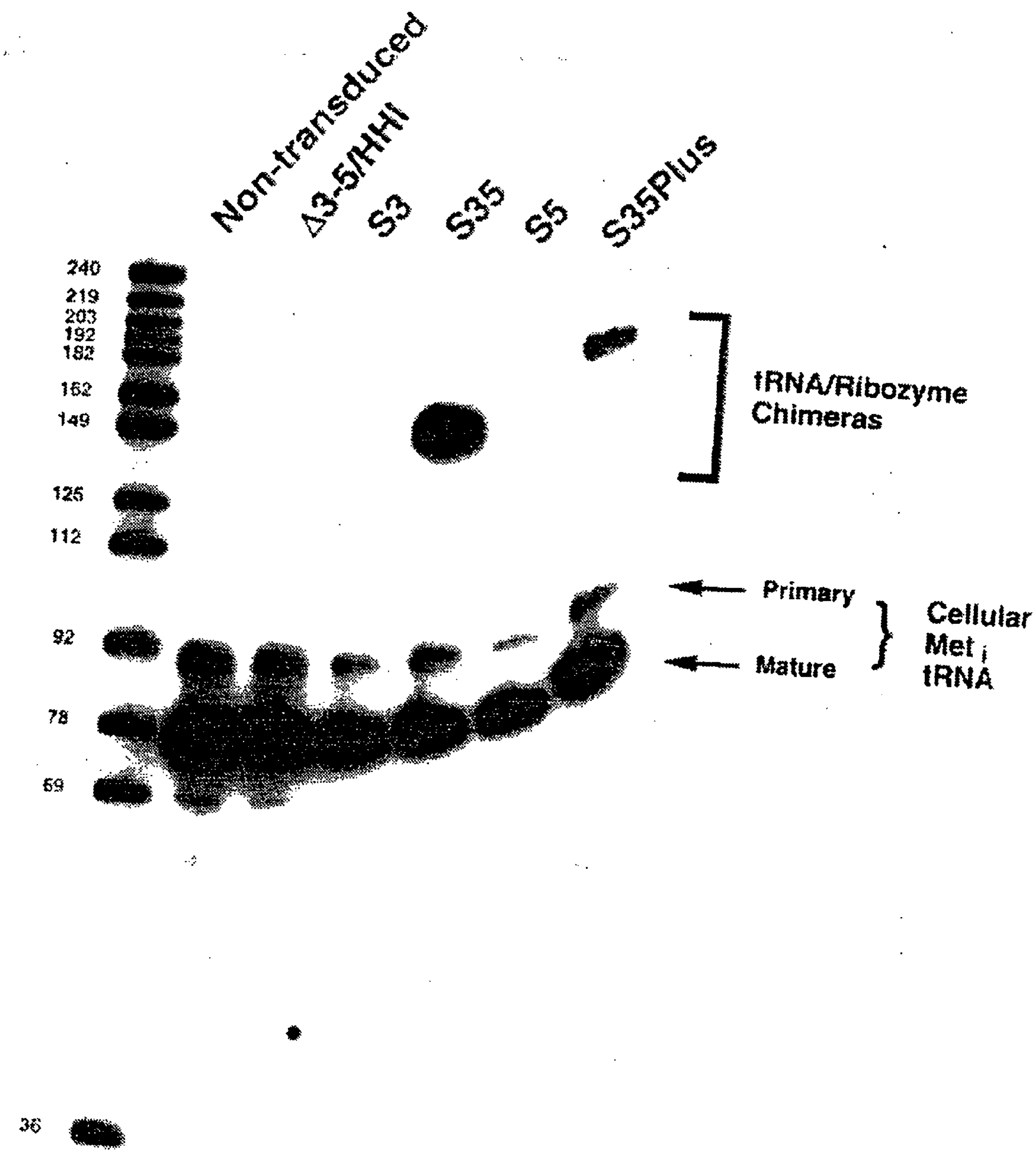
Non-transduced
Δ3-5/HHI
S3
S35
S5
S35Plus
240
219
203
192
182
162
149
125
112
92
78
69
36
tRNA/Ribozyme Chimeras
Primary
Mature
Cellular Met i tRNA

FIG. 35.

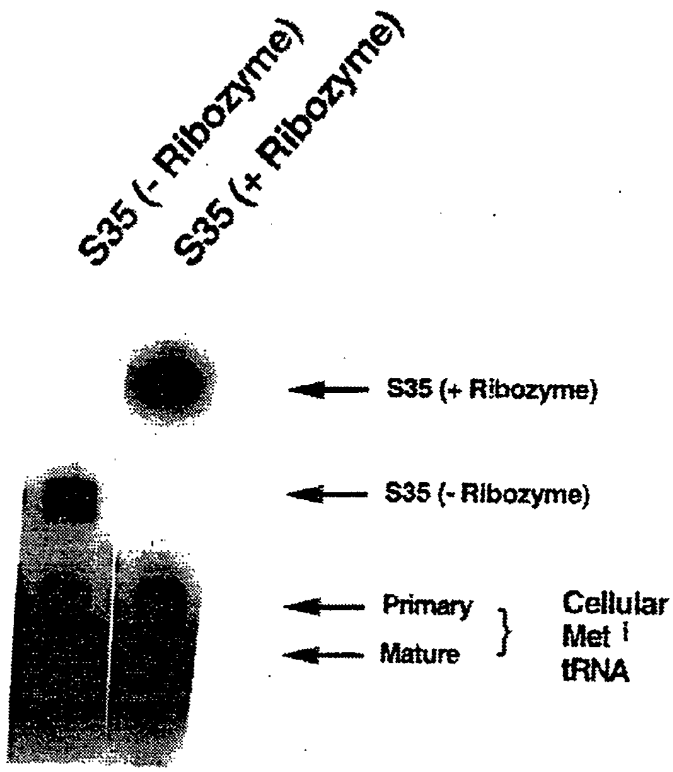
S35 (- Ribozyme)
S35 (+ Ribozyme)
S35 (+ Ribozyme)
S35 (- Ribozyme)
Primary
Mature
Cellular
Met i
tRNA

FIG. 36.

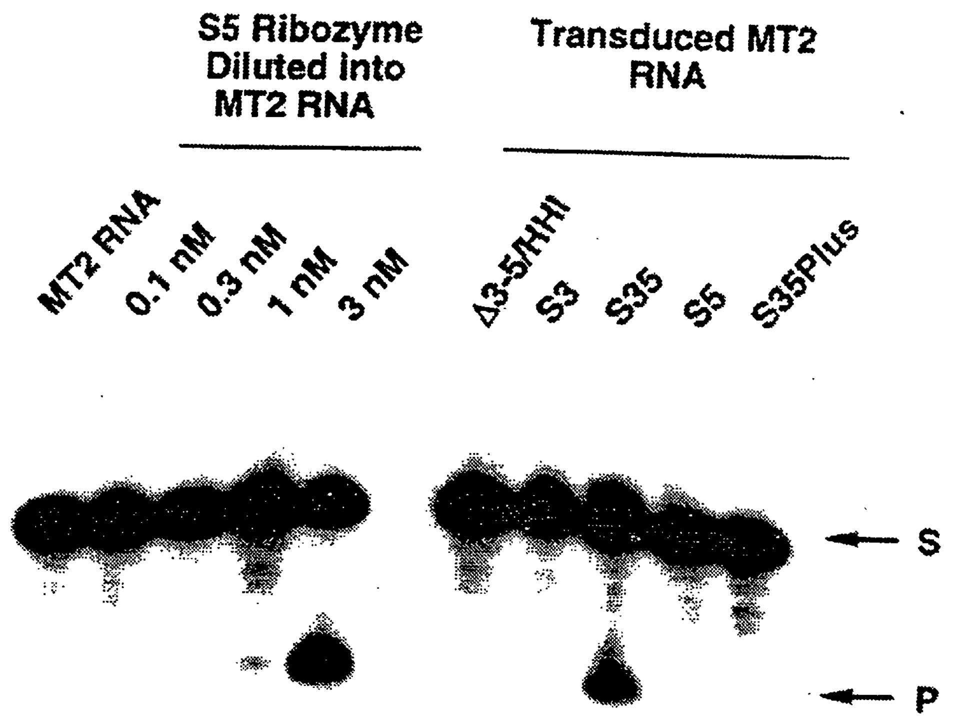
S5 Ribozyme Diluted into MT2 RNA
Transduced MT2 RNA
MT2 RNA
0.1 nM
0.3 nM
1 nM
3 nM
Δ3-5/RHI
S3
S35
S5
S35Plus
S
P

FIG. 37.

FIG. 38.

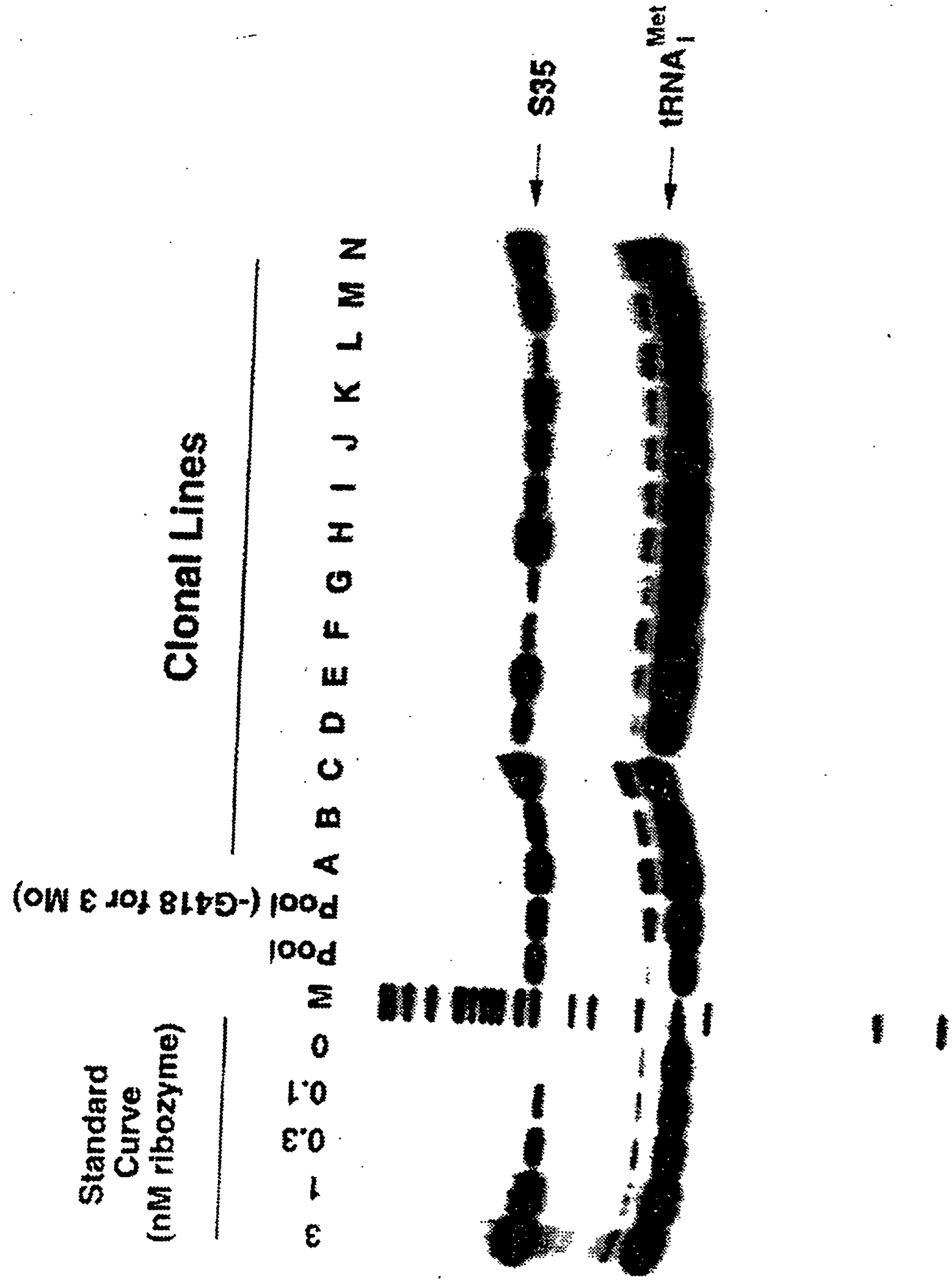
Standard Curve (nM ribozyme)
3
1
0.3
0.1
0
M
Pool
Pool (-G418 for 3 Mo)
Clonal Lines
A B C D E F G H I J K L M N
S35
tRNA$_i^{Met}$

FIG. 39.

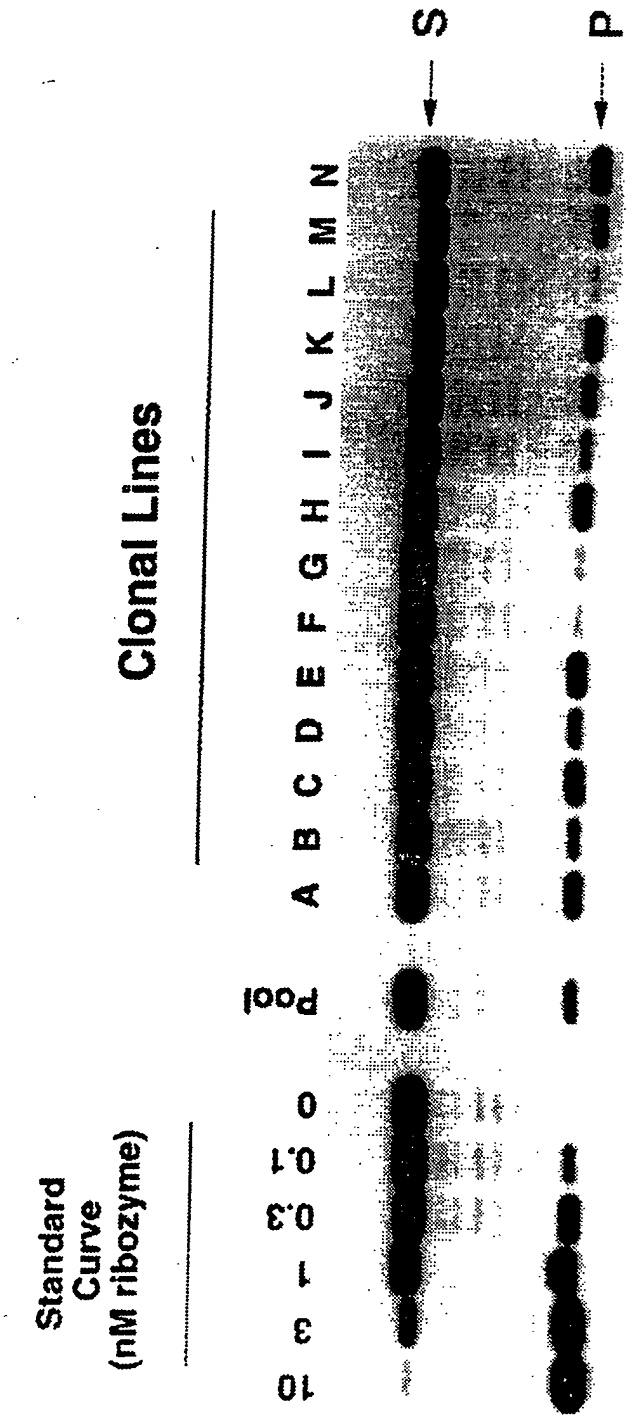
Standard Curve (nM ribozyme)
10
3
1
0.3
0.1
0
Pool
Clonal Lines
A B C D E F G H I J K L M N
S
P

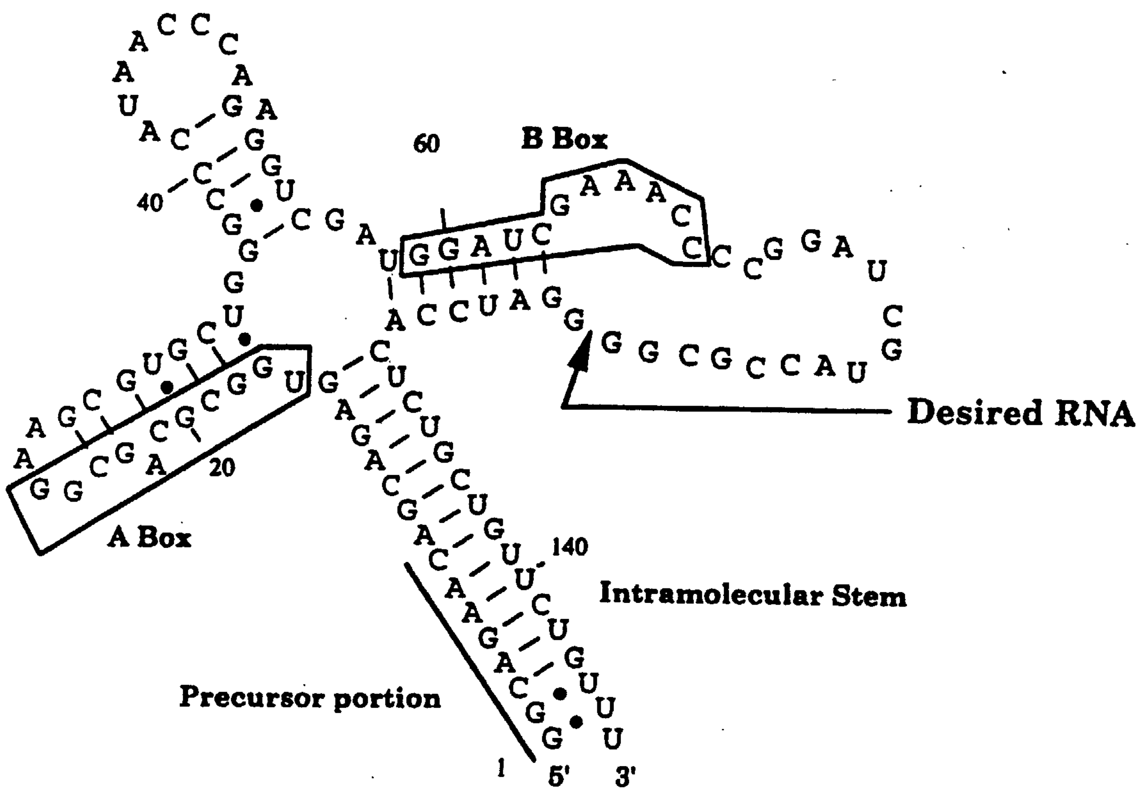
B Box
A Box
Desired RNA
Intramolecular Stem
Precursor portion
60
40
20
140
1
5'
3'

FIG. 40.

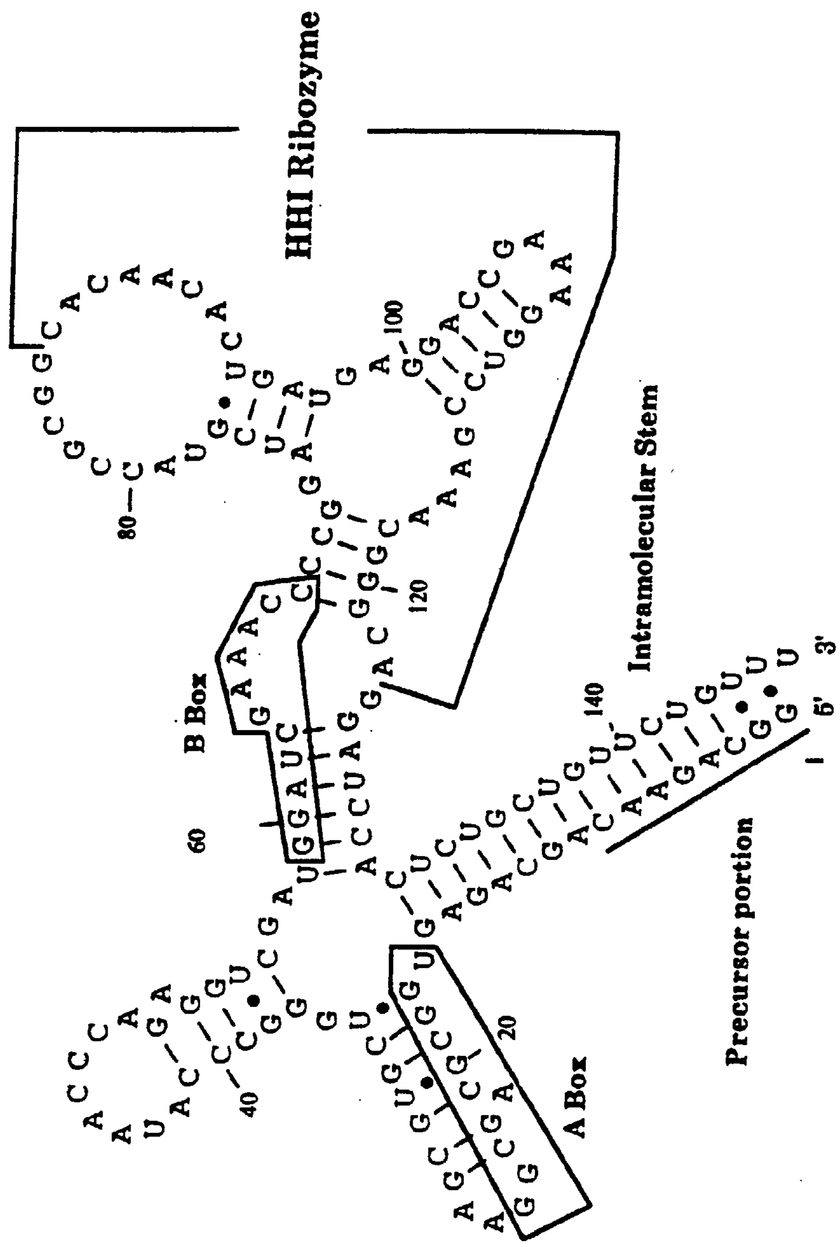
HHI Ribozyme
B Box
A Box
Intramolecular Stem
Precursor portion

FIG. 41.

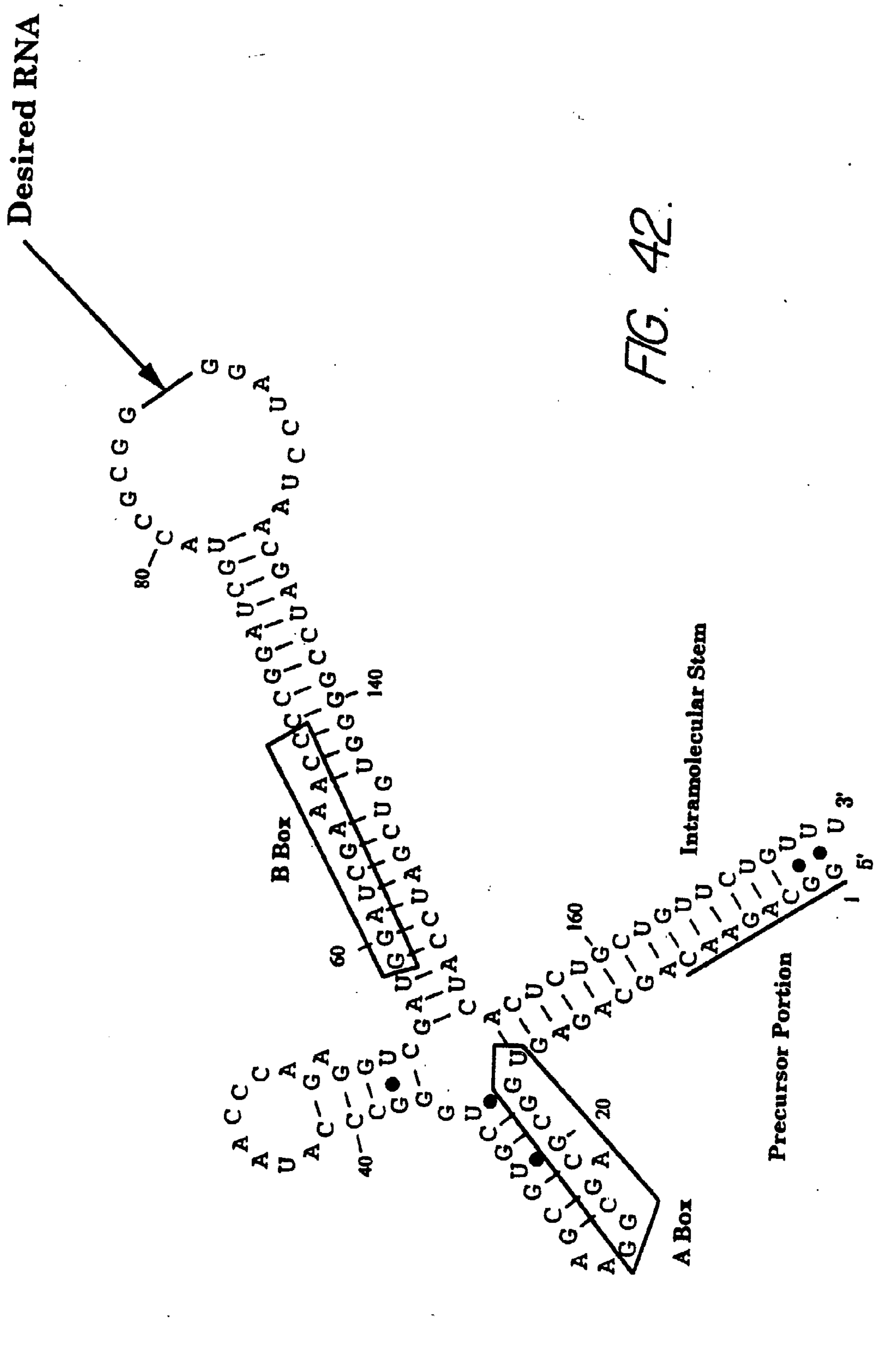
Desired RNA
B Box
A Box
Intramolecular Stem
Precursor Portion
80
140
60
40
20
160
1
5'
3'

FIG. 42.

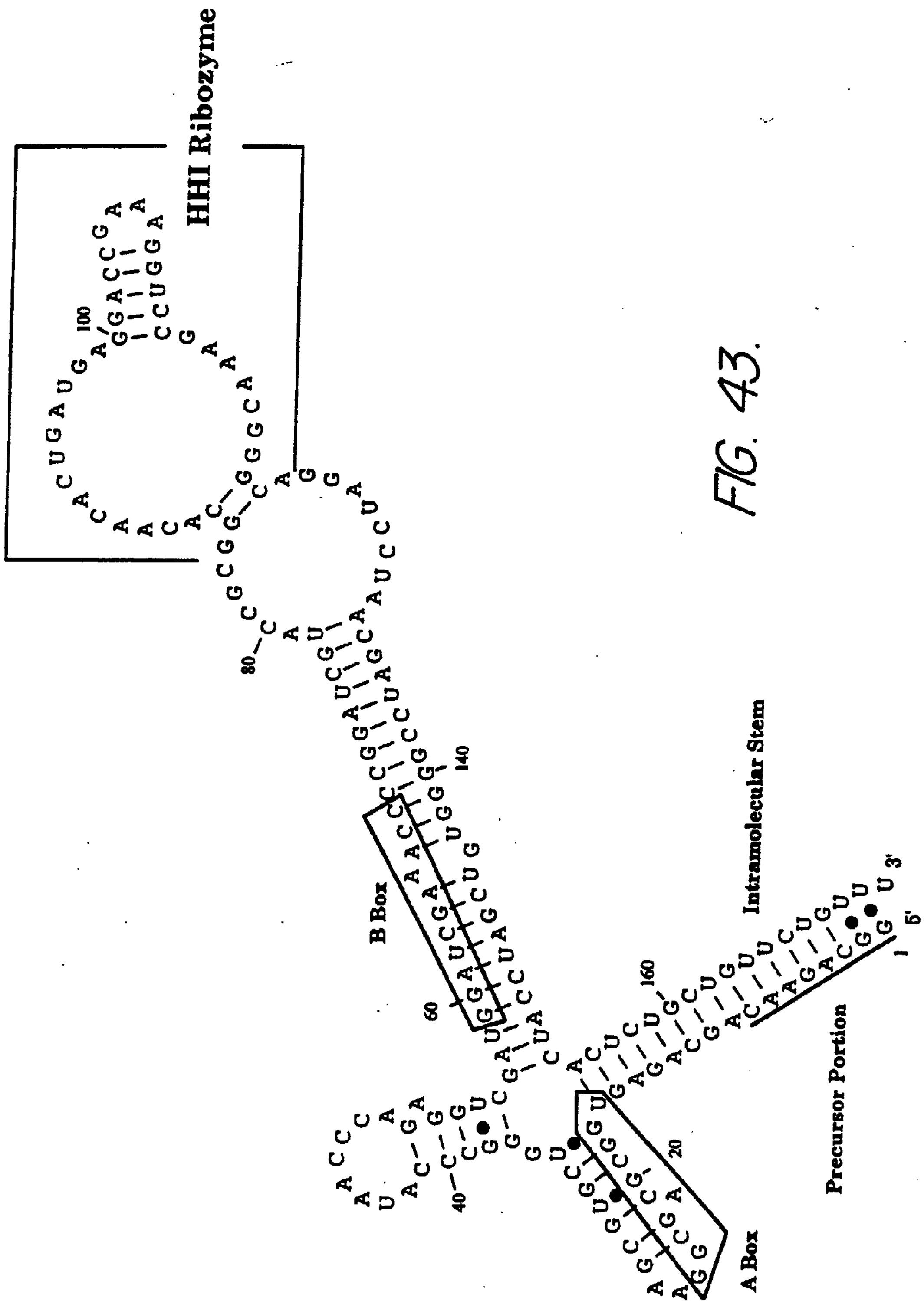
HHI Ribozyme
B Box
A Box
Intramolecular Stem
Precursor Portion

FIG. 43.

FIG. 44. **S35 Sequence**

GGCAGAACAG CAGAGUGGCG CAGCGGAAGC GUGCUGGGCC CAUAACCCAG 50

AGGUCGAUGG AUCGAAACCC CGGAUCGUAC CGCGGUGGAU CCACUCUGCU 100

GUUCUGUUU 109

FIG. 45. **HHIS35**

GGCAGAACAG CAGAGUGGCG CAGCGGAAGC GUGCUGGGCC CAUAACCCAG 50

AGGUCGAUGG AUCGAAACCC CGGAUCGUAC CGCGGCACAA CACUGAUGAG 100

GACCGAAAGG UCCGAAACGG GCAGGAUCCA CUCUGCUGUU CUGUUU 146

**Underlined bases indicate the HHI ribozyme sequence**

FIG. 46. **S35 Plus Sequence**

GGCAGAACAG CAGAGUGGCG CAGCGGAAGC GUGCUGGGCC CAUAACCCAG 50

AGGUCGAUGG AUCGAAACCC CGGAUCGUAC CGCGGGGAUC CUAACGAUCC 100

GGGGUGUCGA UCCAUCACUC UGCUGUUCUG UU U 133

FIG. 47. **HHIS35 Plus**

GGCAGAACAG CAGAGUGGCG CAGCGGAAGC GUGCUGGGCC CAUAACCCAG 50

AGGUCGAUGG AUCGAAACCC CGGAUCGUAC CGCGGCACAA CACUGAUGAG 100

GACCGAAAGG UCCGAAACGG GCAGGAUCCU AACGAUCCGG GGUGUCGAUC 150

CAUCACUCUG CUGUUCUGUU U 171

FIG. 48.

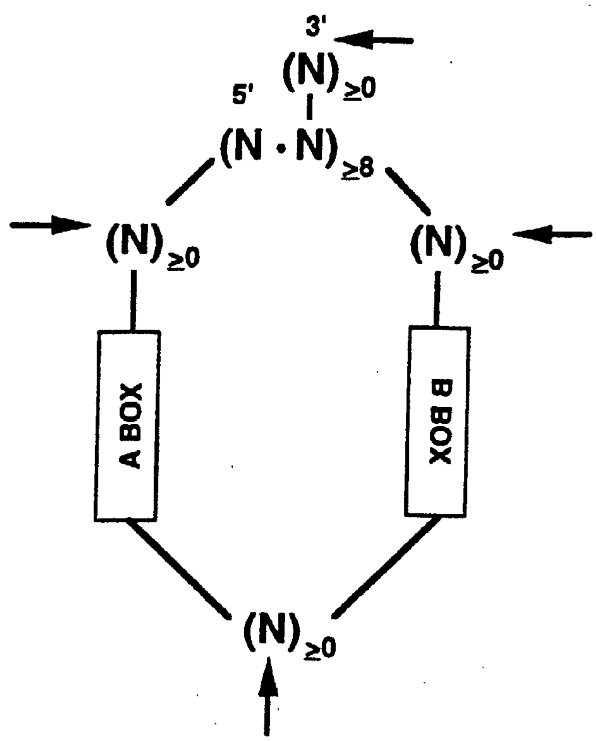


A BOX = URGCNNAGYGG
B BOX = GGUUCGANUCC

This is based on Geiduschek & Tocchini-Valentini, (1988) Annu. Review Biochem. 57, 873-914. However this consensus sequence is not meant to be limiting

N = A, U, G, or C

R = Purine

Y = Pyrimidine

• = Indicates base-pairing

— = Indicates covalent linkage

➔ = Indicates sites at which desired RNAs can be cloned

FIG. 49.

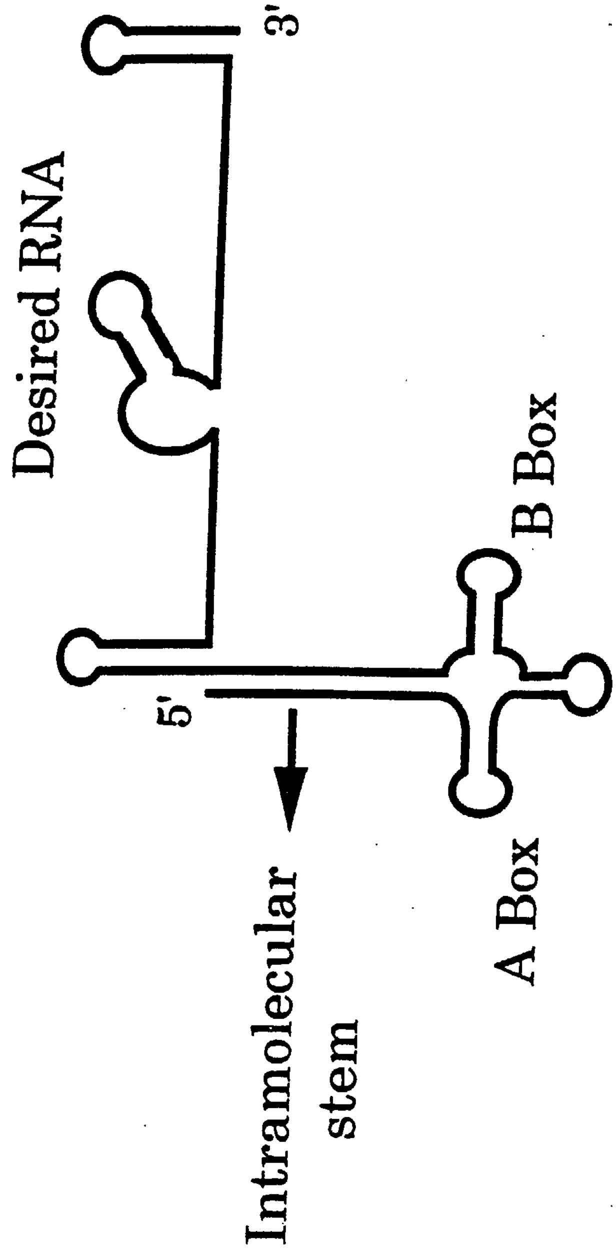

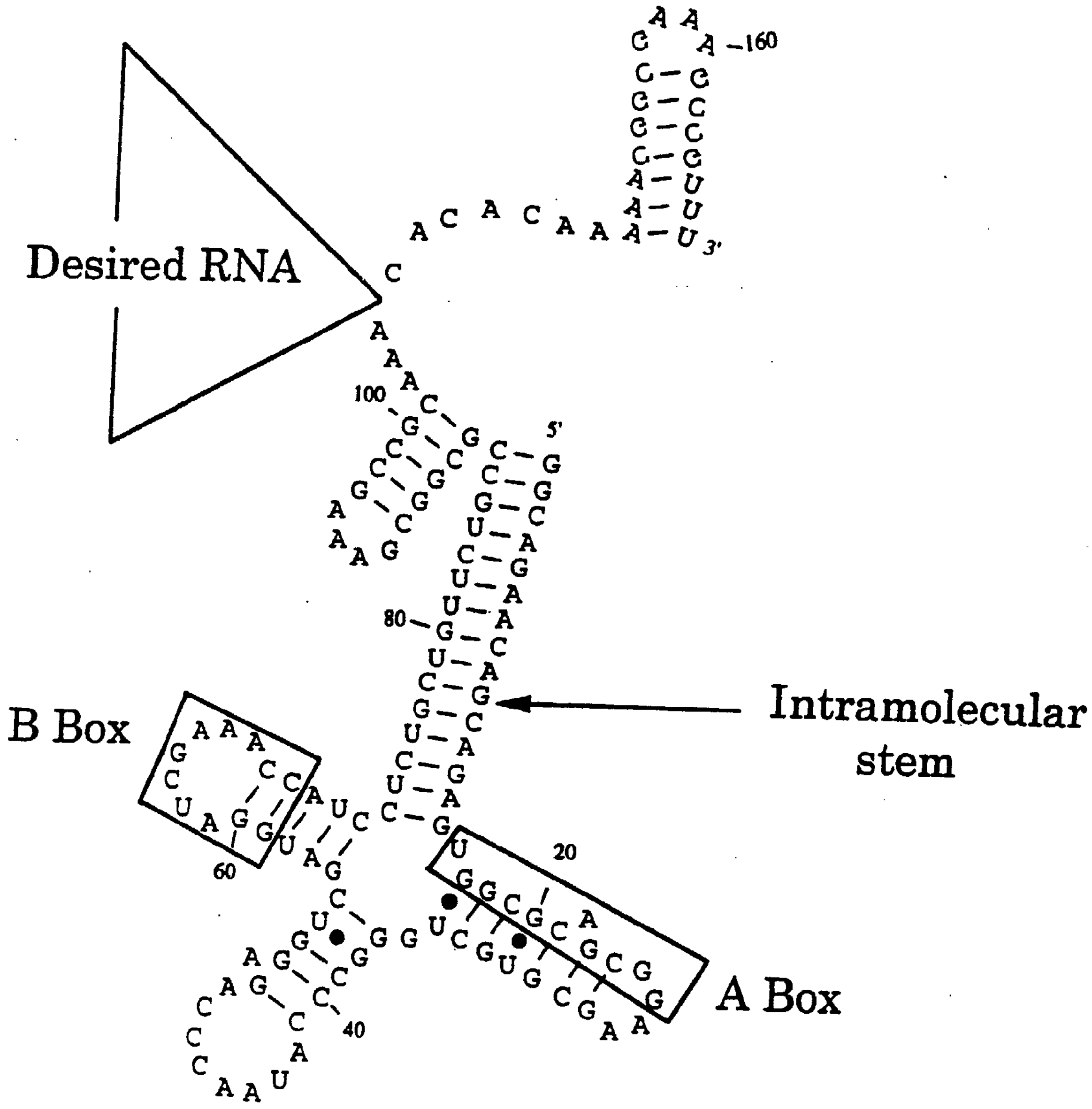
Desired RNA
B Box
A Box
Intramolecular stem
5'
3'
160
100
80
60
40
20

FIG. 50.

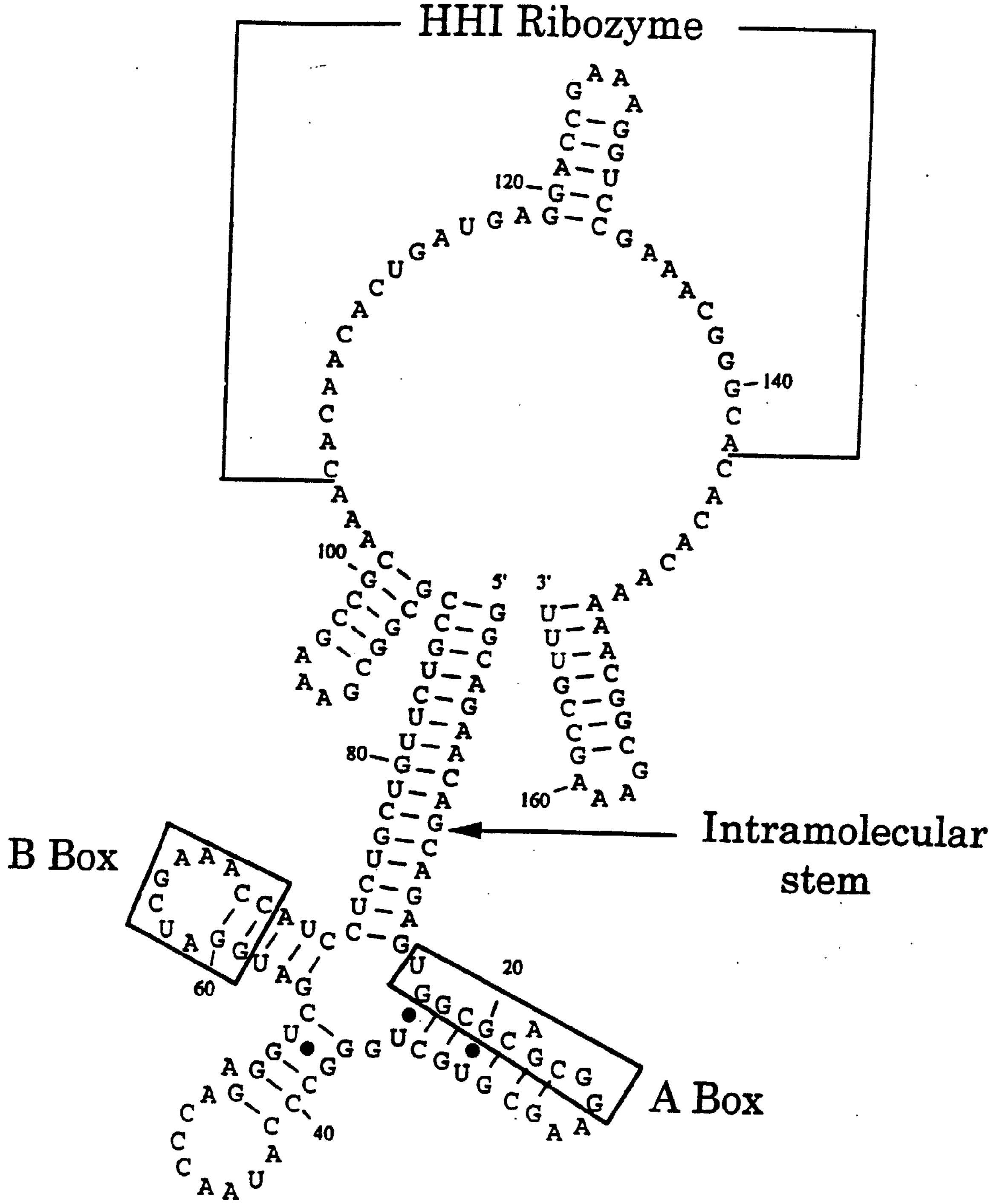
HHI Ribozyme
Intramolecular stem
B Box
A Box
5'
3'
20
40
60
80
100
120
140
160

*FIG. 51.*

FIG. 52a.

A: TRZ-A

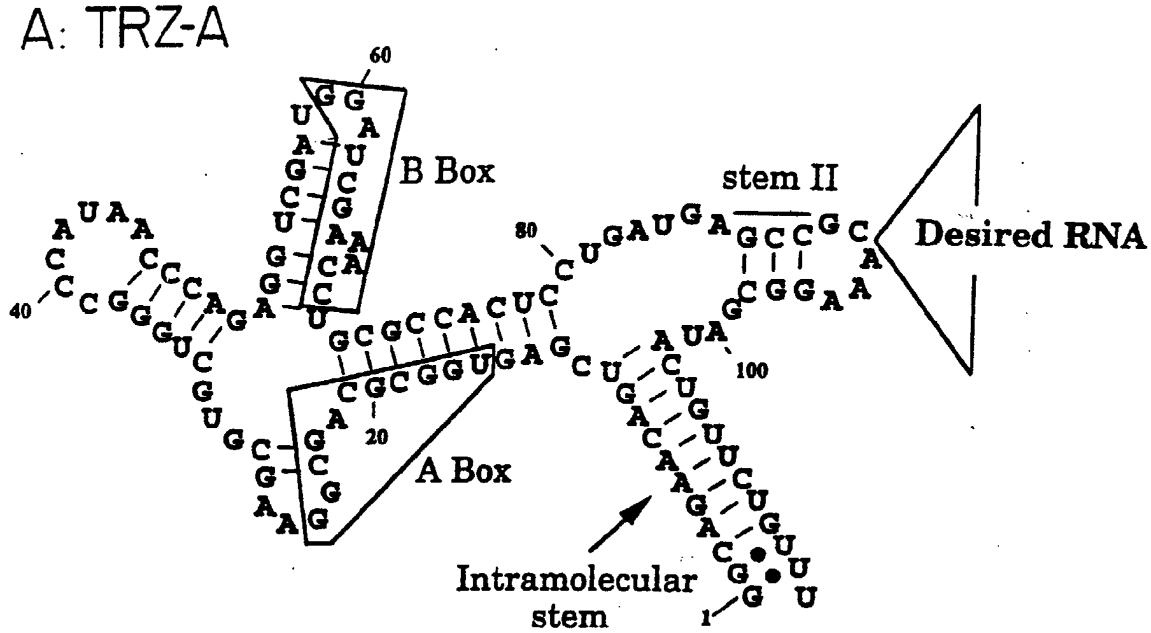


FIG. 52b.

B: TRZ-B

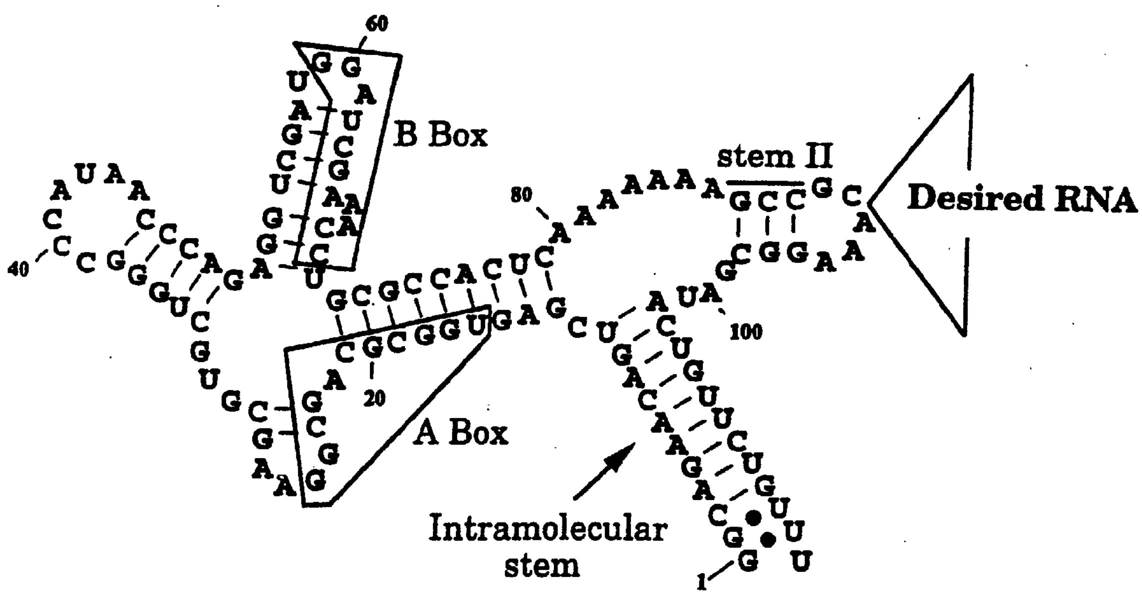

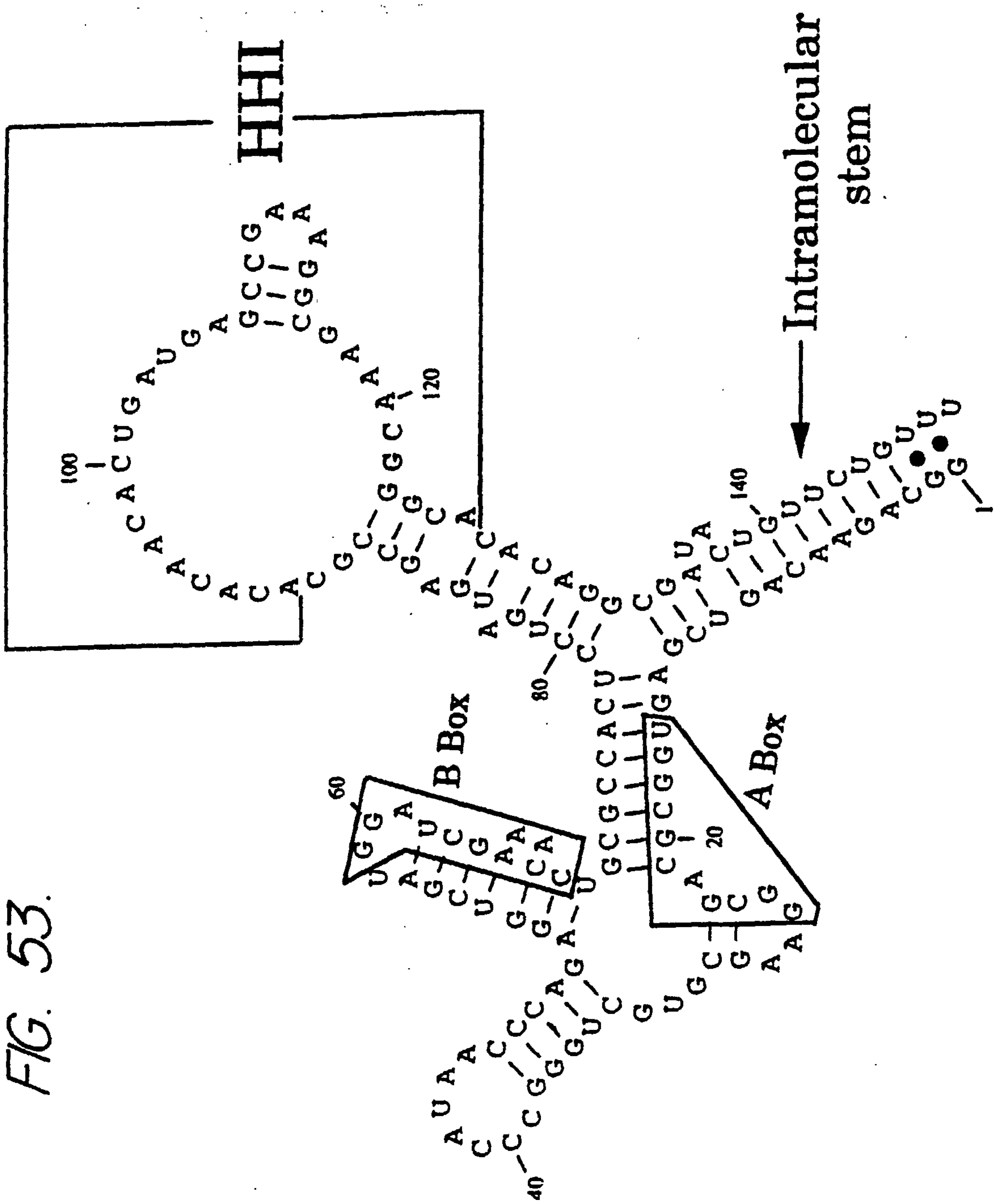


FIG. 53.

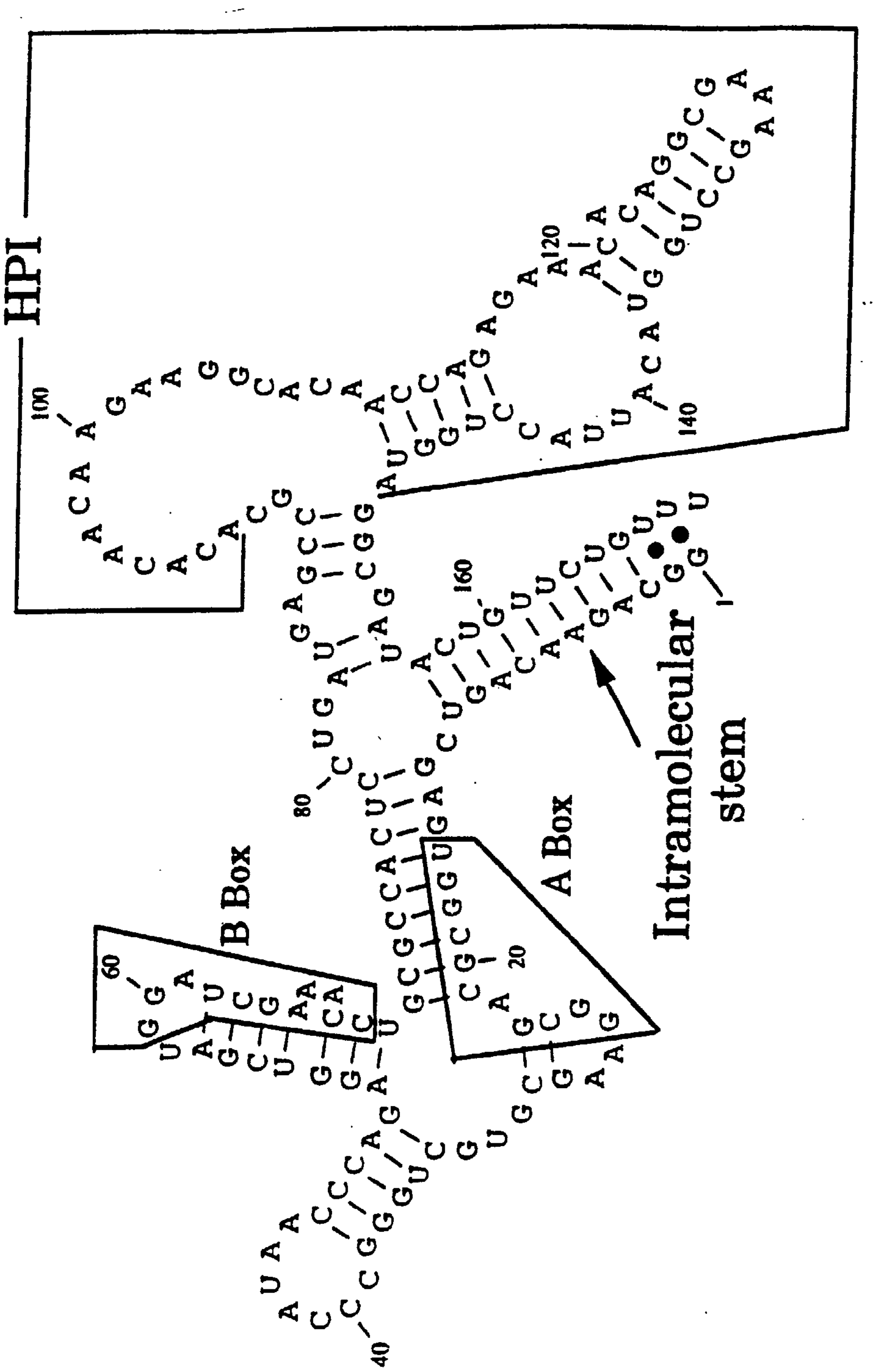


FIG. 54.

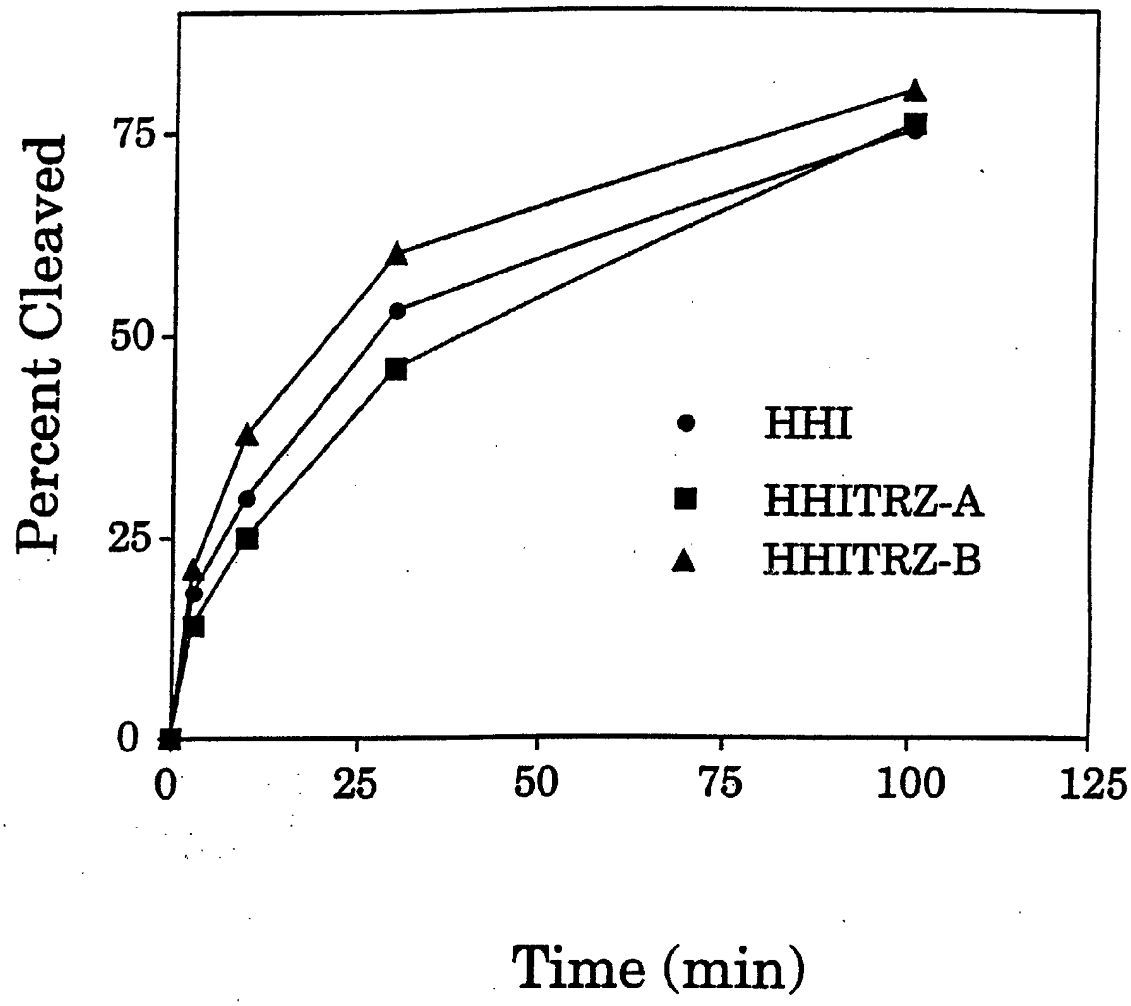
Percent Cleaved
75
50
25
0
0
25
50
75
100
125
HHI
HHITRZ-A
HHITRZ-B
Time (min)

FIG. 55.

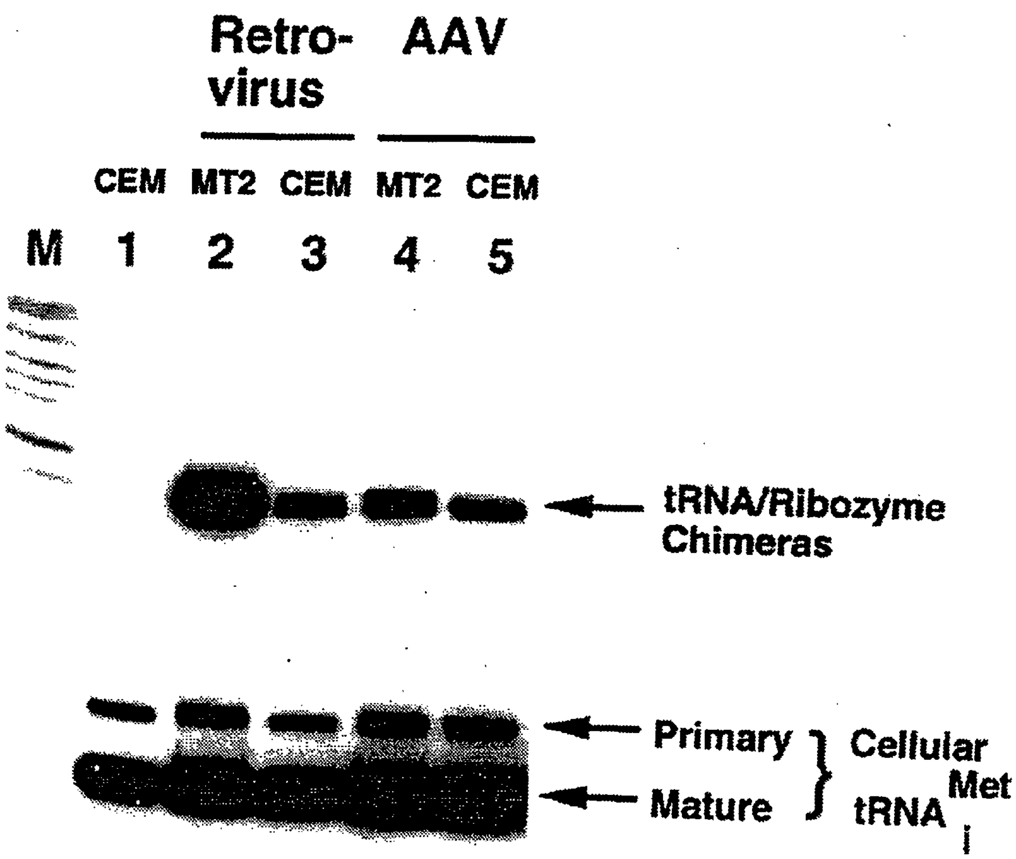
Retro-
virus
AAV
CEM
MT2
CEM
MT2
CEM
M
1
2
3
4
5
tRNA/Ribozyme
Chimeras
Primary
Mature
Cellular
tRNA Met i

FIG. 56.

FIG. 57a.

AAV Vector

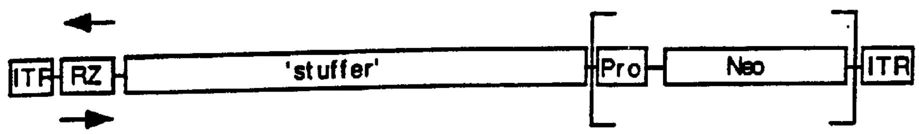


FIG. 57b.

Adenovirus Vector

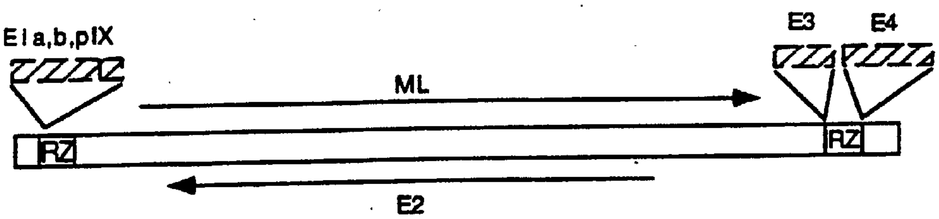

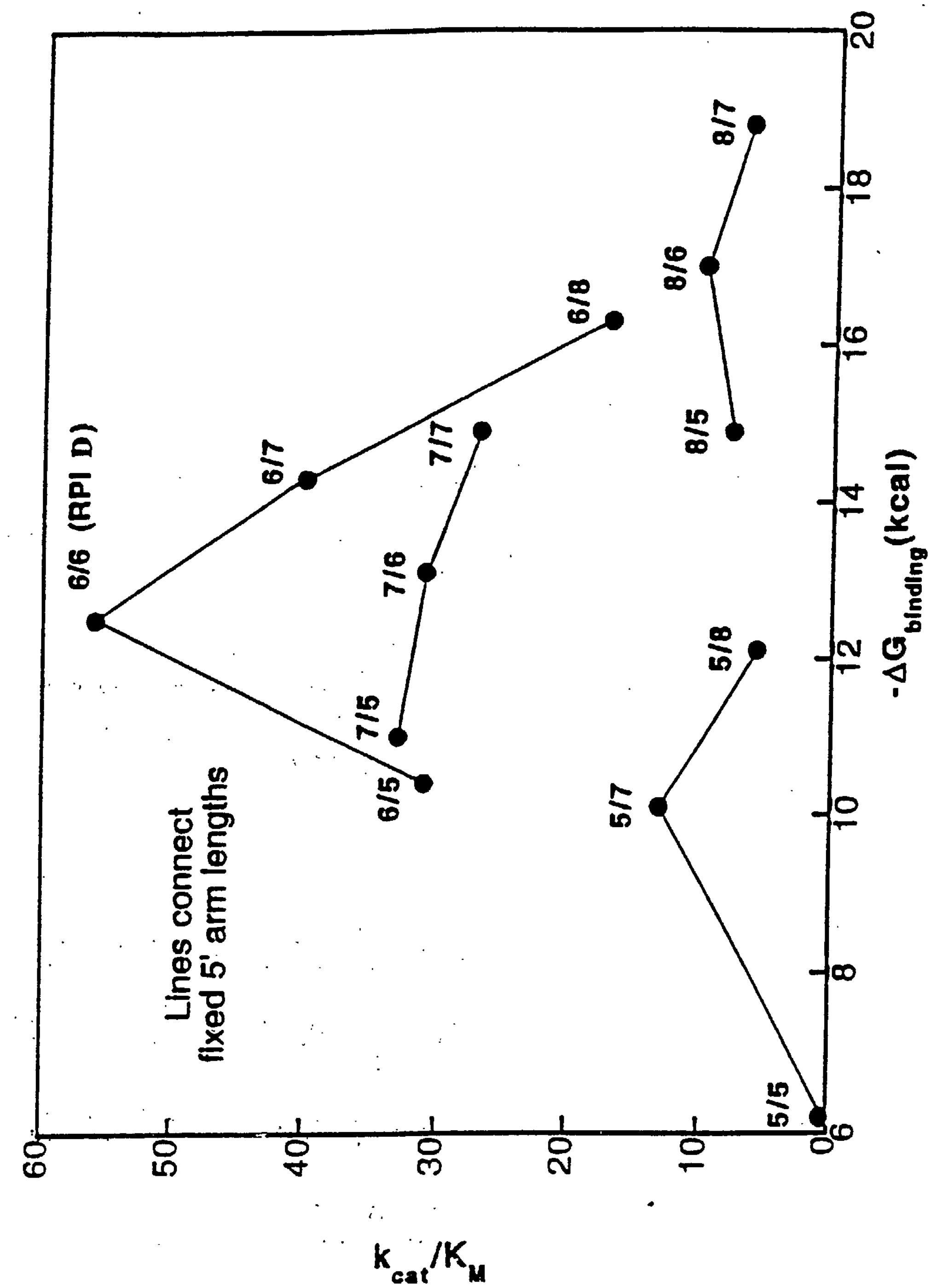
Lines connect
fixed 5' arm lengths
6/6 (RPI D)
6/7
6/8
6/5
7/5
7/6
7/7
5/5
5/7
5/8
8/5
8/6
8/7
$k_{cat}/K_M$
$-\Delta G_{binding}$(kcal)
0
10
20
30
40
50
60
6
8
10
12
14
16
18
20

FIG. 58.

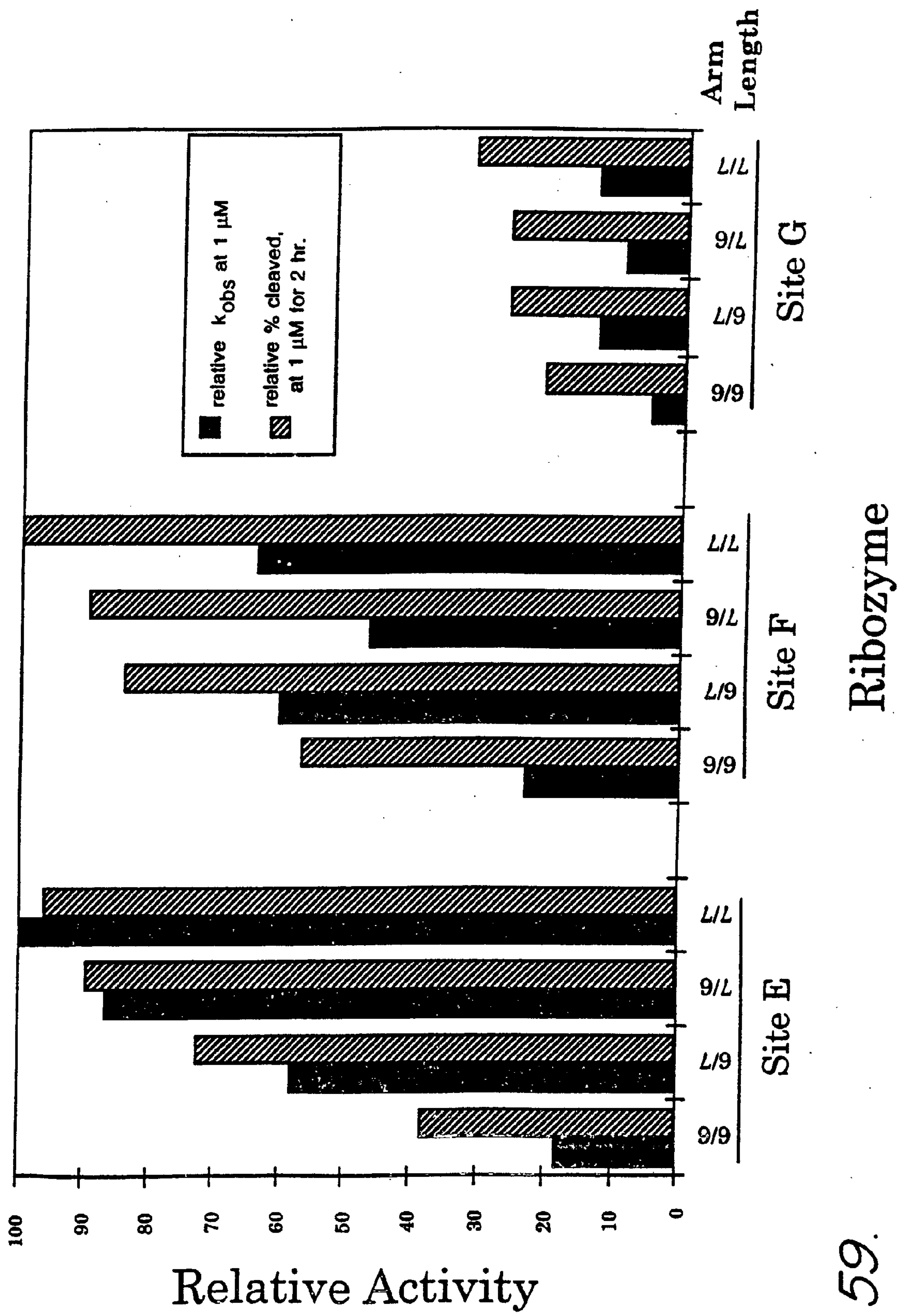

Relative Activity

FIG. 59.

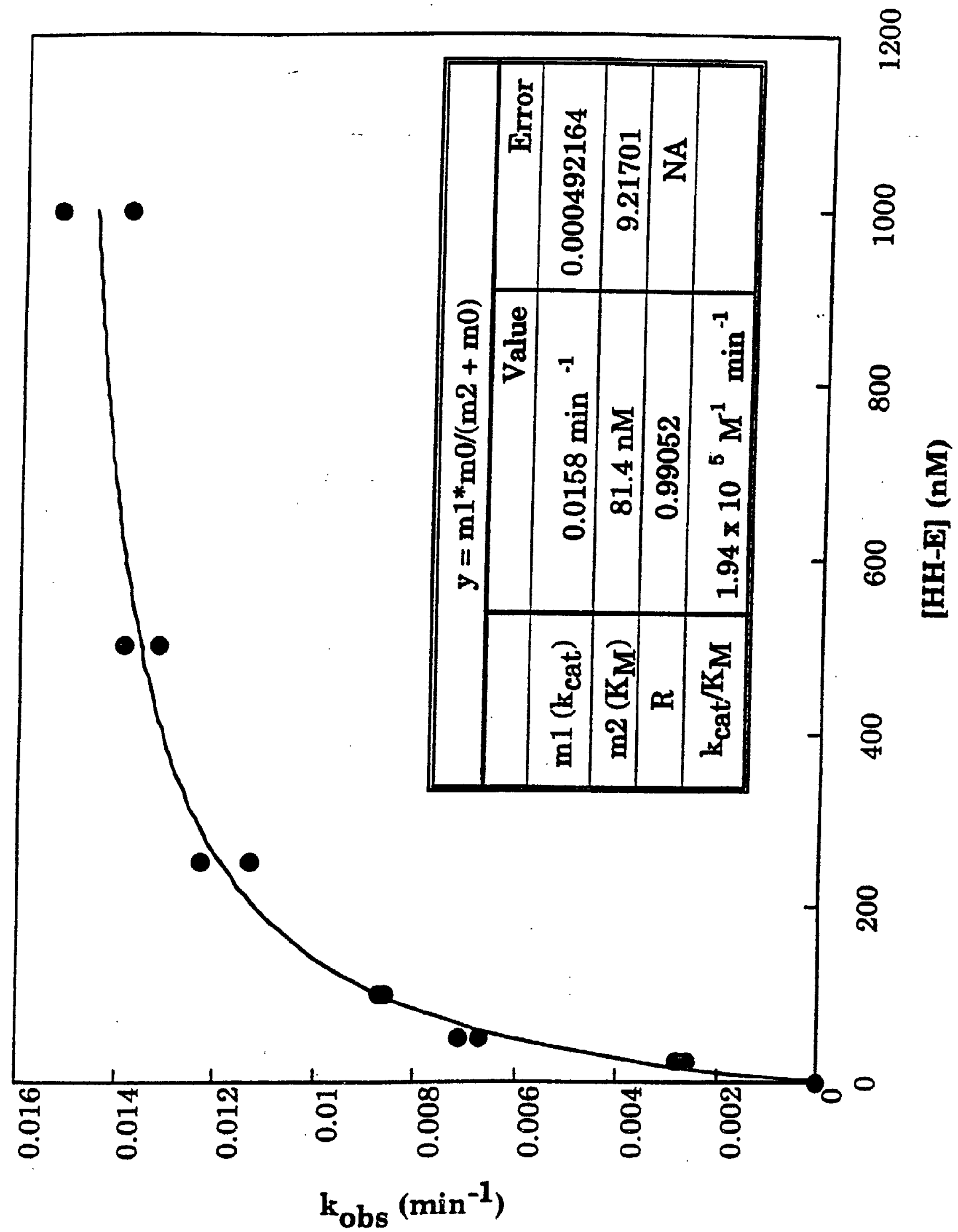

y = m1*m0/(m2 + m0)
Value
Error
m1 ($k_{cat}$)
0.0158 $min^{-1}$
0.000492164
m2 ($K_M$)
81.4 nM
9.21701
R
0.99052
NA
$k_{cat}/K_M$
1.94 x $10^5$ $M^{-1}$ $min^{-1}$
$k_{obs}$ ($min^{-1}$)
0.016
0.014
0.012
0.01
0.008
0.006
0.004
0.002
0
[HH-E] (nM)
0
200
400
600
800
1000
1200


FIG. 60.

FIG. 61.

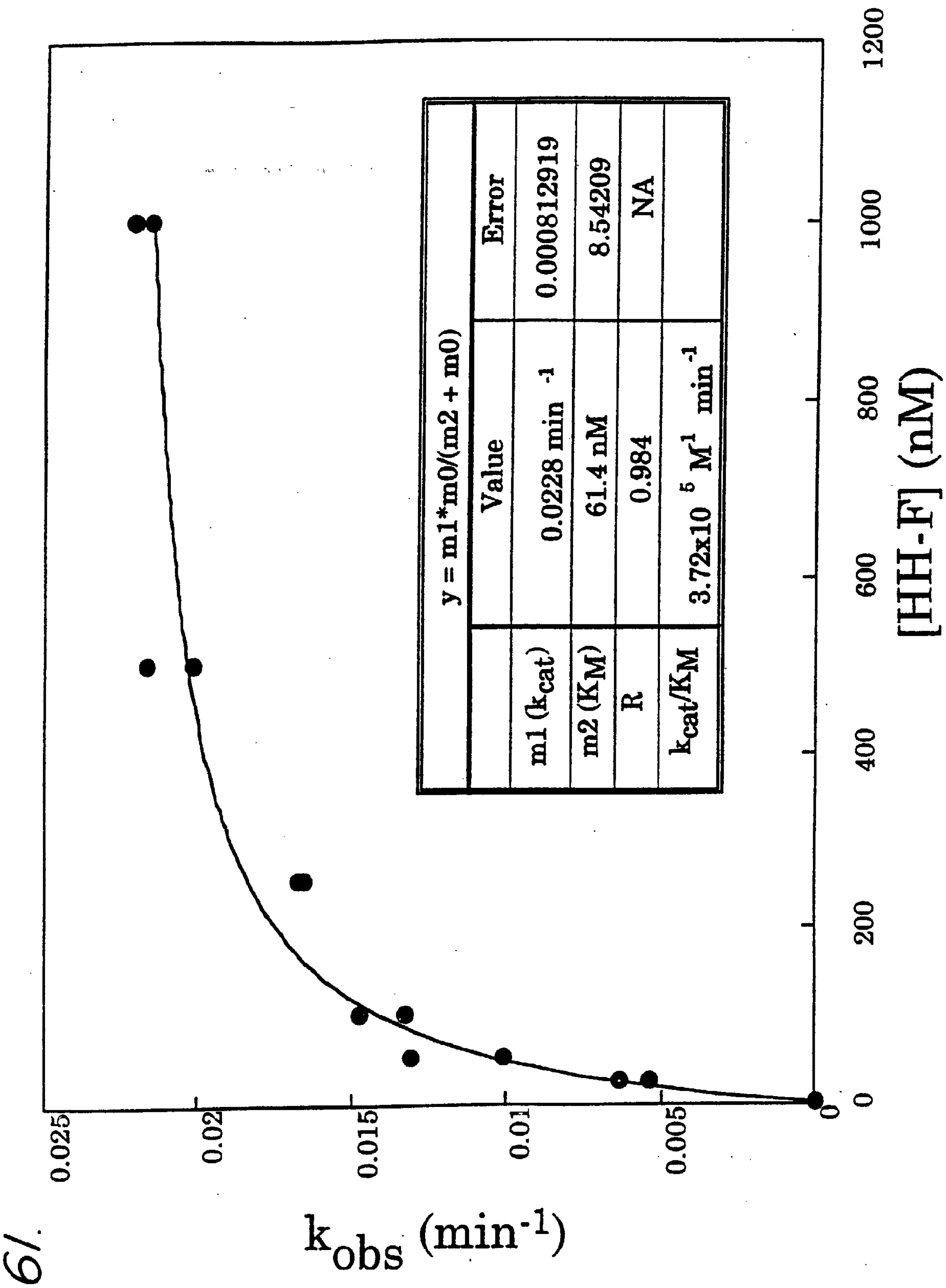

y = m1*m0/(m2 + m0)
Value
Error
m1 (kcat)
0.0228 min -1
0.000812919
m2 (KM)
61.4 nM
8.54209
R
0.984
NA
kcat/KM
3.72x10 5 M-1 min-1
kobs (min-1)
[HH-F] (nM)
0.025
0.02
0.015
0.01
0.005
0
0
200
400
600
800
1000
1200

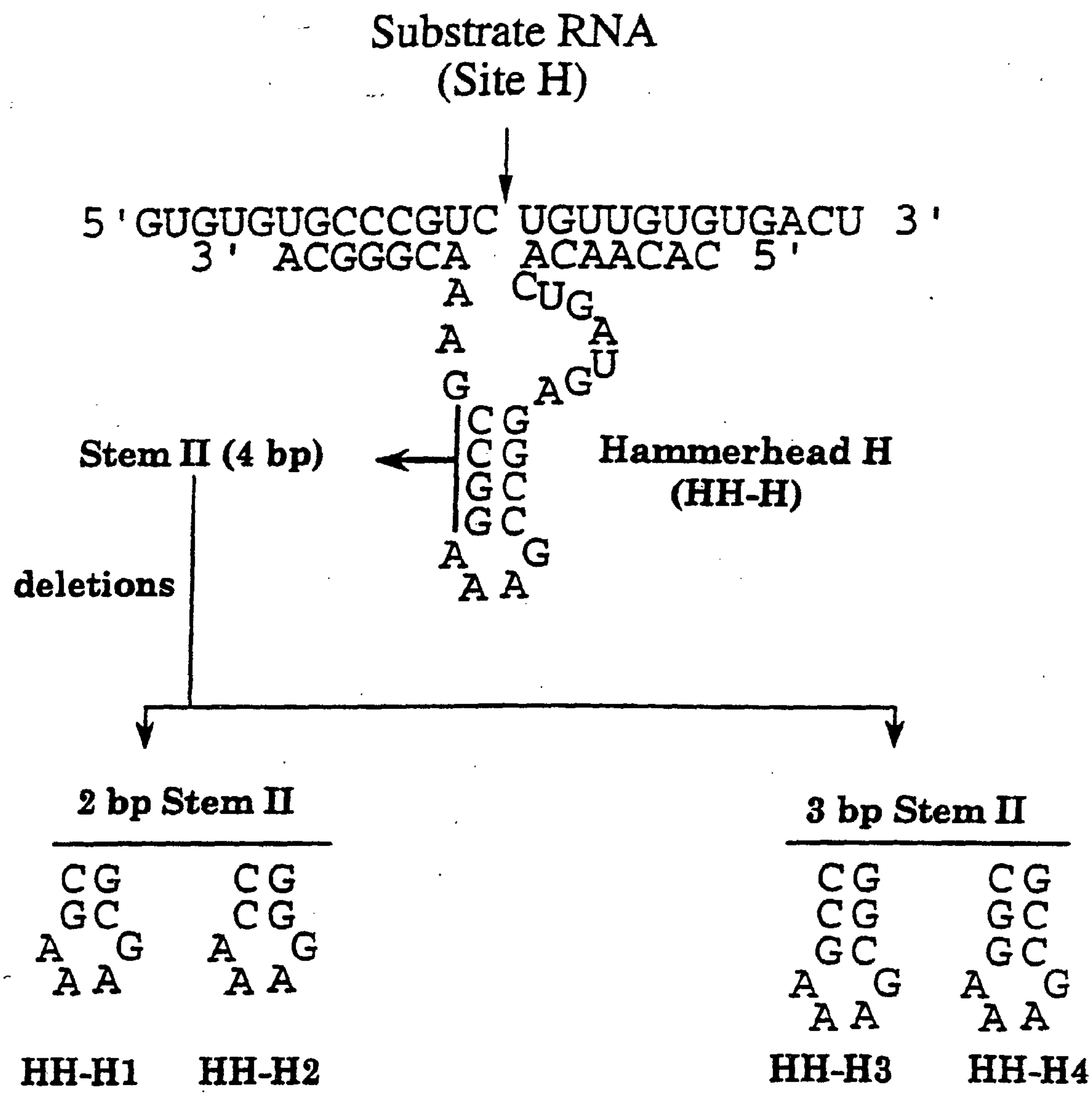
Substrate RNA
(Site H)
5'GUGUGUGCCCGUC UGUUGUGUGACU 3'
3' ACGGGCA ACAACAC 5'
Stem II (4 bp)
Hammerhead H
(HH-H)
deletions
2 bp Stem II
3 bp Stem II
HH-H1
HH-H2
HH-H3
HH-H4

FIG. 62.

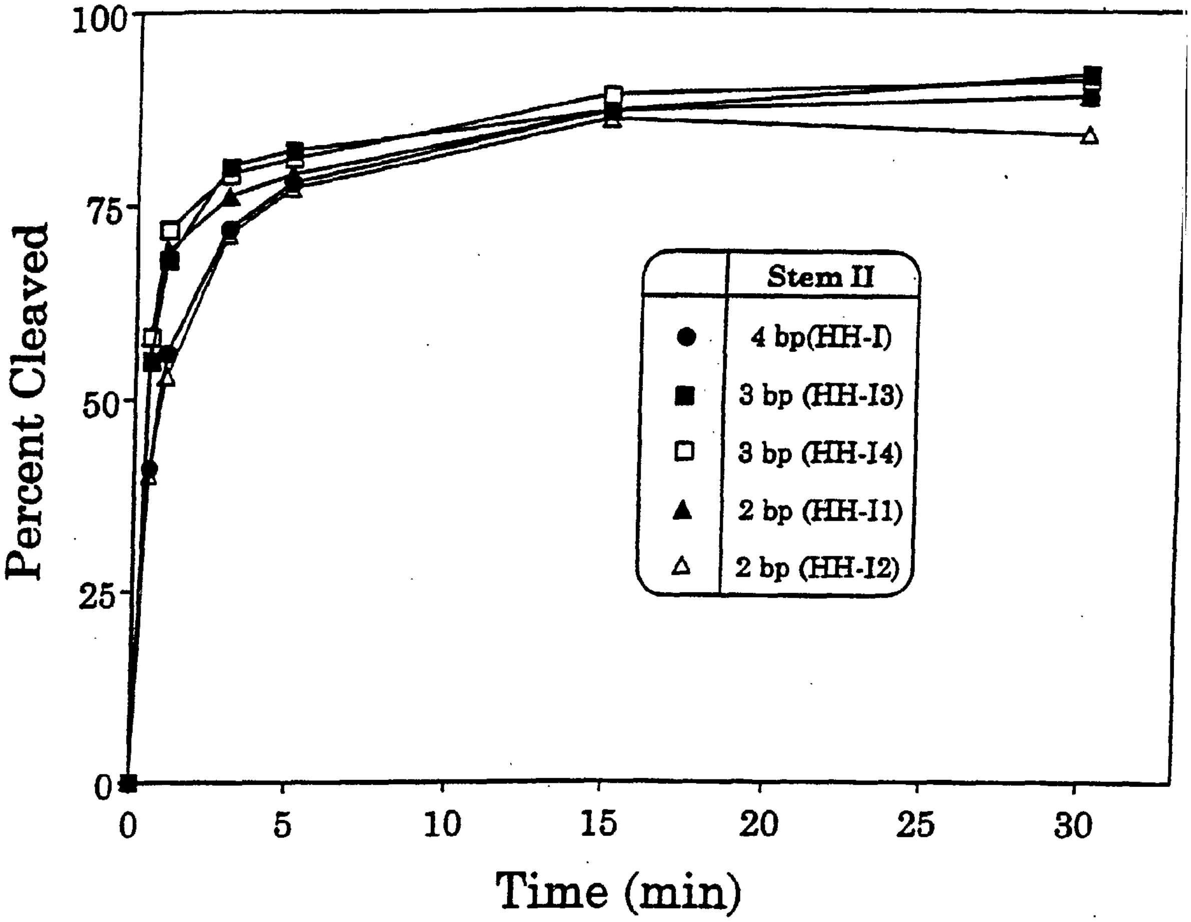


[Ribozyme] = 25 nM [Substrate] = ~ 1 nM

FIG. 63.

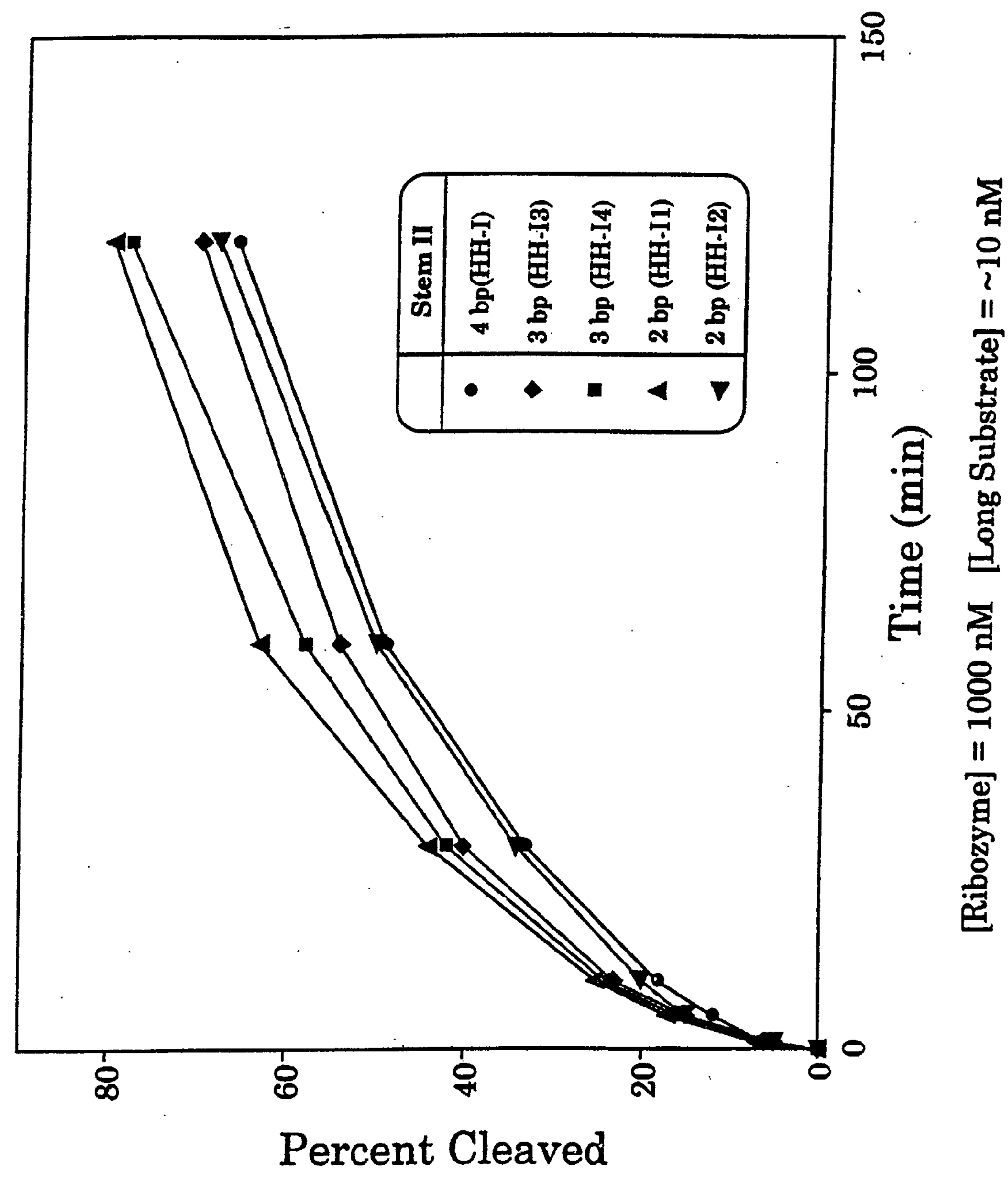
Percent Cleaved
0
20
40
60
80
0
50
100
150
Time (min)
[Ribozyme] = 1000 nM [Long Substrate] = ~10 nM
Stem II
4 bp(HH-I)
3 bp (HH-I3)
3 bp (HH-I4)
2 bp (HH-I1)
2 bp (HH-I2)

FIG. 64.

FIG. 65b.

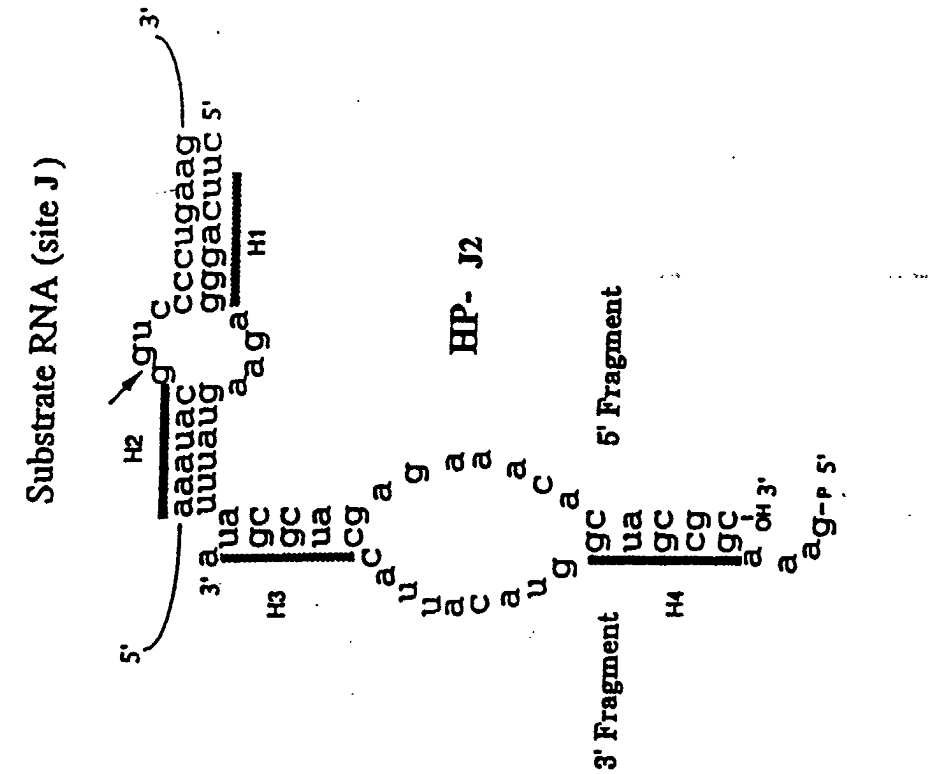


FIG. 65a.

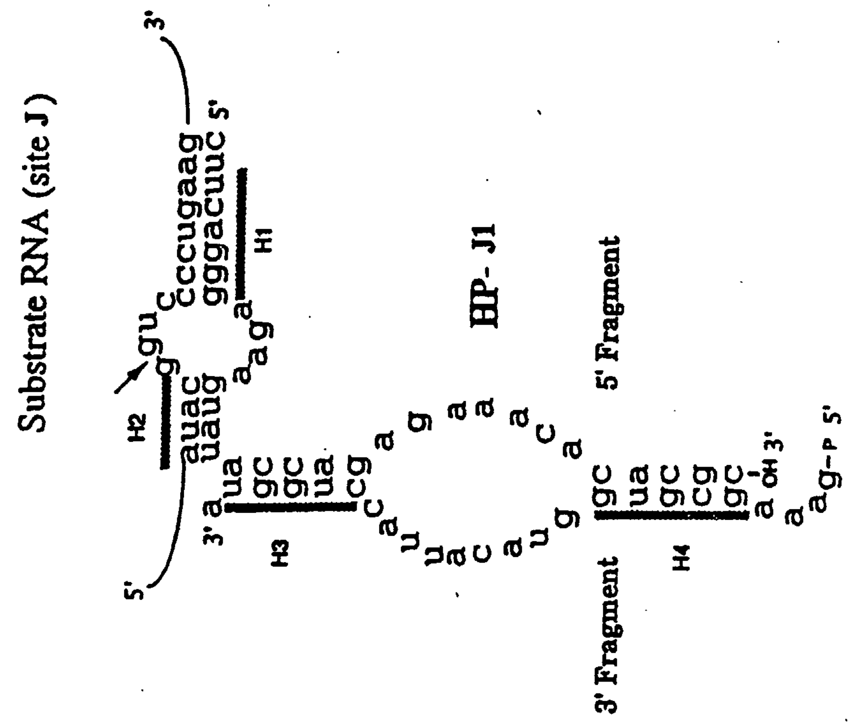

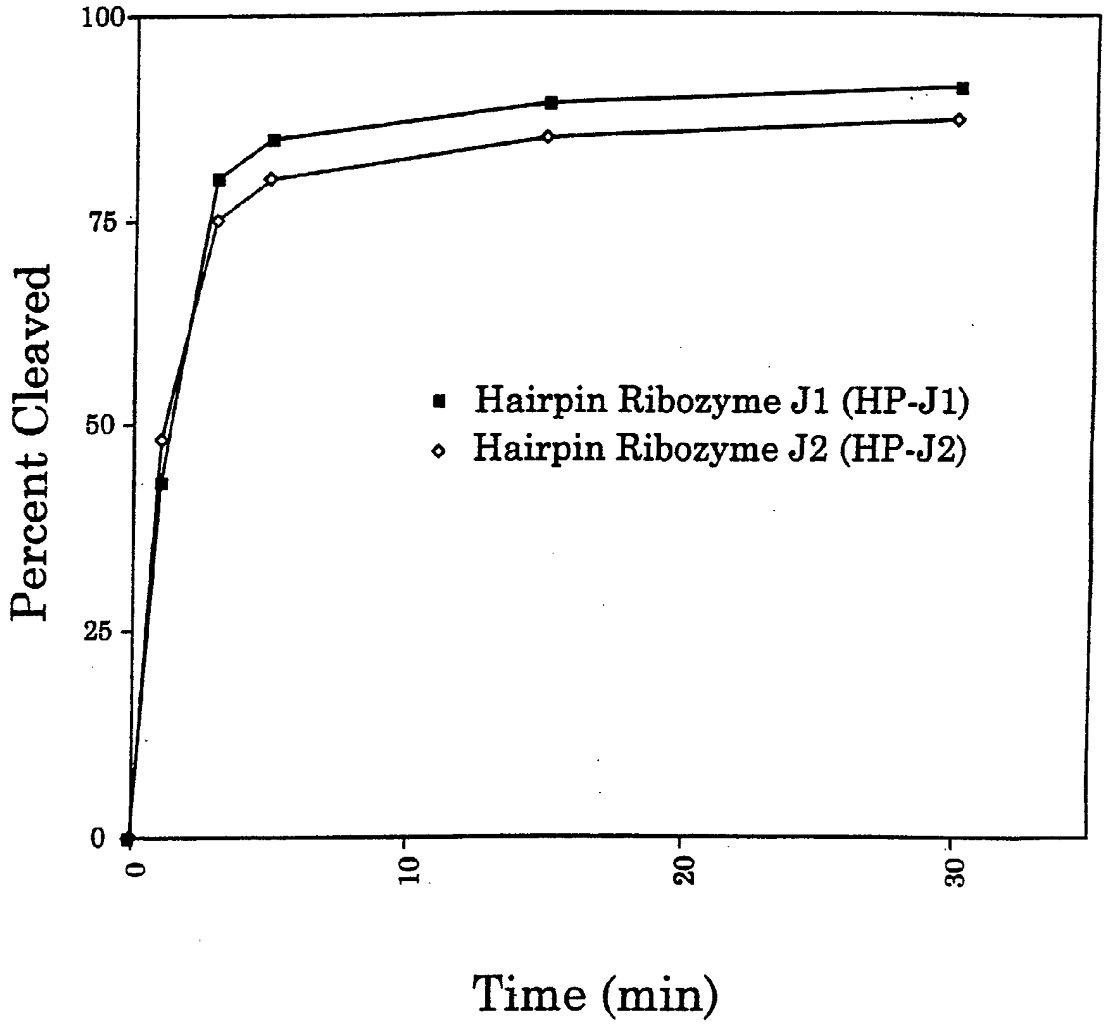
100
75
50
25
0
Percent Cleaved
0
10
20
30
Time (min)
Hairpin Ribozyme J1 (HP-J1)
Hairpin Ribozyme J2 (HP-J2)

*FIG. 66.*

FIG. 67a.

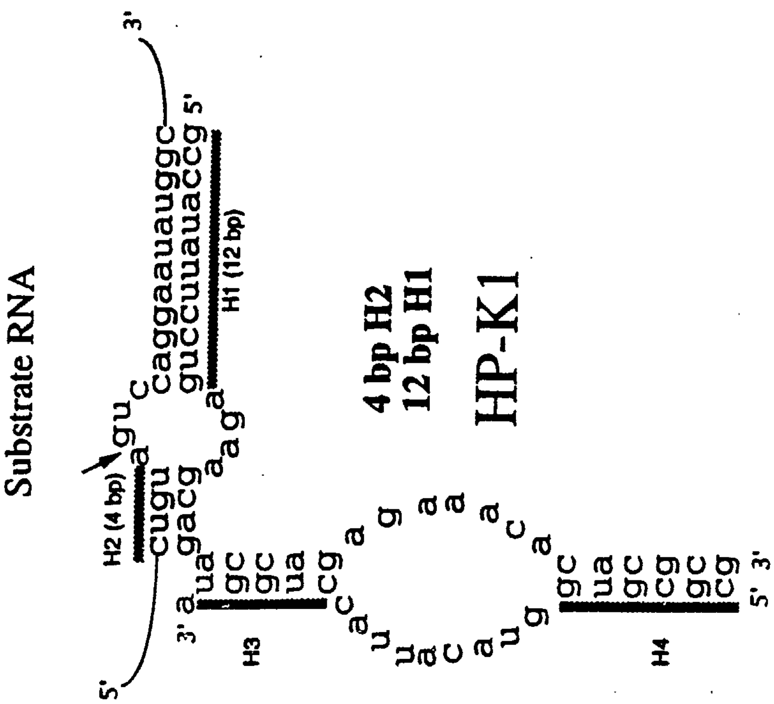


FIG. 67b.

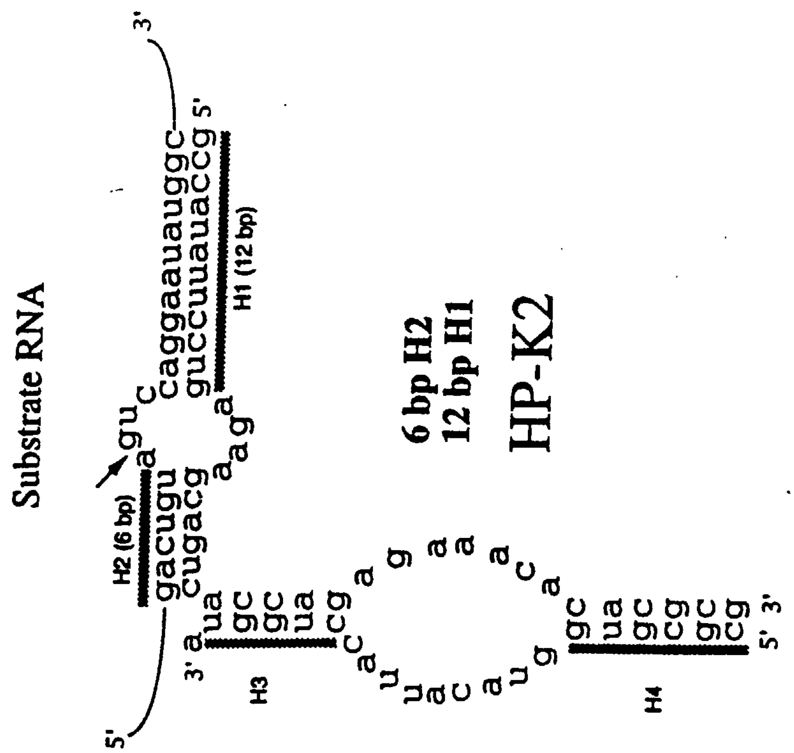

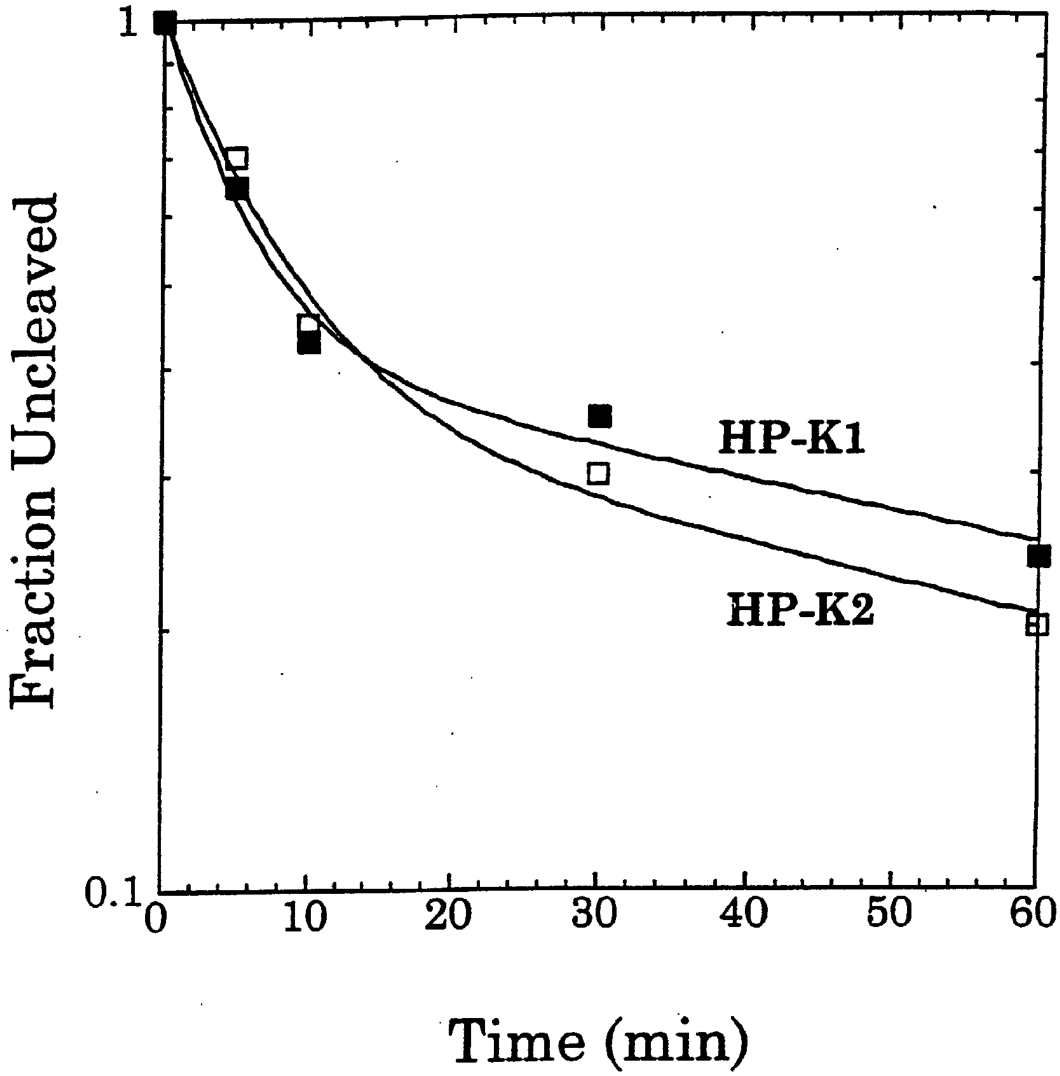


FIG. 68.

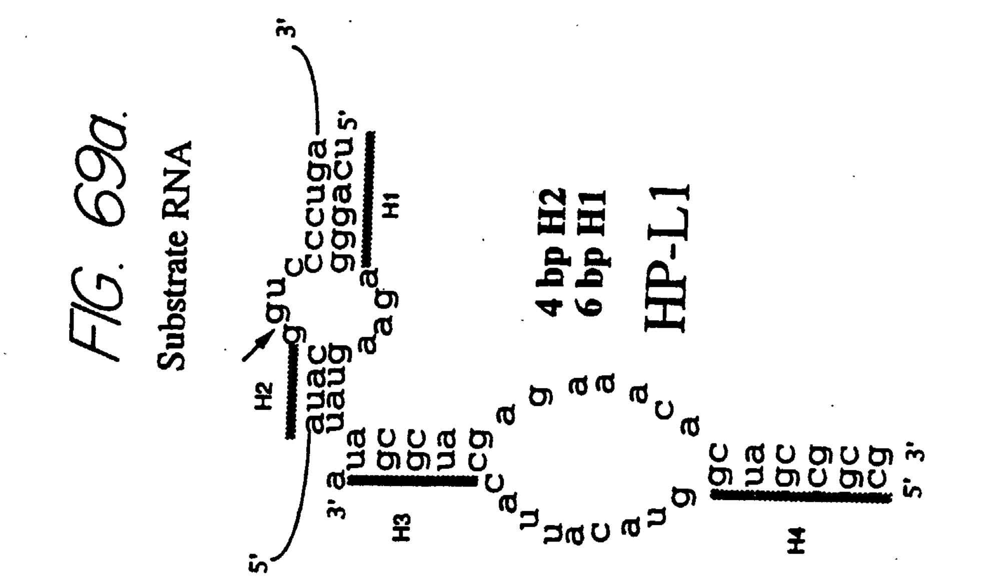


FIG. 69a.

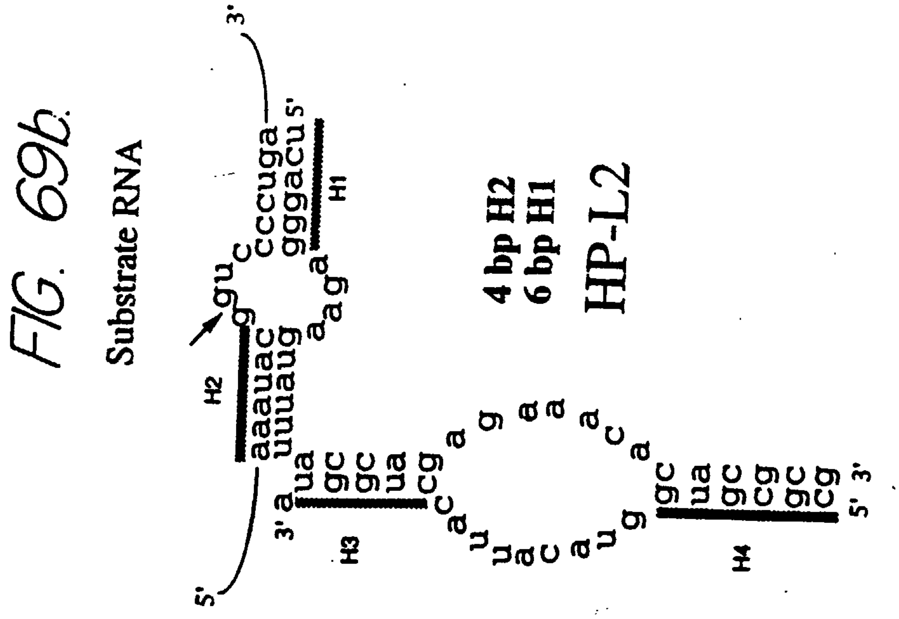


FIG. 69b.

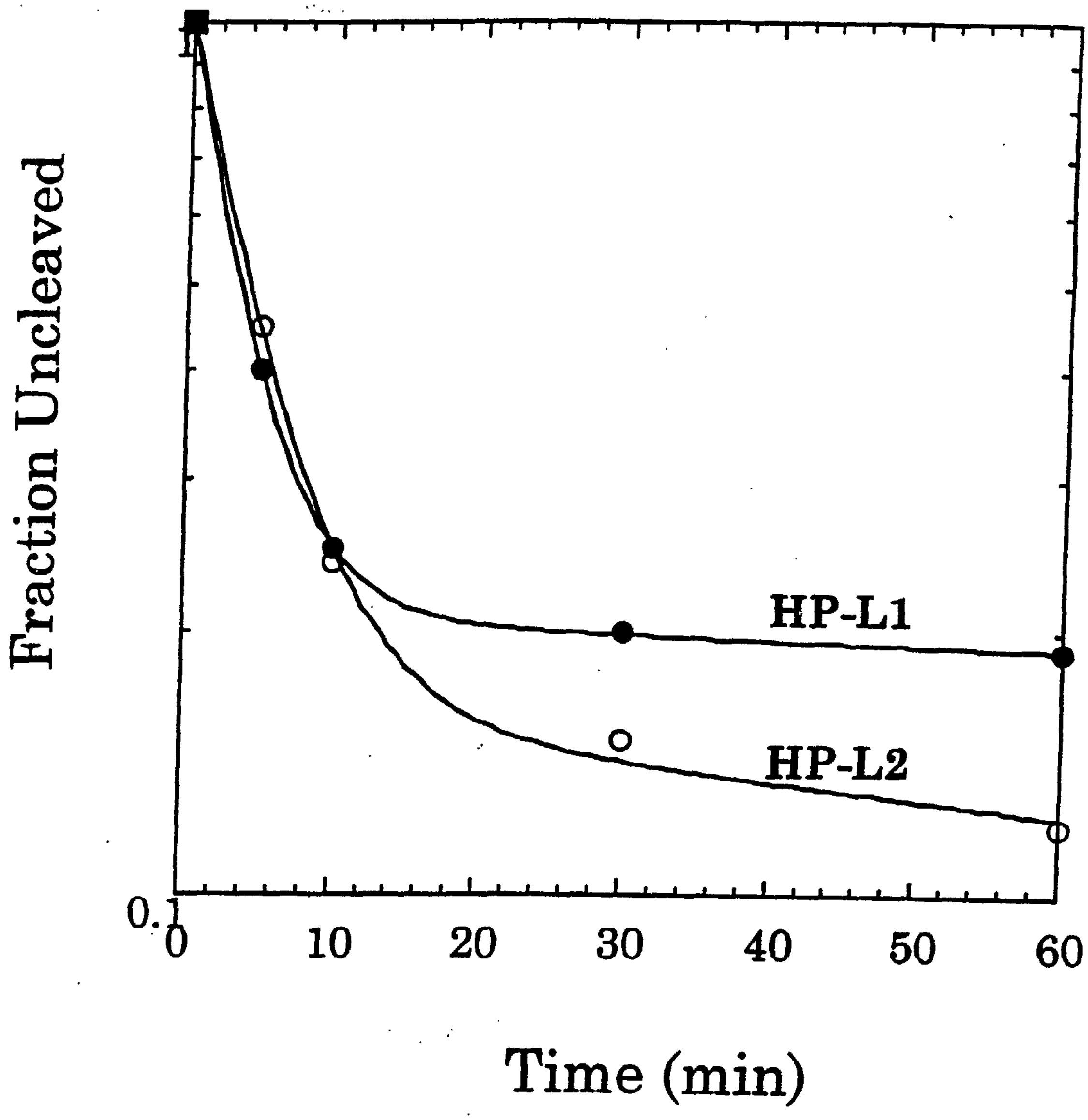


FIG. 70.

FIG. 7a.

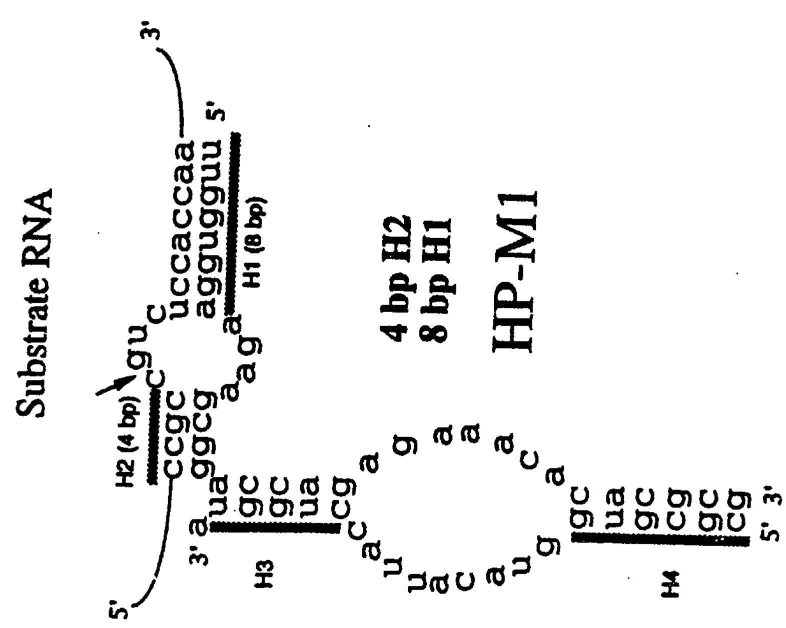
Substrate RNA
H2 (4 bp)
H1 (8 bp)
H3
H4
4 bp H2
8 bp H1
HP-M1

FIG. 7b.

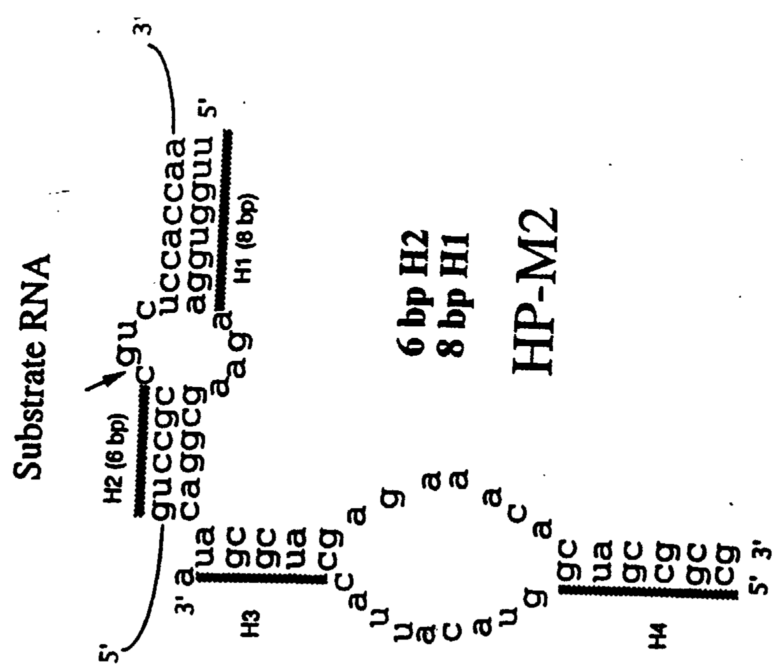
Substrate RNA
H2 (6 bp)
H1 (8 bp)
H3
H4
6 bp H2
8 bp H1
HP-M2

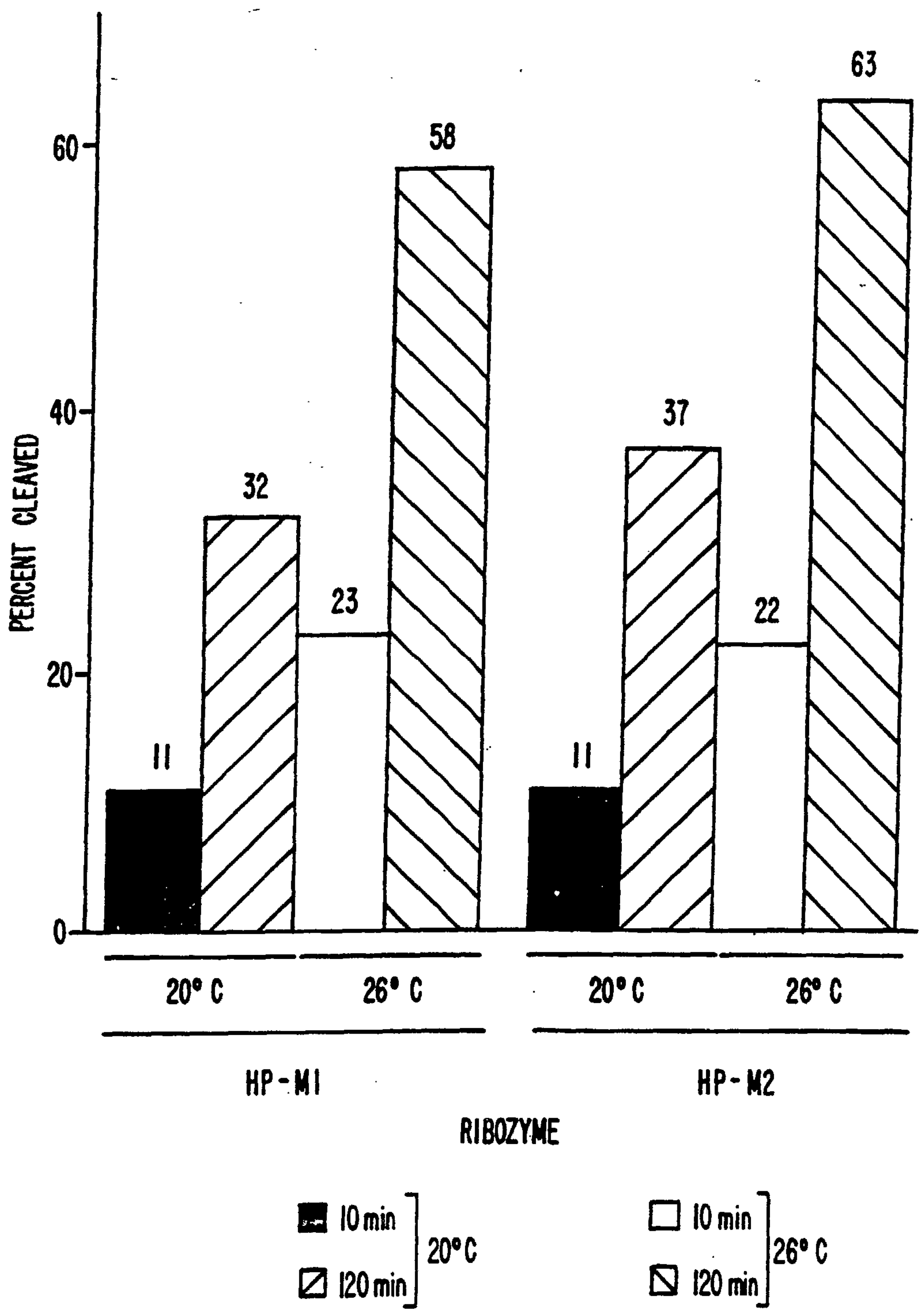

63
58
37
32
23
22
11
11
60
40
20
0
PERCENT CLEAVED
20° C
26° C
20° C
26° C
HP-M1
HP-M2
RIBOZYME
10 min
120 min
20° C
10 min
120 min
26° C


FIG. 72.

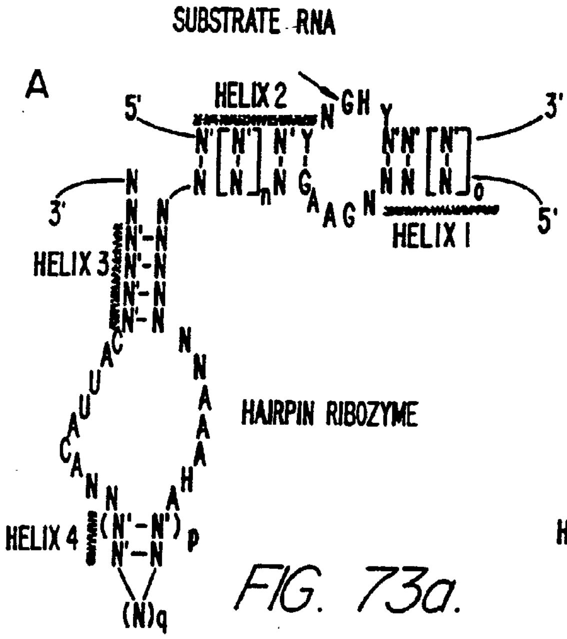


FIG. 73a.

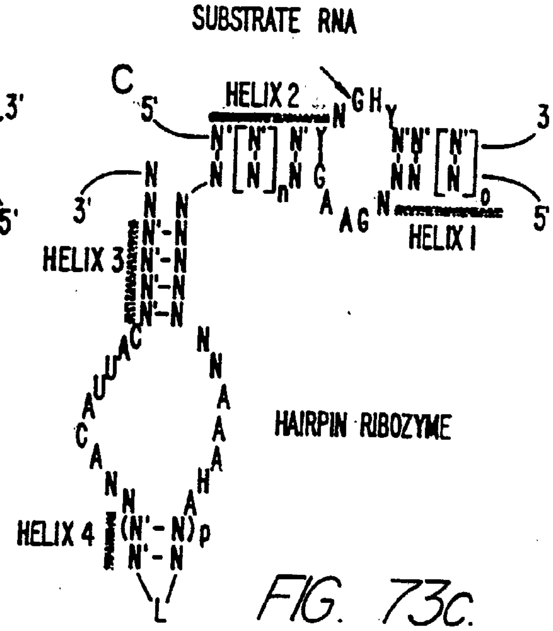


FIG. 73c.

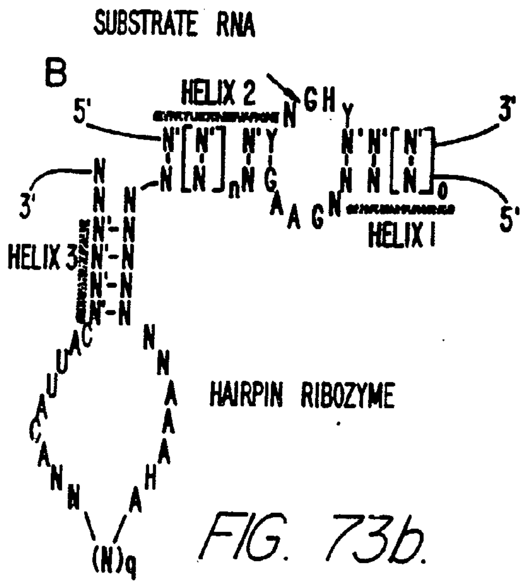


FIG. 73b.

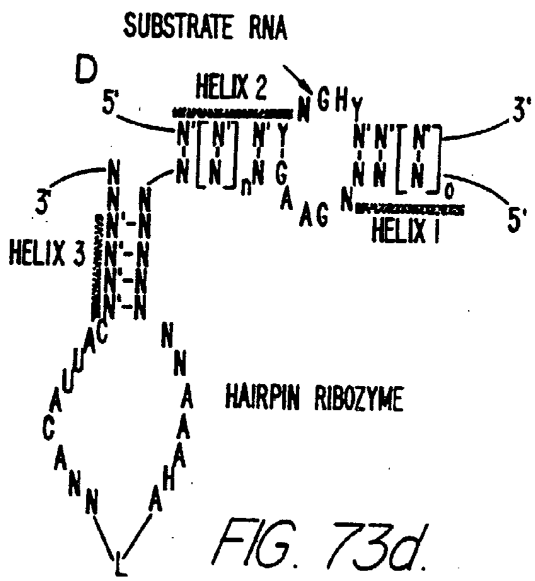


FIG. 73d.

A

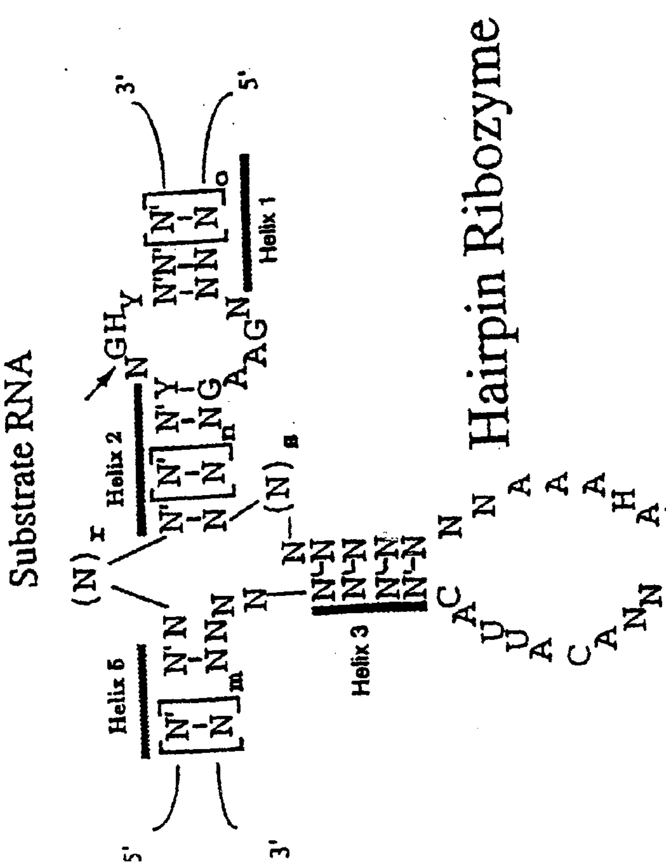


FIG. 74a.

B

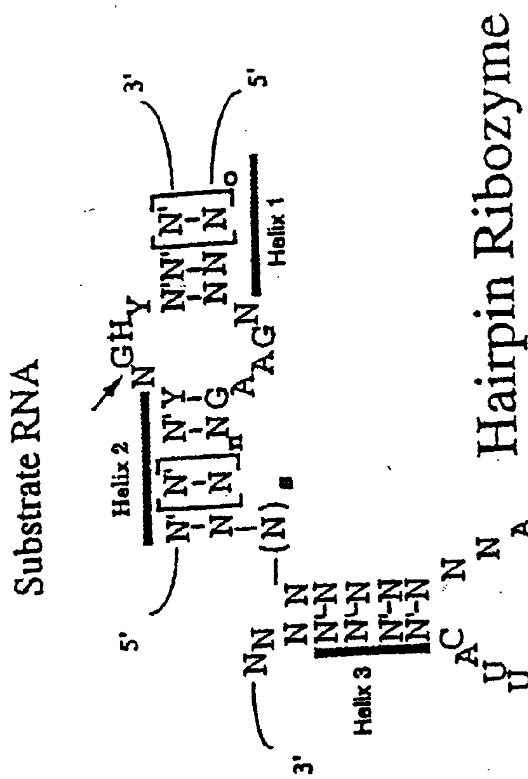


FIG. 74b.

FIG. 75a.

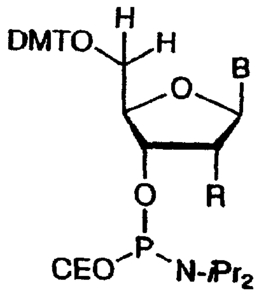

1

D-Ribose Family

FIG. 75b.

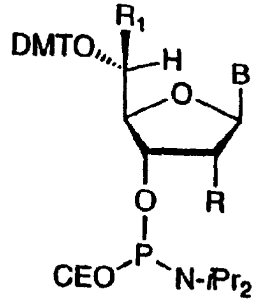

2

D-Allose Family

FIG. 75c.

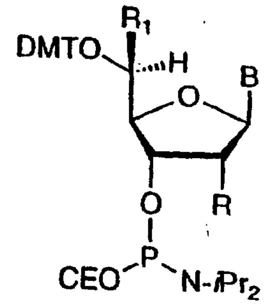

3

L-Talose Family

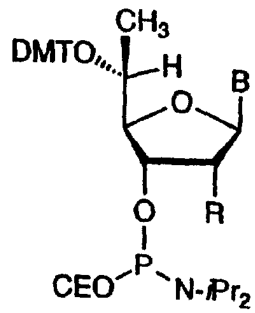

29-32

FIG. 75d.

D-Allose

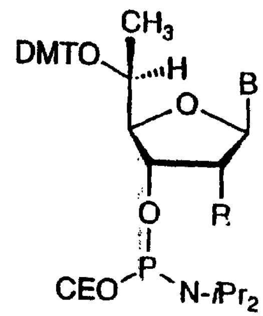

58-61

FIG. 75e.

L-Talose

B = Protected A, C, G, U, T, 2AP, I, DiAP, P *etc.*

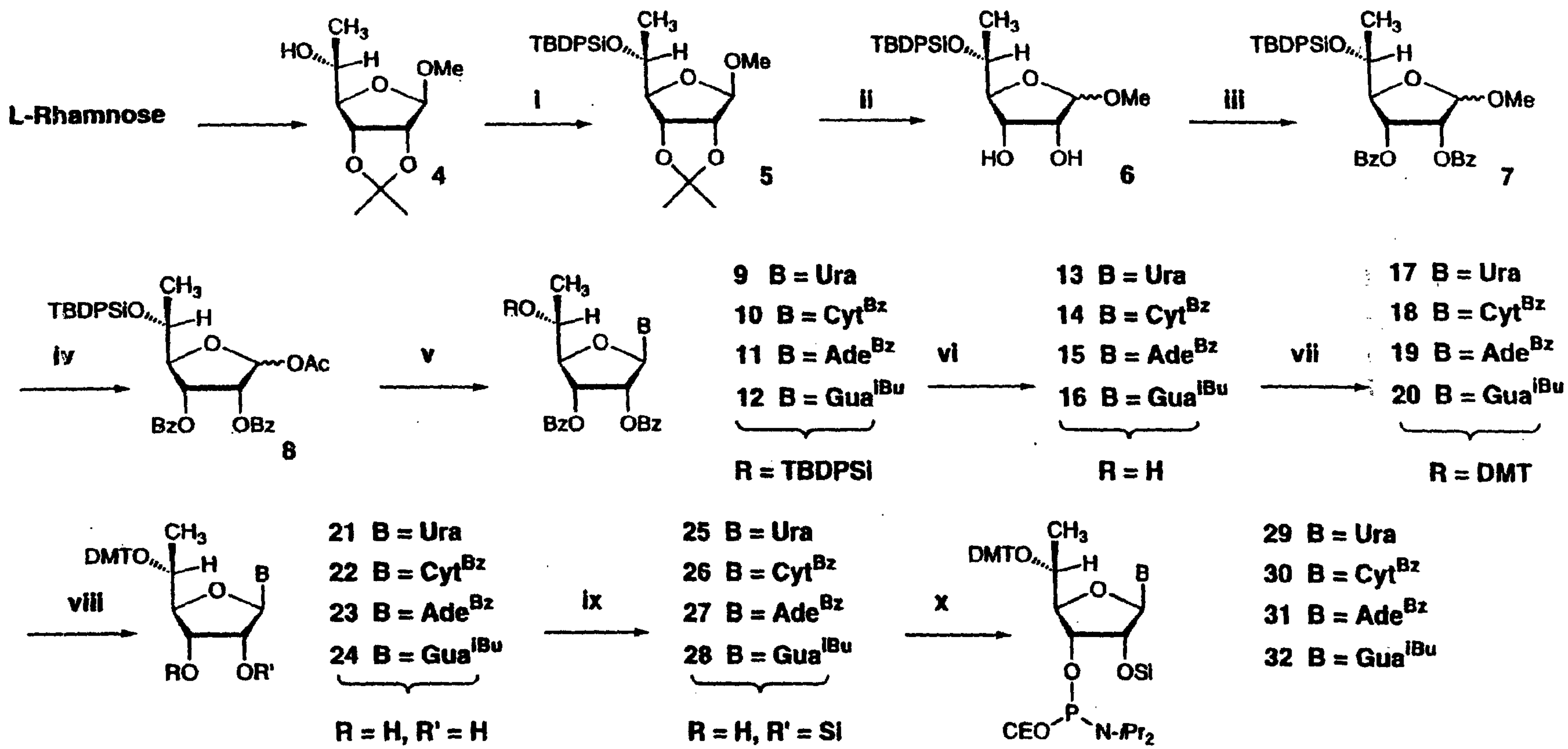


i) = TBDPSi-Cl
ii) = $H^+$
iii) = Bz-Cl/Pyr
iv) = $AcOH/Ac_2O/H^+$
v) = $B^{TMS}/CF_3SO_3SiMe_3$
vi) = TBAF
vii) = $DMT\text{-}Cl/AgNO_3$
viii) = $OH^-$
ix) = TBDMSi-Cl
x) = $P(OCE)(N\text{-}iPr_2)Cl$

FIG. 76.

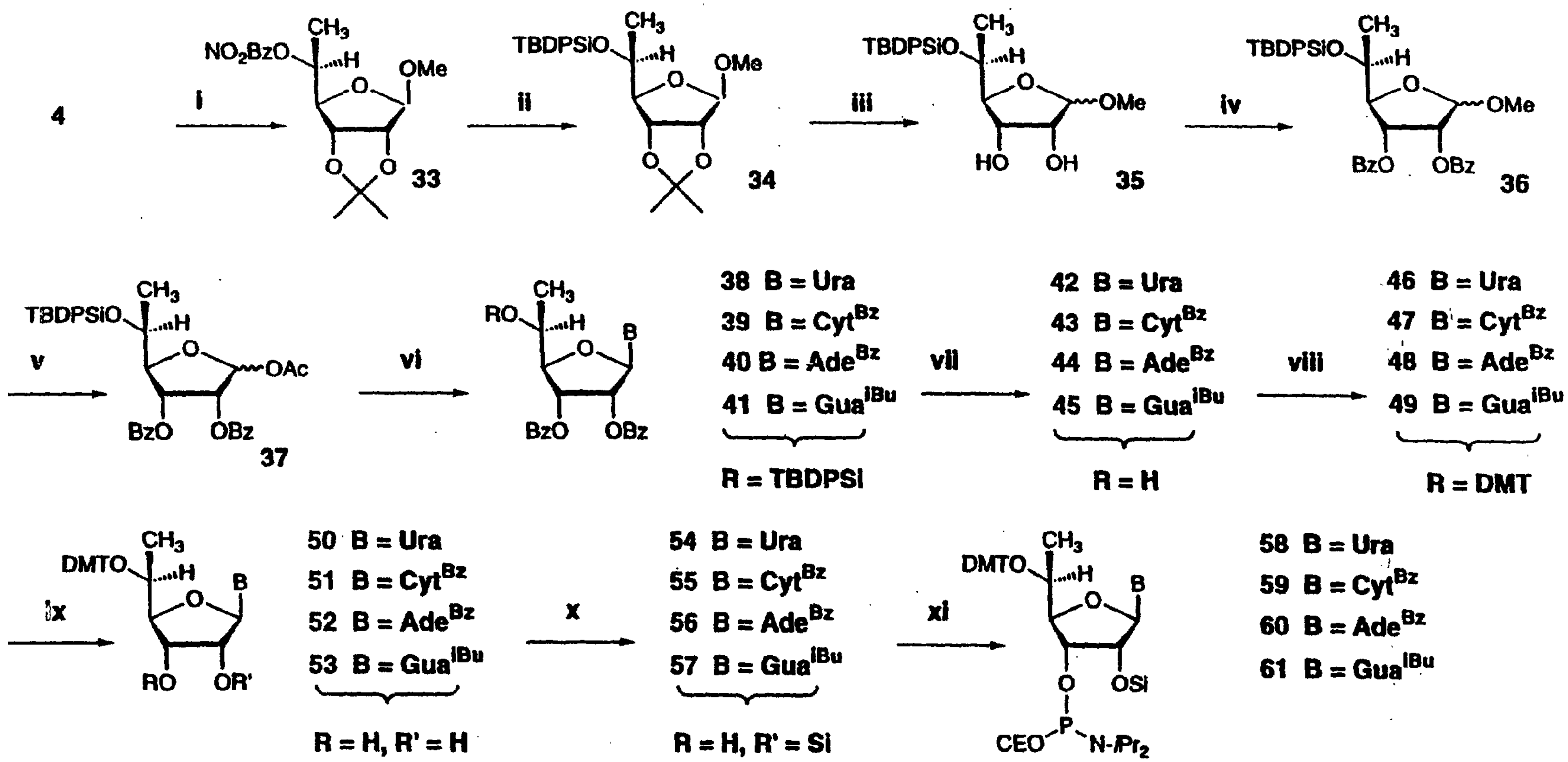


i) = $Ph_3P$/DEAD/p-$NO_2PhCOOH$
ii) = $OH^-$, TBDPSi-Cl
iii) = $H^+$
iv) = Bz-Cl/Pyr
v) = $AcOH/Ac_2O/H^+$
vi) = $B^{TMS}/CF_3SO_3SiMe_3$
vii) = TBAF
viii) = DMT-Cl/$AgNO_3$
ix) = $OH^-$
x) = TBDMSi-Cl
xi) = $P(OCE)(N\text{-}iPr_2)Cl$

FIG. 77.

# FIG. 78.

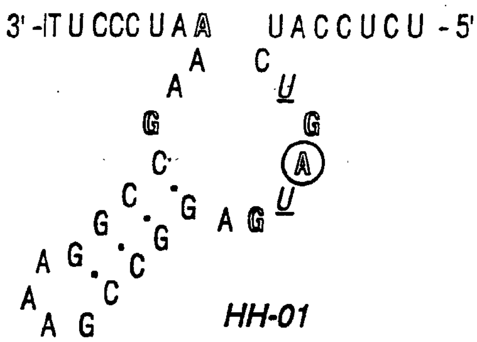


HH-01

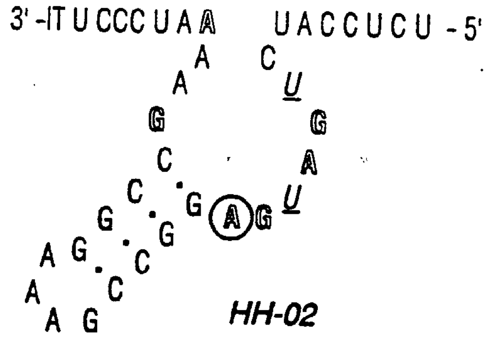


HH-02

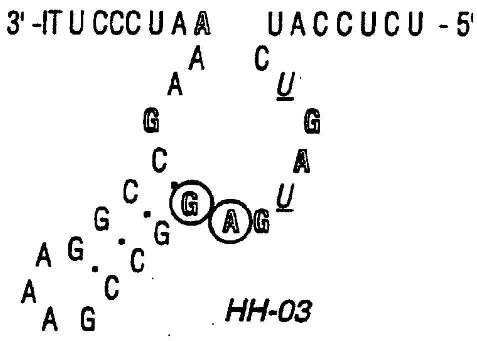


HH-03

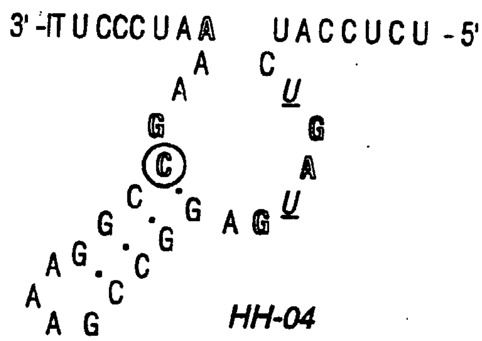


HH-04

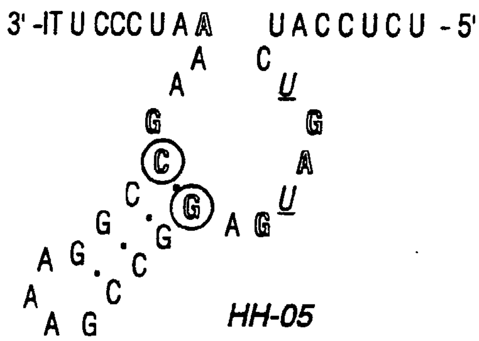


HH-05

iT =

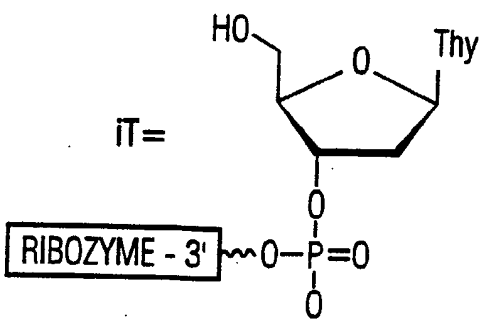

| | |
|---|---|
| N = 2'-*O*-Me | N = RIBO |
| *U* = 2'-$NH_2$ U | (N) = TALO |

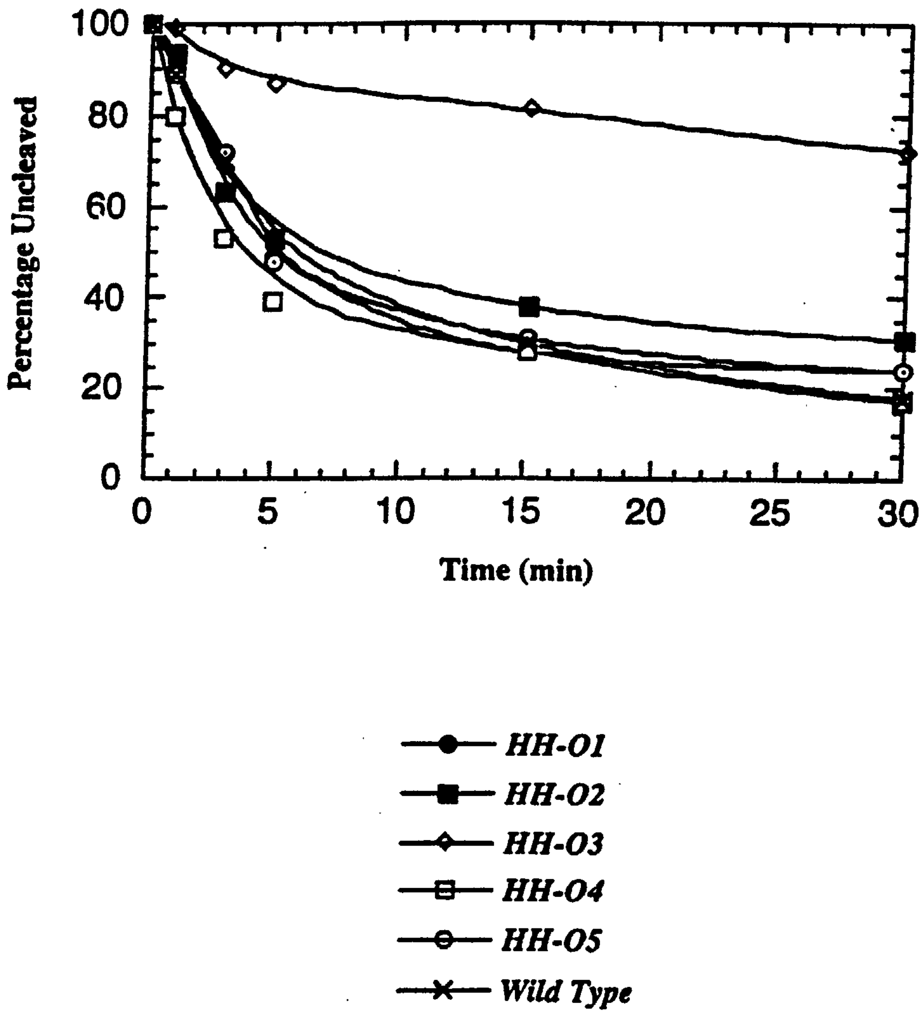
100
80
60
40
20
0
Percentage Uncleaved
0
5
10
15
20
25
30
Time (min)
HH-O1
HH-O2
HH-O3
HH-O4
HH-O5
Wild Type

FIG. 79.

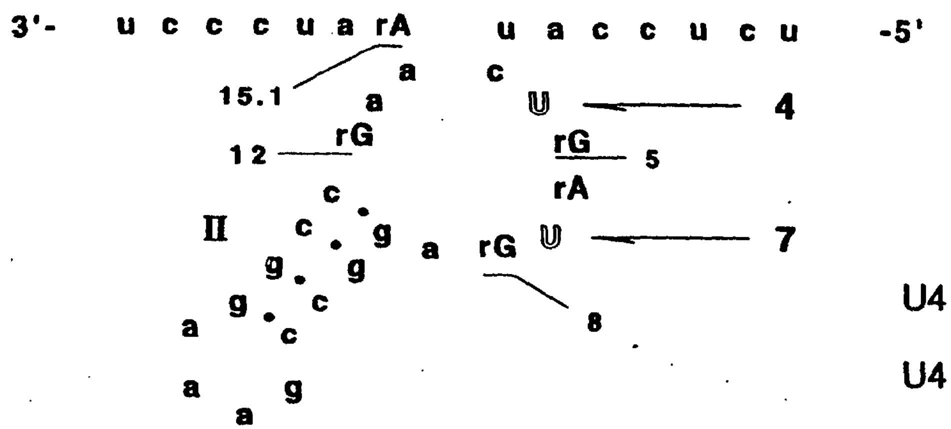


Lower case = 2'-*O*-Me

rN = ribonucleotide

| | | Table 1 Entries |
|---|---|---|
| U4 & U7 = | 2'-C-Allyl-U | 12-14 |
| U4 & U7 = | 2'-F-ribo-U | 9-11 |
| U4 & U7 = | 2'=$CH_2$-U | 3-5 |
| U4 & U7 = | 2'=$CF_2$-U | 6-8 |
| U4 & U7 = | 2'-dU | 21-22 |
| U4 & U7 = | 2'-F-ara-U | 15-17 |
| U4 & U7 = | 2'-$NH_2$-U | 18-20 |
| U4 & U7 = | 2'-O-Me-ribo-U | 2 |

*FIG. 80.*

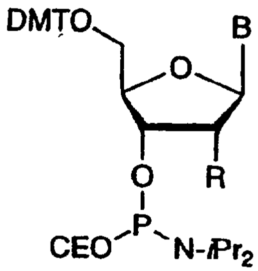

1

FIG. 81a.

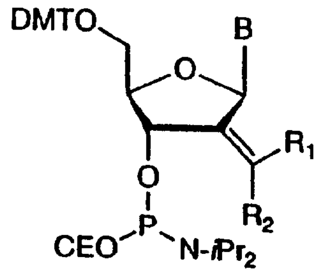

2

FIG. 81b.

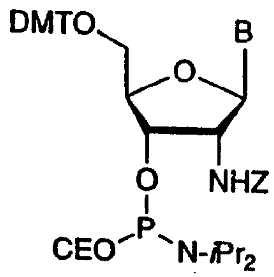

3

FIG. 81c.

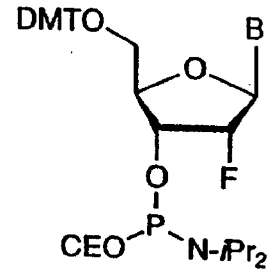

4

FIG. 81d.

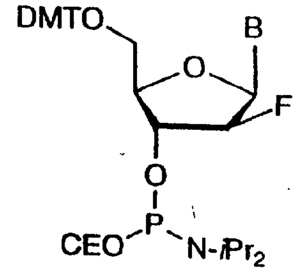

5

FIG. 81e.

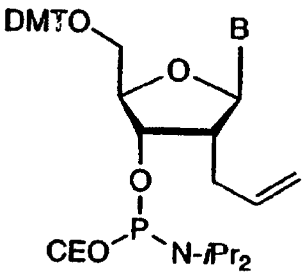

10 & 12

FIG. 81f.

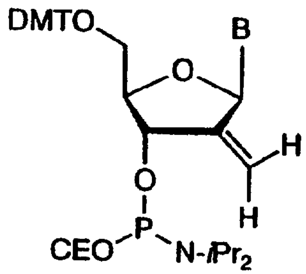

17, 22 & 31

FIG. 81g.

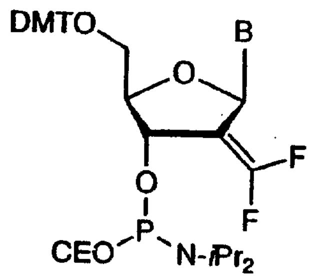

18, 26 & 32

FIG. 81h.

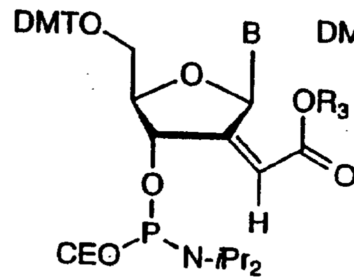

36

FIG. 81i.

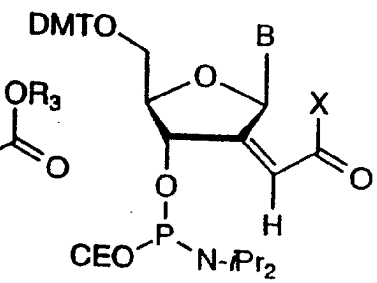

38

FIG. 81j.

B = Protected A, C, G, U, T, 2AP, I, DiAP, P *etc.*

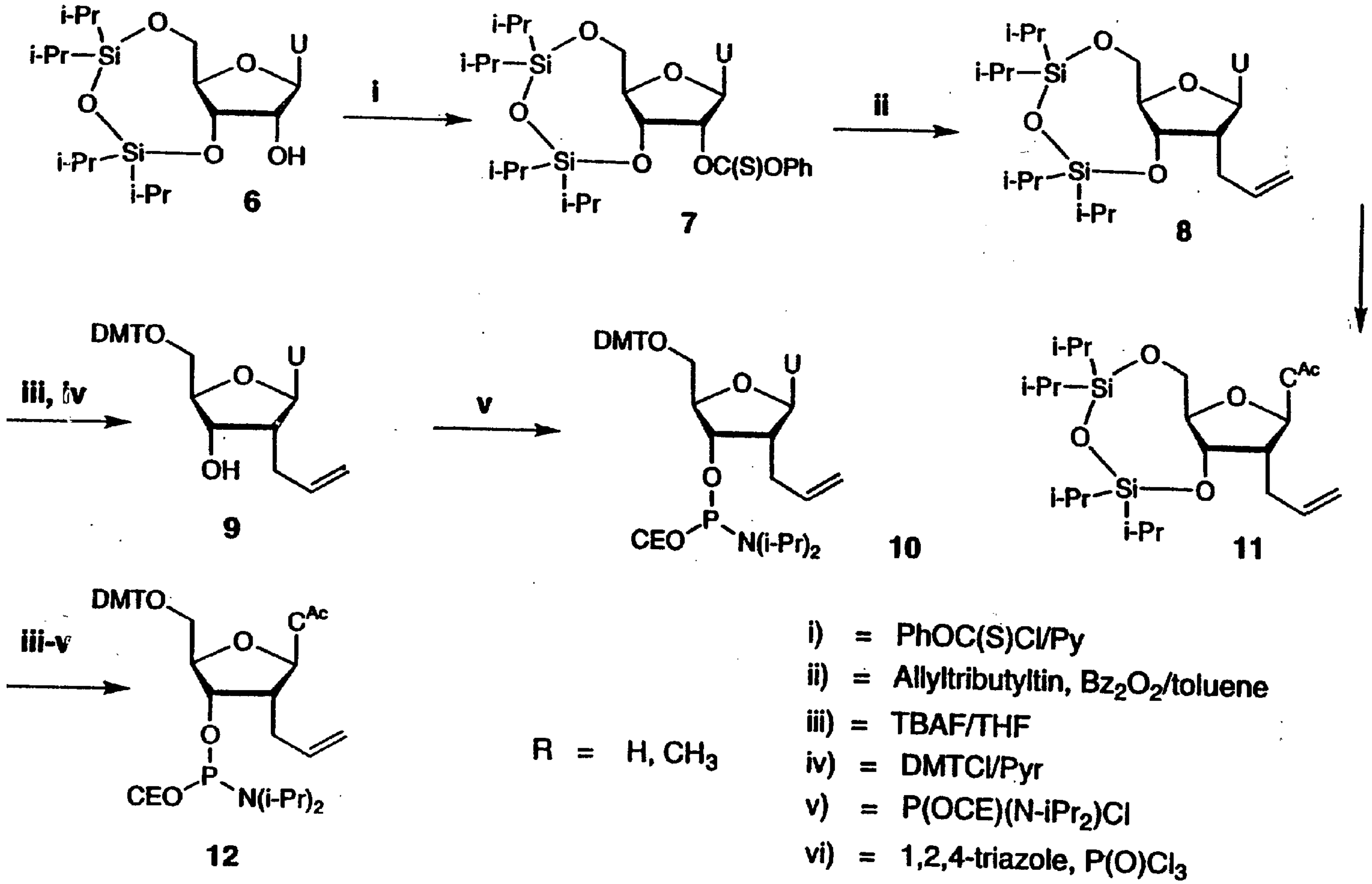


R = H, $CH_3$

i) = PhOC(S)Cl/Py
ii) = Allyltributyltin, $Bz_2O_2$/toluene
iii) = TBAF/THF
iv) = DMTCl/Pyr
v) = P(OCE)(N-i$Pr_2$)Cl
vi) = 1,2,4-triazole, P(O)$Cl_3$
vii) = 29% $NH_4OH$/dioxane, $Ac_2O$/Pyr

FIG. 82.

FIG. 83.

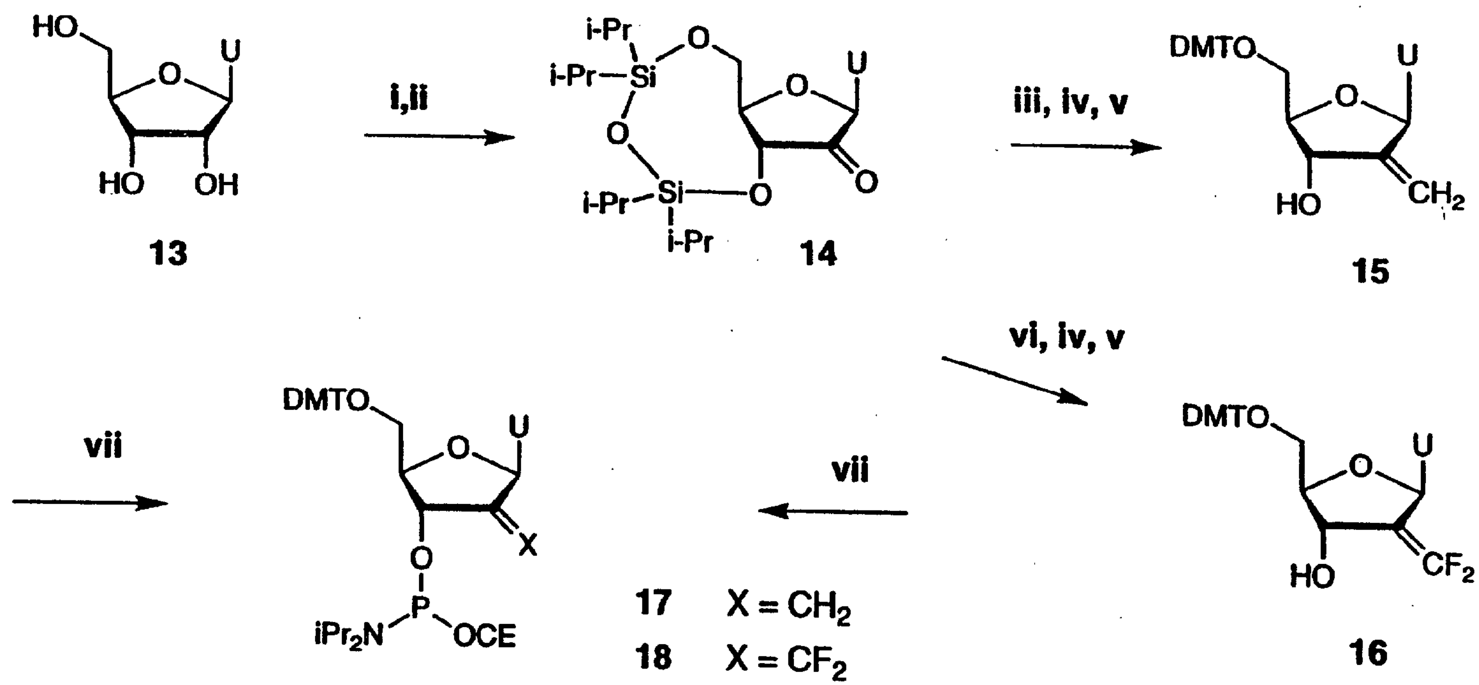


13

14

15

16

17 X = $CH_2$

18 X = $CF_2$

i) = Markiewicz reagent

ii) = DMSO & $Ac_2O$

iii) = $Ph_3PCH_3I$

iv) = TBAF/THF

v) = DMTCl/Pyr

vi) = $Ph_3P$, $ClCF_2COONa$

vii) = $P(OCE)(N\text{-}iPr_2)Cl$

FIG. 84.

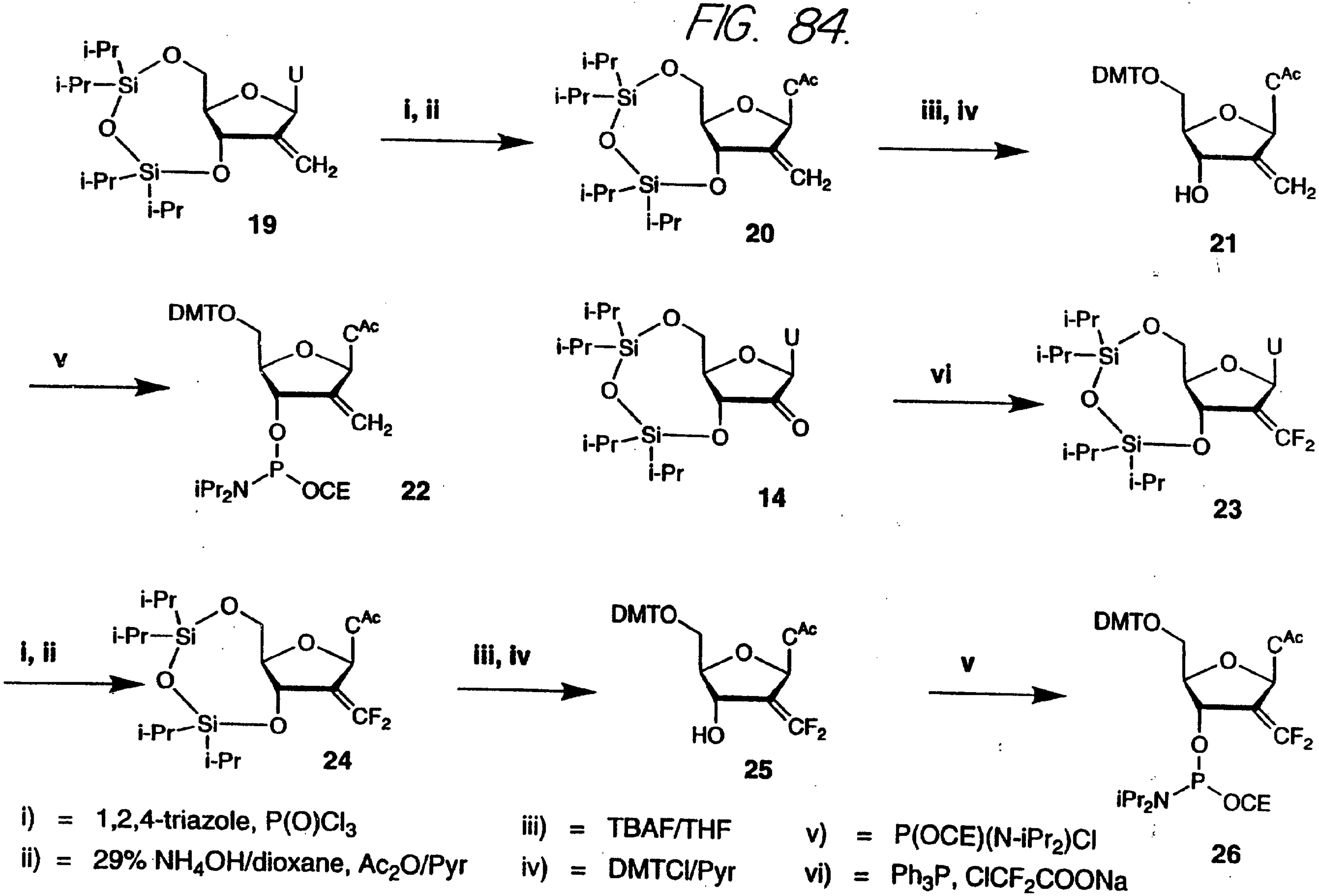


19 20 21 22 14 23 24 25 26

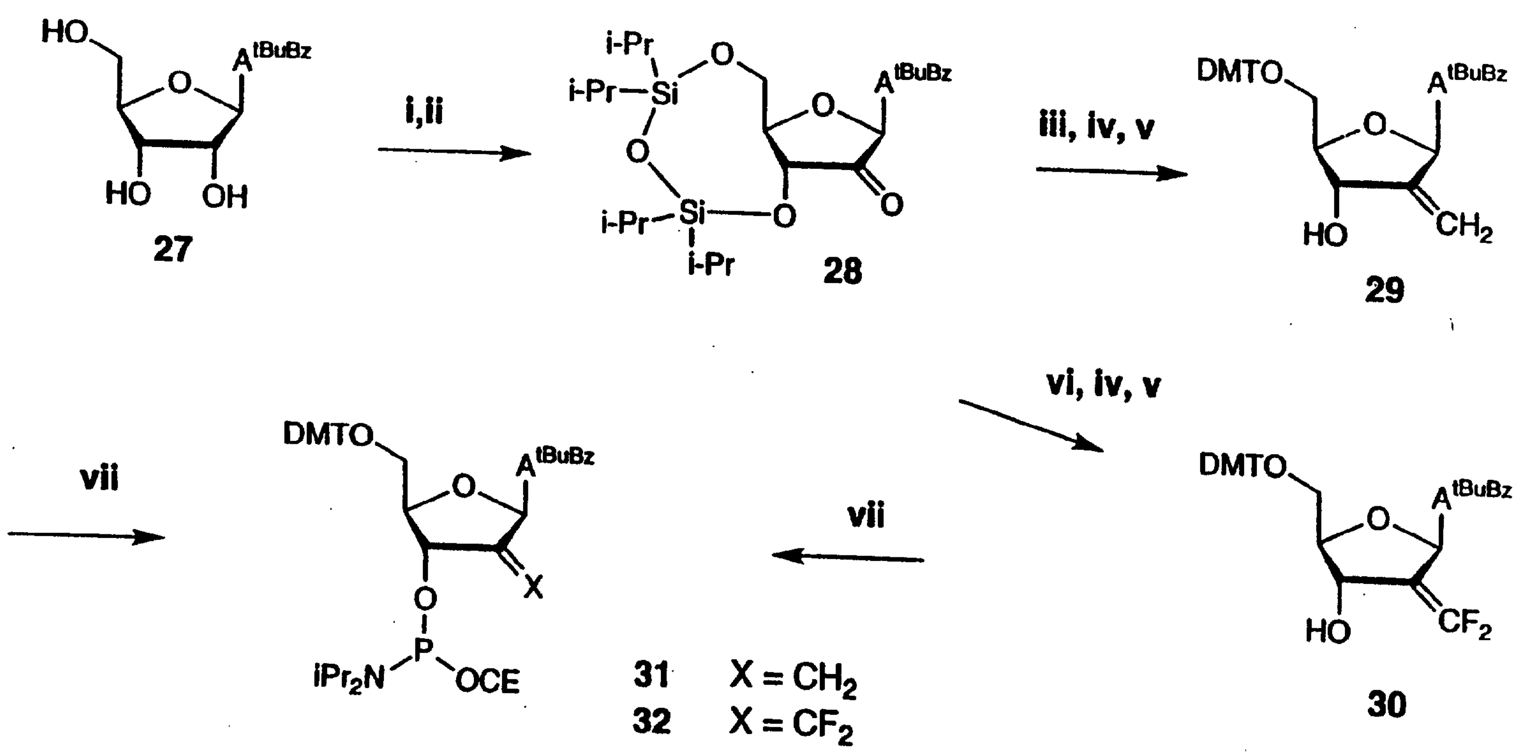


27 28 29 30

31 X = $CH_2$
32 X = $CF_2$

i) = Markiewicz reagent
ii) = DMSO & $Ac_2O$
iii) = $Ph_3PCH_3I$
iv) = TBAF/THF
v) = DMTCl/Pyr
vi) = $Ph_3P$, $ClCF_2COONa$
vii) = $P(OCE)(N\text{-}iPr_2)Cl$

FIG. 85.

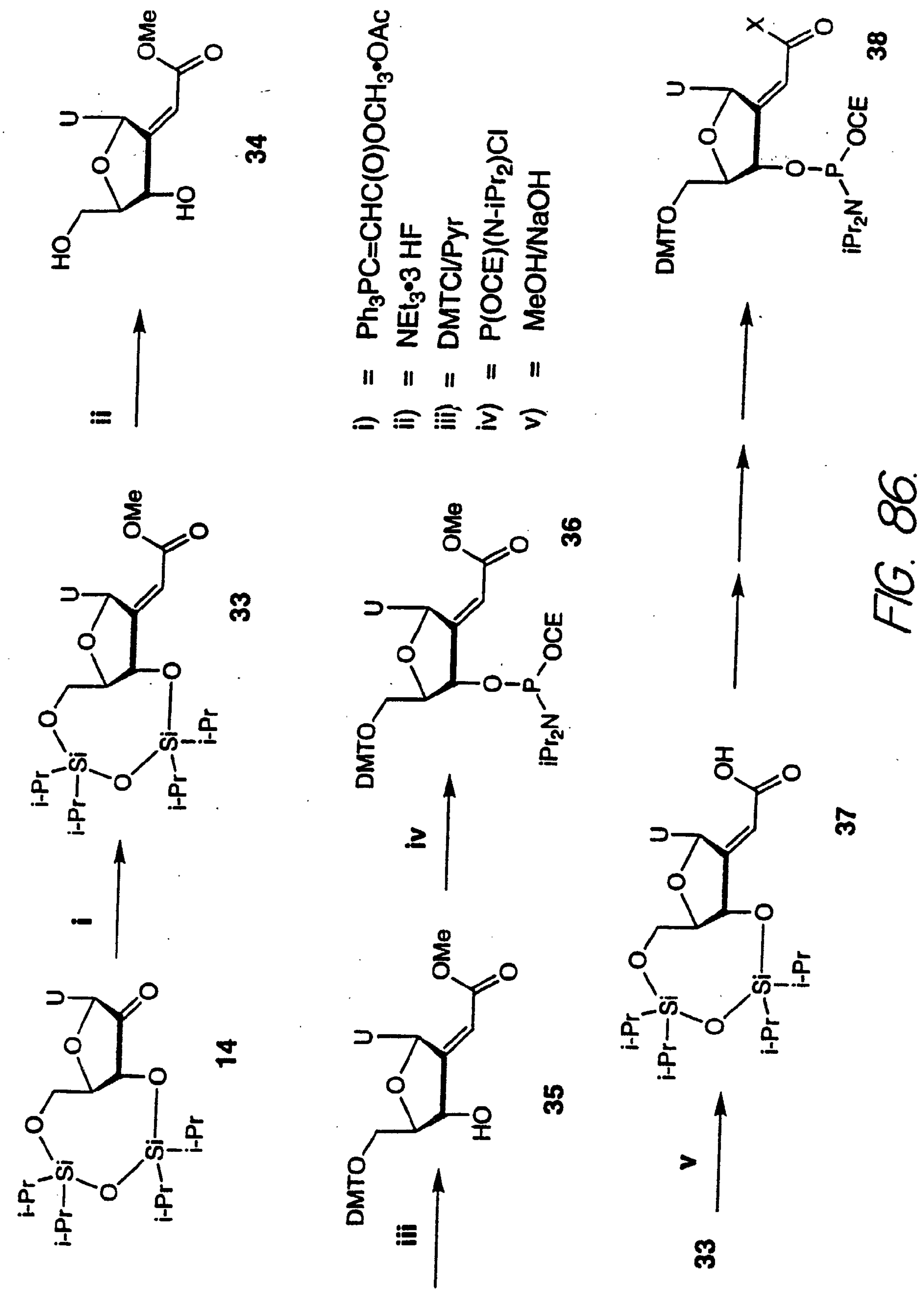


14 33 34 35 36 33 37 38

FIG. 86.

FIG. 87.

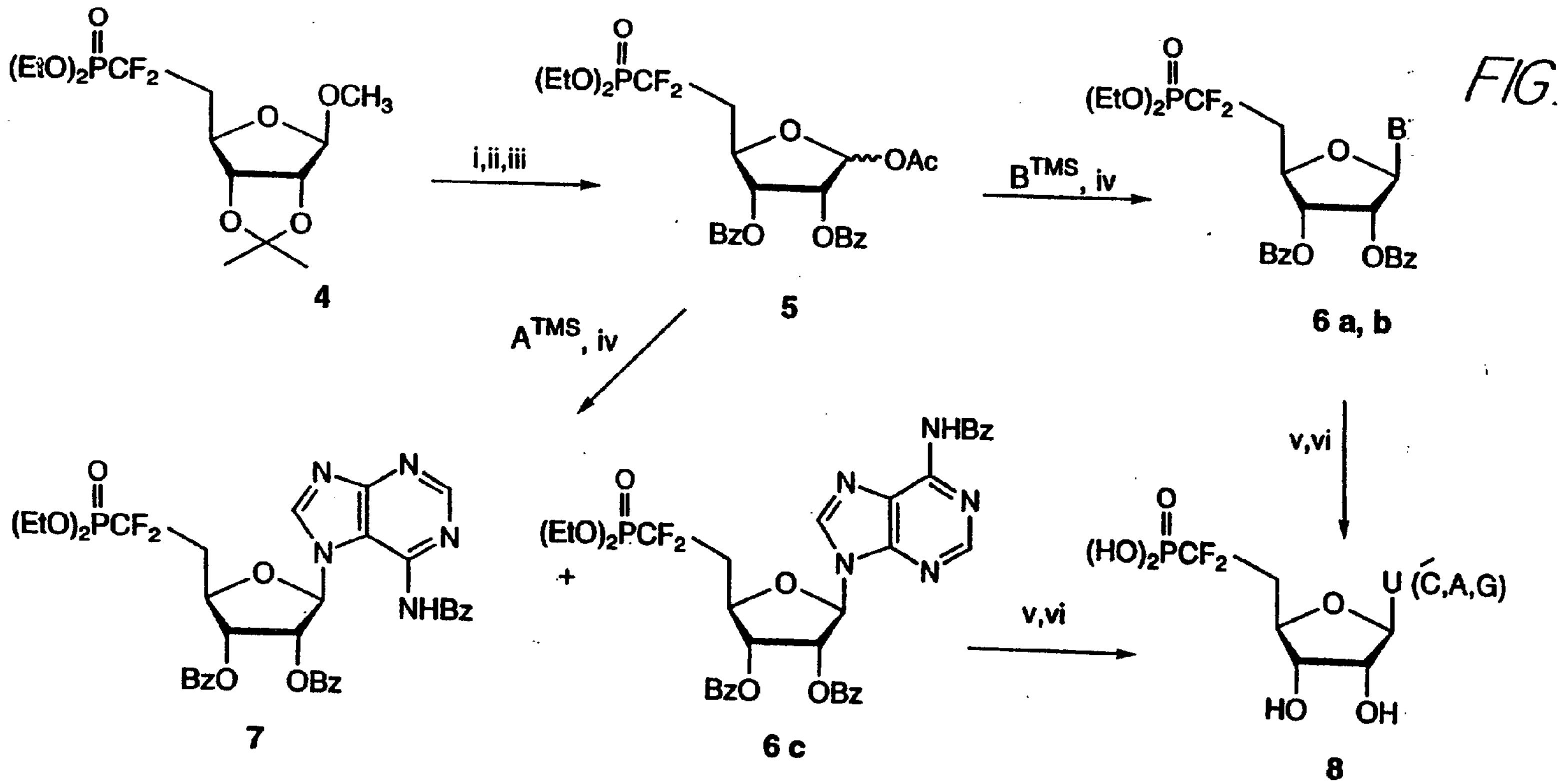


4

5

6 a, b

7

6 c

8

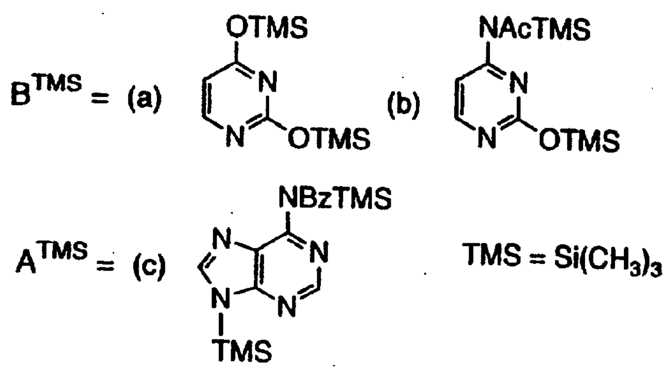


TMS = $Si(CH_3)_3$

**Reagents and Conditions:**

i) $I_2$-MeOH, reflux, 18 h or Dowex 50 WX8 ($H^+$), MeOH, RT, 3 days
ii) BzCl, Py, RT, 16 h
iii) $Ac_2O$, AcOH, $H_2SO_4$, EtOAc, 0 °C, 18 h
iv) $SnCl_4$, $CH_3CN$, reflux, 2 h
v) $(CH_3)_3SiBr$, DMF, RT, 72 h
vi) conc. $NH_4OH$-MeOH (3:1), 60 °C, 18 h

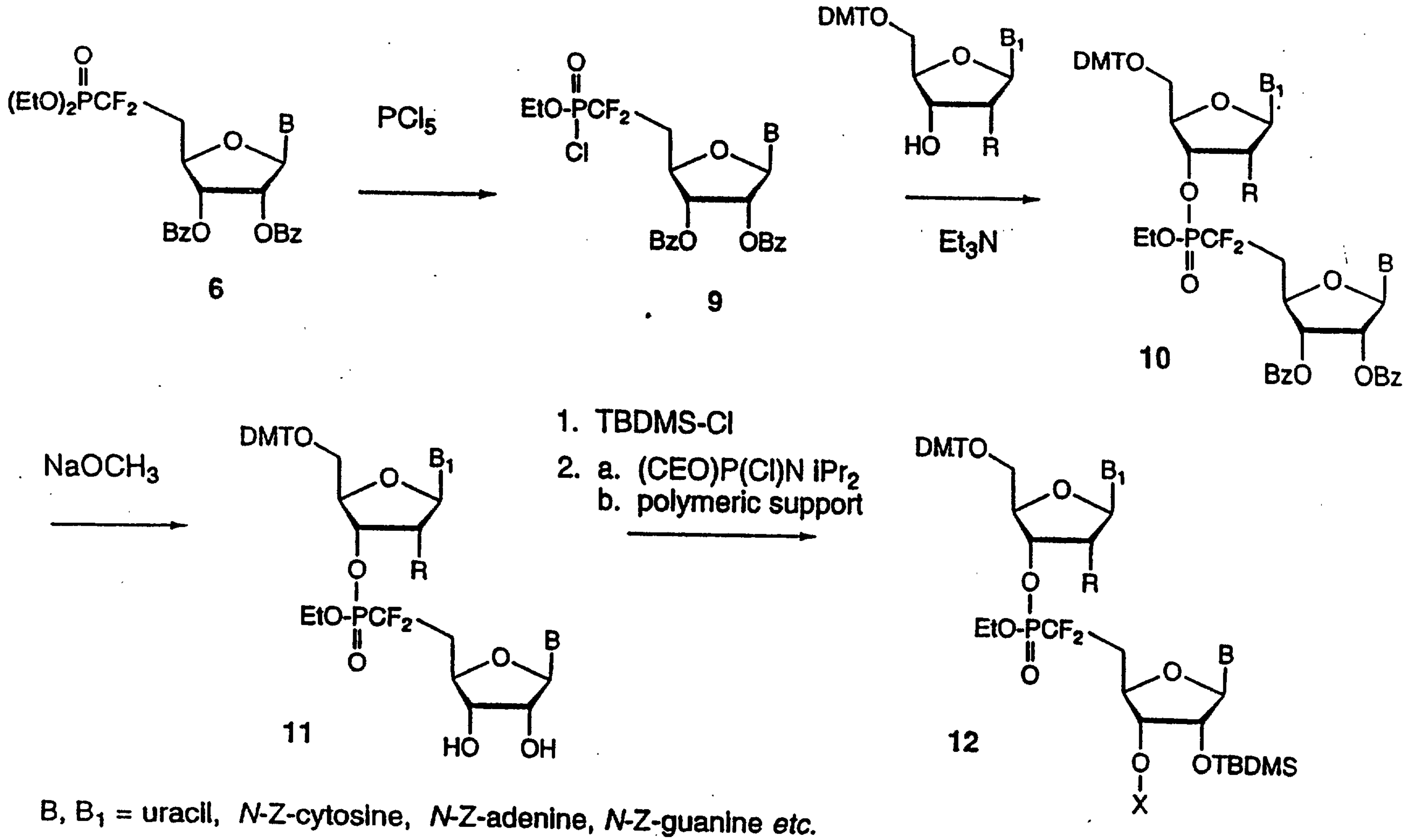


6 9 10 11 12

B, B$_1$ = uracil, *N*-Z-cytosine, *N*-Z-adenine, *N*-Z-guanine *etc.*

Z = amino-protecting group

R = OTBDMS, OCH$_3$, H

a. X = P(OCE)N iPr$_2$

X = polymeric support

FIG. 88.

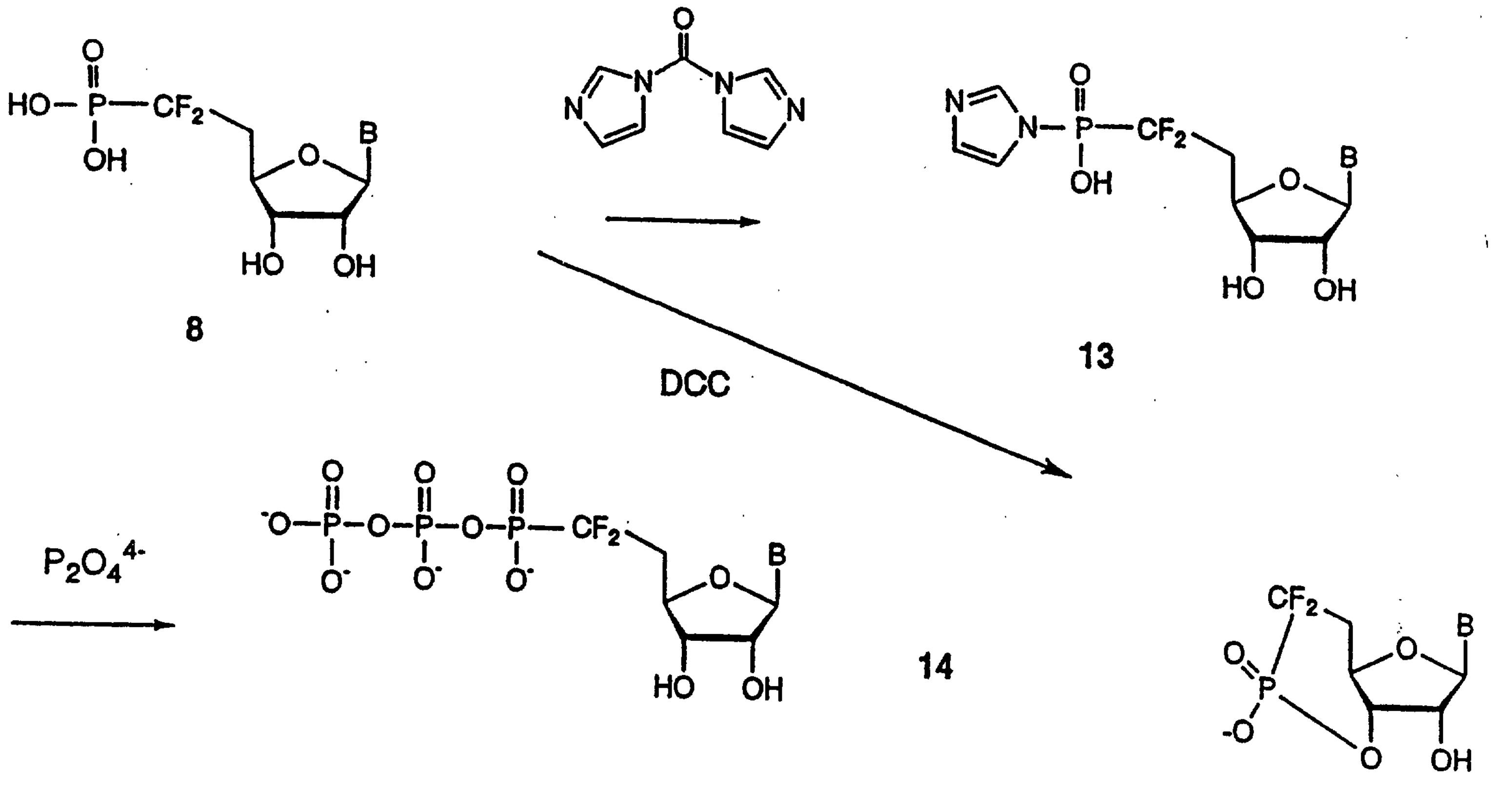


8

13

14

15

B = uracil, *N*-Z-cytosine, *N*-Z-adenine, *N*-Z-guanine *etc.*

Z = amino-protecting group

FIG. 89.

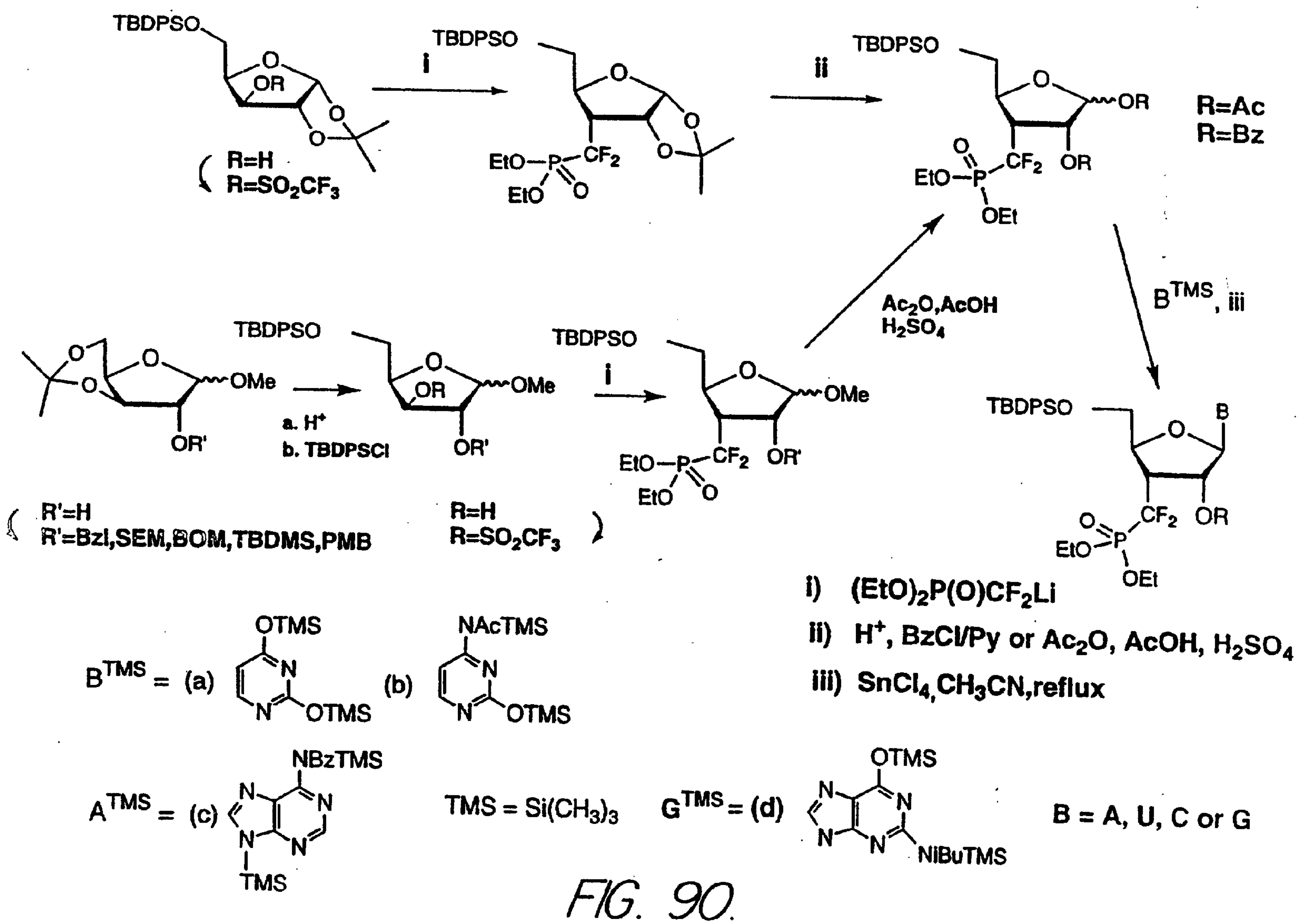
i
ii
a. H+
b. TBDPSCl
i
Ac2O,AcOH
H2SO4
BTMS, iii
i) (EtO)2P(O)CF2Li
ii) H+, BzCl/Py or Ac2O, AcOH, H2SO4
iii) SnCl4,CH3CN,reflux
TMS = Si(CH3)3
B = A, U, C or G

FIG. 90

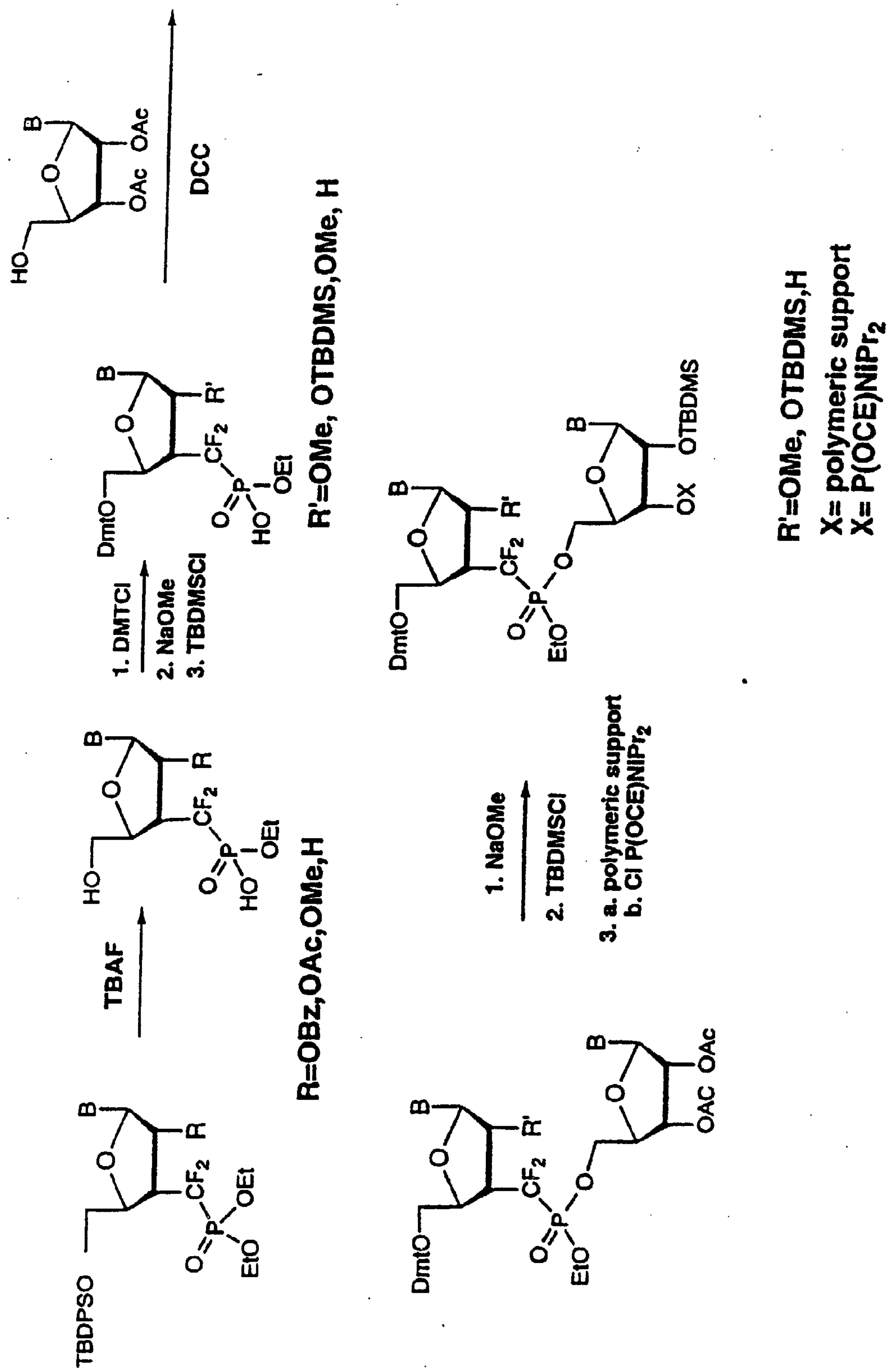
DCC
1. DMTCl
2. NaOMe
3. TBDMSCl
TBAF
R=OBz,OAc,OMe,H
R'=OMe, OTBDMS,OMe, H
1. NaOMe
2. TBDMSCl
3. a. polymeric support
b. Cl P(OCE)NiPr$_2$
R'=OMe, OTBDMS,H
X= polymeric support
X= P(OCE)NiPr$_2$

FIG. 91.

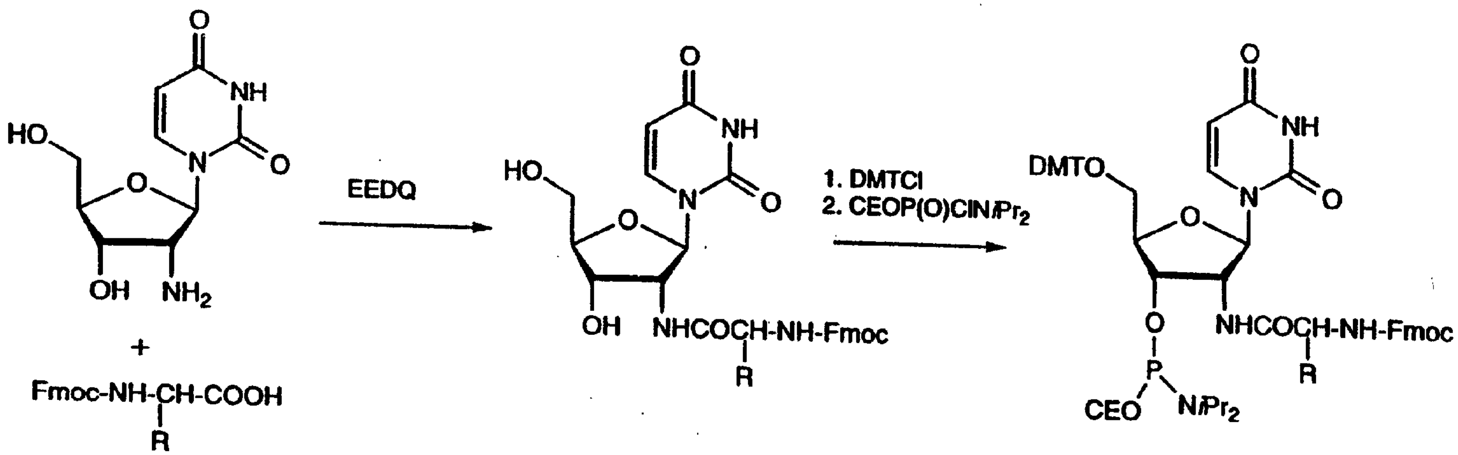


EEDQ = N-ethoxycarbonyl-2-ethoxy-1,2-dihydroquinoline

Fmoc = 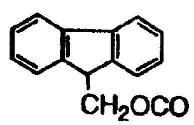

R = $CH_3$ (ala), 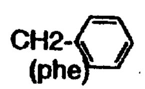 (phe), $(CH_2)_4$NH-Fmoc (lys), $(CH_2)_4$NH-CBZ (lys), $CH_2COOBzl$ (asp)

CBZ = 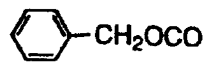

Bzl = 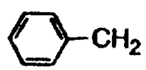

*FIG. 92.*

FIG. 93a.

A

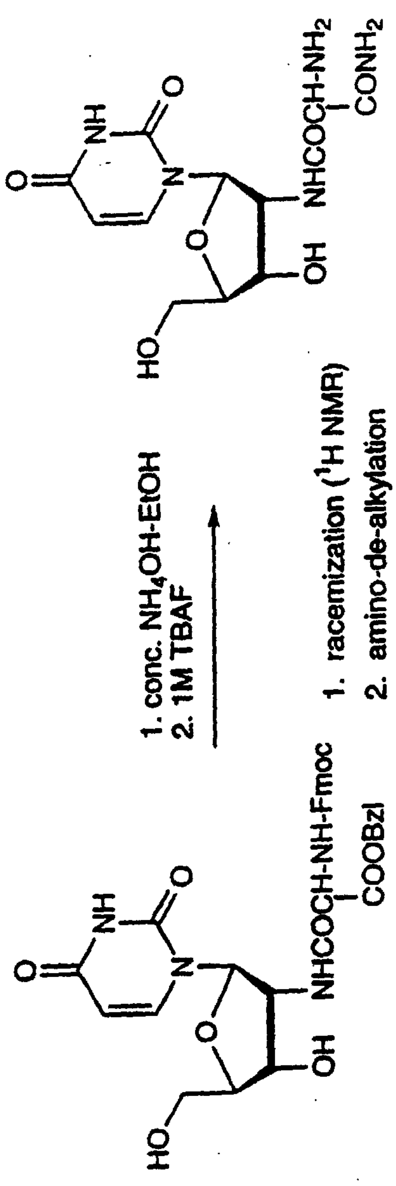


FIG. 93b.

B

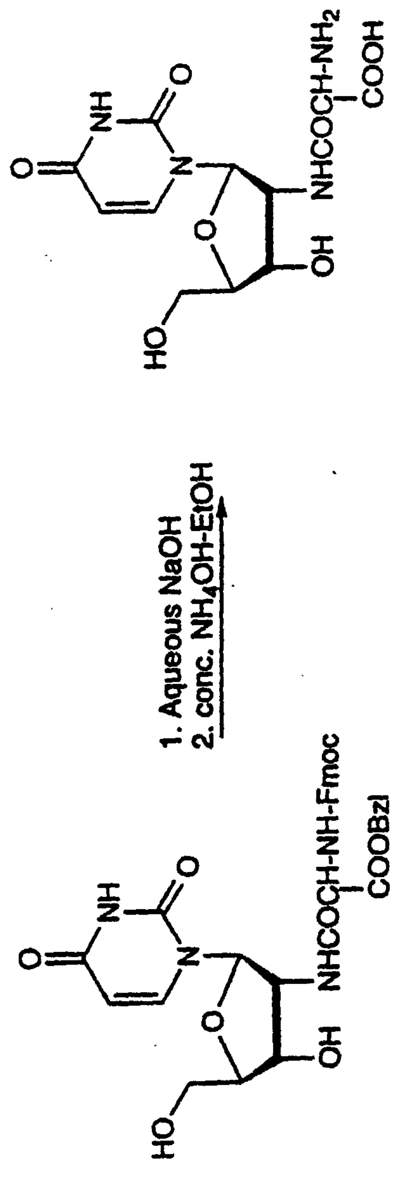

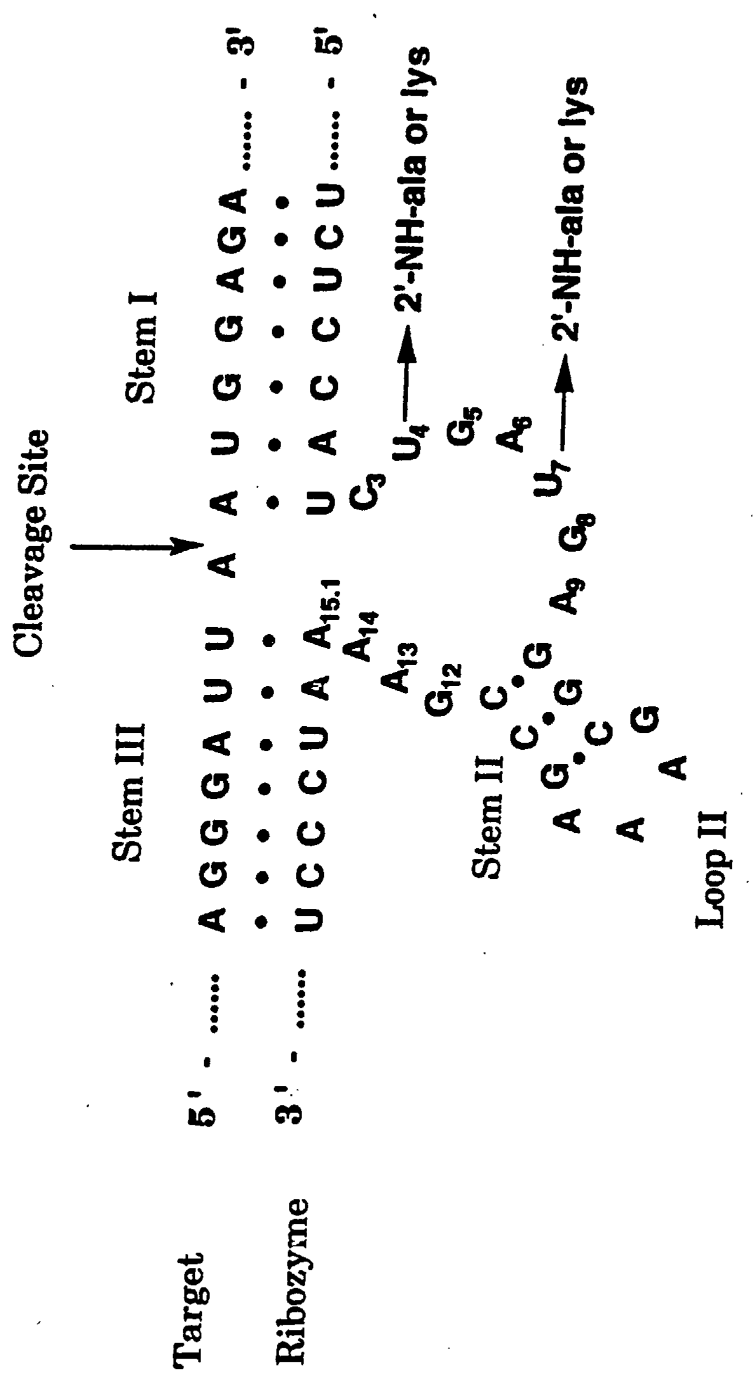

Cleavage Site
Stem III
Stem I
Target
Ribozyme
5' - ...... A G G G A U U U A A U G G A G A ...... - 3'
3' - ...... U C C C U A A A U A C C U C U ...... - 5'
Stem II
Loop II
2'-NH-ala or lys
2'-NH-ala or lys


FIG. 94.

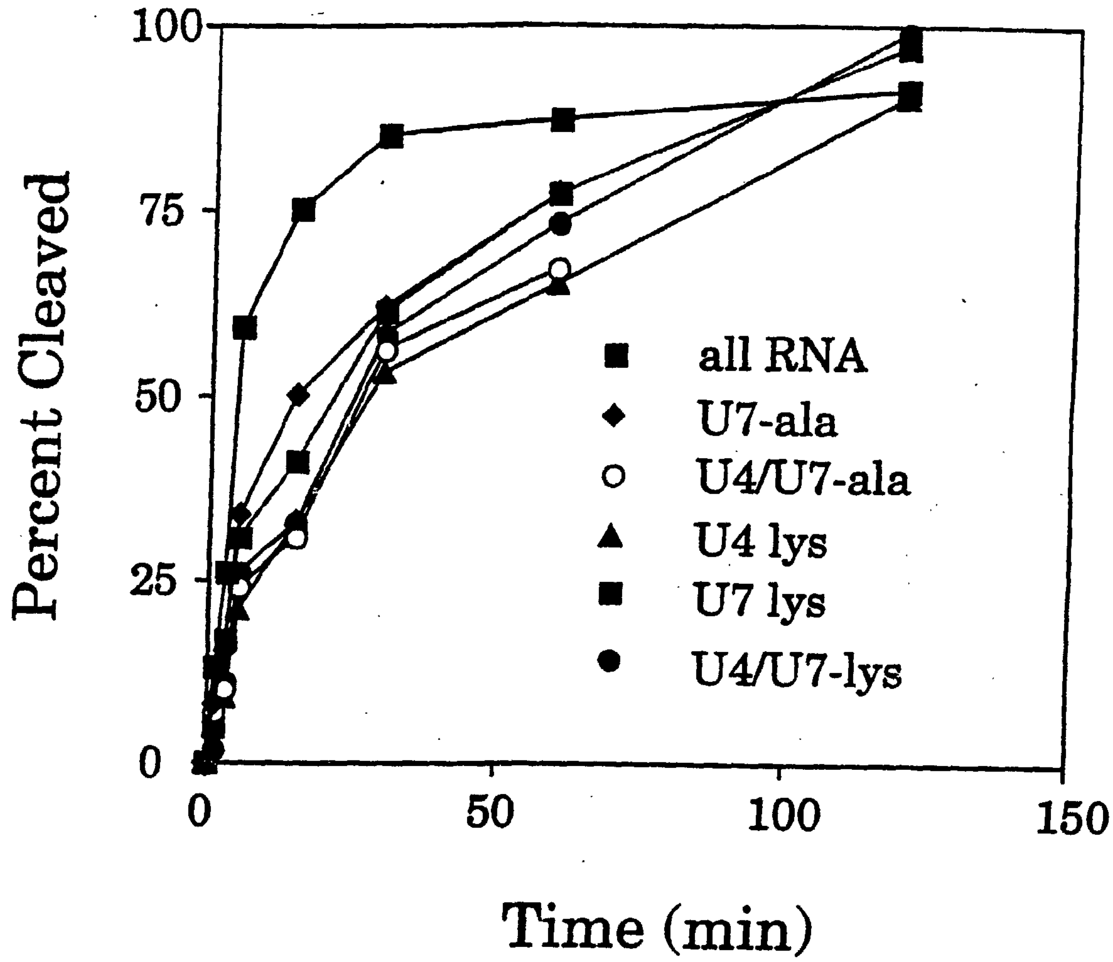


[Ribozyme] = 40 nM [Substrate] = ~1nM

FIG. 95.

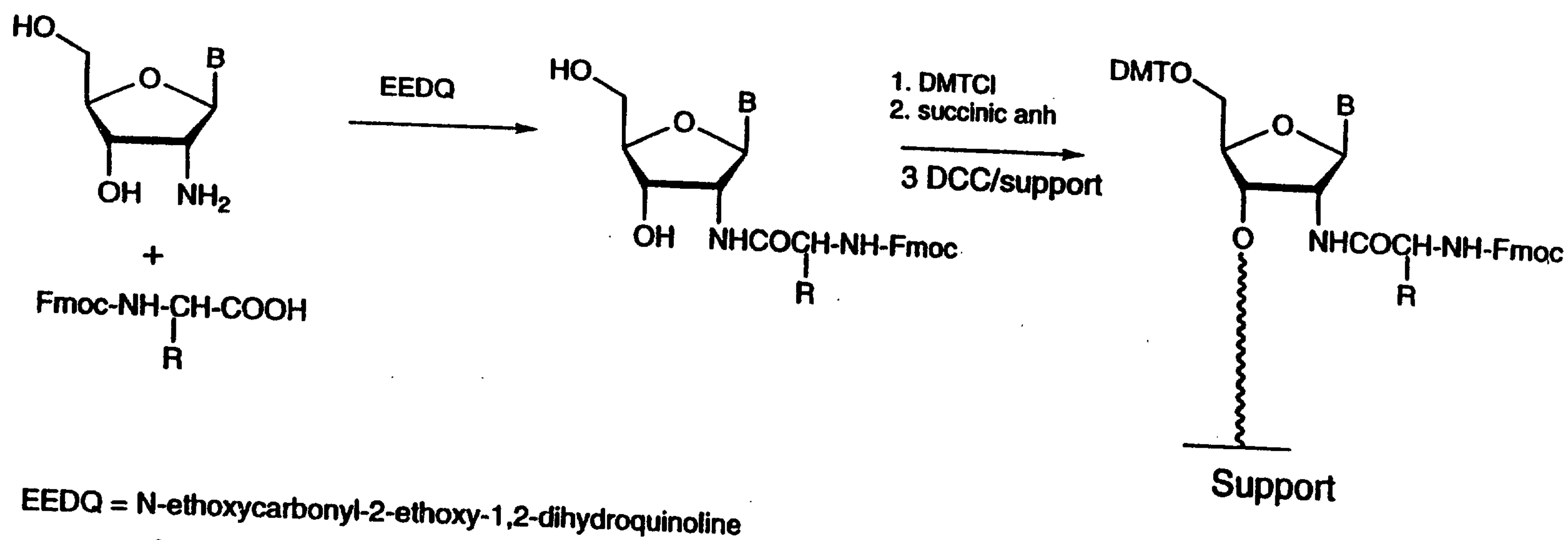


EEDQ = N-ethoxycarbonyl-2-ethoxy-1,2-dihydroquinoline

Fmoc = 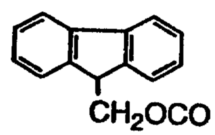

B= Ura, $Cyt^{bz}$, $Ade^{bz}$, $Gua^{ibu}$, mod. base, H

R = CH3 (ala), CH2- 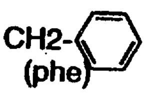 (phe), $(CH_2)_4$NH-Fmoc (lys), $(CH_2)_4$NH-CBZ (lys), $CH_2$COOBzl (asp)

CBZ = 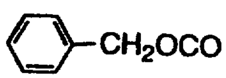

Bzl = 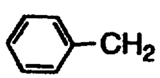

FIG. 96.

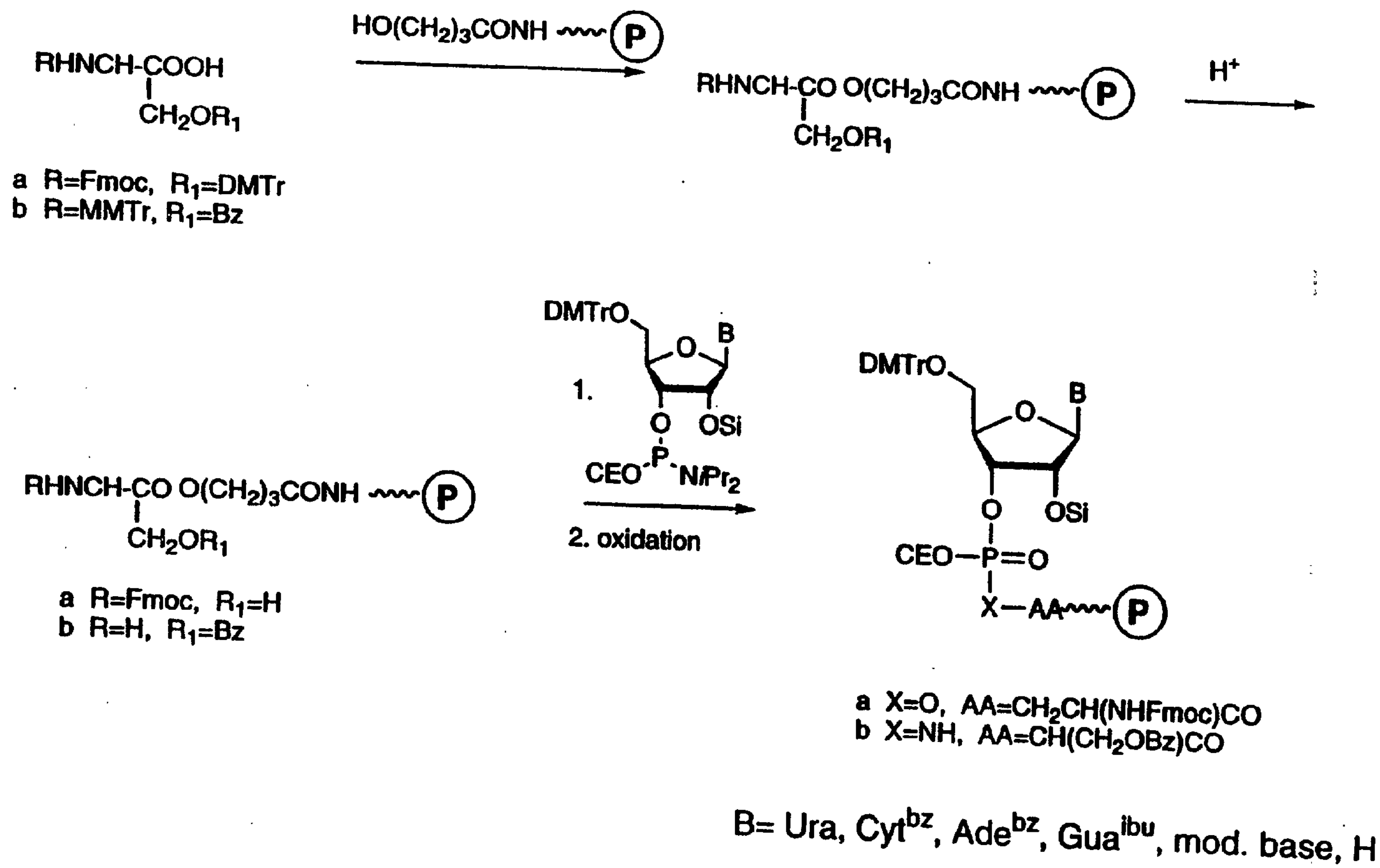

a R=Fmoc, $R_1$=DMTr
b R=MMTr, $R_1$=Bz
H⁺
a R=Fmoc, $R_1$=H
b R=H, $R_1$=Bz
1.
2. oxidation
a X=O, AA=$CH_2CH(NHFmoc)CO$
b X=NH, AA=$CH(CH_2OBz)CO$
B= Ura, $Cyt^{bz}$, $Ade^{bz}$, $Gua^{ibu}$, mod. base, H


FIG. 97.

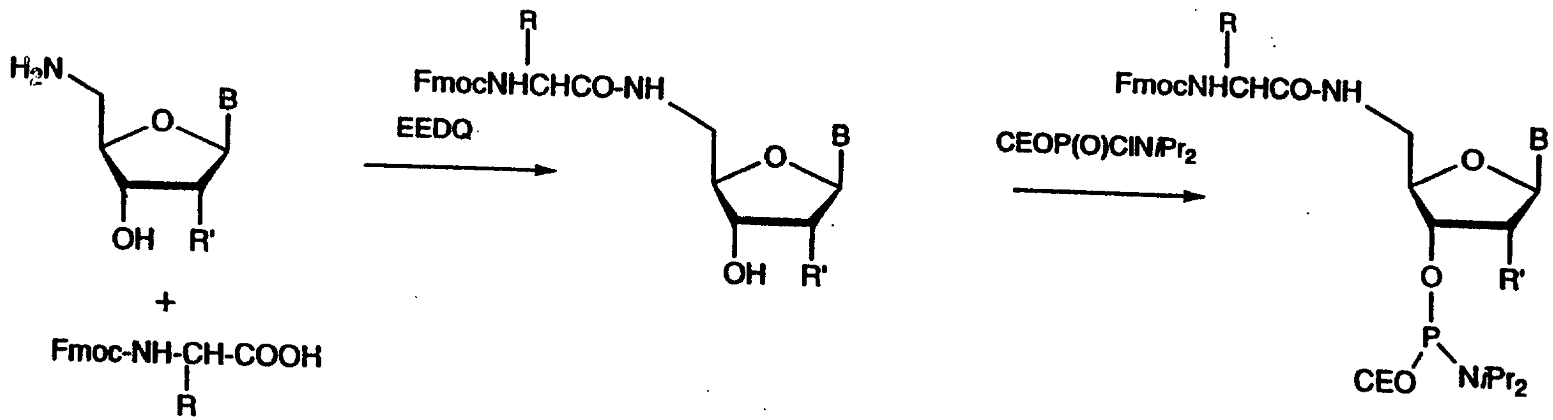


EEDQ = N-ethoxycarbonyl-2-ethoxy-1,2-dihydroquinoline

Fmoc = 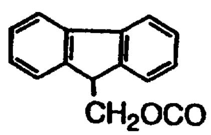

R = CH3 (ala), CH2-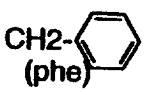 (phe), $(CH_2)_4$NH-Fmoc (lys), $(CH_2)_4$NH-CBZ (lys), $CH_2$COOBzl (asp)

CBZ = 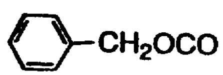

Bzl = 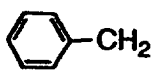

R'= H,OMe, OTBDMSi

B= Ura, Cyt$^{bz}$, Ade$^{bz}$, Gua$^{ibu}$, mod. base, H

FIG. 98.

FIG. 99.

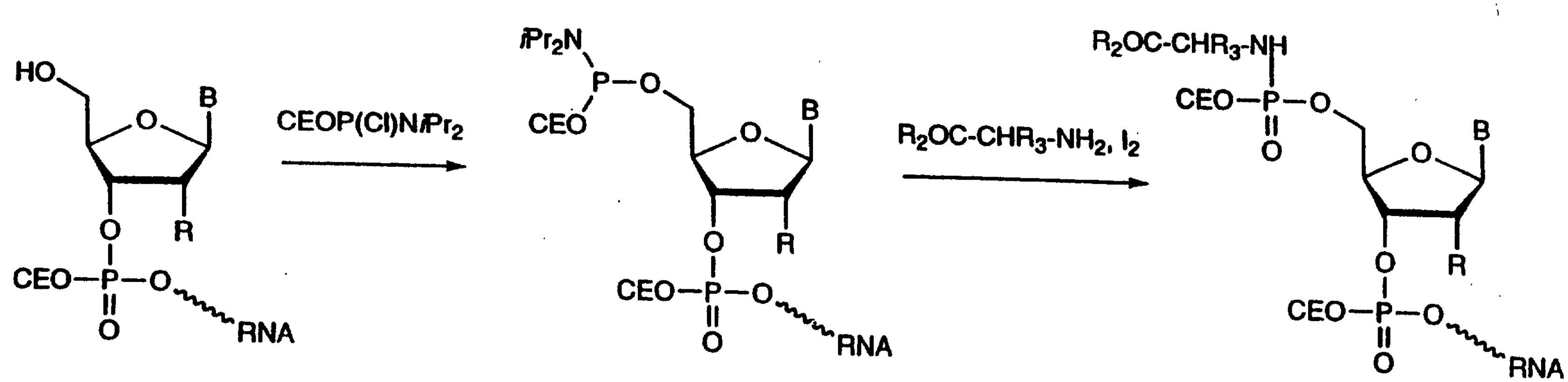


B =Ura, Cyt$^{bz}$, Ade$^{bz}$, Gua$^{ibu}$, mod. base, H
R = H, $OCH_3$, OTBDMS, Hal, $NHR_1$
$R_2$ = OBzl, peptidyl

FIG. 100.

Reversion of mutant RNA

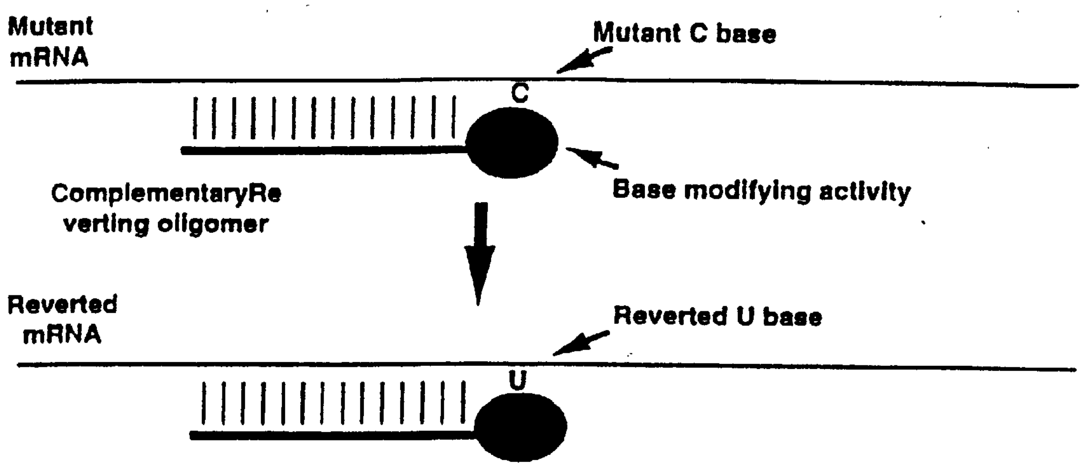


FIG. 101.

Reversion of mutant DNA

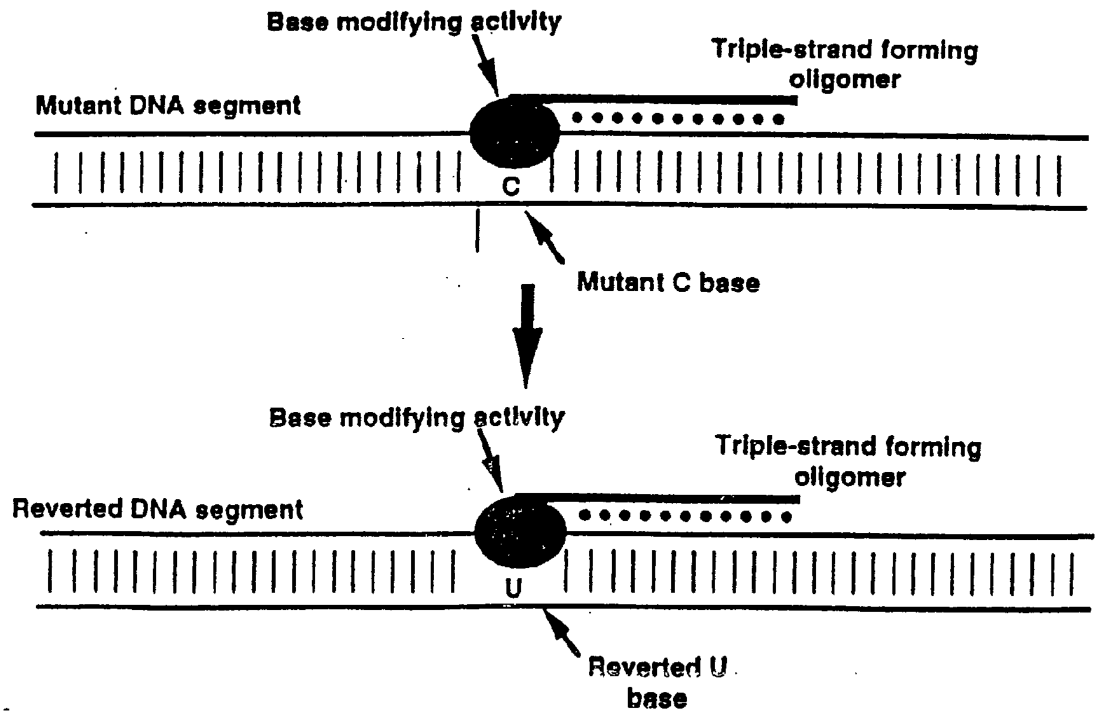

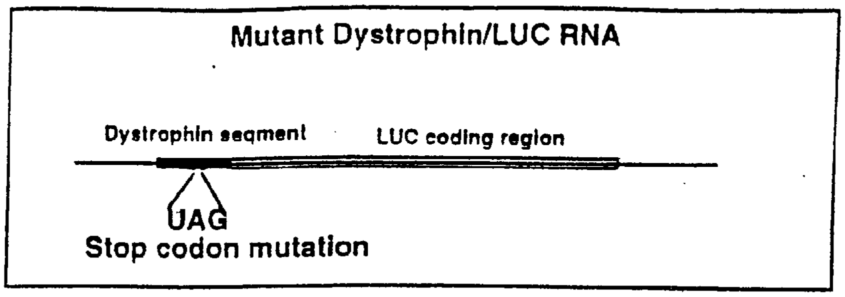


FIG. 102a.

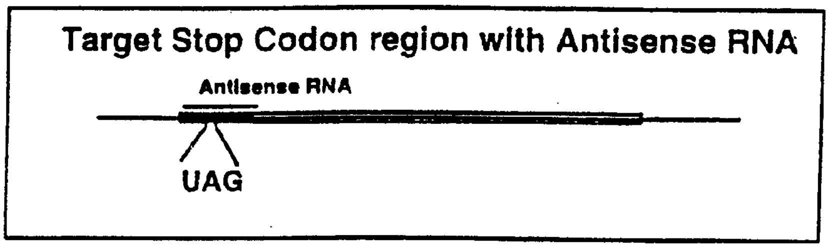


FIG. 102b.

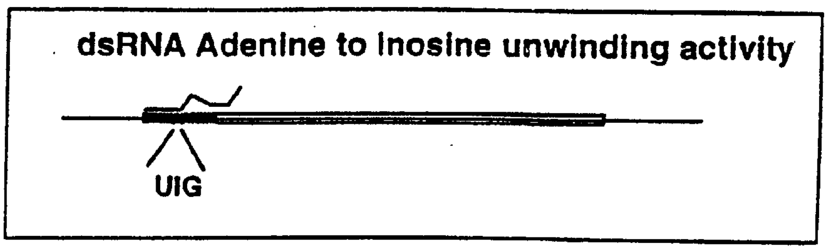


FIG. 102c.

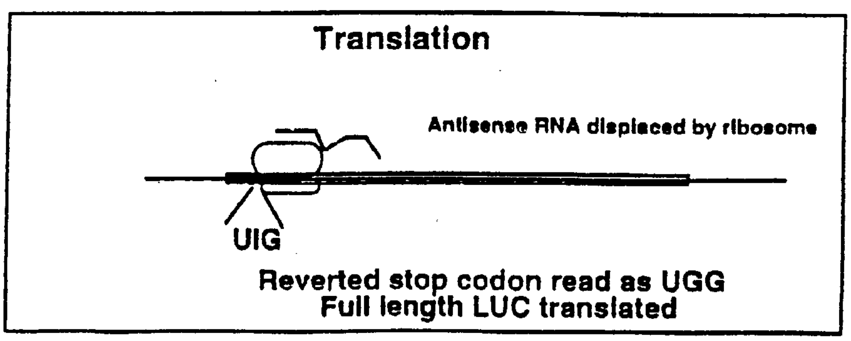


FIG. 102d.

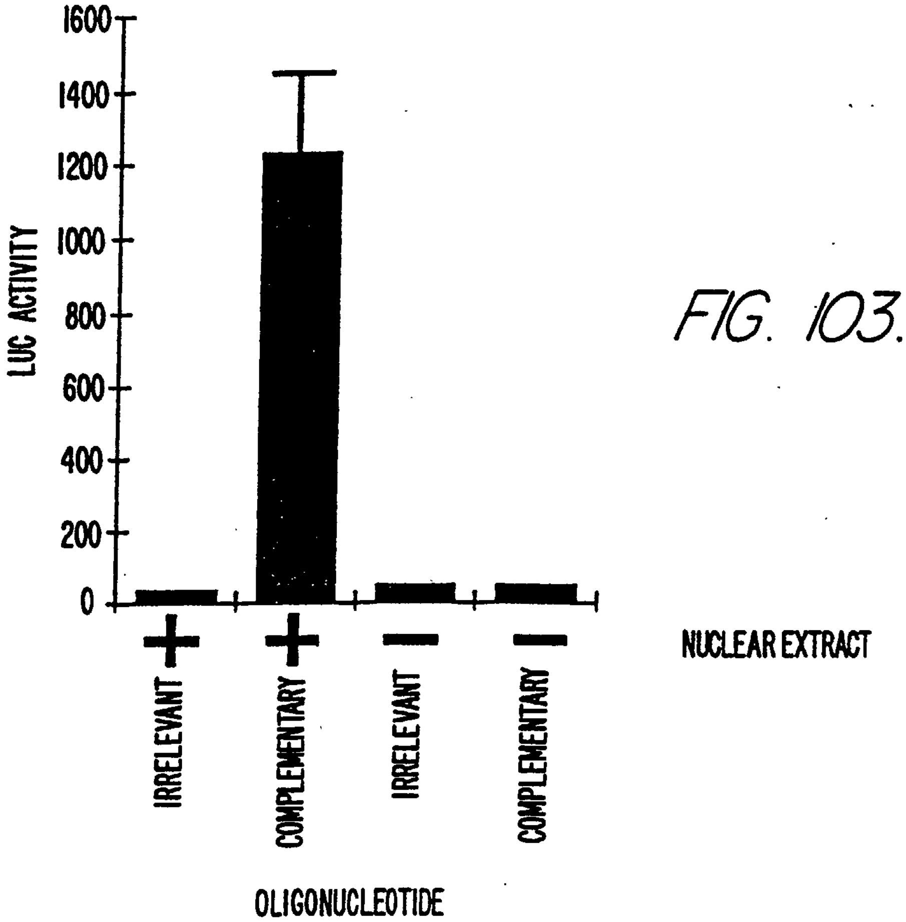
1600
1400
1200
1000
800
600
400
200
0
LUC ACTIVITY
+
+
−
−
NUCLEAR EXTRACT
IRRELEVANT
COMPLEMENTARY
IRRELEVANT
COMPLEMENTARY
OLIGONUCLEOTIDE

FIG. 103.

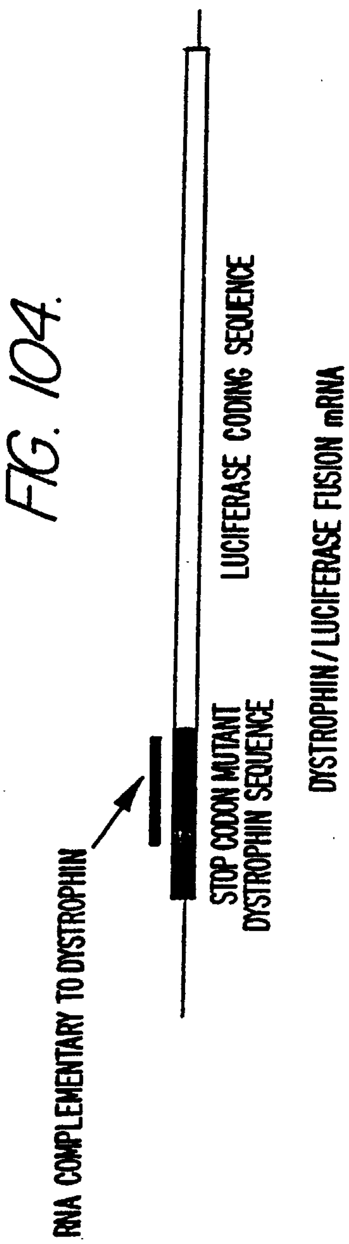
RNA COMPLEMENTARY TO DYSTROPHIN
STOP CODON MUTANT
DYSTROPHIN SEQUENCE
LUCIFERASE CODING SEQUENCE
DYSTROPHIN/LUCIFERASE FUSION mRNA

FIG. 104.

FIG. 105.

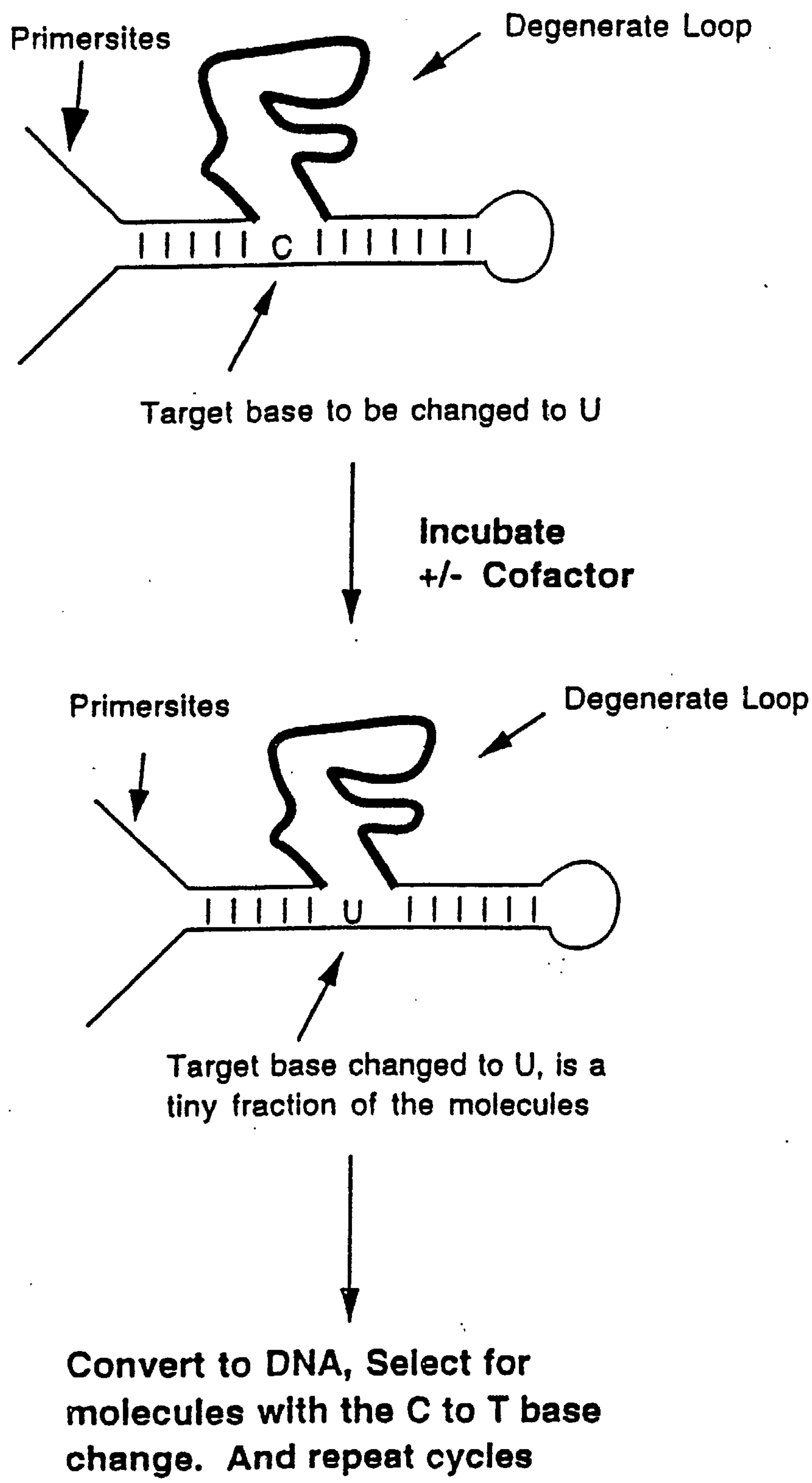
Primersites
Degenerate Loop
C
Target base to be changed to U
Incubate
+/- Cofactor
Primersites
Degenerate Loop
U
Target base changed to U, is a
tiny fraction of the molecules
Convert to DNA, Select for
molecules with the C to T base
change. And repeat cycles

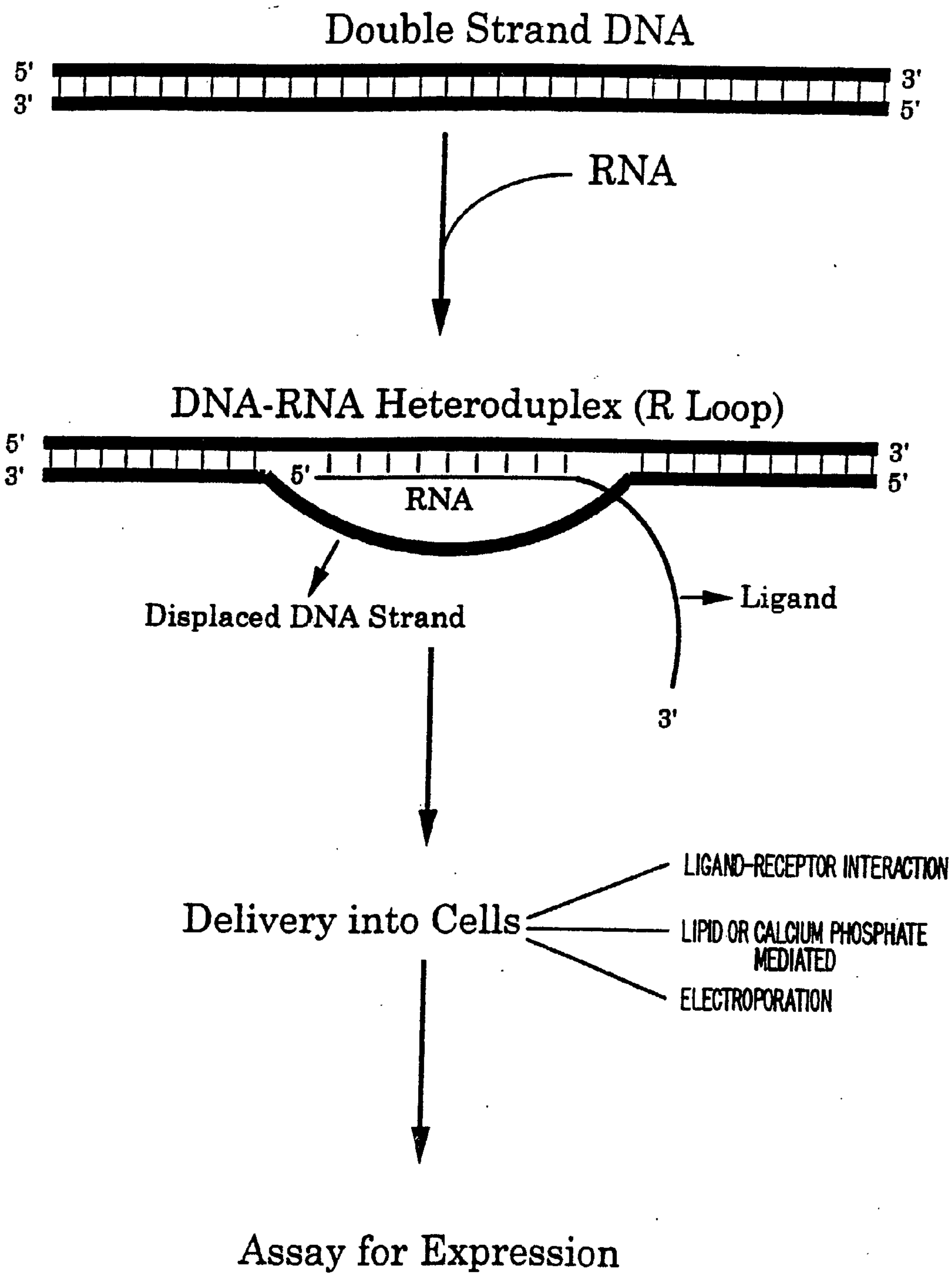
Double Strand DNA
5'
3'
3'
5'
RNA
DNA-RNA Heteroduplex (R Loop)
5'
3'
5'
RNA
3'
5'
Displaced DNA Strand
Ligand
3'
Delivery into Cells
LIGAND-RECEPTOR INTERACTION
LIPID OR CALCIUM PHOSPHATE MEDIATED
ELECTROPORATION
Assay for Expression

FIG. 106.

FIG. 107.

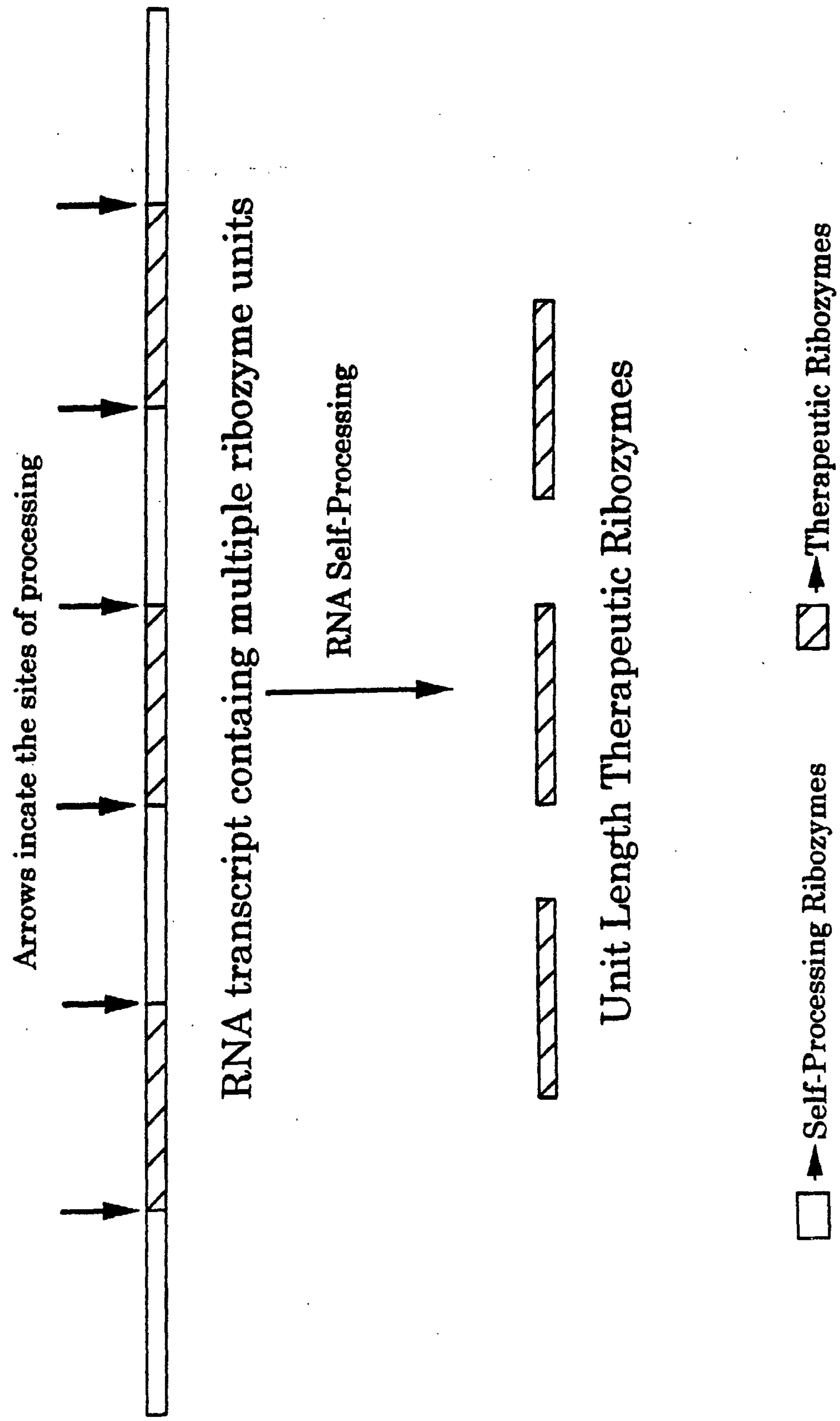
Arrows incate the sites of processing
RNA transcript containg multiple ribozyme units
RNA Self-Processing
Unit Length Therapeutic Ribozymes
Self-Processing Ribozymes
Therapeutic Ribozymes

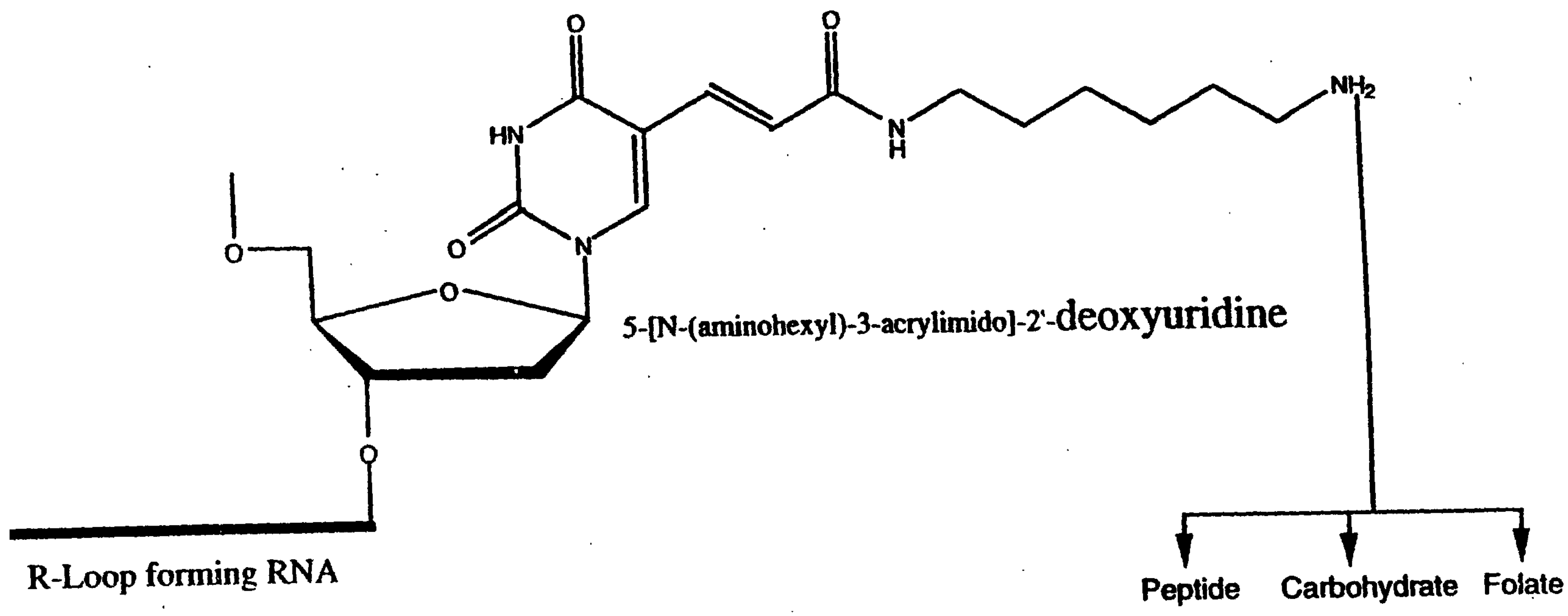


5-[N-(aminohexyl)-3-acrylimido]-2'-deoxyuridine

FIG. 108.